# EUROPEAN PATENT APPLICATION

(11) **EP 4 837 637 A2**
(43) Date of publication of application: **11.11.2026**
(21) Application number: 26198940.4
(22) Date of filing: 25.01.2022
(51) Int. Cl.: A61P 35/00

(54) **BIOACTIVE SUBSTANCE CONJUGATE, PREPARATION METHOD THEREFOR AND USE THEREOF**

(30) Priority: 09.02.2021 CN 202110178136; 30.03.2021 CN 202110340806; 21.07.2021 CN 202110825932; 21.07.2021 CN 202110825922; 21.07.2021 CN 202110825906
(62) Document number(s) of the earlier application(s) in accordance with Art. 76 EPC: 22752132.5 / 4 257 154
(71) Applicant: Medilink Therapeutics (Suzhou) Co., Ltd., Suzhou, Jiangsu 215000 (CN)
(72) Inventor: CAI, Jiaqiang, Suzhou, Jiangsu 215000 (CN)
(74) Representative: Mewburn Ellis LLP

(57) **Abstract**

A bioactive substance conjugates, a preparation method therefor and the use thereof. The present disclosure relates to ligand-drug conjugates, as represented by formula XV, a preparation method therefor, and the use thereof in the prevention and/or treatment of diseases related to abnormal cell activity, including but not limited to the use in the prevention and/or treatment of tumor diseases.

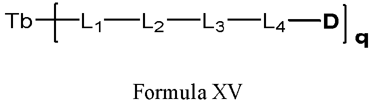

## Description

The present application claims the priority of Chinese patent application No. 2021101781361 filed on February 9, 2021, the priority of Chinese patent application No. 2021103408065 filed on March 30, 2021, the priority of Chinese patent application No. 202110825932X filed on July 21, 2021, the priority of Chinese patent application No. 2021108259226 filed on July 21, 2021, wherein a sequence listing in txt format was also filed along with the Chinese patent application No. 2021108259226 on July 21, 2021, the priority of Chinese patent application No. 2021108259067 filed on July 21, 2021, wherein a sequence listing in txt format was also filed along with the Chinese patent application No. 2021108259067 on July 21, 2021, the contents of which are all incorporated herein by reference in their entirety. The sequence listing contained in the present application is part of the specification, which is incorporated herein by reference in its entirety.

### Technical field

The present disclosure belongs to the field of pharmaceutical technology, and relates to bioactive substance conjugates (ligand drug conjugates), compounds, drug linker conjugates, and methods for preparing the same, as well as their use in the prevention and/or treatment of diseases associated with abnormal cell activity, including but not limited to the prevention and/or treatment of tumor diseases.

### Background technology

Chemotherapy using cytotoxic agents was once the standard treatment for cancer, but highly lethal cytotoxic molecules can kill normal cells, causing serious toxic side effects. Tumor targeting drugs have become a hot topic in the field of cancer research due to their simultaneous tumor specificity and anti-tumor activity. However, high toxicity and side effects are often produced due to the problems such as target selectivity of targeting drugs, which often limit the therapeutic effect of such targeting drugs. Biologics, such as antibodies or antibody fragments, although highly target selective, often have limited or no therapeutic effect on solid tumors. ADC is a conjugate of antibody and small molecule drug, which combines the targeting effect of antibody and the activity of bioactive molecules. ADC has become a biological missile with promising advantages of efficacy and safety. The Antibody guides the ADC to bind to target cells, which are subsequently internalized by cells, and then small molecule drugs are released inside the cells by enzymatic hydrolysis under the action of specific enzymes to treat diseases.

ADC drug research has developed rapidly in recent years, 14 ADCs have been approved and marketed: (1) Mylotarg (Gemtuzumab Ozogamicin, gemtuzumab (CD33)-calicheamicin), for CD33-positive acute myeloid leukemia (AML), approved by FDA in 2000, withdrawn from the market in 2010, and then re-approved in 2017; (2) Adcetris (Brentuximab Vedotin, CD30 monoclonal antibody-MMAE), for classic Hodgkin's lymphoma and anaplastic large cell lymphoma, approved by FDA in 2011; (3) Kadcyla (Trastuzumab Emtansine, trastuzumab monoclonal antibody (Her2)-maytansine TM1), for HER2-positive breast cancer, approved by FDA in 2013; (4) Besponsa (Inotuzumab ozogamicin, CD22 monoclonal antibody-calicheamicin), for adult relapsed or refractory B-cell lymphocytic leukemia, approved in 2017; (5) Lumoxiti (Moxetumomab pasudotox-tdfk, CD22-Pseudomonas exotoxin), for relapsed or refractory hairy cell leukemia (HCL), marketed in 2018; (6) Polivy (Polatuzumab vedotin-piiq, CD79b-MMAE), for relapsed or refractory diffuse large B-cell lymphoma (R/R DLBCL), marketed in 2019; (7) Padcev (Enfortumab vedotin-ejfv, Nectin 4-MMAE), for locally advanced or metastatic urothelial cancer (UC), marketed in 2019; (8) Enhertu (Famtrastuzumab deruxtecan-nxki, Her2-topoisomerase I inhibitor), for breast cancer, marketed in 2019; (9) Trodelvy (Trop2-topoisomerase I inhibitor ADC), for triple-negative breast cancer, approved by FDA in April 2020; (10) BCMA ADC Belantamab mafodotin (GSK2857916); (11) CD19 ADC Zynlonta (loncastuximab tesirine); (12) EGFR ADC Akalux; (13) Her2 ADC Disitamab Vedotin RC48; (14) Tissue Factor ADC Tivadak (tisotumab vedotin).

ADC drugs consist of three components: antibody, bioactive molecule (drug molecule) and linker. The bioactive molecule is covalently coupled to the antibody via a linker. In addition to targeting and biological activity, the coupling mode of bioactive molecules and antibodies plays a key role for ADC drugs, which determines the uniformity and stability of drugs and greatly affects the pharmacokinetic properties and toxicity of ADC. The improvement of the stability of the linking part between bioactive molecules and antibodies will improve the stability of ADC during systematic circulation, reduce the release of drugs in non-targeted tissues, and thus relatively increase the amount of drugs brought into and around the target cells, increase the amount of bioactive molecules released in and around the target cells, and further enhance the efficacy and attenuate the toxicity. Therefore, increasing the stability of the connection between bioactive molecules and antibodies is one of the most important technical aspects in ADC drug research.

At present, there are two main ways of coupling between antibodies and linkers for 10 marketed ADCs: (1) Lysine is the most common linking site in antibodies, and its ε-amino group can react with activated carboxyl group of a linker to form an amide bond. At present, there are technologies that can realize site-specific coupling of this kind, that is, activating the carboxyl group of the linker with an activating group, and then forming an amide bond with the specific lysine ε-amino group in the antibody to complete the coupling. On the one hand, site-specific quantitative coupling through such amide bonds does not have broad-spectrum practicability and can only be used under certain specific conditions; on the other hand, under the action of enzymes *in vivo,* hydrolysis is easy to take place, resulting in the dissociation of bioactive molecules from antibodies before reaching the target cells and thus increasing toxicity. (2) The cysteine thiol (SH) of the antibody exists in the form of disulfide bonds. Cleaving the disulfide bonds in the antibody can provide multiple free thiol groups as coupling sites. For coupling with thiol of the antibody, one such method is the Michael addition reaction between free thiol in the antibody and a maleimide, or double Michael addition reactions between specific substrates and free thiol in the antibody to form a structurally unique sulfur bridge. However, there are reports in the literatures that 30% or more ADCs obtained by thiol-Michael addition method can undergo retro Michael addition in systemic circulation, resulting in early toxin shedding and toxic reactions.

Antibody drug conjugates can be divided into four generations according to their development history:
The first-generation antibody drug conjugates are based on mouse-derived antibodies, which often have strong toxic side effects due to their complex immunogenicity;
The second-generation antibody drug conjugates use humanized antibodies or fully human antibodies on the basis of the first generation. The drug-ability of the second-generation antibody drug conjugates have been greatly improved. The first two generations adopted a relatively random coupling between the antibody and the drugs, so the final antibody-conjugated drug product is a multi-component mixture. This kind of drug product is not only toxic, but also difficult to control in terms of quality;
The third-generation antibody drug conjugates simultaneously apply the site-specific quantitative conjugation of toxin-linker and antibody on the basis that humanized or all-human antibodies are used, thus generating homogeneous single-molecule antibody drug conjugates. The toxicity and quality control of these antibody drug conjugates are greatly improved compared with the second-generation;
The fourth generation of antibody drug conjugates has innovated in the toxin-linker part from the third generation. The fourth generation of antibody drug conjugates changed the strategy of using highly toxic toxins for antibody drug conjugatesin the past, and selected relatively low-toxicity camptothecin molecules as bioactive components, and at the same time used high toxin-antibody ratio (DAR), such as a DAR value of 8. This type of ADC can also achieve a good therapeutic effect on tumors with low tumor surface antigen expression. Despite a late start, the fourth generation of antibody drug conjugates has quickly gained clinical recognition due to their remarkable therapeutic efficacy. The representative drugs Enhertu and Trodelvy have been approved by FDA for marketing. However, the fourth-generation ADC still has problems such as poor stability or low solubility, and the existing technology lacks universality. It is still necessary to further develop new toxin-linkers to solve these problems.

Existing antibody drug conjugates function through binding of the antibodies in ADCs to tumor cell surface antigens, and then internalize into endosomes via endocytosis, followed by transferring from endosomes to lysosomes, where bioactive molecules (toxins or payload) are then cleaved off from ADC by the action of lysosome enzymes. The released bioactive molecules move from the lysosome into the cytoplasm to kill tumor cells The bioactive molecules that escape after killing of tumor cells can further kill tumor cells nearby whose peripheral antigens are not expressed or have low expression via a so-called bystander effect. During the whole process, any changes during treatment such as antigen expression levels, weakening or even loss of endocytosis, loss of function of endosomes or lysosomes etc. can make ADC become treatment resistant or lose the therapeutic effect. Therefore, it will be of great significance in clinical treatment to find an ADC that is stable during systemic circulation *in vivo,* and at the same time, that can kill tumor cells by releasing toxins through extracellular cleavage in tumor microenvironment without the need of endocytosis. This kind of ADC should be able to overcome the mechanisms related multiple drug resistance problems of traditional ADC products.

Although there is evidence that Trodelvy from Immunomedics and Gilead can be cleaved outside tumor cells without endocytosis, however Trodelvy is highly unstable in plasma and aqueous solutions with a plasma half-life of less than 24 hours, this type of ADC can be suitable only for those composed of toxins with low biological activity, to avoid off-target toxic side effects caused by early cleavage of ADC toxins.

In addition, the function of the linker of traditional ADC is mostly for the lysosomal enzymatic cleavage in cells or to regulate water solubility, etc. How to use the linker to realize the enrichment of ADC in tumor tissues at the same time maintaining its enzymatic cleavage capability is also of great significance to reduce toxic side effects of ADC in normal tissues.

B7H3 is a type I transmembrane protein of the B7 family and has two isotypes, 2Ig-B7H3 and 4Ig-B7H3, in humans. B7H3 is widely expressed in normal tissues, only constitutively expressed in non-immune resting fibroblasts, endothelial cells, osteoblasts and amniotic fluid stem cells, and inducible expressed in activated T cells, NK cells, DC cells and macrophages, but no positive expression was detected in lymphoid organs (Chapoval, A.I., et al., B7H3: A costimulatory molecule for T cell activation and IFN production. Nature Immunology, 2001. 2(3): p. 269-274). Studies have shown that about 76.5% of patients with early-stage liver cancer have about 3 times higher serum B7H3 than normal persons (60.79±19.45 Vs 20.52±8.46 ng/mL), but the correlation with disease progression is unclear (Zhao, L., et al., Early Detection of Hepatocellular Carcinoma in Patients with Hepatocirrhosis by Soluble B7H3. Journal of Gastrointestinal Surgery. 21(5): p. 807-812).

Currently, there are therapeutic approaches aiming at B7H3 targets in preclinical studies. For example, antibodies against mouse B7H3 will enhance infiltrating CD8 positive T cells in tumors and inhibit tumor growth (Mod. Pathol. 2010 Aug; 23(8): 1104-12). In addition, WO2008/066691 showed that the antibody which recognizes B7H3 variant, B7H3a, has an antitumor effect against adenocarcinoma *in vivo.* In clinical studies, a combination of mouse B7H3 antibodies and radioiodine 131 can significantly inhibit the growth of neuroblastoma in patients (J Neurooncol 97(3): 409-18 (2010)).

### Summary of the invention

In order to improve the therapeutic effect of antibody drug conjugates (ADC) or other ligand drug conjugates (PDC), reduce toxic and side effects of drugs, and enlarge the therapeutic window, the present disclosure provides a ligand drug conjugate of formula (XV), or a pharmaceutically acceptable salt or solvate thereof, which contains a bioactive molecule (drug molecule), a linker and a targeting moiety that is linked to the linker via an reactive group (e.g., a sulfhydryl group) to form a ligand drug conjugate. In the present disclosure, an antibody containing a variable light chain domain and/or a variable heavy chain (VH) domain that binds B7H3 molecules has also been developed, which has derived from a human antibody.

Thus, in a first aspect of the present disclosure, the present disclosure provides a ligand drug conjugate of formula XV, 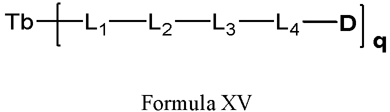
or a stereoisomer of said ligand drug conjugate, a prodrug thereof, a pharmaceutically acceptable salt thereof, or a pharmaceutically acceptable solvate thereof,
wherein:
   Tb is a ligand or a targeting moiety that binds to a target;
   q is a drug-to-ligand coupling ratio;
   D is a bioactive molecule fragment;
   L₁ is an extension unit;
   L₂ is absent or a linking unit; L₃ is selected from amino acid residues or short peptides consisting of 2-10 amino acid residues;
   L₄ is absent or present, and when L₄ is present, L₄ is selected from 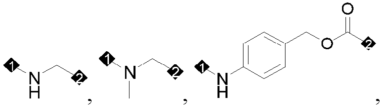 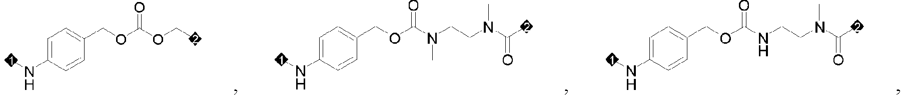 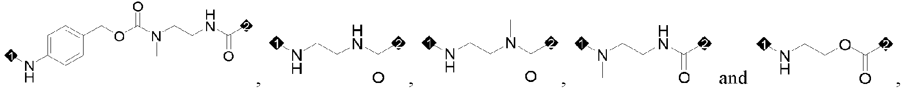
   wherein position 1 is attached to L₃ and position 2 is attached to D.

In addition, it should also be noted that, for "position 1 of L₁ is attached to Tb via S atom", it can be understood by those skilled in the art that position 1 of L₁ is connected to the sulfhydryl group contained in Tb after disulfide bonds are opened (for example, reduction of the disulfide bonds by the reducing agent TCEP can break the disulfide bond to generate sulfhydryl -SH groups). That is, -S- between L₁ and Tb is not an additional extraneous sulfur atom.

For example, in 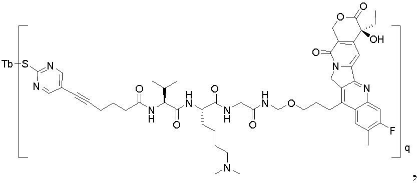 -S- is not an additional extraneous sulfur atom, but is -S- formed by linking the sulfhydryl group contained in Tb after opening the disulfide bond to position 1 of L₁ such as

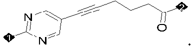

The extension unit is a component of a ligand drug conjugate or of drug linker conjugate or of linker, its function is to connect the ligand or targeting moiety that binds to a target with the rest of the ligand drug conjugate or the rest of the linker. The extension unit is capable of attaching Tb unit to either L₂ (if present) or L₃, and specific examples include, but are not limited to (wherein position 1 is attached to the ligand or targeting moiety that binds to a target, and position 2 is attached to L₂ or L₃):

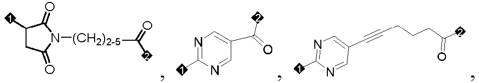

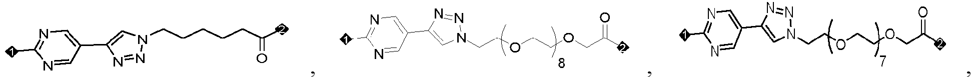

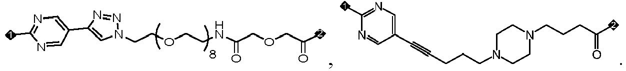

In some embodiments, L₁ is selected from: 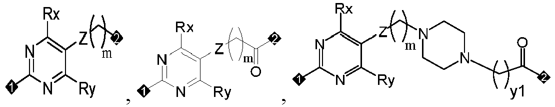 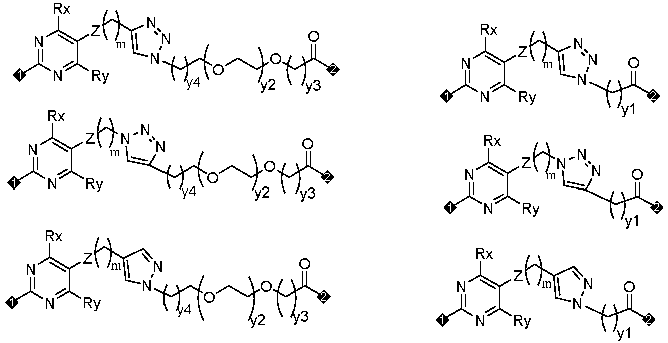 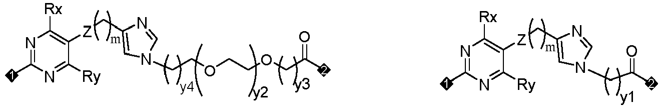 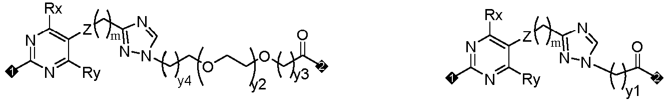 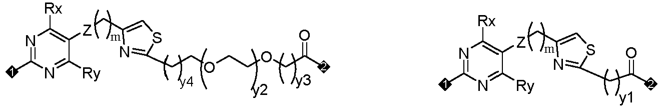 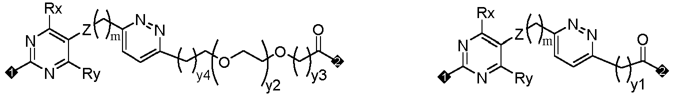 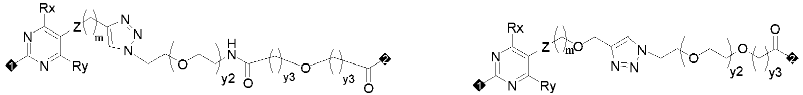 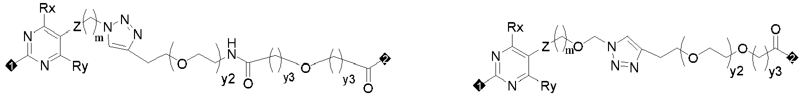 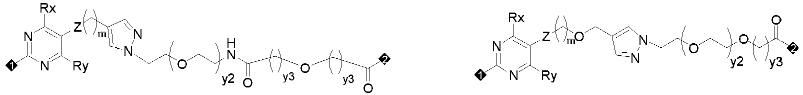 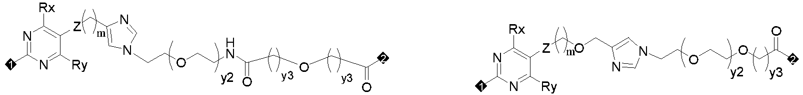 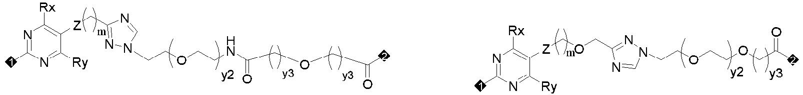 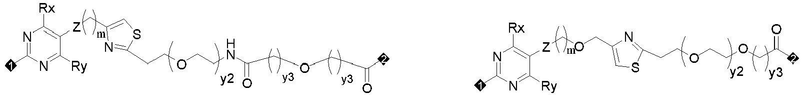 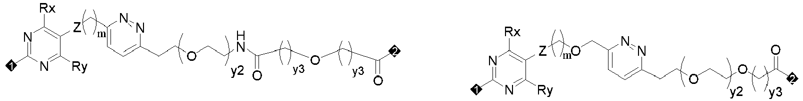 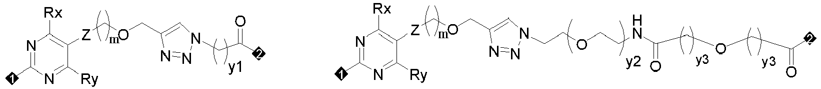 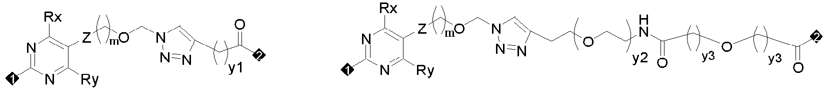 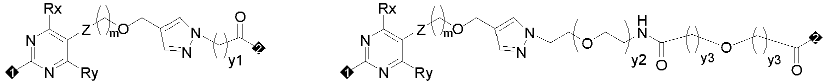 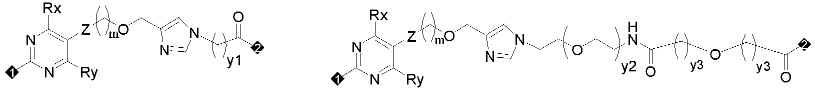 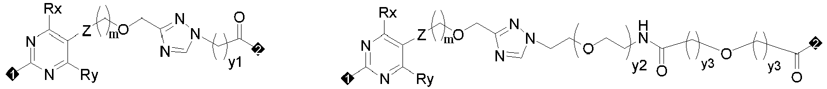 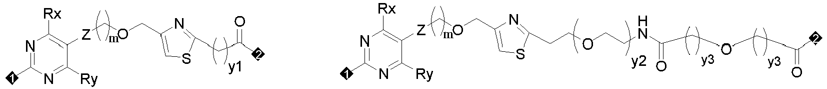 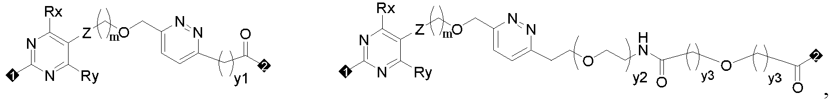
each Z is independently selected from a direct bond, a carbon-carbon triple bond, a carbon-carbon double bond, C6-10 aryl, 5-10 membered heteroaryl, amido, sulfonamido, imino, and CF₂;
Rx and Ry are each independently selected from H and C1-4 alkyl;
each m is independently selected from 0, 1, 2, 3, 4, 5 and 6;
y1, y2, y3, and y4 are each independently selected from any integer between 0 and 20;
position 1 is attached to Tb via an S atom, and position 2 is attached to L₂ or L₃.

The linking unit is a component of a ligand drug conjugate or of drug linker conjugate or of linker, its function is to bind the extension unit to amino acid residues or short peptides consisting of 2-10 amino acid residues. When the linking unit is present, it can connect L₁ to L₃. Specific examples include, but are not limited to (wherein position 1 is attached to the extension unit, and position 2 is attached to L₃): 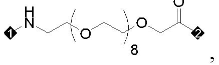 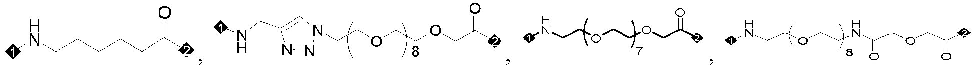 and

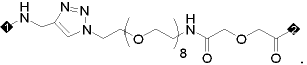

In some embodiments, L₂ is absent or present, and when L₂ is present, L₂ is selected from: 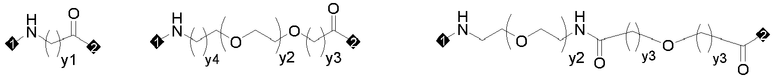 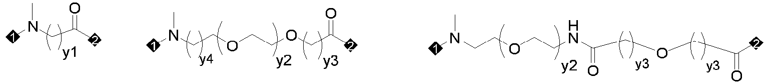 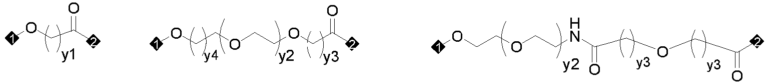 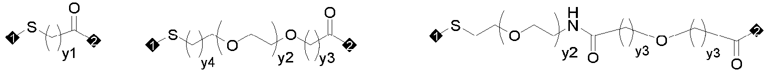 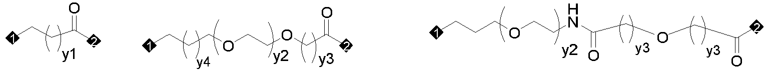 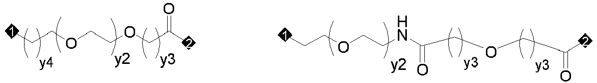 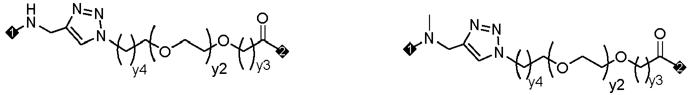 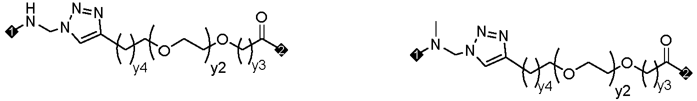 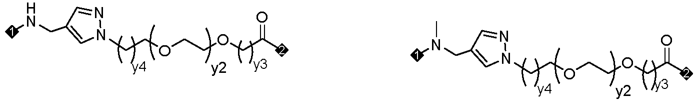 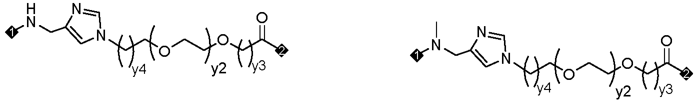 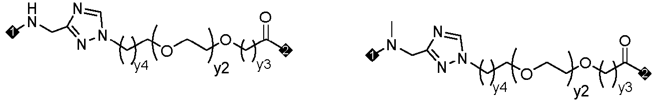 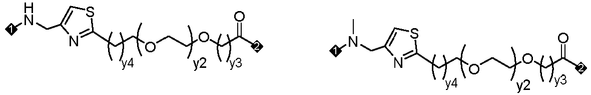 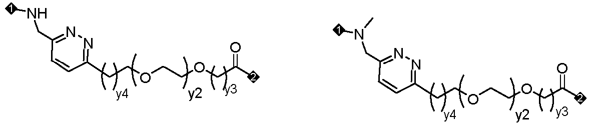 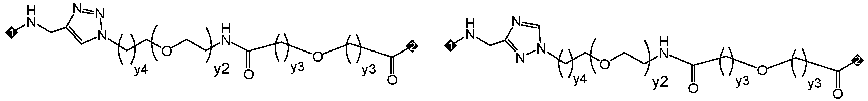 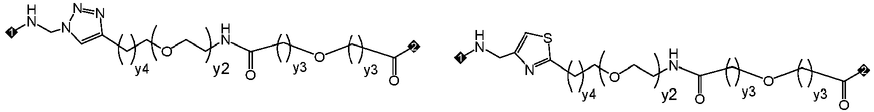 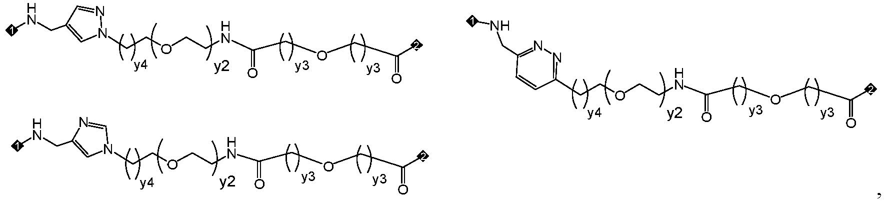 y1, y2, y3, and y4 are each independently selected from any integer between 0 and 20, position 1 is attached to L₁ and position 2 is attached to L₃.

In some embodiments, L₁ is

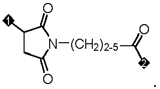

In some embodiments, L₁ is selected from 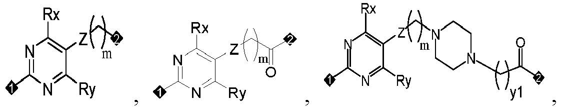 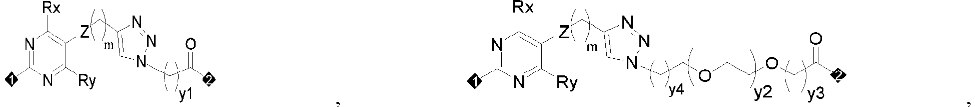 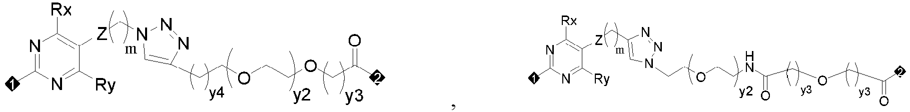 and 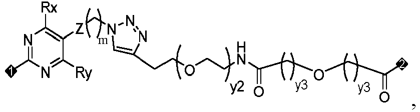 each Z is independently selected from a direct bond, a carbon-carbon triple bond, a carbon-carbon double bond, C6-10 aryl, 5-10 membered heteroaryl and amido (preferably selected from a direct bond, a carbon-carbon triple bond and a carbon-carbon double bond); Rx and Ry are each independently selected from H and C1-4 alkyl; each m is independently selected from 0, 1, 2, 3, 4, 5 and 6; y1 is selected from any integer between 1 and 6 (such as 4, 5, 6), each y2 is independently selected from any integer between 0 and 15 (such as between 6 and 15); each y3 is independently selected from 1, 2 and 3; and each y4 is independently selected from 0 and 1; position 1 is attached to Tb via an S atom, and position 2 is attached to L₂ or L₃.

In some embodiments, L₁ is selected from 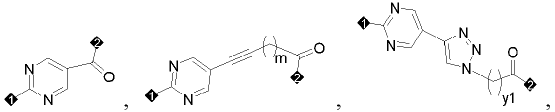 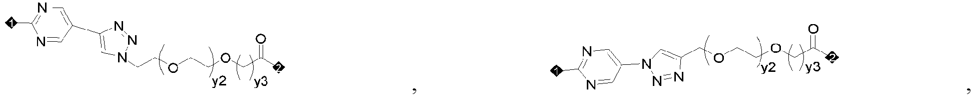 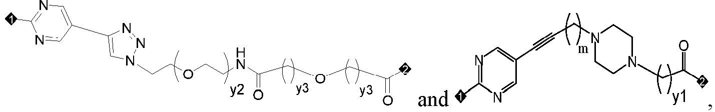 m is selected from 2, 3 and 4; y1 is selected from any integer between 1 and 6 (such as 4, 5, 6), each y2 is independently selected from any integer between 0 and 10 (such as between 6 and 10); each y3 is independently selected from 1 and 2, position 1 is attached to Tb via an S atom, and position 2 is attached to L₂ or L₃.

In some embodiments, L₁ is selected from 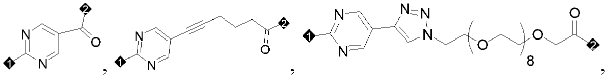 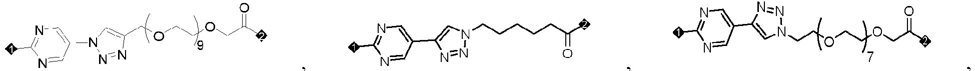 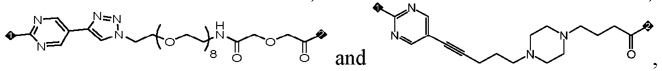 position 1 is attached to Tb via an S atom, and position 2 is attached to L₂ or L₃.

In some embodiments, L₁ is selected from

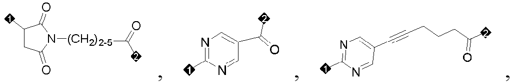

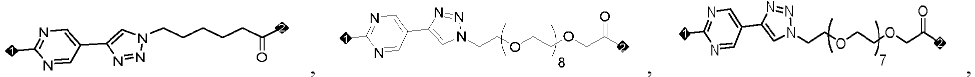

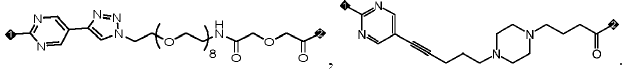

In some embodiments, L₁ is selected from 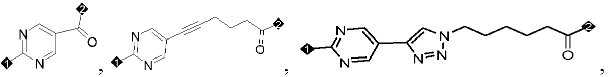 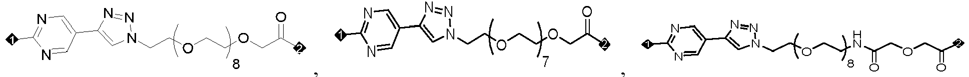 and 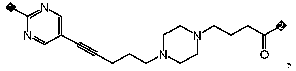 position 1 is attached to Tb via an S atom, and position 2 is attached to L₂ or L₃.

In some embodiments, L₁ is selected from 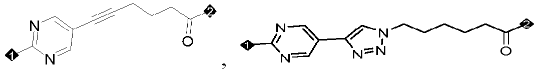 and 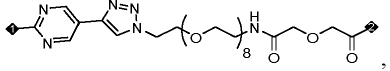 position 1 is attached to Tb via an S atom, and position 2 is attached to L₂ or L₃.

In some embodiments, L₁ is selected from 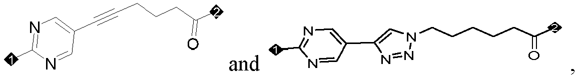 position 1 is attached to Tb via an S atom, and position 2 is attached to L₂ or L₃.

In some embodiments, L₂ is absent or present, and when L₂ is present, L₂ is selected from 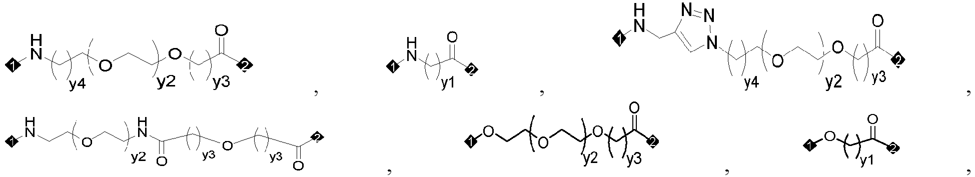 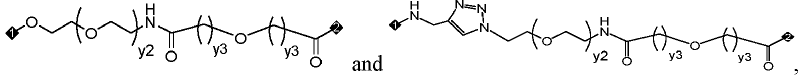 y1 is selected from any integer between 1 and 6 (such as 4, 5, 6), each y2 is independently selected from any integer between 0 and 10 (such as between 6 and 10); each y3 is independently selected from 1 or 2, each y4 is independently selected from 0 or 1, position 1 is attached to L₁, and position 2 is attached to L₃.

In some embodiments, L₂ is absent or present, and when L₂ is present, L₂ is selected from 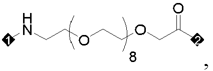 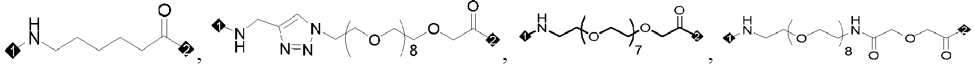 and 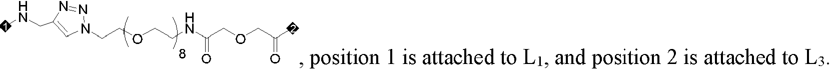 position 1 is attached to L₁, and position 2 in attached to L₃.

In some embodiments, L₂ is absent or present, and when L₂ is present, L₂ is selected from 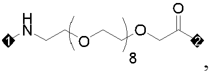 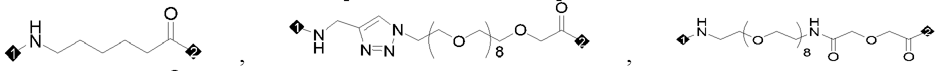 and 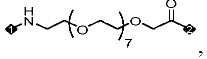 position 1 is attached to L₁, and position 2 is attached to L₃.

In some embodiments, L₂ is absent or present, and when L₂ is present, L₂ is selected from 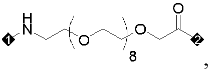 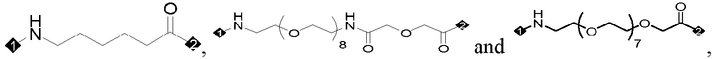 position 1 is attached to L₁, and position 2 is attached to L₃.

In some embodiments, L₂ is absent.

In some embodiments, L₂ is selected from

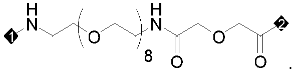

In some embodiments, L₃ is selected from an amino acid residue or short peptides consisting of 2-10 amino acid residues; the amino acid residues are selected from natural amino acid residues, non-natural amino acid residues, or selected from amino acid residues represented by AA¹ or stereoisomer thereof.

In some embodiments, L₃ is selected from amino acid residues Val, D-Val, Cit, Phe, Lys, Lys(Ac), Leu, Gly, Ala, Asn, Asp, Arg, and AA¹, or short peptides consisting of 2-10 amino acid residues selected from Val, Cit, Phe, Lys, D-Val, Leu, Gly, Ala, Asn, Asp, and AA¹.

In some embodiments, L₃ is selected from Val, Cit, Phe, Lys, D-Val, Leu, Gly, Ala, Asn, AA¹, Val-Cit, Cit-Val, Cit-Ala, Val-Ala, Lys-Val, Val-Lys(Ac), Phe-Lys, Phe-Lys(Ac), Ala-Ala, Val-AA¹, Ala-AA¹, Gly-AA¹, AA¹-Gly, Ala-Ala-Ala, Ala-Ala-Asn, Ala-Ala-Asp, Val-AA¹-Gly, Ala-AA¹-Gly, Gly-AA¹-Gly, Lys-Ala-Ala-Asn, Lys-Ala-Ala-Asp, Gly-Phe-Gly, Gly-Gly-Phe-Gly, D-Val-Leu-Lys, Gly-Gly-Arg, Ala-Ala-Asn, Gly-Gly-Phe, Val-Lys-Gly, Val-Lys-Gly-Gly, Val-Lys and Lys-Ala-Asn.

In some embodiments, L₃ is selected from AA¹, AA¹-Gly, Val-Cit, Val-AA¹-Gly, AA¹-Ala-Asn and Gly-Gly-Phe-Gly.

In some embodiments, L₃ is selected from AA¹ and Val-AA¹-Gly.

In some embodiments, L₃ is selected from Val-AA¹-Gly.

In some embodiments, L₃ is selected from 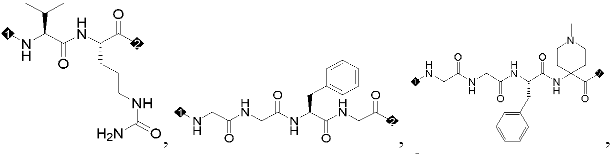 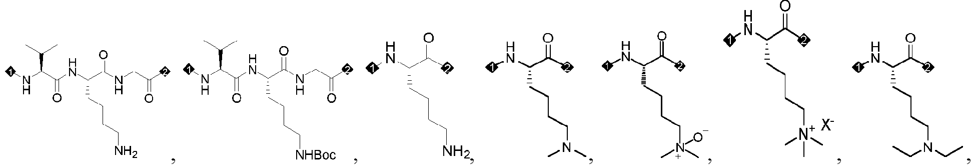 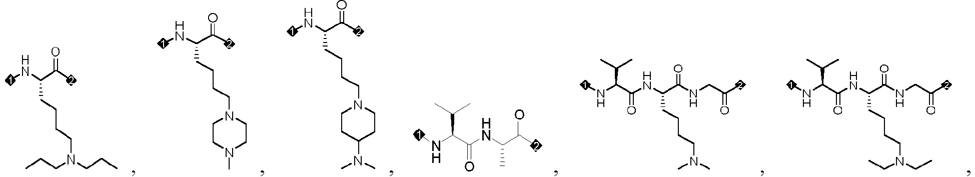 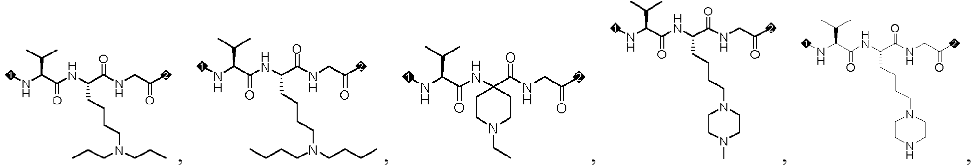 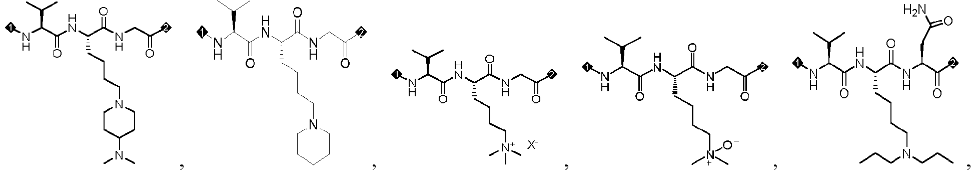 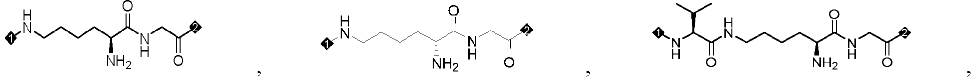 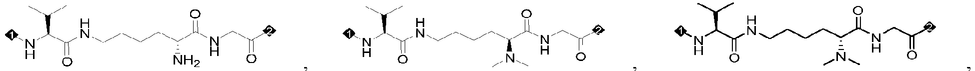 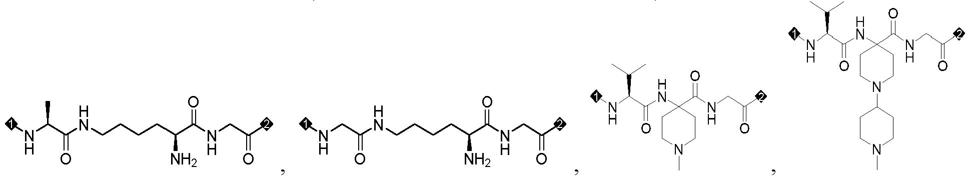 and

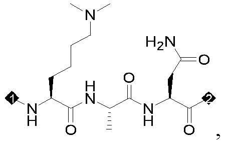

X⁻ is selected from halide ion, carboxylate ion, sulfate ion, hydrogen sulfate ion and OH⁻, position 1 is attached to L₁ or L₂, position 2 is attached to L₄ or D.

In some embodiments, L₃ is selected from 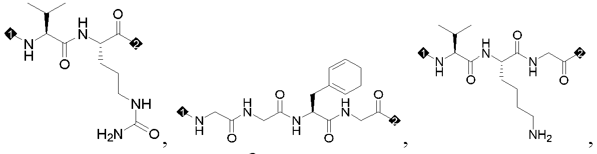 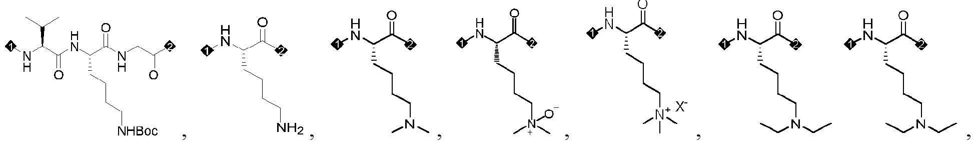 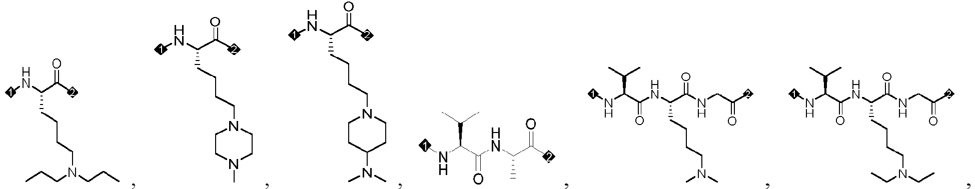 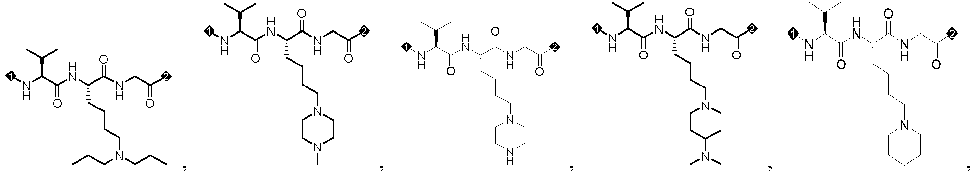 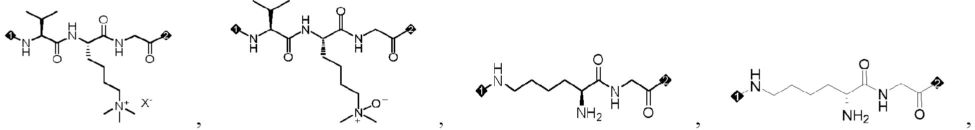 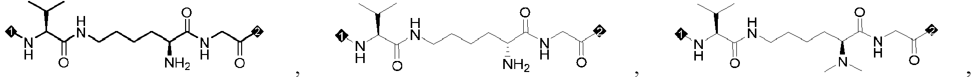 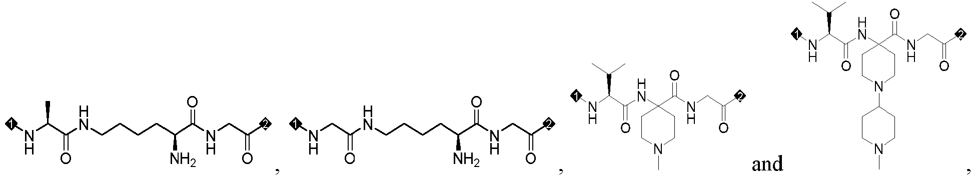 X⁻ is selected from halide ion, carboxylate ion, sulfate ion, hydrogen sulfate ion and OH⁻, position 1 is attached to L₁ or L₂, position 2 is attached to L₄ or D.

In some embodiments, L₃ is selected from 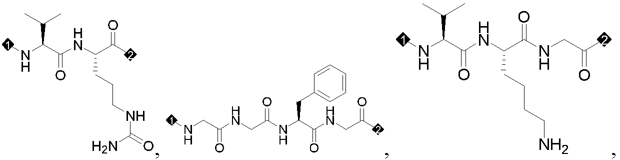 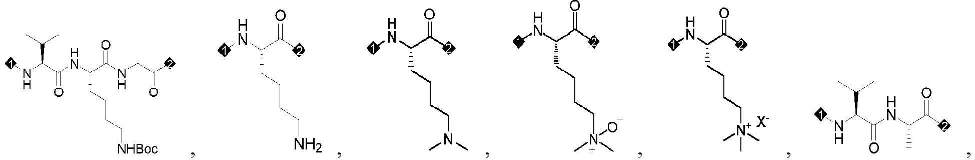 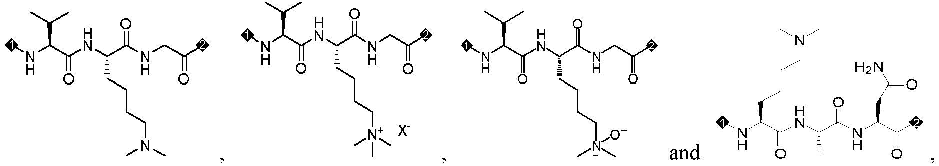 X⁻ is selected from halide ion, carboxylate ion, sulfate ion, hydrogen sulfate ion and OH⁻, position 1 is attached to L₁ or L₂, position 2 is attached to L₄ or D.

In some embodiments, L₃ is selected from 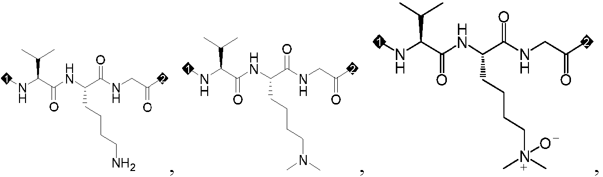 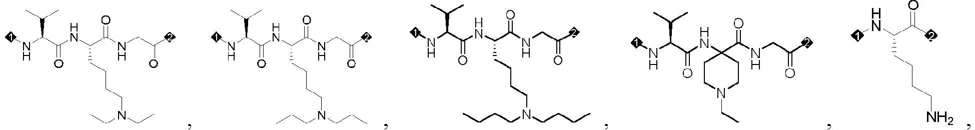 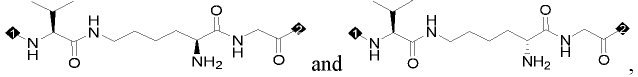 position 1 is attached to L₁ or L₂, position 2 is attached to L₄ or D.

In some embodiments, L₃ is selected from 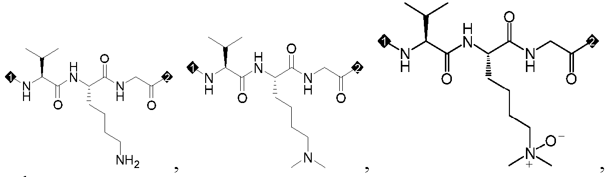 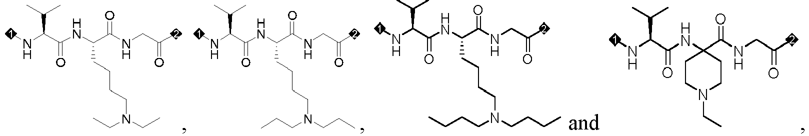 position 1 is attached to L₁ or L₂, position 2 is attached to L₄ or D.

In some embodiments, the structure of the amino acid residues represented by AA¹ is shown below, 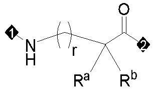 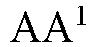 wherein:
R^{a} and R^{b} are each independently selected from H, 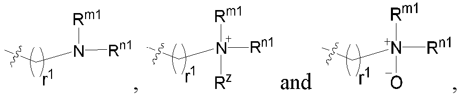 and R^{a} and R^{b} are not both H;
or, R^{a} and R^{b} together with the carbon atom to which they are both attached form a 4-10 membered heterocyclic ring, and said 4-10 membered heterocyclic ring is optionally substituted with one or more R⁰;
r, r¹ are each independently selected from any integer from 0 to 20;
R^{m1}, Rⁿ¹ are each independently selected from H, C1-6 alkyl, C3-6 cycloalkyl and -COOR^{x1};
R^{x1} is selected from C1-6 alkyl;
or, R^{m1} and Rⁿ¹ together with the nitrogen atom to which they are both attached form a 4-10 membered heterocyclic ring, said 4-10 membered heterocyclic ring is optionally substituted with one or more R^{0'};
R^{z} is selected from C1-6 alkyl;
R⁰, R^{0'} are each independently selected from C1-6 alkyl, C3-6 cycloalkyl, -NR^{m2}Rⁿ² and 4-10 membered heterocyclyl optionally substituted with C1-6 alkyl;
R^{m2}, Rⁿ² are each independently selected from H and C1-6 alkyl.

In some embodiments, one of R^{a} and R^{b} is H, and the other is selected from 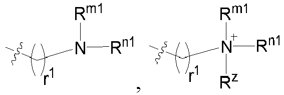 and

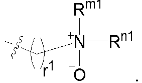

In some embodiments, one of R^{a} and R^{b} is H, and the other is selected from

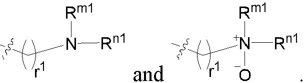

In some embodiments, R^{a} and R^{b} together with the carbon atom to which they are both attached form a 5-6 membered heterocyclic ring substituted with R⁰.

In some embodiments, R^{a} and R^{b} together with the carbon atom to which they are both attached form a piperidine ring or piperazine ring substituted with R⁰.

In some embodiments, R^{a} and R^{b} together with the carbon atom to which they are both attached form a piperidine ring substituted with R⁰.

In some embodiments, R^{a} and R^{b} together with the carbon atom to which they are both attached form 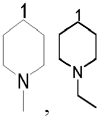 or 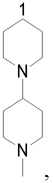 and the carbon atom marked with number 1 is the carbon atom to which R^{a} and R^{b} are both attached.

In some embodiments, R^{a} and R^{b} together with the carbon atom to which they are both attached form 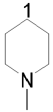 or 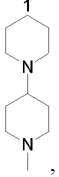 and the carbon atom marked with number 1 is the carbon atom to which R^{a} and R^{b} are both attached.

In some embodiments, r and r¹ are each independently selected from 0, 1, 2, 3, 4 and 5.

In some embodiments, r and r¹ are each independently selected from 0 and 4.

In some embodiments, one of r and r¹ is 0, and the other is 4.

In some embodiments, R^{m1} and Rⁿ¹ are each independently selected from H, methyl, ethyl, n-propyl, n-butyl, - COOCH3, -COOCH2CH3, -COOCH2CH2CH3, -COOCH(CH3)2, -COOC(CH3)3 and-COOCH2CH2CH2CH3.

In some embodiments, R^{m1} and Rⁿ¹ are each independently selected from H, C1-6 alkyl, C3-6 cycloalkyl and tert-butoxycarbonyl.

In some embodiments, R^{m1} and Rⁿ¹ are each independently selected from H and C1-6 alkyl.

In some embodiments, R^{m1} and Rⁿ¹ are each independently selected from H, methyl, ethyl and n-propyl.

In some embodiments, for r and r¹, when r is 4 and r¹ is 0, R^{m1} and Rⁿ¹ are each independently selected from H and C1-6 alkyl (such as H and CH₃); when r is 0 and r¹ is 4, R^{m1} and Rⁿ¹ are each independently selected from C1-6 alkyl (such as methyl, ethyl, n-propyl), and preferably selected from C2-6 alkyl (such as ethyl, n-propyl).

In some embodiments, R^{m1} and Rⁿ¹ together with the nitrogen atom to which they are both attached form a 5-6 membered heterocyclic ring optionally substituted with R^{0'}.

In some embodiments, R^{m1} and Rⁿ¹ together with the nitrogen atom to which they are both attached form a piperidine or piperazine ring optionally substituted with R^{0'}.

In some embodiments, R^{m1} and Rⁿ¹ together with the nitrogen atom to which they are both attached form 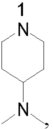 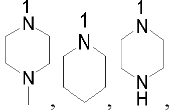 nitrogen atom marked with number 1 is the nitrogen atom to which R^{m1} and Rⁿ¹ are both attached.

In some embodiments, R^{z} is methyl.

In some embodiments, R⁰, R^{0'} are each independently selected from C1-6 alkyl, -NR^{m2}Rⁿ² and 5-6 membered heterocyclyl optionally substituted with C1-6 alkyl.

In some embodiments, R⁰ is selected from C1-6 alkyl and 5-6 membered heterocyclyl substituted with C1-6 alkyl, and said 5-6 membered heterocyclyl is selected from piperidinyl and piperazinyl.

In some embodiments, R⁰ is selected from methyl, ethyl and 5-6 membered heterocyclyl substituted with methyl, and said 5-6 membered heterocyclyl is piperidinyl.

In some embodiments, R⁰ is selected from methyl and 5-6 membered heterocyclyl substituted with methyl, and said 5-6 membered heterocyclyl is piperidinyl.

In some embodiments, R⁰ is selected from methyl, ethyl and

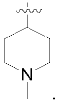

In some embodiments, R⁰ is selected from methyl and

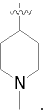

In some embodiments, R^{0'} is selected from C1-6 alkyl and -NR^{m2}Rⁿ².

In some embodiments, R^{0'} is selected from methyl and -NR^{m2}Rⁿ².

In some embodiments, R^{m2} and Rⁿ² are methyl.

In some embodiments, the amino acid residue represented by AA¹ is selected from

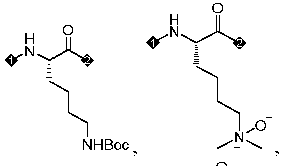

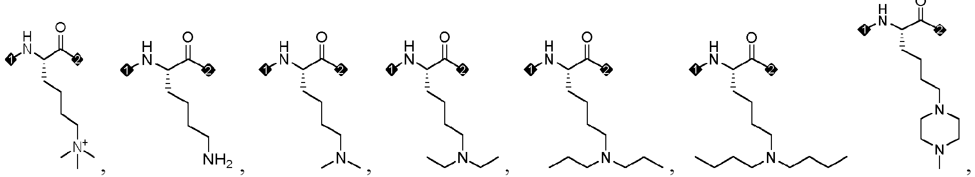

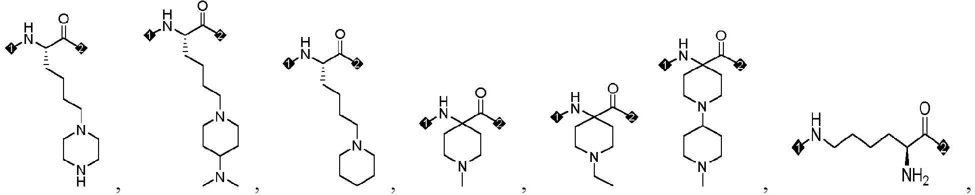

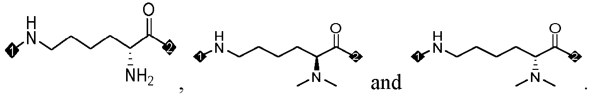

In some embodiments, the amino acid residue represented by AA¹ is selected from

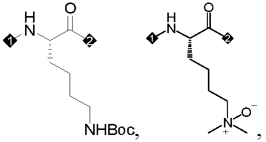

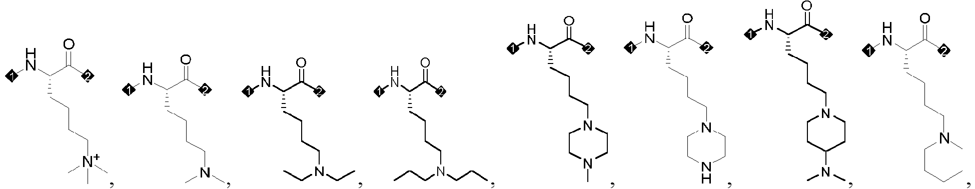

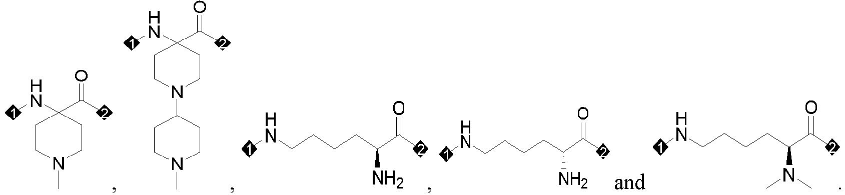

In some embodiments, the amino acid residue represented by AA¹ is selected from 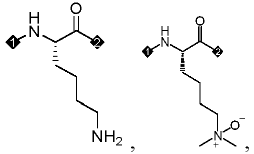 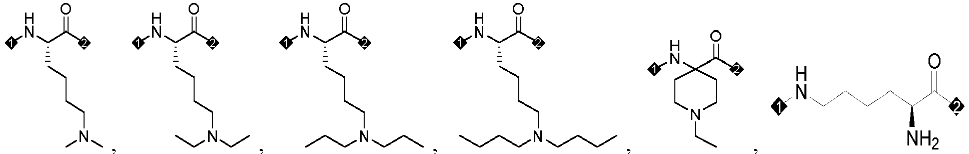 and

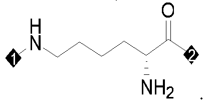

In some embodiments, the amino acid residue represented by AA¹ is selected from

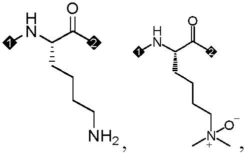

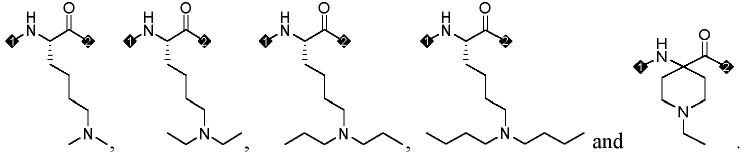

In some embodiments, L₄ is absent or present, and when L₄ is present, L₄ is selected from 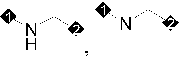 and 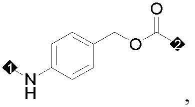 wherein position 1 is attached to L₃ and position 2 is attached to D.

In some embodiments, L₄ is absent or present, and when L₄ is present, L₄ is 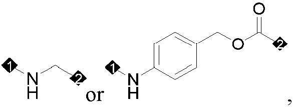 wherein position 1 is attached to L₃ and position 2 is attached to D.

In some embodiments, L₄ is absent.

In some embodiments, L₄ is selected from 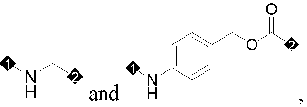 wherein position 1 is attached to L₃ and position 2 is attached to D.

In some embodiments, L₄ is selected from 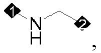 wherein position 1 is attached to L₃ and position 2 is attached to D.

In some embodiments, the structure 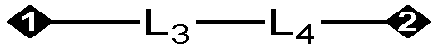 is selected from the following structural fragments:

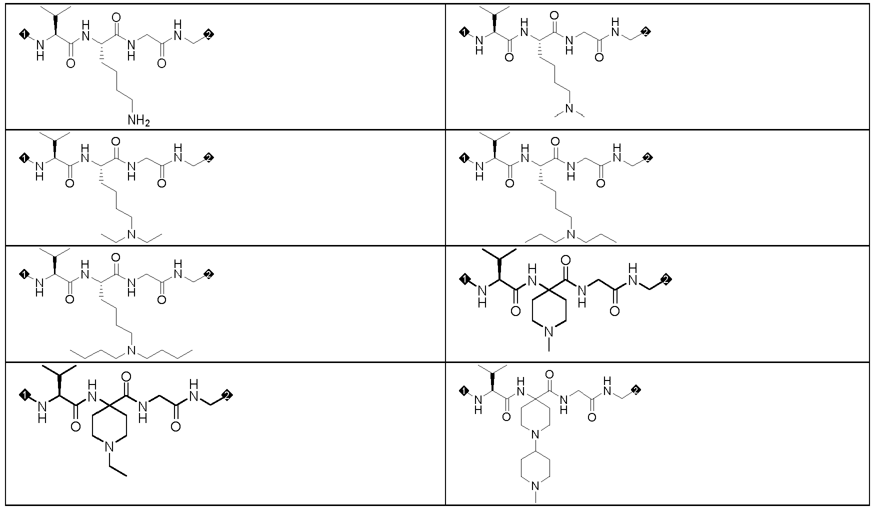

In some embodiments, Tb is an antibody or antigen-binding fragment thereof.

In some embodiments, the antibody or antigen binding fragment thereof as well as monoclonal antibody or antigen-binding fragment thereof comprise Fab, Fab', F(ab')₂, Fd, Fv (e.g. scFv), dAb, complementarity determining region fragment, non-human antibody, humanized antibody, chimeric antibody, fully human antibody, Probody, monoclonal antibody, bispecific antibody, or multi-specific antibody.

In some embodiments, Tb is an antibody or antigen-binding fragment thereof with endocytosis or without endocytosis.

In some embodiments, Tb is an antibody or antigen-binding fragment thereof with endocytosis.

In some embodiments, Tb is an antibody or antigen-binding fragment thereof having the activity of binding to a free antigen in tumor tissue and/or to a tumor cell surface antigen.

In some embodiments, Tb is an antibody or antigen-binding fragment thereof, which has tumor cell endocytosis activity and has the activity of binding to free antigen in tumor tissue or to a tumor cell surface antigen.

In some embodiments, Tb is an antibody or antigen-binding fragment thereof, which has tumor cell endocytosis activity and has the activity of binding to free antigen in tumor tissue and to a tumor cell surface antigen.

In some embodiments, Tb is an antibody or antigen-binding fragment thereof which has tumor cells endocytosis activity and has no activity of binding to free antigen in tumor tissues.

In some embodiments, Tb is an antibody or antigen-binding fragment thereof which has tumor cells endocytosis activity and has no activity of binding to a tumor cell surface antigen.

In some embodiments, Tb is an antibody or antigen-binding fragment thereof which has no or weak endocytosis activity.

In some embodiments, Tb is an antibody or antigen-binding fragment thereof that does not have the activity of binding to a free antigen in tumor tissue and/or to a tumor cell surface antigen.

In some embodiments, Tb is an antibody or antigen-binding fragment thereof which has no or weak tumor cells endocytosis activity, and has the activity of binding to a free antigen in tumor tissues or a tumor cell surface antigen.

In some embodiments, Tb is an antibody or antigen-binding fragment thereof which has no or weak tumor cells endocytosis activity, and has the activity of binding to a free antigen in tumor tissues and a tumor cell surface antigen.

In some embodiments, Tb is an antibody or antigen-binding fragment thereof which has no or weak tumor cells endocytosis activity, and has no activity of binding to a tumor cell surface antigen. In some embodiments, Tb is an antibody or antigen-binding fragment thereof which has no or weak tumor cells endocytosis activity, and has no activity of binding to a free antigen in tumor tissues.

In some embodiments, Tb is an antibody or antigen-binding fragment thereof which has no tumor cells endocytosis activity, and has no corresponding antigen in the human body.

In some embodiments, Tb is an antibody without tumor cell-associated antigen binding.

In some embodiments, the antibody or antigen-binding fragment thereof is selected from IgG isotype antibody.

In some embodiments, the antibody or antigen-binding fragment thereof is selected from isotype IgG1, isotype IgG2, isotype IgG3 or isotype IgG4

In some embodiments, the antibody or antigen-binding fragment thereof is an anti-chicken lysozyme human IgG1 isotype antibody.

In some embodiments, Tb is an antibody or antigen-binding fragment thereof having the activity of binding to a tumor cell surface non-endocytic antigen (e.g., ALCAM/CD166).

In some embodiments, Tb is an antibody with tumor cell-associated antigen binding.

In some embodiments, Tb is an antibody or antigen-binding fragment thereof having the activity of binding to a free antigen in tumor tissue or to a tumor cell surface antigen.

In some embodiments, Tb is an antibody or antigen-binding fragment thereof having the activity of binding to a free antigen in tumor tissue and to a tumor cell surface antigen.

In some embodiments, Tb is an antibody or antigen-binding fragment thereof with B7H3-2Ig and/or B7H-4Ig.

In some embodiments, Tb is an antibody or antigen-binding fragment thereof with the higher activity of binding to B7H3-4Ig than B7H3-2Ig.

In some embodiments of the present disclosure, Tb is a ligand or a targeting moiety that binds to a target.

In some embodiments, the target of Tb is selected from targets whose expression in tumor cells is higher than that in normal cells.

In some embodiments, the target of Tb is selected from the targets with high expression in tumor cells and low expression in normal cells.

In some embodiments, the target of Tb is selected from B7H3, CD20, CD19, CD30, GPNMB, Her2, Trop-2, EGFR, Her3, GD-2, CD79b and BCMA, et al.

In some embodiments, Tb is an antibody or antigen-binding fragment thereof.

In some embodiments, Tb is a non-human antibody, a humanized antibody, a chimeric antibody, and a fully human antibody.

In some embodiments, Tb is a monoclonal antibody, a bispecific antibody, or a multi-specific antibody.

In some embodiments, Tb is a monoclonal antibody or antigen-binding fragment thereof.

In some embodiments, Tb is an anti-B7H3 antibody or antigen-binding fragment thereof, an anti-Trop-2 antibody or antigen-binding fragment thereof, an anti-Her2 antibody or antigen-binding fragment thereof, an anti-Her3 antibody or antigen-binding fragment thereof, an anti-EGFR antibody or antigen-binding fragment thereof.

In some embodiments, Tb is an anti-B7H3 antibody or antigen-binding fragment thereof, such as antibody 1D1, antibody 1D1-01, antibody 2E3, antibody 2E3-02, enoblituzumab, mirzotamab, omburtamab or antigen-binding fragment thereof.

The sequence of 1D1 is as shown by SEQ ID NO: 1. The VH sequence of 1D1-01 is as shown by SEQ ID NO:3, the VL sequence is as shown by SEQ ID NO:13. The heavy chain sequence of 1D1-01 is as shown by SEQ ID NO:45, and the light chain sequence is as shown by SEQ ID NO:46. The sequence of 2E3 is as shown by SEQ ID NO:2. The VH sequence of 2E3-02 is as shown by SEQ ID NO:23, and the VL sequence is as shown by SEQ ID NO:33. The heavy chain sequence of 2E3-02 is as shown by SEQ ID NO:47, and the light chain sequence is as shown by SEQ ID NO:48.

In some embodiments, Tb is an anti-B7H3 monoclonal antibody or antigen-binding fragment thereof.

In some embodiments, Tb is an anti-Trop-2 antibody or antigen-binding fragment thereof, such as datopotamab, sacituzumab or antigen-binding fragment thereof.

In some embodiments, Tb is an anti-Trop-2 monoclonal antibody or antigen-binding fragment thereof.

In some embodiments, Tb is an anti-Her2 antibody or antigen-binding fragment thereof, such as anbenitamab, coprelotamab, disitamab, gancotamab, margetuximab, pertuzumab, timigutuzumab, zanidatamab, Trastuzumab, Pertuzumab or antigen-binding fragment thereof.

In some embodiments, Tb is an anti-Her2 monoclonal antibody or antigen-binding fragment thereof, such as trastuzumab, pertuzumab or antigen-binding fragment thereof.

In some embodiments, Tb is an anti-Her3 antibody or antigen-binding fragment thereof, such as barecetamab, duligotuzumab, elgemtumab, istiratumab, lumretuzumab, patritumab, seribantumab, zenocutuzumab, antibody 202-2-1 or antigen-binding fragment thereof.

In some embodiments, Tb is an anti-Her3 monoclonal antibody or antigen-binding fragment thereof.

In some embodiments, Tb is an anti-EGFR antibody or antigen-binding fragment thereof, such as demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, Cetuximab or antigen-binding fragment thereof.

In some embodiments, Tb is an anti-EGFR monoclonal antibody or antigen-binding fragment thereof.

In some embodiments, the antibody is an antibody which has the activity of binding to an antigen and doesn't have endocytic activity. In some embodiments, the antibody is an antibody which has the activity of binding to an antigen such as B7H3, CD20, CD19, CD30, GPNMB, Her2, Trop-2, EGFR, Her3, GD-2, CD79b, and BCMA, and doesn't have endocytic activity.

In some embodiments, the antibody is an antibody which has the activity of binding to antigen B7H3 and doesn't have endocytic activity, such as INV721 and 17-01 disclosed in WO2021168379A1. More specifically, anti-B7H3 antibodies have VH of SEQ ID NO:2 and VL of SEQ ID NO:1 as described in WO2021168379A1.

In some embodiments, the antibody is an antibody which has the activity of binding to antigen GD2 and doesn't have endocytic activity, such as INV721 and GD2-5 disclosed in WO2021168379A1. More specifically, anti-GD2 antibodies have VH of SEQ ID NO:4 and VL of SEQ ID NO:3 as described in WO2021168379A1.

In some embodiments, the antibody is an antibody which has the activity of binding to antigen HER3 and doesn't have endocytic activity, such as ANTI-HER3 antibody 21F06 set forth in SEQ ID NO: 22 in US10808032B2.

In some embodiments, the antibody is an antibody which has the activity of binding to antigen CD20 and doesn't have endocytic activity. Although it has been shown that it is difficult for so-called "type II" CD20-specific antibodies to be internalized by CD20-positive target cells, other so-called "type I" CD20-specific antibodies have been found to be internalized and degraded to some extent, depending on activation and inhibition of FcyR expression levels in target cells interacting with them. In some embodiments, the antibody which has the activity of binding to antigen CD20 and doesn't have endocytic activity is "type II" CD20 specific antibody, such as obinutuzumab.

In some embodiments, the antibody is an antibody which has the activity of binding to non-endocytic antigen (e.g., ALCAM/CD166). In some embodiments, the antibody is an antibody comprising VH of SEQ ID No:73 and VL of SEQ ID No:74, VH of SEQ ID No:75 and VL of SEQ ID No:76, VH of SEQ ID No:77 and VL of SEQ ID No:78, or VH of SEQ ID No:79 and VL of SEQ ID No:88, as described in EP3911682A1.

In some embodiments, Tb is a targeting moiety, such as ligands, proteins, polypeptides, non-protein reagents (e.g., sugars, RNA or DNA), antibody mimics, etc.

In some embodiments, q is selected from any numerical value between 0.1 and 16.0; in preferred embodiments, q is selected from any integer between 0.1 and 16.0.

In some embodiments, q is selected from any numerical value between 0.1 and 8.0; in preferred embodiments, q is selected from any integer between 0.1 and 8.0.

In some embodiments, q is selected from any numerical value between 2 and 8.

In some embodiments, q is selected from any numerical value between 3 and 8.

In some embodiments, q is selected from any numerical value between 4 and 8.

In some embodiments, q is selected from any numerical value between 6 and 8.

In some embodiments, q is selected from any integer between 2 and 8.

In some embodiments, q is selected from any integer between 3 and 8.

In some embodiments, q is selected from any integer between 4 and 8.

In some embodiments, q is selected from any integer between 6 and 8.

In some embodiments, q is selected from 1, 2, 3, 4, 5, 6, 7, 8, 9, 10, 11 and 12.

In some embodiments, q is selected from 2, 4, 6 and 8.

In the present disclosure, the bioactive molecular fragment refers to a moiety (fragment or group) in antibody-drug conjugates (ADC, or referred to as antibody-conjugated drugs) capable of forming a bioactive drug (e.g., a small molecule cytotoxic drug, which includes a group after loss of an atom or an atomic group) or a derivative thereof (e.g., a precursor thereof) after cleavage/degradation/enzymatic cleavage of linkers within tumor tissues or inside tumor cells, as well-known in the art. To avoid ambiguity, "drug" does not only mean "drug products" that have been approved by the pharmaceutical regulatory authority, but also includes any molecule that has potential therapeutic bioactivity in clinical practice or in research and development as well as in academic research.

In some embodiments, D is a molecular fragment with antitumor bioactivity.

In some embodiments, D is a molecular fragment with antitumor bioactivity, wherein the bioactive molecules are selected from a cytotoxic agent or a derivative thereof, such as a DNA topoisomerase inhibitor (e.g., camptothecin-type bioactive molecules, such as camptothecin, DXD, camptothecin with modified substituents or DXD with modified substituents) or a tubulin inhibitor (e.g. MMAF type of tubulin inhibitors, MMAE type of tubulin inhibitors).

In some embodiments, the ligand drug conjugate has a structure represented by formula I: 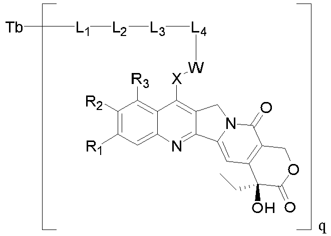 wherein,
R₁, R₂ are each independently selected from H, halogen, -OH, optionally substituted C1-6 alkyl, and optionally substituted C1-6 alkoxy, or,
R₁ and R₂ together with the carbon atoms to which they are attached form a 5-7 membered carbocyclic ring or a 5-7 membered heterocyclic ring containing one or more O, S, N, carbonyl, sulfoxide group or sulfone group or any combination thereof;
R₃ is selected from H, halogen, -OH, -NH₂, optionally substituted C1-6 alkyl and optionally substituted C1-6 alkoxy, or,
R₃ and X, together with the carbon atom to which they are attached, form a 5-7 membered carbocyclic ring or a 5-7 membered heterocyclic ring containing one or more O, S, N, carbonyl, sulfoxide group or sulfone group or any combination thereof, or,
R₃ and R₂, together with the carbon atoms to which they are attached, form a 5-7 membered carbocyclic ring or a 5-7 membered heterocyclic ring containing one or more O, S, N, carbonyl, sulfoxide group or sulfone group or any combination thereof;
W is absent or present, and when W is present, W is selected from -O-, -S-, -NR₄-, 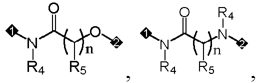 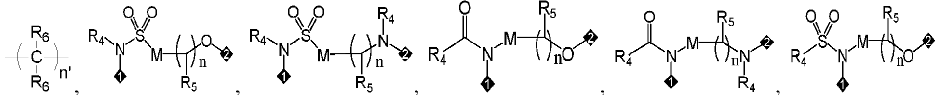 and 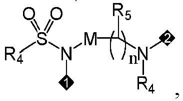 position 1 is attached to X, and position 2 is attached to L₄ or L₃;
X is selected from a direct bond, optionally substituted -O-(CH₂)ₙ₃-, -NR₄-(CH₂)ₙ₃-, -S-(CH₂)ₙ₃-, carbonyl-(CH₂)ₙ₃-, -SO₂-(CH₂)ₙ₃-, 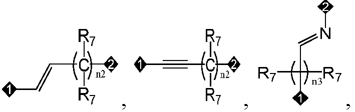 -(CH₂)ₙ₁-, C3-6 cycloalkyl, C6-10 aryl, 5-10 membered heteroaryl and 4-10 membered heterocyclyl, position 1 is attached to the parent ring and position 2 is attached to W or L₄; the substituents are selected from one or more C1-4 alkyl groups, C3-6 cycloalkyl groups, or said more C1-4 alkyl groups together with the carbon atom to which they are both attached form a C3-6 cycloalkyl group;
each M is independently selected from a direct bond and -CR^{5a}R^{5b}-;
R₄, R₅, R^{5a}, R^{5b}, R₆, R₇ are each independently selected from H, optionally substituted C1-4 alkyl, optionally substituted C1-4 alkoxy, and optionally substituted C3-6 cycloalkyl;
n, n', n1, n2, n3 are each independently selected from any integer between 0 and 6;
L₄ is absent or present, and when L₄ is present, L₄ is selected from 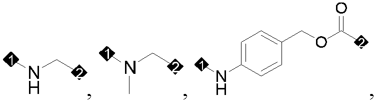 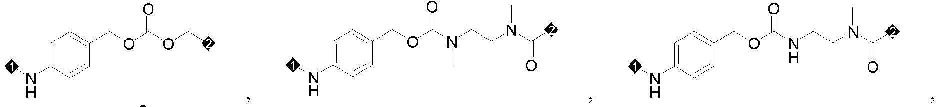 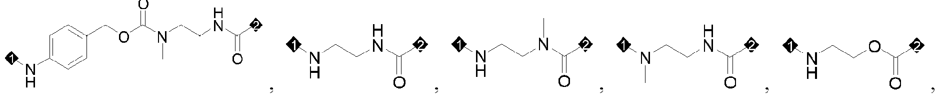
position 1 is attached to L₃, and position 2 is attached to W or X.
Tb, L₁, L₂, L₃ and q are as defined above and in any embodiment specifically described herein;

In some embodiments, R₁ and R₂ are each independently selected from H, halogen, C1-4 alkyl.

In some embodiments, R₁ and R₂, together with the carbon atoms to which they are attached, form a 5-6 membered heterocyclic ring containing 1, 2 or 3 O, S or N or any combination thereof.

In some embodiments, R₁ is selected from H and halogen, R₂ is selected from H and C1-4 alkyl.

In some embodiments, R₁ and R₂, together with the carbon atoms to which they are attached, form 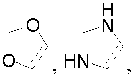 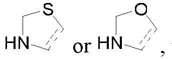 the dotted line indicates where the heterocyclic ring is fused to the benzene ring.

In some embodiments, R₁ is H or F, and R₂ is H or methyl.

In some embodiments, R₁ is F, R₂ is methyl, or R₁ and R₂ together with the carbon atoms to which they are attached form

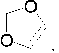

In some embodiments, R₁ is F, R₂ is methyl.

In some embodiments, R₁ and R₂ together with the carbon atoms to which they are attached form

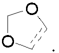

In some embodiments, R₃ is selected from H and C1-4 alkyl.

In some embodiments, R₃ and X, together with the carbon atoms to which they are attached, form a 5-6 membered carbocyclic ring.

In some embodiments, R₃ is H; or, R₃ and X together with the carbon atoms to which they are attached form 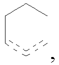 the dotted line indicates where the carbocyclic ring is fused to the benzene ring and pyridine ring.

In some embodiments, R₃ is H.

In some embodiments, W is absent or present, and when W is present, W is selected from -O-, -S-, -NR₄-, 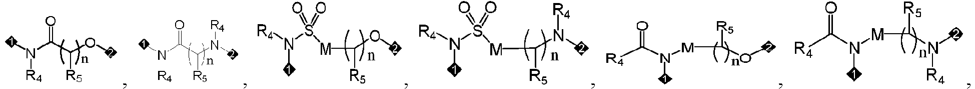 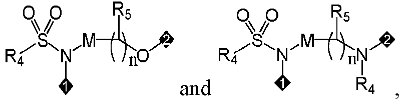 position 1 is attached to X, and position 2 is attached to L₄ or L₃.

In some embodiments, W is absent or present, and when W is present, W is selected from -O-, -S-, -NR₄-, 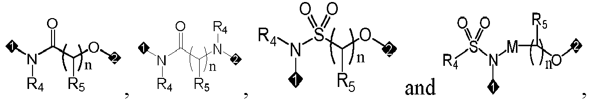 position 1 is attached to X, and position 2 is attached to L₄ or L₃.

In some embodiments, W is selected from -O-, -NR₄- and 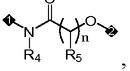 position 1 is attached to X, and position 2 is attached to L₄ or L₃.

In some embodiments, W is selected from -O- and -NR₄-, position 1 is attached to X, and position 2 is attached to L₄ or L₃.

In some embodiments, X is selected from optionally substituted -(CH₂)ₙ₁-, 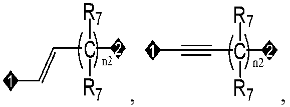 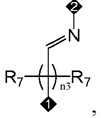 C6-10 aryl, 5-10 membered heteroaryl and 4-10 membered heterocyclyl, position 1 is attached to the parent ring and position 2 is attached to W or L₄; the substituents are selected from 1 or 2 C1-4 alkyl groups, or 2 C1-4 alkyl groups together with the carbon atom to which they are both attached form a C3-6 cycloalkyl group;
In some embodiments, X is selected from optionally substituted 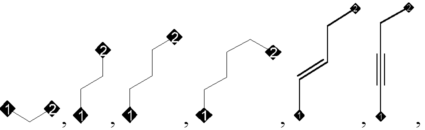 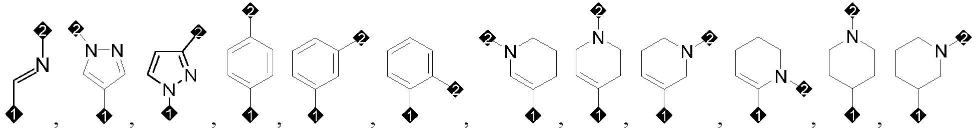 and 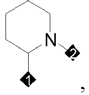 position 1 is attached to the parent ring and position 2 is attached to W or L₄; the substituents are selected from 1 or 2 C1-4 alkyl groups (such as methyl), or 2 C1-4 alkyl groups (such as methyl) together with the carbon atom to which they are both attached form a C3-6 cycloalkyl group (e.g. cyclopropyl).

In some embodiments, X is selected from 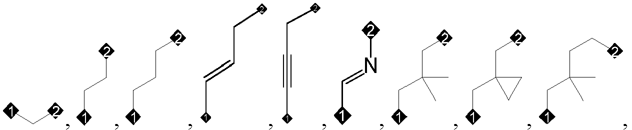 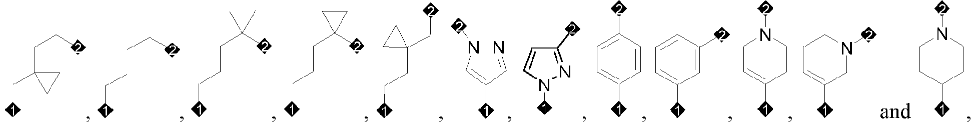 position 1 is attached to the parent ring and position 2 is attached to W or L₄.

In some embodiments, X is selected from 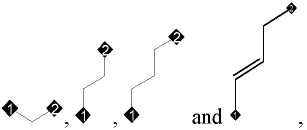 position 1 is attached to the parent ring and position 2 is attached to W.

In some embodiments, when W is absent, X is selected from 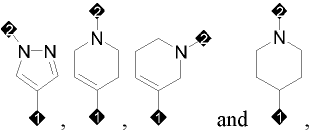 position 1 is attached to the parent ring and position 2 is attached to L₄; when W is present, X is selected from 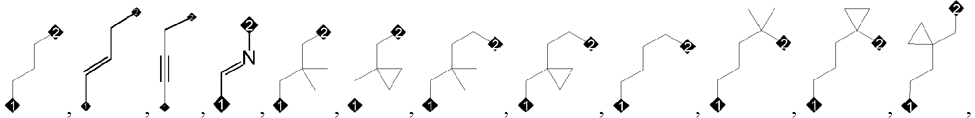 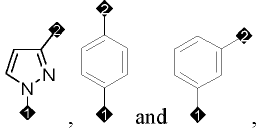 position 1 is attached to the parent ring and position 2 is attached to W.

In some embodiments, W is selected from -O-, -NR₄- and 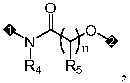 position 1 is attached to X, and position 2 is attached to L₄ or L₃; X is selected from 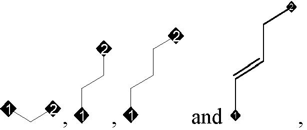 position 1 is attached to the parent ring and position 2 is attached to W.

In some embodiments, R₄, R₅ are each independently selected from H, C1-4 alkyl and C3-6 cycloalkyl.

In some embodiments, each R₄ is independently selected from H, C1-4 alkyl and C3-6 cycloalkyl, R₅ is H.

In some embodiments, each R₄ is independently selected from H, methyl, ethyl, n-propyl, isopropyl, t-butyl, and cyclopropyl, and R₅ is H.

In some embodiments, R^{5a}, R^{5b} are each independently selected from H and C1-4 alkyl.

In some embodiments, R^{5a}, R^{5b} are each independently selected from H and methyl.

In some embodiments, each R₇ is independently selected from H and C1-4 alkyl.

In some embodiments, R₇ is H.

In some embodiments, n is selected from 1, 2 and 3.

In some embodiments, n is 1.

In some embodiments, n1 is selected from 1, 2, 3 and 4.

In some embodiments, n2 is 1.

In some embodiments, n3 is 0.

In some embodiments, L₃ is selected from 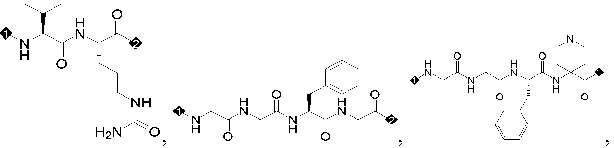 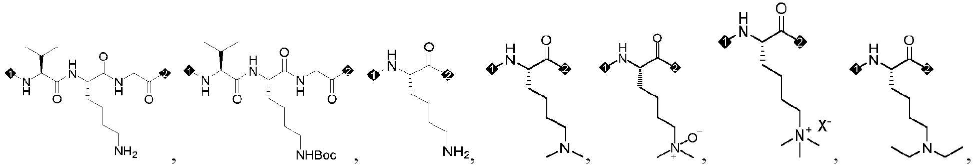 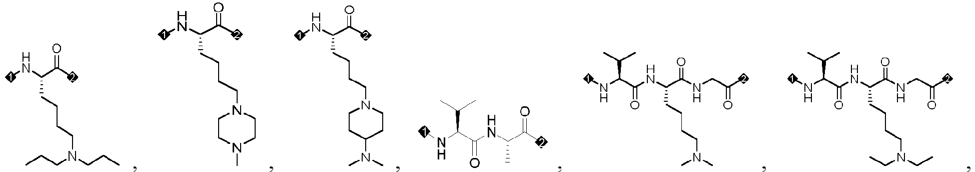 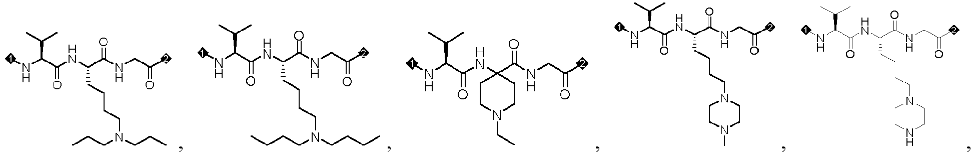 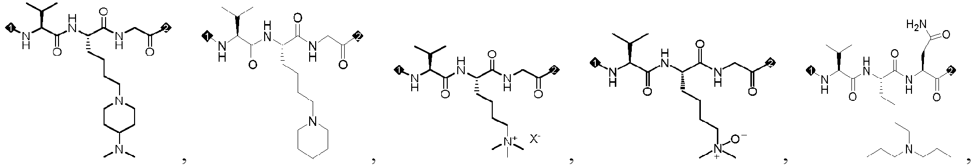 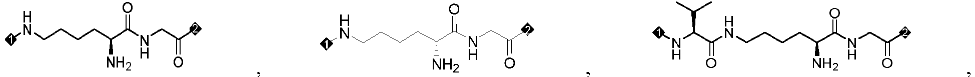 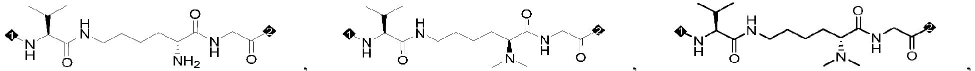 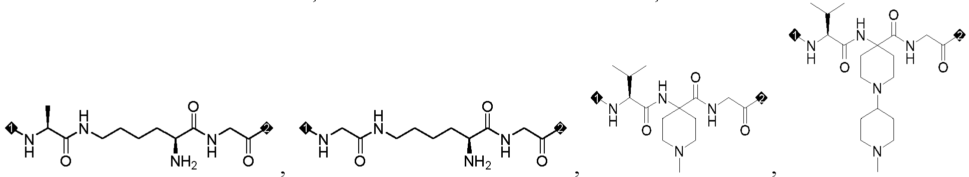 and 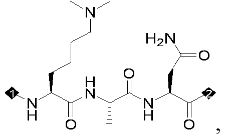 X⁻ is selected from halide ion, carboxylate ion, sulfate ion, hydrogen sulfate ion and OH⁻, position 1 is attached to L₁ or L₂, position 2 is attached to L₄ or W.

In some embodiments, L₃ is selected from 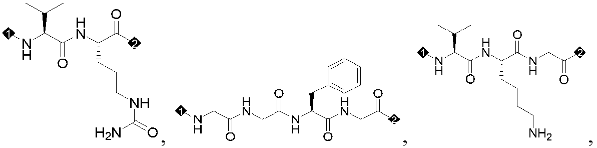 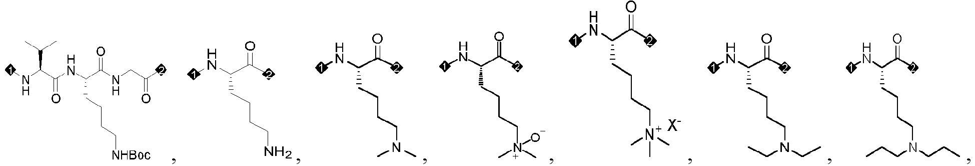 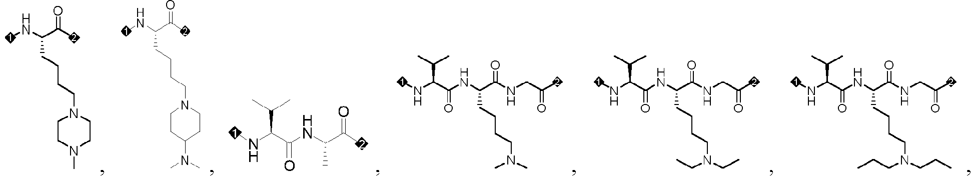 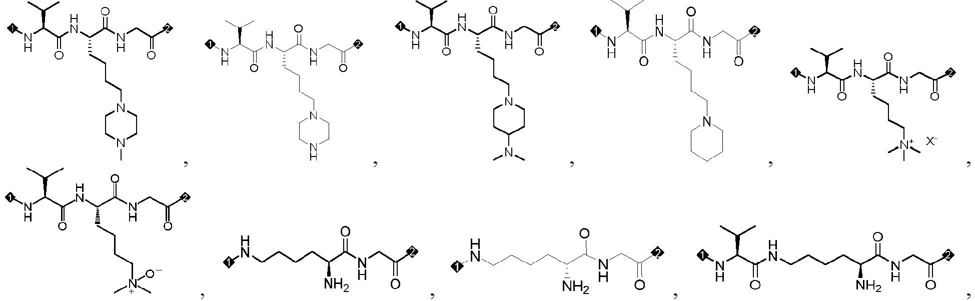 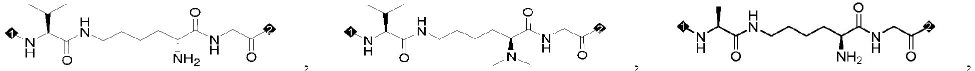 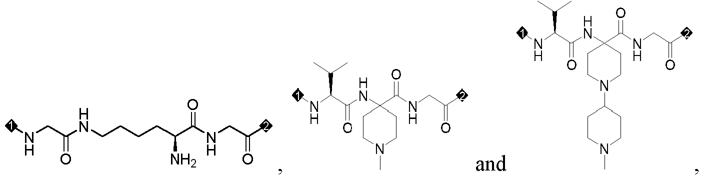 X⁻ is selected from halide ion, carboxylate ion, sulfate ion, hydrogen sulfate ion and OH⁻, position 1 is attached to L₁ or L₂, position 2 is attached to L₄ or W.

In some embodiments, L₃ is selected from 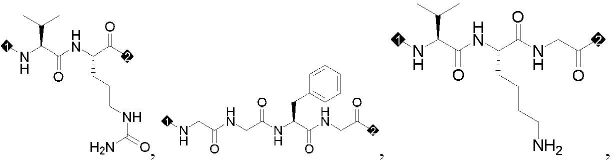 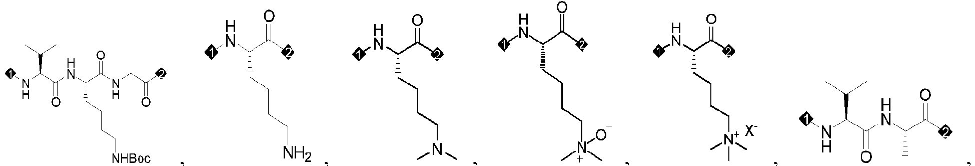 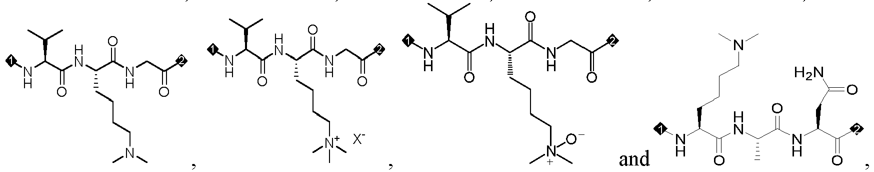 X⁻ is selected from halide ion, carboxylate ion, sulfate ion, hydrogen sulfate ion and OH⁻, position 1 is attached to L₁ or L₂, position 2 is attached to L₄ or W.

In some embodiments, L₃ is selected from 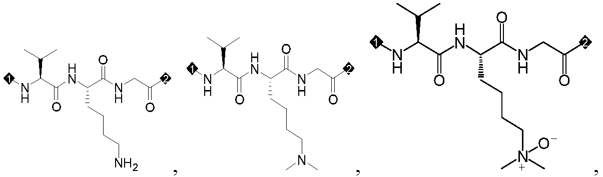 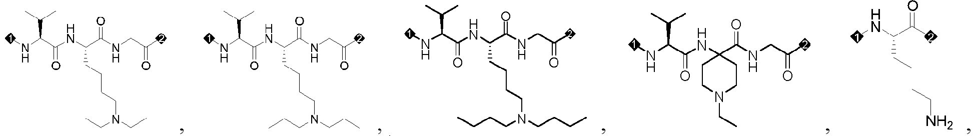 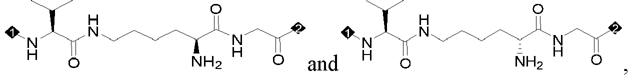 position 1 is attached to L₁ or L₂, and position 2 is attached to L₄ or W.

In some embodiments, L₃ is selected from 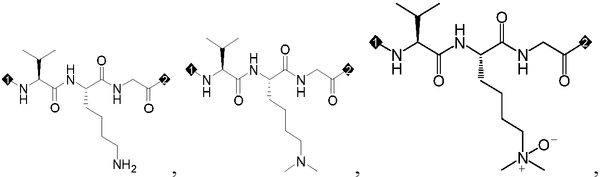 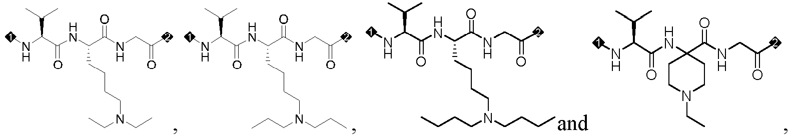 position 1 is attached to L₁ or L₂, and position 2 is attached to L₄ or W.

In some embodiments, L₄ is absent or present, and when L₄ is present, L₄ is selected from 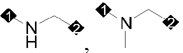 and 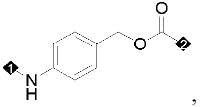 wherein position 1 is attached to L₃ and position 2 is attached to W or X.

In some embodiments, L₄ is absent or present, and when L₄ is present, L₄ is selected from 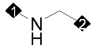 or 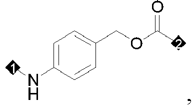 wherein position 1 is attached to L₃ and position 2 is attached to W or X.

In some embodiments, L₄ is absent.

In some embodiments, L₄ is selected from 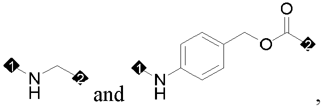 wherein position 1 is attached to L₃ and position 2 is attached to W or X.

In some embodiments, L₄ is select from 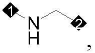 wherein position 1 is attached to L₃ and position 2 is attached to W or X.

It should be noted that, as mentioned above, W is absent or present, and so, when W is absent, position 1 of L₄ is attached to L₃, and position 2 is attached to X; when W is present, position 1 of L₄ is attached to L₃, and position 2 is attached to W. The following connection relationship for L₄ can be understood with reference to the foregoing.

In some embodiments, the structure 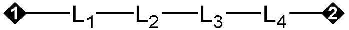 is selected from the following structural fragments:

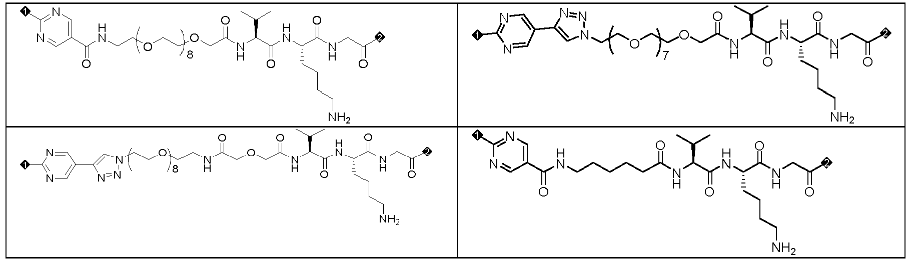

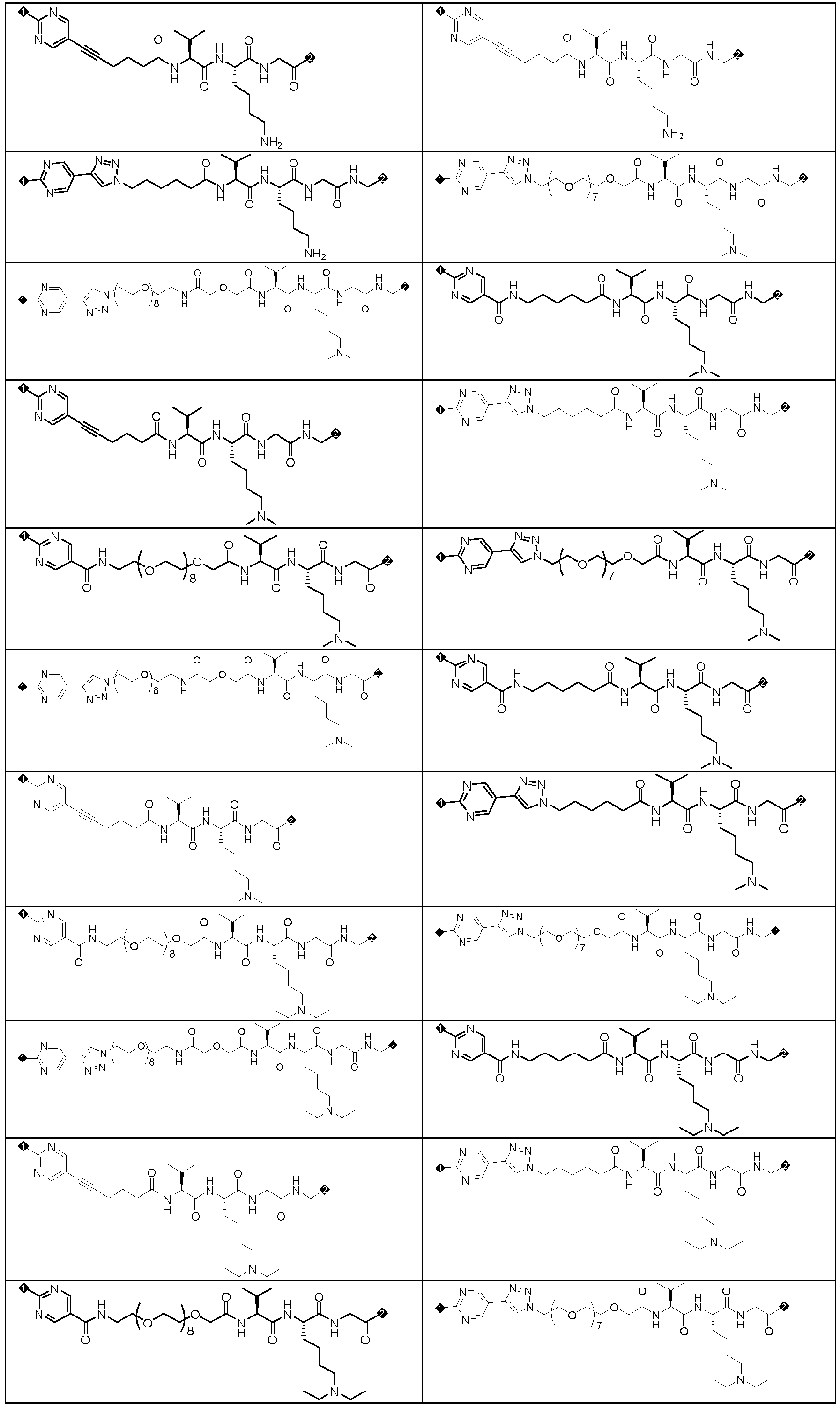

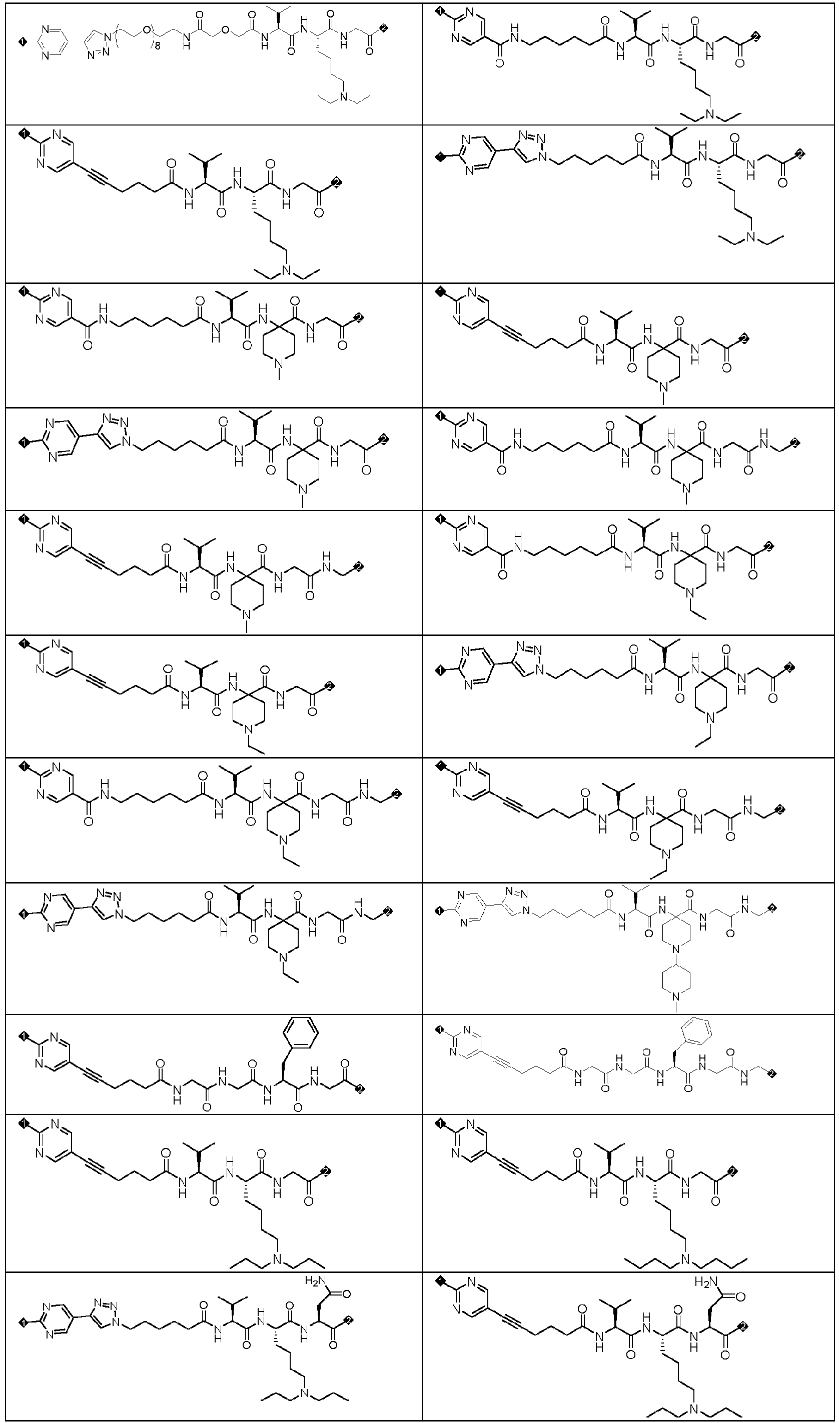

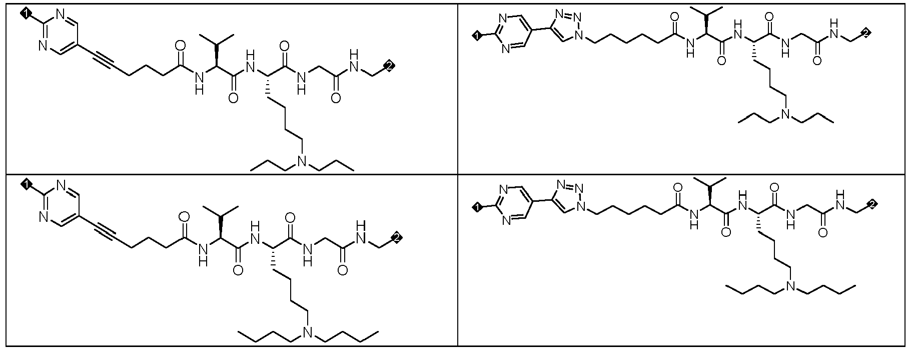

wherein, position 1 is attached to Tb, and position 2 is attached to W.

In some embodiments, D is the structural fragment represented by 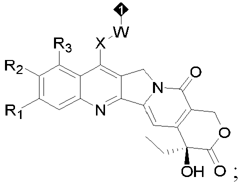 position 1 is attached to L₃ or L₄; such as

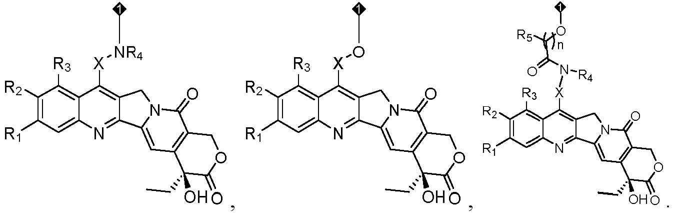

In some embodiments, the structure 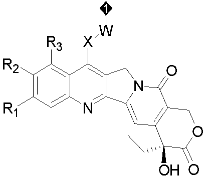 is selected from the following structural fragments: 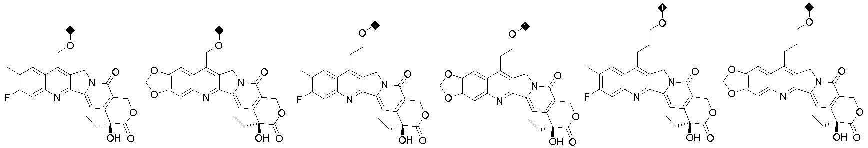 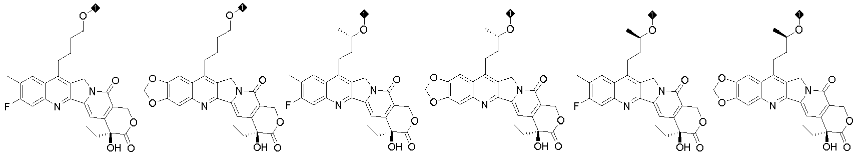 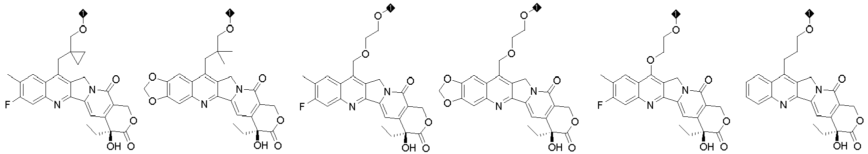 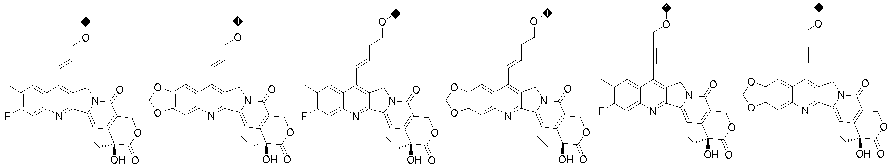 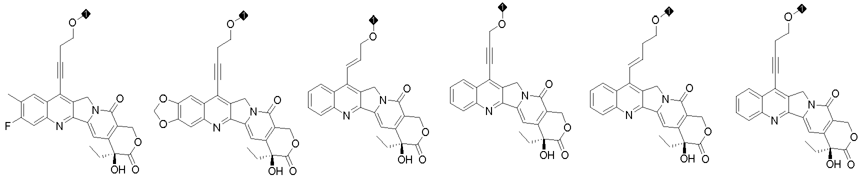 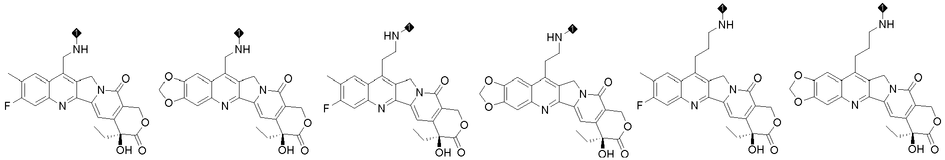 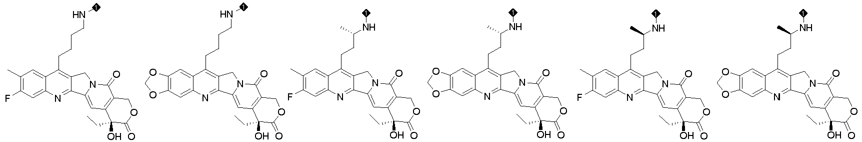 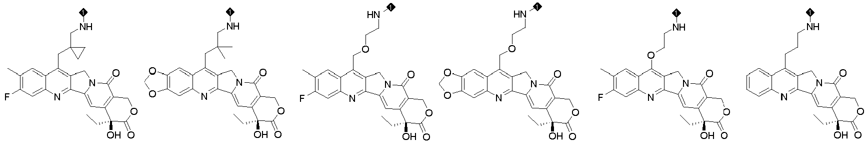 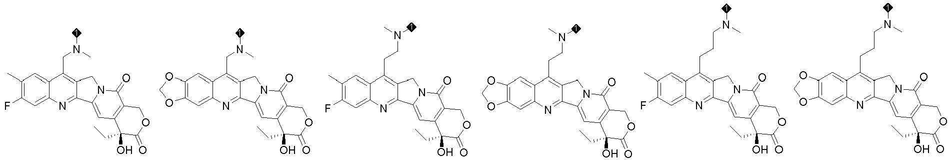 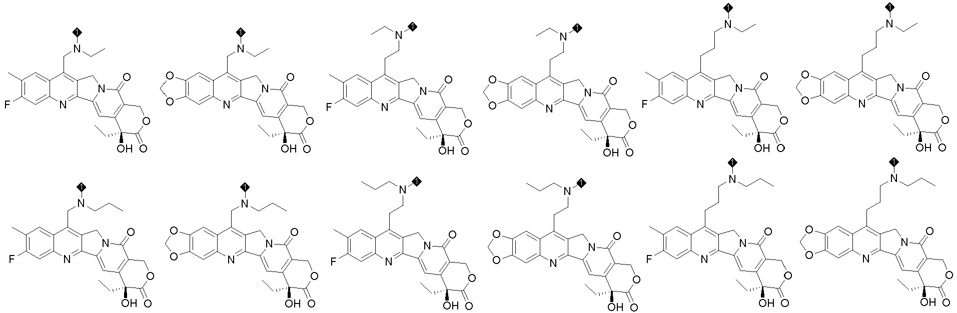 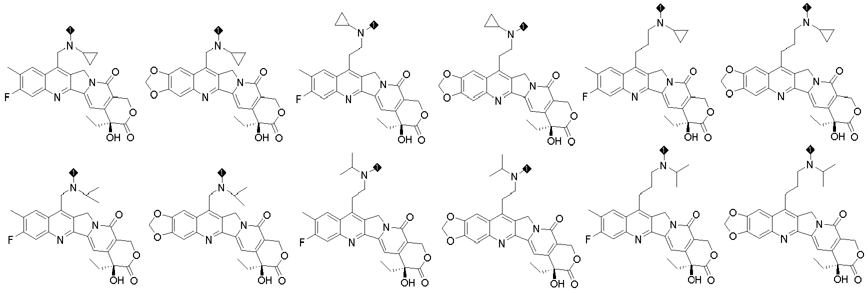 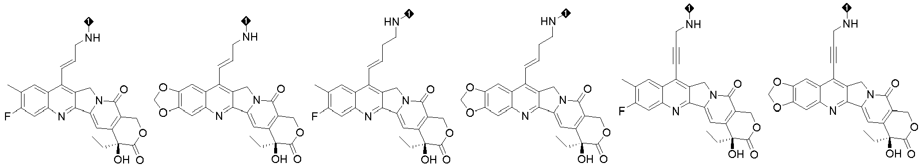 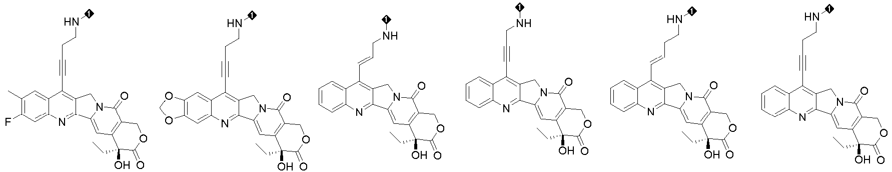 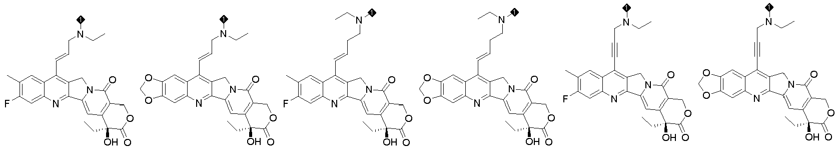 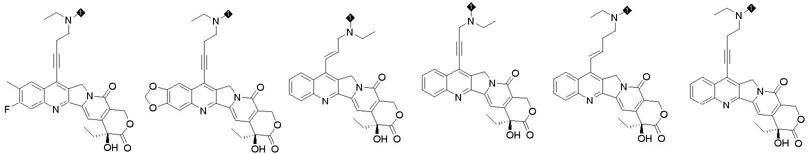 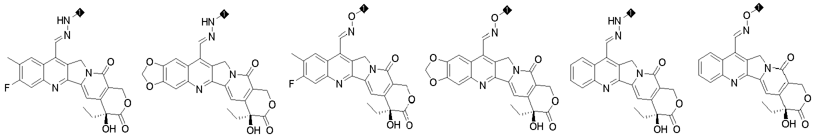 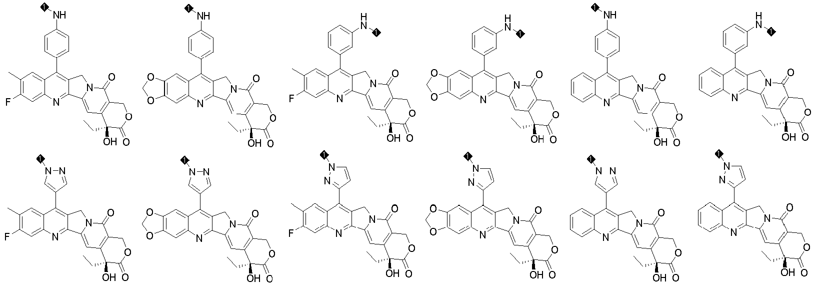 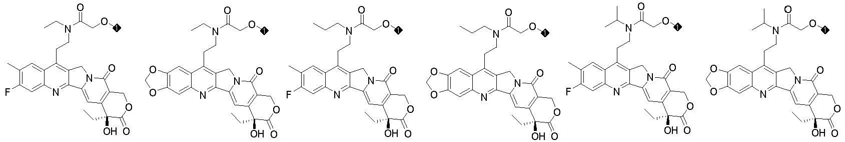 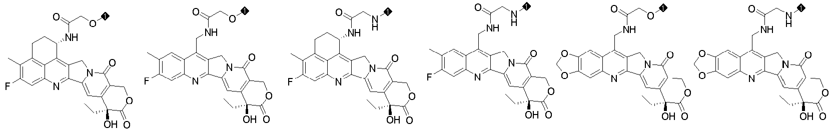 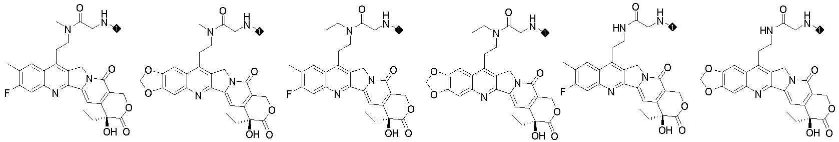 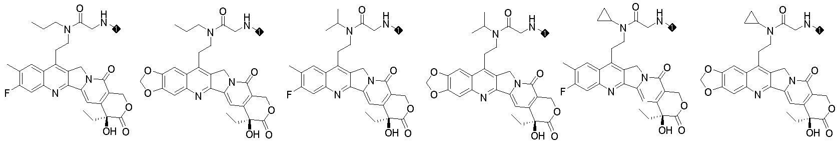 wherein, position 1 is attached to L₄; when L₄ is absent, position 1 is attached to L₃.

In some embodiments, W is absent or present, and when W is present, W is selected from -O-, -S-, -NR₄-, 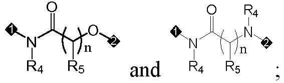 such as absent, -O-, -NR₄- or 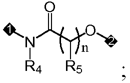 R₄ and R₅ is each independently selected from H and C1-4 alkyl; n is independently selected from 0, 1, 2, 3 and 4;
X is selected from 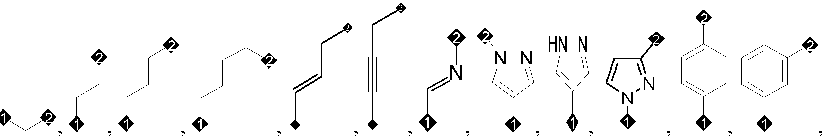 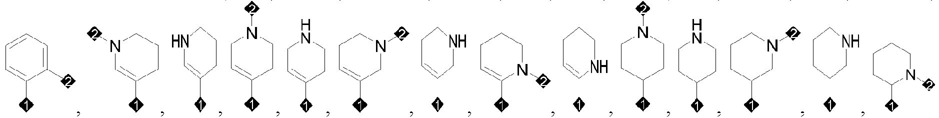 and 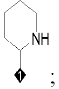
R₁ is selected from H and halogen, R₂ is selected from H and C1-4 alkyl; or, R₁ and R₂, together with the carbon atoms to which they are attached, form 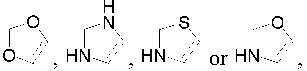 the dotted line indicates where the heterocyclic ring is fused to the benzene ring;
R₃ is H and C1-4 alkyl; or, R₃ and X together with the carbon atoms to which they are attached form a 5-6 membered carbocyclic ring;
preferably, W is absent or present, and when W is present, W is selected from -O-, -NR₄- (e.g. -NH-, -N(CH₃)-, - N(C₂H₅)-), 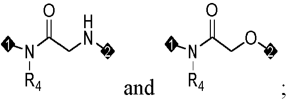 R₄ is independently selected from H, methyl, ethyl, isopropyl, n-propyl, t-butyl, and cyclopropyl;
when W is absent, X is selected from 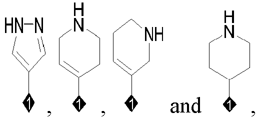 position 1 is attached to the parent ring; when W is present, X is selected from 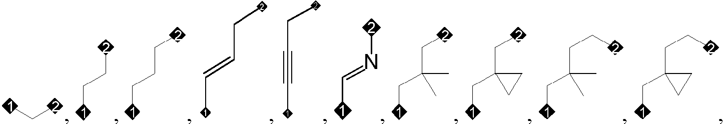 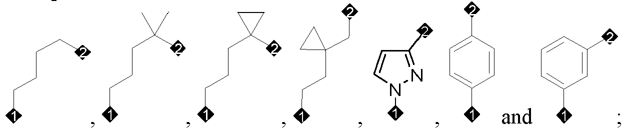 in AA₁ 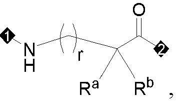 r is selected from 0, 1, 2, 3, 4 and 5;
one of R^{a} and R^{b} is H, the other is selected from 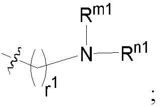 or R^{a} and R^{b} together with the carbon atom to which they are both attached form a 5-6 membered heterocyclic ring substituted with R⁰;
R₁ is selected from H and halogen, and R₂ is selected from H and C1-4 alkyl; or, R₁ and R₂ together with the carbon atoms to which they are attached form 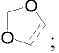
R₃ is H and C1-4 alkyl; or, R₃ and X together with the carbon atoms to which they are attached form 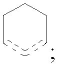
more preferably, W is selected from -O- and -NR₄-, position 1 is attached to X, and position 2 is attached to L₄ or L₃;
X is selected from 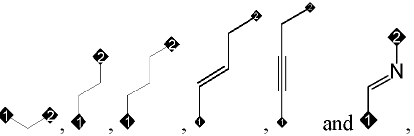 position 1 is attached to the parent ring, and position 2 is attached to W; 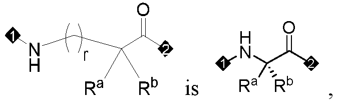 such as 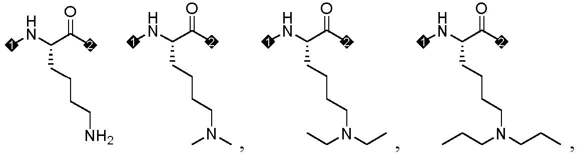 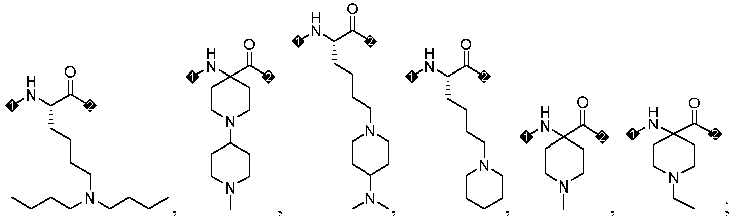
R₁ is F,
R₂ is methyl; or, R₁ and R₂ together with the carbon atoms to which they are attached form 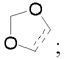
R₃ is H.

In some embodiments, the ligand drug conjugate has the structure represented by formula I-1: 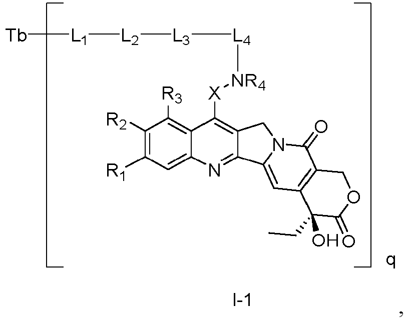 wherein Tb, L₁, L₂, L₃, L₄, X, R₁, R₂, R₃, R₄, and q are as defined in any embodiment specifically described above and herein.

In some embodiments, the ligand drug conjugate has the structure of formula I-1A or formula I-1B: 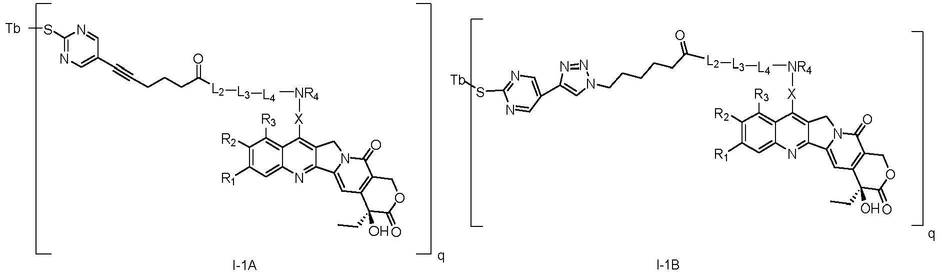 wherein Tb, L₂, L₃, L₄, X, R₁, R₂, R₃, R₄, and q are as defined in any embodiment specifically described above and herein.

In some embodiments, the ligand drug conjugate has the structure represented by formula I-2: 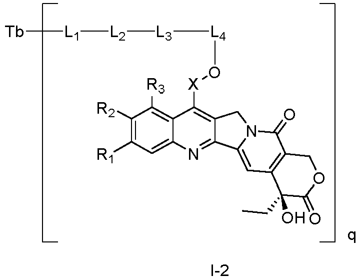 wherein Tb, L₁, L₂, L₃, L₄, X, R₁, R₂, R₃, and q are as defined in any embodiment specifically described above and herein.

In some embodiments, the ligand drug conjugate has the structure of formula I-2A or formula I-2B: 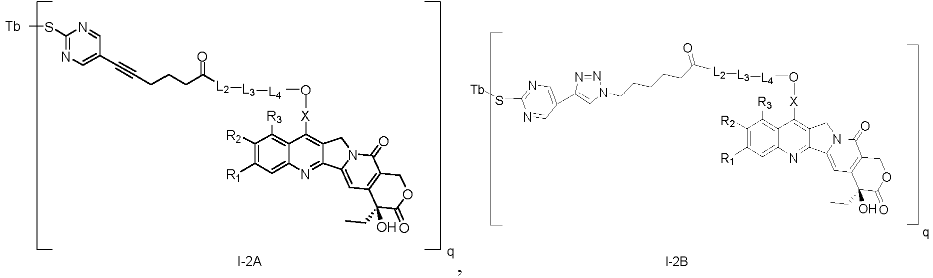 wherein Tb, L₂, L₃, L₄, X, R₁, R₂, R₃, and q are as defined in any embodiment specifically described above and herein.

In some embodiments, the ligand drug conjugate has the structure represented by formula I-3: 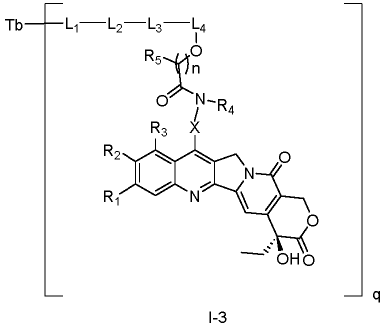 wherein Tb, L₁, L₂, L₃, L₄, X, R₁, R₂, R₃, R₄, R₅, n and q are as defined in any embodiment specifically described above and herein.

In some embodiments, the ligand drug conjugate has the structure of formula I-3A or formula I-3B: 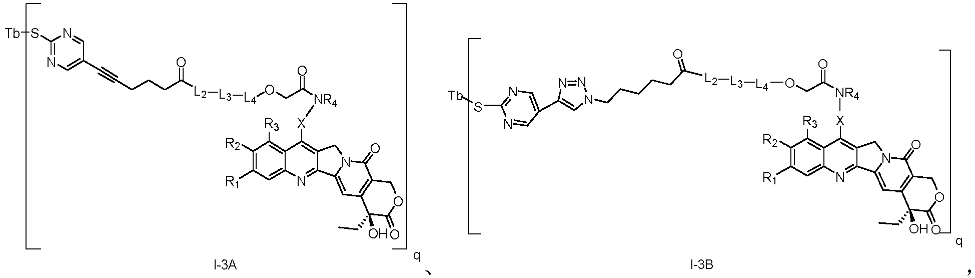 wherein Tb, L₂, L₃, L₄, X, R₁, R₂, R₃, R₄ and q are as defined in any embodiment specifically described above and herein.

In some embodiments, the ligand drug conjugate has the structure represented by formula I-A: 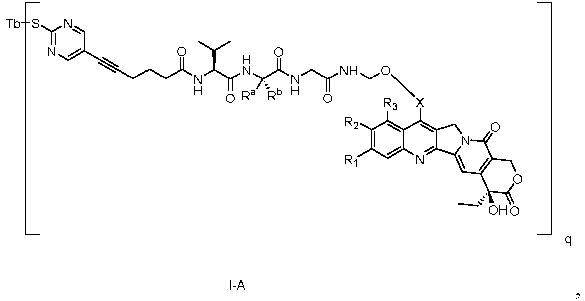 wherein Tb, X, R₁, R₂, R₃, R^{a}, R^{b} and q are as defined in any embodiment specifically described above and herein.

In some embodiments, the ligand drug conjugate has the structure represented by formula I-B: 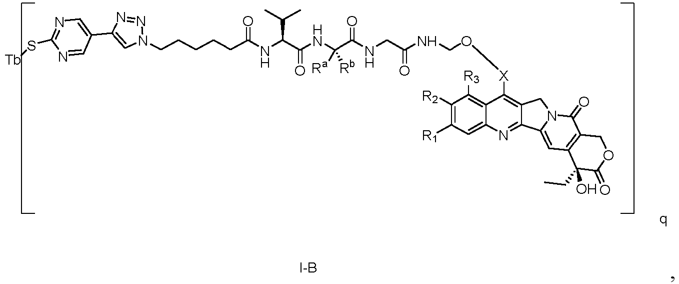 wherein Tb, X, R₁, R₂, R₃, R^{a}, R^{b} and q are as defined in any embodiment specifically described above and herein.

In some embodiments, the ligand drug conjugate is selected from:

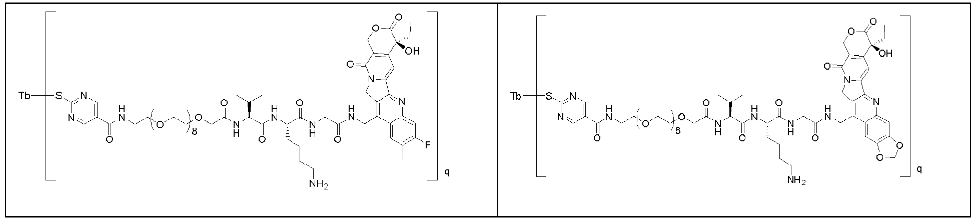

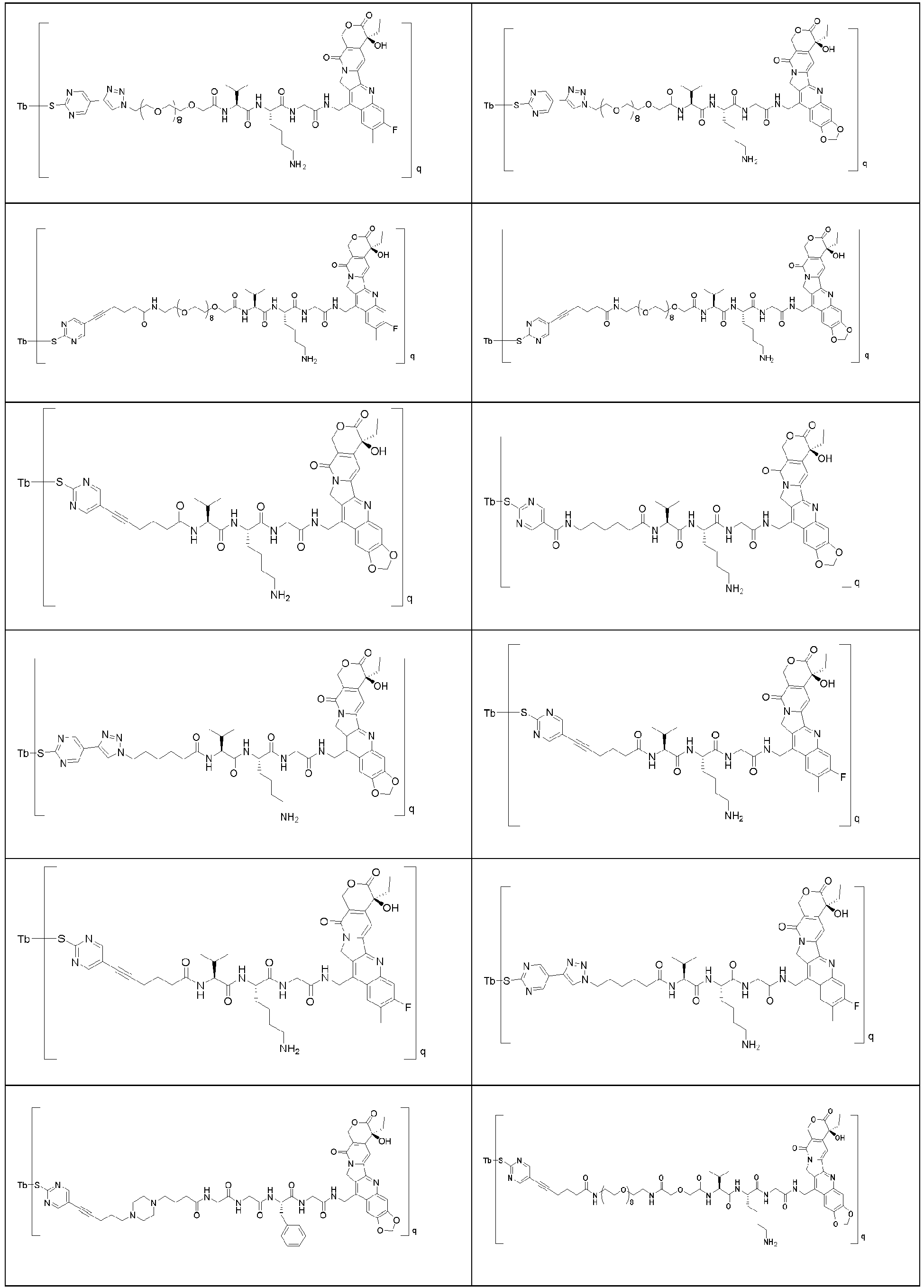

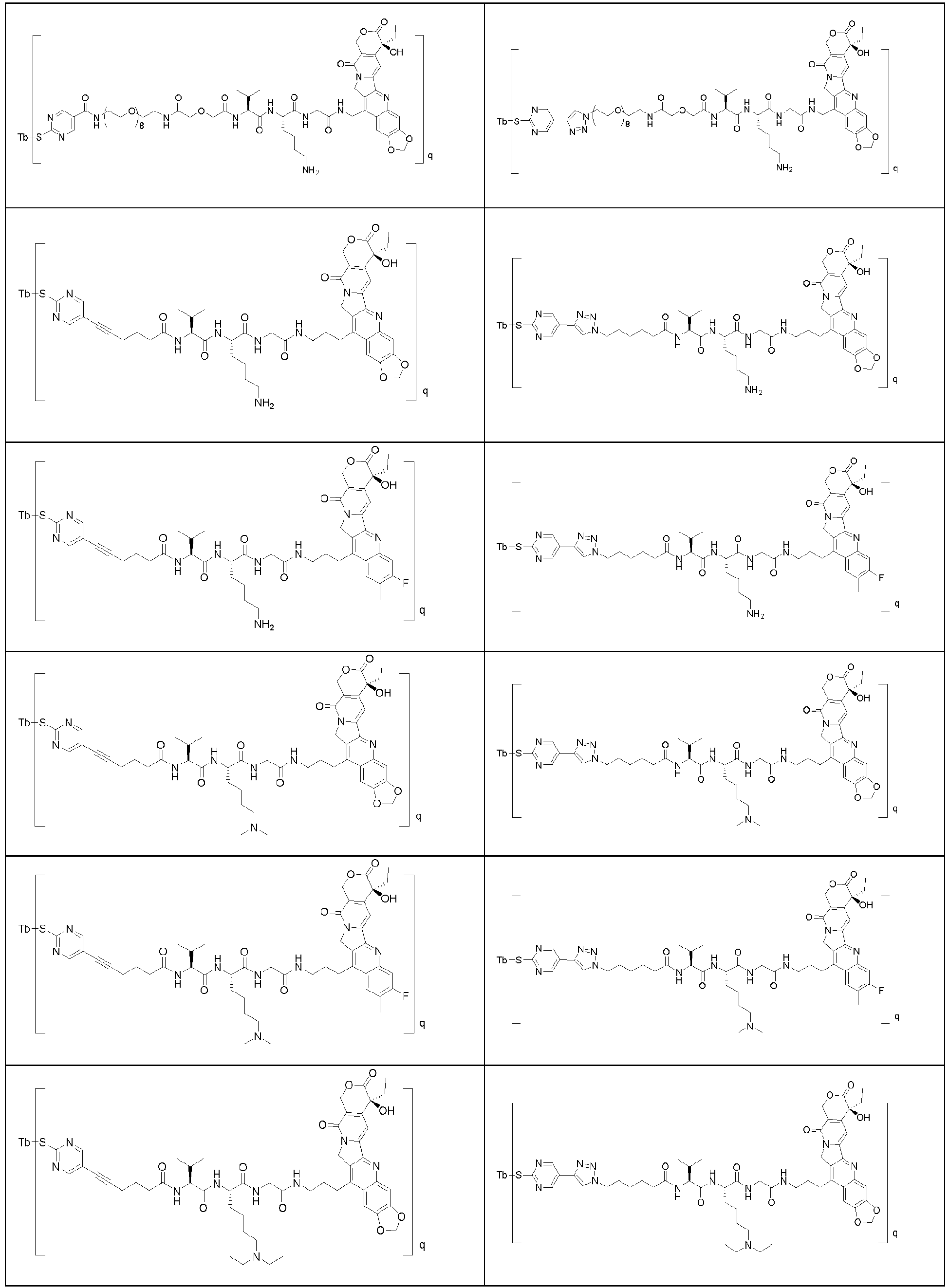

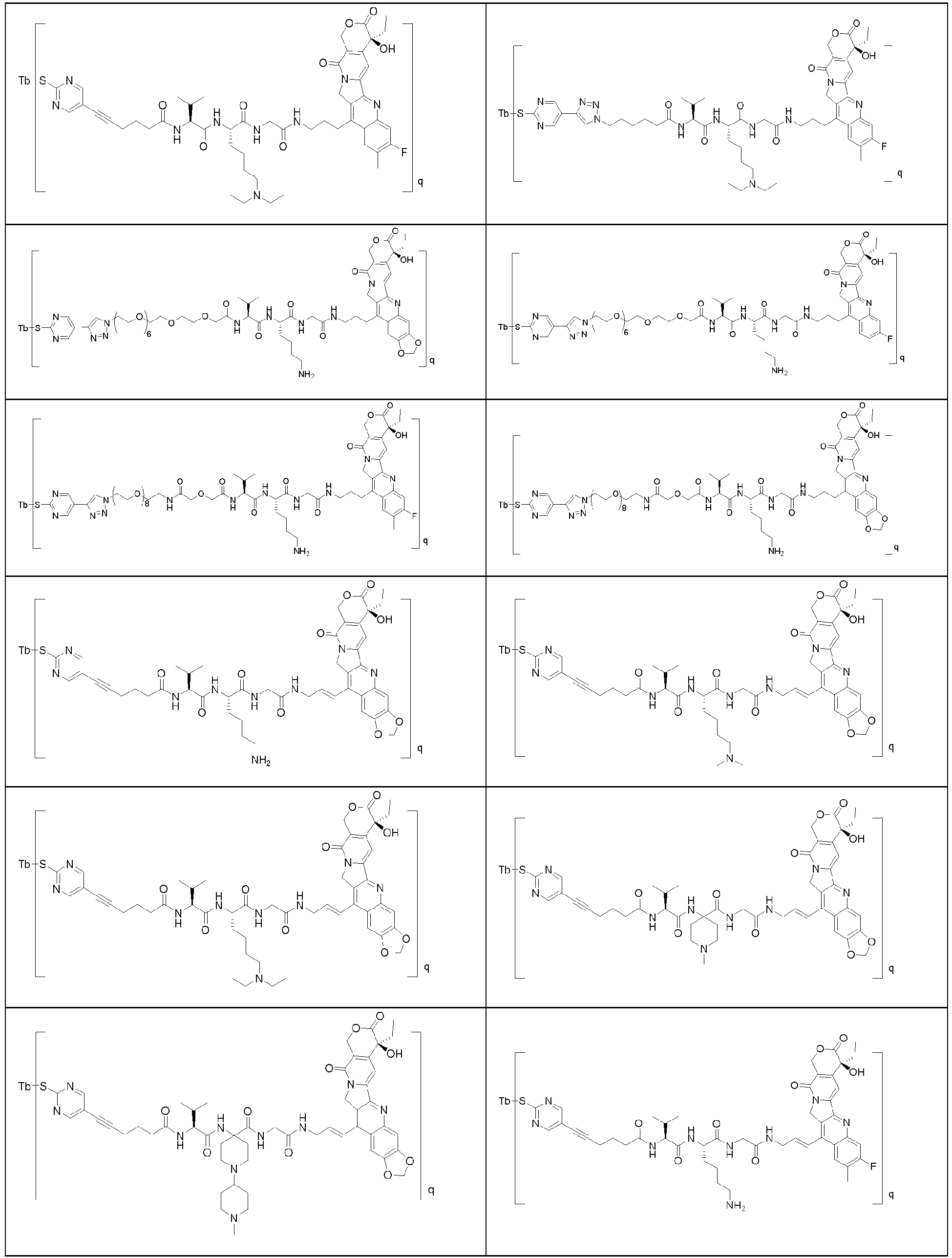

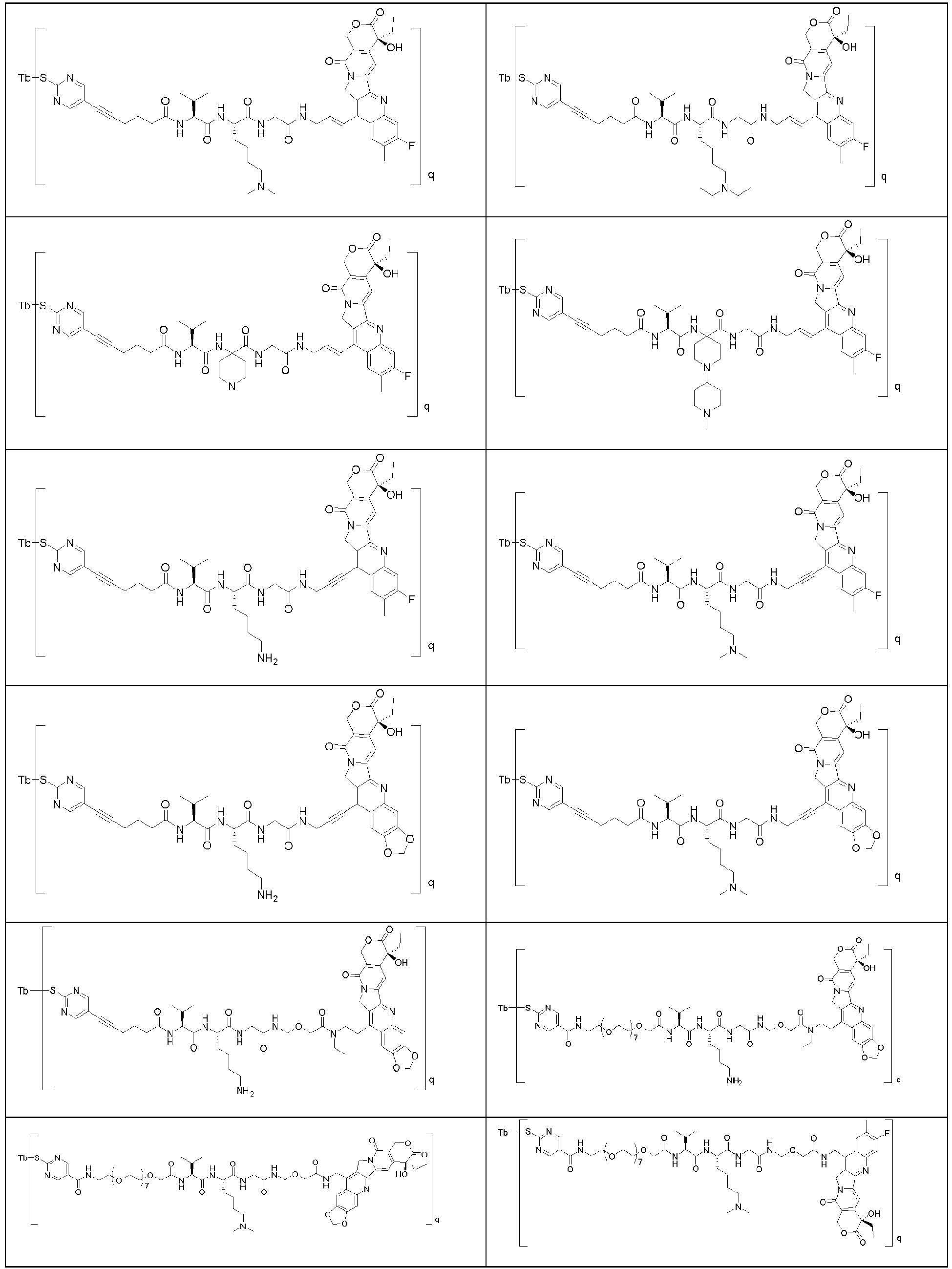

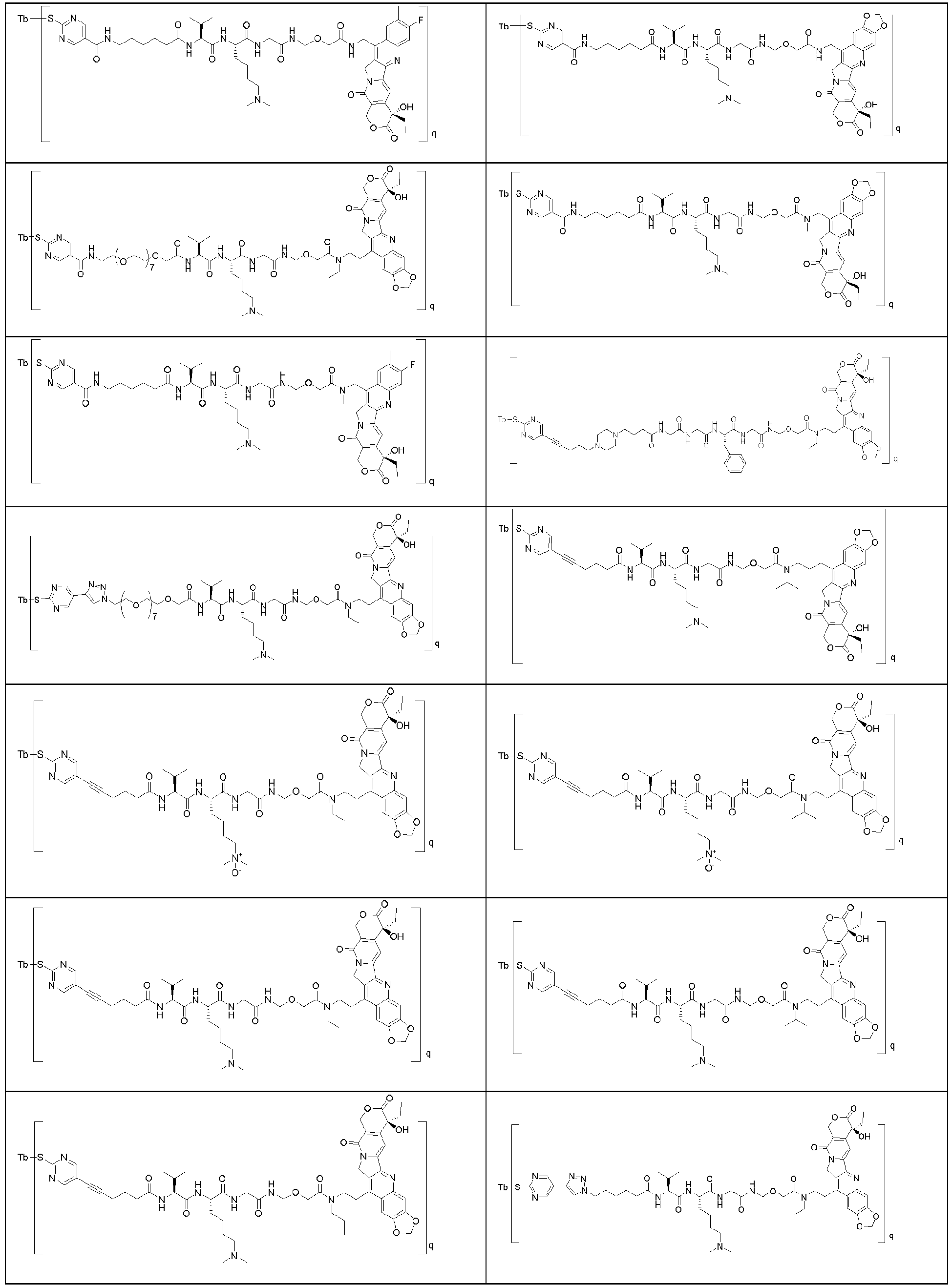

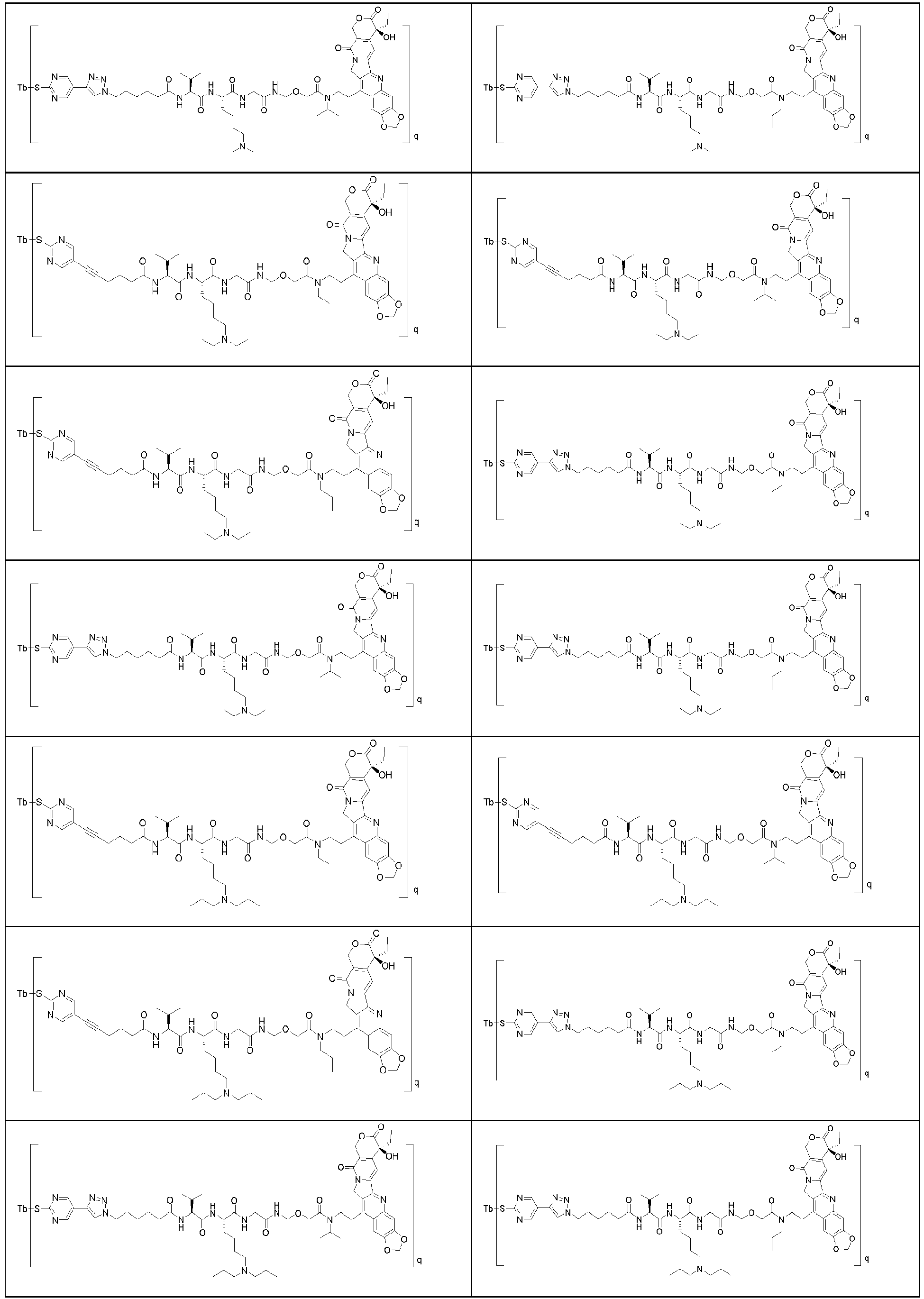

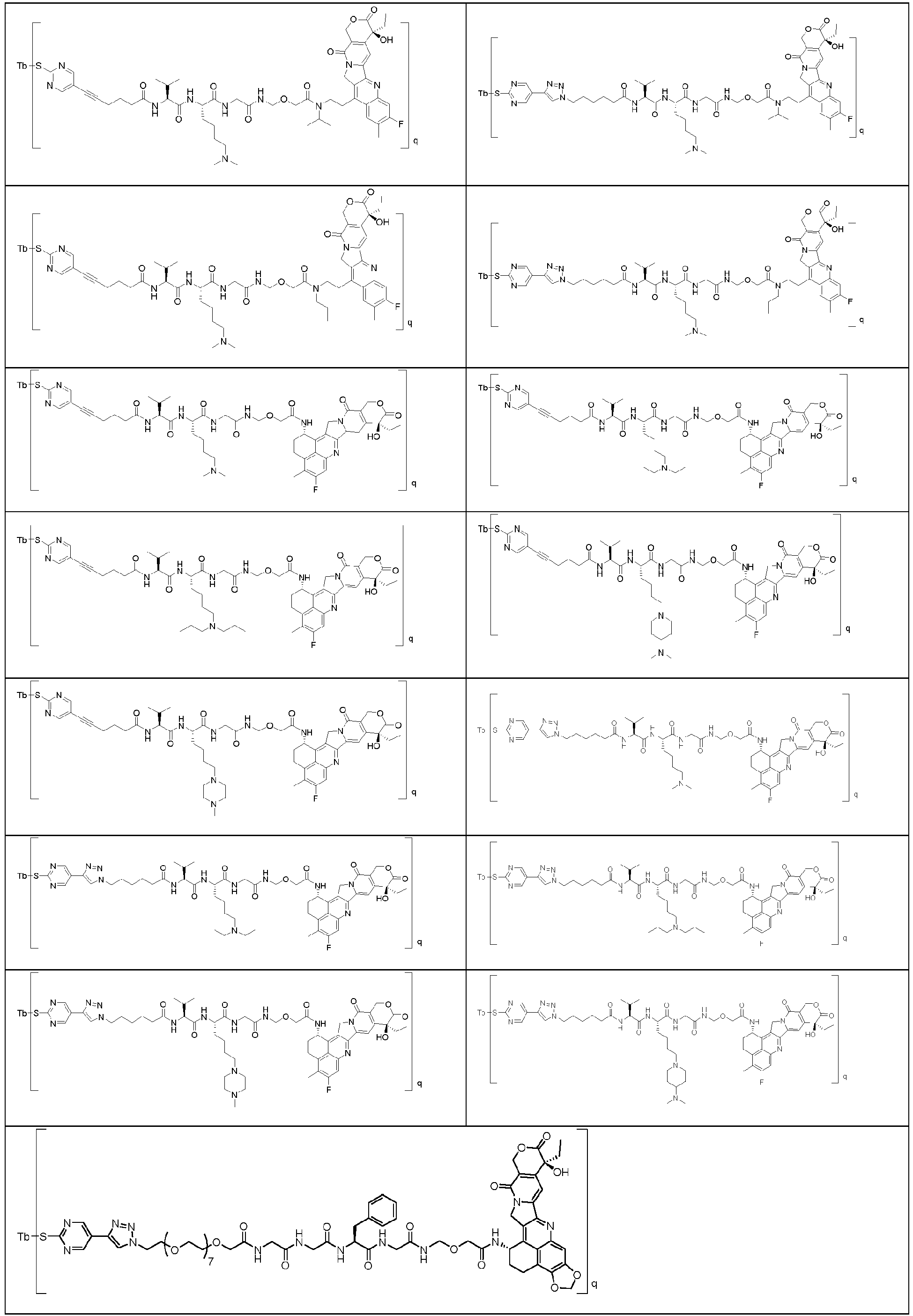

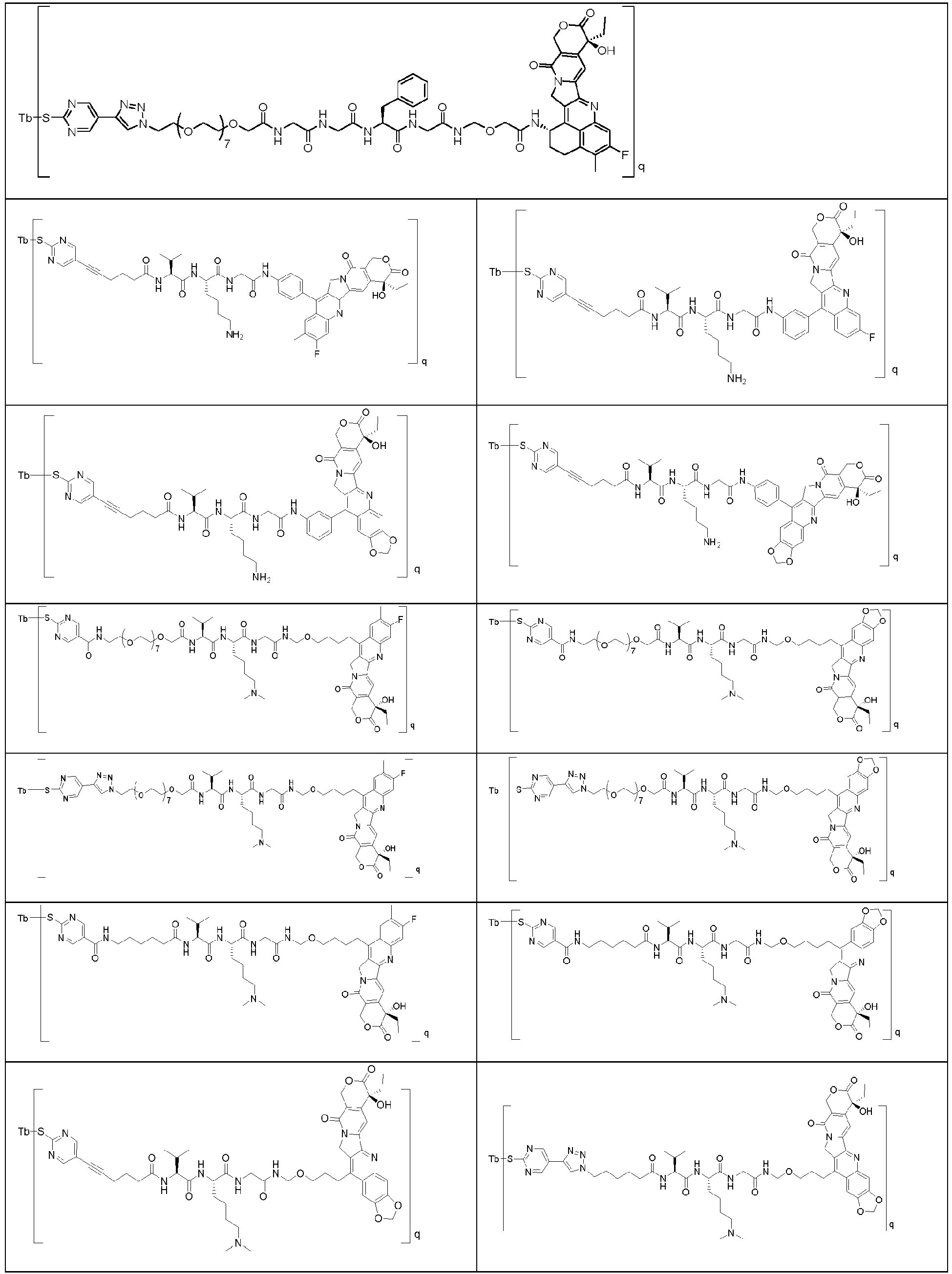

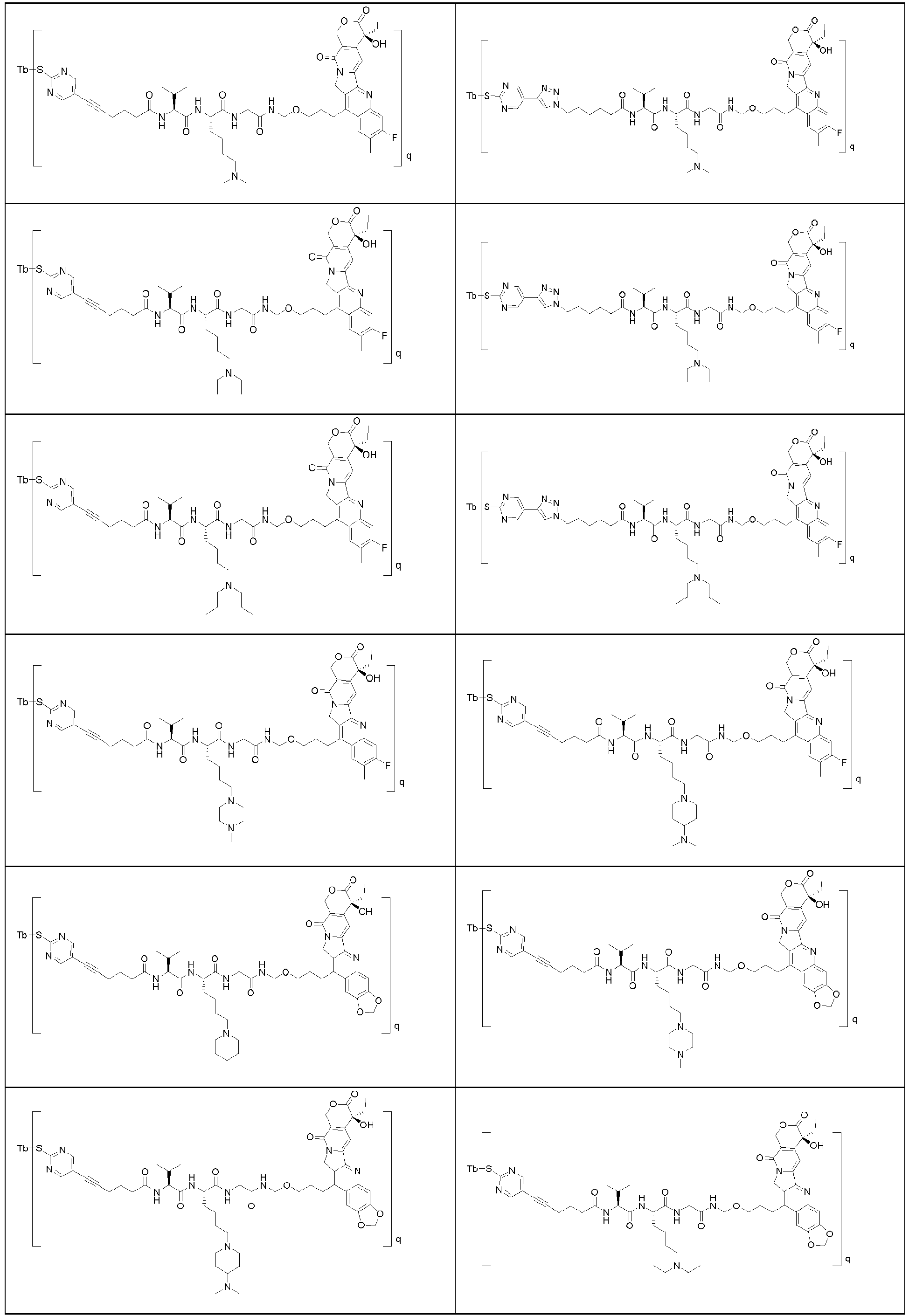

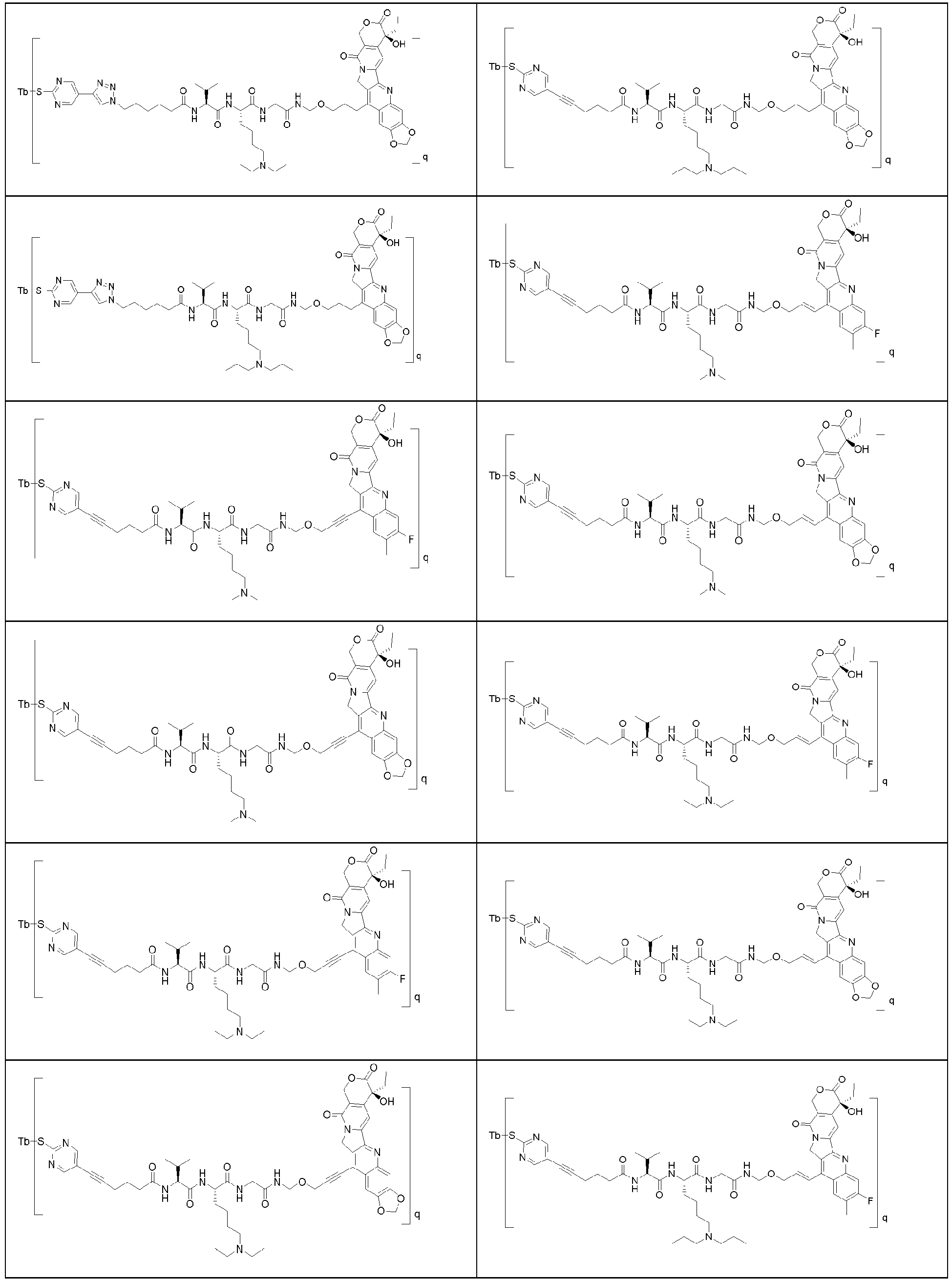

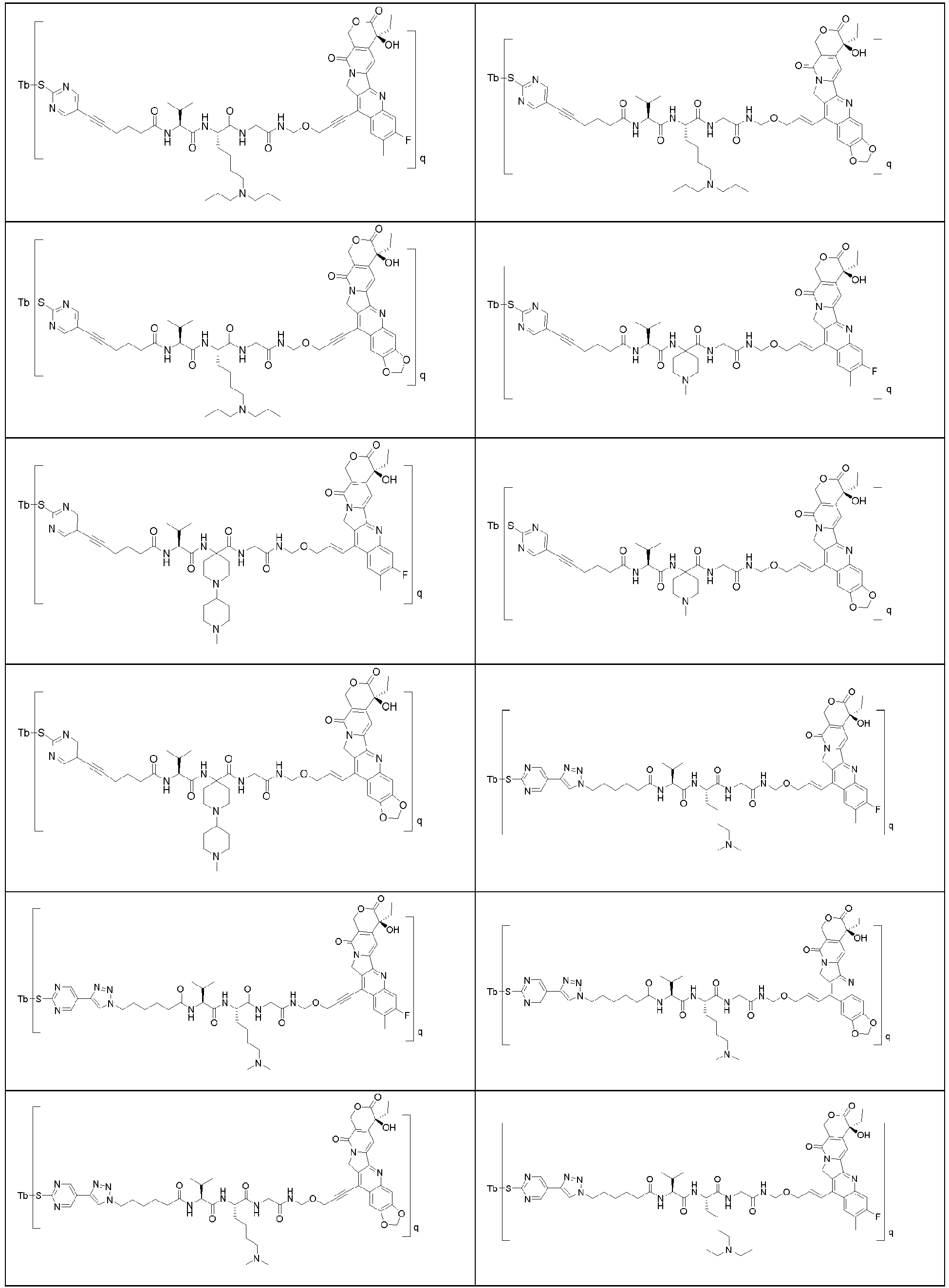

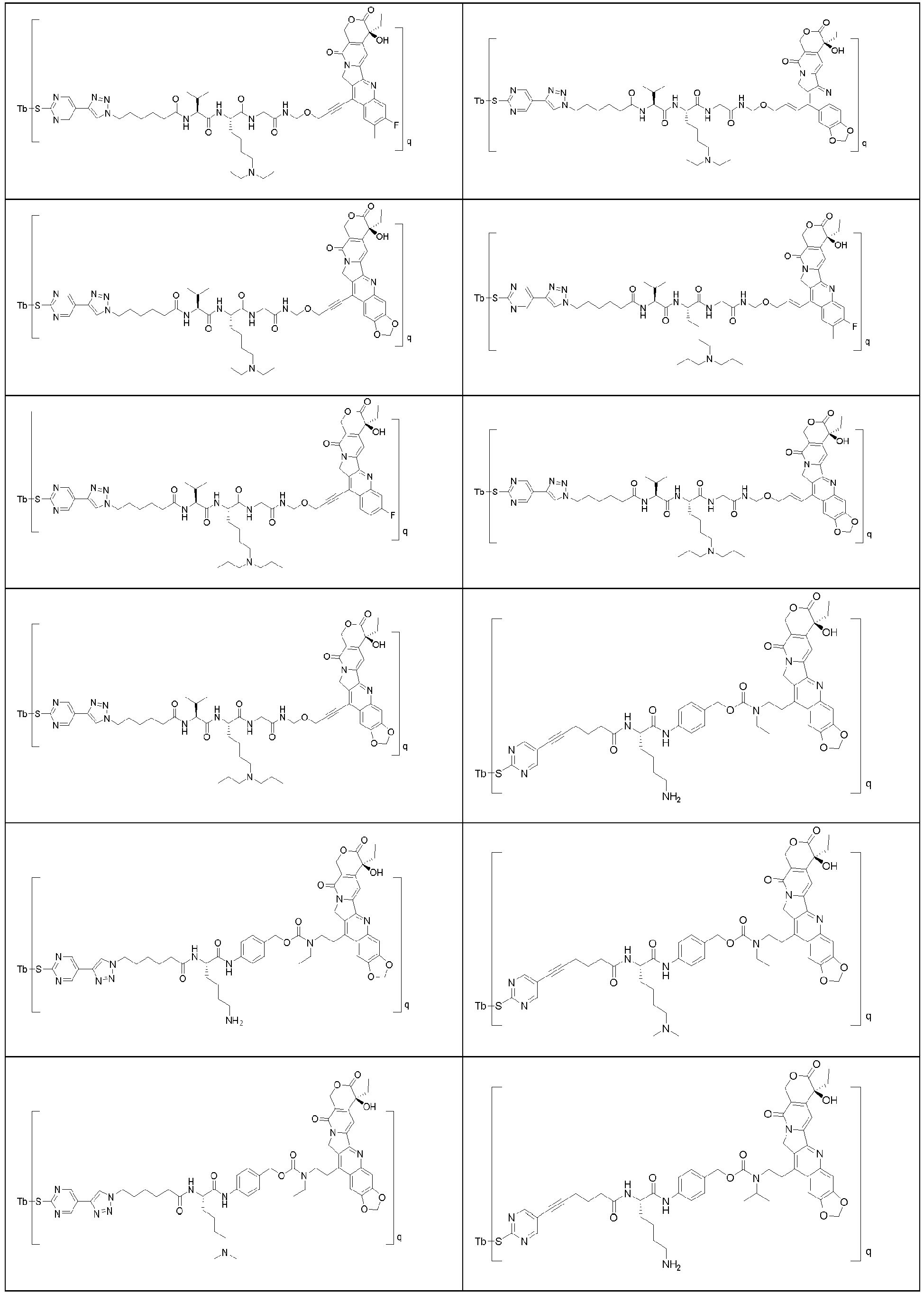

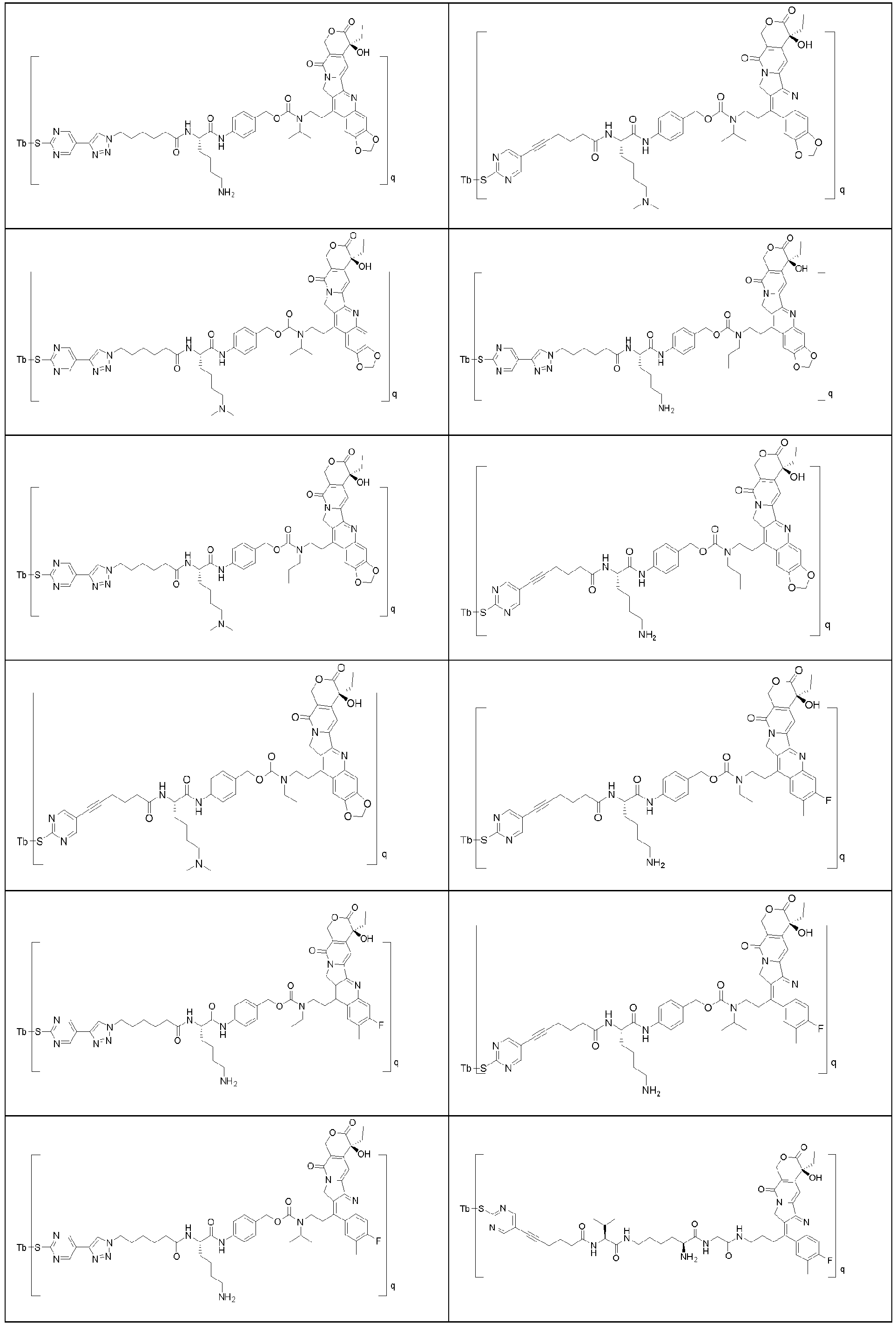

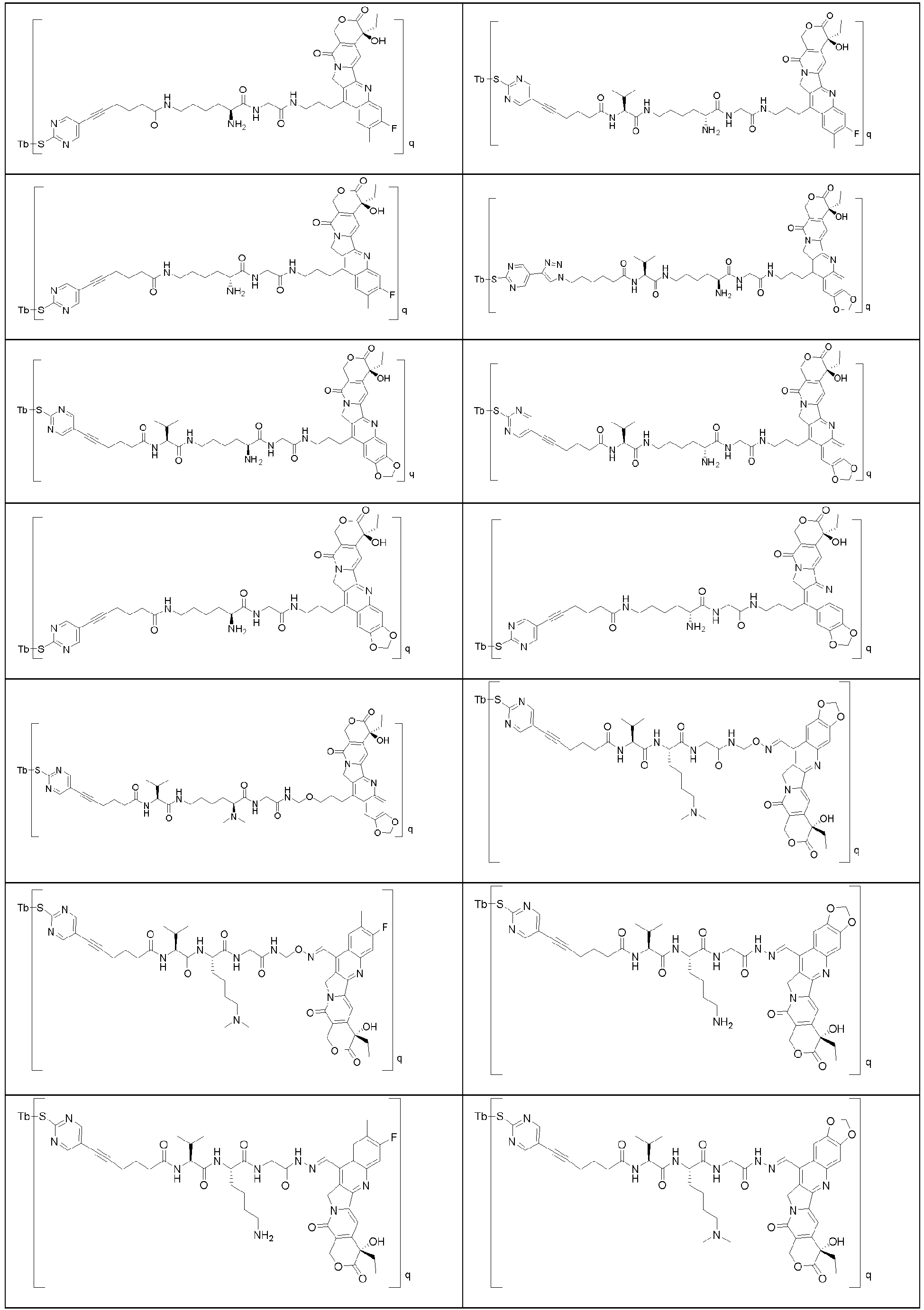

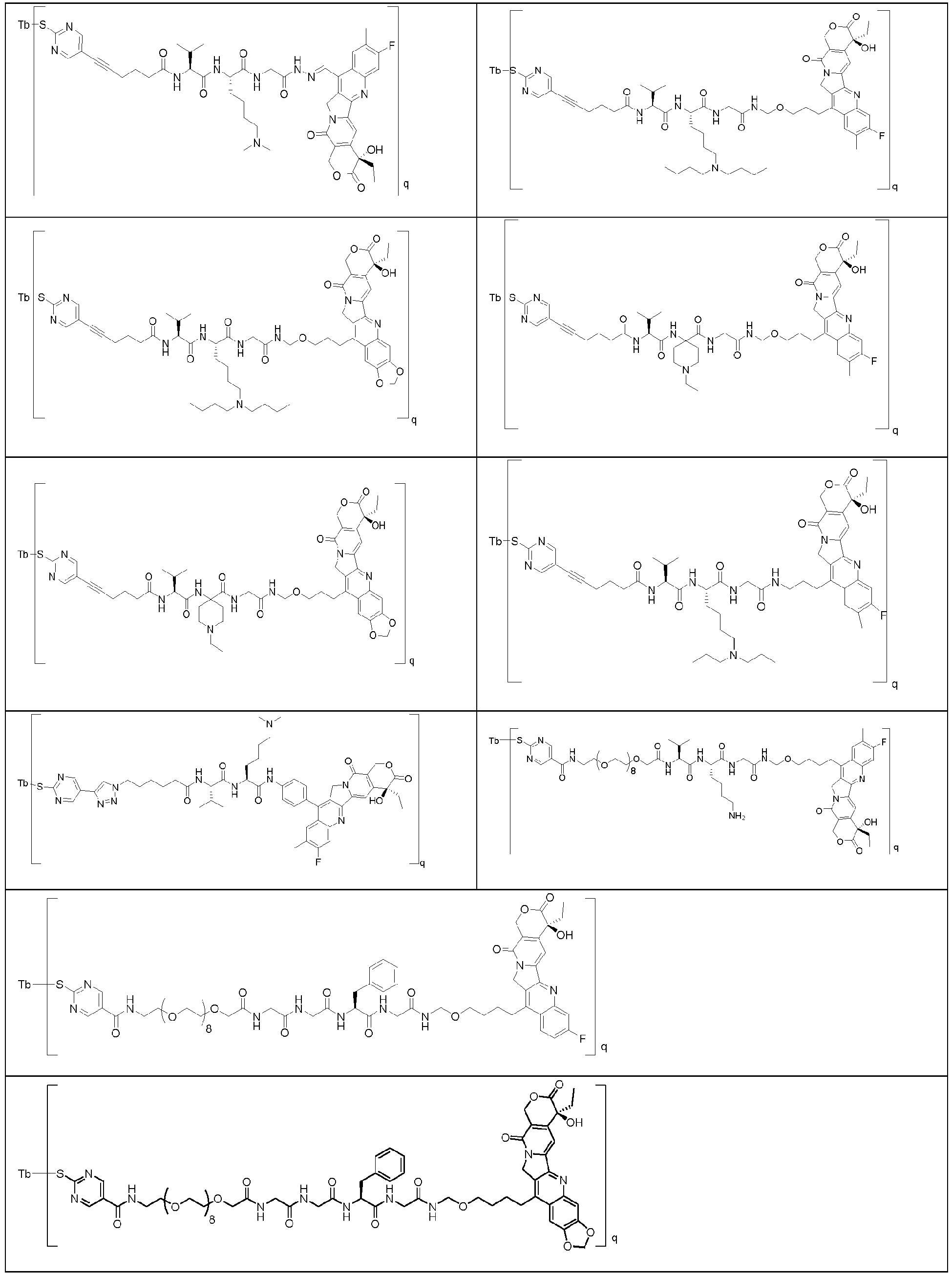

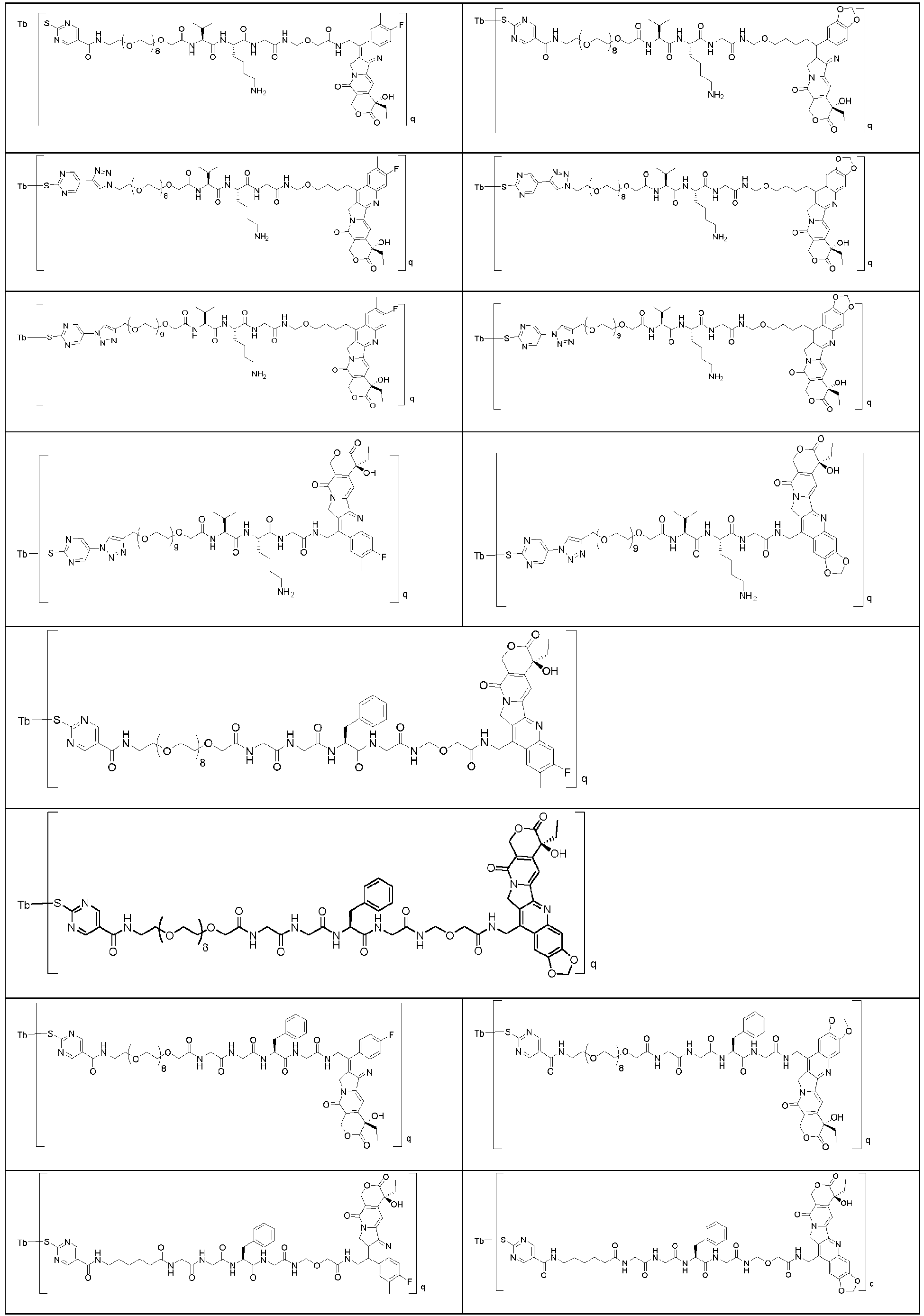

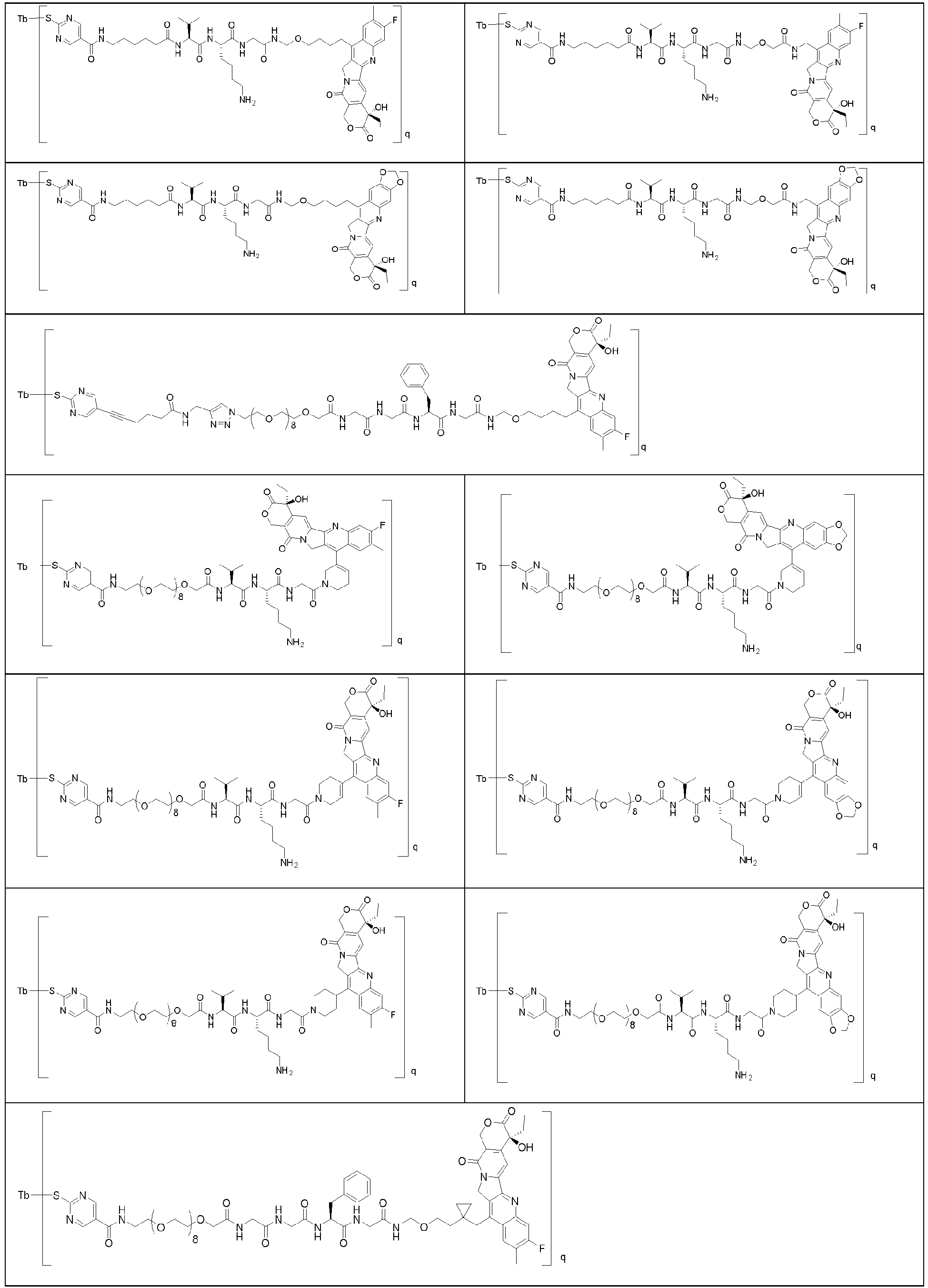

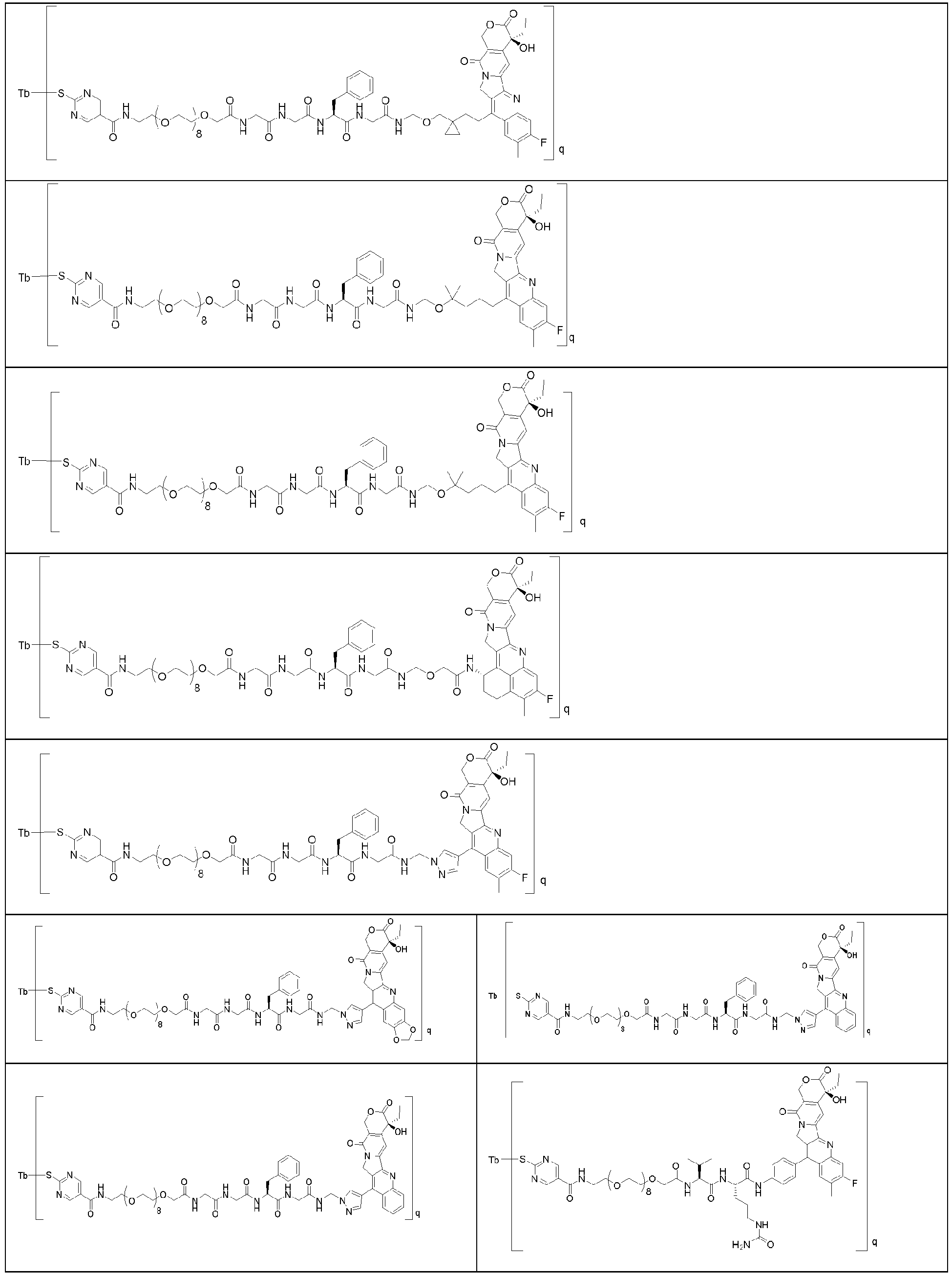

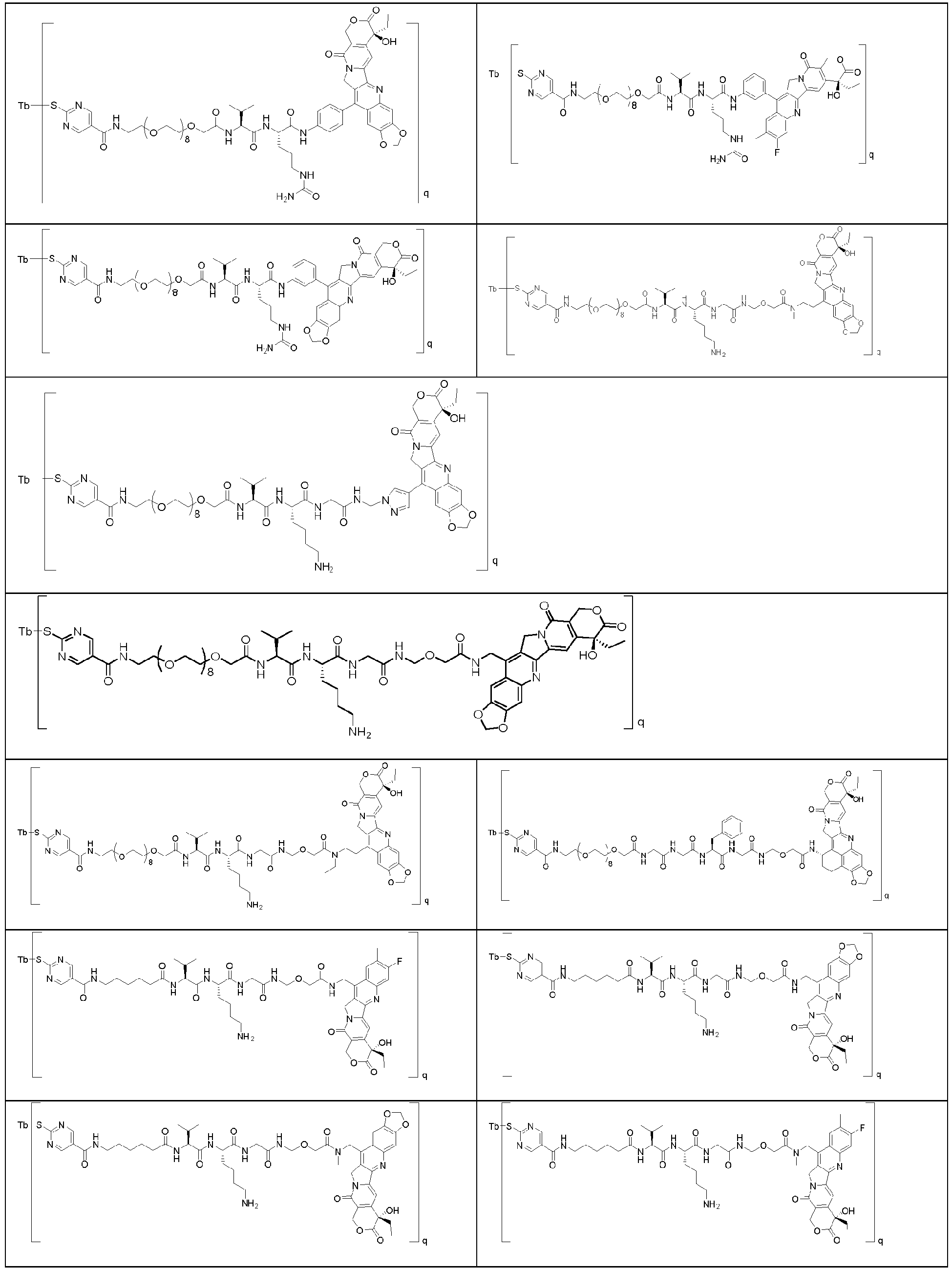

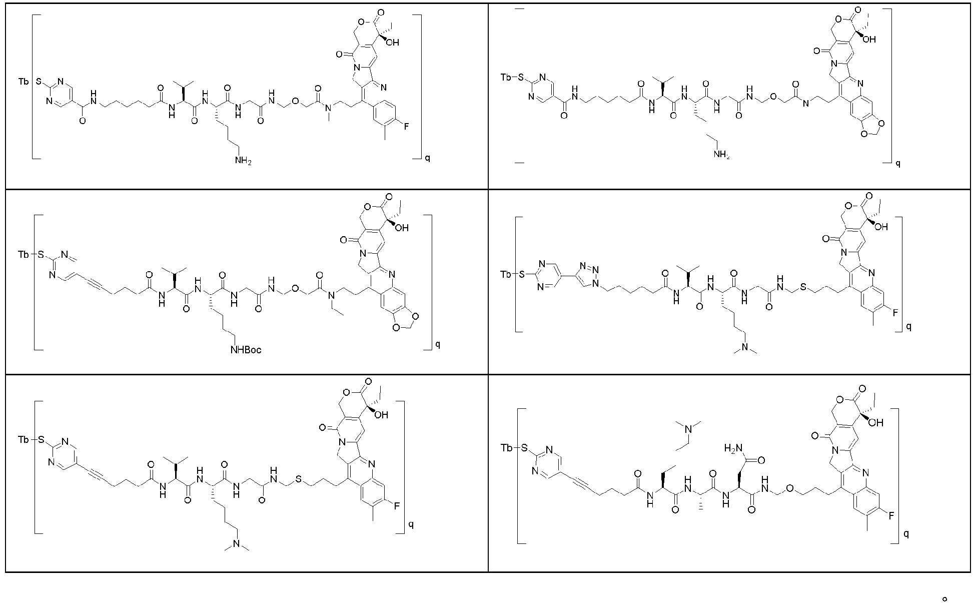

Said Tb antibody or antigen binding fragment thereof may be prepared by various methods known in the art, for example by genetic engineering recombination techniques. For example, DNA molecules encoding the heavy and light chain genes of the antibodies of the present disclosure are obtained by chemical synthesis or PCR amplification. The resulting DNA molecule was inserted into an expression vector and then transfected into the host cell. The transfected host cells are then cultured under specific conditions, and the antibodies of the present disclosure are expressed.

In some embodiments of the present disclosure, the targeting moiety is an anti-B7H3 antibody or an antigen binding fragment thereof. In some embodiments, the anti-B7H3 antibody includes all anti-B7H3 antibodies in prior art, such as enoblituzumab, mirzotamab, omburtamab, as well as anti-B7H3 antibodies referred in CN112521512, WO2021027674, WO2021021543, WO2021006619, CN111662384, CN111454357, WO2020151384, WO2020140094, WO2020103100, WO2020102779, WO2020063673, WO2020047257, WO2020041626, CN110684790, CN110642948, WO2019225787, WO2019226017, US20190338030, CN110305213, WO2018209346, WO2018177393, US9150656, WO2016106004, WO2016044383, WO2016033225, WO2015181267, US20120294796, WO2011109400, CN101104639, WO2004093894, WO2002010187, WO2001018021. In some embodiments, the anti-B7H3 antibody or antigen-binding fragment thereof is selected from antibody M30-H1-L4 in CN 103687945 B and the antibody of mAb-C-DUBA in CN 109069633 A.

In some embodiments, the anti-B7H3 antibody or antigen-binding fragment thereof is antibody comprising Complementarity Determining Regions (CDRs) as shown below or antigen-binding fragment thereof, wherein the CDRs are defined by the IMGT numbering system:
(a) HCDR1 consisting of the sequence of SEQ ID NO:10, HCDR2 consisting of the sequence of SEQ ID NO:11, HCDR3 consisting of the sequence of SEQ ID NO:12; and/or, LCDR1 consisting of the sequence of SEQ ID NO:20, LCDR2 consisting of the sequence of GTF, and LCDR3 consisting of the sequence of SEQ ID NO: 22;
(b) HCDR1 consisting of the sequence of SEQ ID NO:10, HCDR2 consisting of the sequence of SEQ ID NO:11, HCDR3 consisting of the sequence of SEQ ID NO:12; and/or,
   LCDR1 consisting of the sequence of SEQ ID NO:40, LCDR2 consisting of the sequence of GAS, and LCDR3 consisting of the sequence of SEQ ID NO: 42;
(c) HCDR1 consisting of the sequence of SEQ ID NO:30, HCDR2 consisting of the sequence of SEQ ID NO:31, HCDR3 consisting of the sequence of SEQ ID NO:32; and/or,
   LCDR1 consisting of the sequence of SEQ ID NO:40, LCDR2 consisting of the sequence of GAS, and LCDR3 consisting of the sequence of SEQ ID NO: 42;
   or
(d) HCDR1 consisting of the sequence of SEQ ID NO:30, HCDR2 consisting of the sequence of SEQ ID NO:31, HCDR3 consisting of the sequence of SEQ ID NO:32; and/or,
LCDR1 consisting of the sequence of SEQ ID NO:20, LCDR2 consisting of the sequence of GTF, and LCDR3 consisting of the sequence of SEQ ID NO: 22.

In some embodiments, the anti-B7H3 antibody or antigen-binding fragment thereof, defined by the IMGT numbering system, comprises:
a VH comprising heavy chains HCDR1, HCDR2, HCDR3 in (a) and a VL comprising light chains LCDR1, LCDR2, LCDR3 in (a);
a VH comprising heavy chains HCDR1, HCDR2, HCDR3 in (b) and a VL comprising light chains LCDR1, LCDR2, LCDR3 in (b);
a VH comprising heavy chains HCDR1, HCDR2, HCDR3 in (c) and a VL comprising light chains LCDR1, LCDR2, LCDR3 in (c); or
a VH comprising heavy chains HCDR1, HCDR2, HCDR3 in (d) and a VL comprising light chains LCDR1, LCDR2, LCDR3 in (d).

In some embodiments, the anti-B7H3 antibody or antigen-binding fragment thereof comprises the Complementarity Determining Regions (CDRs) as shown below, wherein the CDRs are defined by the Chothia numbering system:
(a) HCDR1 consisting of the sequence of SEQ ID NO:4, HCDR2 consisting of the sequence of SEQ ID NO:5, HCDR3 consisting of the sequence of SEQ ID NO:6; and/or,
   LCDR1 consisting of the sequence of SEQ ID NO:14, LCDR2 consisting of the sequence of SEQ ID NO:15, and LCDR3 consisting of the sequence of SEQ ID NO: 16;
(b) HCDR1 consisting of the sequence of SEQ ID NO:24, HCDR2 consisting of the sequence of SEQ ID NO:25, HCDR3 consisting of the sequence of SEQ ID NO:26; and/or,
   LCDR1 consisting of the sequence of SEQ ID NO:34, LCDR2 consisting of the sequence of SEQ ID NO:35, and LCDR3 consisting of the sequence of SEQ ID NO:36;
(c) HCDR1 consisting of the sequence of SEQ ID NO:4, HCDR2 consisting of the sequence of SEQ ID NO:5, HCDR3 consisting of the sequence of SEQ ID NO:6; and/or,
   LCDR1 consisting of the sequence of SEQ ID NO:34, LCDR2 consisting of the sequence of SEQ ID NO:35, and LCDR3 consisting of the sequence of SEQ ID NO:36;
   or
(d) HCDR1 consisting of the sequence of SEQ ID NO:24, HCDR2 consisting of the sequence of SEQ ID NO:25, HCDR3 consisting of the sequence of SEQ ID NO:26; and/or,
LCDR1 consisting of the sequence of SEQ ID NO:14, LCDR2 consisting of the sequence of SEQ ID NO:15, and LCDR3 consisting of the sequence of SEQ ID NO: 16.

In some embodiments, the anti-B7H3 antibody or antigen-binding fragment thereof, defined by the Chothia numbering system, comprises:
a VH comprising heavy chains HCDR1, HCDR2, HCDR3 in (a) and a VL comprising light chains LCDR1, LCDR2, LCDR3 in (a);
a VH comprising heavy chains HCDR1, HCDR2, HCDR3 in (b) and a VL comprising light chains LCDR1, LCDR2, LCDR3 in (b);
a VH comprising heavy chains HCDR1, HCDR2, HCDR3 in (c) and a VL comprising light chains LCDR1, LCDR2, LCDR3 in (c); or
a VH comprising heavy chains HCDR1, HCDR2, HCDR3 in (d) and a VL comprising light chains LCDR1, LCDR2, LCDR3 in (d).

In some embodiments, the anti-B7H3 antibody or antigen-binding fragment thereof comprises the Complementarity Determining Regions (CDRs) as shown below, wherein the CDRs are defined by the Kabat numbering system:
(a) HCDR1 consisting of the sequence of SEQ ID NO:7, HCDR2 consisting of the sequence of SEQ ID NO:8, HCDR3 consisting of the sequence of SEQ ID NO:9; and/or,
   LCDR1 consisting of the sequence of SEQ ID NO: 17, LCDR2 consisting of the sequence of SEQ ID NO:18, and LCDR3 consisting of the sequence of SEQ ID NO: 19;
(b) HCDR1 consisting of the sequence of SEQ ID NO:27, HCDR2 consisting of the sequence of SEQ ID NO:28, HCDR3 consisting of the sequence of SEQ ID NO:29; and/or,
   LCDR1 consisting of the sequence of SEQ ID NO:37, LCDR2 consisting of the sequence of SEQ ID NO:38, and LCDR3 consisting of the sequence of SEQ ID NO:39;
(c) HCDR1 consisting of the sequence of SEQ ID NO:7, HCDR2 consisting of the sequence of SEQ ID NO:8, HCDR3 consisting of the sequence of SEQ ID NO:9; and/or,
   LCDR1 consisting of the sequence of SEQ ID NO:37, LCDR2 consisting of the sequence of SEQ ID NO:38, and LCDR3 consisting of the sequence of SEQ ID NO:39;
   or
(d) HCDR1 consisting of the sequence of SEQ ID NO:27, HCDR2 consisting of the sequence of SEQ ID NO:28, HCDR3 consisting of the sequence of SEQ ID NO:29; and/or,
LCDR1 consisting of the sequence of SEQ ID NO: 17, LCDR2 consisting of the sequence of SEQ ID NO:18, and LCDR3 consisting of the sequence of SEQ ID NO: 19.

In some embodiments, the anti-B7H3 antibody or antigen-binding fragment thereof, as defined by the Kabat numbering system, comprises:
a VH comprising heavy chains HCDR1, HCDR2, HCDR3 in (a) and a VL comprising light chains LCDR1, LCDR2, LCDR3 in (a);
a VH comprising heavy chains HCDR1, HCDR2, HCDR3 in (b) and a VL comprising light chains LCDR1, LCDR2, LCDR3 in (b);
a VH comprising heavy chains HCDR1, HCDR2, HCDR3 in (c) and a VL comprising light chains LCDR1, LCDR2, LCDR3 in (c); or
a VH comprising heavy chains HCDR1, HCDR2, HCDR3 in (d) and a VL comprising light chains LCDR1, LCDR2, LCDR3 in (d).

In some embodiments, the anti-B7H3 antibody or antigen-binding fragment thereof comprises:
(a) a heavy chain variable region (VH), which comprises an amino acid sequence selected from the following:
   (i) a sequence set forth in SEQ ID NO: 3 or 23;
   (ii) a sequence having one or more amino acid substitutions, deletions or additions or any combination thereof (e.g. 1, 2, 3, 4, 5, 6, 7, 8, 9, or 10 amino acid substitutions, deletions or additions or any combination thereof) compared with the sequence set forth in SEQ ID NO: 3 or 23; or
   (iii) a sequence having at least 70%, at least 75%, at least 80%, at least 85%, at least 90%, at least 91%, at least 92%, at least 93%, at least 94%, at least 95%, at least 96%, at least 97%, at least 98% or at least 99% sequence identity compared with the sequence set forth in SEQ ID NO:3 or 23;
   and/or
(b) a light chain variable region (VL), which comprises an amino acid sequence selected from the following:
   (iv) a sequence set forth in SEQ ID NO: 13 or 33;
   (v) a sequence having one or more amino acid substitutions, deletions or additions or any combination thereof (e.g. 1, 2, 3, 4, 5, 6, 7, 8, 9, or 10 amino acid substitutions, deletions or additions or any combination thereof) compared with the sequence set forth in SEQ ID NO: 13 or 33; or
   (vi) a sequence having at least 70%, at least 75%, at least 80%, at least 85%, at least 90%, at least 91%, at least 92%, at least 93%, at least 94%, at least 95%, at least 96%, at least 97%, at least 98% or at least 99% sequence identity compared with the sequence set forth in SEQ ID NO: 13 or 33.

In some embodiments, the anti-B7H3 antibody or antigen-binding fragment thereof comprises a VH set forth in SEQ ID NO:3, and/or a VL set forth in SEQ ID NO:13.

In some embodiments, the anti-B7H3 antibody or antigen-binding fragment thereof comprises a VH set forth in SEQ ID NO:23, and/or a VL set forth in SEQ ID NO:33.

In some embodiments, the anti-B7H3 antibody or antigen-binding fragment thereof comprises a VH set forth in SEQ ID NO:3, and/or a VL set forth in SEQ ID NO:33.

In some embodiments, the anti-B7H3 antibody or antigen-binding fragment thereof comprises a VH set forth in SEQ ID NO:23, and/or a VL set forth in SEQ ID NO: 13.

In some embodiments, the anti-B7H3 antibody or antigen-binding fragment thereof comprises:
(a) a VH set forth in SEQ ID NO:3 and a VL set forth in SEQ ID NO:13;
(b) a VH set forth in SEQ ID NO:23 and a VL set forth in SEQ ID NO:33;
(c) a VH set forth in SEQ ID NO:3 and a VL set forth in SEQ ID NO:13;
(d) a VH set forth in SEQ ID NO:23 and a VL set forth in SEQ ID NO:13;
(e) a heavy chain variable region (VH) and a light chain variable region (VL), wherein said heavy chain variable region (VH) and light chain variable region (VL) independently have at least 70%, at least 80%, at least 85%, at least 90%, at least 91%, at least 92%, at least 93%, at least 94%, at least 95%, at least 96%, at least 97%, at least 98% or at least 99% sequence identity compared with the VH and VL defined in any one of (a) to (f); or
(f) a heavy chain variable region (VH) and a light chain variable region (VL), wherein said heavy chain variable region (VH) and light chain variable region (VL) independently have one or more amino acid substitutions, deletions or additions or any combination thereof (e.g. 1, 2, 3, 4, 5, 6, 7, 8, 9, or 10 amino acid substitutions, deletions or additions or any combination thereof) compared with the VH and VL defined in any one of (a) to (d). Preferably, the substitution(s) are conservative substitution(s).

In some embodiments, the heavy chain of anti-B7H3 antibody comprises a heavy chain constant region (CH) of a human immunoglobulin or a variant thereof; wherein the variant has up to 50 amino acid conservative substitutions (e.g. up to 45, up to 40, up to 35, up to 30, up to 25, up to 20, up to 15, up to 10 or up to 5 amino acid conservative substitutions; such as 1, 2, 3, 4, 5, 6, 7, 8, 9 or 10 amino acid conservative substitutions), compared with the wild type sequence from which it is derived. In some embodiments, the light chain of anti-B7H3 antibody comprises a light chain constant region (CL) of a human immunoglobulin or a variant thereof; wherein the variant has up to 50 amino acid conservative substitutions (e.g. up to 45, up to 40, up to 35, up to 30, up to 25, up to 20, up to 15, up to 10 or up to 5 amino acid conservative substitutions; such as 1, 2, 3, 4, 5, 6, 7, 8, 9 or 10 amino acid conservative substitutions), compared with the wild type sequence from which it is derived.

In some embodiments, the constant region is altered, e.g., mutated, to modify the properties of the anti-B7H3 antibody molecule (e.g., to alter one or more of the following properties: Fc receptor binding, antibody glycosylation, number of cysteine residues, effector cell function, or complement function). Functional changes can be carried out by substituting at least one amino acid residue in the constant region of the antibody with a different residue, for example, changing the affinity of the antibody for effector ligands (e.g., FcR or complement C1q), thereby changing effector function (e.g., decreasing). The Fc region of the antibody mediates several important effector functions, such as ADCC, phagocytosis (ADCP), CDC, etc.

In some embodiments, the anti-B7H3 antibody or antigen-binding fragment thereof has a heavy chain constant region (CH), which is selected from the heavy chain constant regions of e.g. IgG1, IgG2, IgG3, IgG4, IgM, IgA1, IgA2, IgD, and IgE; particularly from the heavy chain constant regions of e.g. IgG1, IgG2, IgG3, and IgG4; and more particularly from the heavy chain constant region of IgG1 (e.g. human IgG1). In some embodiments, the heavy chain constant region of human IgG1 is as set forth in SEQ ID NO:43. In some embodiments, the antibody or antigen-binding fragment thereof of the present disclosure has a light chain constant region, which is selected from, for example, κ or λ light chain constant region, preferably κ light chain constant region (e.g., human κ light chain constant region). In some embodiments, the light chain constant region has a sequence set forth in SEQ ID NO:44.

In some embodiments, the anti-B7H3 antibody or antigen-binding fragment thereof comprises a CH set forth in SEQ ID NO 43 or a variant thereof, said variant has conservative substitutions of up to 20 amino acids (such as conservative substitutions of up to 20, up to 15, up to 10 or up to 5 amino acids, such as conservative substitutions of 1, 2, 3, 4, 5, 6, 7, 8, 9 or 10 amino acids), compared with SEQ ID NO: 43; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 43.

In some embodiments, the anti-B7H3 antibody or antigen-binding fragment thereof has a light chain constant region or a variant thereof. In some embodiments, the light chain constant region comprises κ light chain constant region. In some embodiments, the light chain constant region comprises a light chain constant region (CL) set forth in SEQ ID NO:44 or a variant thereof, said variant has conservative substitutions of up to 20 amino acids (such as conservative substitutions of up to 20, up to 15, up to 10 or up to 5 amino acids, such as conservative substitutions of 1, 2, 3, 4, 5, 6, 7, 8, 9 or 10 amino acids), compared with SEQ ID NO: 44; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 44;

In some embodiments, the anti-B7H3 antibody or antigen-binding fragment thereof comprises the heavy chain constant region (CH) set forth in SEQ ID NO: 43 and the light chain constant region (CL) set forth in SEQ ID NO: 44.

In some embodiments, the anti-B7H3 antibody or antigen-binding fragment thereof comprises:
(a) a heavy chain, which comprises an amino acid sequence selected from the following:
   (i) a sequence comprising the VH sequence set forth in SEQ ID NO: 3 and the CH sequence set forth in SEQ ID NO: 43;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
(b) a light chain, which comprises an amino acid sequence selected from the following:
   (iv) a sequence comprising the VL sequence set forth in SEQ ID NO: 13 and the CL sequence set forth in SEQ ID NO: 44;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

In some embodiments, the substitution(s) described in (ii) or (v) are conservative substitution(s).

In some embodiments, the anti-B7H3 antibody or antigen-binding fragment thereof comprises:
(a) a heavy chain, which comprises an amino acid sequence selected from the following:
   (i) a sequence comprising the VH sequence set forth in SEQ ID NO: 23 and the CH sequence set forth in SEQ ID NO: 43;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
(b) a light chain, which comprises an amino acid sequence selected from the following:
   (iv) a sequence comprising the VL sequence set forth in SEQ ID NO: 33 and the CL sequence set forth in SEQ ID NO: 44;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

In some embodiments, the substitution(s) described in (ii) or (v) are conservative substitution(s).

In some embodiments, the anti-B7H3 antibody or antigen-binding fragment thereof comprises a heavy chain and a light chain,
the heavy chain comprises:
   (i) the sequence set forth in SEQ ID NO:45;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
the light chain comprises:
   (iv) the sequence set forth in SEQ ID NO:46;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

Preferably, the substitution(s) described in (ii) or (v) are conservative substitution(s).

In some embodiments, the anti-B7H3 antibody or antigen-binding fragment thereof comprises a heavy chain and a light chain,
the heavy chain comprises:
   (i) the sequence set forth in SEQ ID NO:47;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
the light chain comprises:
   (iv) the sequence set forth in SEQ ID NO:48;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

Preferably, the substitution(s) described in (ii) or (v) are conservative substitution(s).

In some embodiments, the anti-B7H3 antibody or antigen-binding fragment thereof is a chimeric, humanized, or fully human antibody.

In some embodiments, the anti-B7H3 antibody or antigen-binding fragment thereof is selected from a scFv, Fab, Fab', (Fab')2, Fv fragments, disulfide-linked Fv(dsFv), and diabody.

In some embodiments, the anti-B7H3 antibody or antigen-binding fragment thereof is scFv. In certain embodiments, the scFv of the present disclosure comprises:
(a) a VH set forth in SEQ ID NO:3 and a VL set forth in SEQ ID NO:13;
(b) a VH set forth in SEQ ID NO:23 and a VL set forth in SEQ ID NO:33;
(c) a VH set forth in SEQ ID NO:3 and a VL set forth in SEQ ID NO:33;
(d) a VH set forth in SEQ ID NO:23 and a VL set forth in SEQ ID NO:13;
(e) a heavy chain variable region (VH) and a light chain variable region (VL), wherein the heavy chain variable region (VH) and the light chain variable region (VL) have at least 70%, at least 80%, at least 85%, at least 90%, at least 91%, at least 92%, at least 93%, at least 94%, at least 95%, at least 96%, at least 97%, at least 98%, or at least 99% sequence identity respectively, compared with the VH and VL in any one of the groups (a) to (d) independently; or
(f) a heavy chain variable region (VH) and a light chain variable region (VL), wherein the heavy chain variable region (VH) and the light chain variable region (VL) independently have one or more amino acid substitutions, deletions or additions or any combination thereof (such as 1, 2, 3, 4, 5, 6, 7, 8, 9 or 10 amino acid substitutions, deletions or additions or any combination thereof) compared with the VH and VL in any one of the groups (a) to (d) respectively. Preferably, the substitution(s) are conservative substitution(s).

In some embodiments, the antibody of the present disclosure is scFv. In certain embodiments, the scFv of the present disclosure comprises:
(i) a sequence set forth in SEQ ID NO:1 or 2;
(ii) a sequence having one or more amino acid substitutions, deletions or additions or any combination thereof (e.g. up to 50, up to 45, up to 40, up to 35, up to 30, up to 25, up to 20, up to 15, up to 10 or up to 5 amino acid substitutions, deletions or additions or any combination thereof; such as 1, 2, 3, 4, 5, 6, 7, 8, 9 or 10 amino acid substitutions, deletions or additions or any combination thereof) compared with the sequence set forth in (i); or
(iii) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence set forth in (i);
preferably, the substitution(s) described in (ii) are conservative substitution(s).

In some embodiments, the anti-B7H3 antibody or antigen-binding fragment thereof is scFv. In certain embodiments, the scFv of the present disclosure comprises a sequence set forth in SEQ ID NO:1 or 2.

In some embodiments, the anti-B7H3 antibody or antigen-binding fragment thereof is selected from antibody 1D1, antibody 1D1-01, antibody 2E3 and antibody 2E3-02.

In some embodiments of the present disclosure, the targeting moiety is trastuzumab or pertuzumab. Trastuzumab is an anti-Her 2 monoclonal antibody whose amino acid sequence is known to those of skill in the art and whose exemplary sequence can be seen, e.g., CN103319599, and the terminal Lys is easily deleted, that does not affect its biological activity (seeing Dick, L. W. et al., Biotechnol. Bioeng., 100: 1132-1143).

Exemplary heavy and light chain sequences of trastuzumab may refer to, for example, IMGT/mAb-DB ID 97. Exemplary heavy and light chain sequences of pertuzumab can refer to SEQ ID No. 16 and SEQ ID No. 15 disclosed in US7560111, and also to IMGT/mAb-DB ID 80.

In some embodiments of the present disclosure, the targeting moiety is an anti-Her3 antibody or antigen binding fragment thereof. In some embodiments, said anti-Her3 antibody comprises all anti-Her3 antibodies in prior art, e.g., see barecetamab, duligotuzumab, elgemtumab, istiratumab, lumretuzumab, patritumab, seribantumab, zenocutuzumab, 202-2-1 antibody, as well as an antibody having a heavy chain set forth in SEQ ID NO: 10 and a light chain set forth in SEQ ID NO:14 disclosed in CN 103189392 B and an antibody represented by IMGT/mAb-DB ID: 546.

In some embodiments, the targeting moiety is an anti-Her3 antibody, and the anti-Her3 antibody or antigen-binding fragment thereof is an antibody comprising Complementarity Determining Regions (CDRs) as shown below or antigen-binding fragment thereof, wherein the CDRs are defined by the IMGT numbering system:
HCDR1 consisting of the sequence of SEQ ID NO: 56, HCDR2 consisting of the sequence of SEQ ID NO: 57, HCDR3 consisting of the sequence of SEQ ID NO: 58; and/or,
LCDR1 consisting of the sequence of SEQ ID NO: 41, LCDR2 consisting of the sequence AAS, LCDR3 consisting of the sequence of SEQ ID NO: 21.

In some embodiments, the anti-Her3 antibody or antigen-binding fragment thereof, as defined by the IMGT numbering system, comprises:
a VH comprising the above-mentioned heavy chain HCDR1, HCDR2, HCDR3 and a VL comprising the above-mentioned light chain LCDR1, LCDR2, LCDR3.

In some embodiments, the anti-Her3 antibody or antigen-binding fragment thereof is an antibody comprising Complementarity Determining Regions (CDRs) as shown below or antigen-binding fragment thereof, wherein the CDRs are defined by the Kabat numbering system:
HCDR1 consisting of the sequence of SEQ ID NO:53, HCDR2 consisting of the sequence of SEQ ID NO:54, HCDR3 consisting of the sequence of SEQ ID NO:55; and/or,
LCDR1 consisting of the sequence of SEQ ID NO:59, LCDR2 consisting of the sequence of SEQ ID NO:60, LCDR3 consisting of the sequence of SEQ ID NO:21.

In some embodiments, the anti-Her3 antibody or antigen-binding fragment thereof, as defined by the Kabat numbering system, comprises: a VH comprising the above-mentioned heavy chain HCDR1, HCDR2, HCDR3 and a VL comprising the above-mentioned light chains LCDR1, LCDR2, LCDR3.

In some embodiments, the anti-Her3 antibody or antigen-binding fragment thereof comprises:
(a) a heavy chain variable region (VH), which comprises an amino acid sequence selected from the following:
   (i) a sequence set forth in SEQ ID NO:49;
   (ii) a sequence having one or more amino acid substitutions, deletions or additions or any combination thereof (e.g. 1, 2, 3, 4, 5, 6, 7, 8, 9, or 10 amino acid substitutions, deletions or additions or any combination thereof) compared with the sequence set forth in SEQ ID NO:49; or
   (iii) a sequence having at least 70%, at least 75%, at least 80%, at least 85%, at least 90%, at least 91%, at least 92%, at least 93%, at least 94%, at least 95%, at least 96%, at least 97%, at least 98% or at least 99% sequence identity compared with the sequence set forth in SEQ ID NO:49;
   and/or
(b) a light chain variable region (VL), which comprises an amino acid sequence selected from the following:
   (iv) a sequence set forth in SEQ ID NO: 50;
   (v) a sequence having one or more amino acid substitutions, deletions or additions or any combination thereof (e.g. 1, 2, 3, 4, 5, 6, 7, 8, 9, or 10 amino acid substitutions, deletions or additions or any combination thereof) compared with the sequence set forth in SEQ ID NO:50; or
   (vi) a sequence having at least 70%, at least 75%, at least 80%, at least 85%, at least 90%, at least 91%, at least 92%, at least 93%, at least 94%, at least 95%, at least 96%, at least 97%, at least 98% or at least 99% sequence identity compared with the sequence set forth in SEQ ID NO:50.

In some embodiments, the anti-Her3 antibody or antigen-binding fragment thereof comprises:
(a) a VH set forth in SEQ ID NO:49, and a VL set forth in SEQ ID NO:50;
(b) a heavy chain variable region (VH) and a light chain variable region (VL), wherein said heavy chain variable region (VH) and light chain variable region (VL) have at least 70%, at least 80%, at least 85%, at least 90%, at least 91%, at least 92%, at least 93%, at least 94%, at least 95%, at least 96%, at least 97%, at least 98% or at least 99% sequence identity respectively, independently compared with the VH and VL described in group (a); or
(c) a heavy chain variable region (VH) and a light chain variable region (VL), wherein said heavy chain variable region (VH) and light chain variable region (VL) independently have one or more amino acid substitutions, deletions or additions or any combination thereof (e.g. 1, 2, 3, 4, 5, 6, 7, 8, 9, or 10 amino acid substitutions, deletions or additions or any combination thereof), compared with the VH and VL described in group (a) respectively. Preferably, the substitution(s) are conservative substitution(s).

In some embodiments, the heavy chain of anti-Her3 antibody comprises a heavy chain constant region (CH) of a human immunoglobulin or a variant thereof; wherein the variant has up to 50 amino acid conservative substitutions (e.g. up to 45, up to 40, up to 35, up to 30, up to 25, up to 20, up to 15, up to 10 or up to 5 amino acid conservative substitutions; such as 1, 2, 3, 4, 5, 6, 7, 8, 9 or 10 amino acid conservative substitutions), compared with the wild type sequence from which it is derived. In some embodiments, the light chain of anti-B7H3 antibody comprises a light chain constant region (CL) of a human immunoglobulin or a variant thereof; wherein the variant has up to 50 amino acid conservative substitutions (e.g. up to 45, up to 40, up to 35, up to 30, up to 25, up to 20, up to 15, up to 10 or up to 5 amino acid conservative substitutions; such as 1, 2, 3, 4, 5, 6, 7, 8, 9 or 10 amino acid conservative substitutions), compared with the wild type sequence from which it is derived.

In some embodiments, the constant region is altered, e.g., mutated, to modify the properties of the anti-Her3 antibody molecule (e.g., to alter one or more of the following properties: Fc receptor binding, antibody glycosylation, number of cysteine residues, effector cell function, or complement function). Functional changes can be carried out by substituting at least one amino acid residue in the constant region of the antibody with a different residue, for example, changing the affinity of the antibody for effector ligands (e.g., FcR or complement C1q), thereby changing effector function (e.g., decreasing). The Fc region of the antibody mediates several important effector functions, such as ADCC, phagocytosis (ADCP), CDC, etc.

In some embodiments, the anti-Her3 antibody or antigen-binding fragment thereof has a heavy chain constant region (CH), which is selected from the heavy chain constant regions of e.g. IgG1, IgG2, IgG3, IgG4, IgM, IgA1, IgA2, IgD, and IgE; particularly from the heavy chain constant regions of e.g. IgG1, IgG2, IgG3, and IgG4; and more particularly from the heavy chain constant region of IgG1 (e.g. human IgG1). In some embodiments, the antibody or antigen-binding fragment thereof of the present disclosure has a light chain constant region, which is selected from, for example, κ or λ light chain constant region, preferably κ light chain constant region (e.g., human κ light chain constant region).

In some embodiments, the anti-Her3 antibody or antigen-binding fragment thereof has a heavy chain constant region (CH) set forth in SEQ ID NO:43 and a light chain constant region set forth in SEQ ID NO:44.

In some embodiments, the anti-Her3 antibody or antigen-binding fragment thereof comprises a heavy chain and a light chain,
the heavy chain comprises:
   (i) a sequence set forth in SEQ ID NO:51;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
a light chain comprises:
   (iv) a sequence set forth in SEQ ID NO:52;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv);
preferably, the substitution(s) described in (ii) or (v) are conservative substitution(s).

In some embodiments, the anti-Her3 antibody or antigen-binding fragment thereof is a chimeric, humanized, or fully human antibody.

In some embodiments, the anti-Her3 antibody or antigen-binding fragment thereof is selected from scFv, Fab, Fab', (Fab')2, Fv fragments, disulfide-linked Fv(dsFv), and diabody.

In some embodiments, the anti-Her3 antibody or antigen-binding fragment thereof is scFv. In certain embodiments, the scFv of the present disclosure comprises:
(a) a VH set forth in SEQ ID NO:49 and a VL set forth in SEQ ID NO:50;
(b) a heavy chain variable region (VH) and a light chain variable region (VL), wherein the heavy chain variable region (VH) and the light chain variable region (VL) have at least 70%, at least 80%, at least 85%, at least 90%, at least 91%, at least 92%, at least 93%, at least 94%, at least 95%, at least 96%, at least 97%, at least 98%, or at least 99% sequence identity respectively, independently compared with the VH and VL in group (a); or
(c) a heavy chain variable region (VH) and a light chain variable region (VL), wherein the heavy chain variable region (VH) and the light chain variable region (VL) independently have one or more amino acid substitutions, deletions or additions or any combination thereof (such as 1, 2, 3, 4, 5, 6, 7, 8, 9 or 10 amino acid substitutions, deletions or additions or any combination thereof) compared with the VH and VL in group (a) respectively. Preferably, the substitution(s) are conservative substitution(s).

In some embodiments, the anti-Her3 antibody or antigen-binding fragment thereof is selected from antibody 202-2-1.

In some embodiments of the present disclosure, the targeting moiety is cetuximab. Cetuximab is an anti-EGFR monoclonal antibody whose amino acid sequence is known to those skilled in the art and whose exemplary sequence can refer to the antibody represented by IMGT/mAb-DB ID 151.

In some embodiments, the ligand drug conjugate is selected from: 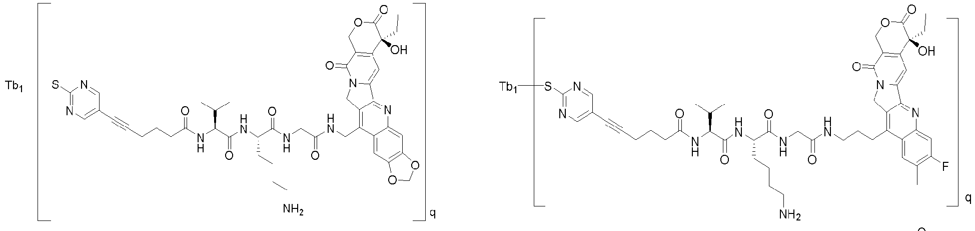 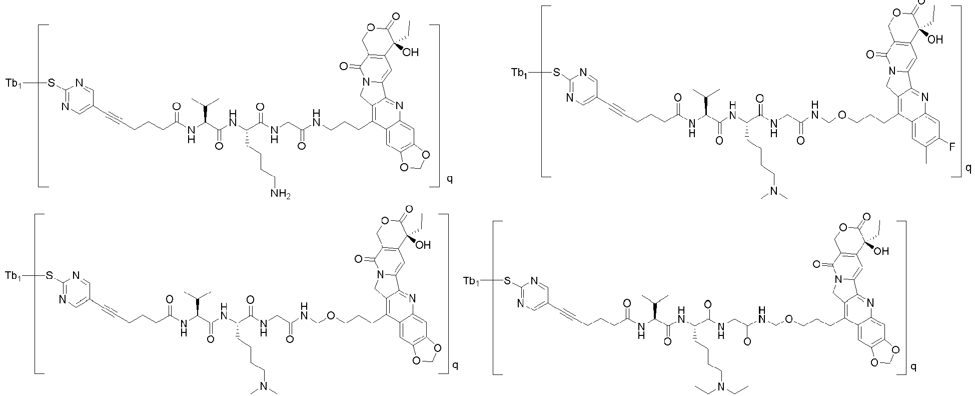 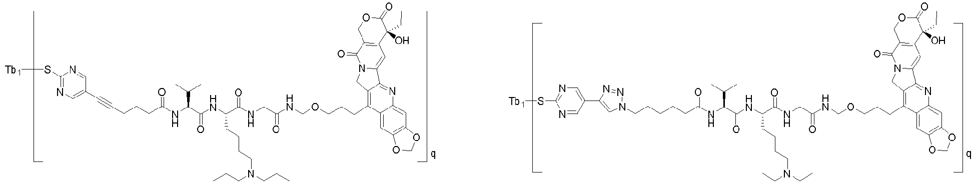 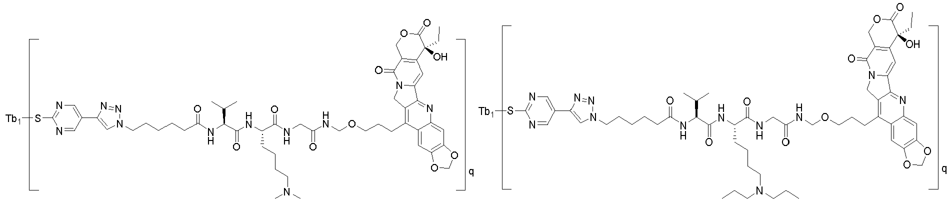 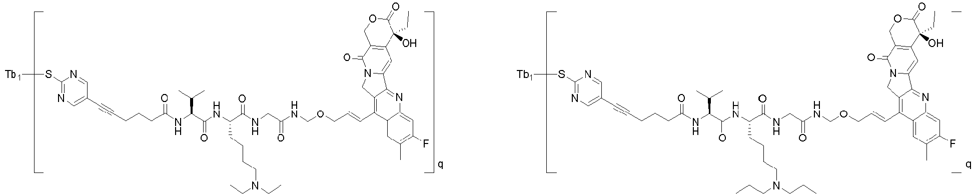 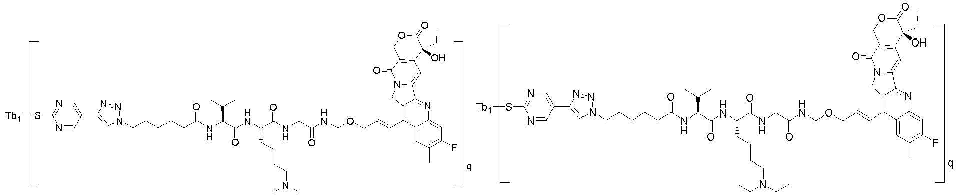 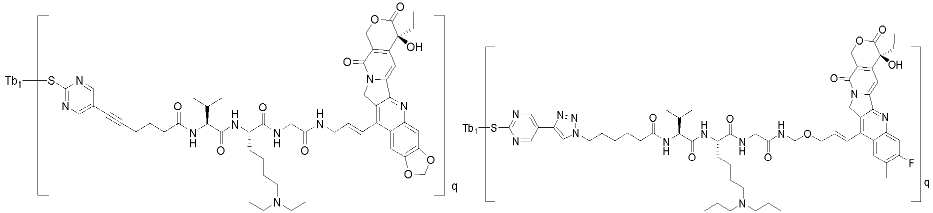 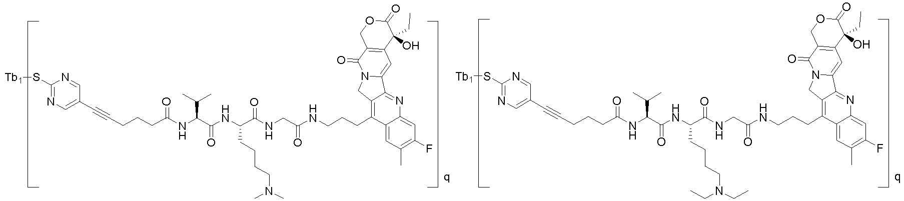 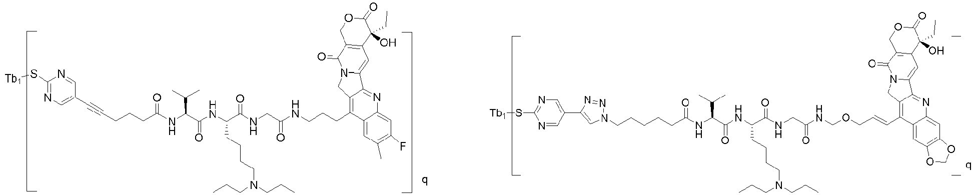 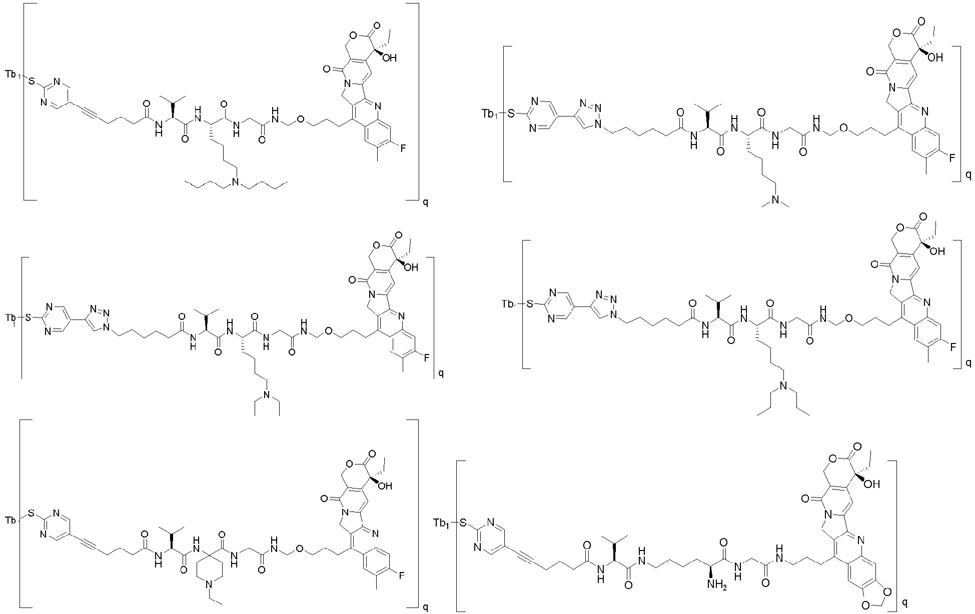 wherein, Tb₁ is an anti-B7H3 antibody or antigen binding fragment thereof, such as enoblituzumab, mirzotamab, omburtamab, antibody 1D1-01, antibody 2E3-02, and preferably antibody 1D1-01 or antibody 2E3-02; q is selected from any numerical value between 0.1 and 16.0, preferably from any numerical value between 2 and 8. In some embodiments, q is 1, 2, 3, 4, 5, 6, 7, 8, 9 or 10. In some preferred embodiments, q is 2, 4, 6 or 8;

In some embodiments, the ligand drug conjugate is selected from: 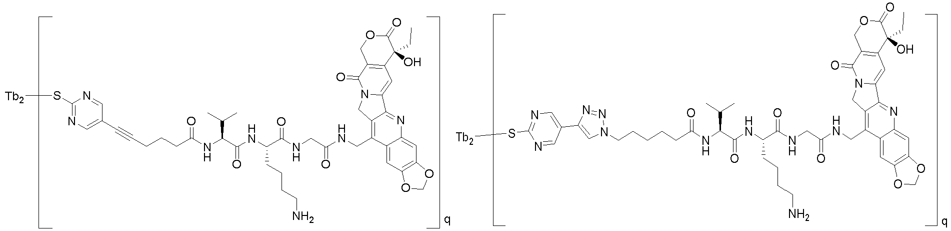 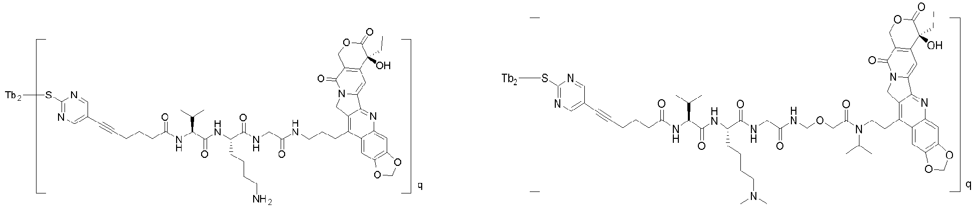 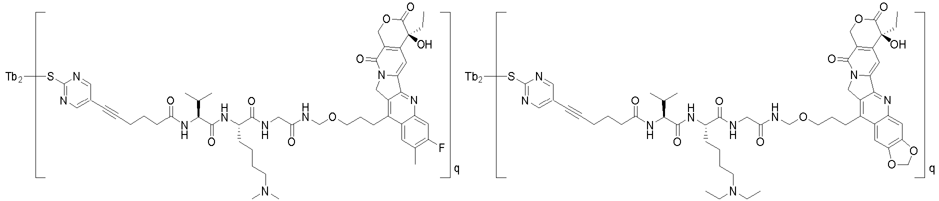 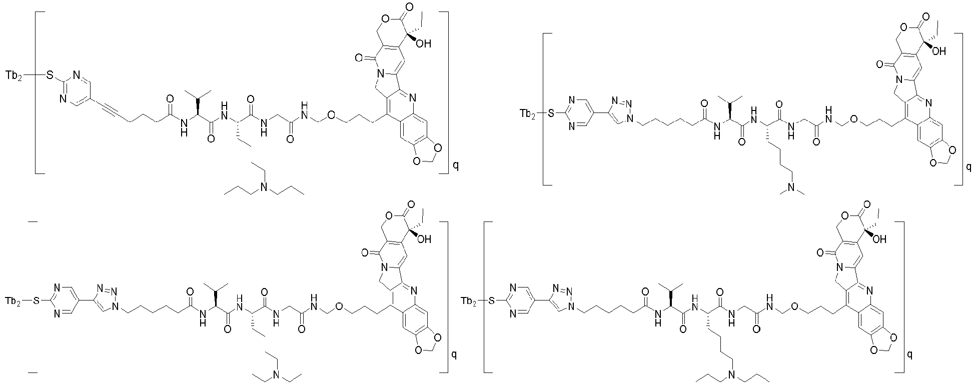 wherein, Tb₂ is an anti-Trop-2 antibody or antigen binding fragment thereof, e.g., datopotamab, sacituzumab, and preferably sacituzumab; q is selected from any numerical value between 0.1 and 16.0, and preferably from any numerical value between 2 and 8. In some embodiments, q is 1, 2, 3, 4, 5, 6, 7, 8, 9 or 10. In some preferred embodiments, more preferably q is 2, 4, 6 or 8.

In some embodiments, the ligand drug conjugate is selected from: 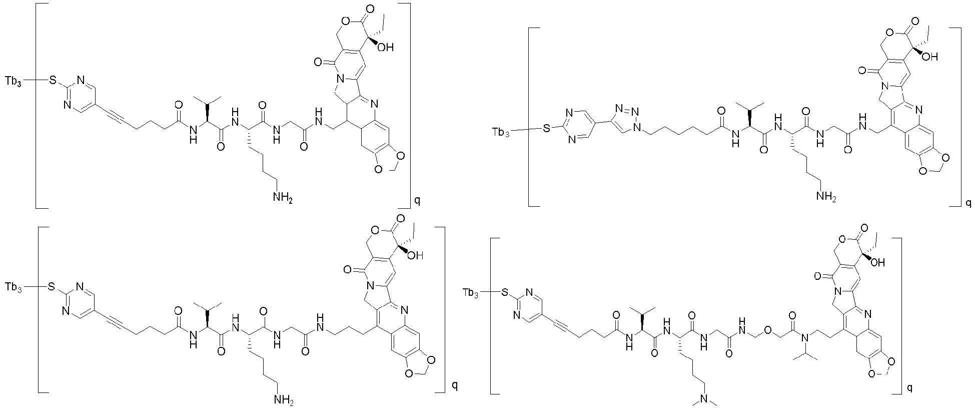 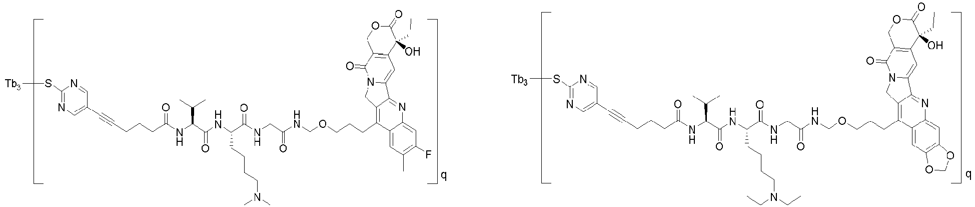 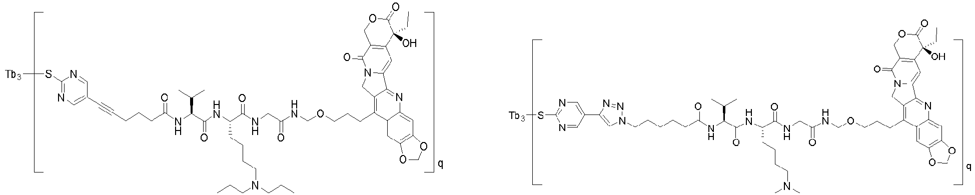 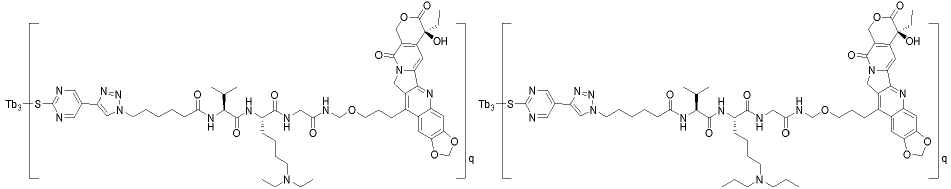 wherein Tb₃ is an anti-Her2 antibody or antigen binding fragment thereof, such as anbenitamab, coprelotamab, disitamab, gancotamab, margetuximab, pertuzumab, timigutuzumab, zanidatamab, Trastuzumab and Pertuzumab, and preferably trastuzumab and Pertuzumab; q is selected from any numerical value between 0.1 and 16.0, and preferably from any numerical value between 2 and 8. In some embodiments, q is 1, 2, 3, 4, 5, 6, 7, 8, 9 or 10. In some preferred embodiments, q is 2, 4, 6 or 8.

In some embodiments, the ligand drug conjugate is selected from: 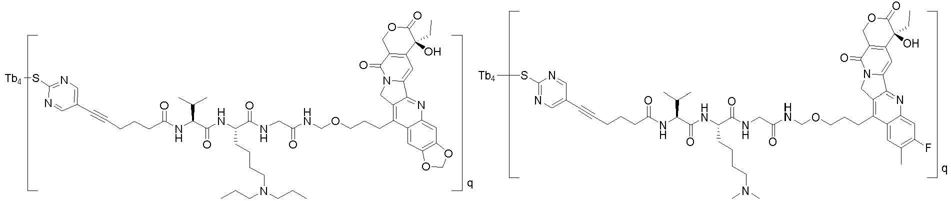 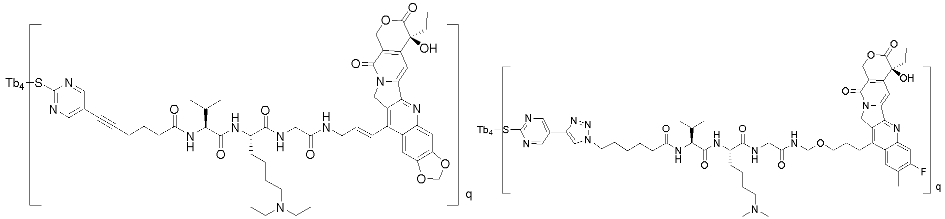 wherein Tb₄ is an anti-Her3 antibody or antigen binding fragment thereof, such as, barecetamab, duligotuzumab, elgemtumab, istiratumab, lumretuzumab, patritumab, seribantumab, zenocutuzumab, antibody 202-2-1, an antibody having a heavy chain set forth in SEQ:10 and a light chain set forth in SEQ:14 disclosed in CN 103189392 B, an antibody represented by IMGT/mAb-DB ID: 546, and antibody 202-2-1, and preferably antibody 202-2-1; q is selected from any numerical value between 0.1 and 16.0, preferably from any numerical value between 2 and 8. In some embodiments, q is 1, 2, 3, 4, 5, 6, 7, 8, 9 or 10. In some preferred embodiments, q is 2, 4, 6 or 8.

In some embodiments, the ligand drug conjugate is selected from: 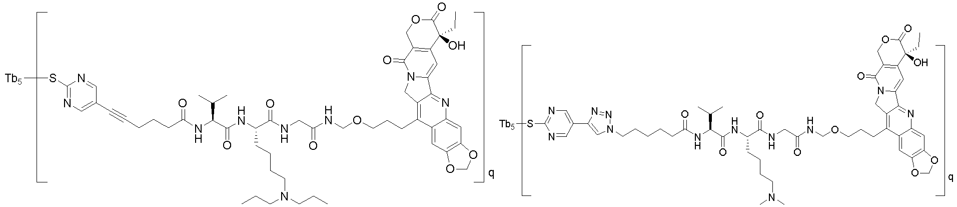 wherein Tb₅ is an anti-EGFR antibody or antigen binding fragment thereof, such as demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, Cetuximab, and preferably Cetuximab; q is selected from any numerical value between 0.1 and 16.0, preferably from any numerical value between 2 and 8. In some embodiments, q is 1, 2, 3, 4, 5, 6, 7, 8, 9 or 10. In some preferred embodiments, q is 2, 4, 6 or 8.

In some embodiments, the ligand drug conjugate is selected from: 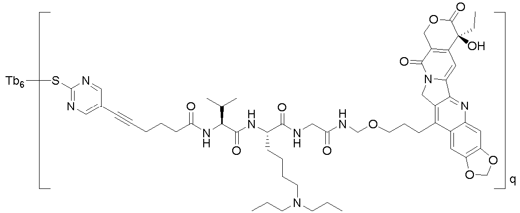 wherein Tb₆ is an antibody that has no tumor cell endocytosis activity, or an antibody that has binding activity to a non-endocytic antigen (e.g., ALCAM/CD 166), such as, antibodies of the IgG isotype which do not have the corresponding cell surface antigen in humans, anti-CD166 antibodies; preferably, anti-chicken lysozyme human IgG1 isotype antibody; q is selected from any numerical value between 0.1 and 16.0, and preferably from any numerical value between 2 and 8. In some embodiments, q is 1, 2, 3, 4, 5, 6, 7, 8, 9 or 10. In some preferred embodiments, q is 2, 4, 6 or 8.

In some embodiments, the ligand drug conjugate is selected from: 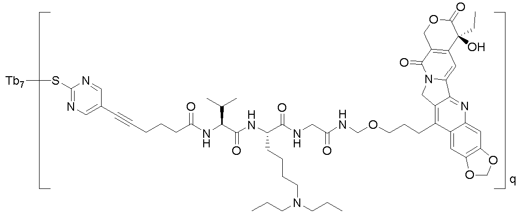 wherein Tb₇ is an antibody with weak or no tumor cell endocytosis activity but with tumor cell surface antigen binding activity; q is selected from any numerical value between 0.1 and 16.0, and preferably from any numerical value between 2 and 8. In some embodiments, q is 1, 2, 3, 4, 5, 6, 7, 8, 9 or 10. In some preferred embodiments, q is 2, 4, 6 or 8.

In some embodiments, the antibodies are antibodies that have activity binding to B7H3 antigens but have no endocytic activity, for example, INV721 and 17-01 disclosed in WO2021168379A1. More specifically, anti-B7H3 antibodies have a VH set forth in SEQ ID NO:2 and a VL set forth in SEQ ID NO:1 disclosed in WO2021168379A1.

In some embodiments, the antibodies are antibodies that have activity binding to GD-2 antigens but have no endocytic activity, for example, INV721 and GD2-5 disclosed in WO2021168379A1. More specifically, anti-GD-2 antibodies have a VH set forth in SEQ ID NO:4 and a VL set forth in SEQ ID NO:3 disclosed in WO2021168379A1.

In some embodiments, the antibodies are antibodies that have activity binding to HER3 antigens but have no endocytic activity, for example, anti-HER3 antibody 21F06 set forth in SEQ ID NO: 22, as disclosed in US10808032B2.

In some embodiments, the antibodies are antibodies that have activity binding to CD20 antigens but have no endocytic activity. Although it has been shown to be difficult for so-called "type II" CD20-specific antibodies to be internalized by CD20-positive target cells, other so-called "type I" CD20-specific antibodies have been found to be internalized and degraded to some extent, depending on activation and inhibition of Fc γR expression levels in target cells interacting with them. In some embodiments, the antibody which has the activity of binding to antigen CD20 and doesn't have endocytic activity is "type II" CD20 specific antibody, such as obinutuzumab.

In some embodiments, the antibody is an antibody which has the activity of binding to a non-endocytic antigen antigen (e.g., ALCAM/CD166). In some embodiments, the antibody is an antibody comprising a VH set forth in SEQ ID No:73 and a VL set forth in SEQ ID No:74, a VH set forth in SEQ ID No:75 and a VL set forth in SEQ ID No:76, a VH set forth in SEQ ID No:77 and a VL set forth in SEQ ID No:78, or a VH set forth in SEQ ID No:79 and a VL set forth in SEQ ID No:88, as described in EP3911682A1.

In some embodiments, the ligand drug conjugate is selected from: 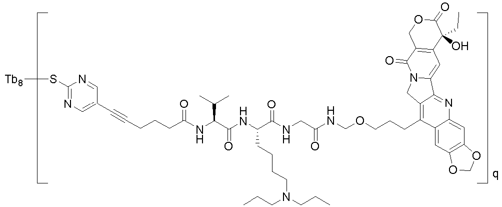 wherein Tb₈ is an antibody having tumor cell endocytosis activity and tumor cell surface antigen binding activity, such as antibody 1D1-01, antibody 2E3-02, sacituzumab, pertuzumab, Trastuzumab, or Cetuximab antibody; q is selected from any numerical value between 0.1 and 16.0, and preferably from any numerical value between 2 and 8. In some embodiments, q is 1, 2, 3, 4, 5, 6, 7, 8, 9 or 10. In some preferred embodiments, q is 2, 4, 6 or 8.

In addition, it should also be noted and it can be understood by those skilled in the art that L is connected to the sulfhydryl group contained in Tb (such as antibody) after disulfide bonds are opened (for example, reduction of the disulfide bond by the reducing agent TCEP can break the disulfide bond to generate sulfhydryl -SH), that is, - S- between L and Tb is not an additional extraneous sulfur atom. For example, wherein, -S- is not an additional extraneous sulfur atom, but is a -S- formed by linking the sulfhydryl group contained in Tb after opening the disulfide bond to L.

In a second aspect of the present disclosure, the present disclosure provides a compound of formula II: 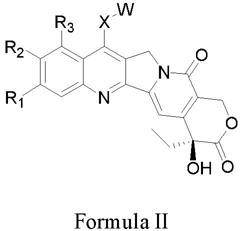
or a stereoisomer of said compound, a prodrug thereof, a pharmaceutically acceptable salt thereof, a pharmaceutically acceptable solvate thereof, or a drug linker conjugate thereof,
wherein:
   R₁, R₂ are each independently selected from H, halogen, -OH, optionally substituted C1-6 alkyl, and optionally substituted C1-6 alkoxy, or,
   R₁ and R₂ together with the carbon atoms to which they are attached form a 5-7 membered carbocyclic ring or a 5-7 membered heterocyclic ring containing one or more O, S, N, carbonyl, sulfoxide group or sulfone group or any combination thereof;
   R₃ is selected from H, halogen, -OH, -NH₂, optionally substituted C1-6 alkyl and optionally substituted C1-6 alkoxy, or,
   R₃ and X, together with the carbon atoms to which they are attached, form a 5-7 membered carbocyclic ring or a 5-7 membered heterocyclic ring containing one or more O, S, N, carbonyl, sulfoxide or sulfone groups or any combination thereof,
   R₃ and R₂, together with the carbon atoms to which they are attached, form a 5-7 membered carbocyclic ring or a 5-7 membered heterocyclic ring containing one or more O, S, N, carbonyl, sulfoxide group or sulfone group or any combination thereof;
   W is absent or present, and when W is present, W is selected from-O-, -S-, -NHR₄ - 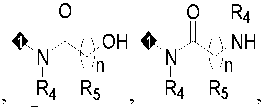 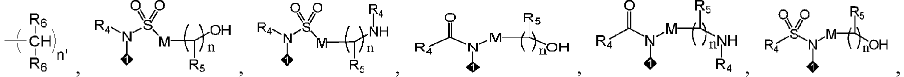 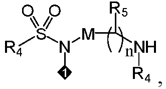 position 1 is attached to X;
   X is selected from a direct bond, optionally substituted -O-(CH₂)ₙ₃-, -NR₄-(CH₂)ₙ₃-, -S-(CH₂)ₙ₃-, carbonyl-(CH₂)ₙ₃-, -SO₂-(CH₂)ₙ₃-, -(CH₂)ₙ₁-, 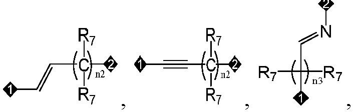 C3-6 cycloalkyl, C6-10 aryl, 5-10 membered heteroaryl and 4-10 membered heterocyclyl, position 1 is attached to the parent ring and position 2 is attached to W; the substituents are selected from one or more C1-4 alkyl groups, C3-6 cycloalkyl groups, or said more C1-4 alkyl groups together with the carbon atom to which they are both attached form a C3-6 cycloalkyl group;
   each M is independently selected from a direct bond and -CR^{5a}R^{5b}-;
   R₄, R₅, R^{5a}, R^{5b}, R₆, R⁷ are each independently selected from H, optionally substituted C1-4 alkyl, optionally substituted C1-4 alkoxy, and optionally substituted C3-6 cycloalkyl;
   n, n', n1, n2, n3 are each independently selected from any integer between 0 and 6;
   wherein, when R₁ and R₂ are both H, and X is-(CH₂)ₙ₁-, and n1 is 1, 2, 3, and 4, W is not -OH or -NHR₄; and the compound of formula II does not comprise:

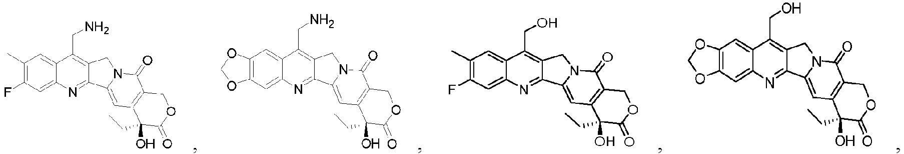

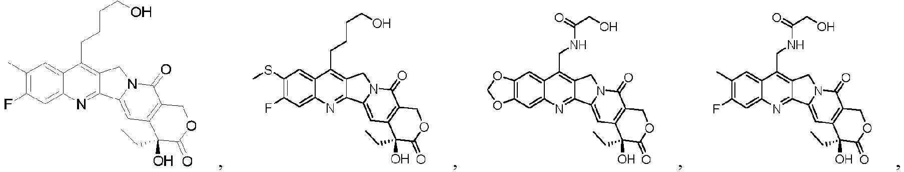

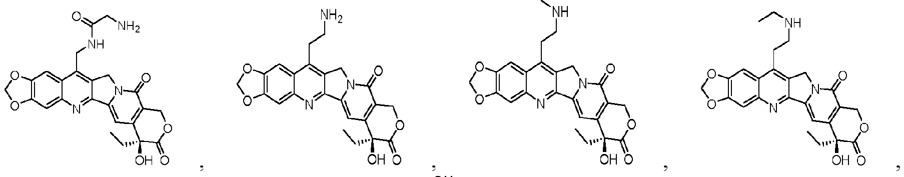

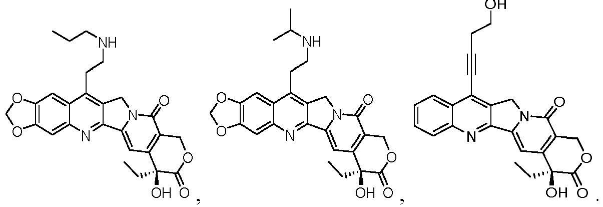

In some embodiments, R₁ and R₂ are each independently selected from H, halogen or C1-4 alkyl.

In some embodiments, R₁ and R₂, together with the carbon atoms to which they are attached, form a 5-6 membered heterocyclic ring containing 1, 2 or 3 O, S or N or any combination thereof.

In some embodiments, R₁ is selected from H and halogen, R₂ is selected from H and C1-4 alkyl.

In some embodiments, R₁ and R₂, together with the carbon atoms to which they are attached, form 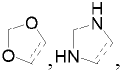 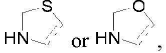 the dotted line indicates where the heterocyclic ring is fused to the benzene ring.

In some embodiments, R₁ is H or F, and R₂ is H or methyl.

In some embodiments, R₁ and R₂ together with the carbon atoms to which they are attached form

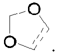

In some embodiments, R₁ is F, R₂ is methyl; or R₁ and R₂ together with the carbon atoms to which they are attached form

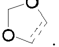

In some embodiments, R₁ is F, R₂ is methyl.

In some embodiments, R₁ and R₂ together with the carbon atoms to which they are attached form

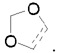

In some embodiments, R₃ is selected from H and C1-4 alkyl.

In some embodiments, R₃ is H.

In some embodiments, W is absent or present, and when W is present, W is selected from -OH, -SH, -NHR₄, 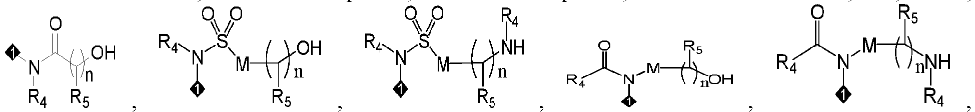 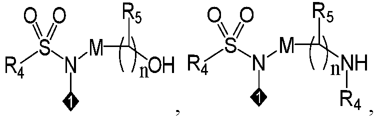 position 1 is attached to X.

In some embodiments, W is absent or present, and when W is present, W is selected from -OH, -SH, -NHR₄, 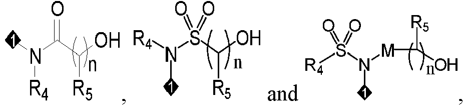 position 1 is attached to X.

In some embodiments, W is selected from -OH, -NHR₄ and 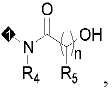 position 1 is attached to X.

In some embodiments, W is selected from -OH and -NHR₄, position 1 is attached to X.

In some embodiments, X is selected from optionally substituted -(CH₂)ₙ₁-, 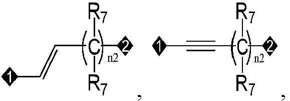 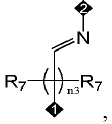 C6-10 aryl, 5-10 membered heteroaryl and 4-10 membered heterocyclyl, at position 1 is attached to the parent ring and position 2 is attached to W; the substituents are selected from 1 or 2 C1-4 alkyl groups, or 2 C1-4 alkyl groups together with the carbon atom to which they are both attached form a C3-6 cycloalkyl group.

In some embodiments, X is selected from optionally substituted 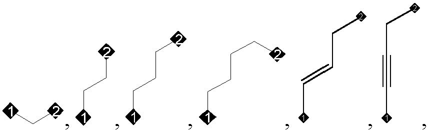 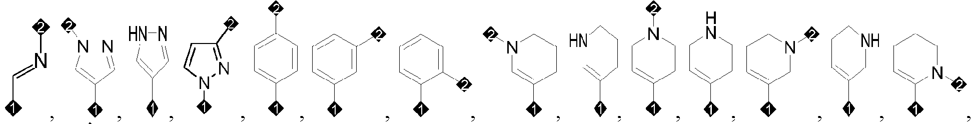 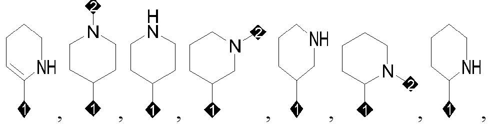 position 1 is attached to the parent ring and position 2 is attached to W; the substituents are selected from 1 or 2 C1-4 alkyl groups (e.g. methyl), or 2 C1-4 alkyl groups (e.g. methyl) together with the carbon atom to which they are both attached form a C3-6 cycloalkyl group (e.g. cyclopropyl).

In some embodiments, X is selected from 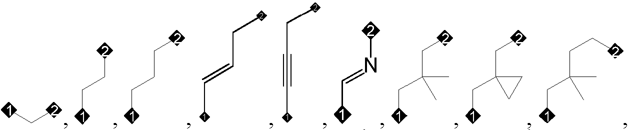 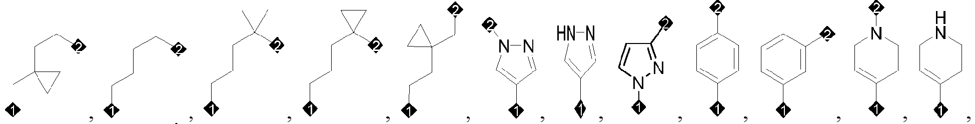 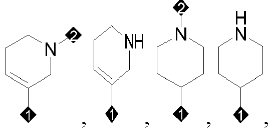 position 1 is attached to the parent ring, and position 2 is attached to W.

In some embodiments, X is selected from 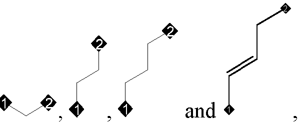 position 1 is attached to the parent ring, and position 2 is attached to W.

In some embodiments, when W is absent, X is selected from 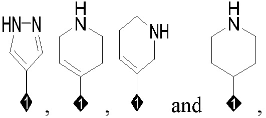 position 1 is attached to the parent ring; when W is present, X is selected from 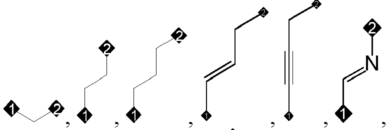 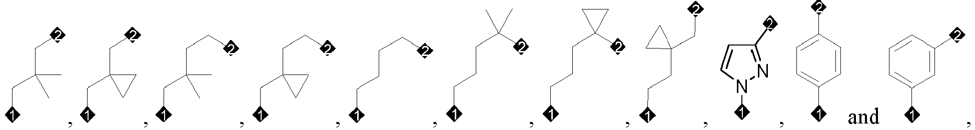 position 1 is attached to the parent ring and position 2 is attached to W.

In some embodiments, W is selected from -OH, -NHR₄ and 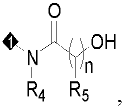 position 1 is attached to X and position 2 is attached to L₄ or L₃; X is selected from 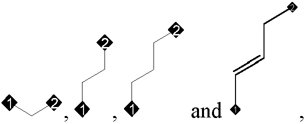 position 1 is attached to the parent ring and position 2 is attached to W. In some embodiments, R₄, and R₅ are each independently selected from H, C1-4 alkyl and C3-6 cycloalkyl.

In some embodiments, each R₄ is independently selected from H, C1-4 alkyl and C3-6 cycloalkyl, R₅ is H.

In some embodiments, each R₄ is independently selected from H, methyl, ethyl, isopropyl, n-propyl, t-butyl, and cyclopropyl, and R₅ is H.

In some embodiments, R^{5a} and R^{5b} are each independently selected from H and C1-4 alkyl;

In some embodiments, R^{5a} and R^{5b} are each independently selected from H and methyl;

In some embodiments, each R⁷ is independently selected from H and C1-4 alkyl;

In some embodiments, R⁷ is H.

In some embodiments, n is 1, 2 or 3.

In some embodiments, n is 1.

In some embodiments, n1 is 1, 2, 3 or 4.

In some embodiments, n2 is 1;

In some embodiments, n3 is 0.

In some embodiments, the compound represented by formula II is selected from any of the following compounds:

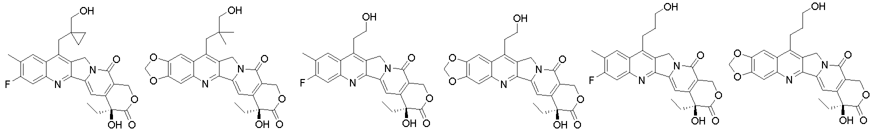

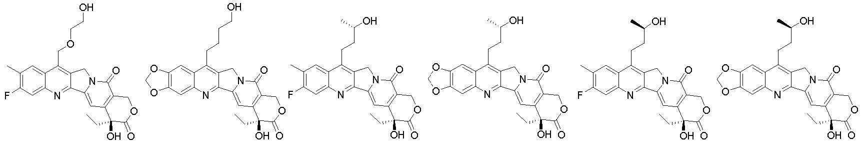

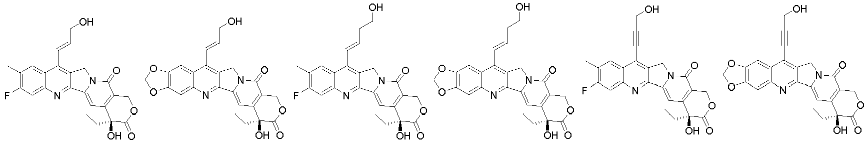

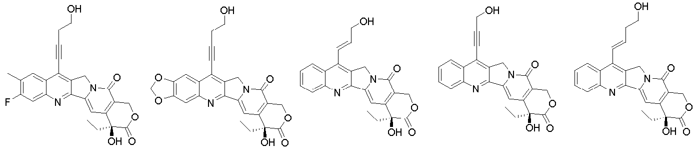

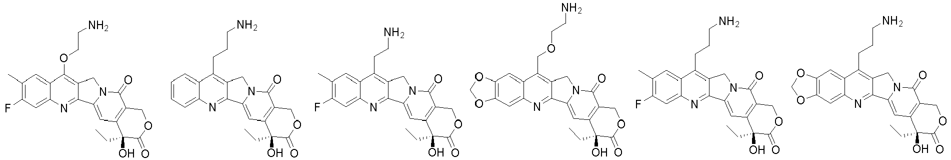

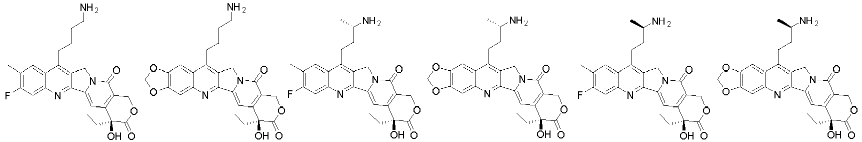

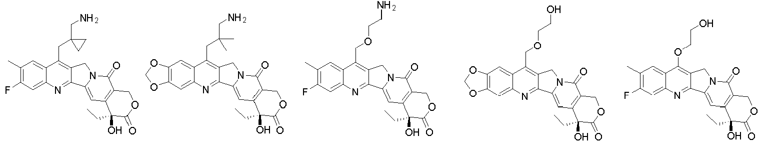

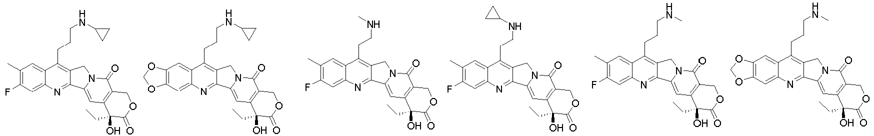

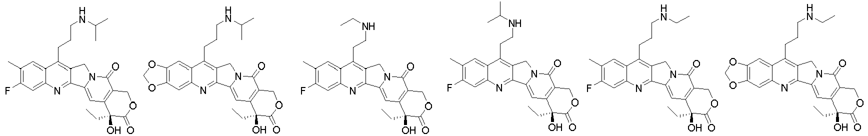

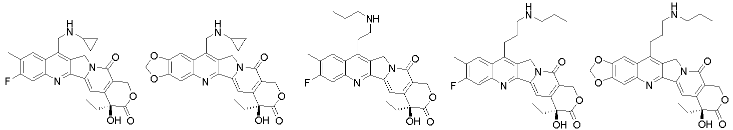

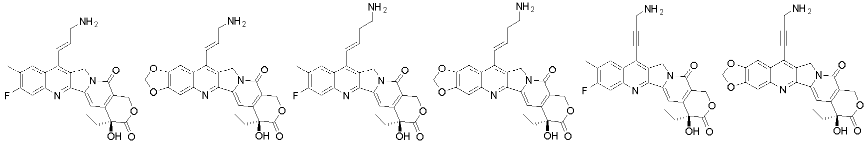

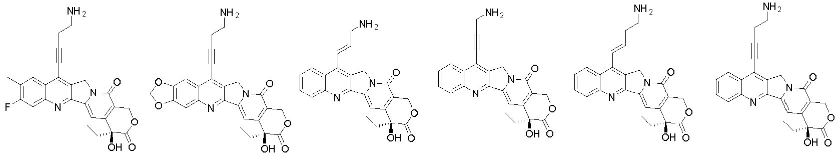

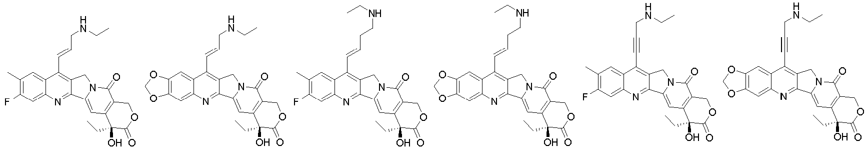

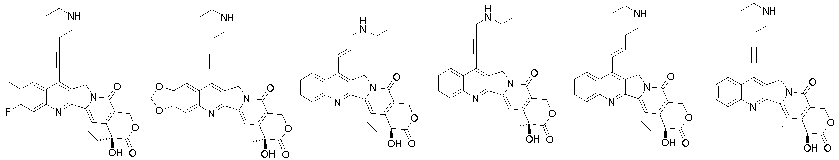

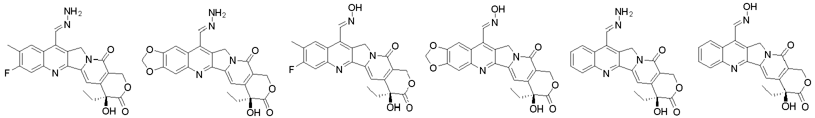

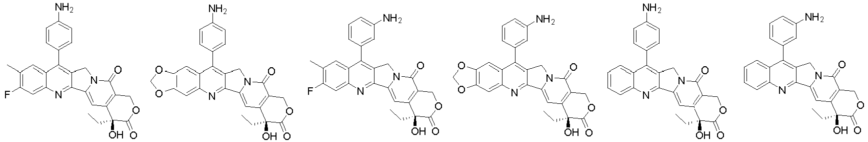

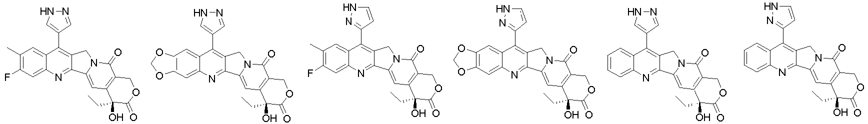

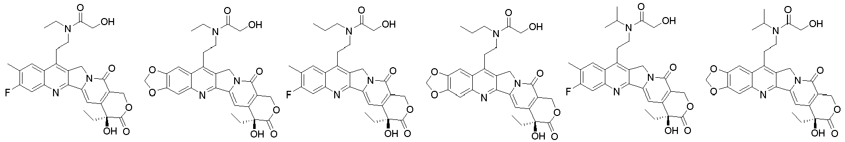

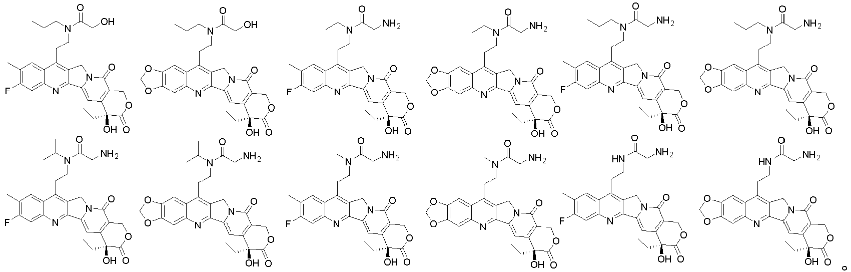

In a third aspect of the present disclosure, the present disclosure provides the drug linker conjugate of formula III, 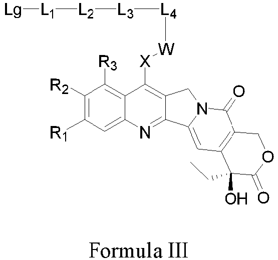
or a stereoisomer of said drug linker conjugate, a prodrug thereof, a pharmaceutically acceptable salt thereof, or a pharmaceutically acceptable solvate thereof,
wherein:
   R₁ and R₂ are each independently selected from H, halogen, -OH, optionally substituted C1-6 alkyl, and optionally substituted C1-6 alkoxy, or,
   R₁ and R₂ together with the carbon atoms to which they are attached form a 5-7 membered carbocyclic ring or a 5-7 membered heterocyclic ring containing one or more O, S, N, carbonyl, sulfoxide group or sulfone group or any combination thereof;
   R₃ is selected from H, halogen, -OH, - NH₂, optionally substituted C1-6 alkyl and optionally substituted C1-6 alkoxy, or,
   R₃ and X, together with the carbon atom to which they are attached, form a 5-7 membered carbocyclic ring or a 5-7 membered heterocyclic ring containing one or more O, S, N, carbonyl, sulfoxide or sulfone groups or any combination thereof, or,
   R₃ and R₂, together with the carbon atoms to which they are attached, form a 5-7 membered carbocyclic ring or a 5-7 membered heterocyclic ring containing one or more O, S, N, carbonyl, sulfoxide group or sulfone group or any combination thereof;
   W is absent or present, and when W is present, W is selected from -O-, -S-, -NR₄-, 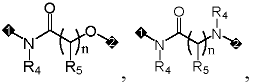 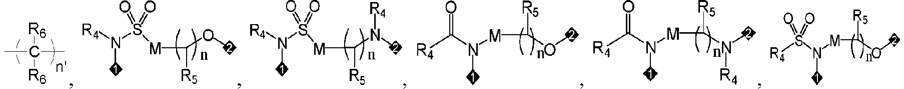 and 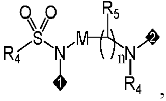 position 1 is attached to X, and position 2 is attached to L₄ or L₃;
   X is selected from a direct bond, optionally substituted -O-(CH₂)ₙ₃-, -N(R₄)-(CH₂)ₙ₃-, -S-(CH₂)ₙ₃-, carbonyl-(CH₂)ₙ₃, -SO₂-(CH₂)ₙ₃-, 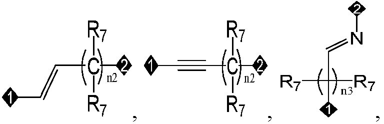 -(CH₂)ₙ₁-, C3-6 cycloalkyl, C6-10 aryl, 5-10 membered heteroaryl and 4-10 membered heterocyclyl, position 1 is attached to the parent ring and position 2 is attached to W or L₄; the substituents are selected from one or more C1-4 alkyl groups, C3-6 cycloalkyl groups, or said more C1-4 alkyl groups together with the carbon atom to which they are both attached form a C3-6 cycloalkyl group;
   each M is independently selected from a direct bond and -CR^{5a}R^{5b}-;
   R₄, R₅, R^{5a}, R^{5b}, R₆, R₇ are each independently selected from H, optionally substituted C1-4 alkyl, optionally substituted C1-4 alkoxy, and optionally substituted C3-6 cycloalkyl;
   n, n', n1, n2, n3 are each independently selected from any integer between 0 and 6;
   L₁ is selected from: 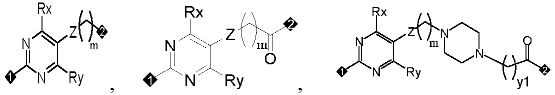 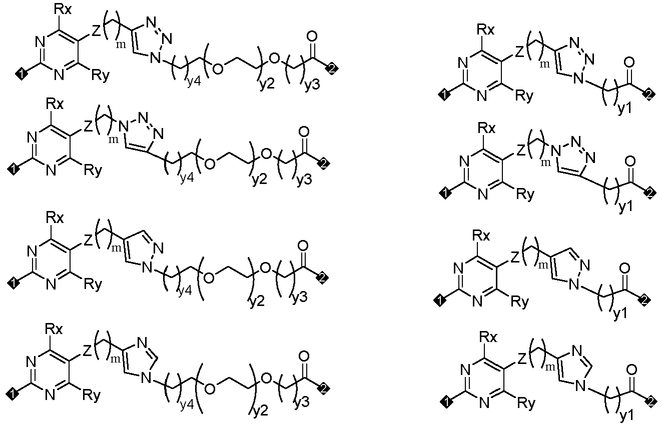 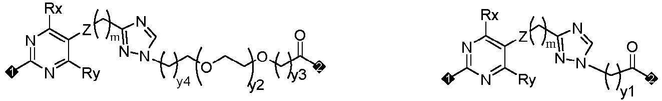 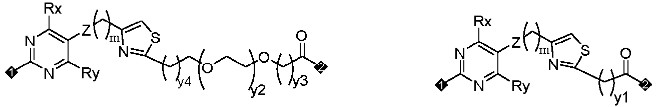 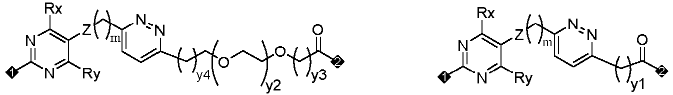 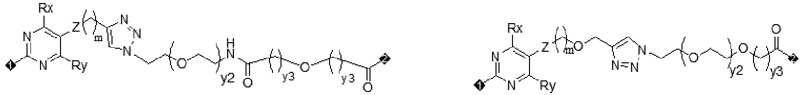 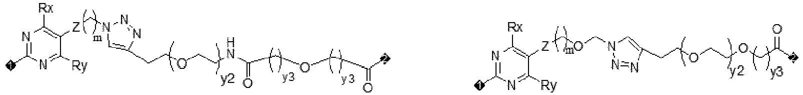 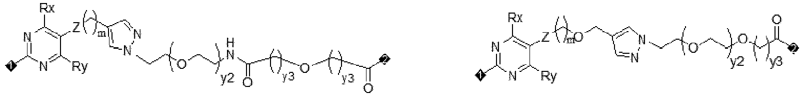 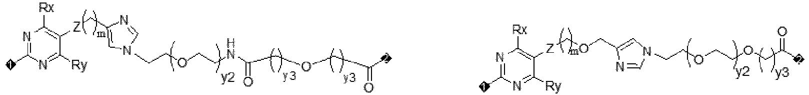 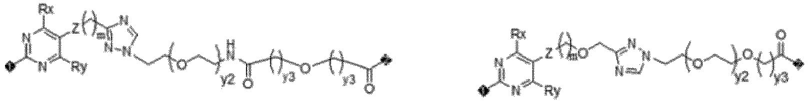 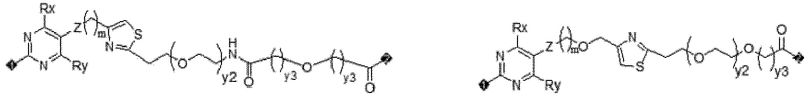 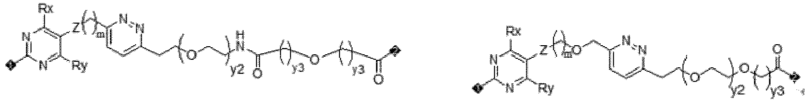 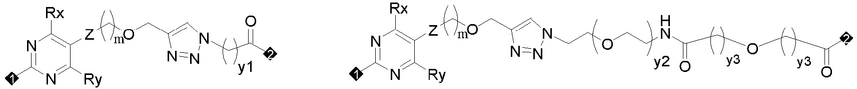 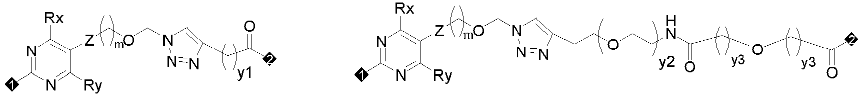 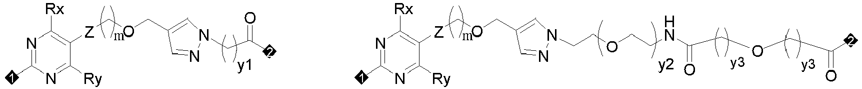 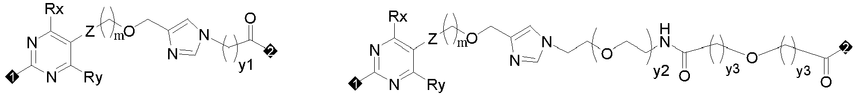 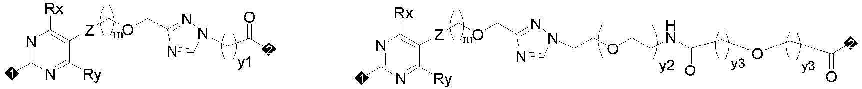 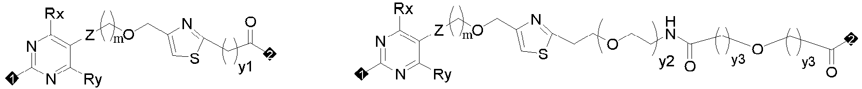 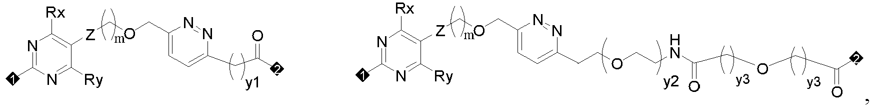
   each Z is independently selected from a direct bond, a carbon-carbon triple bond, a carbon-carbon double bond, C6-10 aryl, 5-10 membered heteroaryl, amido, sulfonamido, imino, and CF₂; Rx and Ry are each independently selected from H and C1-4 alkyl; each m is independently selected from 0, 1, 2, 3, 4, 5 and 6; y1, y2, y3, and y4 are each independently selected from any integer between 0 and 20; position 1 is attached to Lg, and position 2 is attached to L₂ or L₃;
   L₂ is absent or present, and when L₂ is present, L₂ is selected from: 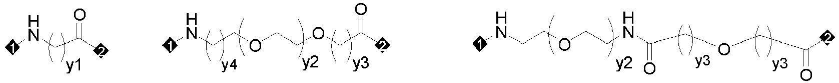 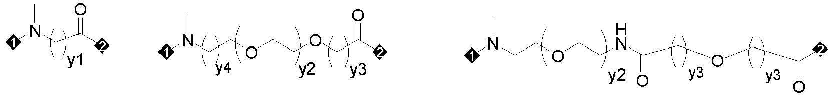 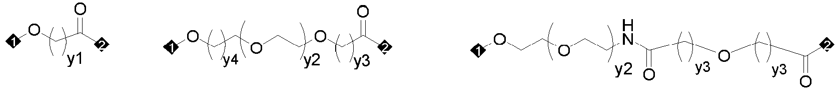 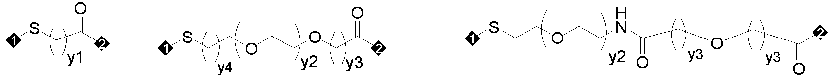 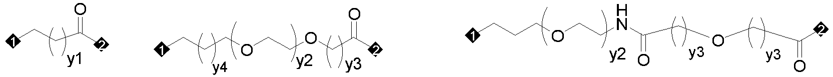 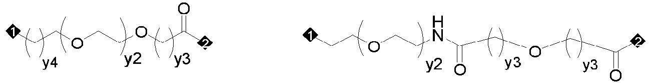 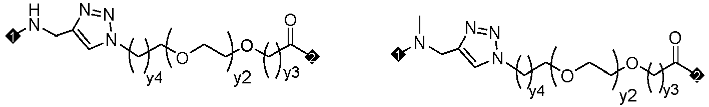 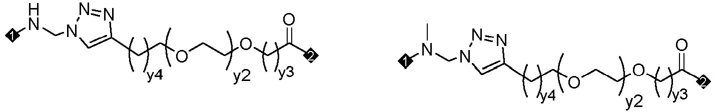 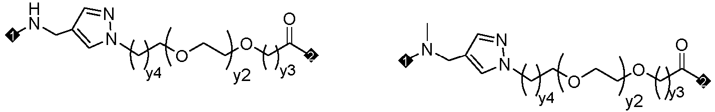 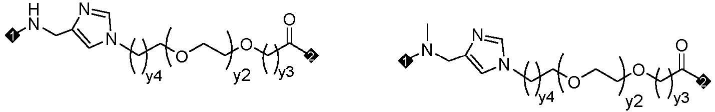 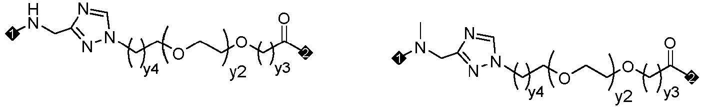 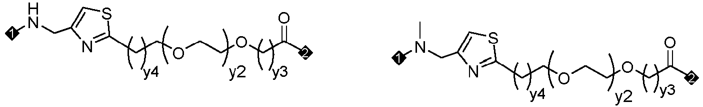 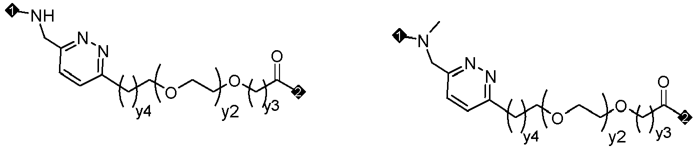 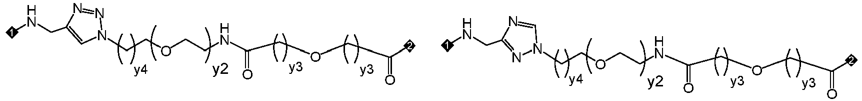 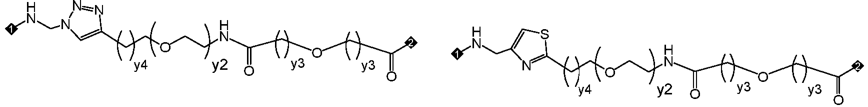 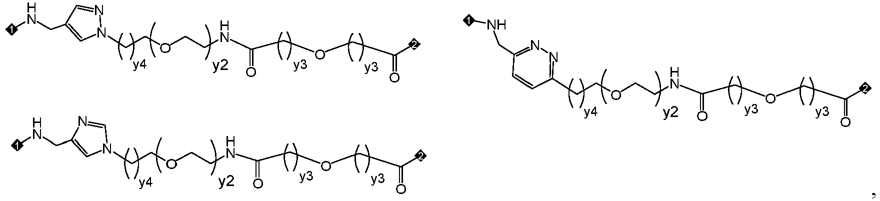
   y1, y2, y3, and y4 are each independently selected from any integer between 0 and 20, position 1 is attached to L₁ and position 2 is attached to L₃;
   L₃ is selected from an amino acid residue or short peptides consisting of 2-10 amino acid residues;
   L₄ is absent or present, and when L₄ is present, L₄ is selected from 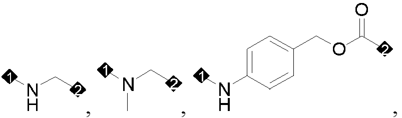 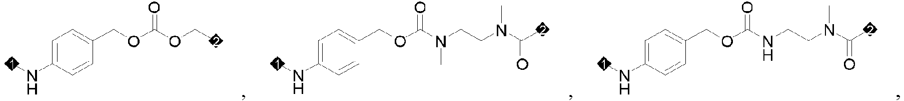 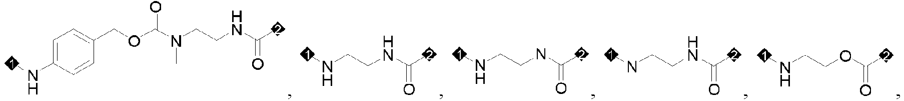
   position 1 is attached to L₃ and position 2 is attached to W or X;
   Lg is a leaving group and Lg is selected from halogen, sulfone group, tertiary amine salt group (Me₃N⁺, Et₃N⁺), diazonium salt group, -OMs, MeSO₂-, and CF₃SO₃-.

In some embodiments, R₁ and R₂ are each independently selected from H, halogen, C1-4 alkyl.

In some embodiments, R₁ and R₂, together with the carbon atoms to which they are attached, form a 5-6 membered heterocyclic ring containing 1, 2 or 3 O, S or N or any combination thereof.

In some embodiments, R₁ is selected from H and halogen, R₂ is selected from H and C1-4 alkyl.

In some embodiments, R₁ and R₂, together with the carbon atoms to which they are attached, form 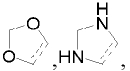 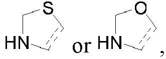 the dotted line indicates where the heterocyclic ring is fused to the benzene ring.

In some embodiments, R₁ is H or F, and R₂ is H or methyl.

In some embodiments, R₁ is F, and R₂ is methyl, or R₁ and R₂, together with the carbon atoms to which they are attached, form

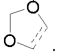

In some embodiments, R₁ is F, and R₂ is methyl.

In some embodiments, R₁ and R₂, together with the carbon atoms to which they are attached, form

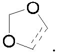

In some embodiments, R₃ is selected from H, C1-4 alkyl.

In some embodiments, R₃ and X, together with the carbon atoms to which they are attached, form a 5-6 membered carbocyclic ring.

In some embodiments, R₃ is H; or, R₃ and X together with the carbon atom to which they are attached form 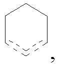 the dotted line indicates where the carbocyclic ring is fused to the benzene ring and pyridine ring.

In some embodiments, R₃ is H.

In some embodiments, R₃ and X, together with the carbon atom to which they are attached, form 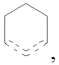 the dotted line indicates where the carbocyclic ring is fused to the benzene ring and pyridine ring.

In some embodiments, W is absent or present, and when W is present, W is selected from -O-, -S-, -NR₄-, 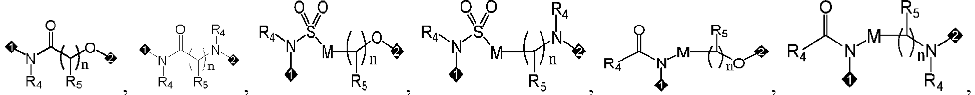 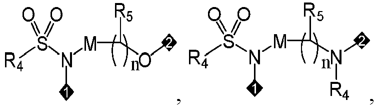 position 1 is attached to X, and position 2 is attached to L₄ or L₃.

In some embodiments, W is absent or present, and when W is present, W is selected from -O-, -S-, -NR₄-, 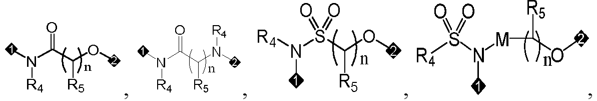 position 1 is attached to X, and position 2 is attached to L₄ or L₃.

In some embodiments, W is selected from -O-, -NR₄-, 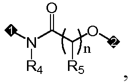 position 1 is attached to X, and position 2 is attached to L₄ or L₃.

In some embodiments, W is selected from -O-, -NR₄-, position 1 is attached to X, and position 2 is attached to L₄ or L₃.

In some embodiments, X is selected from optionally substituted -(CH₂)ₙ₁-, 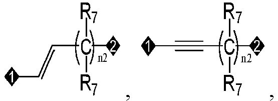 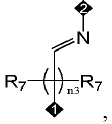 C6-10 aryl, 5-10 membered heteroaryl and 4-10 membered heterocyclyl, position 1 is attached to the parent ring and position 2 is attached to W or L₄ ; the substituents are selected from 1 or 2 C1-4 alkyl groups, or 2 C1-4 alkyl groups together with the carbon atom to which they are both attached form a C3-6 cycloalkyl group.

In some embodiments, X is selected from optionally substituted 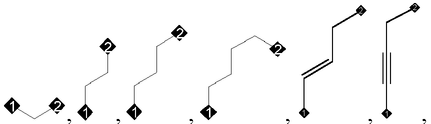 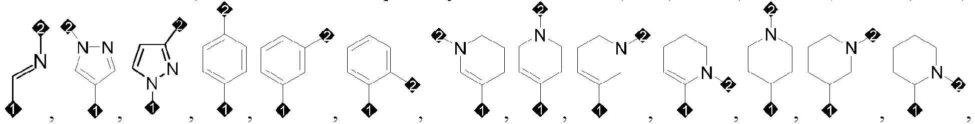 position 1 is attached to the parent ring and position 2 is attached to W or L₄; the substituents are selected from 1 or 2 C1-4 alkyl groups (e.g. methyl), or 2 C1-4 alkyl groups together with the carbon atom to which they are both attached form a C3-6 cycloalkyl group (e.g. cyclopropyl).

In some embodiments, X is selected from 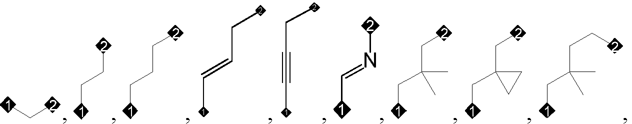 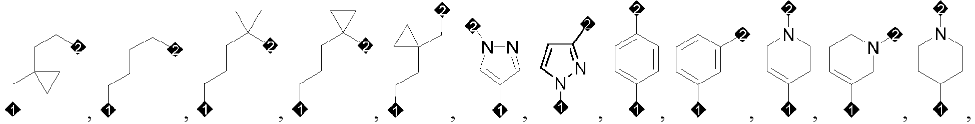 position 1 is attached to the parent ring and position 2 is attached to W or L₄.

In some embodiments, X is selected from 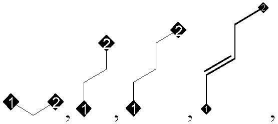 position 1 is attached to the parent ring and position 2 is attached to W.

In some embodiments, when W is absent, X is selected from 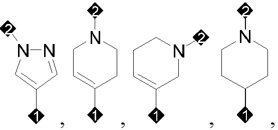 position 1 is attached to the parent ring and position 2 is attached to L₄; when W is present, X is selected from 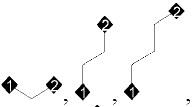 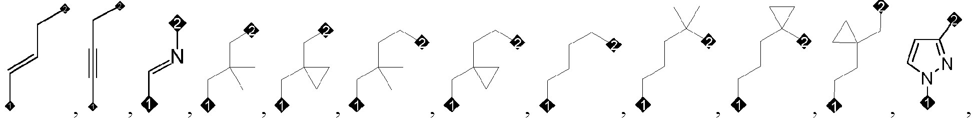 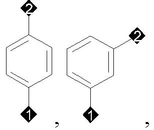 position 1 is attached to the parent ring and position 2 is attached to W.

In some embodiments, W is selected from -O-, -NR₄-, 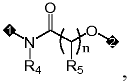 position 1 is attached to X and position 2 is attached to L₄ or L₃.; X is selected from 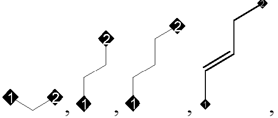 position 1 is attached to the parent ring and position 2 is attached to W.

In some embodiments, R₄, R₅ are each independently selected from H, C1-4 alkyl and C3-6 cycloalkyl.

In some embodiments, each R₄ is independently selected from H, C1-4 alkyl and C3-6 cycloalkyl, R₅ is H.

In some embodiments, each R₄ is independently selected from H, methyl, ethyl, n-propyl, isopropyl, t-butyl, and cyclopropyl, and R₅ is H.

In some embodiments, R^{5a}, R^{5b} are each independently selected from H and C1-4 alkyl.

In some embodiments, R^{5a}, R^{5b} are each independently selected from H and methyl.

In some embodiments, each R⁷ is independently selected from H and C1-4 alkyl.

In some embodiments, R⁷ is H.

In some embodiments, n is selected from 1, 2 or 3.

In some embodiments, n is 1.

In some embodiments, n1 is selected from 1, 2, 3 or 4.

In some embodiments, n2 is 1.

In some embodiments, n3 is 0.

In some embodiments, L₁ is selected from 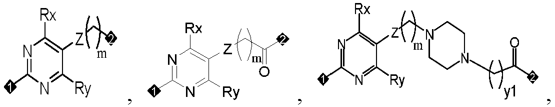 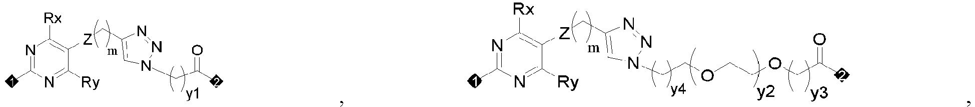 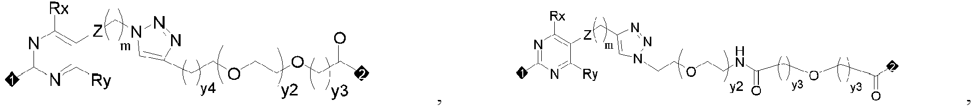 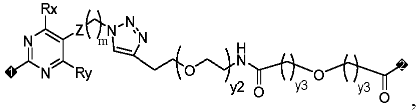 each Z is independently selected from a direct bond, a carbon-carbon triple bond, a carbon-carbon double bond, C6-10 aryl, 5-10 membered heteroaryl, and amido (each Z is preferably selected from a direct bond, a carbon-carbon triple bond, a carbon-carbon double bond); Rx, Ry are each independently selected from H and C1-4 alkyl; each m is independently selected from 0, 1, 2, 3, 4, 5 and 6; y1 is selected from any integer between 1 and 6 (such as 4, 5, 6); each y2 is independently selected from any integer between 0 and 15 (such as 6-15); each y3 is independently selected from 1, 2, or 3; each y4 is independently selected from 0 or 1; position 1 is attached to Lg, and position 2 is attached to L₂ or L₃; for example, each Z is independently selected from a direct bond, a carbon-carbon triple bond, a carbon-carbon double bond; Rx, Ry are each independently selected from H and C1-4 alkyl; each m is independently selected from 0, 1, 2, 3, 4, 5 and 6; y1 is selected from any integer between 1 and 6 (such as 4, 5, 6); each y2 is independently selected from any integer between 0 and 15 (such as 6-15); each y3 is independently selected from 1, 2, or 3; each y4 is independently selected from 0 or 1; position 1 is attached to Lg via an S atom, and position 2 is attached to L₂ or L₃.

In some embodiments, L₁ is selected from 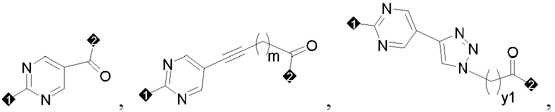 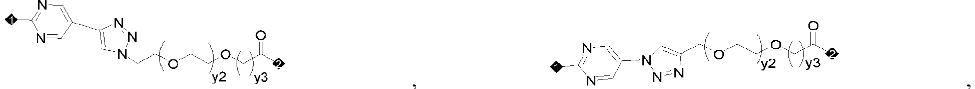 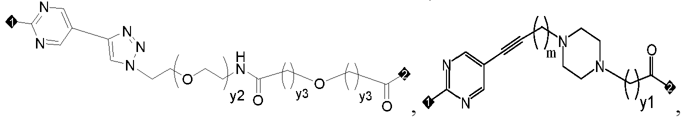 m is selected from 2, 3 and 4, y1 is selected from any integer between 1 and 6 (such as 4, 5, 6), each y2 is independently selected from any integer between 0 and 10 (such as 6-10), each y3 is independently selected from 1 or 2, position 1 is attached to Lg, position 2 is attached to L₂ or L₃.

In some embodiments, L₁ is selected from 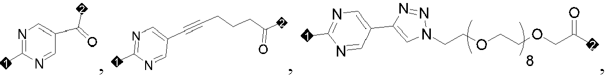 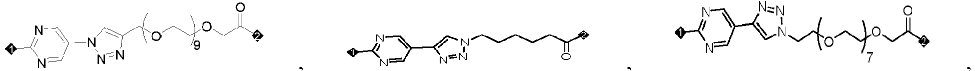 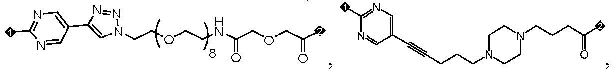 position 1 is attached to Lg, position 2 is attached to L₂ or L₃.

In some embodiments, L₁ is selected from 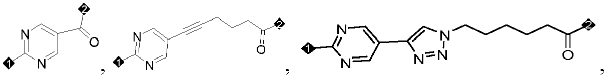 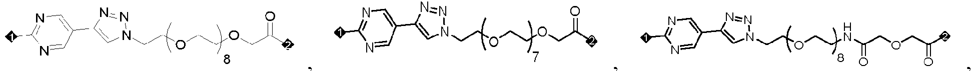 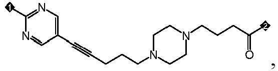 position 1 is attached to Lg, position 2 is attached to L₂ or L₃.

In some embodiments, L₁ is selected from 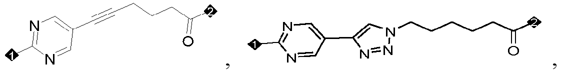 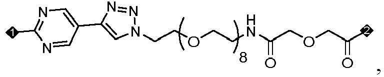 position 1 is attached to Lg, position 2 is attached to L₂ or L₃.

In some embodiments, L₁ is selected from 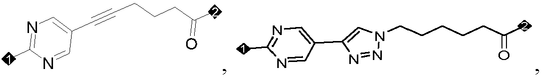 position 1 is attached to Lg, position 2 is attached to L₂ or L₃.

In some embodiments, L₂ is absent or present, and when L₂ is present, L₂ is selected from 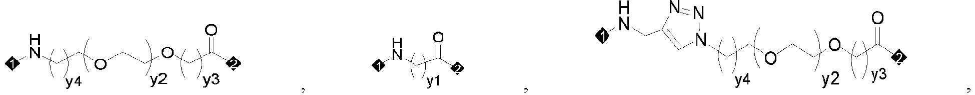 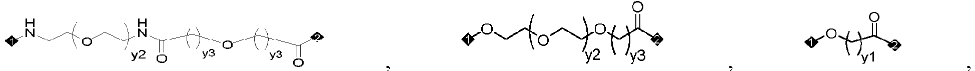 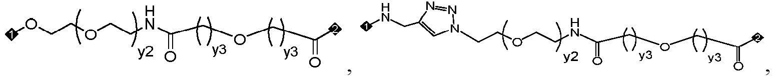 y1 is selected from any integer between 1 and 6 (such as 4, 5, 6); each y2 is independently selected from any integer between 0 and 10 (such as 6-10); each y3 is independently selected from 1 or 2; each y4 is independently selected from 0 or 1; position 1 is attached to L₁ and position 2 is attached to L₃.

In some embodiments, L₂ is absent or present, and when L₂ is present, L₂ is selected from 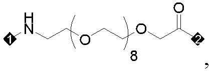 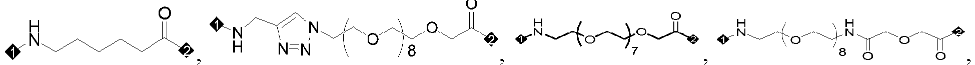 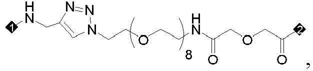 position 1 is attached to L₁ and position 2 is attached to L₃.

In some embodiments, L₂ is absent or present, and when L₂ is present, L₂ is selected from 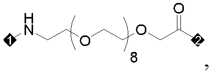 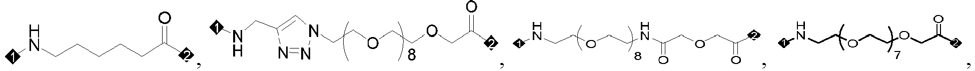 position 1 is attached to L₁ and position 2 is attached to L₃.

In some embodiments, L₂ is absent or present, and when L₂ is present, L₂ is selected from 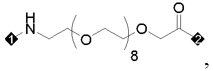 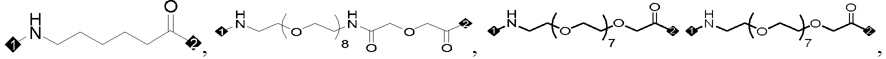 position 1 is attached to L₁ and position 2 is attached to L₃.

In some embodiments, L₂ is absent.

In some embodiments, L₂ is selected from

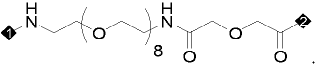

In some embodiments, L₃ is selected from an amino acid residue or short peptides consisting of 2-10 amino acid residues; the amino acid residues are selected from natural amino acid residues, non-natural amino acid residues, or selected from amino acid residues represented by AA¹ or stereoisomer thereof.

In some embodiments, L₃ is selected from amino acid residues Val, D-Val, Cit, Phe, Lys, Lys (Ac), Leu, Gly, Ala, Asn, Asp, Arg, and AA¹, or short peptides consisting of 2-10 amino acid residues selected from Val, Cit, Phe, Lys, D-Val, Leu, Gly, Ala, Asn, Asp, and AA¹;

In some embodiments, L₃ is selected from Val, Cit, Phe, Lys, D-Val, Leu, Gly, Ala, Asn, AA¹, Val-Cit, Cit-Val, Cit-Ala, Val-Ala, Lys-Val, Val-Lys(Ac), Phe-Lys, Phe-Lys(Ac), Ala-Ala, Val-AA¹, Ala-AA¹, Gly-AA¹, AA¹-Gly, Ala-Ala-Ala, Ala-Ala-Asn, Ala-Ala-Asp, Val-AA¹-Gly, Ala-AA¹-Gly, Gly-AA¹-Gly, Lys-Ala-Ala-Asn, Lys-Ala-Ala-Asp, Gly-Phe-Gly, Gly-Gly-Phe-Gly, D-Val-Leu-Lys, Gly-Gly-Arg, Ala-Ala-Asn, Gly-Gly-Phe, Val-Lys-Gly, Val-Lys-Gly-Gly, Val-Lys, and Lys-Ala-Asn.

In some embodiments, L₃ is selected from AA¹, AA¹-Gly, Val-Cit, Val-AA¹-Gly, AA¹-Ala-Asn, and Gly-Gly-Phe-Gly.

In some embodiments, L₃ is selected from AA¹, Val-AA¹-Gly.

In some embodiments, L₃ is selected from Val-AA¹-Gly.

In some embodiments, L₃ is selected from 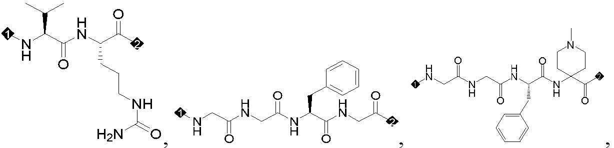 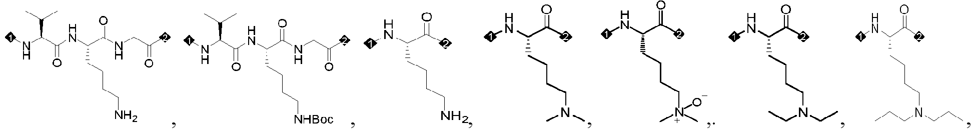 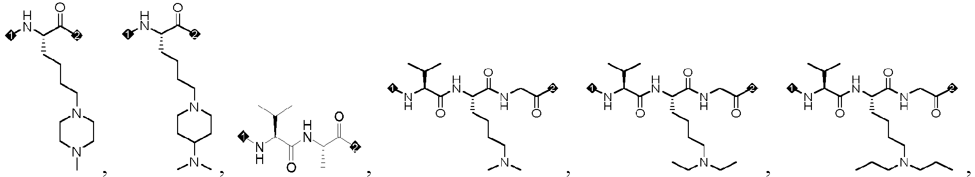 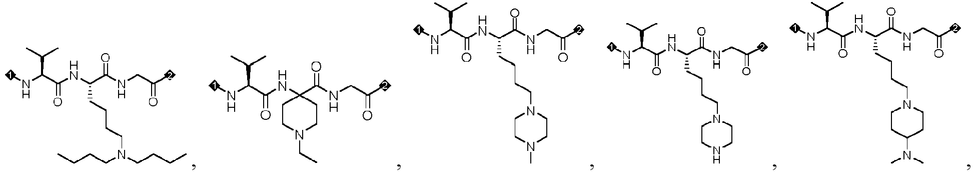 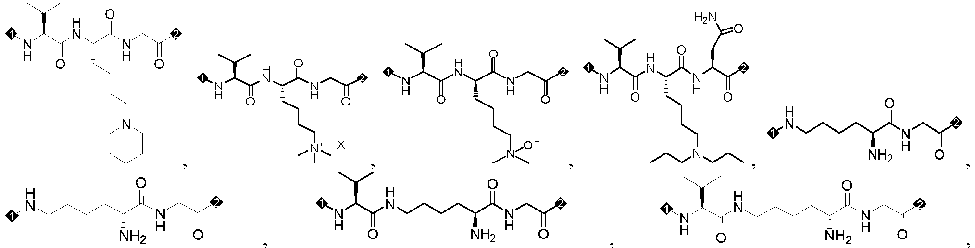 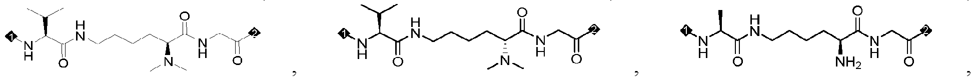 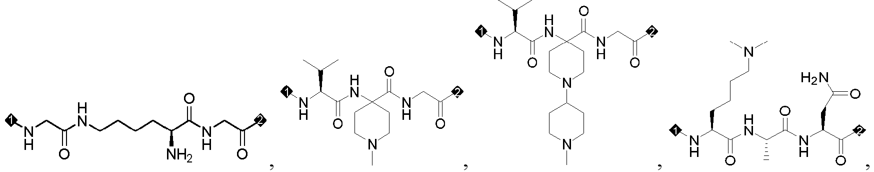 X ⁻ is selected from halide ion, carboxylate ion, sulfate ion, hydrogen sulfate ion and OH⁻, position 1 is attached to L₁ or L₂, position 2 is attached to L₄ or W.

In some embodiments, L₃ is selected from 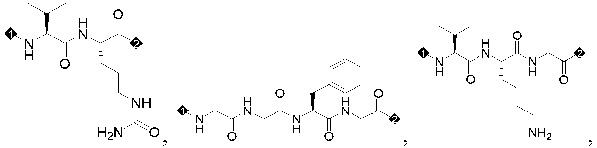 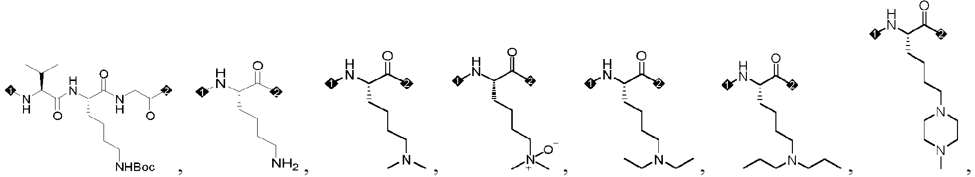 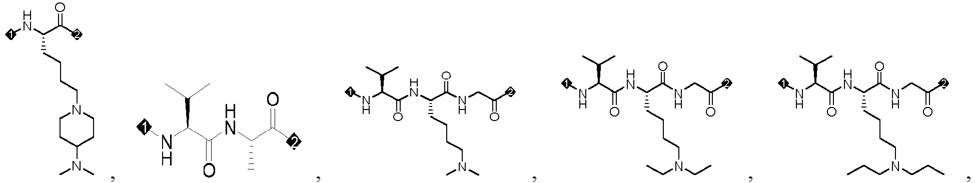 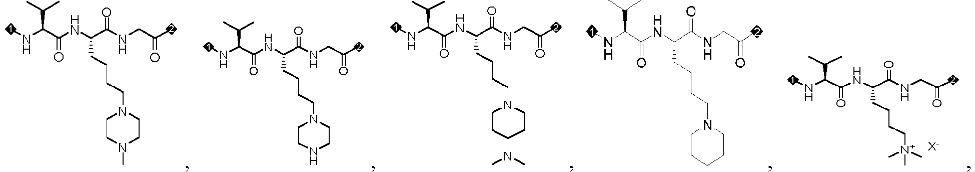 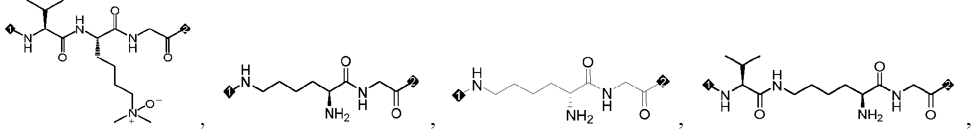 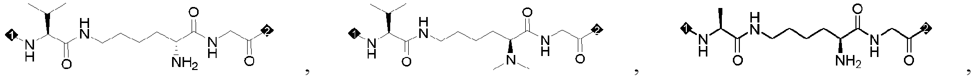 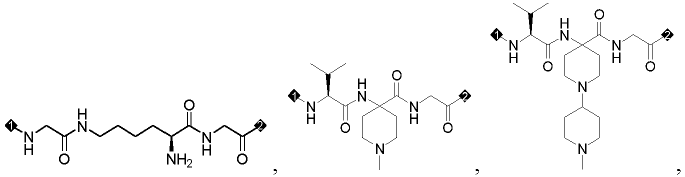 X ⁻ is selected from halide ion, carboxylate ion, sulfate ion, hydrogen sulfate ion, and OH⁻, position 1 is attached to L₁ or L₂, position 2 is attached to L₄ or W.

In some embodiments, L₃ is selected from 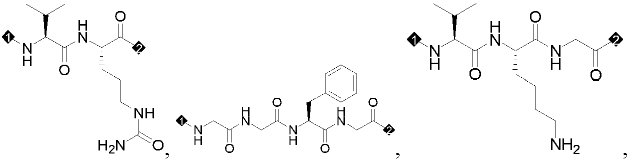 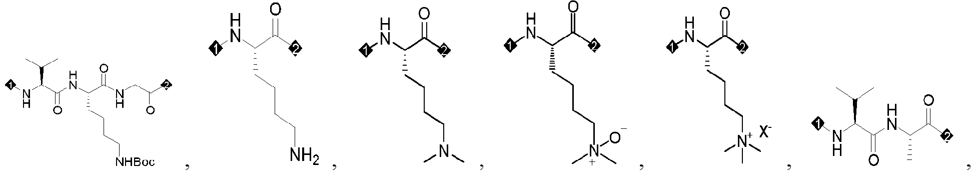 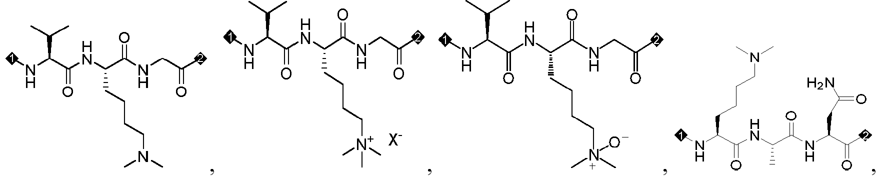 X ⁻ is selected from is halide ion, carboxylate ion, sulfate ion, hydrogen sulfate ion and OH⁻, position 1 is attached to L₁ or L₂, position 2 is attached to L₄ or W. In some embodiments, L₃ is selected from 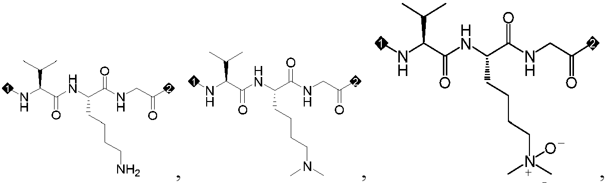 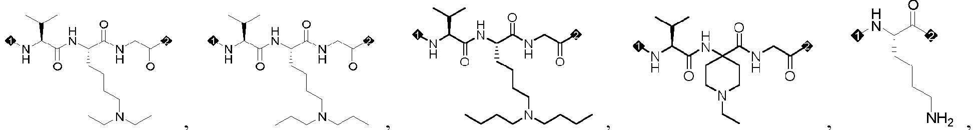 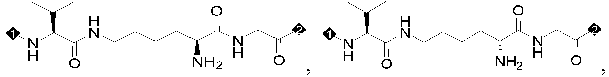 position 1 is attached to L₁ or L₂ and position 2 is attached to L₄ or W.

In some embodiments, L₃ is selected from 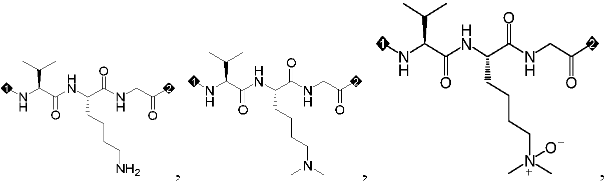 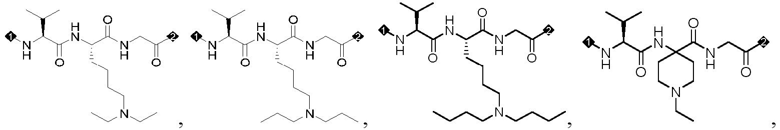 position 1 is attached to L₁ or L₂, position 2 is attached to L₄ or W.

In some embodiments, the structure of the amino acid residues represented by AA¹ is shown below, 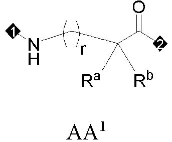 wherein:
R^{a} and R^{b} are each independently selected from H, 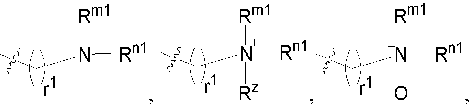 and R^{a} and R^{b} are not both H;
or, R^{a} and R^{b} together with the carbon atom to which they are both attached form a 4-10 membered heterocyclic ring, said 4-10 membered heterocyclic ring is optionally substituted with one or more R⁰;
r, r¹ are each independently selected from any integer from 0 to 20;
R^{m1}, Rⁿ¹ are each independently selected from H, C1-6 alkyl, C3-6 cycloalkyl and -COOR^{x1};
R^{x1} is selected from C1-6 alkyl;
or, R^{m1} and Rⁿ¹ together with the nitrogen atom to which they are both attached form a 4-10 membered heterocyclic ring, said 4-10 membered heterocyclic ring is optionally substituted with one or more R^{0'};
R^{z} is selected from C1-6 alkyl;
R⁰, R^{0'} are each independently selected from C1-6 alkyl, C3-6 cycloalkyl, -NR^{m2}Rⁿ² and 4-10 membered heterocyclyl optionally substituted with C1-6 alkyl;
R^{m2}, Rⁿ² are each independently selected from H and C1-6 alkyl;

In some embodiments, one of R^{a} and R^{b} is H, and the other is selected from

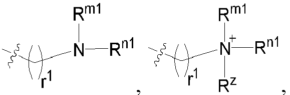

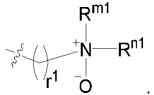

In some embodiments, one of R^{a} and R^{b} is H, and the other is selected from

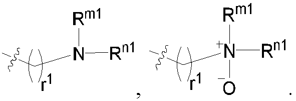

In some embodiments, R^{a} and R^{b} together with the carbon atom to which they are both attached form a 5-6 membered heterocyclic ring substituted with R⁰.

In some embodiments, R^{a} and R^{b} together with the carbon atom to which they are both attached form a piperidine ring or a piperazine ring substituted with R⁰.

In some embodiments, R^{a} and R^{b} together with the carbon atom to which they are both attached form a piperidine ring substituted by R⁰.

In some embodiments, R^{a} and R^{b} together with the carbon atom to which they are both attached form 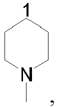 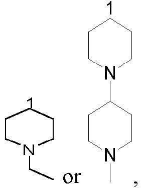 and the carbon atom marked with number 1 is the carbon atom to which R^{a} and R^{b} are both attached.

In some embodiments, R^{a} and R^{b} together with the carbon atom to which they are both attached form 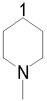 or 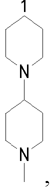 and the carbon atom marked with number 1 is the carbon atom to which R^{a} and R^{b} are both attached.

In some embodiments, r and r¹ are each independently selected from 0, 1, 2, 3, 4 and 5.

In some embodiments, r and r¹ are each independently selected from 0, 4.

In some embodiments, one of r and r¹ is 0, and the other is 4.

In some embodiments, R^{m1} and Rⁿ¹ are each independently selected from H, methyl, ethyl, n-propyl, n-butyl, - COOCH3, -COOCH2CH3, -COOCH2CH2CH3, -COOCH(CH3)2, -COOC(CH3)3 and -COOCH2CH2CH2CH3.

In some embodiments, R^{m1} and Rⁿ¹ are each independently selected from H, C1-6 alkyl, C3-6 cycloalkyl and t-butoxycarbonyl.

In some embodiments, R^{m1} and Rⁿ¹ are each independently selected from H, C1-6 alkyl.

In some embodiments, R^{m1} and Rⁿ¹ are each independently selected from H, methyl, ethyl and n-propyl.

In some embodiments, for r and r¹, when r is 4 and r¹ is 0, R^{m1} and Rⁿ¹ are each independently selected from H and C1-6 alkyl (such as H, methyl); when r is 0 and r¹ is 4, R^{m1} and Rⁿ¹ are each independently selected from C1-6 alkyl (such as methyl, ethyl, n-propyl), preferably C2-6 alkyl (such as ethyl, n-propyl).

In some embodiments, R^{m1} and Rⁿ¹ together with the nitrogen atom to which they are both attached form a 5-6 membered heterocyclic ring substituted with R^{0'}.

In some embodiments, R^{m1} and Rⁿ¹ together with the nitrogen atom to which they are both attached form a piperidine or piperazine ring substituted with R^{0'}.

In some embodiments, R^{m1} and Rⁿ¹ together with the nitrogen atom to which they are both attached form 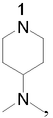 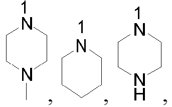 nitrogen atom marked with number 1 is the nitrogen atom to which R^{m1} and Rⁿ¹ are both attached.

In some embodiments, R^{z} is methyl.

In some embodiments, R⁰ and R^{0'} are each independently selected from C1-6 alkyl, -NR^{m2}Rⁿ² and 5-6 membered heterocyclyl optionally substituted with C1-6 alkyl.

In some embodiments, R⁰ is selected from C1-6 alkyl and 5-6 membered heterocyclyl substituted with C1-6 alkyl, said 5-6 membered heterocyclyl is selected from piperidinyl and piperazinyl.

In some embodiments, R⁰ is selected from methyl, ethyl and 5-6 membered heterocyclyl substituted with methyl, said 5-6 membered heterocyclyl is piperidinyl.

In some embodiments, R⁰ is selected from methyl and 5-6 membered heterocyclyl substituted with methyl, said 5-6 membered heterocyclyl is piperidinyl.

In some embodiments, R⁰ is selected from methyl, ethyl and

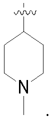

In some embodiments, R⁰ is selected from methyl and

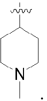

In some embodiments, R^{0'} is selected from C1-6 alkyl and -NR^{m2}Rⁿ².

In some embodiments, R^{0'} is selected from methyl and -NR^{m2}Rⁿ².

In some embodiments, R^{m2} and Rⁿ² are methyl.

In some embodiments, the amino acid residue represented by AA¹ is selected from

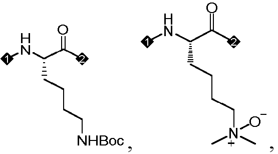

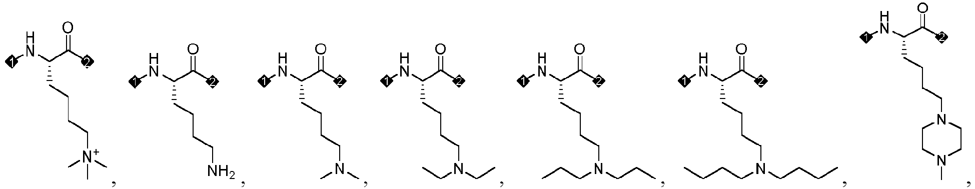

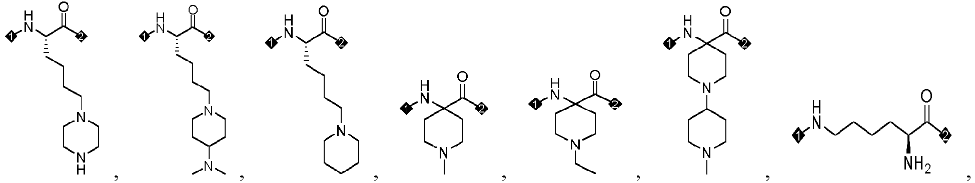

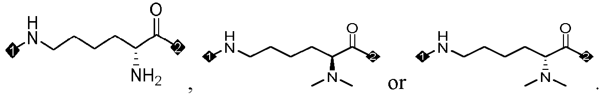

In some embodiments, the amino acid residue represented by AA¹ is selected from

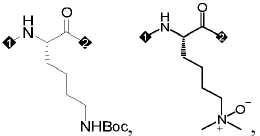

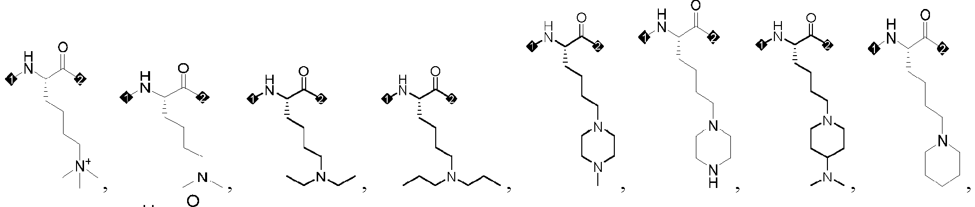

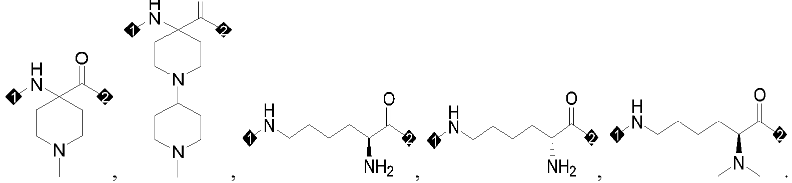

In some embodiments, the amino acid residue represented by AA¹ is selected from

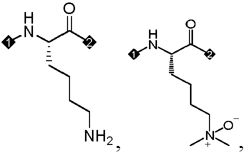

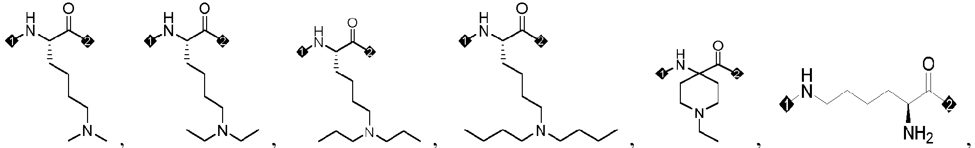

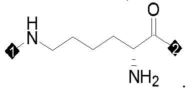

In some embodiments, the amino acid residue represented by AA¹ is selected from

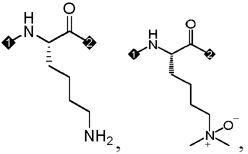

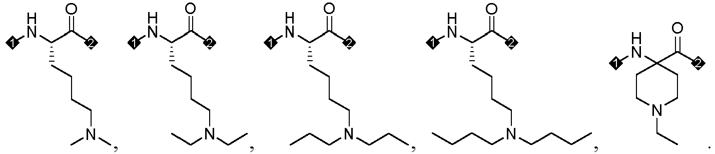

In some embodiments, L₄ is absent or present, and when L₄ is present, L₄ is selected from 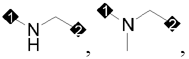 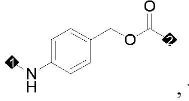 wherein position 1 is attached to L₃ and position 2 is attched to W or X.

In some embodiments, L₄ is absent or present, and when L₄ is present, L₄ is 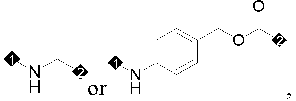 position 1 is attached to L₃ and position 2 is attached to W or X.

In some embodiments, L₄ is absent.

In some embodiments, L₄ is selected from 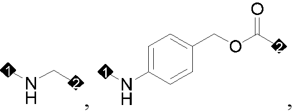 position 1 is attached to L₃ and position 2 is attached to W or X.

In some embodiments, L₄ is selected from 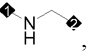 position 1 is attached to L₃ and position 2 is attached to W or X.

In some embodiments, Lg is selected from F, Cl and MeSO₂-.

In some embodiments, Lg is selected from F and MeSO₂-.

In some embodiments, the structure of 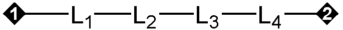 is selected from:

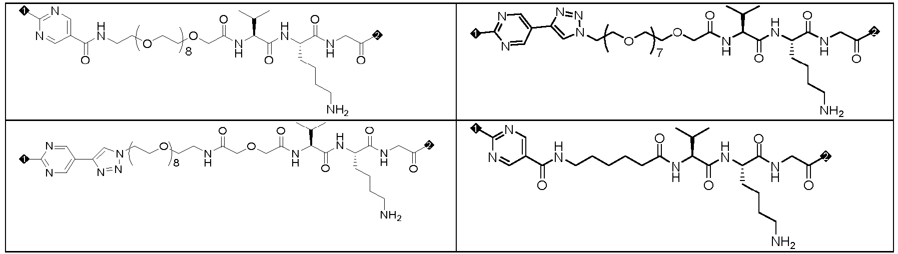

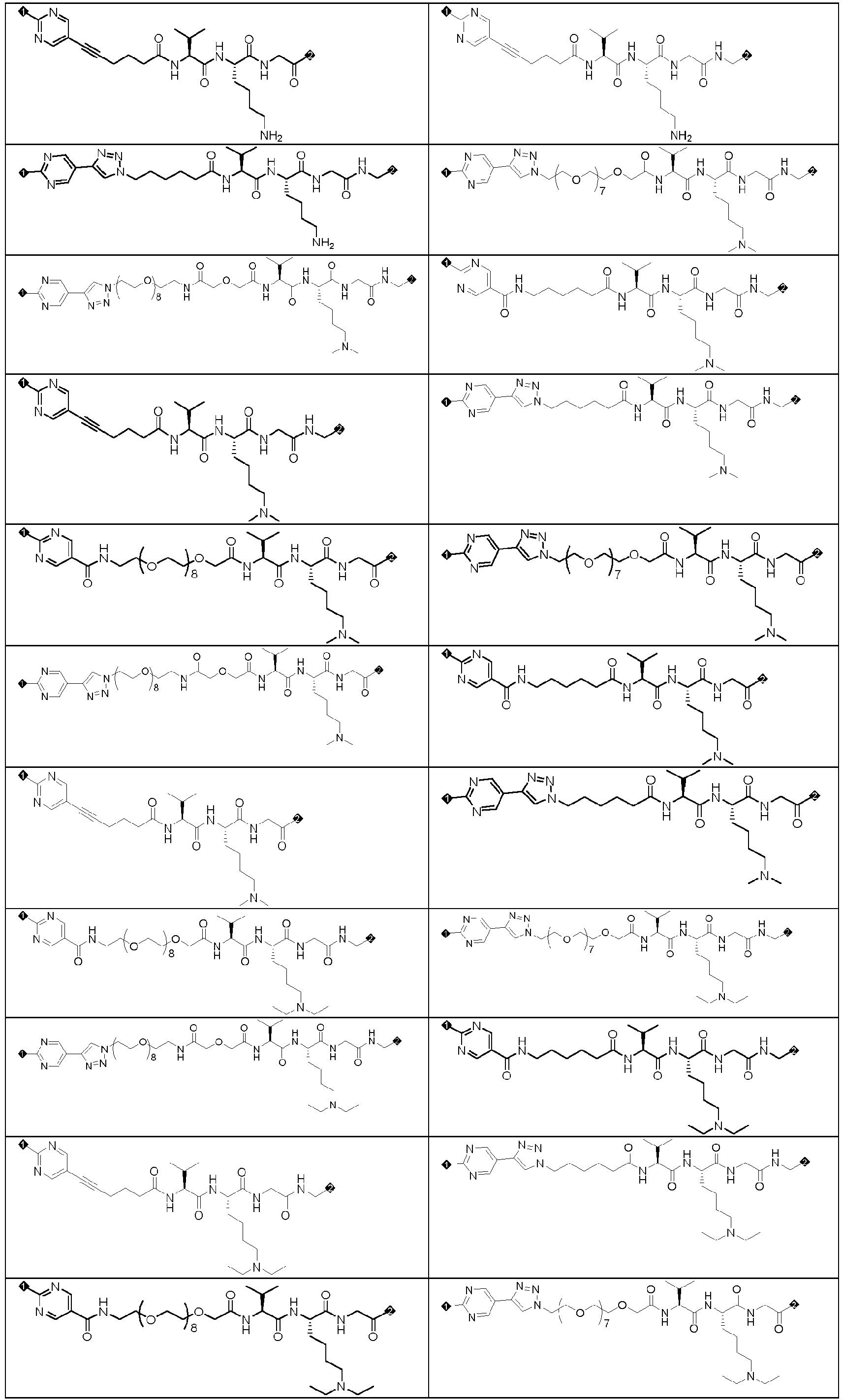

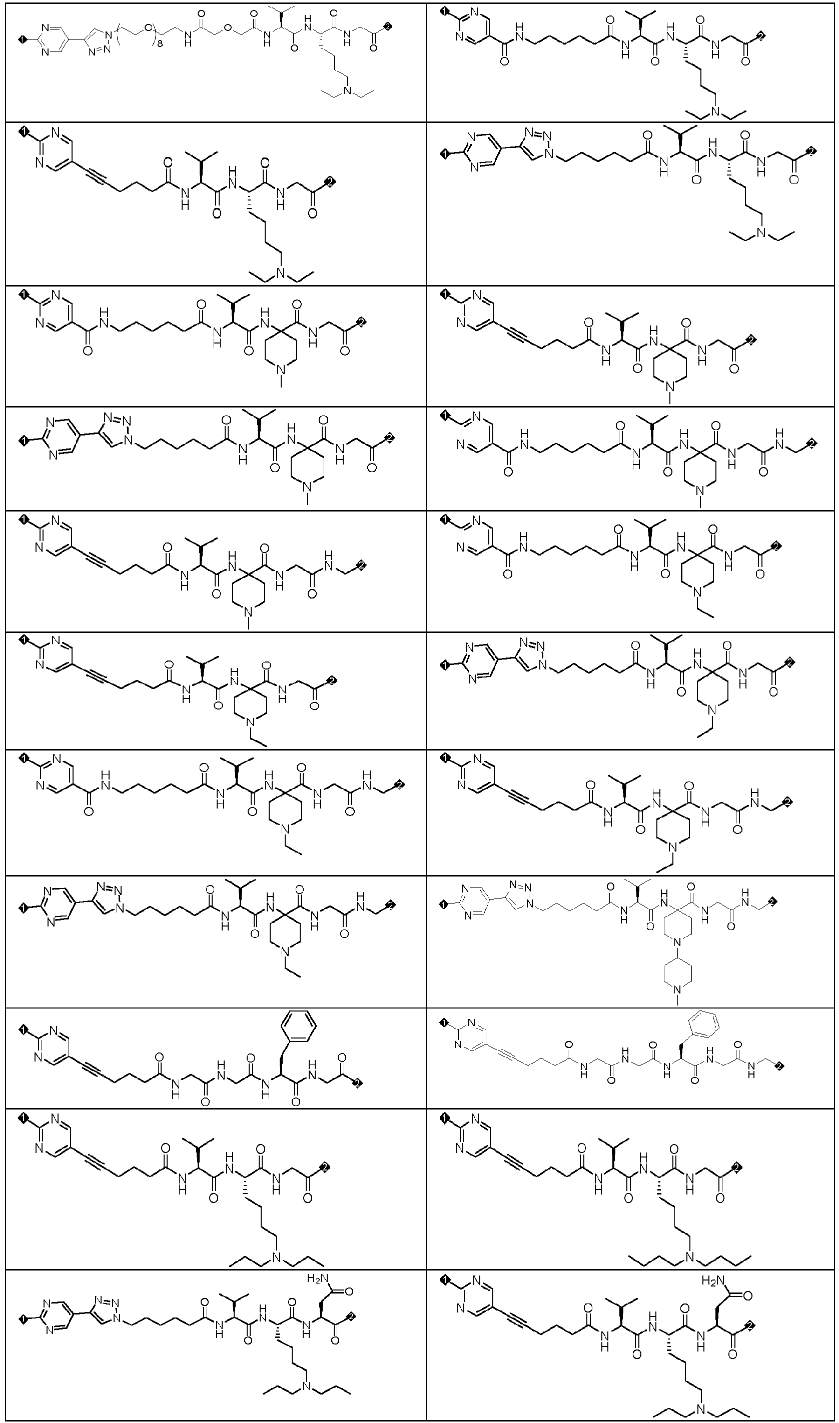

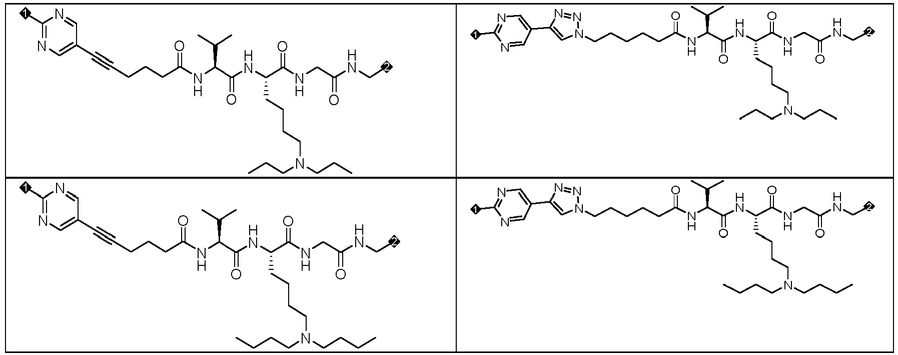

wherein, position 1 is attached to Lg and position 2 is attached to W.

In some embodiments, the structure 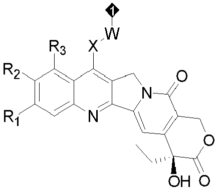 is selected from: 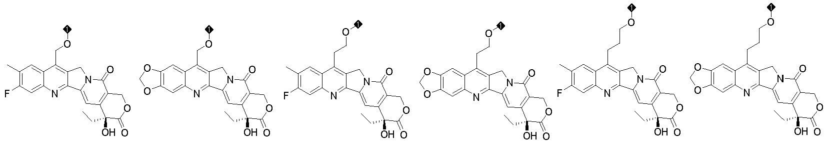 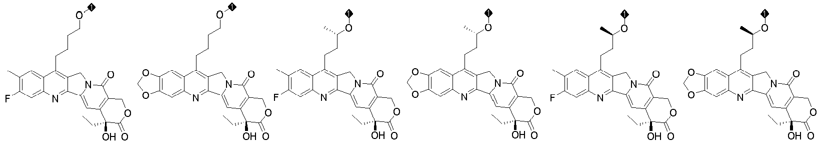 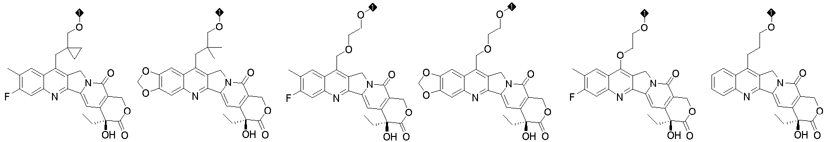 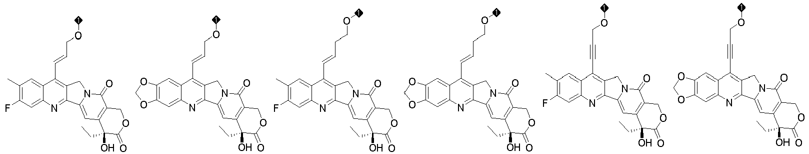 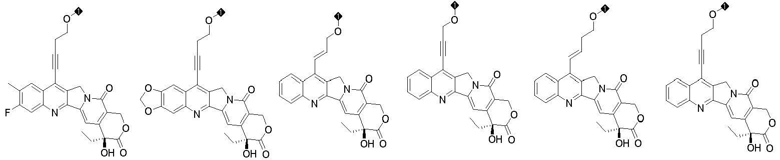 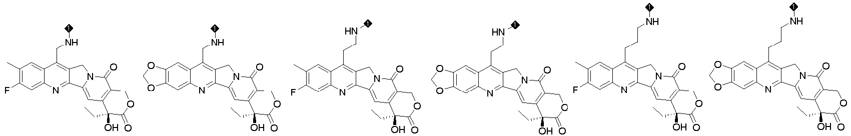 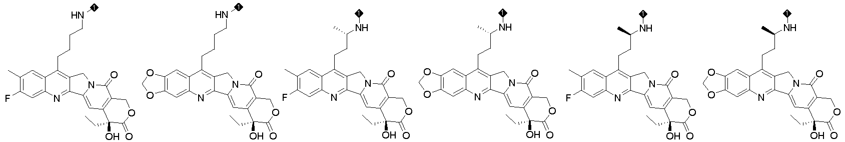 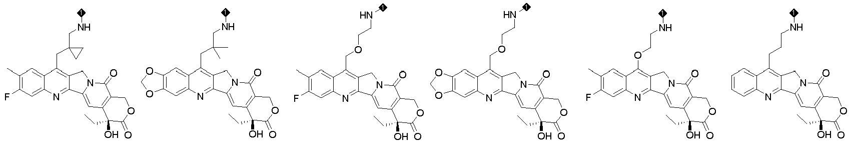 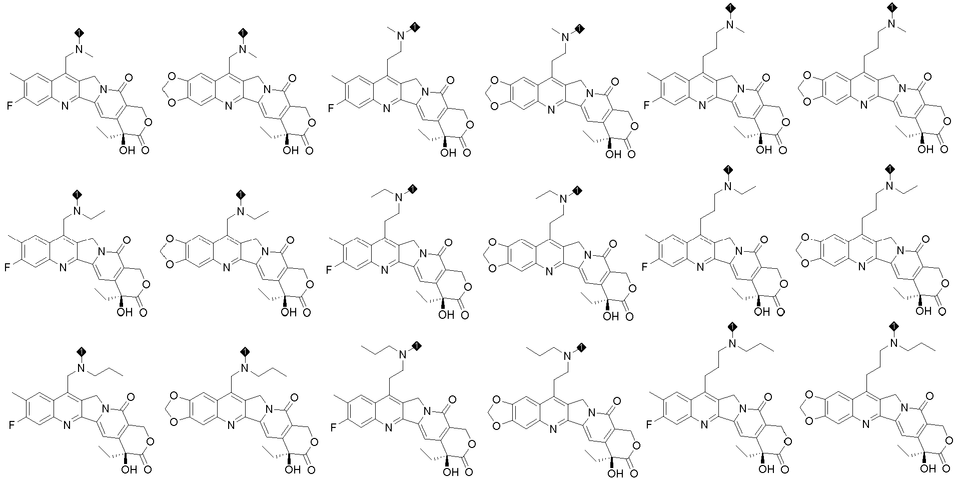 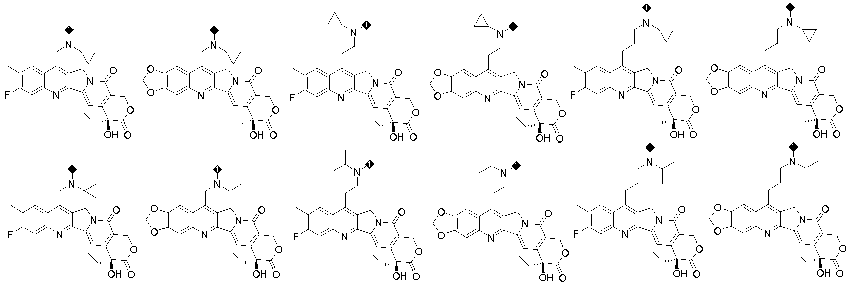 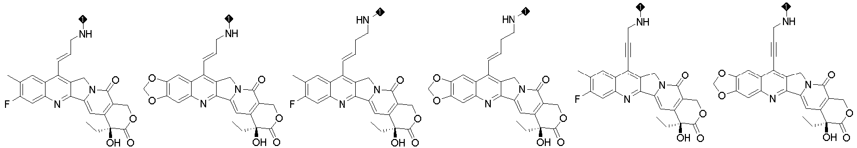 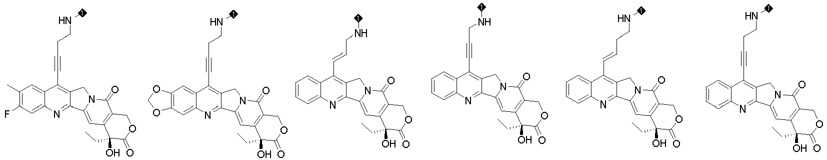 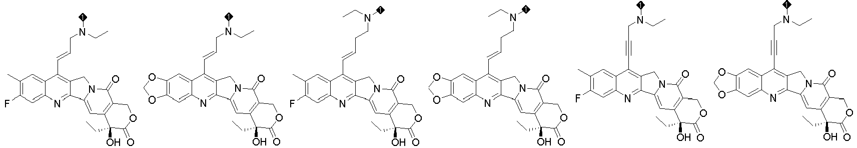 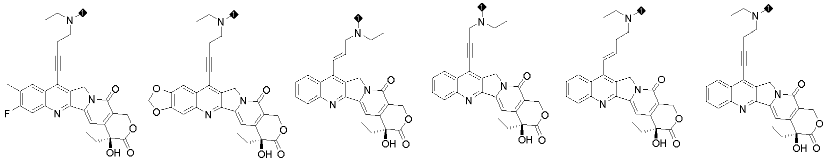 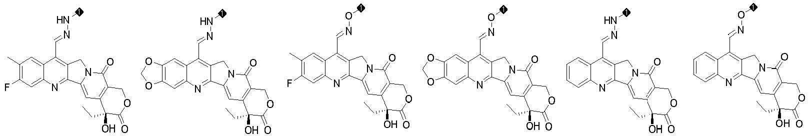 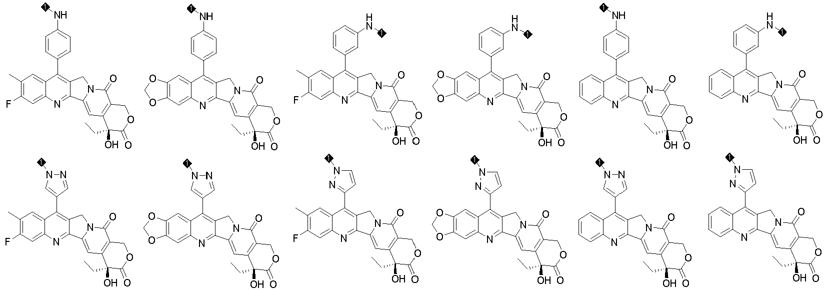 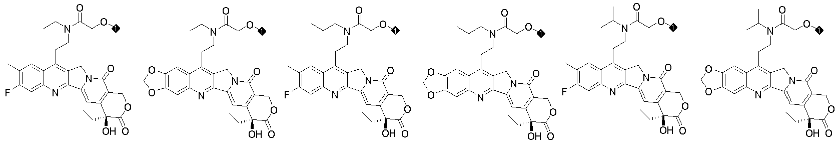 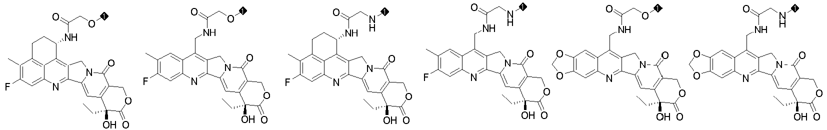 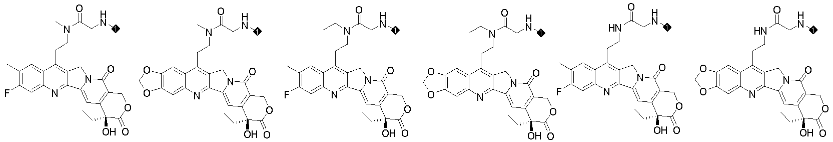 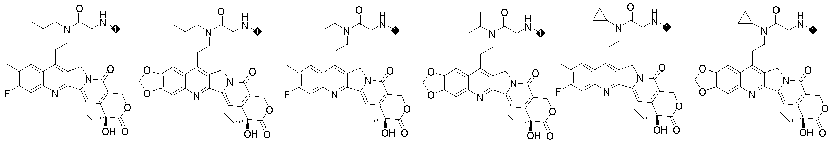 wherein, position 1 is attached to L₄; when L₄ is absent, position 1 is attached to L₃.

In some embodiments, the drug linker conjugate has a structure of formula III-(1): 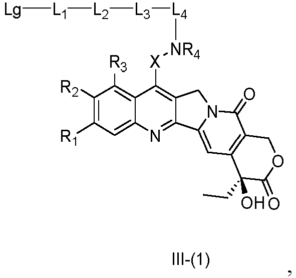 wherein L₁, L₂, L₃, L₄, X, R₁, R₂, R₃, R₄, and Lg are as defined in any embodiment specifically described above and herein.

In some embodiments, the drug linker conjugate has a structure of formula III-(1A) or III-(1B): 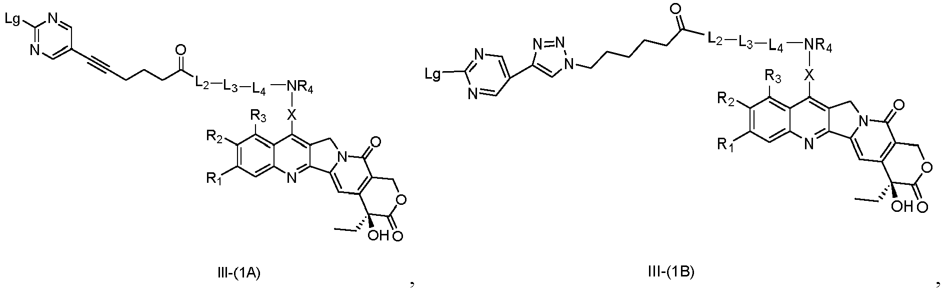 wherein L₂, L₃, L₄, X, R₁, R₂, R₃, R₄, and Lg are as defined in any embodiment specifically described above and herein.

In some embodiments, the drug linker conjugate has a structure of formula III-(2): 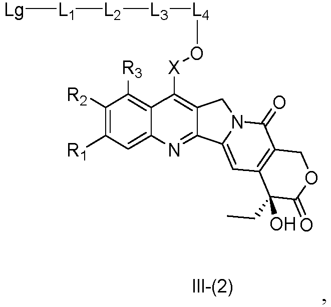 wherein L₁, L₂, L₃, L₄, X, R₁, R₂, R₃ and Lg are as defined in any embodiment specifically described above and herein.

In some embodiments, the drug linker conjugate has a structure of formula III-(2A) or III-(2B): 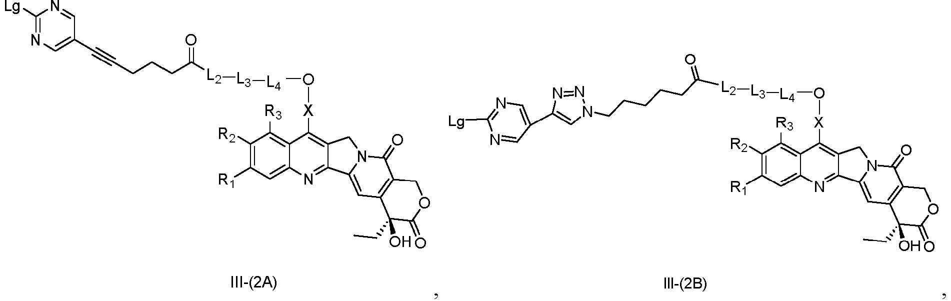 wherein L₂, L₃, L₄, X, R₁, R₂, R₃ and Lg are as defined in any embodiment specifically described above and herein.

In some embodiments, the drug linker conjugate has a structure of formula III-(3): 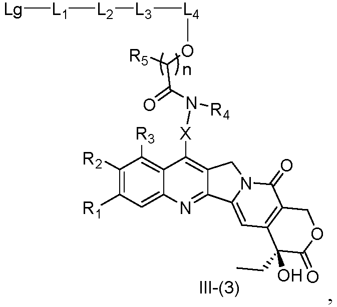 wherein L₁, L₂, L₃, L₄, X, R₁, R₂, R₃, R₄, R₅, n and Lg are as defined in any embodiment specifically described above and herein.

In some embodiments, the drug linker conjugate has a structure of formula III-(3A) or III-(3B): 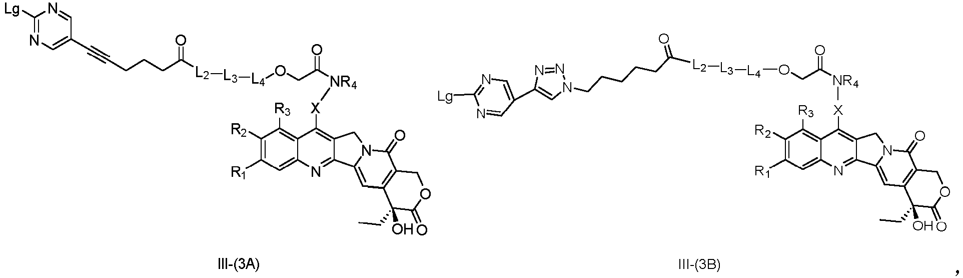 wherein Lg, L₂, L₃, L₄, X, R₁, R₂, R₃, R₄ and Lg are as defined in any embodiment specifically described above and herein.

In some embodiments, the drug linker conjugate has a structure of formula III-A: 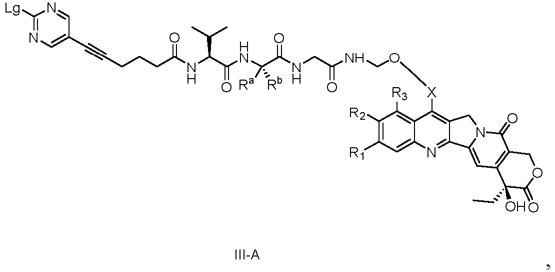 wherein Lg, X, R₁, R₂, R₃ R^{a}, R^{b} and q are as defined in any embodiment specifically described above and herein.

In some embodiments, the drug linker conjugate has a structure of formula III-B: 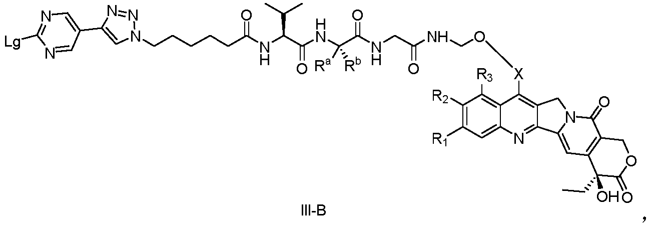 wherein Lg, X, R₁, R₂, R₃, R^{a}, R^{b} and q are as defined in any embodiment specifically described above and herein.

In some embodiments, the drug linker conjugate represented by formula III is selected from the following structures:

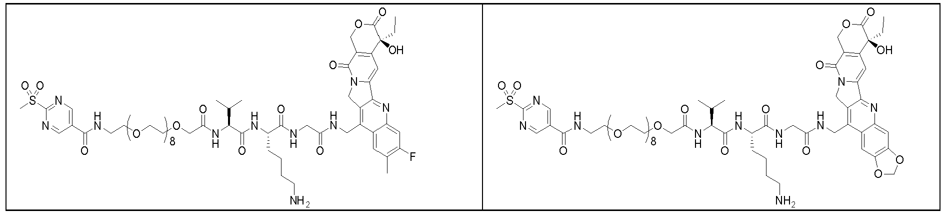

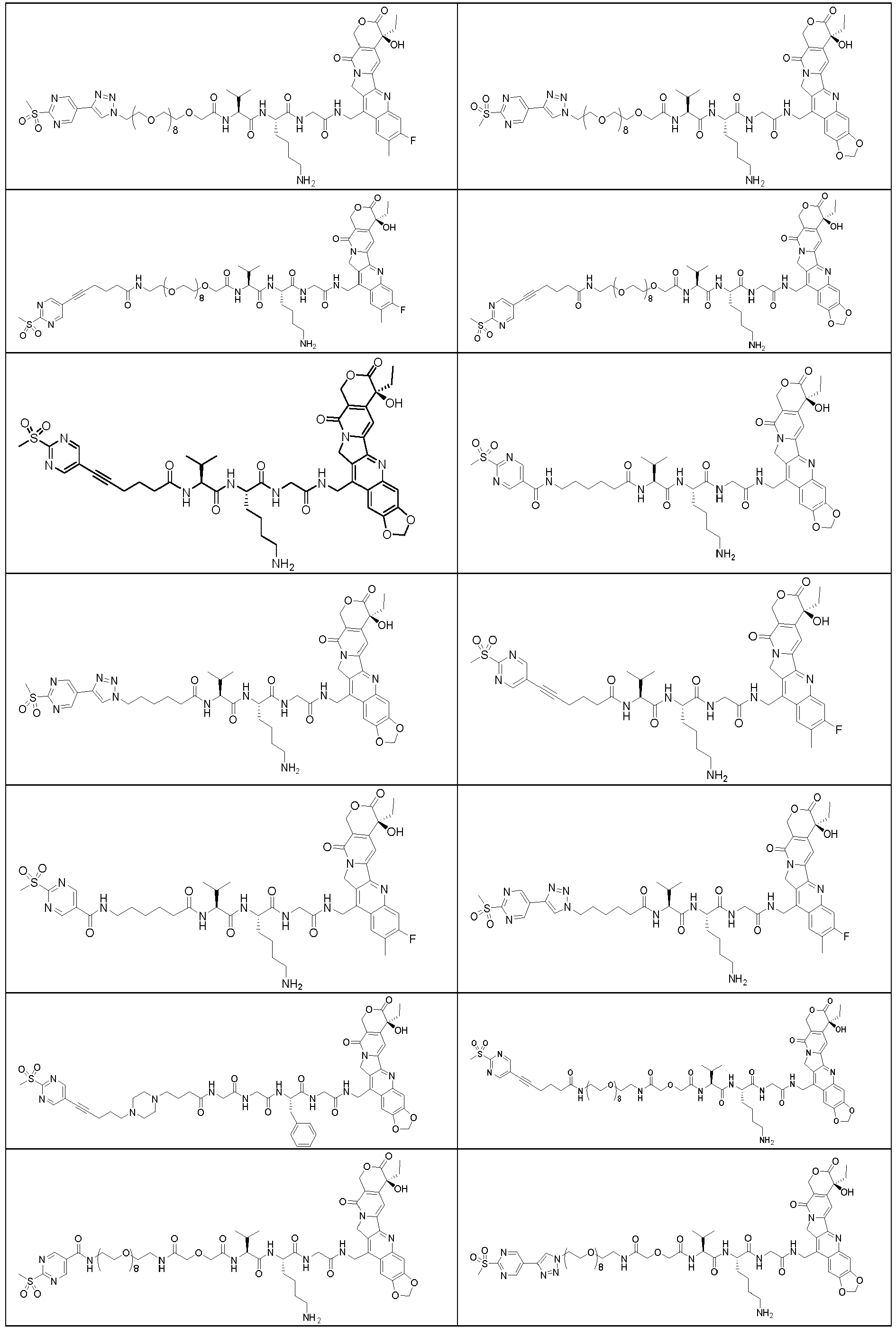

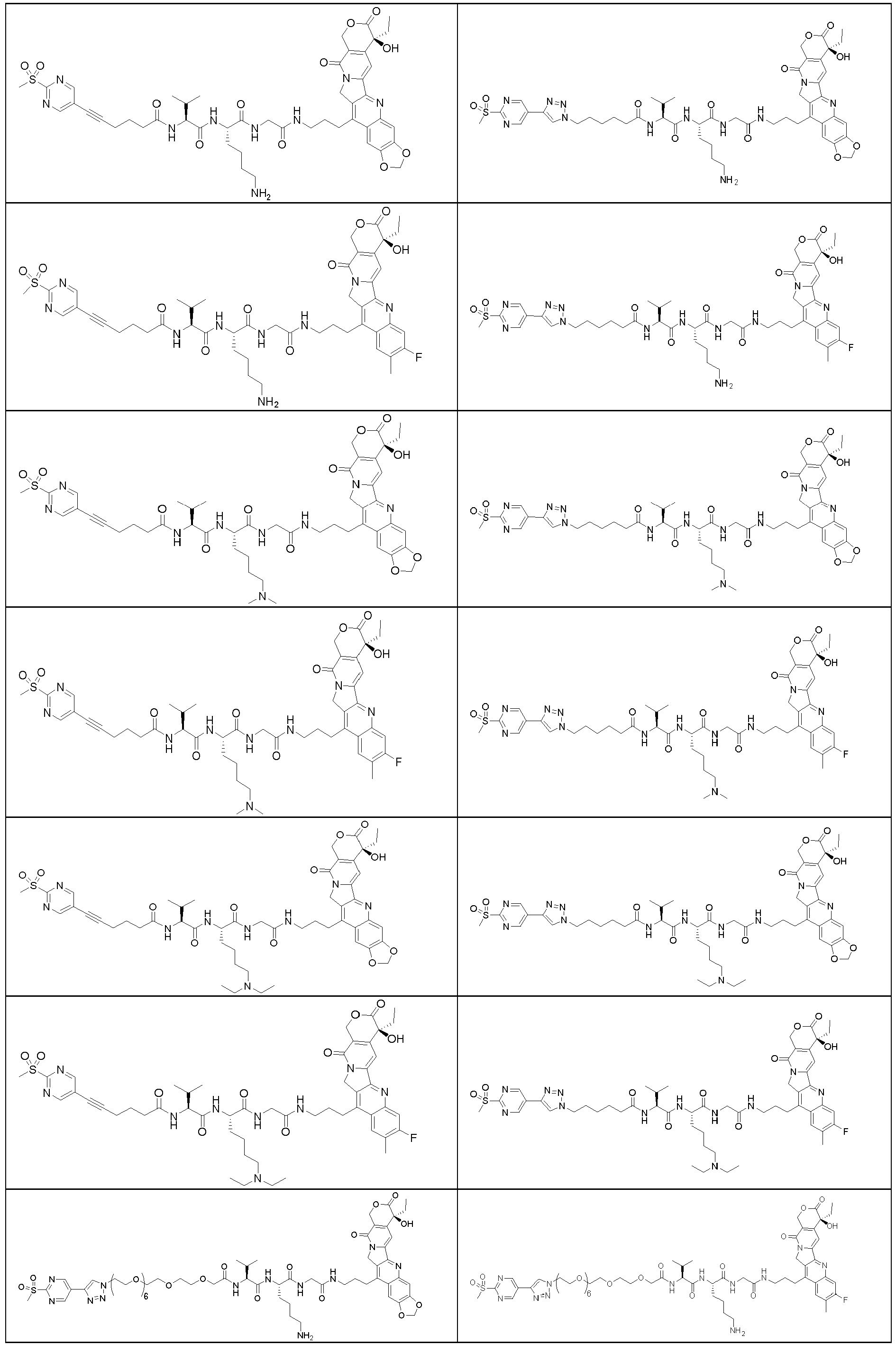

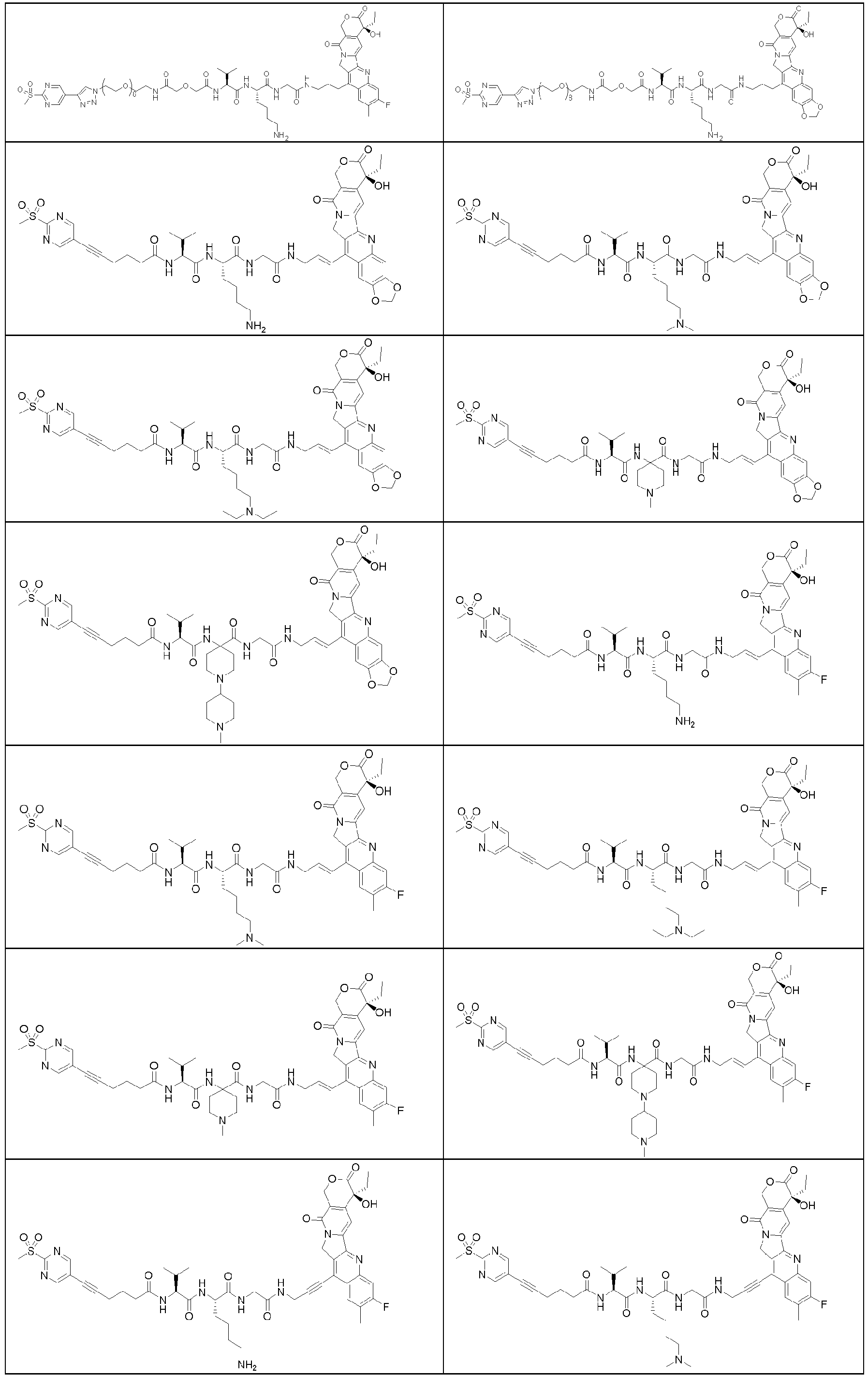

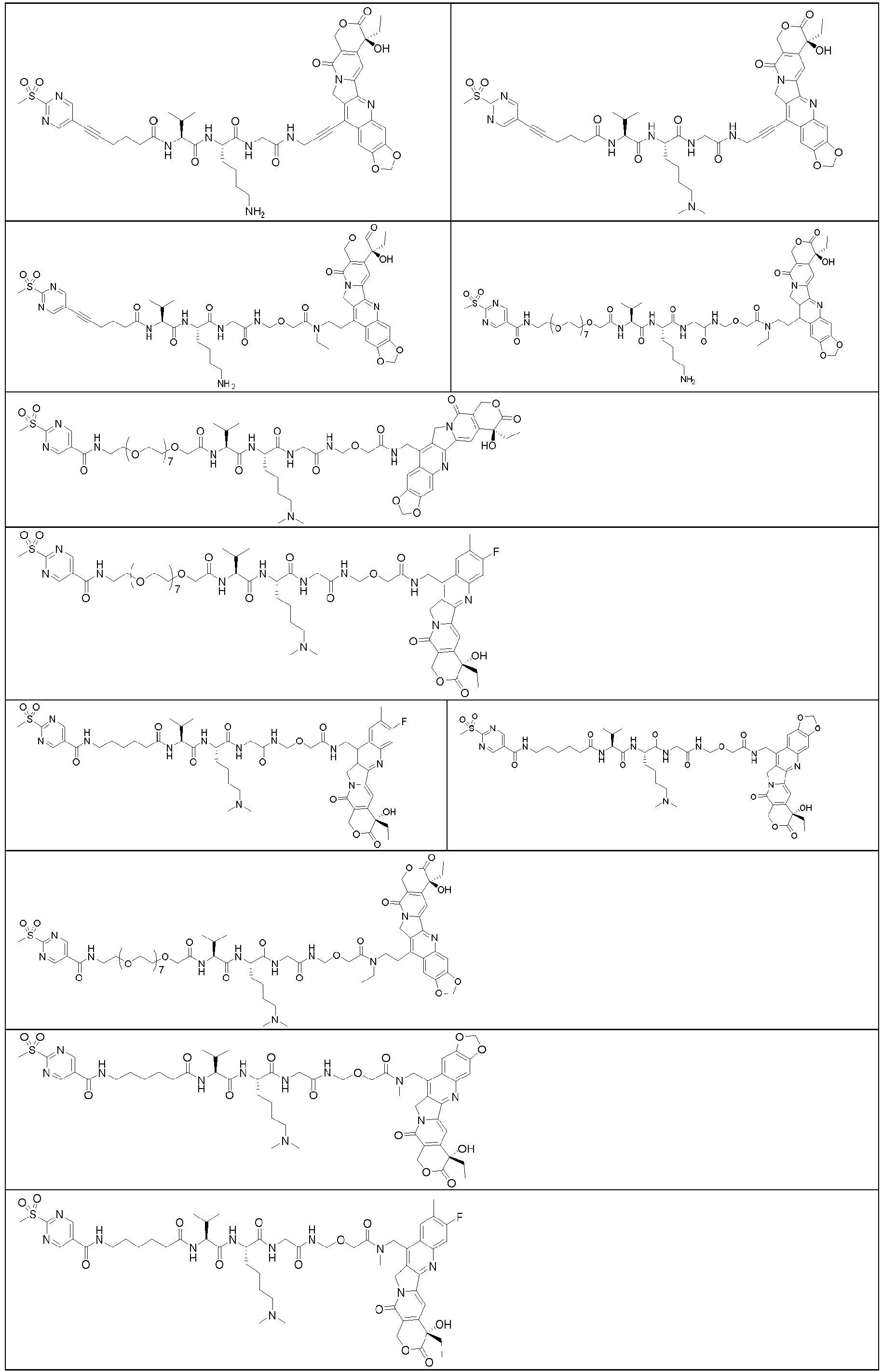

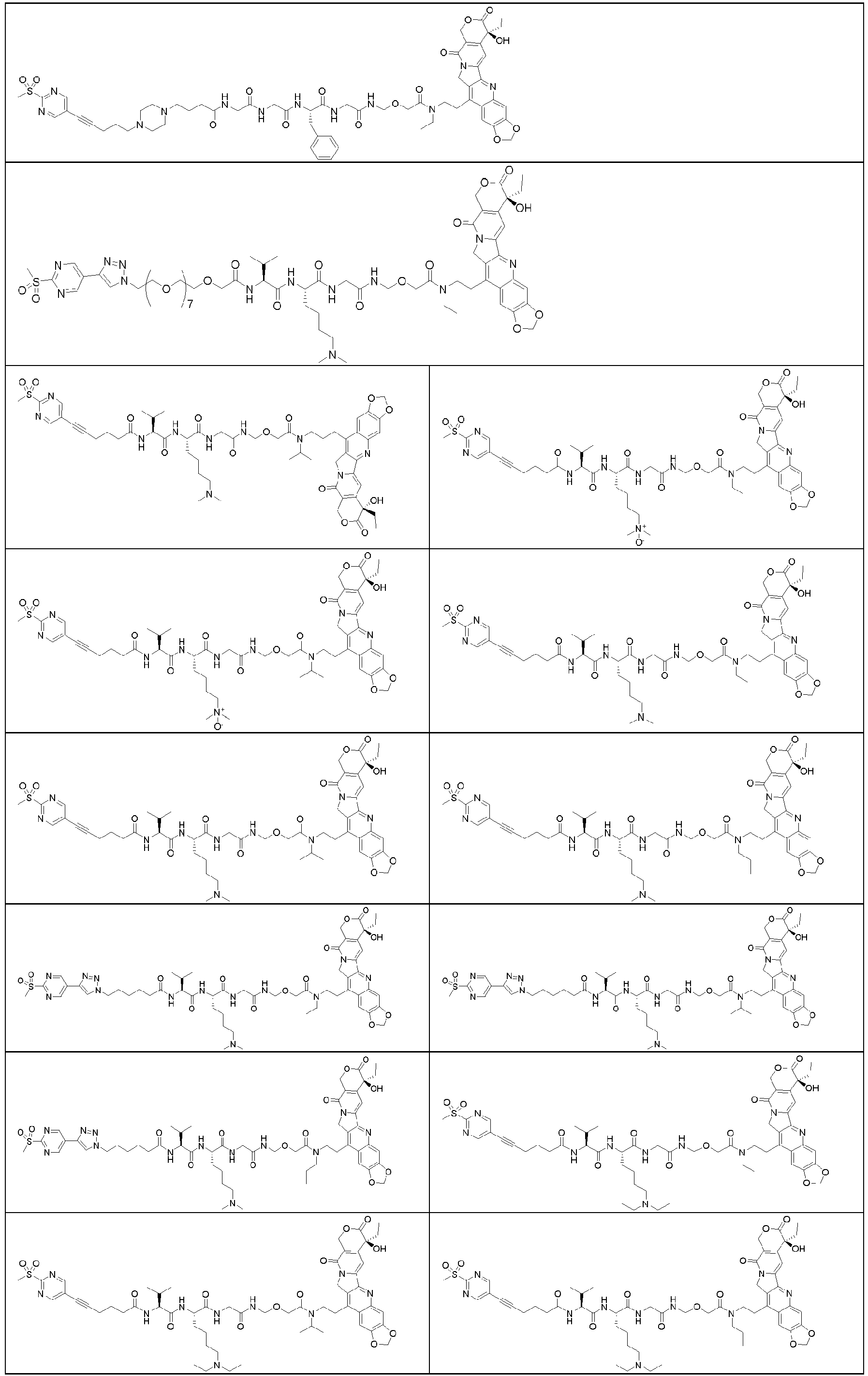

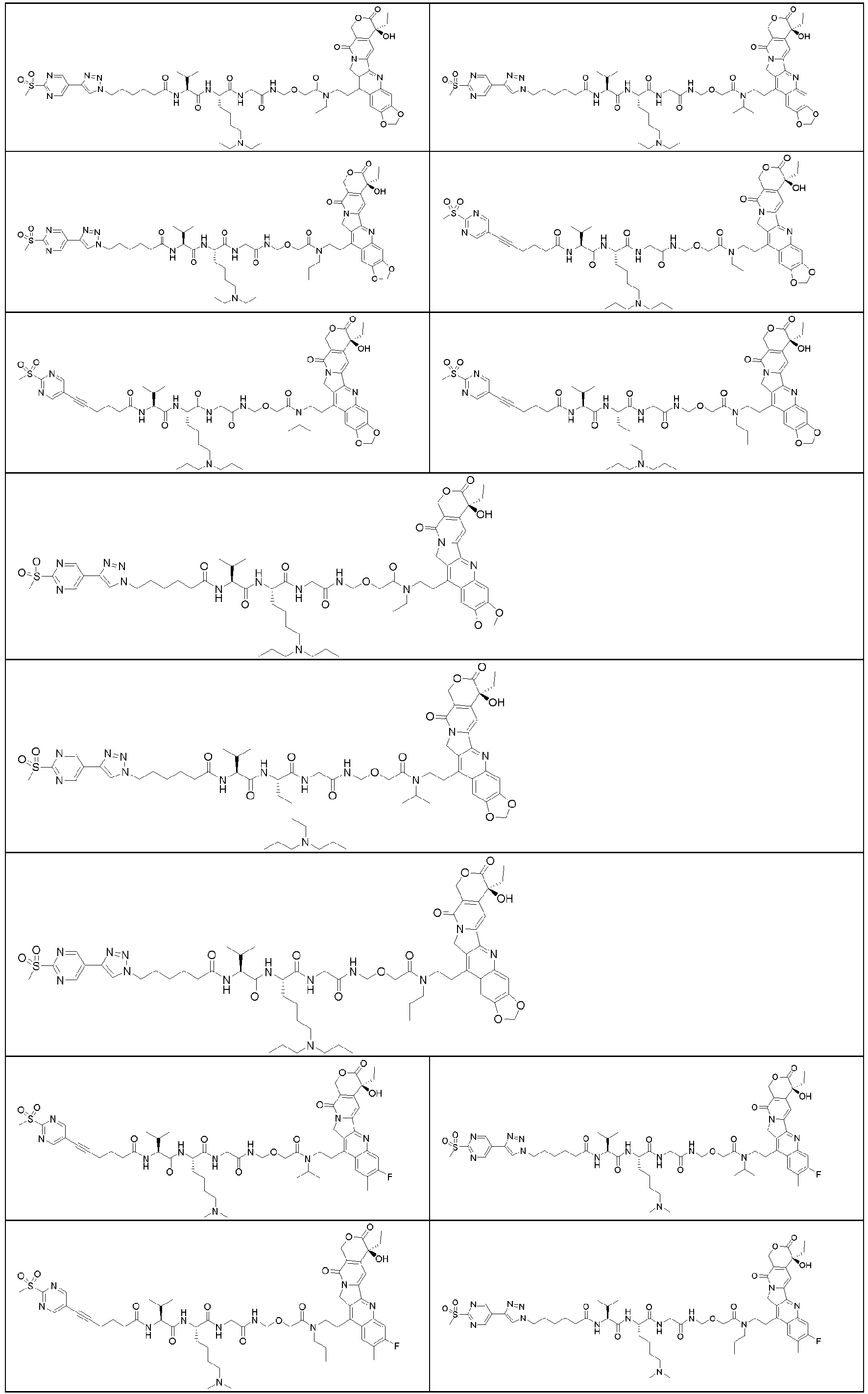

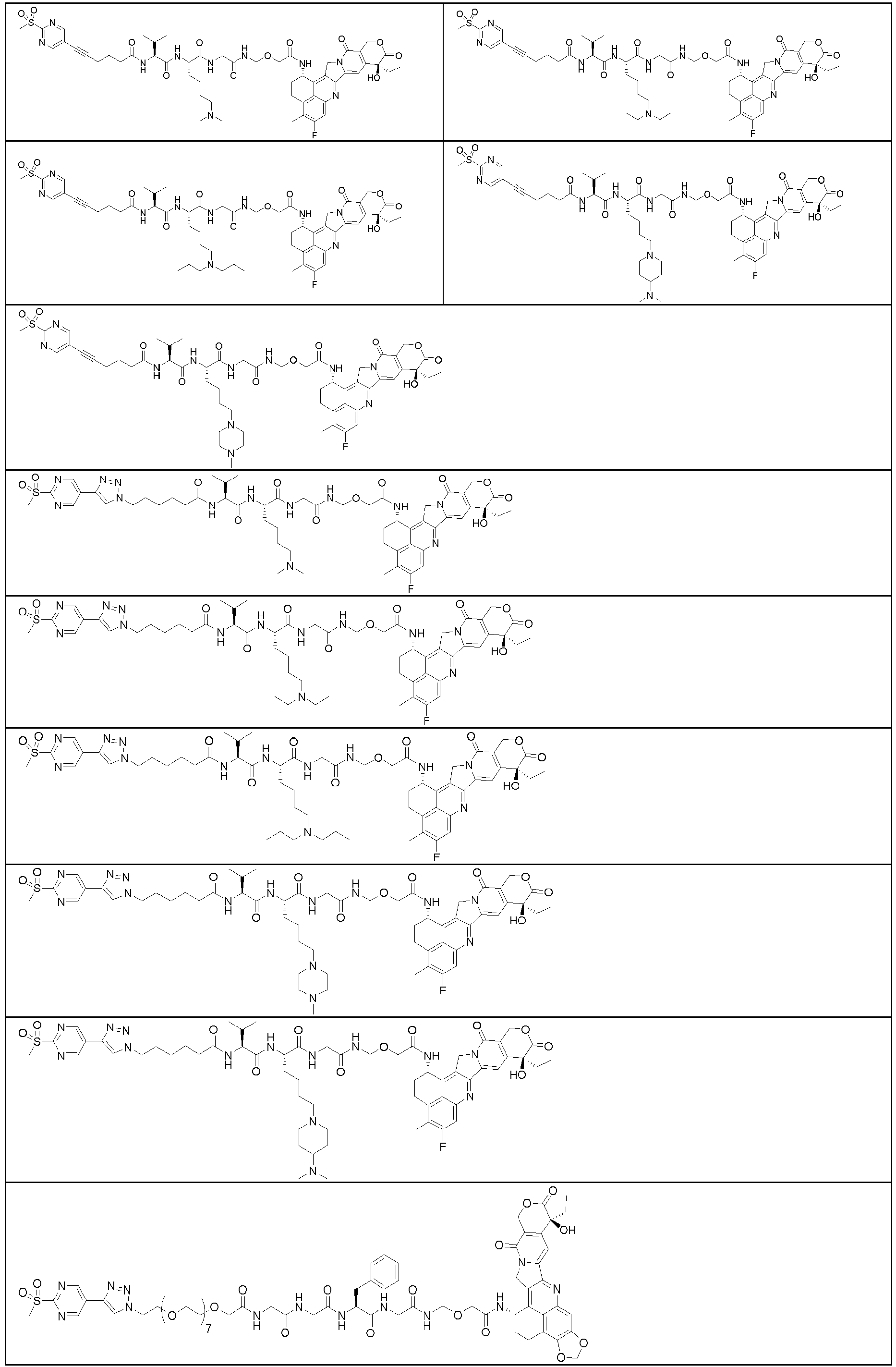

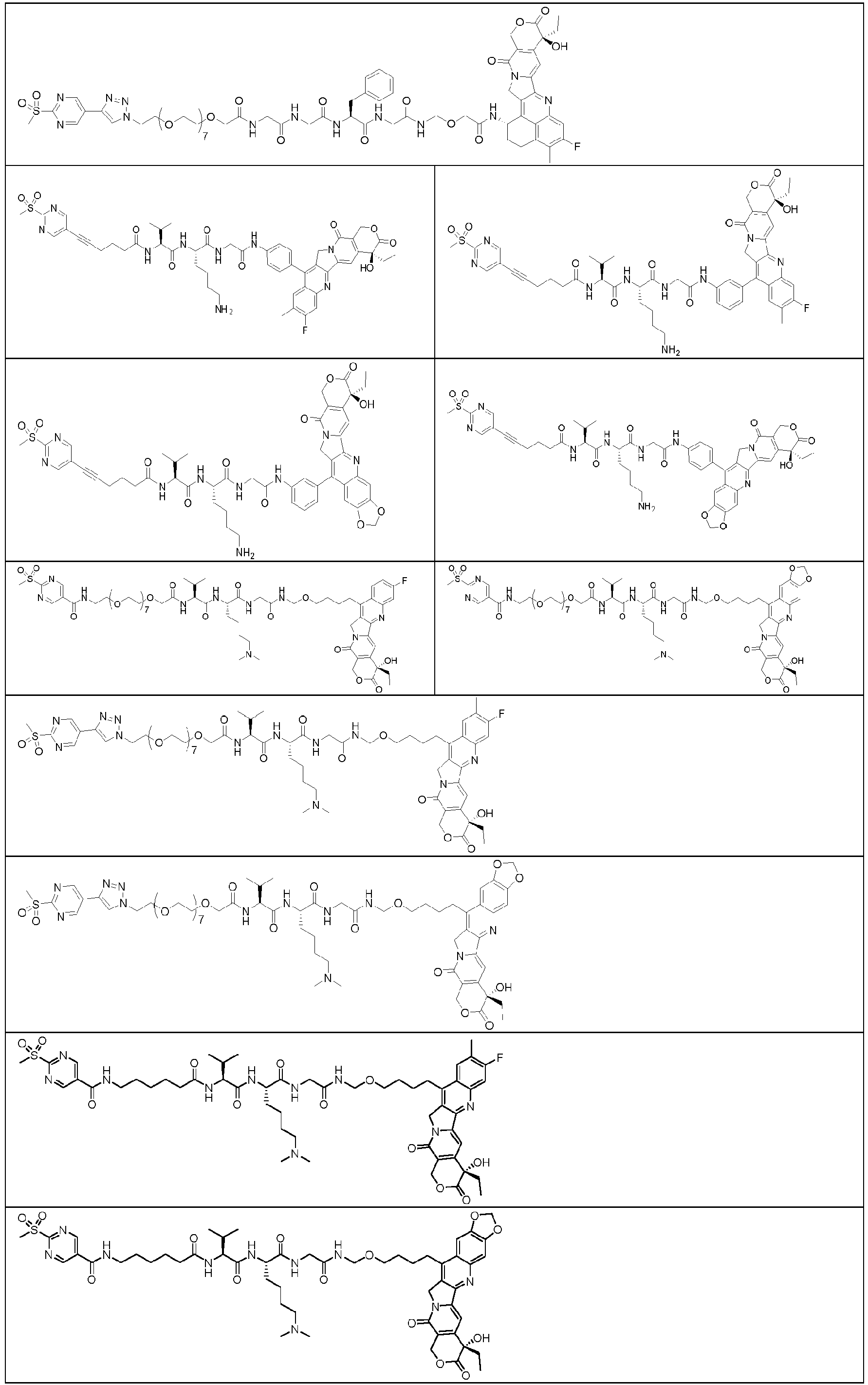

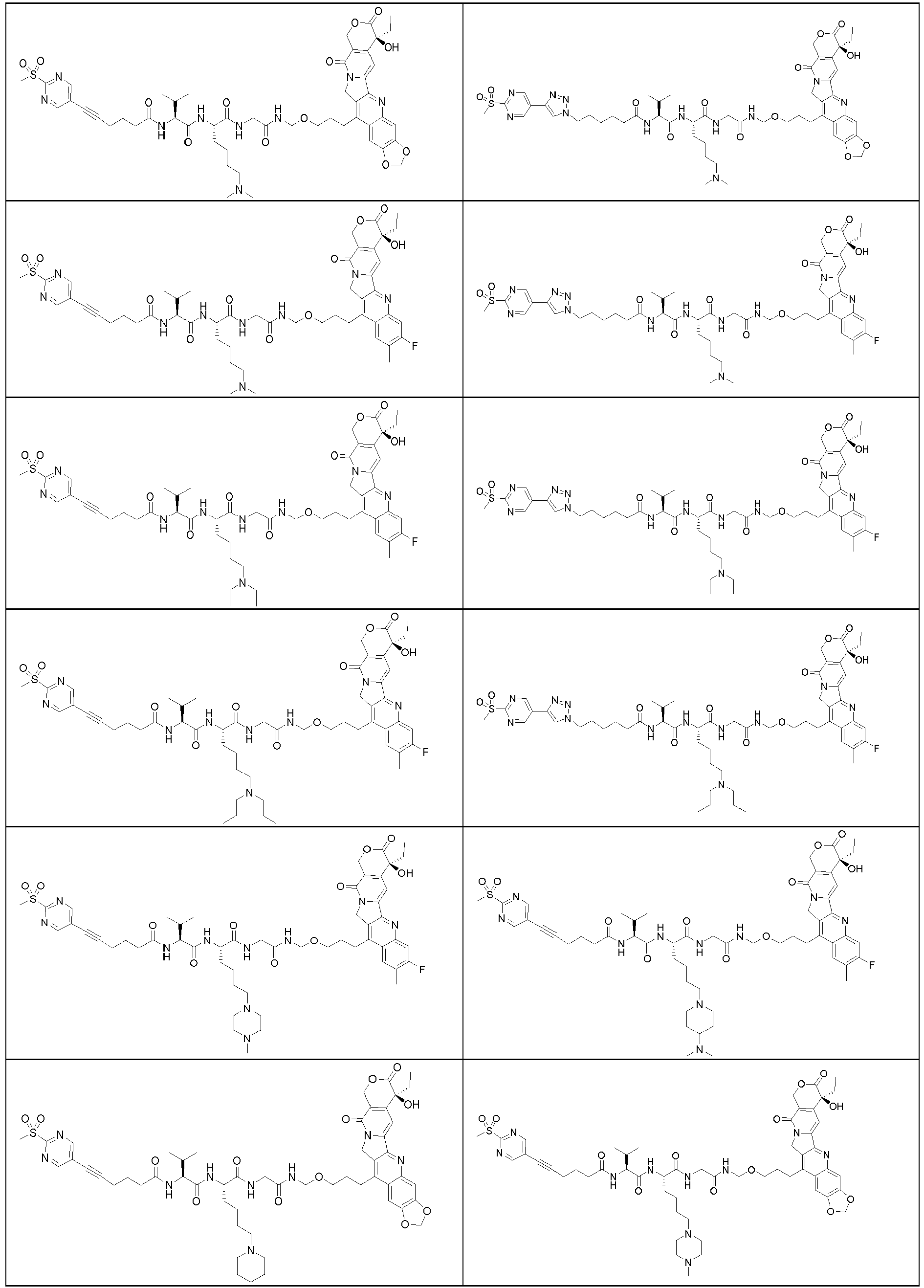

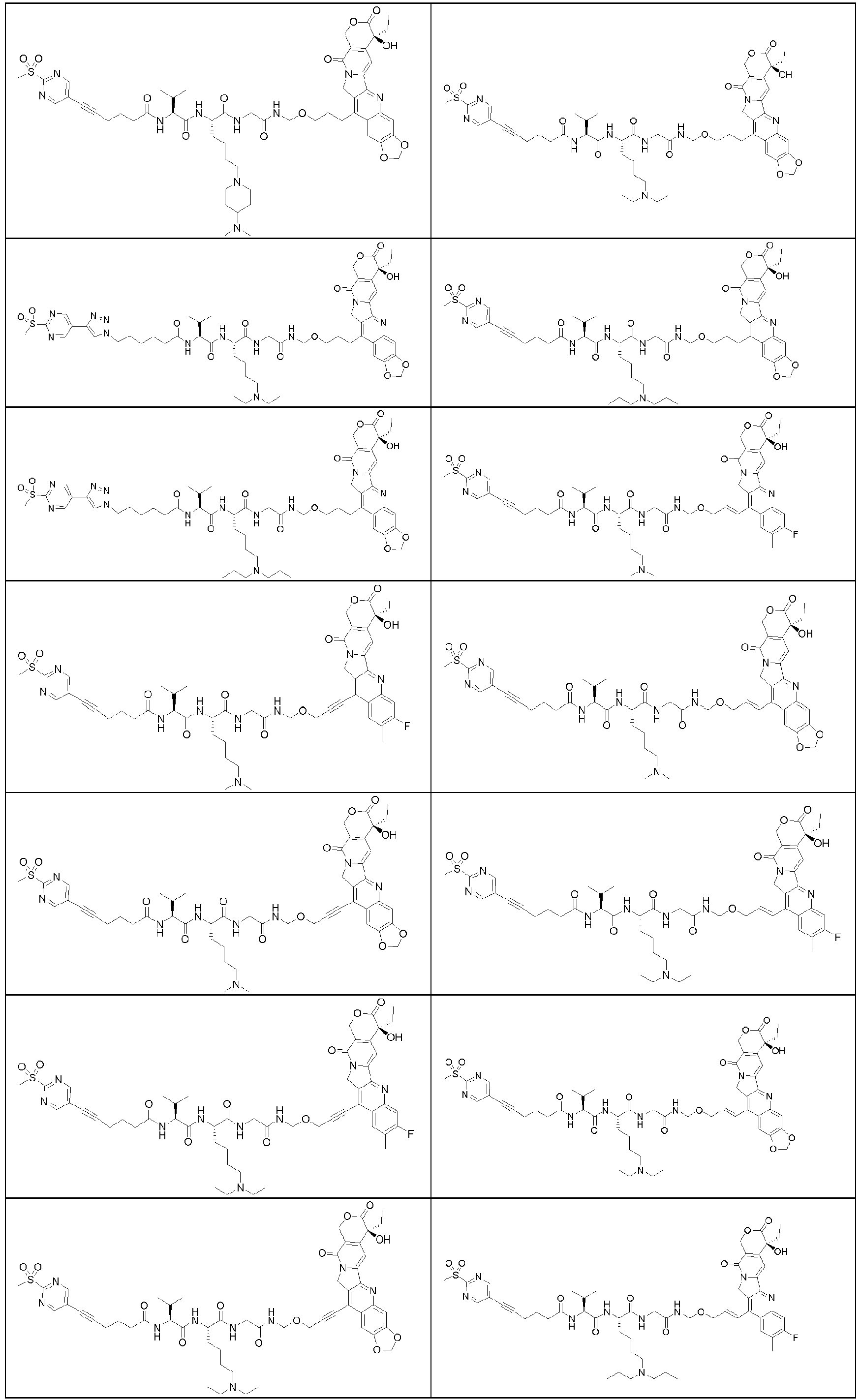

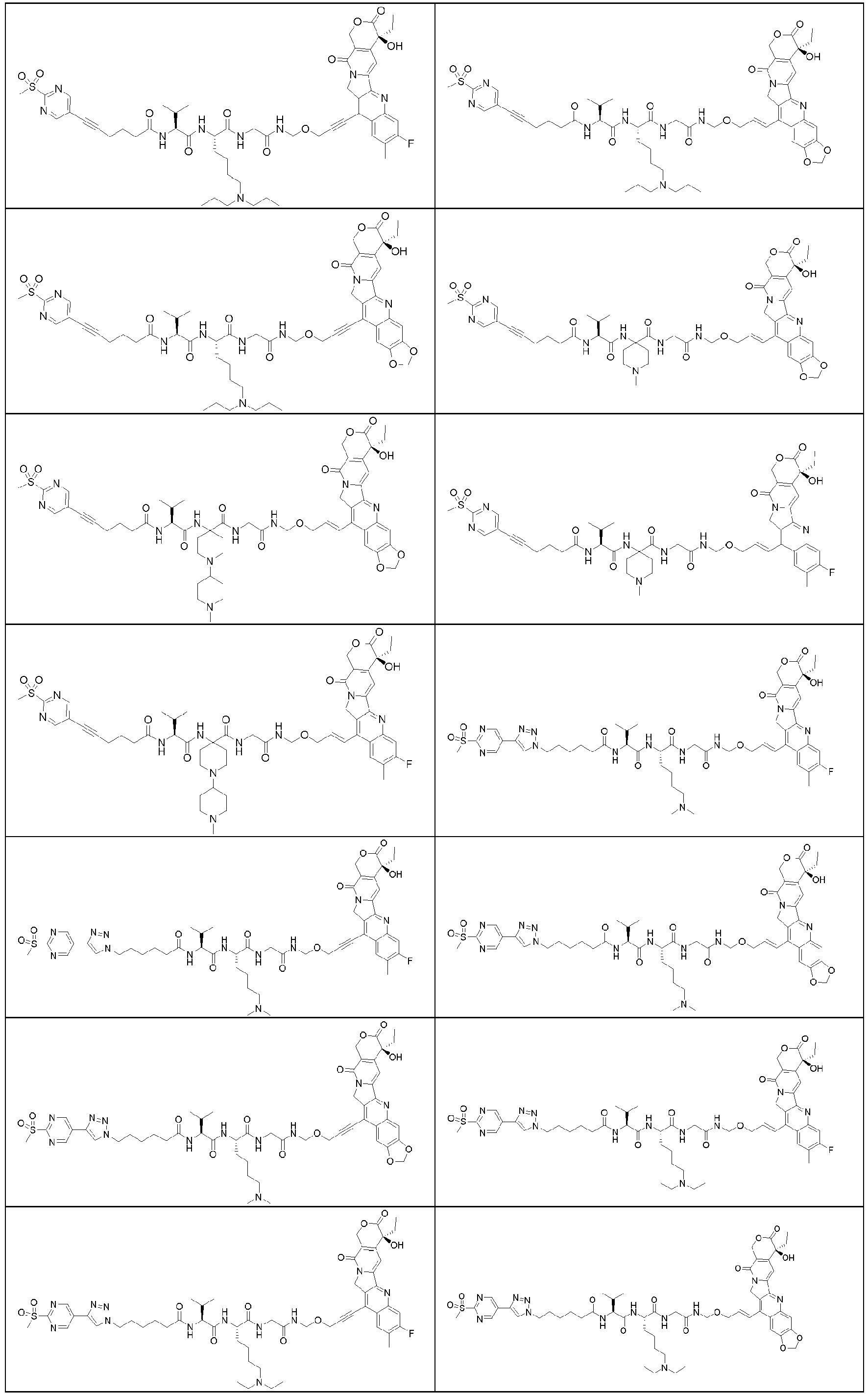

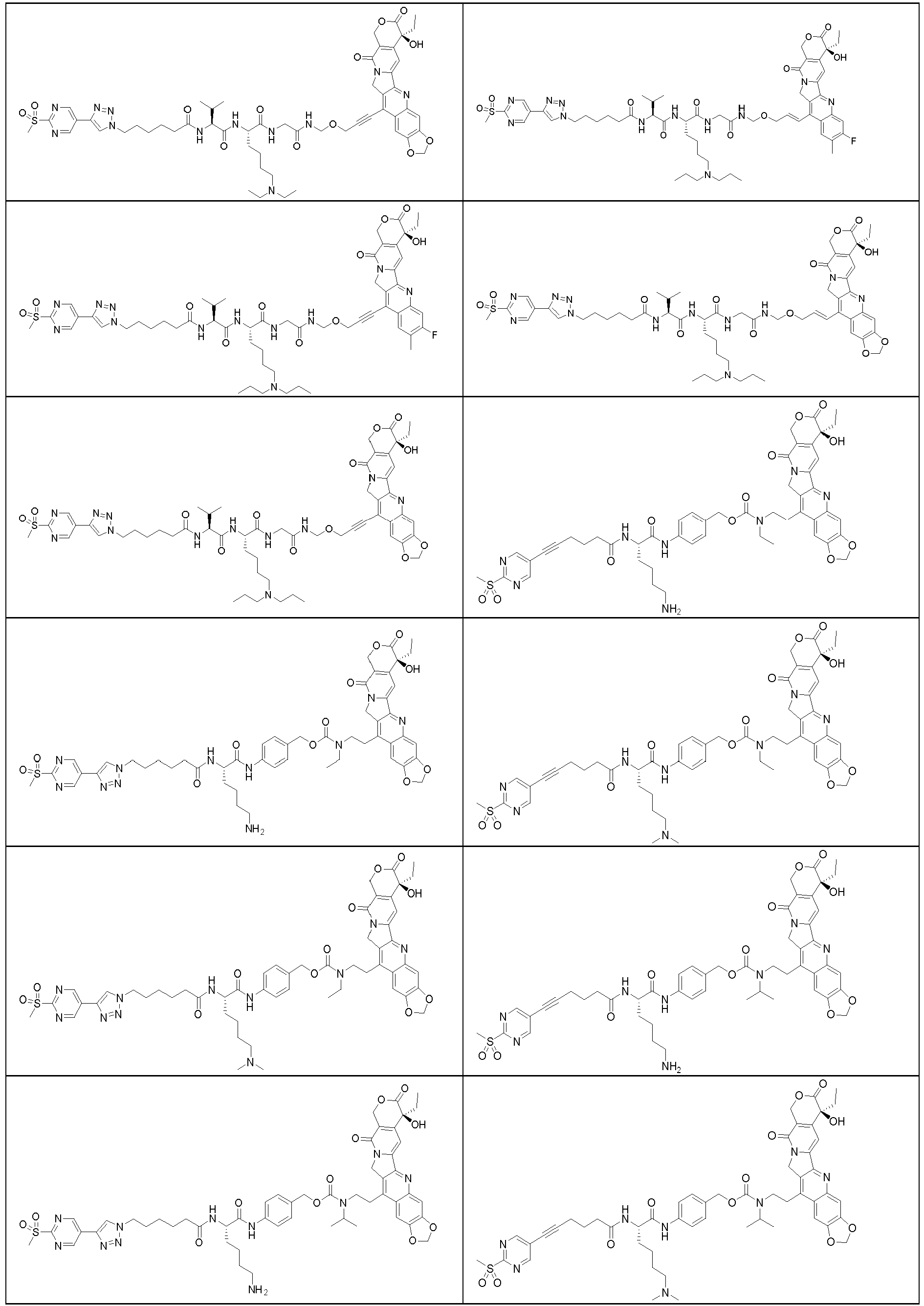

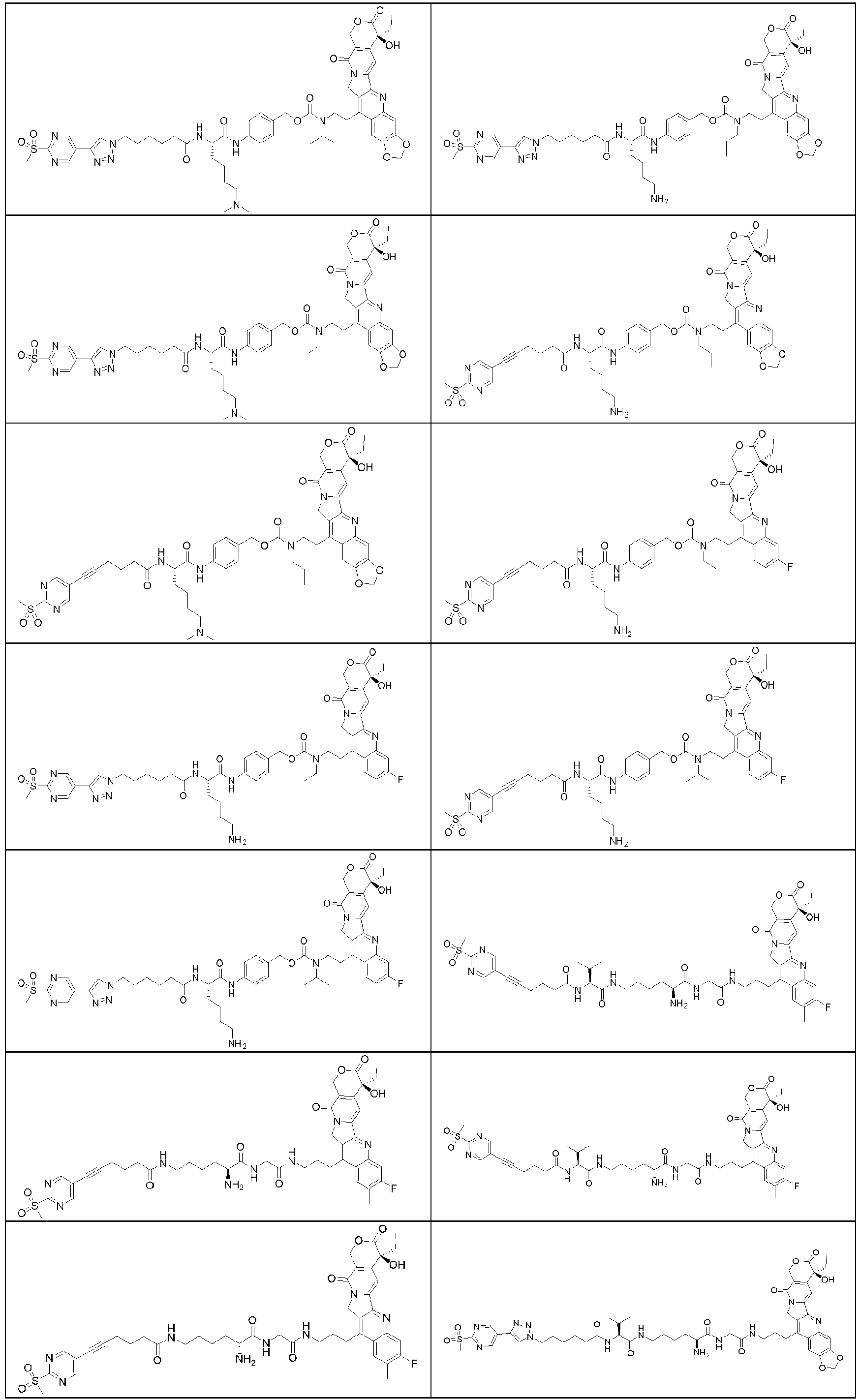

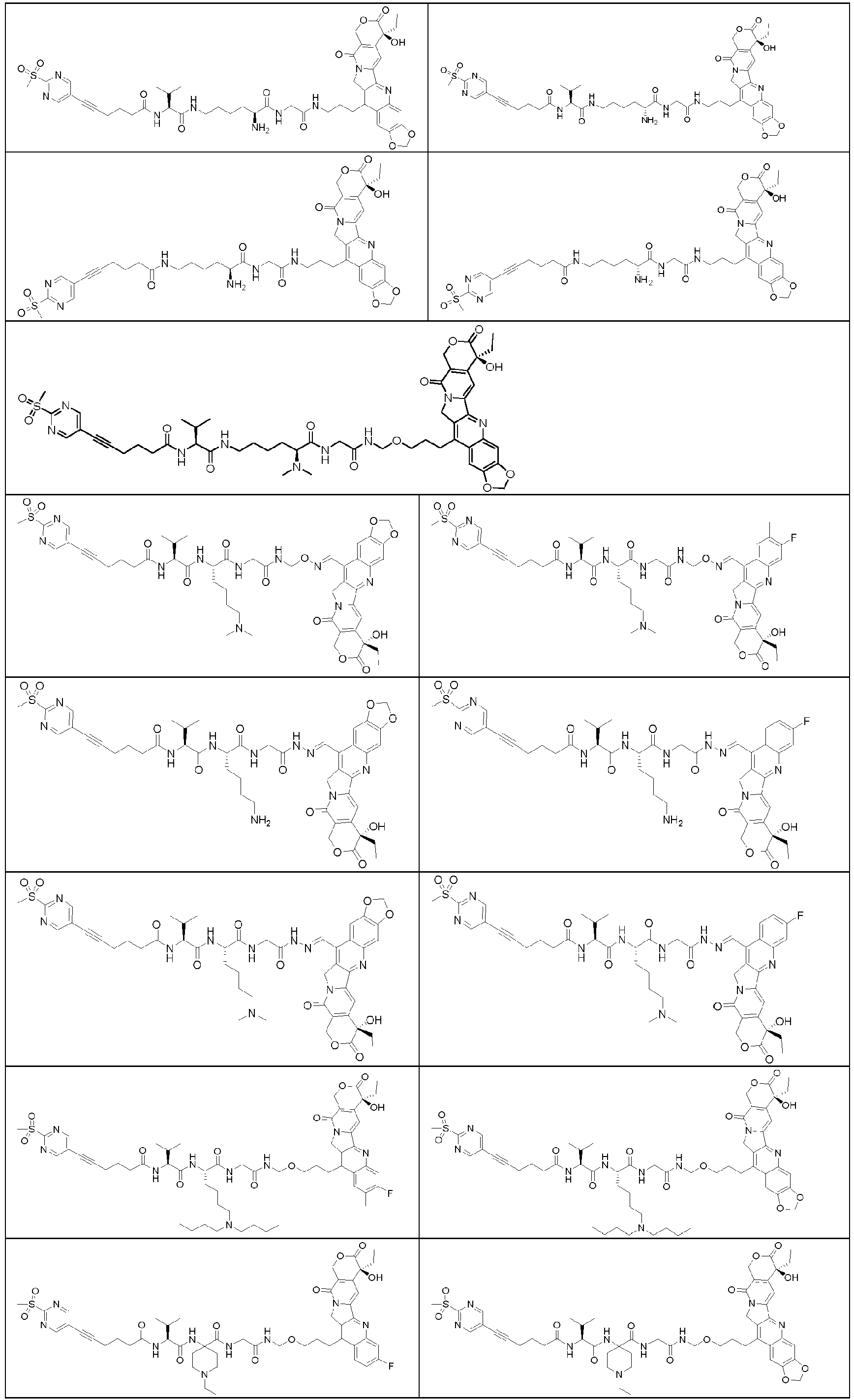

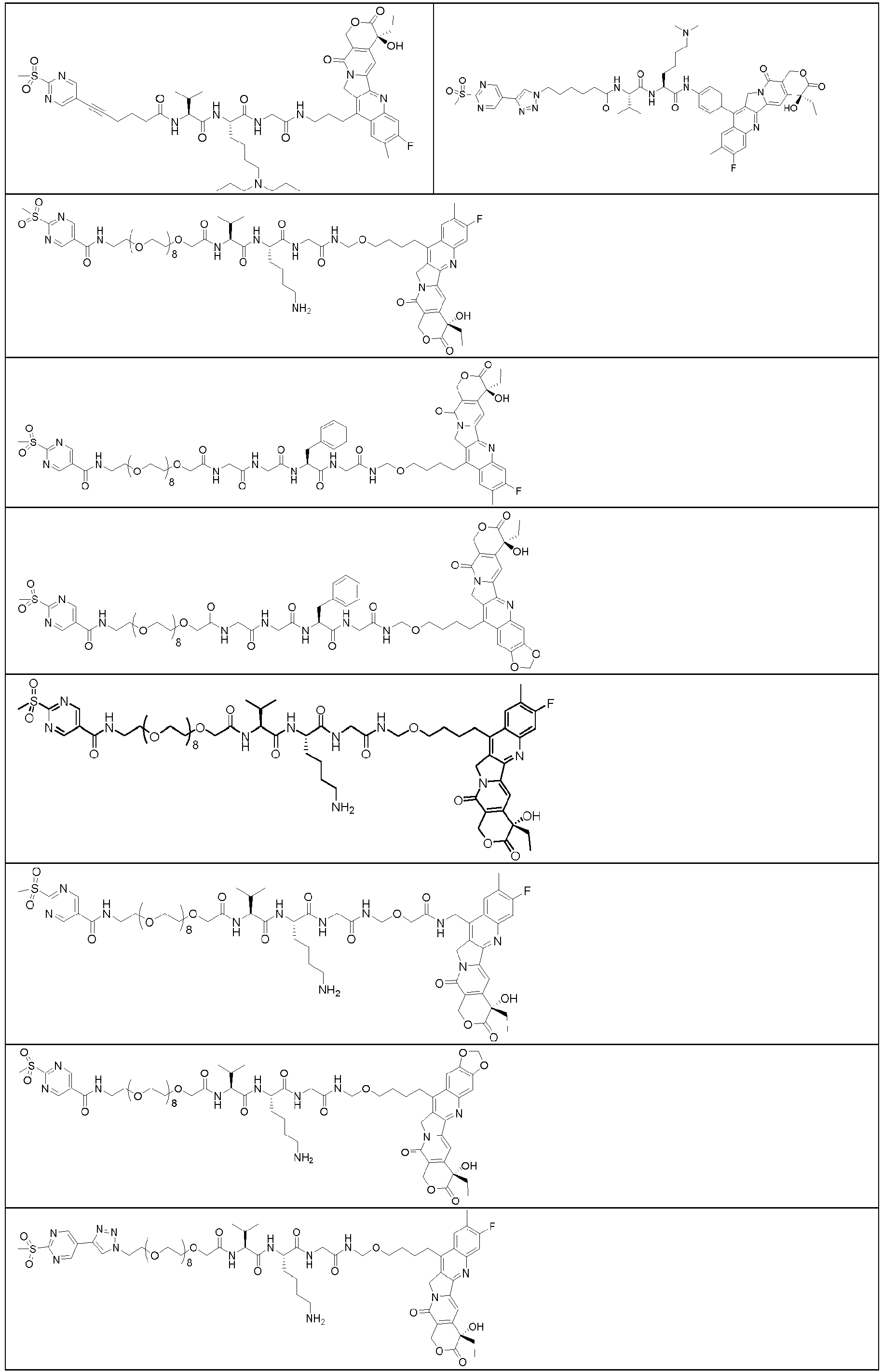

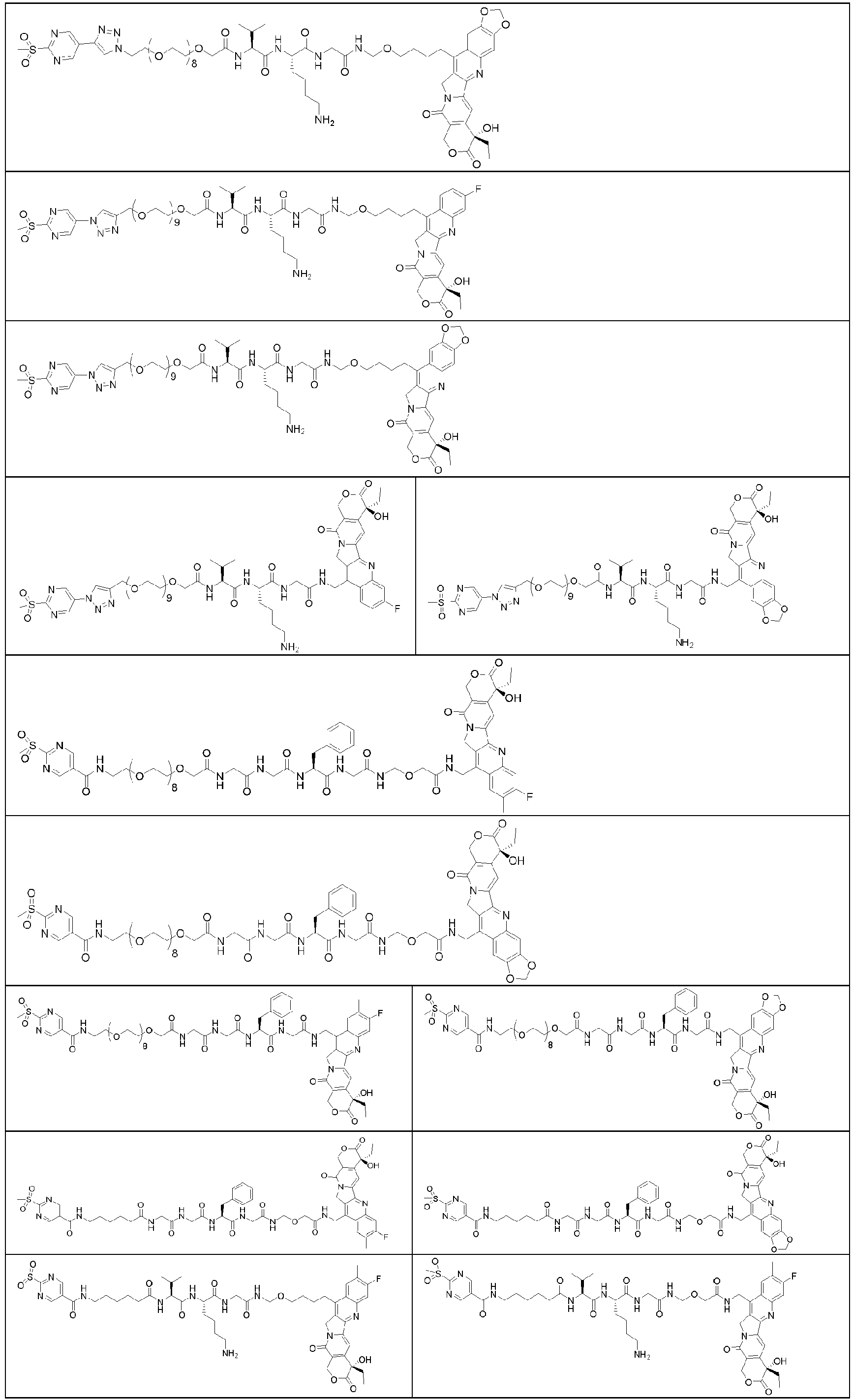

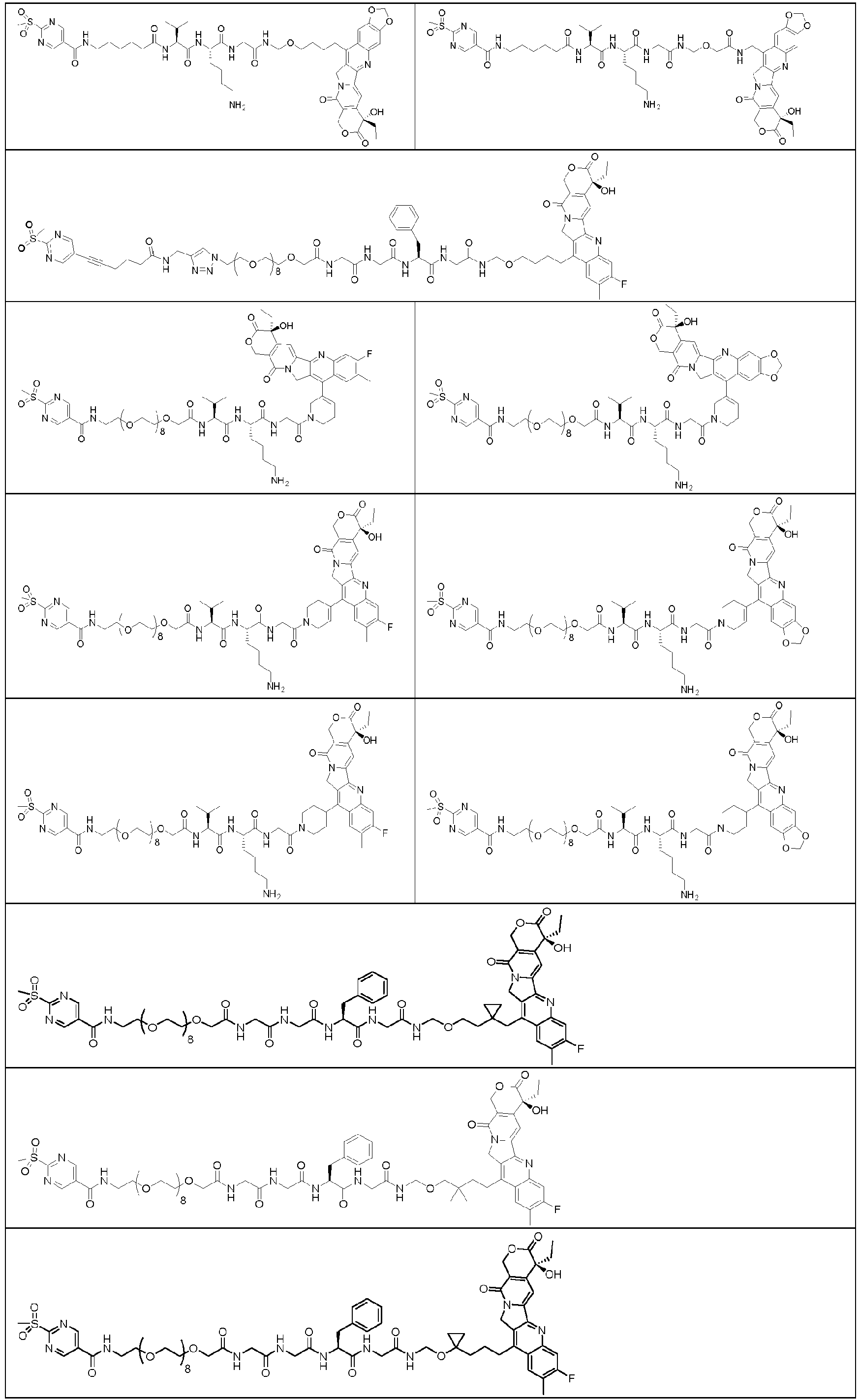

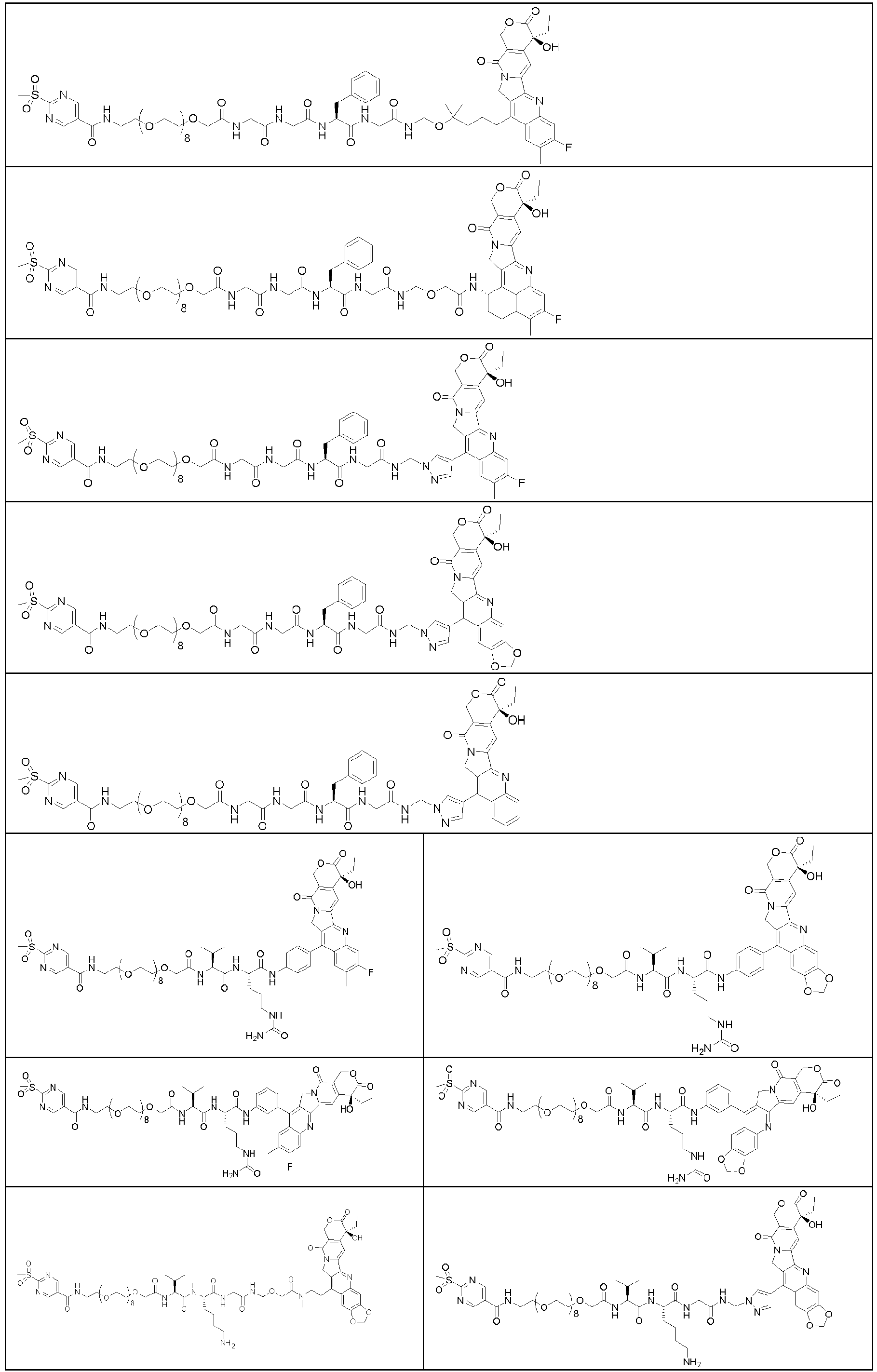

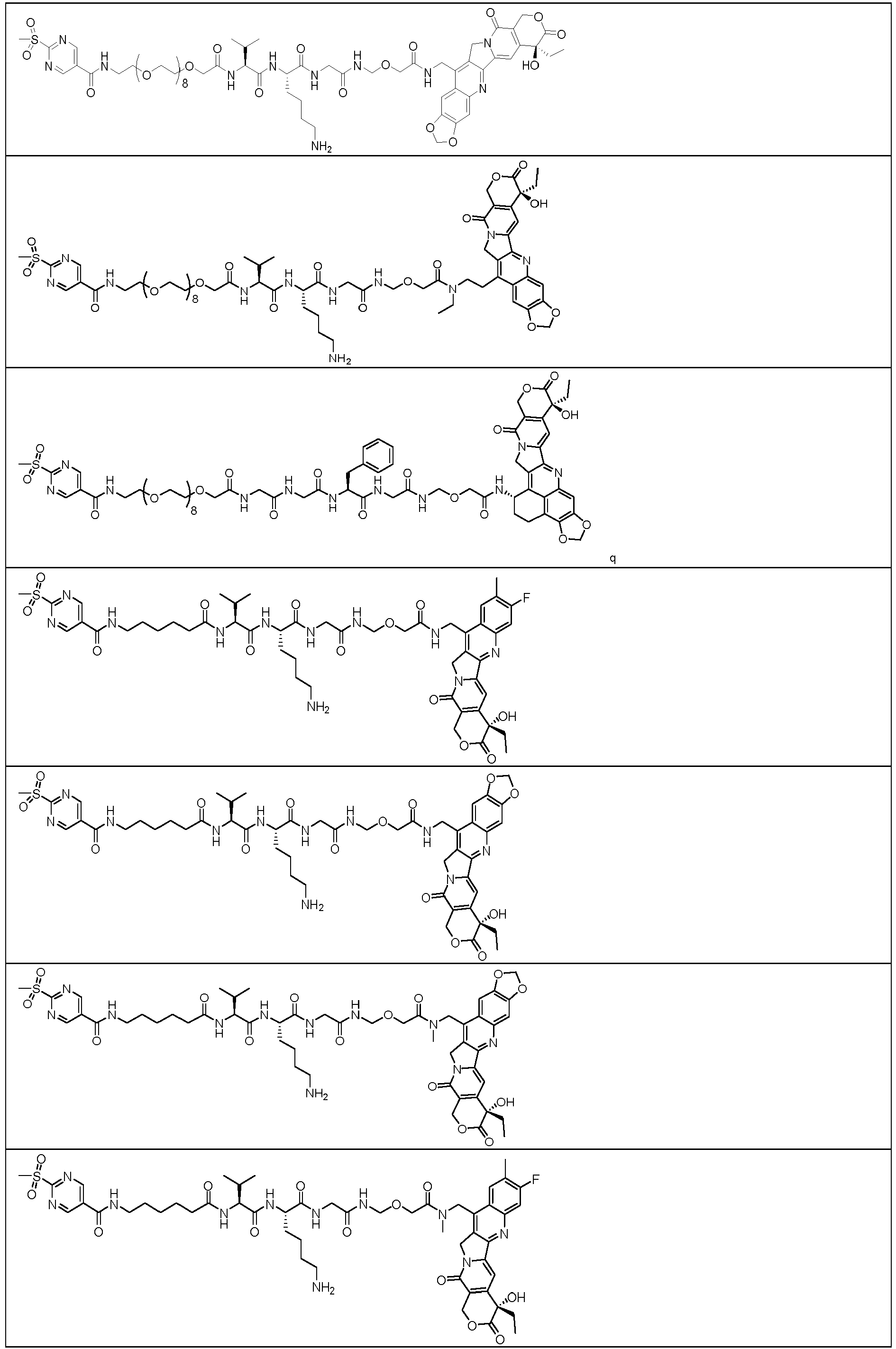

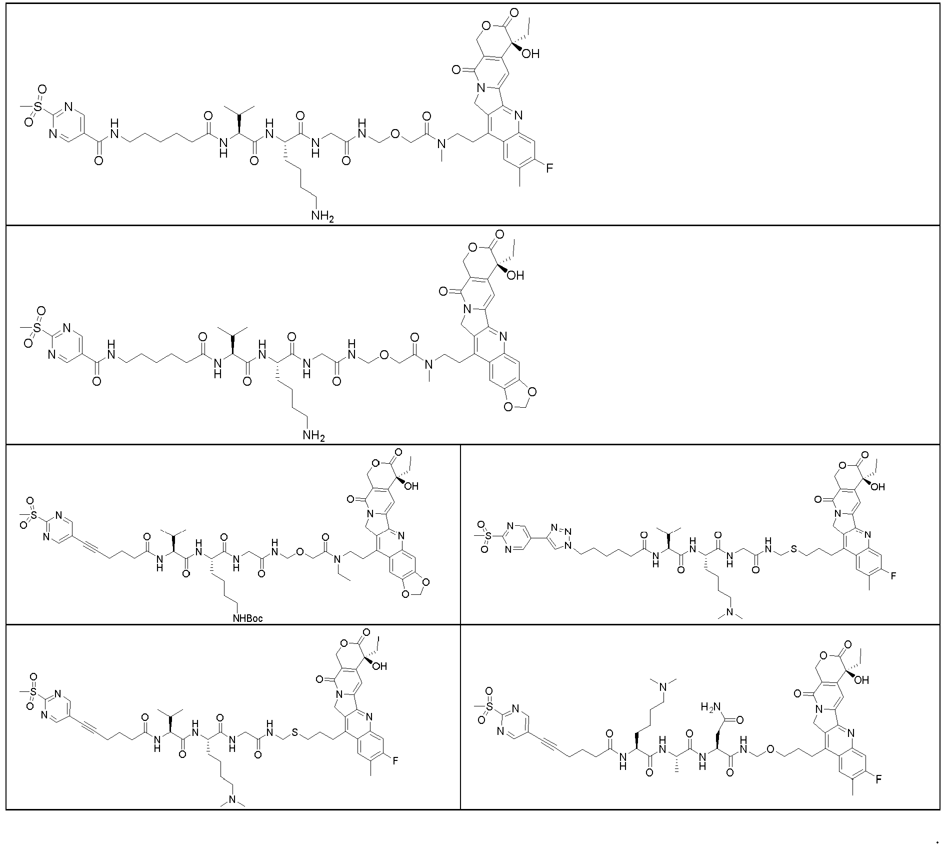

In some embodiments, compounds are provided, wherein L1-L2-L3 unit in the drug linker conjugate of formula III has been partially cleaved, and thus the drug moiety with amino acid residues bound thereto is left.

In some embodiments, the partially released free drug is a compound of formula III-(A): 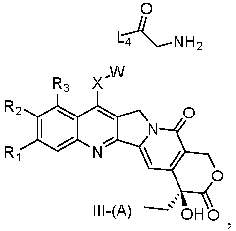 or a stereoisomer thereof or a mixture of stereoisomers thereof, a pharmaceutically acceptable salt thereof, wherein L₄, X, W, R₁, R₂ and R₃ are as defined in any embodiment specifically described above and herein.

In some embodiments, the structure 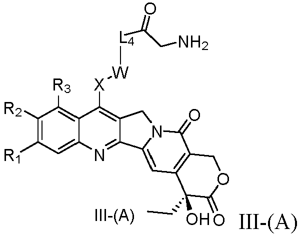 is selected from:

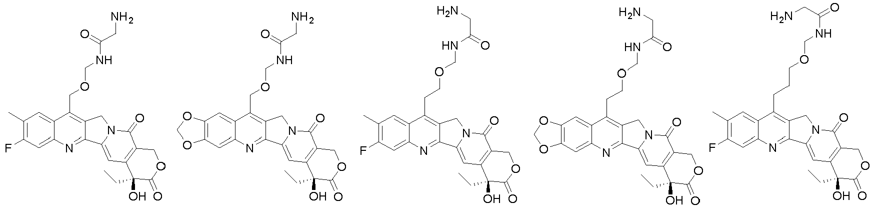

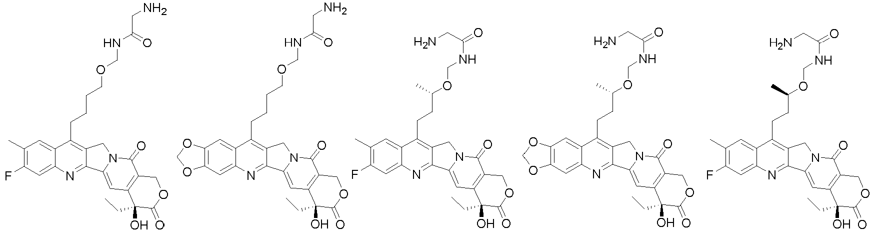

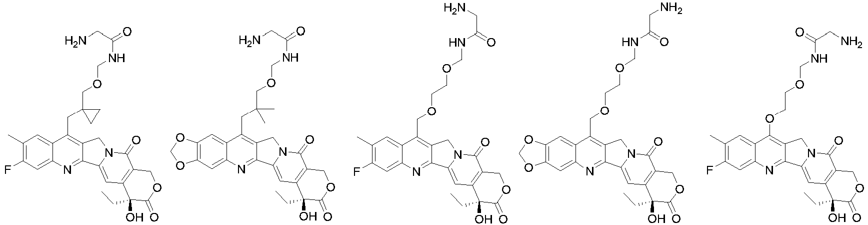

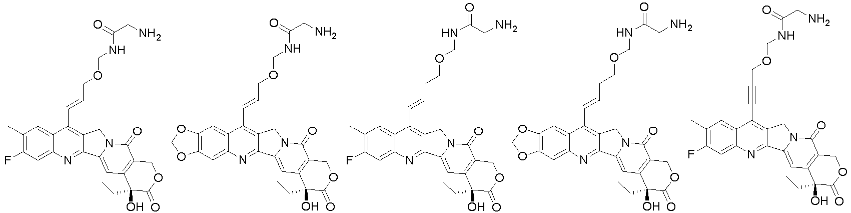

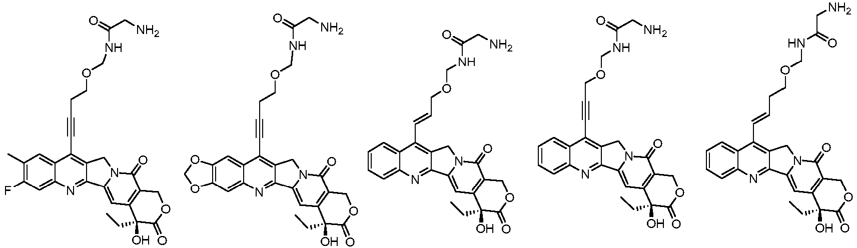

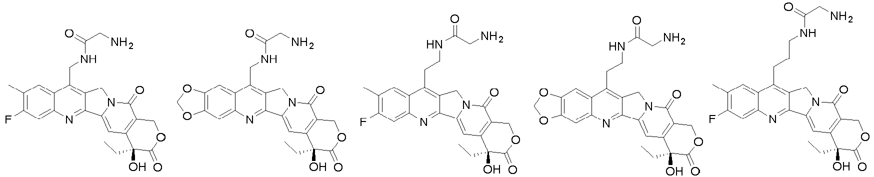

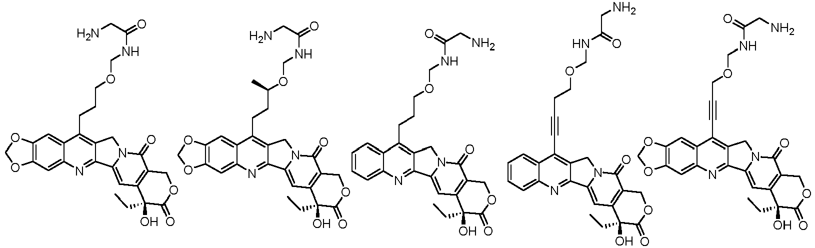

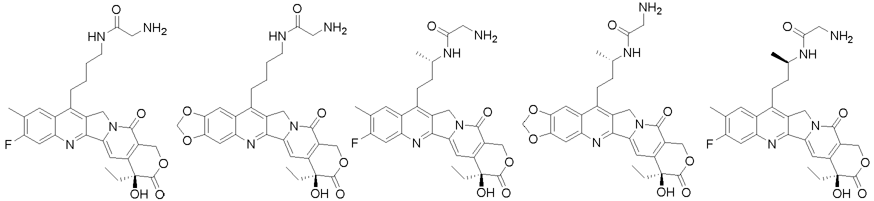

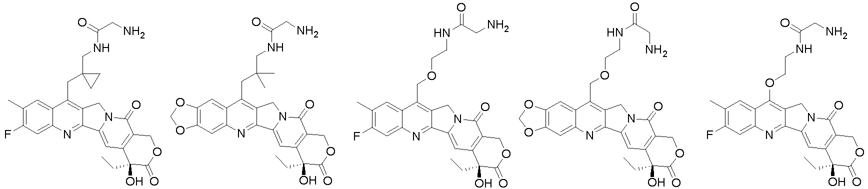

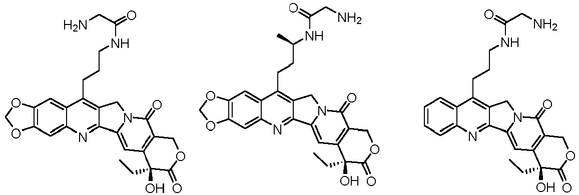

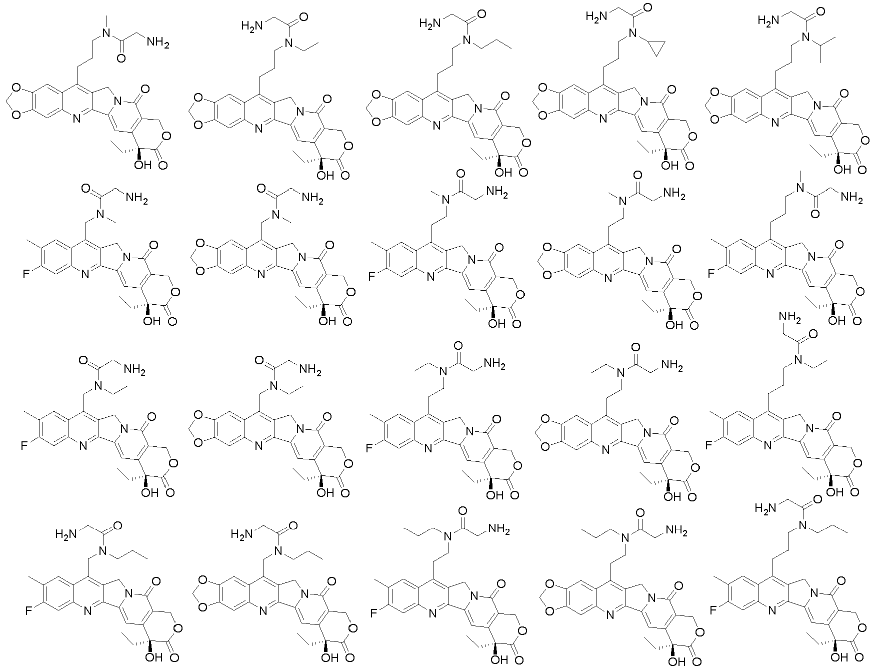

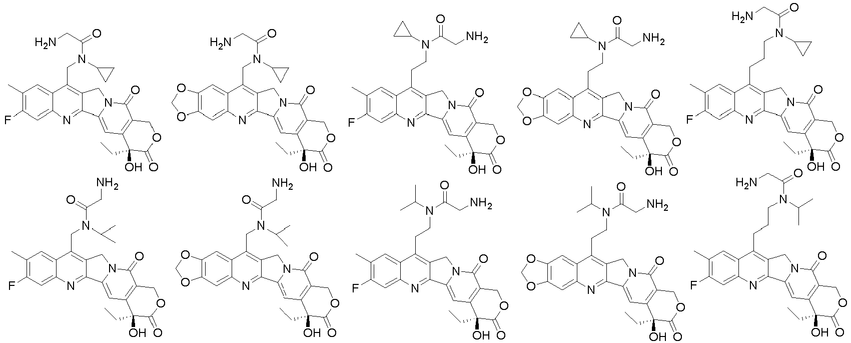

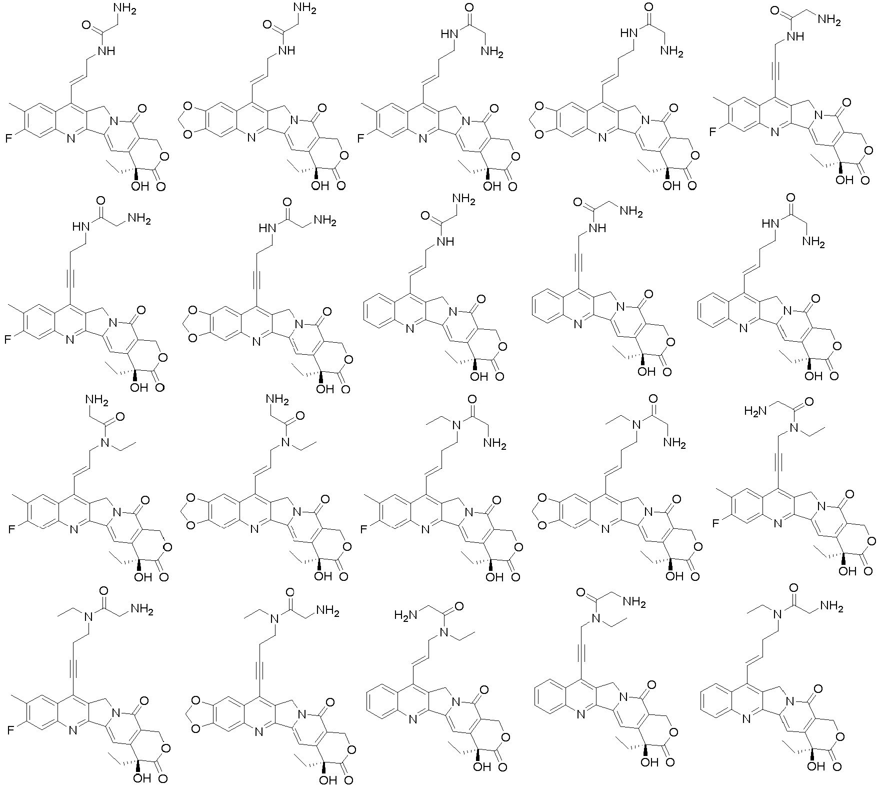

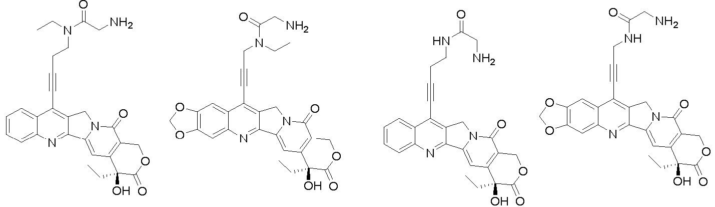

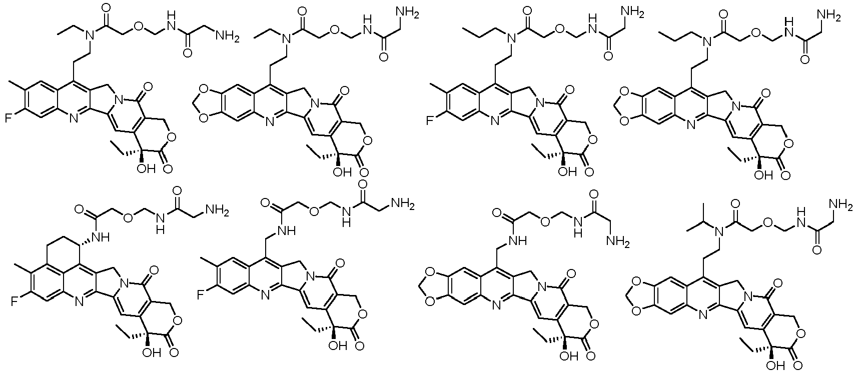

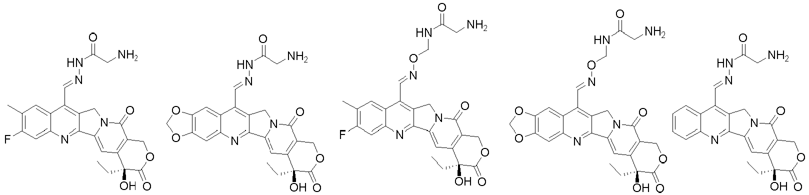

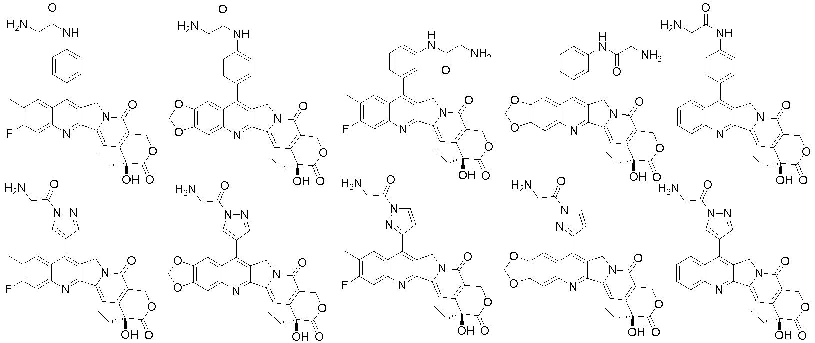

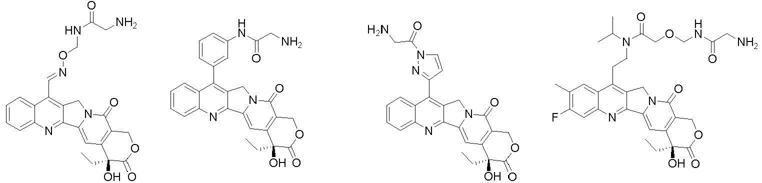

The present disclosure also provides a linker in a ligand drug conjugate, comprising the following fragments: 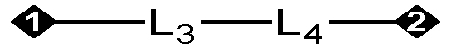
wherein, position 2 is attached to the bioactive molecular fragment; (position 1 is attached to the ligand end, e.g., when it is attached to a linking unit (e.g., L₂), and then to the ligand or the targeting moiety (Tb) via an extension unit (e.g., L₁); or, when the linking unit is not present, position 1 is directly attached to the extension unit and further attached to the ligand);
L₃, L₄ are defined as in any one of embodiments in the present disclosure.

In some embodiments, the linker in the ligand drug conjugate has the following structure: 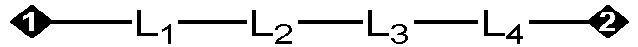
wherein position 1 is attached to a ligand or targeting moiety that binds to a target, and position 2 is attached to a bioactive molecule fragment;
L₁, L₂, L₃, L₄ are defined as in any one of embodiments in the present disclosure;
preferably, said ligand or targeting moiety that binds to a target and said bioactive molecule fragment is defined as for Tb and D in any one of embodiments in the present disclosure, respectively.

In some embodiments, position 1 of the linker is attached to an antibody or antigen-binding fragment thereof, and position 2 is attached to a bioactive molecule fragment.

In some embodiments, position 1 of the linker is attached to the cysteine or lysine in the antibody or its antigen-binding fragment, and position 2 is attached to a bioactive molecule fragment;
preferably, the antibody or antigen-binding fragment thereof is as described in any of the embodiments in the present disclosure.

In a fourth aspect of the present disclosure, the present disclosure also provides the linker represented by formula III-1:

Lg-L₁-L₂-L₃-L₄-Lg1 III-1

Lg, L₁, L₂, L₃ and L₄ are defined in any embodiment specifically described above and herein; Lg1 is the leaving group in the reaction with the drug molecule.

In some embodiments, Lg1 is preferably -OH, 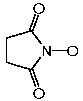 or halogen.

In a fifth aspect of the present disclosure, the present disclosure also provides the linker represented by formula III-2:

Lg-L₁-L₂-L₃-Lg2 III-2

Lg, L₁, L₂ and L₃ are defined in any embodiment specifically described above and herein; Lg2 is a leaving group in the reaction with L4 or a fragment containing a drug molecule.

In some embodiments, Lg2 is preferably -OH, 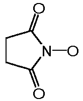 or halogen.

In a sixth aspect of the present disclosure, the present disclosure provides a method for preparing a compound of formula II (drug molecule, bioactive molecule), as well as a drug linker conjugate of formula III.

In one aspect, the present disclosure provides a method for preparing a compound of formula II (pharmaceutical molecule, bioactive molecule). Specifically:

Compound (VI) is obtained by alkylation reaction of compound (IV) with compound (V) catalyzed by iron compounds or other related Minisci reactions.

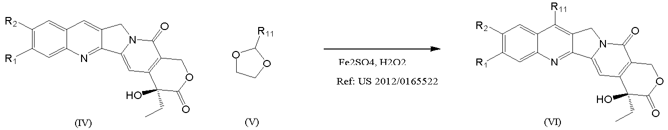

Alternatively, compound (VII) is synthesized by N-oxidation reaction of compound (IV), and then compound (VII) is treated with phosphorus oxyhalide to provide compound (VIII), the halogenated product of compound (IV). Compound (VI) can be obtained from compound (VIII) by various reactions, such as Heck reaction, Suzuki reaction, Buchwald reaction, Nigishi reaction, Stille reaction, etc.

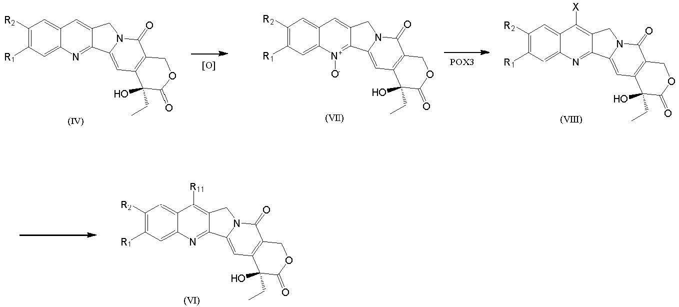

Different or more complex molecules can be synthesized by various chemical transformations of R₁₁ in compound (VI), such as oxidation, reduction, substitution and other methods commonly found in textbooks.

Alternatively, compound (VI) and compound (IV) can also be produced from compound (IX) and compound (X) by a ring-closing reaction as shown below.

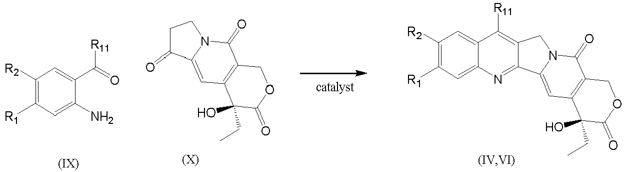

In another aspect, the present disclosure provides a method for preparing the fragment represented by formula III-1 and formula III-2. In particular:
compound III-c can be obtained by the reaction of functional group Fg₁ in compound III-a and functional group Fg₂ in compound III-b, and then compound III-d can be synthesized by the reaction of functional group Fg^{a} in compound III-c. Compounds III-2 and III-1 represented by the general formula can be obtained by similar transformation. Lg^{a}, Lg^{b}, Lg₁ and Lg₂ are reactive leaving groups, such as -OH, 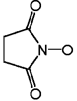 or halogen, etc.

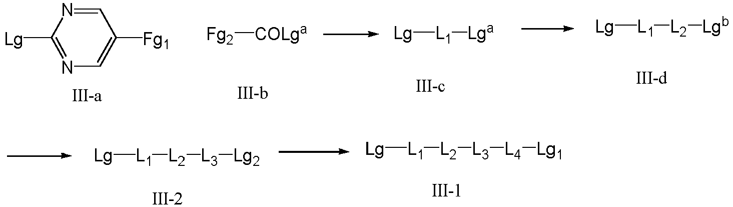

In yet another aspect, the present disclosure provides a method for preparing a drug linker conjugate of formula III. In particular:
The target product can be obtained by the coupling of fragment B and fragment C, as shown in the following reaction scheme. The conjugate product is subjected to some common chemical modifications, such as oxidation, deprotection, etc., to obtain the drug linker conjugate represented by general formula III.

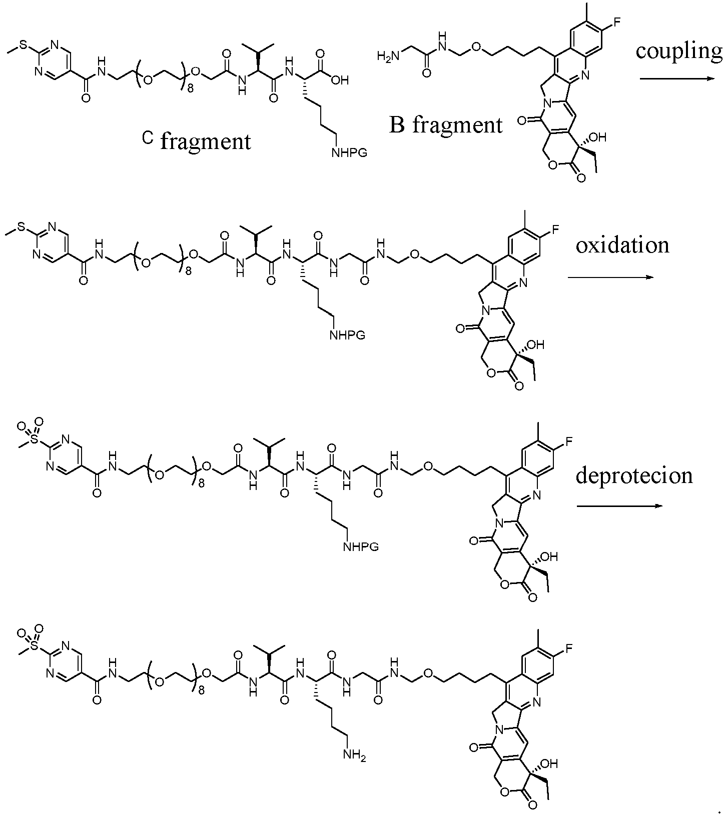

In a seventh aspect of the present disclosure, the present disclosure provides a method for preparing the aforementioned ligand drug conjugate, which comprises:
Tb is coupled to the drug linker conjugate represented by formula III 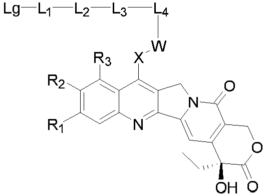 under suitable solvents and conditions;
wherein:
   Tb is as defined in any embodiment specifically described above and herein;
   R₁, R₂, R₃, X, W, L₁, L₂, L₃, L₄ and Lg are defined in any embodiment specifically described above and herein.

In some embodiments, the method comprises the step of performing a coupling reaction between Tb and the drug linker conjugate of formula III 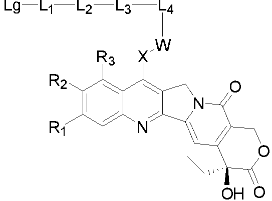 in a suitable solvent and under suitable conditions to form a C-S bond.

In some embodiments, the ratio of said Tb to said drug linker conjugate in molar amountis 1:(1-20), such as 1:2, 1:3, 1:4, 1:5, 1:6, 1:7, 1:8, 1:9, 1:10, 1:12, 1:14, 1:16, 1:18, 1:(10-20), 1:(12-20), 1:(14-20), 1:(16-20) or 1:(18-20).

In some embodiments, the coupling reaction is carried out in water and/or an organic solvent.

In some embodiments, the organic solvent is selected from N,N-dimethylformamide, dimethylsulfoxide, N-methylpyrrolidone, nitriles (e.g. acetonitrile), alcohols (e.g. methanol, ethanol) or any combination thereof.

In some embodiments, the process further comprises the step of purifying the coupling product.

In some embodiments, the coupling product is purified by chromatography.

In some embodiments, the chromatography comprises one or more of ion exchange chromatography, hydrophobic chromatography, reverse phase chromatography or affinity chromatography.

In an eighth aspect of the present disclosure, the present disclosure provides an antibody or antigen-binding fragment thereof that binds B7H3, said antibody or antigen-binding fragment comprises the complementarity determining regions (CDRs) as follows:
HCDR1 or a variant of its sequence, HCDR2 or a variant of its sequence, and HCDR3 or a variant of its sequence as comprised in the heavy chain variable region (VH) set forth in SEQ ID NO:3 or 23; and/or
LCDR1 or a variant of its sequence, LCDR2 or a variant of its sequence, and LCDR3 or a variant of its sequence as comprised in the light chain variable region (VL) set forth in SEQ ID NO: 13 or 33.

In certain embodiments, the antibody or antigen-binding fragment thereof comprises HCDR1 or a variant of its sequence, HCDR2 or a variant of its sequence, and HCDR3 or a variant of its sequence as comprised in the VH set forth in SEQ ID NO:3; and/or LCDR1 or a variant of its sequence, LCDR2 or a variant of its sequence, and LCDR3 or a variant of its sequence as comprised in the VL set forth in SEQ ID NO: 13.

In certain embodiments, the antibody or antigen-binding fragment thereof comprises HCDR1 or a variant of its sequence, HCDR2 or a variant of its sequence, and HCDR3 or a variant of its sequence as comprised in the VH set forth in SEQ ID NO:3; and/or LCDR1 or a variant of its sequence, LCDR2 or a variant of its sequence, and LCDR3 or a variant of its sequence as comprised in the VL set forth in SEQ ID NO: 33.

In certain embodiments, the antibody or antigen-binding fragment thereof comprises HCDR1 or a variant of its sequence, HCDR2 or a variant of its sequence, and HCDR3 or a variant of its sequence as comprised in the VH set forth in SEQ ID NO:23; and/or LCDR1 or a variant of its sequence, LCDR2 or a variant of its sequence, and LCDR3 or a variant of its sequence as comprised in the VL set forth in SEQ ID NO: 33.

In certain embodiments, the antibody or antigen-binding fragment thereof comprises HCDR1 or a variant of its sequence, HCDR2 or a variant of its sequence, and HCDR3 or a variant of its sequence as comprised in the VH set forth in SEQ ID NO:23; and/or LCDR1 or a variant of its sequence, LCDR2 or a variant of its sequence, and LCDR3 or a variant of its sequence as comprised in the VL set forth in SEQ ID NO: 13.

In certain preferred embodiments, the variant of said sequence is a CDR which has one or more amino acid substitutions, deletions or additions (e.g. 1, 2 or 3 amino acid substitutions, deletions or additions), compared with the CDR from which it is derived.

In certain preferred embodiments, the substitution(s) are conservative substitution(s).

Preferably, the CDR is defined according to AbM, Chothia, Kabat or IMGT numbering systems.

In certain embodiments, the VH and/or VL of the antibody or antigen-binding fragment thereof include a framework region (FR) from a human immunoglobulin.

In certain embodiments, the antibody or antigen-binding fragment thereof binds to human B7H3 and/or monkey B7H3. In certain embodiments, the antibody or antigen-binding fragment thereof binds to human 2Ig B7H3. In certain embodiments, the antibody or antigen-binding fragment thereof binds to human 4Ig B7H3. In certain embodiments, the antibody or antigen-binding fragment thereof binds to monkey 4Ig B7H3. In certain embodiments, the antibody or antigen-binding fragment thereof binds to human 2Ig B7H3 and human 4Ig B7H3, and preferably binds to human 4Ig B7H3. In certain embodiments, the antibody or antigen-binding fragment thereof binds to human 4Ig B7H3, not human 2Ig B7H3.

In one aspect, the present disclosure provides an antibody or antigen-binding fragment thereof capable of binding to B7H3, and the antibody or antigen-binding fragment thereof comprises a heavy chain variable region (VH) and/or a light chain variable region (VL).

In certain embodiments, the antibody or antigen-binding fragment thereof according to the present disclosure comprises the following heavy chain variable region (VH) and/or light chain variable region (VL), wherein the CDR is defined according to IMGT numbering system:
(a) the heavy chain variable region (VH) comprising the following 3 CDRs: HCDR1 consisting of the sequence of SEQ ID NO: 10 or a sequence having one or more amino acid substitutions, deletions or additions (e.g. 1, 2 or 3 amino acid substitutions, deletions or additions) compared with SEQ ID NO: 10; HCDR2 consisting of the sequence of SEQ ID NO: 11 or a sequence having one or more amino acid substitutions, deletions or additions (e.g. 1, 2 or 3 amino acid substitutions, deletions or additions) compared with SEQ ID NO: 11; HCDR3 consisting of the sequence of SEQ ID NO: 12 or a sequence having one or more amino acid substitutions, deletions or additions (e.g. 1, 2 or 3 amino acid substitutions, deletions or additions) compared with SEQ ID NO: 12; and/or,
   the light chain variable region (VL) comprising the following 3 CDRs: LCDR1 consisting of the sequence of SEQ ID NO: 20 or a sequence having one or more amino acid substitutions, deletions or additions (e.g. 1, 2 or 3 amino acid substitutions, deletions or additions) compared with SEQ ID NO: 20; LCDR2 consisting of the sequence of GTF or a sequence having one or more amino acid substitutions, deletions or additions (e.g. 1, 2 or 3 amino acid substitutions, deletions or additions) compared with GTF; LCDR3 consisting of the sequence of SEQ ID NO: 22 or a sequence having one or more amino acid substitutions, deletions or additions (e.g. 1, 2 or 3 amino acid substitutions, deletions or additions) compared with SEQ ID NO: 22;
(b) the heavy chain variable region (VH) comprising the following 3 CDRs: HCDR1 consisting of the sequence of SEQ ID NO: 30 or a sequence having one or more amino acid substitutions, deletions or additions (e.g. 1, 2 or 3 amino acid substitutions, deletions or additions) compared with SEQ ID NO: 30; HCDR2 consisting of the sequence of SEQ ID NO: 31 or a sequence having one or more amino acid substitutions, deletions or additions (e.g. 1, 2 or 3 amino acid substitutions, deletions or additions) compared with SEQ ID NO: 31; HCDR3 consisting of the sequence of SEQ ID NO: 32 or a sequence having one or more amino acid substitutions, deletions or additions (e.g. 1, 2 or 3 amino acid substitutions, deletions or additions) compared with SEQ ID NO: 32; and/or,
   the light chain variable region (VL) comprising the following 3 CDRs: LCDR1 consisting of the sequence of SEQ ID NO: 40 or a sequence having one or more amino acid substitutions, deletions or additions (e.g. 1, 2 or 3 amino acid substitutions, deletions or additions) compared with SEQ ID NO: 40; LCDR2 consisting of the sequence of GAS or a sequence having one or more amino acid substitutions, deletions or additions (e.g. 1, 2 or 3 amino acid substitutions, deletions or additions) compared with GAS; LCDR3 consisting of the sequence of SEQ ID NO: 42 or a sequence having one or more amino acid substitutions, deletions or additions (e.g. 1, 2 or 3 amino acid substitutions, deletions or additions) compared with SEQ ID NO: 42;
(c) the heavy chain variable region (VH) comprising the following 3 CDRs: HCDR1 consisting of the sequence of SEQ ID NO: 10 or a sequence having one or more amino acid substitutions, deletions or additions (e.g. 1, 2 or 3 amino acid substitutions, deletions or additions) compared with SEQ ID NO: 10; HCDR2 consisting of the sequence of SEQ ID NO: 11 or a sequence having one or more amino acid substitutions, deletions or additions (e.g. 1, 2 or 3 amino acid substitutions, deletions or additions) compared with SEQ ID NO: 11; HCDR3 consisting of the sequence of SEQ ID NO: 12 or a sequence having one or more amino acid substitutions, deletions or additions (e.g. 1, 2 or 3 amino acid substitutions, deletions or additions) compared with SEQ ID NO: 12; and/or,
   the light chain variable region (VL) comprising the following 3 CDRs: LCDR1 consisting of the sequence of SEQ ID NO: 40 or a sequence having one or more amino acid substitutions, deletions or additions (e.g. 1, 2 or 3 amino acid substitutions, deletions or additions) compared with SEQ ID NO: 40; LCDR2 consisting of the sequence of GAS or a sequence having one or more amino acid substitutions, deletions or additions (e.g. 1, 2 or 3 amino acid substitutions, deletions or additions) compared with GAS; LCDR3 consisting of the sequence of SEQ ID NO: 42 or a sequence having one or more amino acid substitutions, deletions or additions (e.g. 1, 2 or 3 amino acid substitutions, deletions or additions) compared with SEQ ID NO: 42;
   or
(d) the heavy chain variable region (VH) comprising the following 3 CDRs: HCDR1 consisting of the sequence of SEQ ID NO: 30 or a sequence having one or more amino acid substitutions, deletions or additions (e.g. 1, 2 or 3 amino acid substitutions, deletions or additions) compared with SEQ ID NO: 30; HCDR2 consisting of the sequence of SEQ ID NO: 31 or a sequence having one or more amino acid substitutions, deletions or additions (e.g. 1, 2 or 3 amino acid substitutions, deletions or additions) compared with SEQ ID NO: 31; HCDR3 consisting of the sequence of SEQ ID NO: 32 or a sequence having one or more amino acid substitutions, deletions or additions (e.g. 1, 2 or 3 amino acid substitutions, deletions or additions) compared with SEQ ID NO: 32; and/or,
the light chain variable region (VL) comprising the following 3 CDRs: LCDR1 consisting of the sequence of SEQ ID NO: 20 or a sequence having one or more amino acid substitutions, deletions or additions (e.g. 1, 2 or 3 amino acid substitutions, deletions or additions) compared with SEQ ID NO: 20; LCDR2 consisting of the sequence of GTF or a sequence having one or more amino acid substitutions, deletions or additions (e.g. 1, 2 or 3 amino acid substitutions, deletions or additions) compared with GTF; LCDR3 consisting of the sequence of SEQ ID NO: 22 or a sequence having one or more amino acid substitutions, deletions or additions (e.g. 1, 2 or 3 amino acid substitutions, deletions or additions) compared with SEQ ID NO: 22.

In certain embodiments, the antibody or antigen-binding fragment thereof according to the present disclosure comprises the following heavy chain variable region (VH) and/or light chain variable region (VL), wherein the CDRs are defined according to IMGT numbering system:
(a) the heavy chain variable region (VH) comprising the following 3 CDRs: HCDR1 consisting of the sequence of SEQ ID NO: 10, HCDR2 consisting of the sequence of SEQ ID NO: 11, HCDR3 consisting of the sequence of SEQ ID NO: 12; and/or
   the light chain variable region (VL) comprising the following 3 CDRs: LCDR1 consisting of the sequence of SEQ ID NO: 20, LCDR2 consisting of the sequence GTF, and LCDR3 consisting of the sequence of SEQ ID NO: 22;
(b) the heavy chain variable region (VH) comprising the following 3 CDRs: HCDR1 consisting of the sequence of SEQ ID NO: 10, HCDR2 consisting of the sequence of SEQ ID NO: 11, HCDR3 consisting of the sequence of SEQ ID NO: 12; and/or
   the light chain variable region (VL) comprising the following 3 CDRs: LCDR1 consisting of the sequence of SEQ ID NO: 40, LCDR2 consisting of the sequence GAS, and LCDR3 consisting of the sequence of SEQ ID NO: 42;
(c) the heavy chain variable region (VH) comprising the following 3 CDRs: HCDR1 consisting of the sequence of SEQ ID NO: 30, HCDR2 consisting of the sequence of SEQ ID NO: 31, HCDR3 consisting of the sequence of SEQ ID NO: 32; and/or
   the light chain variable region (VL) comprising the following 3 CDRs: LCDR1 consisting of the sequence of SEQ ID NO: 40, LCDR2 consisting of the sequence GAS, and LCDR3 consisting of the sequence of SEQ ID NO: 42;
   or
(d) the heavy chain variable region (VH) comprising the following 3 CDRs: HCDR1 consisting of the sequence of SEQ ID NO: 30, HCDR2 consisting of the sequence of SEQ ID NO: 31, HCDR3 consisting of the sequence of SEQ ID NO: 32; and/or
the light chain variable region (VL) comprising the following 3 CDRs: LCDR1 consisting of the sequence of SEQ ID NO: 20, LCDR2 consisting of the sequence GTF, and LCDR3 consisting of the sequence of SEQ ID NO: 22.

In certain embodiments, the antibody or antigen-binding fragment thereof according to the present disclosure comprises the following heavy chain variable region (VH) and/or light chain variable region (VL), wherein the CDRs are defined according to Chothia numbering system:
(a) the heavy chain variable region (VH) comprising the following 3 CDRs: HCDR1 consisting of the sequence of SEQ ID NO: 4 or a sequence having one or more amino acid substitutions, deletions or additions (e.g. 1, 2 or 3 amino acid substitutions, deletions or additions) compared with SEQ ID NO: 4; HCDR2 consisting of the sequence of SEQ ID NO: 5 or a sequence having one or more amino acid substitutions, deletions or additions (e.g. 1, 2 or 3 amino acid substitutions, deletions or additions) compared with SEQ ID NO: 5; HCDR3 consisting of the sequence of SEQ ID NO: 6 or a sequence having one or more amino acid substitutions, deletions or additions (e.g. 1, 2 or 3 amino acid substitutions, deletions or additions) compared with SEQ ID NO: 6; and/or,
   the light chain variable region (VL) comprising the following 3 CDRs: LCDR1 consisting of the sequence of SEQ ID NO: 14 or a sequence having one or more amino acid substitutions, deletions or additions (e.g. 1, 2 or 3 amino acid substitutions, deletions or additions) compared with SEQ ID NO: 14; LCDR2 consisting of the sequence of SEQ ID NO: 15 or a sequence having one or more amino acid substitutions, deletions or additions (e.g. 1, 2 or 3 amino acid substitutions, deletions or additions) compared with SEQ ID NO: 15; LCDR3 consisting of the sequence of SEQ ID NO: 16 or a sequence having one or more amino acid substitutions, deletions or additions (e.g. 1, 2 or 3 amino acid substitutions, deletions or additions) compared with SEQ ID NO: 16;
(b) the heavy chain variable region (VH) comprising the following 3 CDRs: HCDR1 consisting of the sequence of SEQ ID NO: 4 or a sequence having one or more amino acid substitutions, deletions or additions (e.g. 1, 2 or 3 amino acid substitutions, deletions or additions) compared with SEQ ID NO: 4; HCDR2 consisting of the sequence of SEQ ID NO: 5 or a sequence having one or more amino acid substitutions, deletions or additions (e.g. 1, 2 or 3 amino acid substitutions, deletions or additions) compared with SEQ ID NO: 5; HCDR3 consisting of the sequence of SEQ ID NO: 6 or a sequence having one or more amino acid substitutions, deletions or additions (e.g. 1, 2 or 3 amino acid substitutions, deletions or additions) compared with SEQ ID NO: 6; and/or,
   the light chain variable region (VL) comprising the following 3 CDRs: LCDR1 consisting of the sequence of SEQ ID NO: 34 or a sequence having one or more amino acid substitutions, deletions or additions (e.g. 1, 2 or 3 amino acid substitutions, deletions or additions) compared with SEQ ID NO: 34; LCDR2 consisting of the sequence of SEQ ID NO:35 or a sequence having one or more amino acid substitutions, deletions or additions (e.g. 1, 2 or 3 amino acid substitutions, deletions or additions) compared with SEQ ID NO:35; LCDR3 consisting of the sequence of SEQ ID NO: 36 or a sequence having one or more amino acid substitutions, deletions or additions (e.g. 1, 2 or 3 amino acid substitutions, deletions or additions) compared with SEQ ID NO: 36;
(c) the heavy chain variable region (VH) comprising the following 3 CDRs: HCDR1 consisting of the sequence of SEQ ID NO: 24 or a sequence having one or more amino acid substitutions, deletions or additions (e.g. 1, 2 or 3 amino acid substitutions, deletions or additions) compared with SEQ ID NO: 24; HCDR2 consisting of the sequence of SEQ ID NO: 25 or a sequence having one or more amino acid substitutions, deletions or additions (e.g. 1, 2 or 3 amino acid substitutions, deletions or additions) compared with SEQ ID NO: 25; HCDR3 consisting of the sequence of SEQ ID NO: 26 or a sequence having one or more amino acid substitutions, deletions or additions (e.g. 1, 2 or 3 amino acid substitutions, deletions or additions) compared with SEQ ID NO: 26; and/or,
   the light chain variable region (VL) comprising the following 3 CDRs: LCDR1 consisting of the sequence of SEQ ID NO: 34 or a sequence having one or more amino acid substitutions, deletions or additions (e.g. 1, 2 or 3 amino acid substitutions, deletions or additions) compared with SEQ ID NO: 34; LCDR2 consisting of the sequence of SEQ ID NO:35 or a sequence having one or more amino acid substitutions, deletions or additions (e.g. 1, 2 or 3 amino acid substitutions, deletions or additions) compared with SEQ ID NO:35; LCDR3 consisting of the sequence of SEQ ID NO: 36 or a sequence having one or more amino acid substitutions, deletions or additions (e.g. 1, 2 or 3 amino acid substitutions, deletions or additions) compared with SEQ ID NO: 36;
   or
(d) the heavy chain variable region (VH) comprising the following 3 CDRs: HCDR1 consisting of the sequence of SEQ ID NO: 24 or a sequence having one or more amino acid substitutions, deletions or additions (e.g. 1, 2 or 3 amino acid substitutions, deletions or additions) compared with SEQ ID NO: 24; HCDR2 consisting of the sequence of SEQ ID NO: 25 or a sequence having one or more amino acid substitutions, deletions or additions (e.g. 1, 2 or 3 amino acid substitutions, deletions or additions) compared with SEQ ID NO: 25; HCDR3 consisting of the sequence of SEQ ID NO: 26 or a sequence having one or more amino acid substitutions, deletions or additions (e.g. 1, 2 or 3 amino acid substitutions, deletions or additions) compared with SEQ ID NO: 26; and/or,
the light chain variable region (VL) comprising the following 3 CDRs: LCDR1 consisting of the sequence of SEQ ID NO: 14 or a sequence having one or more amino acid substitutions, deletions or additions (e.g. 1, 2 or 3 amino acid substitutions, deletions or additions) compared with SEQ ID NO: 14; LCDR2 consisting of the sequence of SEQ ID NO: 15 or a sequence having one or more amino acid substitutions, deletions or additions (e.g. 1, 2 or 3 amino acid substitutions, deletions or additions) compared with SEQ ID NO: 15; LCDR3 consisting of the sequence of SEQ ID NO: 16 or a sequence having one or more amino acid substitutions, deletions or additions (e.g. 1, 2 or 3 amino acid substitutions, deletions or additions) compared with SEQ ID NO: 16

In certain embodiments, the antibody or antigen-binding fragment thereof according to the present disclosure comprises the following heavy chain variable region (VH) and/or light chain variable region (VL), wherein the CDR is defined according to chothia numbering system:
(a) the heavy chain variable region (VH) comprising the following 3 CDRs: HCDR1 consisting of the sequence of SEQ ID NO: 4, HCDR2 consisting of the sequence of SEQ ID NO: 5, HCDR3 consisting of the sequence of SEQ ID NO: 6; and/or
   the light chain variable region (VL) comprising the following 3 CDRs: LCDR1 consisting of the sequence of SEQ ID NO: 14, LCDR2 consisting of the sequence SEQ ID NO:15, and LCDR3 consisting of the sequence of SEQ ID NO: 16;
(b) the heavy chain variable region (VH) comprising the following 3 CDRs: HCDR1 consisting of the sequence of SEQ ID NO: 24, HCDR2 consisting of the sequence of SEQ ID NO: 25, HCDR3 consisting of the sequence of SEQ ID NO: 26; and/or
   the light chain variable region (VL) comprising the following 3 CDRs: LCDR1 consisting of the sequence of SEQ ID NO: 34, LCDR2 consisting of the sequence SEQ ID NO:35, and LCDR3 consisting of the sequence of SEQ ID NO: 36;
(c) the heavy chain variable region (VH) comprising the following 3 CDRs: HCDR1 consisting of the sequence of SEQ ID NO: 4, HCDR2 consisting of the sequence of SEQ ID NO: 5, HCDR3 consisting of the sequence of SEQ ID NO: 6; and/or
   the light chain variable region (VL) comprising the following 3 CDRs: LCDR1 consisting of the sequence of SEQ ID NO: 34, LCDR2 consisting of the sequence SEQ ID NO:35, and LCDR3 consisting of the sequence of SEQ ID NO: 36;
   or
(d) the heavy chain variable region (VH) comprising the following 3 CDRs: HCDR1 consisting of the sequence of SEQ ID NO: 24, HCDR2 consisting of the sequence of SEQ ID NO: 25, HCDR3 consisting of the sequence of SEQ ID NO: 26; and/or
the light chain variable region (VL) comprising the following 3 CDRs: LCDR1 consisting of the sequence of SEQ ID NO: 14, LCDR2 consisting of the sequence SEQ ID NO:15, and LCDR3 consisting of the sequence of SEQ ID NO: 16

In certain embodiments, the antibody or antigen-binding fragment thereof according to the present disclosure comprises the following heavy chain variable region (VH) and/or light chain variable region (VL), wherein the CDR is defined according to Kabat numbering system:
(a) the heavy chain variable region (VH) comprising the following 3 CDRs: HCDR1 consisting of the sequence of SEQ ID NO: 7 or a sequence having one or more amino acid substitutions, deletions or additions (e.g. 1, 2 or 3 amino acid substitutions, deletions or additions) compared with SEQ ID NO: 7; HCDR2 consisting of the sequence of SEQ ID NO: 8 or a sequence having one or more amino acid substitutions, deletions or additions (e.g. 1, 2 or 3 amino acid substitutions, deletions or additions) compared with SEQ ID NO: 8; HCDR3 consisting of the sequence of SEQ ID NO: 9 or a sequence having one or more amino acid substitutions, deletions or additions (e.g. 1, 2 or 3 amino acid substitutions, deletions or additions) compared with SEQ ID NO: 9; and/or,
   the light chain variable region (VL) comprising the following 3 CDRs: LCDR1 consisting of the sequence of SEQ ID NO: 17 or a sequence having one or more amino acid substitutions, deletions or additions (e.g. 1, 2 or 3 amino acid substitutions, deletions or additions) compared with SEQ ID NO: 17; LCDR2 consisting of the sequence of SEQ ID NO:18 or a sequence having one or more amino acid substitutions, deletions or additions (e.g. 1, 2 or 3 amino acid substitutions, deletions or additions) compared with SEQ ID NO:18; LCDR3 consisting of the sequence of SEQ ID NO: 19 or a sequence having one or more amino acid substitutions, deletions or additions (e.g. 1, 2 or 3 amino acid substitutions, deletions or additions) compared with SEQ ID NO: 19;
(b) the heavy chain variable region (VH) comprising the following 3 CDRs: HCDR1 consisting of the sequence of SEQ ID NO: 27 or a sequence having one or more amino acid substitutions, deletions or additions (e.g. 1, 2 or 3 amino acid substitutions, deletions or additions) compared with SEQ ID NO: 27; HCDR2 consisting of the sequence of SEQ ID NO: 28 or a sequence having one or more amino acid substitutions, deletions or additions (e.g. 1, 2 or 3 amino acid substitutions, deletions or additions) compared with SEQ ID NO: 28; HCDR3 consisting of the sequence of SEQ ID NO: 29 or a sequence having one or more amino acid substitutions, deletions or additions (e.g. 1, 2 or 3 amino acid substitutions, deletions or additions) compared with SEQ ID NO: 29; and/or,
   the light chain variable region (VL) comprising the following 3 CDRs: LCDR1 consisting of the sequence of SEQ ID NO: 37 or a sequence having one or more amino acid substitutions, deletions or additions (e.g. 1, 2 or 3 amino acid substitutions, deletions or additions) compared with SEQ ID NO: 37; LCDR2 consisting of the sequence of SEQ ID NO:38 or a sequence having one or more amino acid substitutions, deletions or additions (e.g. 1, 2 or 3 amino acid substitutions, deletions or additions) compared with SEQ ID NO:38; LCDR3 consisting of the sequence of SEQ ID NO: 39 or a sequence having one or more amino acid substitutions, deletions or additions (e.g. 1, 2 or 3 amino acid substitutions, deletions or additions) compared with SEQ ID NO: 39;
(c) the heavy chain variable region (VH) comprising the following 3 CDRs: HCDR1 consisting of the sequence of SEQ ID NO: 7 or a sequence having one or more amino acid substitutions, deletions or additions (e.g. 1, 2 or 3 amino acid substitutions, deletions or additions) compared with SEQ ID NO: 7; HCDR2 consisting of the sequence of SEQ ID NO: 8 or a sequence having one or more amino acid substitutions, deletions or additions (e.g. 1, 2 or 3 amino acid substitutions, deletions or additions) compared with SEQ ID NO: 8; HCDR3 consisting of the sequence of SEQ ID NO: 9 or a sequence having one or more amino acid substitutions, deletions or additions (e.g. 1, 2 or 3 amino acid substitutions, deletions or additions) compared with SEQ ID NO: 9; and/or,
   the light chain variable region (VL) comprising the following 3 CDRs: LCDR1 consisting of the sequence of SEQ ID NO: 37 or a sequence having one or more amino acid substitutions, deletions or additions (e.g. 1, 2 or 3 amino acid substitutions, deletions or additions) compared with SEQ ID NO: 37; LCDR2 consisting of the sequence of SEQ ID NO:38 or a sequence having one or more amino acid substitutions, deletions or additions (e.g. 1, 2 or 3 amino acid substitutions, deletions or additions) compared with SEQ ID NO:38; LCDR3 consisting of the sequence of SEQ ID NO: 39 or a sequence having one or more amino acid substitutions, deletions or additions (e.g. 1, 2 or 3 amino acid substitutions, deletions or additions) compared with SEQ ID NO: 39;
   or
(d) the heavy chain variable region (VH) comprising the following 3 CDRs: HCDR1 consisting of the sequence of SEQ ID NO: 27 or a sequence having one or more amino acid substitutions, deletions or additions (e.g. 1, 2 or 3 amino acid substitutions, deletions or additions) compared with SEQ ID NO: 27; HCDR2 consisting of the sequence of SEQ ID NO: 28 or a sequence having one or more amino acid substitutions, deletions or additions (e.g. 1, 2 or 3 amino acid substitutions, deletions or additions) compared with SEQ ID NO: 28; HCDR3 consisting of the sequence of SEQ ID NO: 29 or a sequence having one or more amino acid substitutions, deletions or additions (e.g. 1, 2 or 3 amino acid substitutions, deletions or additions) compared with SEQ ID NO: 29; and/or, the light chain variable region (VL) comprising the following 3 CDRs: LCDR1 consisting of the sequence of SEQ ID NO: 17 or a sequence having one or more amino acid substitutions, deletions or additions (e.g. 1, 2 or 3 amino acid substitutions, deletions or additions) compared with SEQ ID NO: 17; LCDR2 consisting of the sequence of SEQ ID NO:18 or a sequence having one or more amino acid substitutions, deletions or additions (e.g. 1, 2 or 3 amino acid substitutions, deletions or additions) compared with SEQ ID NO:18; LCDR3 consisting of the sequence of SEQ ID NO: 19 or a sequence having one or more amino acid substitutions, deletions or additions (e.g. 1, 2 or 3 amino acid substitutions, deletions or additions) compared with SEQ ID NO: 19.

In certain embodiments, the antibody or antigen-binding fragment thereof according to the present disclosure comprises the following heavy chain variable region (VH) and/or light chain variable region (VL), wherein the CDR is defined according to Kabat numbering system:
(a) the heavy chain variable region (VH) comprising the following 3 CDRs: HCDR1 consisting of the sequence of SEQ ID NO: 7, HCDR2 consisting of the sequence of SEQ ID NO: 8, HCDR3 consisting of the sequence of SEQ ID NO: 9; and/or
   the light chain variable region (VL) comprising the following 3 CDRs: LCDR1 consisting of the sequence of SEQ ID NO: 17, LCDR2 consisting of the sequence SEQ ID NO:18, and LCDR3 consisting of the sequence of SEQ ID NO: 19;
(b) the heavy chain variable region (VH) comprising the following 3 CDRs: HCDR1 consisting of the sequence of SEQ ID NO: 27, HCDR2 consisting of the sequence of SEQ ID NO: 28, HCDR3 consisting of the sequence of SEQ ID NO: 29; and/or
   the light chain variable region (VL) comprising the following 3 CDRs: LCDR1 consisting of the sequence of SEQ ID NO: 37, LCDR2 consisting of the sequence SEQ ID NO:38, and LCDR3 consisting of the sequence of SEQ ID NO: 39;
(c) the heavy chain variable region (VH) comprising the following 3 CDRs: HCDR1 consisting of the sequence of SEQ ID NO: 7, HCDR2 consisting of the sequence of SEQ ID NO: 8, HCDR3 consisting of the sequence of SEQ ID NO: 9; and/or
   the light chain variable region (VL) comprising the following 3 CDRs: LCDR1 consisting of the sequence of SEQ ID NO: 37, LCDR2 consisting of the sequence SEQ ID NO:38, and LCDR3 consisting of the sequence of SEQ ID NO: 39;
   or
(d) the heavy chain variable region (VH) comprising the following 3 CDRs: HCDR1 consisting of the sequence of SEQ ID NO: 27, HCDR2 consisting of the sequence of SEQ ID NO: 28, HCDR3 consisting of the sequence of SEQ ID NO: 29; and/or
the light chain variable region (VL) comprising the following 3 CDRs: LCDR1 consisting of the sequence of SEQ ID NO: 17, LCDR2 consisting of the sequence SEQ ID NO:18, and LCDR3 consisting of the sequence of SEQ ID NO: 19.

In certain embodiments, the antibody or antigen binding fragment thereof of the present disclosure comprises a heavy chain variable region (VH) and/or a light chain variable region (VL) wherein at least one CDR of the heavy chain variable region (VH) and/or light chain variable region (VL) contains a mutation, compared with the CDRs defined by the aforementioned IMGT, chothia or Kabat, wherein said mutation(s) are one or more amino acid substitutions, deletions or additions or any combination thereof (1, 2 or 3 amino acid substitutions, deletions or additions or any combination thereof).

Preferably, the substitution mentioned in the present disclosure is a conservative substitution.

In certain embodiments, the VH of the antibody or antigen-binding fragment thereof of the present disclosure comprises a framework region (FR) derived from the heavy chain variable region (VH) of human immunoglobulin, and/or the VL of the antibody or antigen-binding fragment thereof comprises a framework region (FR) derived from the light chain variable region (VL) of human immunoglobulin. Thus, in certain embodiments, the antibodies or antigen-binding fragments thereof of the present disclosure are humanized. In certain embodiments, the antibodies or an antigen-binding fragment thereof of the present disclosure are fully humanized.

In certain embodiments, the VH of the antibody or antigen-binding fragment thereof of the present disclosure comprises a framework region (FR) derived from the heavy chain variable region (VH) of human immunoglobulin, and/or the VL of the antibody or antigen-binding fragment thereof comprises a framework region (FR) derived from the light chain variable region (VL) of human immunoglobulin. Thus, in certain embodiments, the antibodies or antigen-binding fragments thereof of the present disclosure are humanized. In certain embodiments, the antibodies or an antigen-binding fragment thereof of the present disclosure are fully humanized.

In certain embodiments, the antibody or antigen-binding fragment thereof of the present disclosure comprises:
(a) the heavy chain framework region of human immunoglobulins or variants thereof, the variants have up to conservative substitutions of 20 amino acids (e.g., conservative substitutions of up to 20, up to 15, up to 10 or up to 5 amino acids; e.g., conservative substitutions of 1, 2, 3, 4, 5, 6, 7, 8, 9 or 10 amino acids), compared with the amino acid sequence encoded by the germline antibody gene from which it is derived; and/or
(b) the light chain framework region of human immunoglobulins or variants thereof, the variants have up to conservative substitutions of 20 amino acid (e.g., conservative substitutions of up to 20, up to 15, up to 10 or up to 5 amino acids; e.g., conservative substitutions of 1, 2, 3, 4, 5, 6, 7, 8, 9 or 10 amino acid), compared with the amino acid sequence encoded by the germline antibody gene from which it is derived

In certain embodiments, the humanized degree of the antibody or antigen-binding fragment thereof of the present disclosure is at least 75%, at least 80%, at least 85%, at least 90%, at least 91%, at least 92%, at least 93%, at least 94%, at least 95%, at least 96%, at least 97%, at least 98% or at least 99%.

In certain embodiments, the antibody or antigen-binding fragment thereof of the present disclosure comprises:
(a) the heavy chain variable region (VH), which contains an amino acid sequence selected from the following:
   (i) a sequence set forth in SEQ ID NO:3 or 23;
   (ii) a sequence having one or more substitutions, deletions or additions or any combination thereof (e.g., 1, 2, 3, 4, 5, 6, 7, 8, 9 or 10 amino acid substitutions, deletions or additions or any combination thereof) compared with the sequence set forth in SEQ ID NO:3 or 23; or
   (iii) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared with the sequence set forth in SEQ ID NO:3 or 23;
   and/or
(b) the light chain variable region (VL), which contains an amino acid sequence selected from the following:
   (iv) a sequence set forth in SEQ ID NO: 13 or 33;
   (v) a sequence having one or more substitutions, deletions or additions or any combination thereof (e.g., 1, 2, 3, 4, 5, 6, 7, 8, 9 or 10 amino acid substitutions, deletions or additions or any combination thereof) compared with the sequence set forth in SEQ ID NO: 13 or 33; or
   (vi) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared with the sequence set forth in SEQ ID NO: 13 or 33.

In certain embodiments, the antibody or antigen-binding fragment thereof of the present disclosure comprises the VH set forth in SEQ ID NO: 3 and/or the VL set forth in SEQ ID NO: 13.

In certain embodiments, the antibody or antigen-binding fragment thereof of the present disclosure comprises the VH set forth in SEQ ID NO: 23 and/or the VL set forth in SEQ ID NO: 33.

In certain embodiments, the antibody or antigen-binding fragment thereof of the present disclosure comprises the VH set forth in SEQ ID NO: 3 and/or the VL set forth in SEQ ID NO: 33.

In certain embodiments, the antibody or antigen-binding fragment thereof of the present disclosure comprises the VH set forth in SEQ ID NO: 23 and/or the VL set forth in SEQ ID NO: 13.

In certain embodiments, the antibody or antigen-binding fragment thereof of the present disclosure comprises:
(a) the VH set forth in SEQ ID NO: 3 and the VL set forth in SEQ ID NO: 13;
(b) the VH set forth in SEQ ID NO: 23 and the VL set forth in SEQ ID NO: 33;
(c) the VH set forth in SEQ ID NO: 3 and the VL set forth in SEQ ID NO: 33;
(d) the VH set forth in SEQ ID NO: 23 and the VL set forth in SEQ ID NO: 13.
(e) a heavy chain variable region (VH) and/or a light chain variable region (VL), wherein the heavy chain variable region (VH) and the light chain variable region (VL) have at least 70%, at least 80%, at least 85%, at least 90%, at least 91%, at least 92%, at least 93%, at least 94%, at least 95%, at least 96%, at least 97%, at least 98% or at least 99% sequence identity respectively, independently compared with the VH and VL in anyone of (a) to (f); or
(f) a heavy chain variable region (VH) and/or a light chain variable region (VL), wherein the heavy chain variable region (VH) and the light chain variable region (VL) independently have one or more amino acid substitutions, deletions or additions or any combination thereof (e.g. 1, 2, 3, 4, 5, 6, 7, 8, 9 or 10 amino acid substitutions, deletions or additions or any combination thereof), compared with the VH and VL in anyone of (a) to (d), respectively. Preferably, the substitution(s) are conservative substitution(s).

In certain embodiments, the antibody or an antigen-binding fragment thereof of the present disclosure comprises a heavy chain constant region (CH) of a human immunoglobulin or a variant thereof, and the variant has up to conservative substitutions of 50 amino acids (e.g., conservative substitutions of up to 45, up to 40, up to 35, up to 30, up to 25, up to 20, up to 15, up to 10 or up to 5 amino acids; e.g., conservative substitutions of 1, 2, 3, 4, 5, 6, 7, 8, 9 or 10 amino acids), compared with the wild type sequence from which it is derived. In certain embodiments, the antibody or an antigen-binding fragment thereof of the present disclosure comprises a light chain constant region (CL) of a human immunoglobulin or a variant thereof, and the variant has up to conservative substitutions of 50 amino acids (e.g., conservative substitutions of up to 45, up to 40, up to 35, up to 30, up to 25, up to 20, up to 15, up to 10 or up to 5 amino acids; e.g., conservative substitutions of 1, 2, 3, 4, 5, 6, 7, 8, 9 or 10 amino acids), compared with the wild type sequence from which it is derived.

In some embodiments, the constant region is altered, e.g., mutated, to modify the properties of the anti-B7H3 antibody molecule (e.g., to alter one or more of the following properties: Fc receptor binding, antibody glycosylation, number of cysteine residues, effector cell function, or complement function). Functional changes can be carried out by substituting at least one amino acid residue in the constant region of the antibody with a different residue, for example, changing the affinity of the antibody for effector ligands (e.g., FcR or complement C1q), thereby changing effector function (e.g., decreasing). The Fc region of the antibody mediates several important effector functions, such as ADCC, phagocytosis (ADCP), CDC, etc.

In some embodiments, the antibody or antigen-binding fragment thereof of the present disclosure has a heavy chain constant region (CH), which is selected from the heavy chain constant regions of e.g. IgG1, IgG2, IgG3, IgG4, IgM, IgA1, IgA2, IgD, and IgE; particularly from the heavy chain constant regions of e.g. IgG1, IgG2, IgG3, and IgG4; and more particularly from the heavy chain constant region of IgG1 (e.g. human IgG1). In some embodiments, the heavy chain constant region of human IgG1 has a sequence set forth in SEQ ID NO:43. In some embodiments, the antibody or antigen-binding fragment thereof of the present disclosure has a light chain constant region, which is selected from, for example, κ or λ light chain constant region, preferably κ light chain constant region (e.g., human κ light chain constant region). In some embodiments, the light chain constant region has a sequence set forth in SEQ ID NO:44.

In some embodiments, the antibody or antigen-binding fragment thereof has a CH set forth in SEQ ID NO 43 or a variant thereof, said variant has conservative substitutions of up to 20 amino acids (such as conservative substitutions of up to 20, up to 15, up to 10 or up to 5 amino acids, such as conservative substitutions of 1, 2, 3, 4, 5, 6, 7, 8, 9 or 10 amino acids), compared with SEQ ID NO: 43; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 43.

In some embodiments, the antibody or antigen-binding fragment thereof has a light chain constant region or a variant thereof. In some embodiments, the light chain constant region comprises κ light chain constant region. In some embodiments, the light chain constant region comprises a light chain constant region (CL) set forth in SEQ ID NO:44 or a variant thereof, said variant has conservative substitutions of up to 20 amino acids (such as conservative substitutions of up to 20, up to 15, up to 10 or up to 5 amino acids, such as conservative substitutions of 1, 2, 3, 4, 5, 6, 7, 8, 9 or 10 amino acids), compared with SEQ ID NO: 44; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 16;

In some embodiments, the antibody or antigen-binding fragment thereof comprises the heavy chain constant region (CH) set forth in SEQ ID NO: 43 and the light chain constant region (CL) set forth in SEQ ID NO: 44.

In some embodiments, the antibody or antigen-binding fragment thereof of the present disclosure comprises:
(a) a heavy chain, which comprises an amino acid sequence selected from the following:
   (i) a sequence comprising the VH sequence set forth in SEQ ID NO: 3 and the CH sequence set forth in SEQ ID NO: 43;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
(b) a light chain, which comprises an amino acid sequence selected from the following:
   (iv) a sequence comprising the VL sequence set forth in SEQ ID NO: 13 and the CL sequence set forth in SEQ ID NO: 44;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

In some embodiments, the substitution(s) described in (ii) or (v) are conservative substitution(s).

In some embodiments, the antibody or antigen-binding fragment thereof of the present disclosure comprises:
(a) a heavy chain, which comprises an amino acid sequence selected from the following:
   (i) a sequence comprising the VH sequence set forth in SEQ ID NO: 23 and the CH sequence set forth in SEQ ID NO: 43;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
(b) a light chain, which comprises an amino acid sequence selected from the following:
   (iv) a sequence comprising the VL sequence set forth in SEQ ID NO: 33 and the CL sequence set forth in SEQ ID NO: 44;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

In some embodiments, the substitution(s) described in (ii) or (v) are conservative substitution(s).

In some embodiments, the antibody or antigen-binding fragment thereof of the present disclosure comprises a heavy chain and a light chain,
The heavy chain comprises:
   (i) a sequence set forth in SEQ ID NO: 45;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
the light chain comprises:
   (iv) a sequence set forth in SEQ ID NO: 46;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv);
preferably, the substitution(s) described in (ii) or (v) are conservative substitution(s).

In some embodiments, the antibody or antigen-binding fragment thereof of the present disclosure comprises a heavy chain and a light chain,
the heavy chain comprises:
   (i) a sequence set forth in SEQ ID NO: 47;
   (ii) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
the light chain comprises:
   (iv) a sequence set forth in SEQ ID NO: 48;
   (v) a sequence having one or more amino acid substitutions, deletions or additions or any combination thereof (e.g. up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv);
preferably, the substitution(s) described in (ii) or (v) are conservative substitution(s).

In some embodiments, the antibody or antigen-binding fragment thereof of the present disclosure is a chimeric, humanized, or fully human antibody. In some embodiments, the antibody or antigen-binding fragment thereof of the present disclosure is selected from scFv, Fab, Fab', (Fab')2, Fv fragments, disulfide-linked Fv(dsFv), and diabody.

In some embodiments, the antibody of the present disclosure is scFv. In certain embodiments, the scFv of the present disclosure comprises:
(a) a VH set forth in SEQ ID NO:3 and a VL set forth in SEQ ID NO:13;
(b) a VH set forth in SEQ ID NO:23 and a VL set forth in SEQ ID NO:33;
(c) a VH set forth in SEQ ID NO:3 and a VL set forth in SEQ ID NO:33;
(d) a VH set forth in SEQ ID NO:23 and a VL set forth in SEQ ID NO:13;
(e) a heavy chain variable region (VH) and a light chain variable region (VL), wherein the heavy chain variable region (VH) and the light chain variable region (VL) have at least 70%, at least 80%, at least 85%, at least 90%, at least 91%, at least 92%, at least 93%, at least 94%, at least 95%, at least 96%, at least 97%, at least 98%, or at least 99% sequence identity respectively, independently compared with the VH and VL in any one of the groups (a) to (d); or
(f) a heavy chain variable region (VH) and a light chain variable region (VL), wherein the heavy chain variable region (VH) and the light chain variable region (VL) independently have one or more amino acid substitutions, deletions or additions or any combination thereof (such as 1, 2, 3, 4, 5, 6, 7, 8, 9 or 10 amino acid substitutions, deletions or additions or any combination thereof) compared with the VH and VL in any one of the groups (a) to (d) respectively. Preferably, the substitution(s) are conservative substitution(s).

In some embodiments, the antibody of the present disclosure is scFv. In certain embodiments, the scFv of the present disclosure comprises:
(i) a sequence set forth in SEQ ID NO:1 or 2;
(ii) a sequence having one or more amino acid substitutions, deletions or additions or any combination thereof (e.g. up to 50, up to 45, up to 40, up to 35, up to 30, up to 25, up to 20, up to 15, up to 10 or up to 5 amino acid substitutions, deletions or additions or any combination thereof; such as 1, 2, 3, 4, 5, 6, 7, 8, 9 or 10 amino acid substitutions, deletions or additions or any combination thereof) compared with the sequence set forth in (i); or
(iii) a sequence having at least 80%, at least 85%, at least 90%, at least 91%, at least 92%, at least 93%, at least 94%, at least 95%, at least 96%, at least 97%, at least 98%, or at least 99% sequence identity to the sequence set forth in (i);
preferably, the substitution(s) described in (ii) are conservative substitution(s).

In some embodiments, the antibody of the present disclosure is scFv.

In some embodiments, the scFv of the present disclosure comprises a sequence set forth in SEQ ID NO:1 or 2.

### Antibody derivatives

An antibody or antigen-binding fragment thereof of the present disclosure may be derivatized, for example, linked to another molecule (e.g., another polypeptide or protein). In general, derivatization (e.g., labeling) of an antibody or antigen-binding fragment thereof does not adversely affect its binding to B7H3 (particularly human B7H3). Thus, the antibody or antigen-binding fragment thereof of the present disclosure is also intended to include such derivatized forms. For example, an antibody or antigen-binding fragment thereof of the present disclosure may be linked (by chemical coupling, gene fusion, non-covalent linking, or otherwise) to one or more other molecular groups, such as another antibody (e.g., to form a bispecific antibody), a detection agent, a pharmaceutical agent and/or a protein or polypeptide capable of mediating the binding of an antibody or antigen-binding fragment thereof to another molecule (e.g. avidin or polyhistidine tag).

One type of derivatized antibody (e.g., bispecific antibody) is produced by cross-linking 2 or more antibodies (belonging to the same or different types). Methods for obtaining bispecific antibodies are well known in the art, examples of which include, but are not limited to, chemical crosslinking method, cell engineering method (hybridoma method), or genetic engineering method.

Another type of derivatized antibody is the labeled antibody. For example, an antibody or antigen-binding fragment thereof of the present disclosure may be attached to a detectable marker. The detectable marker described in the present disclosure may be any substance detectable by fluorescence, spectroscopic, photochemical, biochemical, immunological, electrical, optical, or chemical means. Such markers are well known in the art and examples include, but are not limited to, enzymes (e.g., horseradish peroxidase, alkaline phosphatase, β-galactosidase, urease, glucose oxidase, etc.), radionuclides (e.g., 3H, 125I, 35S, 14C, or 32P), fluorescent dyes (e.g., fluorescein isothiocyanate (FITC), fluorescein, tetramethylrhodamine isothiocyanate (TRITC), phycoerythrin (PE), Texas red, rhodamine, quantum dots or cyanine dye derivatives (e.g. Cy7, Alexa 750)), acridines esters, magnetic beads (e.g., Dynabeads^{®}), calorimetric markers such as colloidal gold or colored glass or plastic (e.g., polystyrene, polypropylene, latex, etc.) beads, and biotin for binding avidin (e.g., streptavidin) modified by the above marker(s). Patents that teach the use of the marker include, but are not limited to, U.S. patent No.3,817,837; 3,850,752; 3,939,350; 3,996,345; 4,277,437; 4,275,149; and 4,366,241 (which are all hereby incorporated by reference in their entirety). Detectable markers as described above can be detected by methods known in the art. For example, radioactive markers can be detected using photographic film or a scintillation counter, while fluorescent markers can be detected using a photodetector to detect emitted light. Enzyme markers are generally detected by supplying the substrate to the enzyme and detecting the reaction products produced by the enzyme's action on the substrate, while calorimetric markers are detected by simple visualization of colored markers. In certain embodiments, such labeling can be suitable for immunological detection (e.g., enzyme-linked immunoassay, radioimmunoassay, fluorescence immunoassay, chemiluminescent immunoassay, etc.). In certain embodiments, the detectable marker as described above can be attached to the antibody or antigen-binding fragment thereof of the present disclosure by linkers having different lengths, to reduce potential steric hindrance.

In addition, the antibody or antigen-binding fragment thereof of the present disclosure can also be derivatized with chemical groups, such as polyethylene glycol (PEG), methyl or ethyl, or glycosyl. These groups can be used to improve the biological properties of antibodies, such as increasing serum half-life.

The antigen-binding fragment of the present disclosure can be obtained by hydrolyzing intact antibody molecules (c.f., Morimoto et al., J. Biochem. Biophys. Methods 24: 107-117 (1992) and Brennan et al., Science 229: 81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab, or F(ab')₂ fragments can also be directly isolated from recombinant host cell cultures. Other techniques for preparing these antigen-binding fragments are fully known to those of ordinary skill in the art.

The IgG isotype control antibodies of the present disclosure are fully known to one of ordinary skill in the art and can be purchased or prepared. For example, the sequence of human anti-Hen Egg Lysozyme IgG (anti-HEL, such as human IgG1, abbreviated as hIgG1) are derived from the variable region of Fab F10.6.6 sequence in "A affinity maturation increases the stability and plasticity of the Fv domain of anti-protein antibodies" published by Acierno et al. (Acierno et al. J Mol Biol. 2007; 374(1): 130-46). The preparation method is as follows: the amino acid codon optimization and gene synthesis of the heavy and light chain (full sequence or variable region) gene of human IgG antibody are performed by Nanjing GenScript Biotech Corp., according to the standard technology introduced in the "Molecular Cloning, A Laboratory Manual (3rd Edition)". The heavy and light chain genes were respectively subcloned into antibody heavy chain expression vector and antibody light chain expression vector of mammalian expression system by standard molecular cloning techniques such as PCR, enzyme digestion, DNA gel recovering, ligation transformation, colony PCR or enzyme digestion identification, and the heavy and light chain genes of the recombinant expression vector were further sequenced. After the sequence was verified correctly by sequencing, a large number of expression plasmids of endotoxin-free grade were prepared and then the weight and light chain expression plasmids were transiently cotransfected into HEK293 cells for recombinant antibody expression. After 7 days of culture, the cell culture was collected and purified by rProtein A affinity column (GE). The quality of the harvested antibody samples was determined by SDS-PAGE and SEC-HPLC standard analytical techniques.

In a ninth aspect, the present disclosure provides a multi-specific antibody comprising the antibody or antigen-binding fragment thereof described in any item of the eighth aspects of the present disclosure, and additional antibody or fragment thereof or antibody mimic.

In certain embodiments, the multi-specific antibody is a bispecific antibody or a tri-specific antibody or a tetra-specific antibody.

Thus, in a tenth aspect, the present disclosure provides an isolated nucleic acid molecule which comprises a nucleotide sequence encoding an antibody or antigen-binding fragment thereof, or a heavy and/or light chain variable region thereof, or one or more CDRs thereof according to the present disclosure. In certain embodiments, the nucleotide sequence may be substituted according to codon degeneracy, as is known in the art. In certain embodiments, the nucleotide sequence is codon-optimized.

In certain embodiments, the isolated nucleic acid molecule of the present disclosure comprises: (i) a first nucleic acid and a second nucleic acid encoding a heavy chain variable region and a light chain variable region, respectively, of an antibody or antigen binding fragment thereof of the present disclosure, or (ii) a first nucleic acid and a second nucleic acid encoding a heavy chain variable region and a heavy chain constant region, ,as well as a light chain variable region and a light chain constant region respectively, of an antibody or antigen binding fragment thereof of the present disclosure, or (iii) a first nuclei acid and a second nucleic acid encoding the heavy and light chains, respectively, of the antibody or antigen-binding fragment thereof of the present disclosure. In certain embodiments, the first nucleic acid and the second nucleic acid comprises a nucleic acid that is the degenerate sequence of or substantially identical to the sequence of the first and second nucleic acids in any of items (i)-(iii) above. In certain embodiments, the degenerate sequence or substantially identical sequence refers to a sequence having at least about 85%, 90%, 95%, or greater sequence identity or having one or more nucleotide substitutions, or having a difference in no more than 3, 6, 15, 30 or 45 nucleotides, compared with the nucleic acid molecules set forth in items (i)-(iii).

In an eleventh aspect, a vector (e.g., a cloning vector or an expression vector) comprising an isolated nucleic acid molecule of the present disclosure is provided. In certain embodiments, the vectors of the present disclosure are, for example, plasmids, Cosmids, bacteriophages, lentiviruses, etc. In certain embodiments, the vector is capable of expressing an antibody or antigen-binding fragment thereof of the present disclosure *in vivo* in a subject (e.g., a mammal, such as a human).

In a twelfth aspect, there is provided a host cell comprising an isolated nucleic acid molecule of the present disclosure or a vector of the present disclosure. The host cell may be a eukaryotic cell (e.g., mammalian cell, insect cell, yeast cell) or a prokaryotic cell (e.g., *Escherichia coli*)*.* Suitable eukaryotic cells include, but are not limited to, NS0 cells, Vero cells, Hela cells, COS cells, CHO cells, HEK293 cells, BHK cells, and MDCKII cells. Suitable insect cells include, but are not limited to, Sf9 cells. In certain embodiments, the host cell of the present disclosure is a mammalian cell, such as CHO (e.g., CHO-K1, CHO-S, CHO DXB11, CHO DG44).

In a thirteenth aspect, there is provided a method of preparing an antibody or antigen-binding fragment thereof, or a multi-specific antibody according to the present disclosure, comprising culturing host cells of the present disclosure under conditions that allow expression of the antibody or antigen-binding fragment thereof, or a multi-specific antibody, and recovering the antibody or antigen-binding fragment thereof from the cultured host cell culture.

In a fourteenth aspect, an antibody-drug conjugate is provided, wherein the antibody is the above-mentioned anti-B7H3 antibody, and said antibody is linked by a linker to a coupling moiety selected from: a detectable marker, a radioisotope, a fluorescent agent, a luminescent agent, a colored agent, an enzyme, polyethylene glycol (PEG), a nuclide, a nucleic acid, a small molecule toxin, a polypeptide having binding activity, a protein, a receptor, a ligand, and other active agent that inhibits tumor cell growth, and promotes tumor cell apoptosis or necrosis.

In certain examples, an antibody-drug conjugate comprises the anti-B7H3 antibody drug conjugate (B7H3-ADC) of the following formula:

Ab-(L-D)n,

wherein:
Ab is the antibody or antigen-binding fragment thereof in the eighth aspect, and;
D is a small molecule toxin drug moiety;
L is a bond or linker which covalently links Ab and D;
n is an integer between 1 and 16, and represents the number of L-D covalently linked to Ab.

In some examples, L in the antibody-drug conjugate is selected from an amino acid residue or short peptides consisting of 2-10 amino acid residues; the amino acid residues are selected from natural amino acid residues, non-natural amino acid residues, and amino acid residue represented by AA¹ or stereoisomer thereof; 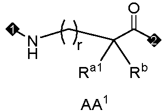 in the amino acid residues represented by AA¹:
R^{a1} and R^{b} are each independently selected from H, 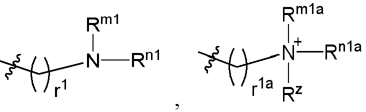 and 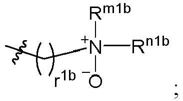
or, R^{a1} and R^{b} together with the carbon atom to which they are both attached form a 4-10 membered heterocyclic ring, in said 4-10 membered heterocyclic ring, the heteroatom is selected from one, two, three or four O, N and S; and said 4-10 membered heterocyclic ring is optionally substituted with one or more R⁰;
r, r¹, r^{1a} and r^{1b} are each independently selected from any integer from 0 to 20;
R^{m1}, Rⁿ¹, R^{m1a}, R^{n1a}, R^{m1b} and R^{n1b} are each independently selected from H, C₁₋₆ alkyl, C₃₋₆ cycloalkyl and-COOR^{x1}, wherein R^{x1} is C₁₋₆ alkyl;
or, R^{m1} and Rⁿ¹, R^{m1a} and R^{n1a}, and R^{m1b} and R^{n1b}, together with the nitrogen atom to which they are both attached, form a 4-10 membered heterocyclic ring, in said 4-10 membered heterocyclic ring the heteroatom is selected from one, two, three or four O, N and S; said 4-10 membered heterocyclic ring is optionally substituted with one or more R^{0'};
R^{z} is selected from C₁₋₆ alkyl;
R⁰, R^{0'} are each independently selected from C₁₋₆ alkyl, C₃₋₆ cycloalkyl, -NR^{m2}Rⁿ² and 4-10 membered heterocyclyl optionally substituted with C₁₋₆ alkyl; in said 4-10 membered heterocyclic ring, the heteroatom is selected from one, two, three or four O, N and S;
R^{m2}, Rⁿ² are each independently selected from H and C₁₋₆ alkyl.

In some embodiments, in the amino acid residues represented by AA¹, R^{a1} and R^{b} together with the carbon atom to which they are both attached form a 5-6 membered heterocyclic ring, in which the heteroatom is N, and the number of heteroatoms is 1 or 2; said 5-6 membered heterocyclic ring is optionally substituted with one or more R⁰. Said 5-6 membered heterocycle is preferably a piperidine ring or a piperazine ring, and further preferably a piperidine ring, e.g. 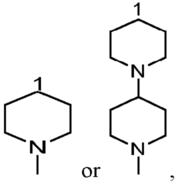 and the carbon atom marked with number 1 is the carbon atom to which R^{a1} and R^{b} are both attached.

In some embodiments, r, r¹, r^{1a} and r^{1b} are each independently 0, 1, 2, 3, 4, or 5; preferably, r, r¹, r^{1a} and r^{1b} are each independently 0 or 4; further preferably, r is 0, r¹, r^{1a} and r^{1b}are 4; or r is 4, r¹, r^{1a} and r^{1b} are 0.

In some embodiments, r, r¹, r^{1a} and r^{1b} are not all 0.

In some embodiments, R^{m1}, Rⁿ¹, R^{m1a}, R^{n1a}, R^{m1b} and R^{n1b} are each independently H, C₁₋₆ alkyl, or -COOR^{x1}, wherein R^{x1} is C₁₋₆ alkyl; preferably, are each independently H, methyl, ethyl, n-propyl, -COOCH₃, -COOCH₂CH₃, -COOCH₂CH₂CH₃, -COOCH(CH₃)₂, -COOC(CH₃)₃ or -COOCH₂CH₂CH₂CH₃.

In some embodiments, R^{m1} and Rⁿ¹, R^{m1a} and R^{n1a}, and R^{m1b} and R^{n1b}, together with the nitrogen atom to which they are both attached, form a 5-6 membered heterocyclic ring, in which the heteroatom is selected from 1 or 2 N atoms; said 5-6 membered heterocyclic ring is optionally substituted with one or more R^{0'}; the 5-6 membered heterocycle is preferably a piperidine ring or a piperazine ring, and further preferably is 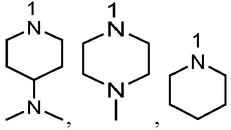 or 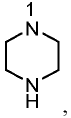 and the nitrogen atom marked with number 1 is the nitrogen atom to which R^{m1} and Rⁿ¹ are both attached.

In some embodiments, R^{z} is preferably C₁₋₆ alkyl; and more preferably methyl.

In some embodiments, R⁰ and R^{0'} are each independently C₁₋₆ alkyl, -NR^{m2}Rⁿ² or 5-6 membered heterocyclyl optionally substituted with C₁₋₆ alkyl; in said 5-6 membered heterocyclyl, the heteroatom is selected from 1 or 2 N atoms.

In some embodiments, R⁰ is preferably C₁₋₆ alkyl or 5-6 membered heterocyclyl substituted with C₁₋₆ alkyl; said 5-6 membered heterocyclyl is piperidinyl or piperazinyl, and further preferably is methyl or piperidinyl substituted with methyl; such as methyl or

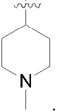

In some embodiments, each R^{01'} is independently preferably C₁₋₆ alkyl, -NR^{m2}Rⁿ² or 5-6 membered heterocyclyl optionally substituted with C₁₋₆ alkyl; in said 5-6 membered heterocyclyl, the heteroatom is selected from one or two N atoms; further preferably, each R^{01'} is C₁₋₆ alkyl or -NR^{m2}Rⁿ²; more preferably, each R^{01'} is independently methyl or -NR^{m2}Rⁿ², wherein R^{m2} and Rⁿ² are each independently preferably H or C₁₋₆ alkyl, and more preferably, are methyl.

In some embodiments, R^{0'} is preferably C₁₋₆ alkyl or -NR^{m2}Rⁿ²; R^{m2} and Rⁿ² are each independently preferably H or C₁₋₆ alkyl (such as methyl).

In some embodiments, in the amino acid residue of AA¹, one of R^{a1} and R^{b} is H, the other is 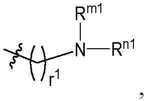 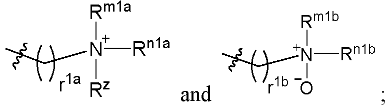 preferably, one of R^{a1} and R^{b} is H, the other is selected from

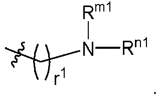

In some embodiments, the amino acid residue represented by AA¹ is preferably 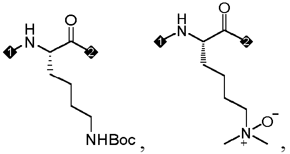 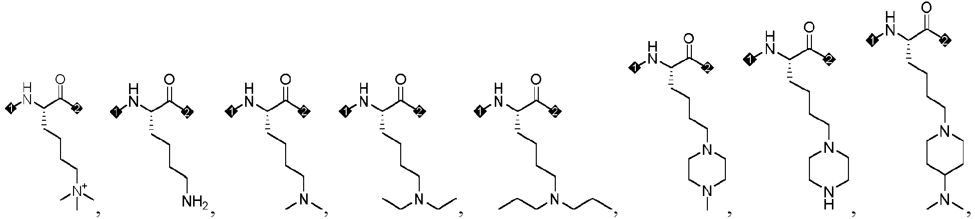 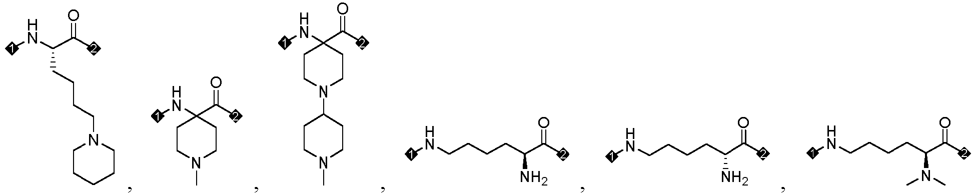 or 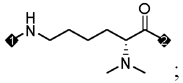 further preferably, is 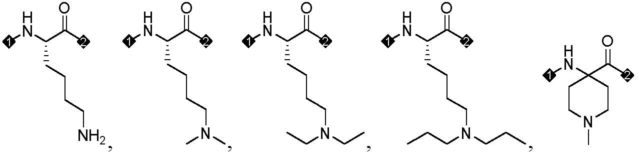 or 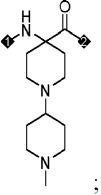 more preferably, is 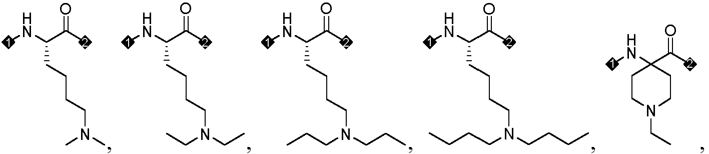 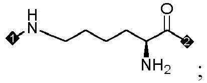 most preferably, is

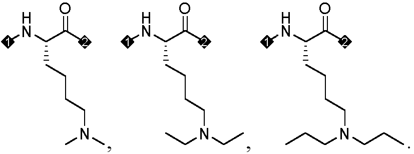

In some embodiments, the antibody-drug conjugate consists of an antibody-linker-drug, wherein the drug is selected from microtubule blocking drugs, DNA damage drugs, Bcl-xL inhibitors, etc.

In some embodiments, the antibody-drug conjugate consists of an antibody-linker-drug, wherein the drug is selected from auristatins (e.g. MMAF, MMAE), maytansine derivatives (e.g., DM1,DM4), tubulysins, cryptomycins, antimitotic inhibitors, pyrrolobenzazepines and indole chlorobenzazepines, ducamycin, camptothecin, calicheamicin, and others.

In some embodiments, the antibody-drug conjugate consists of an antibody-linker-drug, wherein the drug is selected from camptothecin (CPT) and its derivatives.

In some embodiments, the antibody-drug conjugate consists of an antibody-linker-drug, wherein the drug is selected from topoisomerase I inhibitors.

In some embodiments, the antibody-drug conjugate consists of an antibody-linker-drug, wherein the drug is selected from SN-38 and DXd.

In some embodiments, D in the antibody-drug conjugate is a drug unit represented by formula II: 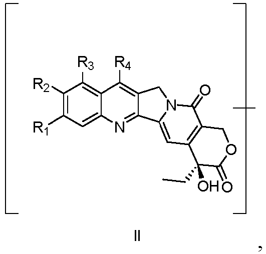 wherein,
R₁ is H or F,
R₂ is H or methyl;
or R₁ and R₂ together with the carbon atoms to which they are attached form 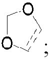
R₃ is H;
R₄ is selected from H, -(C1-C4 alkyl)-OH, -(C1-C4 alkenyl)-OH, -(C1-C4 alkyl)-NH₂ or -(C1-C4 alkenyl)-NH₂;
wherein L is linked by a hydroxyl or amine group in D.

In some embodiments, L in the antibody-drug conjugate is selected from: 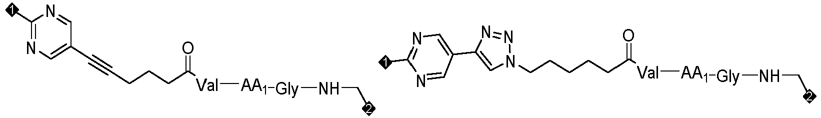 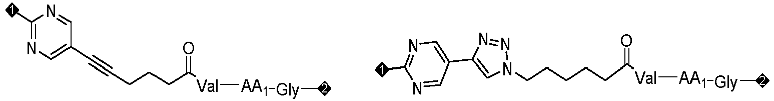 wherein, position 1 is attached to Ab, position 2 is attached to D; AA1 is as defined above.

In addition, it should also be noted and it can be understood by those skilled in the art that L is connected to the sulfhydryl group contained in Ab (such as antibody) after disulfide bonds are opened (for example, reduction of the disulfide bond by the reducing agent TCEP can break the disulfide bond to generate sulfhydryl -SH groups), that is, -S- between L and Ab is not an additional extraneous sulfur atom. For example, wherein -S- is not an additional extraneous sulfur atom, but is a -S- formed by linking the sulfhydryl group contained in Tb after disulfide bonds are opened to L.

In some preferred embodiments, the antibody-drug conjugate is selected from:

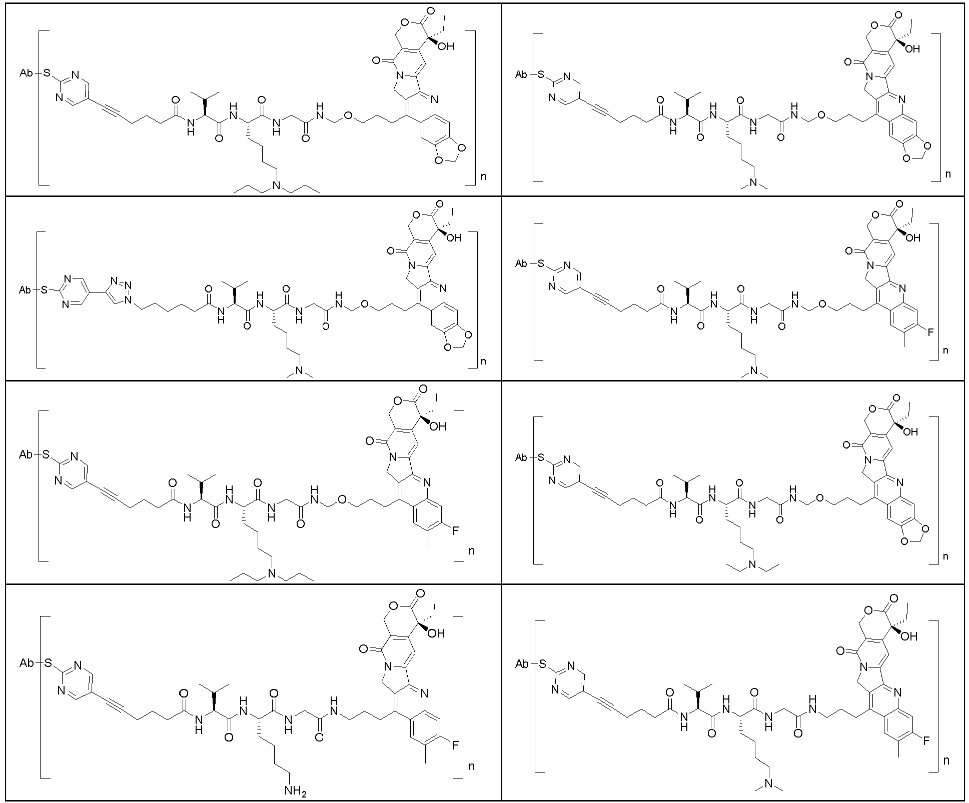

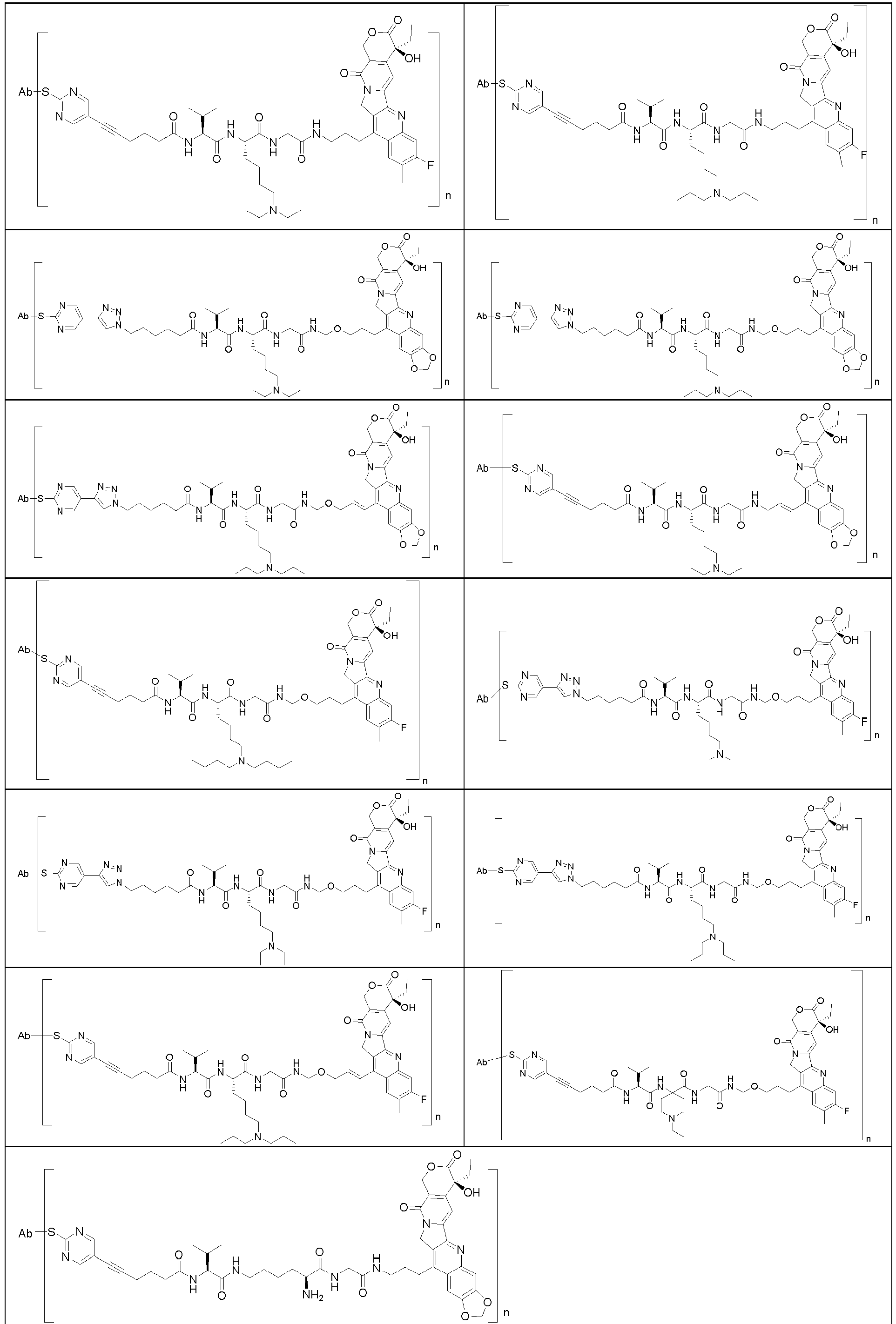

wherein Ab is anti-B7H3 antibody 1D1-01 or 2E3-02, n is 1, 2, 3, 4, 5, 6, 7, 8, 9, 10, 11, 12, 13, 14, 15, 16, and q is preferably 1, 2, 3, 4, 5, 6, 7, 8.

In a fifteenth aspect of the present disclosure, the present disclosure provides ligand drug conjugates comprising the ligand drug conjugates mentioned above or the antibody-drug conjugates mentioned above. The ligand drug conjugates have two or more q values, and the antibody-drug conjugates have two or more n values. The ligand drug conjugates refer to ligand drug conjugates or antibody-drug conjugates (ADC) comprising a heterogeneous DAR distribution.

In some embodiments, when in the ligand drug conjugates, the ligand drug conjugates having a certain q value or n value accounts for the majority, the q value or n value is close to DAR.

In some embodiments, the ratio of drug to antibody (DAR) in the ligand drug conjugates is selected from an integer or decimal between 1-10.

In some embodiments, the ratio of drug to antibody (DAR) in the ligand drug conjugates is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9 and 9.

In some embodiments, the ligand drug conjugates ADCs comprise ADCs having a DAR distribution of 1 to 8, such as 1.5, 2, 4, 6 and 8 (i.e. 1.5, 2, 4, 6 and 8 payload species). It should be noted that degradation products may be generated, such that the ligand drug conjugates may also contain DAR of 1, 3, 5, and 7. Furthermore, the ADC in the ligand drug conjugates may also have a DAR greater than 8. The ligand drug conjugate is produced by reduction of interchain disulfide followed by coupling. In some embodiments, the ligand drug conjugate comprises both the following: the ligand drug conjugate having a DAR of 4 or less (i.e., the payload species are 4 or less) and the ligand drug conjugate having a DAR of 6 or more (i.e., the payload species are 6 or more).

In another aspect, the present disclosure provides the use of an antibody or antigen-binding fragment thereof of the present disclosure in the manufacture of a kit for detecting the presence of B7H3 in a sample or its level. In another aspect, the present disclosure provides diagnostic or therapeutic kits comprising one or more of the following agents: an antibody or antigen binding fragment thereof, a nucleic acid, a vector, a host cell, a multi-specific antibody, a conjugate, or a pharmaceutical composition of the present disclosure. Optionally, the diagnostic or therapeutic kit further includes instructions for use.

In a sixteenth aspect of the present disclosure, the present disclosure provides a pharmaceutical composition comprising substance A and optionally one or more pharmaceutically acceptable adjuvants; said substance A is the ligand drug conjugate mentioned above, or a stereoisomer of the ligand drug conjugate, a prodrug thereof, a pharmaceutically acceptable salt thereof, or a pharmaceutically acceptable solvate thereof, or the aforementioned antibody or antigen-binding fragment thereof, nucleic acid molecule, vector, host cell, multi-specific antibody, and/or ligand drug conjugate, a conjugate, or the aforementioned antibody-drug conjugate, or the aforementioned compound, or the stereoisomer of said compound, prodrug thereof, pharmaceutically acceptable salt thereof or pharmaceutically acceptable solvate thereof. The substance A may be in a therapeutically effective amount.

In certain embodiments, the pharmaceutical composition of the present disclosure comprises an antibody or antigen-binding fragment thereof of the present disclosure, and a pharmaceutically acceptable carrier and/or excipient.

In certain embodiments, the pharmaceutical composition of the present disclosure comprises a host cell of the present disclosure, and a pharmaceutically acceptable carrier and/or excipient, wherein the host cell comprises an isolated nucleic acid molecule or vector as previously described.

In certain embodiments, the pharmaceutical composition of the present disclosure comprises the multi-specific antibodies of the present disclosure, and a pharmaceutically acceptable carrier and/or excipient.

In certain embodiments, the pharmaceutical composition of the present disclosure comprises the conjugates of the present disclosure and a pharmaceutically acceptable carrier and/or excipient.

In a seventeenth aspect of the present disclosure, the present disclosure provides the aforementioned substance A or the aforementioned pharmaceutical composition in the manufacture of a medicament for the treatment and/or prevention of a disease (e.g. cancer disease) associated with abnormal cell activity. Said substance A or the aforementioned pharmaceutical composition may be in a therapeutically effective amount.

In some examples, the present disclosure provides the use of an antibody or antigen-binding fragment thereof, a nucleic acid, a vector, a host cell, an antibody drug conjugate, or a multi-specific antibody according to the present disclosure in the manufacture of a medicament, and the medicament is used for modulating (inhibiting or blocking) the activity of B7H3.

In some examples, the present disclosure provides the use of an antibody or antigen-binding fragment thereof, a nucleic acid, a vector, a host cell, an antibody drug conjugate, or a multi-specific antibody according to the present disclosure in the manufacture of a medicament, and the medicament is used for the treatment or prevention of diseases related to B7H3 activity

In some examples, the present disclosure provides the use of an antibody or antigen-binding fragment thereof, a nucleic acid, a vector, a host cell, an antibody drug conjugate, or a multi-specific antibody according to the present disclosure in the manufacture of a medicament, and the medicament is used for the treatment or prevention of tumors related to B7H3 activity.

In some embodiments, the cancer disease is selected from esophageal cancer (e.g. esophageal adenocarcinoma and esophageal squamous cell carcinoma), brain tumor, lung cancer (e.g., small cell lung cancer, non-small cell lung cancer, or lung adenocarcinoma), squamous cell carcinoma, bladder cancer, stomach cancer, ovarian cancer, peritoneal cancer, pancreatic cancer, breast cancer, head and neck cancer, cervical cancer, endometrial cancer, colon cancer (e.g., human colon adenocarcinoma), rectal cancer, colorectal cancer, liver cancer, kidney cancer, urothelial cancer, epidermal cancer, non-Hodgkin lymphoma, central nervous system tumor (e.g. neuroglioma, glioblastoma multiforme, glioma or sarcoma), prostate cancer or thyroid cancer.

In some embodiments, the cancer disease is a cancer disease associated with Trop-2, Her 2, B7H3, Her 3, EGFR. In some embodiments, the cancer disease is a cancer disease associated with Trop-2 or Her 2.

In some embodiments, the cancer disease is a cancer disease associated with Trop-2.

In some embodiments, the cancer disease is a cancer disease associated with Her 3.

In some embodiments, the cancer disease is a cancer disease associated with EGFR.

In some embodiments, the cancer disease is a cancer disease associated with B7H3.

In some embodiments, the cancer disease is solid tumors.

In some embodiments, the cancer disease is breast cancer or lung cancer (preferably non-small cell lung cancer).

In a seventeenth aspect of the present disclosure, the present disclosure provides the aforementioned substance A or the aforementioned pharmaceutical composition, which is used for the treatment and/or prevention of diseases associated with abnormal cell activity (e.g., cancer diseases).

In some embodiments, the cancer disease is selected from esophageal cancer (e.g. esophageal adenocarcinoma and esophageal squamous cell carcinoma), brain tumor, lung cancer (e.g., small cell lung cancer, non-small cell lung cancer, or lung adenocarcinoma), squamous cell carcinoma, bladder cancer, stomach cancer, ovarian cancer, peritoneal cancer, pancreatic cancer, breast cancer, head and neck cancer, cervical cancer, endometrial cancer, colon cancer (e.g., human colon adenocarcinoma), rectal cancer, colorectal cancer, liver cancer, kidney cancer, urothelial cancer, epidermal cancer, non-Hodgkin lymphoma, central nervous system tumor (e.g. neuroglioma, glioblastoma multiforme, glioma or sarcoma), prostate cancer or thyroid cancer.

In some embodiments, the cancer disease is a cancer disease associated with Trop-2, Her 2, B7H3, Her 3, EGFR. In some embodiments, the cancer disease is a cancer disease associated with Trop-2 or Her 2.

In some embodiments, the cancer disease is a cancer disease associated with Trop-2.

In some embodiments, the cancer disease is a cancer disease associated with Her 3.

In some embodiments, the cancer disease is a cancer disease associated with EGFR.

In some embodiments, the cancer disease is a cancer disease associated with B7H3.

In some embodiments, the cancer disease is solid tumors. In some embodiments, the cancer disease is breast cancer or lung cancer (preferably non-small cell lung cancer).

In an eighteenth aspect of the present disclosure, the present disclosure provides a method of preventing and/or treating a disease associated with abnormal cell activity (e.g. cancer diseases), which comprises: administering to an individual in need thereof a prophylactic and/or therapeutically effective amount of the aforementioned substance A or the aforementioned pharmaceutical composition.

In some embodiments, the cancer disease is selected from esophageal cancer (e.g. esophageal adenocarcinoma and esophageal squamous cell carcinoma), brain tumor, lung cancer (e.g., small cell lung cancer, non-small cell lung cancer, or lung adenocarcinoma), squamous cell carcinoma, bladder cancer, stomach cancer, ovarian cancer, peritoneal cancer, pancreatic cancer, breast cancer, head and neck cancer, cervical cancer, endometrial cancer, colon cancer (e.g., human colon adenocarcinoma), rectal cancer, colorectal cancer, liver cancer, kidney cancer, urothelial cancer, epidermal cancer, non-Hodgkin lymphoma, central nervous system tumor (e.g. neuroglioma, glioblastoma multiforme, glioma or sarcoma), prostate cancer or thyroid cancer.

In some embodiments, the cancer disease is a cancer disease associated with Trop-2, Her 2, B7H3, Her 3, EGFR.

In some embodiments, the cancer disease is a cancer disease associated with Trop-2 or Her 2.

In some embodiments, the cancer disease is a cancer disease associated with Trop-2.

In some embodiments, the cancer disease is a cancer disease associated with Her 3.

In some embodiments, the cancer disease is a cancer disease associated with EGFR.

In some embodiments, the cancer disease is a cancer disease associated with B7H3.

In some embodiments, the cancer disease is solid tumors.

In some embodiments, the cancer disease is breast cancer or lung cancer (preferably non-small cell lung cancer).

In the present disclosure, unless otherwise stated, the scientific and technical terms used herein have the meanings that are generally understood by those skilled in the art. Moreover, the cell culture, molecular genetics, nucleic acid chemistry, and immunology laboratory procedures used herein are all routine procedures widely used in the corresponding field. Meanwhile, for a better understanding of the present disclosure, definitions and explanations of relevant terms are provided below.

As used herein, examples of the term "pharmaceutically acceptable salt" are organic acid addition salts formed with organic acids that provide pharmaceutically acceptable anions, the organic acid addition salts include but are not limited to formate, acetate, propionate, benzoate, maleate, fumarate, succinate, tartrate, citrate, ascorbate, α-ketoglutarate, α-glycerophosphate, alkylsulfonate or arylsulfonate; preferably, the alkylsulfonate is methylsulfonate or ethylsulfonate; the arylsulfonate is a benzenesulfonate or p-toluenesulfonate. Suitable inorganic salts may also be formed, including but not limited to hydrochloride, hydrobromide, hydroiodate, nitrate, bicarbonate and carbonate, sulfate or phosphate, etc.

As used herein, the term "pharmaceutically acceptable carrier and/or excipient" refers to a carrier and/or excipient that is pharmacologically and/or physiologically compatible with the subject and active ingredients, which is known in the art (seeing such as Remington's Pharmaceutical Sciences. Edited by Gennaro AR, 19th ed. Pennsylvania: Mack Publishing Company, 1995), and includes, but is not limited to: pH adjusters, surfactants, adjuvants, ionic strength enhancers, diluents, agents to maintain osmotic pressure, agents to delay absorption, preservatives.

Pharmaceutically acceptable salts can be obtained using standard procedures well known in the art, e.g. by reacting a sufficient amount of basic compound with a suitable acid providing a pharmaceutically acceptable anion.

In the present disclosure, the pharmaceutically acceptable adjuvant refers to the excipient and additive used in the production of medicaments and the formulation, refers to the substances contained in a pharmaceutical preparation whose safety has been reasonably evaluated, besides the active ingredients. In addition to shaping, acting as a carrier and improving stability, pharmaceutically acceptable adjuvants also have important functions such as solubilization, hydro trope, slow and controlled release, and are important components that may affect the quality, safety and effectiveness of drugs. According to its source, it can be divided into natural, semi-synthetic and fully synthetic. According to its functions and uses, it can be divided into: solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adherents, antioxidants, chelating agents, penetration enhancers, pH adjusters, buffers, plasticizers, surfactants, foaming agents, defoaming agents, thickeners, inclusion agents, humectants, absorbents, diluents, flocculants and deflocculants, filter aids, release blockers, etc.; it can be divided into oral, injectable, mucosal, transdermal or topical administration, nasal or oral inhalation administration and ocular administration according to its routes of administration. The same pharmaceutical acceptable adjuvant can be used in pharmaceutical formulations with different routes of administration, and has different effects and uses.

The pharmaceutical composition can be prepared into various suitable dosage forms according to the route of administration. For example, tablets, capsules, granules, oral solutions, oral suspensions, oral emulsions, powders, tinctures, syrups, injections, suppositories, ointments, creams, pastes, ophthalmic formulation, pills, implants, aerosols, powder aerosols, sprays, etc., in which the pharmaceutical composition or suitable dosage form may contain 0.01 mg to 1000 mg of the compound of the present disclosure or a pharmaceutically acceptable salt or conjugate thereof, suitably 0.1 mg to 800 mg, preferably 0.5 mg to 500 mg, preferably 0.5 mg to 350 mg, and particularly preferably 1 mg to 250 mg.

The pharmaceutical composition can be administered in the form of injection, including injection solution, sterile powder for injection and concentrated solution for injection. Among them, useful vehicles and solvents include water, Ringer's solution and isotonic sodium chloride solution. In addition, sterile non-volatile oils can also be employed as a solvent or suspending medium, such as mono- or diglycerides.

The term "treatment" as used herein generally refers to obtaining a desired pharmacological and/or physiological effect. The effect may be prophylactic in terms of complete or partial prevention of the disease or its symptoms; and/or therapeutic in terms of partial or complete stabilization or cure of the disease and/or side effects due to the disease. "Treatment" as used herein encompasses any treatment of a disease in a patient, including: (a) prevention of a diseases or a symptom in a patient susceptible to the disease or symptom but not yet diagnosed to have the disease or symptom; (b) suppression of symptoms of the disease, that is, preventing its development; or (c) alleviating symptoms of the disease, i.e., causing the disease or symptoms to regress.

In the present disclosure, the term "individual" includes human or non-human animals. Exemplary human individuals include human individuals (referred to as patients) with a disease (e.g., a disease described herein) or normal individuals. The term "non-human animal" in the present disclosure includes all vertebrates, such as non-mammals (e.g., birds, amphibians, reptiles) and mammals, such as non-human primates, livestock and/or domesticated animals (e.g., sheep, dogs, cats, cows, pigs, etc.).

In the present disclosure, the term "effective dose" refers to the amount of a compound that, after administered, alleviates one or more symptoms of the disorders being treated to some extent.

In the present disclosure, the term "ligand drug conjugate" refers to a substance in which a bioactive molecule (drug molecule) is linked to a targeted moiety. In some embodiments of the present disclosure, the bioactive molecule is linked to the targeting moiety by a linker. The linker can be broken in a specific environment (e.g., in the presence of hydrolase within the tumor and/or a low pH environment) or under a specific action (e.g., the action of lysosomal proteases), so that the bioactive molecule was separated from the target moiety. In some embodiments of the present disclosure, the linker comprises cleavable or non-cleavable units, such as peptides or disulfide linkage. In some embodiments of the present disclosure, the bioactive molecule is directly linked to the target moiety by a covalent bond that can be broken under a particular environment or action, thereby separating the bioactive molecule from the target moiety. In some embodiments of the present disclosure, the ligand drug conjugate comprises a targeting moiety, a linker, and a compound fragment of formula II of the present disclosure.

In the present disclosure, the term "bioactive substance", "bioactive molecule" or "drug molecule" refers to a substance that inhibits or prevents the function of cells and/or causes cell death or destruction. In some embodiments of the present disclosure, the bioactive substance, bioactive molecule or drug molecule in the conjugate is a molecule with anti-tumor biological activity. For example: radioisotopes such as At²¹¹, I¹³¹, I¹²⁵, Y⁹⁰, Re¹⁸⁶, Re¹⁸⁸, Sm¹⁵³, Bi²¹², P³², Pb²¹²and Lu; metal complexes, such as metal platinum complexes, metal gold complexes, oxaliplatin, etc.; glycopeptide antibiotics, such as bleomycin, pingyangmycin; DNA topoisomerase inhibitors, such as topoisomerase I inhibitors, camptothecin, hydroxylcamptothecin, 9-aminocamptothecin, SN-38, irinotecan, topotecan, belotecan, rubitecan, topoisomerase II inhibitor, actinomycin D, adriamycin, doxorubicin, duocarmycin, daunorubicin, mitoxantrone, podophyllotoxin, etoposide, etc.; drugs that interfere with DNA synthesis, such as methotrexate, 5-fluorouracil, cytarabine, gemcitabine, mercaptopurine, pentostatin, fludarabine, cladribine, nelarabine, etc.; drugs acting on structural proteins, such as tubulin inhibitors, vinca alkaloids, vincristine, vinblastine, paclitaxel, docetaxel, cabazitaxel, etc.; tumor signaling pathway inhibitors, such as serine/threonine kinase inhibitors, tyrosine kinase inhibitors, aspartate kinase inhibitors or histidine kinase inhibitors, etc.; also including proteasome inhibitors, histone deacetylation enzyme inhibitors, tumor angiogenesis inhibitors, cyclin inhibitors, maytansine derivatives, calicheamicin derivatives, auristatin derivatives, pyrrolobenzodiazepines (PBD) derivatives, melphalan, mitomycin C, chlorambucil, or other active substances that inhibit tumor cell growth, promote tumor cell apoptosis and necrosis; enzymes and fragments thereof, such as nucleolysin; antibiotics; toxins, such as small molecule toxins or enzymatically active toxins originated from bacteria, fungi, plants or animals, including fragments and/or variants thereof; growth inhibitors; drug modules. The term "toxin" refers to a substance capable of producing deleterious effects on the growth or proliferation of cells.

In the present disclosure, the term "small molecule" refers to a bioactive small molecule drug. The term small molecule toxin has cell-damaging activity, i.e. a state of causing pathological changes of cells in some forms, and cell damage is not limited to direct damage, including all kinds of damage to the structure and function of cells, such as DNA cleavage, base-dimer formation, chromosome cleavage, damage to cell division mechanism and reduction of various enzymatic activities.

In the present disclosure, the term "linker" refers to a fragment that links a bioactive molecule (drug molecule) to a targeting moiety.

In the present disclosure, the term "targeting moiety" refers to a moiety in a conjugate that is capable of specifically binding to a target (or a portion of a target) on the cell surface. Through the interaction of the targeting moiety with the target, the conjugate can be delivered to a specific cell population.

In the present disclosure, when the targeting moiety in a conjugate is an antibody, the conjugate may be referred to as a "drug-antibody conjugate".

In the present disclosure, an antibody or antigen-binding fragment thereof includes a derivatized antibody or antigen-binding fragment thereof, such as an antibody or antigen-binding fragment thereof having a thiol group, wherein the derivatization allows the antibody to have a group or ability reactive with a drug-linker conjugate. The thiol -SH can be derivatized by breaking the disulfide bond (e.g., by reduction with the reducing agent TCEP).

As used herein, the terms "cancer" and "tumor" are used with the same meaning.

The term "gene" used herein includes not only DNA but also its mRNA, its cDNA and its cRNA.

The term "polynucleotide" as used herein is used in the same sense as nucleic acid and also includes DNA, RNA, probes, oligonucleotides and primers.

The term "Tb" as used herein is an abbreviation for "target binding" and includes antibodies or any molecule that binds to a target, and the term "Ab" is an abbreviation for "antibody" and is used interchangeably with "antibody".

As used herein, the terms "polypeptide" and "protein" are used interchangeably.

The term "cell" as used herein also includes cells within an individual animal and cultured cells.

The B7H3 involved in the anti-B7H3 antibodies described in the present disclosure may be conventional B7H3 in the art, such as soluble B7H3, B7H3 in membrane form, etc., and also represents B7H3 variant 1 and/or B7H3 variant 2.

KD refers to the dissociation constant obtained from the ratio (or Kd/Ka, expressed in molar concentration (M)) of Kd (dissociation rate of specific binding molecule-target protein interaction) to Ka (binding rate of specific binding molecule-target protein interaction). KD values can be determined using methods well established in the art. The preferred method for determining the KD of binding molecules is by using surface plasmon resonance, for example a biosensor system, such as the Biacore TM (GE Healthcare Life Sciences) system.

The term "B7H3 variant" refers to a polypeptide having similar or identical functions to a B7H3 polypeptide, B7H3 fragment, anti-B7H3 antibody or antibody fragment thereof, but not necessarily comprising the amino acid sequences of similar or identical B7H3 polypeptide, B7H3 fragment, anti-B7H3 antibodies or fragments thereof, or having the structures of similar or identical B7H3 polypeptides, B7H3 fragments, anti-B7H3 antibodies or fragments thereof.

The term "Her3 variant" refers to a polypeptide having similar or identical functions to a HER3 polypeptide, HER3 fragment, anti-HER3 antibody or antibody fragment thereof, but not necessarily comprising the amino acid sequences of similar or identical HER3 polypeptide, HER3 fragment, anti-HER3 antibodies or fragments thereof, or the structures of similar or identical HER3 polypeptides, HER3 fragments, anti-Her3 antibodies or fragments thereof.

As used herein, the percent homology between two amino acid sequences is equal to the percent identity between the two sequences. The percent sequence identity between two sequences is a function of the number of identical positions shared by the sequences (i.e., % homology = number of identical positions/total number of positions X 100), taking into account the number of gaps and the length of each gap, which needs to be introduced for optimal alignment of the two sequences. Sequence comparison and determination of the percentage identity between sequences can be carried out by methods generally known in the art, and such sequence comparison and determination of the percentage identity can be realized by mathematical algorithms. For example, the algorithm in Meyers and Miller, 1988, Comput. Appl. Biosci. 4: 11-17 (which has been integrated into the ALIGN program (version 2.0)) can be used to determine the percentage identity between amino acid sequences and/or between nucleotide sequences. In addition, the GAP program in the GCG package obtained online from Accelrys (using its default parameters) can be used to determine the percentage identity between amino acid sequences or between nucleotide sequences. In one embodiment, the two sequences are of equal length.

The term "epitope" refers to a portion of a B7H3 polypeptide or protein that has antigenic or immunogenic activity in an animal, preferably a mammal. The epitope of the anti-B7H3 antibody or antigen-binding fragment thereof of the present disclosure can be determined by existing techniques, such as synthetic peptide method, immune informatics prediction, determination of polypeptide activity, epitope peptide scanning method, phage display technology, X-ray diffraction and nuclear magnetic resonance analysis, homologous antibody modeling protein docking prediction method. The phrase "antibodies that bind to the same epitope" as used herein refers to different antibodies that bind to a common epitope. If the second antibody binds to a part of the peptide or part of the tertiary structure to which the first antibody binds, it can be determined that the first antibody and the second antibody bind to the same epitope.

In the present disclosure, the term "antibody" is used in its broadest interpretation to include intact monoclonal antibodies, polyclonal antibodies, and multi-specific antibodies (e.g., bispecific antibodies) formed from at least two intact antibodies, so long as they have all required biological activity. In the present disclosure, "antibody" and "immunoglobulin" are used interchangeably. "Antibody molecule" or "antibody" as used herein refers to immunoglobulin molecules and immunologically active portions of immunoglobulin molecules, i.e. molecules that contain an antigen-binding site allowing to immune specifically binds an antigen. Thus, the term antibody broadly encompasses not only a whole antibody molecule, but also fragments of said antibody as well as variants (including derivatives) of said antibody and antibody fragments. When "antibody molecule" or "antibody" is used in the same context as antigen-binding fragment, "antibody molecule" or "antibody" refers to an intact antibody molecule or a full-length antibody. The term antibody molecule mentioned in the present specification, for example, includes, but is not limited to single-chain Fv (scFv), Fab fragments, Fab' fragments, F(ab')2, disulfide-linked Fv(sdFv), Fv, and intact antibodies or full-length antibodies. The term "single chain Fv" or "scFv" refers to a polypeptide comprising the VL domain of an antibody linked to the VH domain of the antibody. For example, antibodies that immune specifically bind to B7H3 can cross-react with other antigens, and preferably, can not cross-react with other antigens. Antibodies that immune specifically bind to B7H3 can be identified, for example, by immunoassays or other methods known to those skilled in the art. "Intact" antibody or "full-length" antibody refers to a protein containing two heavy chains (H) and two light chains (L) linked to each other by disulfide bonds, and the protein comprises: (1) for the heavy chain, a variable region (abbreviated herein as "VH") and a constant region of the heavy chain containing three domains CH1, CH2, CH3; and (2) for the light chain, a variable region (abbreviated herein as "VL") of the light chain and a constant region of the light chain containing a domain CL. Antibodies of the present disclosure include, but are not limited to, monoclonal, multi-specific, human or chimeric antibodies, single chain antibodies, Fab fragments, F(ab') fragments, anti-idiotypic (anti-Id) antibodies (including, for example, anti-Id antibody of an antibody of the present disclosure), and epitope-binding fragments of any of the aforementioned antibodies. The immunoglobulin molecules of the present disclosure can be any type (e.g., IgG, IgE, IgM, IgD, IgA and IgY), any class (e.g., IgGl, IgG2, IgG3, IgG4, IgAl, and IgA2), or any subclass of immunoglobulins. Preferably, the antibodies of the present disclosure comprise or consist of a VH domain, a VH CDR (referred herein as HCDR), a VL domain, or a VL CDR (often represented herein by LCDR) having any of the amino acid sequences or fragments or variants thereof described in the Sequence and its Specific Information Tables.

In the present disclosure, the term "monoclonal antibody" refers to an antibody from a population of substantially homogeneous antibodies, i.e., each antibody consisting of the population is identical except for potential minor natural mutations. Monoclonal antibodies have high specificity against one determinant (epitope) of an antigen, while the polyclonal antibodies comprise different antibodies against different determinants (epitopes). In addition to specificity, the advantage of monoclonal antibodies is that they can be synthesized free from contamination by other antibodies. The modifier "monoclonal" as used herein means that the antibody is characterized as being from a substantially homogeneous population of antibodies and should not be construed as requiring a particular method of preparation.

In some embodiments of the present disclosure, monoclonal antibodies also particularly include chimeric antibodies, i.e., a portion of the heavy and/or light chain is identical or homologous to a type, a class or a subclass of antibodies, and the remainder is identical or homologous to another type, another class or another subclass of antibodies, as long as they have the desired biological activity (see e.g. US 4,816,567; and Morrison et al., 1984, PNAS, 81: 6851-6855). Chimeric antibodies useful in the present disclosure include primatized antibodies comprising variable region antigen-binding sequences and human constant region sequences from non-human primates (eg, ancient monkeys, chimpanzees, etc.).

The term "antigen-binding fragment" refers to a portion of an antibody, preferably the antigen-binding or variable region. Examples of antibody fragments include Fab, Fab', F(ab')₂, Fd, Fv, dAb and complementarity determining region fragments, diabodies, linear antibodies and single chain antibody molecules. The term "antigen-binding fragment" as used herein refers to a partial fragment of an antibody having antigen-binding activity, wherein the fragment has full or partial functionality of the antibody, including, but not limited to, single-chain Fv (scFv), Fab, Fab', F(ab')2, disulfide-linked Fv (sdFv), Fv, di-scFv, etc. The term also includes Fab', which is a monovalent fragment of the variable region of an antibody obtained by treating F(ab')2 under reducing conditions. However, the term is not limited to these molecules as long as the fragment has a binding affinity for the antigen. Furthermore, these functional fragments include not only fragments obtained by treating full-length molecules of antibody proteins with appropriate enzymes, but also proteins produced in appropriate host cells using genetically modified antibody genes.

The term "Fab" as used herein denotes a monovalent fragment of a variable region of a antibody obtained by treating F(ab')2 under reducing conditions as described above. However, Fab' of the present disclosure also includes Fab' produced using genetically modified antibody genes.

As used herein, the term "scFv" refers to a single polypeptide chain comprising VL and VH domains, wherein the VL and VH are linked by a linker or directly (see, e.g., Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, Eds. Roseburg and Moore, Springer-Verlag, New York, pp. 269-315 (1994)). Such scFv molecules can have the general structure: NH2-VL-linker-VH-COOH or NH2-VH-linker-VL-COOH. Suitable linkers in prior art consist of repeated GGGGS amino acid sequences or variants thereof. For example, a linker with the amino acid sequence (GGGGS)4 can be used, and variants thereof can also be used (Holliger et al. (1993), Proc. Natl. Acad. Sci. USA 90: 6444-6448). Other linkers that may be used in the present disclosure were described in Alfthan et al. (1995), Protein Eng. 8: 725-731, Choi et al. (2001), Eur. J. Immunol. 31: 94-106, Hu et al (1996), Cancer Res. 56: 3055-3061, Kipriyanov et al (1999), J. Mol. Biol. 293: 41-56 and Roovers et al (2001), Cancer Immunol. In some cases, disulfide bonds may also exist between VH and VL of scFv. As used herein, the term "di-scFv" refers to an antibody fragment formed by linking two scFv.

As used herein, the antibody or antigen-binding fragment thereof, even if it contains variants, amino acid substitutions, deletions or additions, still has activity to bind the antigen.

The term "bispecific antibody", also known as "bifunctional antibody conjugate", refers to a conjugate formed by a first antibody (fragment) and a second antibody (fragment) through a coupling arm, and the conjugate retains the activity of the respective antibody, so has bifunctionality and bi-specificity.

The term "multi-specific antibody" includes, for example, tri-specific antibodies, which are antibodies with three different antigen binding specificities, and tetra-specific antibodies, which are antibodies with four different antigen binding specificities.

The term "intact antibody" or "full-length antibody" refers to an antibody comprising an antigen-binding variable region and a light chain constant region (CL), a heavy chain constant region (CH1, CH2, and CH3). The constant region may be a natural sequence (e.g., a human natural constant region sequence) or a variant of its amino acid sequence. The intact antibody is preferably the one with one or more effector functions.

The term "probody" is a modified antibody, including an antibody or an antibody fragment, capable of specifically binding to its target and capable of being coupled to a masking group, wherein the masking group means the cleavage constant of the binding capacity of the antibody or antibody fragment with the masking group to its target is at least 100-fold or 1000-fold, or 10,000-fold greater than that of the antibody or antibody fragment without the masking group.

In the present disclosure, "humanized" forms of non-human (e.g., murine) antibodies refer to chimeric antibodies that comprise minimal non-human immunoglobulin sequences. Most humanized antibodies are human recipient immunoglobulins with hypervariable region residue(s) replaced by non-human (e.g. mouse, rat, rabbit or non-human primate) hypervariable region residues (donor antibody) having the desired specificity, affinity and function. In some embodiments, framework region (FR) residues of the human immunoglobulin are also replaced with non-human residues. Furthermore, a humanized antibody may also contain residues not found in the recipient antibody or donor antibody. These modifications are intended to further optimize the performance of the antibody. Humanized antibodies generally comprise at least one and usually two variable regions, in which all or nearly all of the hypervariable loops correspond to those of non-human immunoglobulins, and the FRs are full or nearly full human immunoglobulins protein sequence. Humanized antibodies may also comprise at least a portion of an immunoglobulin constant region (Fc, typically a human immunoglobulin Fc). For details see, e.g., Jones et al, 1986, Nature, 321: 522-525; Riechmann et al, 1988, Nature, 332: 323-329; and Presta, 1992, Curr Op Struct Bwl 2: 593-596.

Intact antibodies can be divided into different "classes" based on the amino acid sequence of the heavy chain constant region. The main five classes are IgA, IgD, IgE, IgG and IgM, several of which can also be divided into different "subclasses" (isotypes), such as IgG1, IgG2, IgG3, IgG4, IgA1 and IgA2. The heavy chain constant regions of the different classes of antibodies are called α, β, ε, γ and µ, respectively. The subunit structures and three-dimensional configurations of different classes of immunoglobulins are well known in the art.

Herein, the CDRs contained by the antibodies or antigen-binding fragments thereof of the present disclosure can be identified according to various numbering systems known in the art. In certain embodiments, the CDRs contained by an antibody or antigen-binding fragment thereof of the present disclosure are preferably determined by the Kabat, Chothia or AbM or IMGT numbering systems. According to the IMGT numbering system, the corresponding numbers of CDRs and FRs are as follows:

| | FR1-IMGT | CDR1-IMGT | FR2-IMGT | CDR2-IMGT | FR3-IMGT | For germline V-GENE (*) CDR3-IMGT | For rearranged V-J-GENEs and V-D-J-GENEs | |
|---|---|---|---|---|---|---|---|---|
| | | | | | | Germline CDR3-IMGT | Rearranged CDR3-IMGT | FR4-IMGT |
| Amino acid numbering | 1 --> 26 (C 23) | 27 --> 38 | 39 --> 55 (W 41) | 56 --> 65 | 66 --> 104 (C 104) | 105 --> 116 | 105 --> 117 (112.1, 111.1, 112.2, 111.2, etc.) | 118 --> 129 |
| number of amino acids | 25-26 aa | 5-12 aa | 16-17 aa | 0-10 aa | 36-39 aa | 2-12 aa | 2-13 aa (more than 13 aa) | 10-12 aa |

As used herein, the term "region of framework residues" or "FR residues" refers to those amino acid residues in the variable region of an antibody other than the CDR residues as defined above.

As used herein, the term "germline antibody gene" is a gene encoding an immunoglobulin expressed by non-lymphocytes, which has not undergone the maturation process leading to the genetic rearrangement and maturation of the specific immunoglobulin expressed. One advantage provided by various embodiments of the present disclosure lies in the recognition that the amino acid sequences encoded by germline antibody genes retain more characteristic important amino acid sequence structures of individuals of animal species than that encoded by mature antibody genes. Thus, when applied therapeutically to that species, it is less likely to be recognized as a foreign substance by that species.

As for amino acid substitutions, conservative amino acid substitutions are preferred in this specification. Conservative amino acid substitutions refer to substitutions that occur within a set of amino acids associated with amino acid side chains. Preferred sets of amino acids are as follows: acidic sets (aspartic acid and glutamic acid); basic sets (lysine, arginine and histidine); non-polar sets (alanine, valine, leucine, isoleucine, proline, phenylalanine, methionine, and tryptophan); and the uncharged polar families (glycine, asparagine, glutamine, cysteine, serine, threonine and tyrosine). More preferred sets of amino acids are as follows: aliphatic hydroxyls (serine and threonine); amide-containing sets (asparagine and glutamine); aliphatic sets (alanine, valine, leucine and isoleucine); and aromatic sets (phenylalanine, tryptophan, and tyrosine). Such amino acid substitutions are preferably made within a set that does not impair the properties of the substance having the original amino acid sequence.

In addition, it is known that the lysine residue at the carboxyl end of the heavy chain of the antibody produced in cultured mammalian cells is deleted (Journal of Chromatography A, 705: 129-134 (1995)), as well as it is also known that the two amino acid residues (glycine and lysine) at the carboxyl end of the heavy chain of the antibody produced in cultured mammalian cells are deleted, and the new proline residue at the carboxyl terminus is amidated (Analytical Biochemistry, 360: 75-83 (2007)). However, such deletions and modifications of the heavy chain sequence do not affect the antigen binding affinity and effector function of the antibody (complement activation, antibody-dependent cell cytotoxicity, etc.).

The twenty conventional amino acids referred to herein have been written following conventional usage. See, e.g., Immunology-A Synthesis (2nd Edition, E. S. Golub and D. R. Gren, Eds., Sinauer Associates, Sunderland, Mass. (1991)), which is incorporated herein by reference. As used herein, the terms "polypeptide" and "protein" have the same meaning and are used interchangeably. And in the present disclosure, amino acids are generally represented by one-letter and three-letter abbreviations well known in the art. For example, alanine can be represented by A or Ala; arginine can be represented by R or Arg; glycine can be represented by G or Gly; glutamine can be represented by Q or Gln.

As used herein, the term "prevention" refers to a method performed to prevent or delay the occurrence of a disease or disorder or symptom (e.g., tumors and infectious diseases) in a subject. As used herein, the term "treatment" refers to a method performed to obtain a beneficial or desired clinical result. For the purposes of the present disclosure, a beneficial or desired clinical result includes, but is not limited to, alleviation of symptoms, reduction in the extent of the disease, stabilization (i.e., not worsening) of the disease state, delaying or slowing the progression of the disease, amelioration or alleviation of the disease status, and relief of symptoms (whether in part or in full), whether detectable or undetectable. In addition, "treatment" can also mean prolonging survival compared with expected survival (if not receiving treatment).

As used herein, the term "subject" refers to a mammal, e.g., a primate mammal, such as a non-human primate mammal or a human. In certain embodiments, the subject (e.g., human) has tumors and infectious diseases, or is at risk of having such diseases.

As used herein, the term "effective amount" refers to an amount sufficient to achieve, or at least partially achieve, a desired effect. For example, an effective amount to prevent diseases (e.g., tumors and infectious diseases) is an amount sufficient to prevent, arrest, or delay the occurrence of diseases (e.g., tumors and infectious diseases); an effective amount to treat diseases is an amount sufficient to cure or at least partially arrest the diseases and their complications in patients who already have them. The determination of such an effective amount is entirely within the capabilities of those skilled in the art. For example, the amount effective for therapeutic use will depend on the severity of the disease to be treated, the general state of the patient's own immune system, the general conditions of the patient such as age, weight and sex, the way in which the drug is administered, and other treatments administered simultaneously, etc.

As used herein, the term "effector function" refers to those biological activities attributable to an Fc region of an antibody (either a native sequence Fc region or an amino acid sequence variant Fc region), which varies with the antibody isotypes.

The term "pharmaceutically acceptable" means that the molecule itself, molecular fragment, or composition does not produce detrimental, allergic or other adverse reactions when suitably administered to an animal or human. Specific examples of some substances that may be pharmaceutically acceptable carriers or components thereof include sugars (e.g., lactose), starch, cellulose and derivatives thereof, vegetable oils, gelatin, polyols (e.g., propylene glycol), alginic acid, and the like.

The *in vivo* therapeutic effect of antibodies and antibody-drug conjugates on cancers can be determined using experimental animals, e.g., administering the antibody to nude mice implanted with a B7H3-expressing tumor cell line, and measuring any changes in cancer cells.

In the present disclosure, although in most cases, amino acid substitutions in antibodies are substituted with L-amino acids, but are not limited to that. In some embodiments, one or more D-amino acids can be included in the antibody peptide chain. Peptides containing D-amino acids are more stable and less susceptible to degradation in the oral cavity, gut or plasma than peptides containing only L-amino acids.

Monoclonal antibodies used in the present disclosure can be produced by a number of methods. For example, monoclonal antibodies used in the present disclosure can be obtained by the hybridoma method using a number of species (including cells from mouse, hamster, rat and human) (see, e.g., Kohler et al., 1975, Nature, 256: 495), or by recombinant DNA techniques (see e.g. US 4,816,567), or can be isolated from phage antibody libraries (see e.g. Clackson et al, 1991, Nature, 352: 624-628; and Marks et al, 1991, Journal of Molecular Biology, 222: 581-597).

Herein, unless it is clearly stated otherwise, the expression ways "each... independently selected from" and "...each independently selected from" used throughout this specification can be interchanged, and should be understood in a broad sense, which may mean that in different groups, the specific options expressed for the same or different symbols do not affect each other, and which can also mean that in the same group, the specific options expressed for the same or different symbols do not affect each other.

Herein, the term "direct bond" means that the groups on both sides thereof are directly connected, such as in a compound represented by formula II 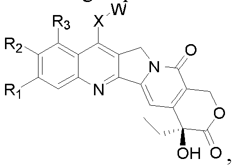 if X is a direct bond, its structural formula is 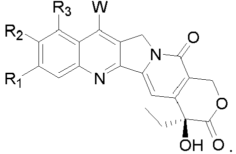 Other direct bonds can be understood with reference to the foregoing content.

As used herein, the term "absent" means that the group is not present, such as in a compound represented by formula II 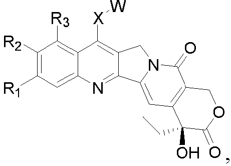 if W is absent, its structural formula is

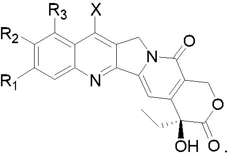

In a compound represented by formula II 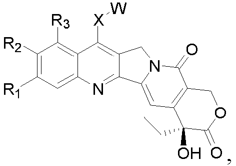 R₁ and R₂ together with the carbons atom to which they are both attached form 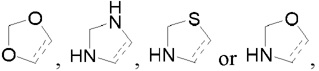 the "dotted line" indicates where the heterocyclic ring is fused to the benzene ring, such as forming

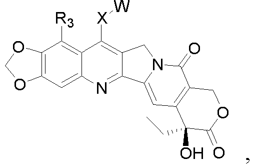

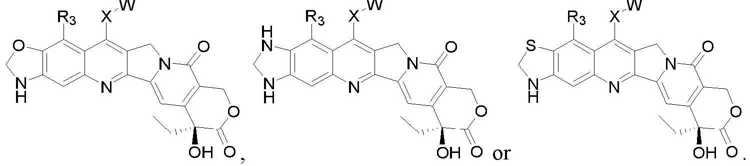

Herein, in the drug linker conjugate represented by formula III 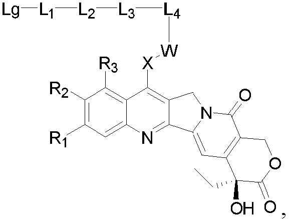 when L₄ is 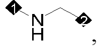 wherein the mark number 1 and 2 indicate the linkage positions of L₄ and other groups, and specifically, when position 1 is attached to L₃, and position 2 is attached to a drug molecule, namely 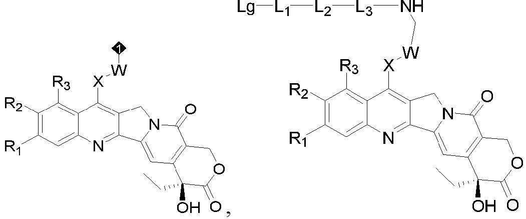 is formed. The remaining mark numbers 1 and 2 can be understood with reference to the foregoing content.

Herein, in a compound represented by formula II 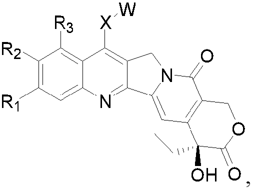 R₃ and X together with the carbon atoms to which they are both attached form 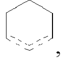 the "dotted line" indicates where the heterocyclic ring is fused to the benzene ring, forming

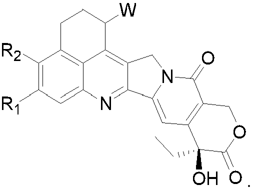

Herein, X is defined as, for example, "X is selected from optionally substituted 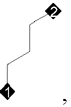 and the substituent is selected from 1 or 2 C1-4 alkyl groups (such as methyl)", then X can be, for example, 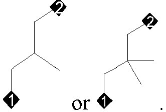 Other similar definitions of X can be understood with reference to the foregoing content.

Herein, X is defined as, for example, "X is selected from optionally substituted 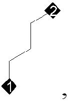 and the substituents are selected from 2 C1-4 alkyl groups (such as methyl) and which together with the carbon atom to which they are both attached form a C3-6 cycloalkyl (e.g. cyclopropyl)", then X can be, for example, 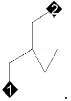 Other similar definitions of X can be understood with reference to the foregoing content.

In the structure of amino acid residue represented by AA¹ 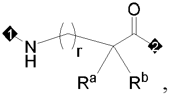 if r is 0, it will be understood by those skilled in the art that the structure of the amino acid residue represented by AA¹ will become

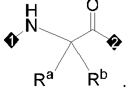

In the structure of amino acid residues represented by AA¹ 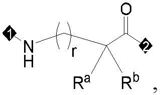 if R^{a} and R^{b} together with the carbon atom to which they are both attached form 4-10-membered heterocycle, and the 4-10-membered heterocycle is optionally substituted with one or more R⁰, wherein the term "the 4-10-membered heterocycle is optionally substituted with one or more R⁰" means that the 4-10-membered heterocycle may be unsubstituted or substituted with one or more R⁰, and in the more R⁰, the definition of each R⁰ may be the same or different. Other similar definitions can be understood with reference to the foregoing content.

Herein, for example, when L₃ is selected from Lys, Val-Cit, Ala-Ala-Asn, Ala-Ala-Asp, Gly-Gly-Phe-Gly, Val-Lys-Gly, Val-Ala, Lys-Ala-Asn, "the distal amino group of the lysine (Lys) is optionally substituted with 1, 2 or 3 substituents selected from tert-butoxycarbonyl, C₁₋₆ alkyl (preferably methyl), O" means that the distal amino group of Lys in each option of L₃ is optionally substituted with 1, 2 or 3 subsituents selected from tert-butoxycarbonyl, C1-6 alkyl (preferably methyl), O. Wherein "the distal amino group of Lys" refers to the naked amino group-NH₂ in the lysine residue 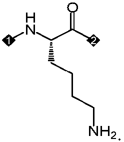 "The distal amino group of the lysine (Lys) is optionally substituted with 1, 2 or 3 substituents selected from tert-butoxycarbonyl, C1-6 alkyl (preferably methyl), O" indicates that the distal amino group of the lysine (Lys) may not be substituted, or may be substituted by 1, 2 or 3 substituents selected from tert-butoxycarbonyl, C1-6 alkyl (preferably methyl), O. For example, it can be substituted by one tert-butoxycarbonyl group, that is, it becomes 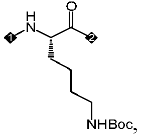 or it can be substituted by two methyls, that is, it becomes 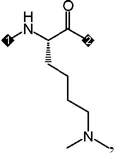 or it can be substituted by two methyls and one O, that is, it becomes 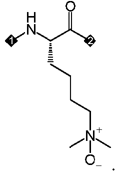 It should be noted that "the distal amino group of the lysine (Lys) is substituted with O" means that the distal amino group of the lysine (Lys) is substituted with oxo, that is, it becomes 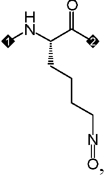 and if the distal amino group is further substituted with two methyl groups, it becomes

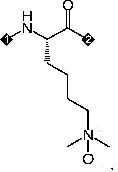

In various parts of this specification, substituents of compounds in the present disclosure are disclosed according to group types or scopes. Specifically, the present disclosure includes each individual subcombination of each member of these group types or scopes. For example, the term "C1-6 alkyl" particularly refers to methyl, ethyl, C3 alkyl, C4 alkyl, C5 alkyl and C6 alkyl as disclosed independently.

As used herein, the term "C1-6 alkyl" refers to a straight or branched chain alkyl group containing 1-6 carbon atoms, including, for example, "C1-3 alkyl" or "C1-4 alkyl", methyl , ethyl, etc., and specific examples include but are not limited to: methyl, ethyl, n-propyl, isopropyl, n-butyl, isobutyl, sec-butyl, tert-butyl, pentyl, hexyl.

As used herein, the term "C₁₋₄ alkyl" refers to straight or branched chain alkyl groups containing 1-4 carbon atoms, including, for example, "C1-3 alkyl", methyl, ethyl, etc. Specific examples include but are not limited to: methyl, ethyl, n-propyl, isopropyl, n-butyl, isobutyl, sec-butyl, tert-butyl.

As used herein, the term "C₂₋₆ alkenyl" refers to a straight-chain, branched-chain or cyclic alkenyl group containing at least one double bond and 2-6 carbon atoms, including, for example, "C₂₋₄ alkenyl" and the like. Examples include, but are not limited to: vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl and the like.

As used herein, the term "C₂₋₆ alkynyl" refers to a straight or branched alkynyl group containing at least one triple bond and 2-6 carbon atoms, including, for example, "C₂₋₄ alkynyl" and the like. Examples include, but are not limited to: ethynyl, propynyl, 2-butynyl, 2-pentynyl, 3-pentynyl, 4-methyl-2-pentynyl, 2-hexynyl, 3-hexynyl, 5-methyl-2-hexynyl, etc.

As used herein, the term "halogen" includes fluorine, chlorine, bromine, and iodine.

As used herein, the term "3-6-membered cycloalkyl" or "C₃₋₆ cycloalkyl" refers to a saturated cyclic alkyl containing 3-6 carbon atoms, including cyclopropanyl (i.e., cyclopropyl), cyclobutanyl (i.e. cyclobutyl), cyclopentanyl (i.e. cyclopentyl), cyclohexyl.

As used herein, the term "3-7-membered carbocycloalkyl" or "C₃₋₇ cycloalkyl" refers to a saturated cyclic alkyl containing 3-7 carbon atoms, including cyclopropyl, cyclobutyl, cyclopentyl, cyclohexyl, cycloheptyl.

As used herein, the term "C₁₋₆ alkoxy" refers to an alkyl group as defined above which is attached to the parent molecular moiety by an oxygen atom. Specific examples include, but are not limited to, methoxy, ethoxy, propoxy, isopropoxy, n-propoxy, isopropoxy, n-butoxy, isobutoxy, tert-butoxy, pentoxy, hexyloxy, etc.

As used herein, the term "C₁₋₄ alkoxy" refers to an alkyl group as defined above which is attached to the parent molecular moiety by an oxygen atom. Specific examples include, but are not limited to, methoxy, ethoxy, propoxy, isopropoxy, n-propoxy, isopropoxy, n-butoxy, isobutoxy, tert-butoxy, etc.

As used herein, the term "4-10-membered heterocyclic group" refers to a cyclic group containing 4 to 10 ring atoms (wherein at least one ring atom is a heteroatom, such as nitrogen, oxygen, or sulfur atom). The term "4-6 membered heterocyclic group" refers to a cyclic group containing 4-6 ring atoms (wherein at least one ring atom is a heteroatom, such as nitrogen, oxygen, or sulfur atom). Optionally, ring atoms (e.g., carbon, nitrogen, or sulfur atoms) in the ring structure may be substituted by oxygen. "4-8-membered heterocyclic groups" include, for example, "4-8-membered nitrogen-containing heterocyclic group", "4-8-membered oxygen-containing heterocyclic group", "4-7-membered heterocyclic group", "4-7-membered oxygen-containing heterocyclic group", "4-7-membered heterocyclic group", "4-6-membered heterocyclic group", "5-7-membered heterocyclic group", "5-6-membered heterocyclic group", "5-6-membered nitrogen-containing heterocyclic group", including, but not limited to, oxocyclobutanyl, pyrrolidinyl, tetrahydrofuryl, piperidinyl, piperazinyl, tetrahydropyranyl, homopiperazinyl, etc.

As used herein, the term "4-10-membered heterocycle" refers to a ring containing 4-10 ring atoms (wherein at least one ring atom is a heteroatom such as nitrogen, oxygen or sulfur atom). The term "5-6 membered heterocycle" refers to a ring containing 5-6 ring atoms (wherein at least one ring atom is a heteroatom such as nitrogen, oxygen or sulfur atom), including but not limited to pyrrolidine, tetrahydrofuran, piperidine, piperazine, tetrahydropyran, etc.

As used herein, the term "aryl" refers to a monocyclic or polycyclic hydrocarbon group having aromaticity, such as 6-10-membered aryl, 5-8-membered aryl, and the like. Specific examples include, but are not limited to, phenyl, naphthyl, anthracenyl, phenanthryl, and the like. The "6-10-membered aryl" refers to an aryl having 6-10 ring atoms. The "C6-10 aryl" refers to an aryl group containing 6-10 carbon atoms.

As used herein, the term "heteroaryl" refers to a cyclic group having aromaticity, in which at least one ring atom is a heteroatom, such as nitrogen, oxygen or sulfur atom. Optionally, ring atoms (e.g., carbon, nitrogen, or sulfur atoms) in the ring structure may be substituted by oxygen. Specific examples include, but are not limited to, 5-10 membered heteroaryl, 5-6 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, etc., such as furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, 1,4-dioxinyl, 2*H*-1,2-oxazinyl, 4*H-*1,2-oxazinyl, 6*H*-1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azacycloheptatrienyl, 1,3-diazacycloheptatrienyl, azacyclooctatetraenyl, etc.

A bond in a structural formula represented herein by a wavy line "~~" is intended to indicate that the structure represents cis or trans isomer, or a mixture of cis and trans isomers in any ratio.

The term "the ratio of drug to antibody" or "DAR" refers to the amount of drug, e.g., a small molecule toxin attached to the antibody of the ADC. The DAR of an ADC can range from 1 to 16, but depends on the number of attachment sites in the antibody, thus higher loads (e.g. 20) are also possible. The term DAR can be used when referring to the amount of drug loaded into a single antibody, or alternatively, when referring to the average or mean DAR of a group of ADCs.

### Beneficial effects of the invention:

Through extensive research, the present invention provided: highly potent and high cell permeable bioactive drugs (bioactive molecules or payloads); linkers with high *in vivo* systemic circulation stability and high chemical stability; linker driven ADC enrichment in tumor microenvironment through linkers' physicochemical properties adjustment, such as basicity and lipophilicity; the unique *in vivo* linker cleavage characteristics; coupling modes between the targeting moieties and inkers. The above discoveries combined with a large number of *in vitro* and *in vivo* screenings and testings, provided a novel class of antibody drug conjugates which offers multiple of the following surprising technical advantages:
The conjugate products obtained according to the above method has better solubility and excellent chemical stability, avoiding retro-Michael reaction of traditional maleimide based ADCs. As such, the ADC products of current disclosure can have high drug-to-antibody ratio (DAR). In some embodiments, the DAR value of the conjugates can reach 6-8;
High coupling efficiency. In some embodiments, the coupling efficiency can reach or exceed 90%;
Through a large number of experimental studies, a class of highly plasma stable linker was discovered, which can be cleaved in the tumor microenvironment both intracellularly and extracellularly. The ADC products of current disclosure can achieve good anti-tumor effect in tumors with low or no antigen expression;
By adjusting the physical and chemical properties of the linker and the overall ADC molecule, the ADC products obtained according to the above method increase the exposure in the relatively acidic tumor environment, so ADC products of current disclosure have better tumor tissue targeting. The enrichment ability of ADC in the tumor microenvironment increases the concentration ratio of payload between tumor and blood. It reduces ADC mechanism related toxicity (i.e., the toxicity of the ADC after binding to cell surface antigens in non-tumor tissues and endocytosis, or referred to as "on-target toxicity"), so it can achieve a higher therapeutic index;
The conjugates obtained according to the above method have high stability in circulation *in vivo,* low cleavage of drug molecules in non-target tissues, and thus weakens the "off-target" toxicity caused by the cleavage of toxins in non-target tissues;
The bioactive molecule of the conjugate has higher anti-tumor cell activity, so it has an excellent by-stander effect, and the ADC can more effectively kill tumor cells with high antigen expression, as well as tumor cells with low or no antigen expression in tumor tissue;
By utilizing the extracellular cleavage ability of the invented linker in the tumor microenvironment, the toxin-linker of the present disclosure may form an antibody drug conjugate with an antibody that has no endocytosis ability, such an antibody drug conjugate has high *in vivo* anti-tumor activity;
By utilizing both extracellular cleavage ability and the tumor microenvironment enrichment ability, the toxin-linker of the present disclosure may form an antibody drug conjugate with an antibody that has no endocytosis ability or is incapable of cell surface antigen binding, such an antibody drug conjugate has high *in vivo* anti-tumor activity;
the antibodies provided in the present disclosure are highly humanized, or fully human antibodies, so that they can be safely administered to human subjects without eliciting an immunogenic response. The molecules have high binding capacity to human and non-human B7H3, and antibody molecules having cross-reactivity with B7H3 of non-human primates (e.g., cynomolgus monkeys) are particularly involved;
In conclusion, the linker, antibody and ADC of the present disclosure have great clinical value.

### Description of Figures

Figure 1A. Binding detection of anti-B7H3 antibody 1D1-01 to human B7H3-4IgG-His protein by ELISA.
Figure 1B. Binding detection of anti-B7H3 antibody 2E3-02 to human B7H3-4IgG-His protein by ELISA.
Figure 2A. Binding detection of anti-B7H3 antibody 1D1-01 to monkey B7H3-4IgG-His protein by ELISA.
Figure 2B. Binding detection of anti-B7H3 antibody 2E3-02 to monkey B7H3-4IgG-His protein by ELISA.
Figure 3A. Binding detection of anti-B7H3 antibody 1D1-01 to MCF-7 tumor cells by flow cytometry.
Figure 3B. Binding detection of anti-B7H3 antibody 2E3-02 to MCF-7 tumor cells by flow cytometry.
Figure 4A. Binding detection of anti-B7H3 antibody 1D1-01 to A549 tumor cells by flow cytometry.
Figure 4B. Binding detection of anti-B7H3 antibody 2E3-02 to A549 tumor cells by flow cytometry.
Figure 5. Binding detection of anti-B7H3 antibody 2E3-02 to PC-3 tumor cells by flow cytometry.
Figure 6A. Detection of specific binding of anti-B7H3 antibody 1D1-01 to B7H3 protein.
Figure 6B. Detection of specific binding of anti-B7H3 antibody 2E3-02 to B7H3 protein.
Figure 7A. Detection of endocytosis of tumor cells A549 for candidate antibody 1D1-01.
Figure 7B. Detection of endocytosis of tumor cells A549 for candidate antibody 2E3-02.
Figure 7C. Detection of endocytosis of tumor cells A549 for candidate antibody 202-2-1.
Figure 8. SEC profiles before and after conjugation.
Figure 9. Assay I for anti-B7H3-ADC to inhibit tumor growth in NCI-HT29 xenograft model.
Figure 10. Assay II for anti-B7H3-ADC to inhibit tumor growth in NCI-HT29 xenograft model.
Figure 11. The assay for anti-B7H3-ADC to inhibit tumor growth in NCI-H358 xenograft model.
Figure 12. The assay for Anti-B7H3-ADC to inhibit tumor growth in esophageal squamous cell carcinoma PDX model.
Figure 13. The assay for anti-B7H3-ADC to inhibit tumor growth in prostate PDX model.
Figure 14. The assay for anti-Her3-ADC to inhibit tumor growth in NCI-H358 xenograft model.
Figure 15. Assay I for anti-Her3-ADC to inhibit tumor growth in SW480 xenograft model.
Figure 16. Assay II for anti-Her3-ADC to inhibit tumor growth in SW480 xenograft model.
Figure 17. Distribution ratio of A1.9 in plasma and tumor in HT29 model.
Figure 18. Distribution ratio of ADC in plasma and tumor in HT29 model.
Figure 19A. Detection results of incubation of antibody drug conjugates in tumor homogenate.
Figure 19B. Incubation results of antibody drug conjugates in muscle tissue homogenate.

### Examples

By following description of specific examples, the present disclosure is further illustrated, but this is not intended to limit the present disclosure. Based on the teachings of the present disclosure, those skilled in the art may make various modifications or improvements without departing from the basic spirit and scope of the present disclosure. The reagents or instruments used without the manufacturer's indication are conventional products that can be commercially available.

The abbreviations in this disclosure have the following meanings:

| | | | |
|---|---|---|---|
| OMs | Methanesulfonate | FA | Formic acid |
| OTs | p-toluenesulfonate | ACN | Acetonitrile |
| OTf | Trifluoromethanesulfonate | CCK8 reagent | 2-(2-methoxy-4-nitrophenyl)-3-(4-nitrophenyl)-5-(2,4- disulfophenyl)-2H-tetrazolium monosodium salt |
| TBS | tert-butyldimethylsilyl | FBS | fetal bovine serum |
| MMT | p-methoxyphenyldiphenylmethyl | DMSO | dimethyl sulfoxide |
| PB/PBS | Phosphate buffer | HBTU | O-benzotriazolyl-tetramethyluronium hexafluorophosphate |
| HOBT | 1-Hydroxybenzotriazole | HATU | 2-(7-aza-benzotriazolyl)-N,N,N',N'-tetramethyl uronium hexafluorophosphate |
| NBS | N-Bromosuccinimide | TFA | Trifluoroacetic acid |
| PPTS | Pyridine p-toluenesulfonate | DCM | dichloromethane |
| THF | Tetrahydrofuran | DMF | N,N-Dimethylformamide |
| EDC | 1-(3-Dimethylaminopropyl)-3-ethyl carbodiimide hydrochloride | IgG | Immunoglobulin G |
| CDR | Complementarity determining regions in immunoglobulin variable regions | HRP | Horseradish peroxidase |
| FR | Antibody framework region: amino acid residues other than CDR residues in the variable region of an antibody | hFc | Human IgG antibody Fc fragment |
| VH | Heavy chain variable region of antibody | KD | Equilibrium dissociation constant |
| VL | Light chain variable region of antibody | HCDR1 | Complementarity determining region 1 in the heavy chain variable region of an immunoglobulin |
| AbM | The definition of AbM CDR is originated from Martin's related research (Martin ACR, Cheetham JC, Rees AR (1989) Modelling antibody hypervariable loops: A combined algorithm. Proc Natl Acad Sci USA 86: 9268-9272), and this definition method integrates some of the definitions of Kabat and Chothia. | HCDR2 | Complementarity determining region 2 in the heavy chain variable region of an immunoglobulin |
| Kabat | Immunoglobulin alignment and numbering system by Elvin A. Kabat (see, such as Kabat et al., Sequences of Proteins of Immunological Interest, 5th Ed. Public Health Service, National Institutes of Health, Bethesda, Md., 1991). | HCDR3 | Complementarity determining region 3 in the heavy chain variable region of an immunoglobulin |
| Chothia | The immunoglobulin numbering system proposed by Chothia et al., which is a classical rule for identifying CDR region boundaries based on the position of structural loop regions (see, such as Chothia & Lesk (1987) J. Mol. Biol. 196:901-917; Chothia et al., (1989) Nature 342:878-883). | LCDR1 | Complementarity determining region 1 in the light chain variable region of an immunoglobulin |
| IMGT | Based on the numbering system of the International ImMunoGeneTics information system ^{®} (IMGT) sponsored by Lefranc et al, see Lefranc et al., Dev. Comparat. Immunol. 27:55-77, 2003. | LCDR2 | Complementarity determining region 2 in the light chain variable region of an immunoglobulin |
| mAb | Monoclonal antibodies | LCDR3 | Complementarity determining region 3 in the light chain variable region of an immunoglobulin |
| EC50 | Concentration to produce 50% efficacy or binding | SEC-HPLC | Size exclusion high performance liquid chromatography |
| ELISA | Enzyme linked immunosorbent assay | HC | Heavy chain of immunoglobulin |
| PCR | polymerase chain reaction | LC | Light chain of immunoglobulin |

### Sequence and its specific information:

| **SEQ ID NO** | **Sequence name** | **Amino acid sequence** |
|---|---|---|
| 1 | scFv 1D1 | EGQLVESGGGLVQPGGSLRLSCAASGFTISSYWMNWVRQAPGKGLEWVA NIKQDGSEKYYVDSVQGRFTISRDNAKNSLYLQMNSLRAEDTAVYYCAR RMYSSGWYWGAFDMWGQGTMVTVSSGGGGSGGGGSGGGGSEIVLTQSP GTLSLSPGERATLSCRASQSVSSIYLAWNQQKPGQAPRLLIYGTFKRATGIP DRFSGSGSGTDFTLTISSLEPEDFAVYYCQQYDTSSITFGQGTRLEIK |
| 2 | scFv 2E3 | EVQLLESGGGLVQPGGSLRLSCAASGFTFSSYAMNWVRQAPGKGLEWVS GISGSGGSTYYADSVQGRFTISRDNSKNTLYLQMNSLRAEDTAVYYCAKA RSGYDYFFDYWGQGTLVTVSSGGGGSGGGGSGGGGSEIVLTQSPGTLSLS PGERATLSCRASQRISSSFLAWYQQKPGQAPSLLIYGASSRATGIPDRFSGS GSGTDFTLTISSLEPEDFAVFYCQQYSNSPLTFGGGTKVEIK |
| 3 | 1D1-01 VH | EGQLVESGGGLVQPGGSLRLSCAASGFTISSYWMNWVRQAPGKGLEWVA NIKQDGSEKYYVDSVQGRFTISRDNAKNSLYLQMNSLRAEDTAVYYCAR RMYSSGWYWGAFDMWGQGTMVTVSS |
| 4 | 1D1-01 chothia HCDR1 | GFTISSY |
| 5 | 1D1-01 chothia HCDR2 | KQDGSE |
| 6 | 1D1-01 chothia HCDR3 | RMYSSGWYWGAFDM |
| 7 | 1D1-01 Kabat HCDR1 | SYWMN |
| 8 | 1D1-01 Kabat HCDR2 | NIKQDGSEKYYVDSVQG |
| 9 | 1D1-01 Kabat HCDR3 | RMYSSGWYWGAFDM |
| 10 | 1D1-01 IMGT HCDR1 | GFTISSYW |
| 11 | 1D1-01 IMGT HCDR2 | IKQDGSEK |
| 12 | 1D1-01 IMGT HCDR3 | ARRMYSSGWYWGAFDM |
| 13 | 1D1-01 VL | EIVLTQSPGTLSLSPGERATLSCRASQSVSSIYLAWNQQKPGQAPRLLIYGT FKRATGIPDRFSGSGSGTDFTLTISSLEPEDFAVYYCQQYDTSSITFGQGTRL EIK |
| 14 | 1D1-01 chothia LCDR1 | RASQSVSSIYLA |
| 15 | 1D1-01 chothia LCDR2 | GTFKRAT |
| 16 | 1D1-01 chothia LCDR3 | QQYDTSSIT |
| 17 | 1D1-01 Kabat LCDR1 | RASQSVSSIYLA |
| 18 | 1D1-01 Kabat LCDR2 | GTFKRAT |
| 19 | 1D1-01 Kabat LCDR3 | QQYDTSSIT |
| 20 | 1D1-01 IMGT LCDR1 | QSVSSIY |
| \ | 1D1-01 IMGT LCDR2 | GTF |
| 21 | 202-2-1 Kabat/IM GT LCDR3 | QQLNSYPLT |
| 22 | 1D1-01 IMGT LCDR3 | QQYDTSSIT |
| 23 | 2E3-02 VH | EVQLLESGGGLVQPGGSLRLSCAASGFTFSSYAMNWVRQAPGKGLEWVS GISGSGGSTYYADSVQGRFTISRDNSKNTLYLQMNSLRAEDTAVYYCAKA RSGYDYFFDYWGQGTLVTVSS |
| 24 | 2E3-02 chothia HCDR1 | GFTFSSY |
| 25 | 2E3-02 chothia HCDR2 | SGSGGS |
| 26 | 2E3-02 chothia HCDR3 | ARSGYDYFFDY |
| 27 | 2E3-02 Kabat HCDR1 | SYAMN |
| 28 | 2E3-02 Kabat HCDR2 | GISGSGGSTYYADSVQG |
| 29 | 2E3-02 Kabat HCDR3 | ARSGYDYFFDY |
| 30 | 2E3-02 IMGT HCDR1 | GFTFSSYA |
| 31 | 2E3-02 IMGT HCDR2 | ISGSGGST |
| 32 | 2E3-02 IMGT HCDR3 | AKARSGYDYFFDY |
| 33 | 2E3-02 VL | EIVLTQSPGTLSLSPGERATLSCRASQRISSSFLAWYQQKPGQAPSLLIYGA SSRATGIPDRFSGSGSGTDFTLTISSLEPEDFAVFYCQQYSNSPLTFGGGTK VEIK |
| 34 | 2E3-02 chothia LCDR1 | RASQRISSSFLA |
| 35 | 2E3-02 chothia LCDR2 | GASSRAT |
| 36 | 2E3-02 chothia LCDR3 | QQYSNSPLT |
| 37 | 2E3-02 Kabat LCDR1 | RASQRISSSFLA |
| 38 | 2E3-02 Kabat LCDR2 | GASSRAT |
| 39 | 2E3-02 Kabat LCDR3 | QQYSNSPLT |
| 40 | 2E3-02 IMGT LCDR1 | QRISSSF |
| \ | 2E3-02 IMGT LCDR2 | GAS |
| 41 | 202-2-1 IMGT LCDR1 | QGISSY |
| 42 | 2E3-02 IMGT LCDR3 | QQYSNSPLT |
| 43 | IgG1 CH (namely , IgG1 constant region) | |
| 44 | Kappa CL (namely, κ constant region) | |
| 45 | 1D1-01 HC ( namely 1D1-01 Heavy Chain) | EGQLVESGGGLVQPGGSLRLSCAASGFTISSYWMNWVRQAPGKGLEWVA NIKQDGSEKYYVDSVQGRFTISRDNAKNSLYLQMNSLRAEDTAVYYCAR RMYSSGWYWGAFDMWGQGTMVTVSSASTKGPSVFPLAPSSKSTSGGTA ALGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 46 | 1D1-01 LC (namely 1D1-01 Light Chain) | EIVLTQSPGTLSLSPGERATLSCRASQSVSSIYLAWNQQKPGQAPRLLIYGT FKRATGIPDRFSGSGSGTDFTLTISSLEPEDFAVYYCQQYDTSSITFGQGTRL EIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSP VTKSFNRGEC |
| 47 | 2E3-02 HC (namely 2E3-02 Heavy Chain) | EVQLLESGGGLVQPGGSLRLSCAASGFTFSSYAMNWVRQAPGKGLEWVS GISGSGGSTYYADSVQGRFTISRDNSKNTLYLQMNSLRAEDTAVYYCAKA RSGYDYFFDYWGQGTLVTVSSASTKGPSVFPLAPSSKSTSGGTAALGCLV KDYFPEPVTVSWNSGALTSGVHTFPAVLQSSGLYSLSSVVTVPSSSLGTQT YICNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 48 | 2E3-02 LC (namely 2E3-02 Light Chain) | EIVLTQSPGTLSLSPGERATLSCRASQRISSSFLAWYQQKPGQAPSLLIYGA SSRATGIPDRFSGSGSGTDFTLTISSLEPEDFAVFYCQQYSNSPLTFGGGTK VEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC |
| 49 | 202-2-1 VH | EVQLVESGGGVVQPGRSLRLSCAASGFTFSTYGMHWVRQAPGKGLEWV AVIWYDVSHKYYADSVKGRFTISRDNSKNTLSLQMNSLRAEDTAVYYCA RDWGDPDAFDIWGQGTMVTVSS |
| 50 | 202-2-1 VL | DIQLTQSPSFLSASVGDRVTITCRASQGISSYLAWYQQKPGKAPKLLIYAAS TLQSGVPSRFSGSGSGTEFTLTISSLQPEDFATYYCQQLNSYPLTFGGGTKV EIK |
| 51 | 202-2-1 HC (namely 202-2-1 Heavy Chain)) | EVQLVESGGGVVQPGRSLRLSCAASGFTFSTYGMHWVRQAPGKGLEWV AVIWYDVSHKYYADSVKGRFTISRDNSKNTLSLQMNSLRAEDTAVYYCA RDWGDPDAFDIWGQGTMVTVSSASTKGPSVFPLAPSSKSTSGGTAALGCL VKDYFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFPPKP KDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG K |
| 52 | 202-2-1 LC (namely 202-2-1 Light Chain) | DIQLTQSPSFLSASVGDRVTITCRASQGISSYLAWYQQKPGKAPKLLIYAAS TLQSGVPSRFSGSGSGTEFTLTISSLQPEDFATYYCQQLNSYPLTFGGGTKV EIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSP VTKSFNRGEC |
| 53 | 202-2-1 Kabat HCDR1 | TYGMH |
| 54 | 202-2-1 Kabat HCDR2 | VIWYDVSHKYYADSVKG |
| 55 | 202-2-1 Kabat HCDR3 | DWGDPDAFDI |
| 56 | 202-2-1 IMGT HCDR1 | GFTFSTYG |
| 57 | 202-2-1 IMGT HCDR2 | IWYDVSHK |
| 58 | 202-2-1 IMGT HCDR3 | ARDWGDPDAFDI |
| 59 | 202-2-1 Kabat LCDR1 | RASQGISSYLA |
| 60 | 202-2-1 Kabat LCDR2 | AASTLQS |
| \ | 202-2-1 IMGT LCDR2 | AAS |

The present disclosure is now described with reference to the following examples intended to illustrate the present disclosure (not to limit the present disclosure).

Unless otherwise specified, the molecular biology experimental methods and immune detection methods used in the present disclosure are basically referred to the methods described in J. Sambrook et al., Molecular Cloning: Laboratory Handbook, 2nd Edition, Cold Spring Harbor Laboratory Press, 1989, and F. M. Ausubel et al., Short Protocols in Molecular Biology, 3rd Edition, John Wiley & Sons, Inc., 1995. Those skilled in the art know that the examples illustrate the present disclosure by way of examples, and are not intended to limit the protection scope claimed in the present disclosure.

### Preparation procedures

The structures of the compounds described in the following examples were determined by nuclear magnetic resonance (¹H NMR) or mass spectrometry (MS).

The instrument for detecting nuclear magnetic resonance (¹H NMR) is a Bruker 400 MHz nuclear magnetic resonance instrument; the test solvent is deuterated methanol (CD₃OD), deuterated chloroform (CDCl₃) or hexadeuterodimethyl sulfoxide (DMSO-d₆); the internal standard substance is tetramethylsilane (TMS).

Abbreviations in nuclear magnetic resonance (NMR) spectra used in the examples are shown below.
s: singlet, d: doublet, t: triplet, q: quartet, dd: double doublet, qd: quartet doublet, ddd: double double doublet, ddt: double double triplet, dddd: double double double doublet, m: multiplet, br: broad, J: coupling constant, Hz: hertz, DMSO-d₆: deuterodimethyl sulfoxide. δ value is expressed in ppm.

An Agilent (ESI) mass spectrometer (model Agilent 6120B) was used as the instrument to test mass spectrometry (MS).

### I. Synthesis of bioactive molecules and intermediates used in the synthetic procedures of "drug-linker compounds"

### A. Synthesis of bioactive molecules

### Example A1.1: Synthesis of (S)-4-ethyl-8-fluoro-4-hydroxy-9-methyl-11-(1H-pyrazol-4-yl)-1,12-dihydro-14H-pyrano[3',4':6,7]indolizino[1,2-b]quinolin-3,14(4H)-dione (A1.1)

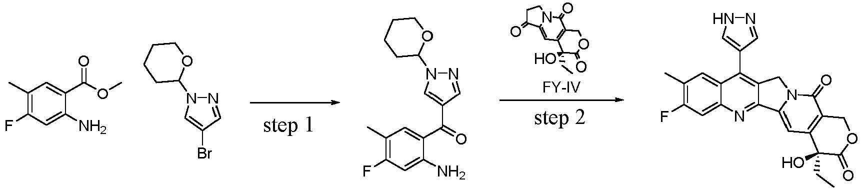

Step 1: 4-Bromo-1-(tetrahydro-2*H*-pyran-2-yl)-1H-pyrazole (4.6 g) was dissolved in anhydrous tetrahydrofuran (50 ml), and the solution was cooled to -78°C in dry ice-acetone under the protection of nitrogen, to which was then added n-butyl lithium (12 ml, 2 M) dropwise. The reaction solution was stirred at -78 °C for 20 minutes, and then methyl 2-amino-4-fluoro-5-methylbenzoate (1.83 g) was added. After addition, the reaction was naturally warmed up to room temperature and stirred continuously for 5 hours. The reaction was quenched by addition of methanol (3ml), and then ethyl acetate (200 ml) was added. The solution was washed with water (100 ml x 3), and then the organic phase was dried. The organic solvent was removed, and the residue was separated by silica gel column chromatography to obtain the target product (2-amino-4-fluoro-5-methylphenyl)(1-(tetrahydro-2H-pyran-2-yl)-1H-pyrazol-4-yl)methanone, ESI-MS (*m*/*z*)*:* 304 [M+H]⁺.

Step 2: (S)-4-Ethyl-4-hydroxy-7,8-dihydro-1H-pyrano[3,4-f]indolizin-3,6,10(4H)-trione (2.63g), (2-amino-4-fluoro-5-methylphenyl)(1-(tetrahydro-2H-pyran-2-yl)-1H-pyrazol-4-yl)methanone (3.03g), and p-toluenesulfonic acid (1.74 g) were dissolved in dichloromethane (50 ml), and then the solvent was removed. The mixture was heated to 120 °C under the protection of nitrogen and allowed to react for 4 hours. The mixture was dissolved in ethyl acetate (300 ml), and after the organic phase was washed with water (100 ml x 2) and dried, the organic solvent was removed. The residue was separated by silica gel column chromatography to obtain the target product (S)-4-ethyl-8-fluoro-4-hydroxy-9-methyl-11-(1H-pyrazol-4-yl)-1,12-dihydro-14H-pyrano[3',4':6,7]indolizino[1,2-b]quinolin-3,14(4H)-dione (A1.1). ESI-MS (*m*/*z*): 447 [M+H]⁺.

### Example A1.2: Synthesis of (S)-7-ethyl-7-hydroxy-14-(1H-pyrazol-4-yl)-10,13-dihydro-11H-[1,3]dioxolo[4,5-g]pyrano[3',4':6,7]indolizino[1,2-b]quinolin-8,11(7H)-dione (A1.2)

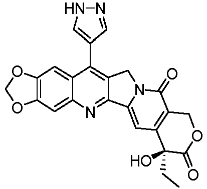

Using the same method and reaction conditions of Example A1.1, methyl 2-amino-4-fluoro-5-methylbenzoate was replaced by methyl 6-aminobenzo[d][1,3]dioxolan-5-carboxylate, to obtain the target product (S)-7-ethyl-7-hydroxy-14-(1H-pyrazol-4-yl)-10,13-dihydro-11H-[1,3]dioxolo[4,5-g]pyrano[3',4':6,7]indolizino[1,2-b]quinolin-8,11(7H)-dione (A1.2). ESI-MS (*m*/*z*)*:* 459 [M+H]⁺.

### Example A1.3: Synthesis of (S)-14-(3-aminophenyl)-7-ethyl-7-hydroxy-10,13-dihydro-11H-[1,3]dioxolo[4,5-g]pyrano[3',4':6,7]indolizino[1,2-b]quinolin-8,11(7H)-dione (A1.3)

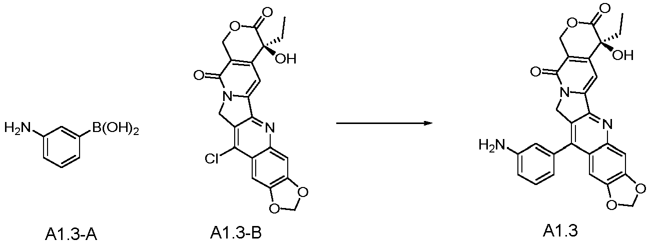

Compound (A1.3-A) (1.4 g), compound (A1.3-B) (2.2 g), Pd₂(DBA)₃ (300 mg), tricyclohexylphosphane (300 mg), and potassium acetate (1.1 g) were successively added to the mixed solvent of dioxane (30 ml) and water (5 ml), and the mixture was heated to 100 °C and reacted for 12 h under stirring and the protection of nitrogen. After cooling, ethyl acetate (200 ml) was added, and then the resultant solution was washed with water (100 ml). After drying, the target product (S)-14-(3-aminophenyl)-7-ethyl-7-hydroxy-10,13-dihydro-11H-[1,3] dioxolo[4,5-g]pyrano[3',4':6,7]indolizino[1,2-b]quinolin-8,11(7H)-dione (A1.3) was obtained by separation over silica gel column chromatography. ESI-MS (m/z): 484 [M+H]⁺.

### Example A1.4: Synthesis of (S)-14-(4-aminophenyl)-7-ethyl-7-hydroxy-10,13-dihydro-11H-[1,3]dioxolo[4,5-g]pyrano[3',4':6,7]indolizino[1,2-b]quinolin-8,11(7H)-dione (A1.4)

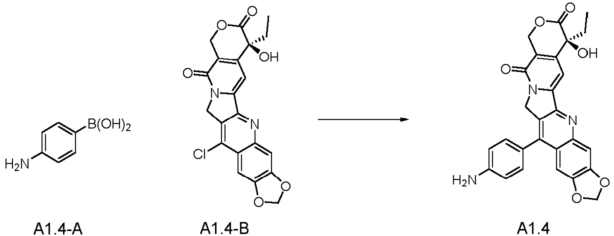

Compound (A1.4-A) (1.4 g), compound (A1.4-B) (2.2 g), Pd₂(DBA)₃ (300 mg), tricyclohexylphosphane (300 mg), and potassium acetate (1.1 g) were successively added to the mixed solvent of dioxane (30 ml) and water (5 ml), and the mixture was heated to 100 °C and reacted for 12 h under stirring and the protection of nitrogen. After cooling, ethyl acetate (200 ml) was added, and then the resultant solution was washed with water (100 ml). After drying, the target product (S)-14-(4-aminophenyl)-7-ethyl-7-hydroxy-10,13-dihydro-11H-[1,3] dioxolo[4,5-g]pyrano[3',4':6,7]indolizino[1,2-b]quinolin-8,11(7H)-dione (A1.4) was obtained by separation over silica gel column chromatography.

ESI-MS (m/z): 484 [M+H]⁺;
¹H NMR (400 MHz, DMSO) δ 7.56 (s, 1H), 7.34 (d, *J =* 8.0 Hz, 2H), 7.27 (s, 1H), 7.14 (s, 1H), 6.89 (d*, J* = 7.8 Hz, 2H), 6.26 (s, 2H), 5.40 (s, 2H), 5.05 (s, 2H), 1.96 - 1.78 (m, 2H), 0.88 (t, *J =* 7.2 Hz, 3H).

### Example A1.5: Synthesis of (S)-14-(3-aminopropyl)-7-ethyl-7-hydroxy-10,13-dihydro-11H-[1,3]dioxolo[4,5-g]pyrano[3',4':6,7]indolizino[1,2-b]quinolin-8,11(7H)-dione (A1.5)

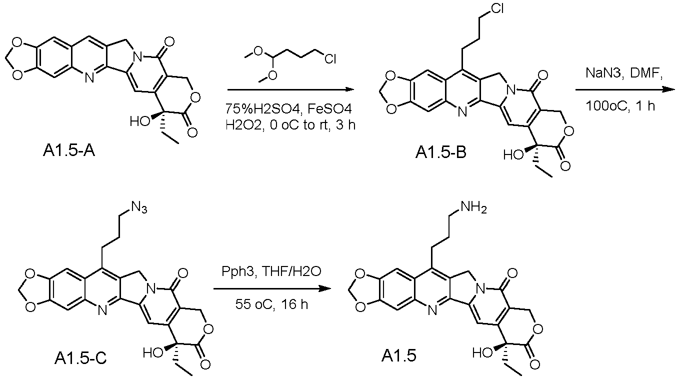

### Step 1: Synthesis of (S)-14-(3-chloropropyl)-7-ethyl-7-hydroxy-10,13-dihydro-11H-[1,3]dioxolo[4,5-g]pyrano[3',4':6,7]indolizino[1,2-b]quinolin-8,11(7H)-dione

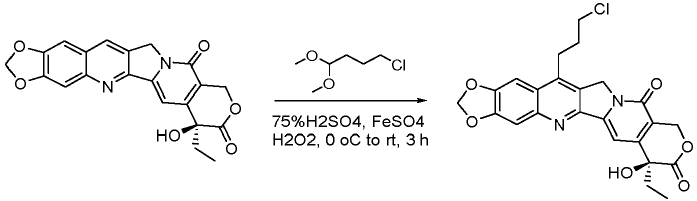

In ice bath, to the solution of compound (S) 7-ethyl-7-hydroxy-10,13-dihydro-11H-[1,3]dioxolo[4,5-g]pyrano[3',4':6,7]indeno[1,2-b]quinolin-8,11(7H)-dione (A1.5-A, 500 mg) in 75% sulfuric acid solution (5 mL), was added ferrous sulfate heptahydrate (570 mg of ferrous sulfate heptahydrate was dissolved in 1 mL of water) and 4-dimethoxychlorobutane (3.89 g), and after the reaction solution was stirred for 3 minutes, hydrogen peroxide (29%, 2.5 mL) was added dropwise. The reaction solution was stirred at 0 °C for 5 minutes, warmed to room temperature, and then reacted for 3 h under stirring. The reaction solution was diluted with water (50 mL), and extracted with ethyl acetate (80 mL × 2). The organic phase was washed with saturated brine, dried over anhydrous sodium sulfate, and concentrated to obtain the crude product, which was further purified by C18 column (acetonitrile/0.05% formic acid aqueous solution: 5%-60%), to obtain the target compound (yellow solid, 400 mg, yield: 67%).

LCMS (ESI) [M+H]⁺: 468.9;
¹H NMR (400 MHz, DMSO-d6) δ 7.65 (s, 1H), 7.51 (s, 1H), 7.24 (s, 1H), 6.50 (s, 1H), 6.30 (s, 2H), 5.42 (s, 2H), 5.26 (s, 2H), 3.81 (d, *J =* 5.9 Hz, 2H), 3.22 (s, 2H), 1.98 (d, *J =* 6.7 Hz, 4H), 0.88 (t, *J =* 7.2 Hz, 3H).

### Step 2: Synthesis of (S)-14-(3-azidopropyl)-7-ethyl-7-hydroxy-10,13-dihydro-11H-[1,3]dioxolo[4,5-g]pyrano[3',4':6,7]indolizino[1,2-b]quinolin-8,11(7H)-dione

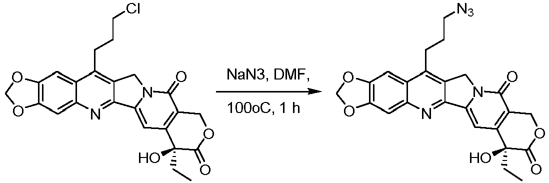

To the solution of compound (S)-14-(3-chloropropyl)-7-ethyl-7-hydroxy-10,13-dihydro-11H-[1,3]dioxolo[4,5-g]pyrano[3',4':6,7]indolizino[1,2-b]quinolin-8,11(7H)-dione (200 mg) in N,N-dimethylformamide (3 mL), was added sodium azide (284 mg), and then the reaction solution was stirred for 1 h at 100 °C. Water (30 mL) was added to the reaction solution, and the resultant solution was extracted with ethyl acetate (60 mL × 2). The organic phase was washed with saturated brine, dried over anhydrous sodium sulfate, and concentrated to obtain the target compound (S)-14-(3-azidopropyl)-7-ethyl-7-hydroxy-10,13-dihydro-11H-[1,3]dioxolo[4,5-g]pyrano[3',4':6,7]indolizino[1,2-b]quinolin-8,11(7H)-dione (yellow solid, 180 mg, yield: 88%).

LCMS (ESI) [M+H]⁺: 476;
¹H NMR (400 MHz, DMSO-d6) δ 7.65 (s, 1H), 7.51 (s, 1H), 7.24 (s, 1H), 6.48 (s, 1H), 6.29 (s, 2H), 5.42 (s, 2H), 5.25 (s, 2H), 3.53 - 3.49 (m, 2H), 3.16 - 3.12 (m, 2H), 1.93 - 1.80 (m, 4H), 0.88 (t, *J =* 7.2 Hz, 3H).

### Step 3: Synthesis of (S)-14-(3-aminopropyl)-7-ethyl-7-hydroxy-10,13-dihydro-11H-[1,3]dioxolo[4,5-g]pyrano[3',4':6,7]indolizino[1,2-b]quinolin-8,11(7H)-dione

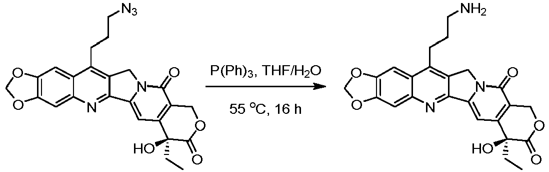

To the solution of compound (S)-14-(3-azidopropyl)-7-ethyl-7-hydroxy-10,13-dihydro-11H-[1,3]dioxolo[4,5-g]pyrano[3',4':6,7]indolizino[1,2-b]quinolin-8,11(7H)-dione (130 mg, 0.274 mmol) in the mixed solvent of tetrahydrofuran (3 mL) and water (1 mL), was added triphenylphosphine (108 mg, 0.411 mmol), and then the mixture was allowed to react at 55 °C for 16 h. LCMS indicated the reaction was completed. Water (5 mL) was added to the reaction solution, and the resultant solution was acidified with 2N hydrochloric acid (3 mL), and then extracted with ethyl acetate (10 mL). The aqueous phase was purified by high performance liquid chromatography (HPLC) (acetonitrile/0.05% formic acid in water), to obtain the target compound (S)-14-(3-aminopropyl)-7-ethyl-7-hydroxy-10,13-dihydro-11H-[1,3]dioxolo[4,5-g]pyrano[3',4':6,7]indolizino[1,2-b]quinolin-8,11(7H)-dione (A1.5, yellow solid, 15 mg, yield: 12%).

LCMS (ESI) [M+H]⁺: 449.9;
¹H NMR (400 MHz, DMSO-d6) δ 7.72 (s, 3H), 7.53 (s, 1H), 7.25 (s, 1H), 6.50 (s, 1H), 6.30 (s, 2H), 5.43 (s, 2H), 5.24 (s, 2H), 3.15 (d, *J =* 6.4 Hz, 2H), 3.03 (t, *J =* 6.9 Hz, 2H), 1.96 - 1.81 (m, 4H), 0.88 (t, *J =* 7.3 Hz, 3H).

### Example A1.6: Synthesis of (S)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.6)

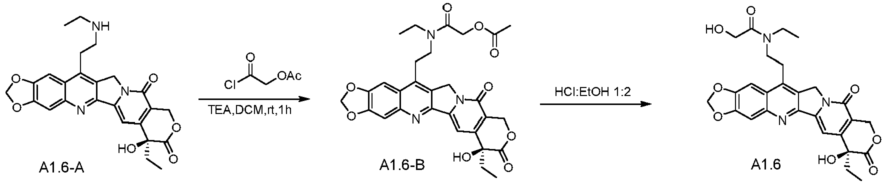

### Step 1: Preparation of (S)-2-(ethyl(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)amino)-2-oxoethyl acetate (A1.6-B):

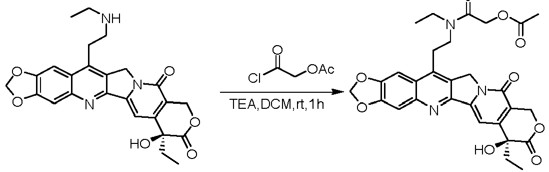

Compound (S)-7-ethyl-14-(2-(ethylamine)ethyl)-7-hydroxy-10,13-dihydro-11H-[1,3]dioxolo[4,5-g]pyrano [3',4':6,7]indolizino[1,2-b]quinolin-8,11(7H)-dione (A1.6-A, 50 mg), acetyloxyacetyl chloride (73 mg), and triethylamine (50 mg) were successively added to dichloromethane (5 mL), and the mixture was stirred and reacted for 1 h at room temperature. The reaction solution was rotatory evaporated to remove the solvent and obtain the crude oily compound 75mg.

LCMS (ESI) [M+H]⁺: 564.2.

### Step 2: Synthesis of (S)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.6)

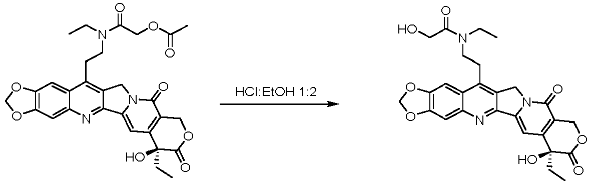

The crude compound (S)-2-(ethyl(2-(7-ethyl-7-hydroxy-8,11-dioxo-7,8,11,13-tetrahydro-10H-[1,3]dioxolo [4,5-g]pyrano[3',4':6,7]indolizino[1,2-b]quinolin-14-yl)ethyl)amino)-2-oxoethyl acetate (75 mg, 0.13 mmol) was dissolved in a mixed solution of concentrated hydrochloric acid and absolute ethanol (volume ratio 1/2, 3 mL), and then the reaction solution was allowed to react at 85 °C for 1 hour under refluxing. LCMS indicated that the reaction was completed. The reaction solution was concentrated, and the crude product was purified by preparative chromatography (0.01% trifluoroacetic acid in water, acetonitrile) to obtain the target compound (S)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.6) (15 mg, yield 26%) as white solid.

LCMS (ESI) [M+H]⁺: 522.0;
¹H NMR (400 MHz, DMSO) δ 7.92 (s, 1H), 7.53 (m, 1H), 7.25 (s, 1H), 6.50 (s, 1H), 6.31 (s, 2H), 5.43 (s, 2H), 5.34 (m, 2H), 4.65 (m, 1H), 4.07 (m, 2H), 3.52 (m, 2H), 3.41 (m, 2H), 2.00 (m, 2H), 1.88 (m, 2H), 1.16 - 1.04 (m, 3H), 0.89 - 0.83 (m, 3H).

### Example A1.7: Synthesis of (S)-N-methyl-N-(2-(7-ethyl-7-hydroxy-8,11-dioxo-7,8,11,13-tetrahydro-10H-[1,3]dioxolo[4,5-g]pyrano[3',4':6,7] indolizino[1,2-b]quinolin-14-yl)ethyl)-2-hydroxyacetamide (A1.7)

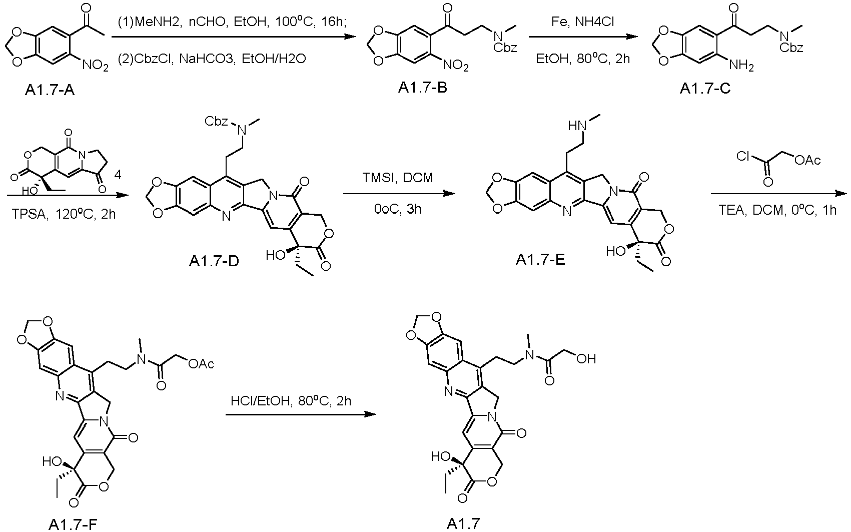

### Step 1: Preparation of compound benzyl methyl(3-(6-nitrobenzo[d][1,3]dioxol-5-yl)-3-oxopropyl)carbamate

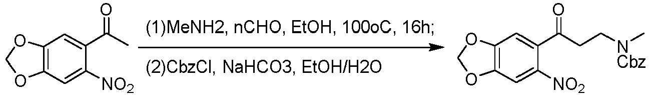

Compound 1-(6-nitrobenzo[d][1,3]dioxin-5-yl)ethane-1-one (A1.7-A, 500 mg), methylamine hydrochloride (1.6 g) and paraformaldehyde (714 mg) were dissolved in ethanol (8 mL), and then the mixture was allowed to react in a sealed vessel at 100 °C for 16 h. The reaction solution was cooled to room temperature and concentrated under reduced pressure. The obtained residue was dissolved in dichloromethane (80 mL), and the organic phase was extracted with water (50 mL × 3). The resultant aqueous phase was adjusted to pH=9 with sodium bicarbonate, and then benzyl chloroformate (513 mg, 3.0 mmol) was added. The reaction was carried out at room temperature for 16 hours. The reaction solution was extracted with ethyl acetate (30 mL × 3), and then the organic phase was washed with saturated brine, dried over anhydrous sodium sulfate, filtered, and concentrated to obtain a crude product, which was separated and purified by C18 column chromatography (acetonitrile/water containing 0.05% formic acid = 5~95%) to obtain the target compound (A1.7-B, 180 mg, yield 23%).

LCMS (ESI) [M+H]⁺: 387.1.

¹H NMR (400 MHz, CDCl₃) δ 7.56 (s, 1H), 7.35 (m, 5H), 7.32 (m, 1H), 6.17 (s, 2H), 5.13 (s, 2H), 3.71 (m, 2H), 3.03 (m, 3H), 2.99 - 2.86 (m, 2H).

### Step 2: Preparation of compound benzyl (3-(6-aminobenzo[d][1,3]dioxin-5-yl)-3-oxopropyl)(methyl)carbamate (A1.7-C)

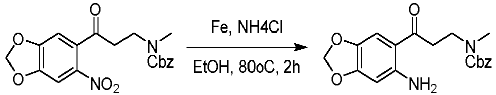

Compound A1.7-B methylbenzyl (3-(6-nitrobenzo[d][1,3]dioxol-5-yl)-3-oxopropyl)carbamate (180 mg, 0.47 mmol) was dissolved in a mixed solution of saturated aqueous ammonium chloride (8 mL) and ethanol (8 mL), and then iron powder (130 mg) was added to the reaction solution. The reaction was stirred at 80°C for 2 hours. The reaction solution was filtered, and the filtrate was concentrated into solid under reduced pressure. The crude product was purified by TLC (petroleum ether:ethyl acetate = 2/1) to obtain the target compound A1.7-C (76 mg).

LCMS (ESI) [M+H]⁺: 357.0.

### Step 3: Preparation of benzyl (S)-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(methyl)carbamate (A1.7-D)

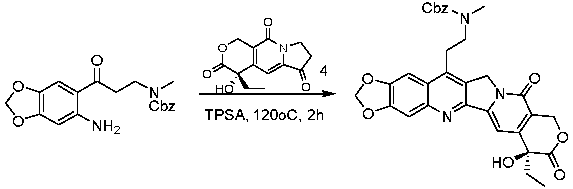

At room temperature, compound A1.7-C benzyl (3-(6-aminobenzo[d][1,3]dioxin-5-yl)-3-oxopropyl)(methyl)carbamate(38 mg), compound (S)-4-ethyl-4-hydroxy-7,8-dihydro-1H-pyrano[3,4-f]indolizin-3,6,10(4H)-trione (29 mg) and p-toluenesulfonic acid (23 mg) were dissolved in dichloromethane (5 mL), and after the solution was clear and mixed homogeneously, it was concentrated under reduced pressure, and pumped to vacuum with an oil pump. The reaction was carried out at 120 °C under vacuum for 2 hours. The reaction was cooled to room temperature, to which was then added water (30 mL), and the resultant mixture was extracted with dichloromethane (30 mL × 3). The combined organic phases were successively dried over anhydrous sodium sulfate, filtered, and the filtrate was concentrated under reduced pressure to obtain the target compound A1.7-D (50 mg).

LCMS (ESI) [M+H]⁺ = 584.0.

### Step 4: Preparation of (S) 7-ethyl-7-hydroxy-14-(2-(methylamino)ethyl)-10,13-dihydro-11H-[1,3]dioxolo[4,5-g]pyrano[3',4':6,7]indolizino[1,2-b]quinolin-8,11(7H)-dione (A1.7-E)

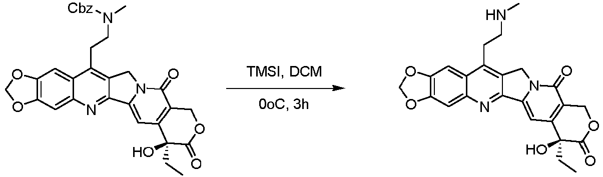

At room temperature, compound A1.7-D benzyl (S)-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(methyl)carbamate (50 mg) was dissolved in dichloromethane (5 mL), to which was added trimethyliodosilane (51 mg, 0.26 mmol) at 0 °C, and then the mixture was allowed to react for 3 h. The reaction solution was concentrated to remove the solvent, and the residue was purified by preparative HPLC (acetonitrile/water containing 0.05% formic acid) to obtain compound A1.7-E (15 mg) as brown solid.

LCMS (ESI) [M+H]⁺: 450.0.

### Step 5: Preparation of compound (S) -2-((2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(methyl)amino)-2-oxoethyl acetate(A1.7-F)

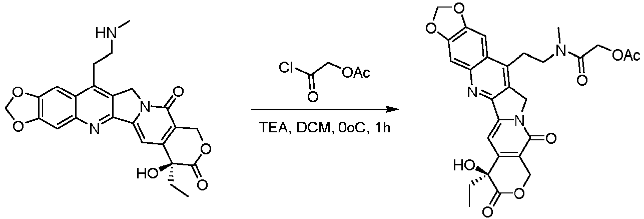

Compound A1.7-E (S) 7-ethyl-7-hydroxy-14-(2-(methylamino)ethyl)-10,13-dihydro-11H-[1,3]dioxolo [4,5-g]pyrano[3',4':6,7]indolizino[1,2-b]quinolin-8,11(7H)-dione (15 mg) and triethylamine (15 mg) were dissolved in dichloromethane (5 mL), to which was added acetyloxyacetyl chloride (20 mg) at 0 °C, and then the mixture was allowed to react for 1 h. The reaction solution was concentrated to remove the solvent and obtain the crude product, which was directly used in the next step without further purification.

LCMS (ESI) [M5+H]⁺ = 550.1.

### Step 6: Preparation of compound (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-methylacetamide (A1.7)

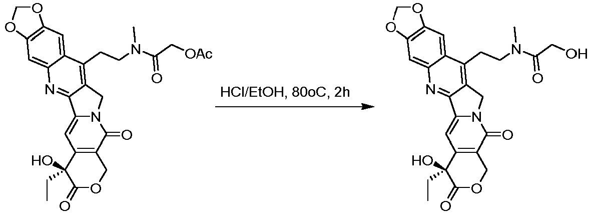

Compound A1.7-F (S)-2-((2-(7-ethyl-7-hydroxy-8,11-dioxo-7,8,11,13-tetrahydro-10H-[1,3]dioxolo[4,5-g] pyrano[3',4':6,7]indolizino[1,2-b]quinolin-14-yl)ethyl)(methyl)amino)-2-oxoethyl acetate (15 mg) was dissolved in ethanol (5 mL), to which was added concentrated hydrochloric acid (1.5 mL), and the mixture was allowed to react at 80 °C for 2 h. The reaction solution was concentrated to remove the solvent, and the residue was purified by preparative HPLC (acetonitrile/water containing 0.05% formic acid) to obtain compound A1.7 (1.6 mg) as white solid.

LCMS (ESI) [M+H]⁺: 508.2.

### Example A1.8: Synthesis of (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-isopropylacetamide (A1.8)

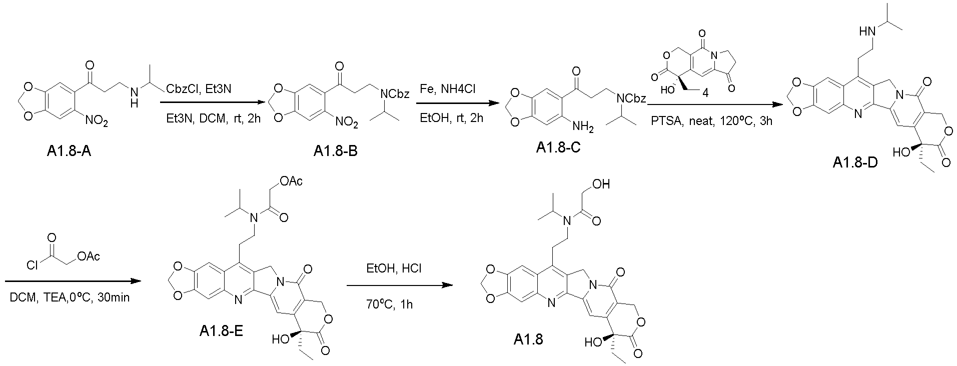

### Step 1: Preparation of isopropylbenzyl (3-(6-nitrobenzo[d][1,3]dioxin-5-yl)-3-oxopropyl)carbamate(A1.8-B)

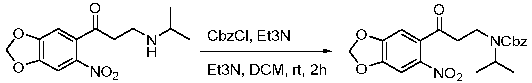

To the solution of compound (A1.8-A) 3-(isopropylamine)-1-(6-nitrobenzo[d][1,3]dioxin-5-yl)propan-1-one (900 mg) in dichloromethane (10 mL), were successively added triethylamine (1.8 g) and benzyloxycarbonyl chloride (734 mg, 4.3 mmol), and then the mixture was allowed to react for 2 h at room temperature. The reaction solution was diluted with water (50 mL), and extracted with dichloromethane (50 mL×3). The organic phase was washed with saturated brine, dried over anhydrous sodium sulfate, and concentrated to obtain a crude product, which was separated and purified by column chromatography (petroleum ether:ethyl acetate = 3/1) to obtain the target compound (A1.8-B) (600 mg).

LCMS (ESI) [M+H]⁺: 414.9;
¹H NMR (400 MHz, DMSO-d6) δ 7.72 (s, 1H), 7.34 (br s, 6H), 6.31 (s, 2H), 5.08 (s, 2H), 4.14 - 4.10 (m, 1H), 3.48 (d, *J =* 7.9 Hz, 2H), 3.03 (s, 2H), 1.13 - 1.11 (m, 6H).

### Step 2: Preparation of compound benzyl (3-(6-aminobenzo[d][1,3]dioxin-5-yl)-3-oxypropyl)(isopropyl)carbamate (A1.8-C)

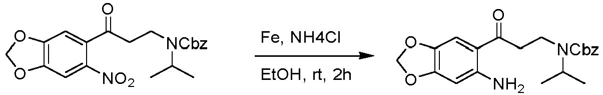

Compound (A1.8-B) isopropylbenzyl (3-(6-nitrobenzo[d][1,3]dioxin-5-yl)-3-oxopropyl)carbamate (200 mg, 0.48 mmol) was dissolved in a mixed solution of saturated ammonium chloride (3 mL) and ethanol (3 mL), and then iron powder (135 mg) was added to the reaction solution. The reaction was stirred at room temperature for 2 hours. The reaction solution was filtered, and then the filtrate was concentrated under reduced pressure to obtain a solid. The crude product was separated and purified by column chromatography (petroleum ether:ethyl acetate=3/1) to obtain the target compound (A1.8-C) (65 mg).

LCMS (ESI) [M+H]⁺: 395.2.

### Step 3: Preparation of (S) 7-ethyl-7-hydroxy-14-(2-(isopropylamine)ethyl)-10,13-dihydro-11H-[1,3]dioxolo[4,5-g]pyrano[3',4':6,7]indolizino[1,2-b]quinolin-8,11(7H)-dione (A1.8-D)

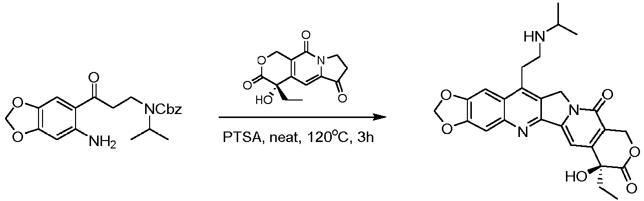

At room temperature, compound (A1.8-C) benzyl (3-(6-aminobenzo[d][1,3]dioxin-5-yl)-3-oxopropyl)(isopropyl)carbamate (65 mg), compound (S)-4-ethyl-4-hydroxy-7,8-dihydro-1H-pyrano[3,4-f]indolizin-3,6,10(4H)-trione (45 mg) and p-toluenesulfonic acid (33 mg, 0.17 mmol) were dissolved in dichloromethane (10 mL), and after the solution was clear and mixed, it was concentrated under reduced pressure, and pumped to vacuum with an oil pump. The reaction was carried out at 120 °C under vacuum for 2 hours. The reaction mixture was cooled to room temperature, to which was then added water (50 mL), and the resultant mixture was extracted with dichloromethane (30 mL × 3). The combined organic phases were successively dried over anhydrous sodium sulfate, filtered, and the filtrate was concentrated under reduced pressure. The crude product was separated and purified by column chromatography (dichloromethane/methanol=10/1), to obtain the target compound (A1.8-D) (40 mg).

LCMS (ESI) [M+H]⁺: 478.0;
¹H NMR (400 MHz, DMSO-d6) δ 8.46 (s, 1H), 7.67 (s, 1H), 7.57 (s, 1H), 7.27 (s, 1H), 6.52 (s, 1H), 6.33 (s, 2H), 5.44 (s, 2H), 5.35 (s, 2H), 3.41 (br s, 2H), 3.23 (br s, 3H), 1.94 - 1.78 (m, 2H), 1.25 (d, *J =* 6.4 Hz, 6H), 0.88 (t, *J* = 7.3 Hz, 3H).

### Step 4: Preparation of compound (S)-2-((2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(isopropyl)amino)-2-oxoethyl acetate (A1.8-E)

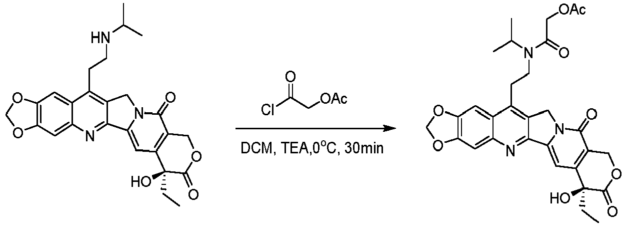

In an ice bath, to the solution of compound (S)-7-ethyl-7-hydroxy-14-(2-(isopropylamino)ethyl)-10,13-dihydro-11H-[1,3]dioxolo[4,5-g]pyrano[3',4':6,7]indolizino[1,2-b]quinolin-8,11(7H)-dione (A1.8-D) (40 mg) in dichloromethane (2 mL), were added triethylamine (35 mg) and 2-chloro-2-oxoethyl acetate (57 mg), and then the mixture was allowed to react for 30 min at 0 °C. The reaction solution was concentrated under reduced pressure, to provide compound A1.8-E, which was directly used in the next step.

LCMS (ESI) [M+H]⁺: 578.0.

### Step 5: Preparation of compound (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-isopropylacetamide (A1.8)

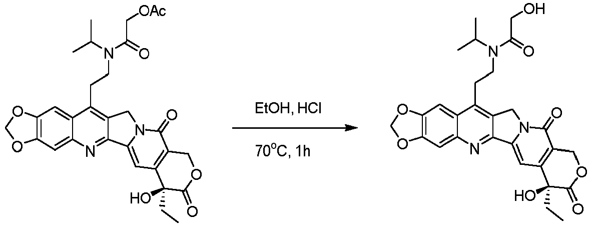

To the solution of compound (S)-2-((2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(isopropyl)amino)-2-oxoethyl acetate (A1.8-E, 50 mg) in ethanol (3 mL), to which was added concentrated hydrochloric acid (0.5 mL), and then the mixture was allowed to react at 70 °C for 1 h. The reaction solution was concentrated to obtain the crude product, which was purified by preparative HPLC (acetonitrile/water containing 0.05% formic acid) to obtain the target compound (A1.8, 3 mg).

LCMS (ESI) [M+H]⁺: 464.0;
¹H NMR (400 MHz, DMSO-d6) δ 7.99 (s, 1H), 7.53 (s, 1H), 7.26 (s, 1H), 6.31 (s, 2H), 5.43 (s, 2H), 5.36 (s, 2H), 4.22 (s, 2H), 3.99 - 3.94 (m, 1H), 3.44 (dd, *J =* 16.7, 7.8 Hz, 2H), 3.33 - 3.21 (m, 2H), 1.95 - 1.79 (m, 2H), 1.19 (dd, *J =* 16.4, 5.8 Hz, 6H), 0.88 (t, *J =* 7.2 Hz, 3H).

### Example A1.9: Synthesis of (S)-7-ethyl-7-hydroxy-14-(3-hydroxypropyl)-10,13-dihydro-11H-[1,3]dioxolo[4,5-g]pyrano[3',4':6,7]indolizino[1,2-b]quinolin-8,11(7H)-dione (A1.9)

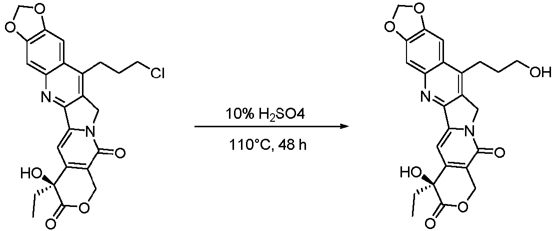

Compound (S)-7-ethyl-7-hydroxy-14-(3-chloropropyl)-10,13-dihydro-11H-[1,3]dioxolo[4,5-g]pyrano[3',4':6,7] indolizino[1,2-b]quinolin-8,11(7H)-dione (100 mg, 0.213 mmol) was dissolved in 10% sulfuric acid (5 mL) solution, and then reacted at 110 °C for 48 h. Saturated sodium bicarbonate (30 mL) solution was added to the reaction solution, and then the resultant solution was extracted with dichloromethane (10 mL × 5), then dried over anhydrous sodium sulfate, filtered, and concentrated under reduced pressure to obtain the crude product, which was purified by preparative HPLC (acetonitrile/water containing 0.05% formic acid) to obtain (S)-7-ethyl-7-hydroxy-14-(3-hydroxypropyl)-10,13-dihydro-11H-[1,3]dioxolo[4,5-g]pyrano[3',4':6,7]indolizino[1,2-b]quinolin-8,11(7H)-dione (A1.9, 1.78 mg).

LCMS (ESI) [M+H]⁺: 451.0;
¹H NMR (400 MHz, DMSO-*d6*) δ 7.63 (s, 1H), 7.50 (s, 1H), 7.24 (s, 1H), 6.48 (s, 1H), 6.28 (s, 2H), 5.47 - 5.37 (m, 2H), 5.32 - 5.19 (m, 2H), 3.51 - 3.46 (m, 2H), 3.17 - 3.13 (m, 2H), 1.92 - 1.76 (m, 4H), 0.90 - 0.84 (m, 3H).

### Example A1.10: Synthesis of (S)-4-ethyl-8-fluoro-4-hydroxy-11-(3-hydroxypropyl)-9-methyl-1,12-dihydro-14H-pyrano[3',4':6,7]indolizino[1,2-b]quinolin-3,14(4H)-dione (A1.10)

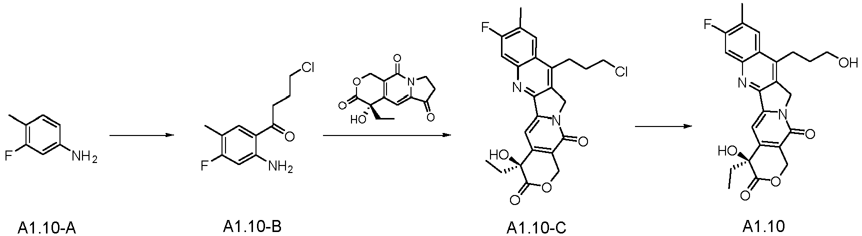

### Step 1:

At 0 °C, to the solution of compound A1.10-A (10 g) in 1,2-dichloroethane (200 mL), were successively added 1 mol/L boron trichloride (96 mL) and 4-chlorobutyronitrile (9.9 g) dropwise. The reaction was stirred at 80 °C for 2 hours. The reaction solution was cooled to room temperature, and then 2 mol/L hydrochloric acid (90 mL) was added, and the mixture was stirred at 80 °C under reflux for 0.5 hours. The reaction solution was cooled to room temperature, diluted with a small amount of water, and extracted with dichloromethane (200 mL × 3). The organic phase was dried over anhydrous sodium sulfate, filtered, and concentrated to obtain a crude product, which was separated and purified by column chromatography (petroleum ether:ethyl acetate=10/1) to obtain the target compound A1.10-B (4 g).

LCMS (ESI) [M+H]⁺: 230.0.

### Step 2:

To compound A1.10-B (50 mg), were successively added (S)-4-ethyl-4-hydroxy-7,8-dihydro-1H-pyrano[3,4-f]indolizin-3,6,10(4H)-trione (35 mg) and p-toluenesulfonic acid monohydrate (41.4 mg), and then the mixture was dissolved in dichloromethane (30 mL). The solution was mixed, concentrated under reduced pressure, pumped to vacuum with an oil pump, and residue reacted under vacuum at 120 ° C for 3 hours. LCMS showed the reaction was completed. The reaction mixture was cooled to room temperature, and then water (20 mL) was added. The resultant was extracted with dichloromethane (20 mL × 3). The combined organic phases were successively dried over anhydrous sodium sulfate, filtered, and the filtrate was concentrated under reduced pressure to obtain a crude product. The crude product was separated and purified by column chromatography (dichloromethane : methanol = 20 : 1) to obtain the target compound A1.10-C (80 mg) as a white solid.

LCMS (ESI) [M+H]⁺: 457.0.

### Step 3:

Compound A1.10-C (75 mg) was dissolved in hexamethylphosphoric triamide, to which was added pure water (0.8 mL), and then the reaction solution was stirred at 100 °C for 72 h. LCMS test showed that the reaction was completed. The target compound (10 mg) was purified by preparative chromatography (0.01% TFA in water, MeCN).

LCMS (ESI) [M+H]⁺: 439.2;
¹H NMR (400 MHz, DMSO-d6) δ 8.22 (d, *J =* 8.4 Hz, 1H), 7.87 (d, *J =* 10.9 Hz, 1H), 7.31 (s, 1H), 6.50 (s, 1H), 5.43 (s, 2H), 5.30 (s, 2H), 4.67 (t, *J =* 4.9 Hz, 1H), 3.55 - 3.47 (m, 2H), 3.28 - 3.20 (m, 2H), 2.51 (s, 3H), 1.93 - 1.81 (m, 4H), 0.88 (t, *J =* 7.3 Hz, 3H).

### Example A1.11: Synthesis of (S)-4-ethyl-8-fluoro-4-hydroxy-11-(3-aminopropyl)-9-methyl-1,12-dihydro-14H-pyrano[3',4':6,7]indolizino[1,2-b]quinolin-3,14(4H)-dione (A1.11)

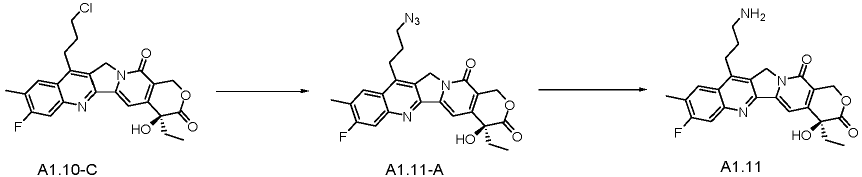

### Step 1:

To the solution of A1.10-C (395 mg) in N'N-dimethylformamide (10 mL), was added sodium azide (432 mg), and then the reaction solution was allowed to react for 16 h at 80 °C. Then, the reaction solution was cooled to room temperature, diluted by adding water (50 mL), and extracted with ethyl acetate (80 mL*3). The organic phases were combined, dried over anhydrous sodium sulfate, and concentrated by suction filtration to obtain the target product A1.11-A (290 mg).

LCMS (ESI) [M+H]⁺: 464.0.

### Step 2:

Compound A1.11-A (93 mg) was dissolved in tetrahydrofuran (5 mL), to which was added triphenylphosphine (78 mg), and then the mixture was allowed to react at room temperature for 4 hours. Then, hydrochloric acid (4M, 1mL) was added to the reaction solution. The reaction solution was heated to 55 °C and reacted for 16 hours. LCMS monitoring showed that the reaction was complete. The reaction solution was directly concentrated, and the crude product was purified by reversed phase column (mobile phase A is 0.05% formic acid in water, and B is acetonitrile) to obtain the target product A1.11 (28 mg, yield: 36%) as white solid.

LCMS (ESI) [M+H]⁺: 438.4;
¹H NMR (400 MHz, CD₃OD) δ 8.52 (s, 1H), 8.16 (d, *J =* 7.8 Hz, 1H), 7.76 (d, *J =* 10.7 Hz, 1H), 7.63 (s, 1H), 5.49 (ABq, *J =* 78.9, 16.3 Hz, 2H), 5.31 (br s, 2H), 3.35 - 3.32 (m, 2H), 3.22 - 3.10 (m, 2H), 2.55 (s, 3H), 2.13 - 2.11 (m, 2H), 1.96 - 1.94 (m, 2H), 1.01 (t, *J =* 7.4 Hz, 3H).

### Example A1.12: Synthesis of (S,E)-14-(3-amino-1-propylen-1-yl)-7-ethyl-7-hydroxy-10,13-dihydro-11H-[1,3]dioxolo[4,5-g]pyrano[3',4':6,7]indolizino[1,2-b]quinolin-8,11(7H)-dione (A1.12)

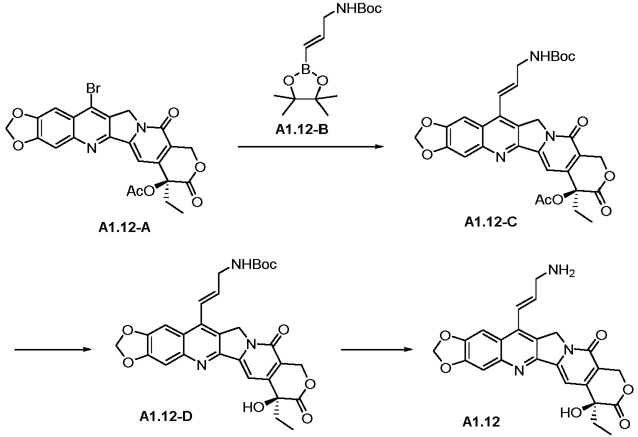

### Step 1:

Compound A1.12-A (200 mg, 0.39 mmol), compound A1.12-B (112 mg, 0.39 mmol), cesium fluoride (152 mg, 0.975 mmol) and tetrakis(triphenylphosphine)palladium (45 mg, 0.039 mmol) were added in 1,4-dioxane (8 mL). The microwave reaction was carried out at 120 °C for 0.5 hour under nitrogen atmosphere. LCMS indicated the reaction was completed. A mixed solvent of dichloromethane (20 mL) and methanol (10 mL) was added to the reaction, and then the resultant solution was filtered. The filtrate was concentrated, and the crude product was purified by preparative TLC (dichloromethane:methanol = 30:1) to obtain the target compound (S,E)-14-(3-((tert-butoxycarbonyl)amino)-1-propylen-1-yl)-7-ethyl-8,11-dioxo-7,8,11,13-tetrahydro-10H-[1,3] dioxolo[4,5-g]pyrano[3',4':6,7]indolizino[1,2-b]quinolin-7-yl acetate (A1.12-C, 60 mg, yield: 26%) as brown solid. LCMS (ESI) [M+H]⁺ = 590.3;
¹H NMR (400 MHz, DMSO-d6) δ 7.63 (s, 1H), 7.49 (s, 1H), 7.35 (s, 1H), 7.09 (d, *J =* 16.7 Hz, 1H), 6.93 (s, 1H), 6.45 (d, *J =* 16.6 Hz, 1H), 6.30 (s, 2H), 5.47 (s, 2H), 5.34 - 5.24 (m, 2H), 3.94 (s, 2H), 2.21 (br s, 3H), 2.03 - 1.96 (m, 2H), 1.45 (s, 9H), 0.91 (t, *J =* 6.7 Hz, 3H).

### Step 2:

To the solution of compound (S,E)-14-(3-((tert-butoxycarbonyl)amino)-1-propylen-1-yl)-7-ethyl-8,11-dioxo-7,8,11,13-tetrahydro-10H-[1,3]dioxolo[4,5-g]pyrano[3',4':6,7]indolizino[1,2-b]quinolin-7-yl acetate (A1.12-C, 50 mg, 0.085 mmol) in methanol (15 mL), was added sodium methoxide (9.2 mg, 0.17 mmol), and then the reaction was stirred at 50 °C for 2 hours. LCMS indicated the reaction was completed. The reaction solution was concentrated to obtain the crude target compound (S,E)-14-(3-((tert-butoxycarbonyl)amino)-1-propylen-1-yl)-7-ethyl-7-hydroxy-10,13-dihydro-11H- [1,3]dioxolo[4,5-g]pyrano[3',4':6,7]indolizino[1,2-b]quinolin-8,11(7H)-dione (A1.12-D, 50 mg) as brown solid. LCMS (ESI) [M+H]⁺ = 548;

### Step 3:

To the solution of compound (S,E)-14-(3-((tert-butoxycarbonyl)amino)-1-propylen-1-yl)-7-ethyl-7-hydroxy-10,13-dihydro-11H-[1,3]dioxolo[4,5-g]pyrano[3',4':6,7]indolizino[1,2-b]quinolin-8,11(7H)-dione (A1.12-D, 50 mg, 0.091 mmol) in dichloromethane (2 mL), was added trifluoroacetic acid (1 mL), and then the reaction was stirred at room temperature for 30 minutes. LCMS showed the reaction was completed. The reaction solution was concentrated, and the crude product was purified by preparative HPLC (acetonitrile/0.05% formic acid in water) to obtain the target compound (S,E)-14-(3-amino-1-propylen-1-yl)-7-ethyl-7-hydroxy-10,13-dihydro-11H-[1,3]dioxolo[4,5-g] pyrano[3',4':6,7]indolizino[1,2-b]quinolin-8,11(7H)-dione (A1.12) (8.1 mg, yield 21%), as brown solid.

LCMS (ESI) [M+H]⁺ = 448.3;
¹H NMR (400 MHz, DMSO-d6) δ 8.20 (s, 2H), 7.72 (s, 1H), 7.54 (s, 1H), 7.40 (d, *J =* 16.5 Hz, 1H), 7.27 (s, 1H), 6.52 - 6.46 (m, 2H), 6.31 (s, 2H), 5.42 (s, 2H), 5.27 (s, 2H), 3.86 (br s, 2H), 1.90 - 1.83 (m, 2H), 0.88 (t, *J =* 7.1 Hz, 3H).

### Example A1.13: Synthesis of (S,E)-14-(3-hydroxy-1-propylen-1-yl)-7-ethyl-7-hydroxy-10,13-dihydro-11H-[1,3] dioxolo[4,5-g]pyrano[3',4':6,7]indolizino[1,2-b]quinolin-8,11(7H)-dione (A1.13)

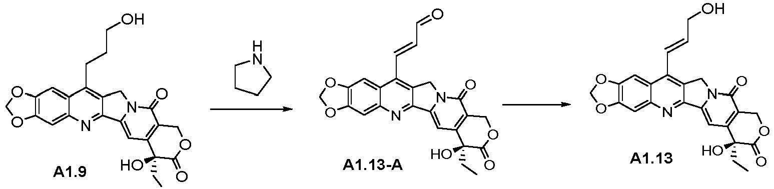

### Step 1:

Compound A1.9 (200 mg, 0.444 mmol) was dissolved in dimethyl sulfoxide (2 mL), to which was added IBX (311 mg, 1.11 mmol), and then the reaction was stirred at room temperature for 2 hours. Then, IBX (186 mg, 0.666 mmol) was added to the reaction solution, and subsequently, tetrahydropyrrole (6.3 mg, 0.089 mmol) and acetonitrile (3 mL) were added to the reaction solution. The reaction solution was stirred at room temperature overnight. The LCMS indicates that the reaction was completed. The reaction solution was extracted with ethyl acetate (20 mL x 3), and the organic phase was washed with saturated brine, dried over anhydrous sodium sulfate, filtered, and rotatory evaporated to dry, to obtain a crude product, which was purified by silica gel column (DCM:MeOH = 50 : 1 to 10 : 1), to provide the target compound (S,E)-14-(3-oxo-1-propylen-1-yl)-7-ethyl-7-hydroxy-10,13-dihydro-11H-[1,3]dioxolo[4,5-g]pyrano[3',4':6,7] indolizino[1,2-b]quinolin-8,11(7H)-dione (A1.13-A, 100 mg, purity 50.0%, yield 25.0%), as yellow solid. LCMS (ESI) [M+H]⁺ = 447.1.

### Step 2:

Compound A1.13-A (40 mg, 0.09 mmol) was dissolved in tetrahydrofuran (1 mL), to which was then added sodium cyanoborohydride (28 mg, 0.448 mmol), and the mixture was stirred at room temperature overnight. LCMS showed the reaction was completed. The reaction solution was concentrated to obtain the crude product, which was purified by prep-HPLC (HCl in water/MeCN) to obtain the target compound (3.56 mg, purity 93.6%, yield 9.0%) as a pale yellow solid.

LCMS (ESI) [M+H]⁺ = 449.2;
1H NMR (400 MHz, DMSO-d6) δ 7.61 (s, 1H), 7.50 (s, 1H), 7.24 (s, 1H), 7.20 (d, *J =* 16.4 Hz, 1H), 6.69 - 6.59 (m, 1H), 6.49 (s, 1H), 6.30 (s, 2H), 5.42 (s, 2H), 5.26 (s, 2H), 5.16 (br s, 1H), 4.34 (br s, 2H), 1.93 - 1.81 (m, 2H), 0.88 (t, *J =* 7.3 Hz, 3H).

### Example A1.14: Synthesis of (S,E)-4-ethyl-8-fluoro-4-hydroxy-11-(3-hydroxy-1-propylen-1-yl)-9-methyl- 1,12-dihydro-14H-pyrano[3',4':6,7]indolizino[1,2-b]quinolin--3,14(4H)-dione (A1.14)

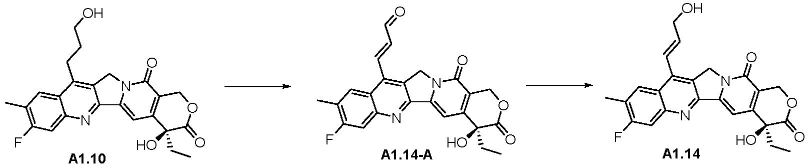

### Step 1:

To a 25 mL single-neck vial, were added compound (S)-4-ethyl-8-fluoro-4-hydroxy-11-(3-hydroxypropyl) -9-methyl-1,12-dihydro-14H-pyrano[3',4':6,7]indolo[1,2-b]quinolin-3,14(4H)-dione (A1.10, 100 mg, 0.228 mmol), DMSO (0.5 mL) and acetonitrile (0.75 mL), followed by successive addition of IBX (160 mg, 0.571 mmol) and (R)-diphenyl(pyrrolidin-2-yl)methanol (12 mg, 0.047 mmol), and then the reaction was continually stirred at room temperature for 16 hours. Then, the mixed solvent of methanol and dichloromethane (200 mL) (DCM:MeOH=10:1) was added to the reaction solution, and the resultant solution was extracted with saturated brine (300 mL). The organic phases were combined, washed with water, dried, and filtered. The filtrate was evaporated to dryness under reduced pressure to obtain the crude product, which was purified by flash chromatography ( DCM:MeOH=10:1) to obtain the target compound (S)-4-ethyl-8-fluoro-4-hydroxy-11- (3-oxopropyl)-9-methyl-1,12-dihydro-14H-pyrano[3',4':6,7]indolo[1,2-b]quinolin-3,14(4H)-dione (A1.14-A, 40 mg, yield 40%).

LCMS (ESI) [M+H]⁺ *=* 435.1.

### Step 2:

To a 50 mL three-neck vial, were added compound (S)-4-ethyl-8-fluoro-4-hydroxy-11-(3-oxopropyl) -9-methyl-1,12-dihydro-14H-pyrano[3',4':6,7]indolo[1,2-b]quinolin-3,14(4H)-dione (A1.14-A, 40 mg, 0.228 mmol) and anhydrous THF (5 mL), and then the temperature was lowered to -78°C under N₂ protection. A solution of lithium tri-sec-butylborohydride in THF (0.11 mL, 1N) was slowly added dropwise, and the reaction was continually stirred at -78°C for 1 hour. After the reaction was completed, saturated aqueous ammonium chloride solution was added to quench the reaction, and then the resultant solution was warmed to room temperature, diluted with saturated brine (50 mL), and extracted with ethyl acetate (20 mL×3). The combined organic phases were washed with water, dried, filtered, and evaporated to dryness under reduced pressure, to provide (S,E)-4-ethyl-8-fluoro-4-hydroxy-11-(3-hydroxy-1-propylen-1-yl)-9-methyl-1,12-dihydro-14H- pyrano[3',4':6,7]indolizino[1,2-b]quinolin- -3,14(4H)-dione (A1.14, 28 mg) as off-white solid.

LCMS (ESI) [M+H]⁺ = 437.1.

### Example A1.15: Synthesis of (S)-7-ethyl-7-hydroxy-14-(2-(n-propylamino)ethyl)-10,13-dihydro-11H-[1,3]dioxolo[4,5-g]pyrano[3',4':6,7]indolizino[1,2-b]quinolin-8,11(7H)-dione (A1.15a) and (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-n-propylacetamide (A1.15b)

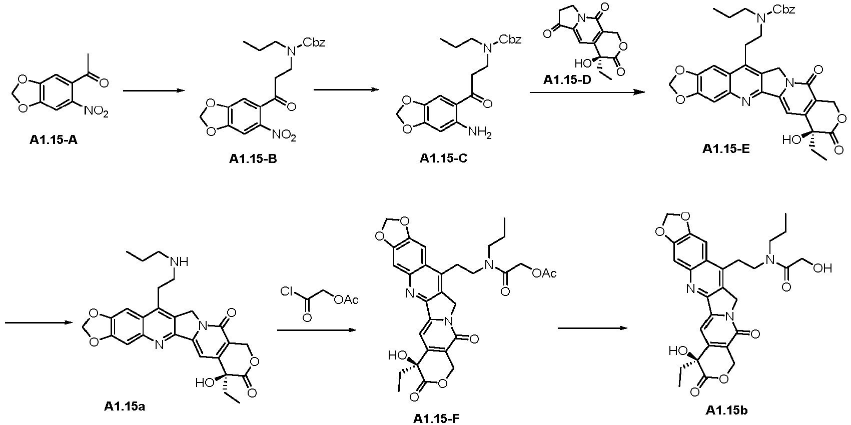

### Step 1:

Compound A1.15-A (5.0 g, 23.9 mmol), propylamine hydrochloride (1.6 g) and paraformaldehyde (7.18 g, 239 mmol) were dissolved in ethanol (100 mL), and then the mixture was allowed to react in a sealed vessle at 110 °C for 12 h. LCMS indicated the completion of the reaction. The reaction solution was cooled to room temperature and concentrated under reduced pressure. Dichloromethane (100 mL) was added into the reaction solid, and then washed with water (80 mL × 3). The resultant aqueous phase was adjusted to pH=9 with sodium bicarbonate, to which was then added benzyloxycarbonyl chloride (3.7 g, 21.8 mmol). The reaction was stirred at room temperature for 2 hours. The reaction was completed by LCMS detecting. The reaction solution was extracted with dichloromethane (100 mL × 3), and then the organic phase was washed with saturated brine, dried over anhydrous sodium sulfate, filtered, and concentrated to obtain a crude product, which was separated and purified by column chromatography (petroleum ether:ethyl acetate = 10/1) to obtain the target compound A1.15-B (1.5 g, yield 20.1%) as yellow oily liquid.

LCMS (ESI) [M+H]⁺ = 415.1;
1H NMR (400 MHz, CDCl₃) δ 7.56 (s, 1H), 7.35 (m, 6H), 6.18 (s, 2H), 5.17 - 5.12 (m, 2H), 3.69 - 3.67 (m, 2H), 3.32 - 3.30 (m, 2H), 3.09 - 2.90 (m, 2H), 1.59 (m, 2H), 0.90 (m, 3H).

### Step 2:

Compound A1.15-B (1.5 g, 3.63 mmol) was dissolved in a mixed solution of saturated aqueous ammonium chloride (20 mL) and ethanol (20 mL), and then iron powder (1.02 g, 18.1 mmol) was added to the reaction solution. The reaction was stirred at 80 °C for 1 h. LCMS indicated the completion of the reaction. The reaction mixture was filtered, and the filtrate was concentrated to solid under reduced pressure. The crude product was separated and purified by C18 reversed-phase column (acetonitrile/0.05% FA in water: 5% to 55%) to obtain the target compound A1.15-C (600 mg, yield 43.0%) as yellow solid.

LCMS (ESI) [M+H]⁺ = 385.2, t_{R} = 1.329 min.

### Step 3:

At room temperature, compound A1.15-C (350 mg, 0.91 mmol), compound (S)-4-ethyl-4-hydroxy-7,8-dihydro-1H-pyrano[3,4-f]indolizin-3,6,10(4H)-trione (A1.15-D, 218 mg, 0.83 mmol) and p-toluenesulfonic acid (170 mg, 0.87 mmol) were dissolved in dichloromethane (5 mL), and after the solution was clear and mixed, it was concentrated under reduced pressure, and pumped to vacuum with an oil pump. The reaction was carried out at 120 °C under vacuum for 2 hours. LCMS indicated the completion of the reaction. The reaction was cooled to room temperature, to which was then added water (50 mL), and the resultant mixture was extracted with dichloromethane (30 mL × 3). The combined organic phases were successively dried over anhydrous sodium sulfate, filtered, and the filtrate was concentrated under reduced pressure to obtain the target compound A1.15-E (390 mg, yield 76%) as brown solid.

LCMS (ESI) [M+H]⁺ = 612.0.

### Step 4:

At room temperature, compound A1.15-E (390 mg, 0.638 mmol) was dissolved in dichloromethane (5 mL), to which was added trimethyliodosilane (510 mg, 2.55 mmol) at 0 °C under nitrogen protection, and then the reaction system was stirred at room temperature for 2 h. Diethyl ether (2 mL) and concentrated hydrochloric acid (4 mL) were added to the reaction solution, and then stirred for 30 min. The reaction system was adjusted to pH = 9 with saturated sodium bicarbonate, and then extracted with dichloromethane (50 mL×5). The combined organic phase was dried over anhydrous sodium sulfate and filtered. The filtrate was concentrated under reduced pressure to obtain the crude product, which was purified by preparative HPLC (acetonitrile/water containing 0.05% formic acid), to provide compound A1.15a (200 mg, yield 66.1%) as an off-white solid.

LCMS (ESI) [M+H]⁺ = 478.2;
1H NMR (400 MHz, DMSO-d6) δ 8.30 (s, 1H, HCO₂H), 7.67 (s, 1H), 7.51 (s, 1H), 7.23 (s, 1H), 6.62 - 6.41 (m, 1H), 6.29 (s, 2H), 5.42 (s, 2H), 5.28 (s, 2H), 3.27 - 3.25 (m, 2H), 2.91 - 2.79 (m, 2H), 2.58 - 2.56 (m, 2H), 1.91 - 1.79 (m, 2H), 1.44 - 1.42 (m, 2H), 0.90 - 0.84 (m, 6H).

### Step 5:

At room temperature, compound A1.15a (120 mg, 0.251 mmol) was dissolved in dichloromethane (5 mL), to which were added compound acetyloxyacetyl chloride (167 mg,1.25 mmol) and triethylamine (133 mg, 1.25 mmol) dropwise at 0°C, and then the mixture was stirred at 0 °C for 30 min. LCMS detection indicated the reaction was completed. The reaction solution was concentrated under reduced pressure to obtain a crude compound A1.15-F (120 mg, yield 82.8%) as a yellow solid. LCMS (ESI) [M+H]⁺ = 578.3.

### Step 6:

At room temperature, compound A1.15-F (120 mg, 0.207 mmol) was dissolved in ethanol (4 mL), and then concentrated hydrochloric acid (2 mL) was added to the reaction solution. The reaction was stirred at 70 °C for 1 h. LCMS detection showed that the reaction was completed. Water (20 mL) was added to the reaction solution, and then the resultant solution was extracted with dichloromethane (30 mL X 5). The combined organic phase was dried over anhydrous sodium sulfate and filtered. The filtrate was concentrated under reduced pressure to obtain the crude product, which was purified by preparative HPLC (acetonitrile/water containing 0.05% formic acid) to obtain compound A1.15b (20 mg, yield 18.1%) as a off-white solid.

LCMS (ESI) [M+H]⁺ = 536.2;
1H NMR (400 MHz, DMSO-d6) δ 7.93 (s, 0.7H), 7.69 (s, 0.3H), 7.51 (s, 1H), 7.24 (s, 1H), 6.49 (s, 1H), 6.30 (s, 2H), 5.42 (s, 2H), 5.35 (s, 1.5H), 5.29 (s, 0.5H), 4.65 - 4.62 (m, 1H), 4.13 - 3.97 (m, 2H), 3.51 - 3.49 (m, 2H), 3.33 - 3.30 (m, 2H), 3.24 - 3.20 (m, 2H), 1.86 - 1.84 (m, 2H), 1.60 - 1.52 (m, 2H), 0.89 - 0.86 (m, 6H).

### Example A1.16: Synthesis of (S)-4-ethyl-8-fluoro-4-hydroxy-11-(3-isopropylaminopropyl)-9-methyl- 1,12-dihydro-14H-pyrano[3',4':6,7]indolizino[1,2-b]quinolin-3,14(4H)-dione (A1.16)

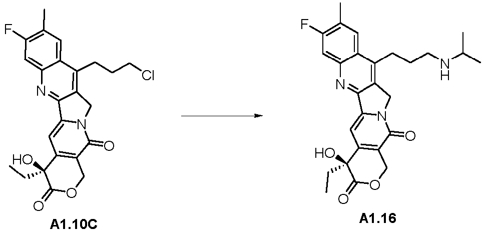

To a solution of A1.10-C (200 mg, 0.439 mmol) and isopropylamine (50 mg, 0.877 mmol) in DMF (10 mL), were added diisopropylethylamine (170 mg, 1.32 mmol) and sodium iodide (99 mg, 0.659 mmol), and then the reaction was carried out at 50°C for 2 hours in a sealed tube. Ethyl acetate was added to the reaction solution, and the organic phase was washed with saturated brine, dried over anhydrous sodium sulfate, filtered, and concentrated under reduced pressure to obtain a crude product, which was separated and purified by pre-TLC (dichloromethane:methanol = 10:1) to obtain a yellow solid, and the compound was further purified by preparative HPLC (acetonitrile/water containing 0.05% formic acid) to obtain the target compound (S)-4-ethyl-8-fluoro-4-hydroxy-11-(3-isopropylaminopropyl)-9-methyl-1,12-dihydro-14H-pyrano[3',4':6,7] indolizino[1,2-b]quinolin-3,14(4H)-dione (A1.16, 2.03 mg).

LCMS (ESI) [M+H]⁺ = 480.2;
¹H NMR (400 MHz, DMSO-d6) δ 8.28 (d, *J =* 8.0 Hz, 1H), 8.27 (s, 1H, HCO2H) 7.92 (d, *J =* 11.0 Hz, 1H), 7.33 (s, 1H), 6.53 (s, 1H), 5.45 (s, 2H), 5.30 (s, 2H), 33.30 - 3.22 (m, 3H), 3.18 - 3.10 (m, 2H), 2.54 (s, 3H), 2.06 - 1.94 (m, 2H), 1.93 - 1.80 (m, 2H), 1.26 - 1.20 (m, 6H), 0.88 (t, *J =* 7.3 Hz, 3H).

### Example A1.17: Synthesis of (S)-4-ethyl-8-fluoro-4-hydroxy-11-(3-cyclopropylaminopropyl)- 9-methyl-1,12-dihydro-14H-pyrano[3',4':6,7]indolizino[1,2-b]quinolin-3,14(4H)-dione (A1.17)

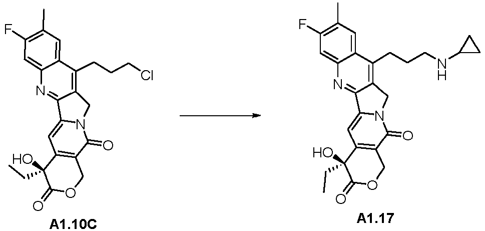

To a solution of A1.10-C (200 mg, 0.439 mmol) and cyclopropylamine (52 mg, 0.881 mmol) in DMF (10 mL), were added diisopropylethylamine (165 mg, 1.28 mmol) and sodium iodide (96 mg, 0.640 mmol), and then the reaction was carried out at 50°C for 2 hours in a sealed tube. LCMS indicated the completion of the reaction.

Ethyl acetate was added to the reaction solution, and the organic phase was washed with saturated brine, dried over anhydrous sodium sulfate, filtered, and concentrated under reduced pressure to obtain a crude product, which was separated and purified by TLC (dichloromethane:methanol = 10:1), followed by preparative HPLC (acetonitrile/water containing 0.05% formic acid) to obtain the target compound (S)-4-ethyl-8-fluoro-4-hydroxy-11-(3-cyclopropylaminopropyl)-9-methyl-1,12-dihydro-14H-pyrano[3',4':6,7] indolizino[1,2-b]quinolin-3,14(4H)-dione (A1.17, 5.12 mg).

LCMS (ESI) [M+H]⁺ = 478.2;
¹H NMR (400 MHz, DMSO-d6) δ 8.22 (br s, 2H, one is HCO2H), 7.86 (br s, 1H), 7.30 (s, 1H), 6.51 (s, 1H), 5.43 (s, 2H), 5.28 (s, 2H), 3.24 - 3.19 (m, 2H), 2.75 - 2.68 (m, 2H), 2.49 (s, 3H), 2.16 - 2.09 (m, 1H), 1.95 - 1.76 (m, 4H), 0.91 - 0.83(t, *J =* 7.3 Hz, 3H), 0.43 - 0.33 (m, 2H), 0.31 - 0.21 (m, 2H).

### Example A1.18: Synthesis of (S)-4-ethyl-8-fluoro-4-hydroxy-11-(4-aminophenyl)-9-methyl-1,12-dihydro- 14H-pyrano[3',4':6,7]indolizino[1,2-b]quinolin-3,14(4H)-dione (A1.18)

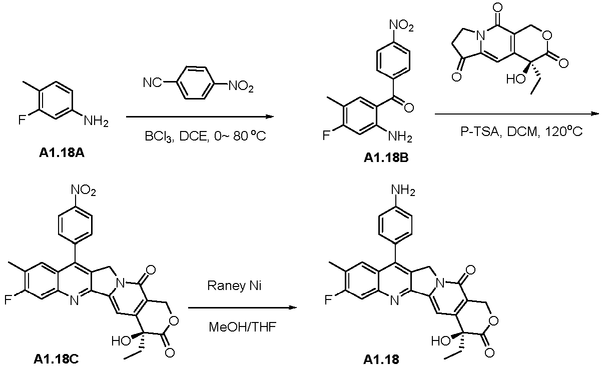

### Step 1:

Under nitrogen protection, 3-fluoro-4-methylaniline (A1.18A, 2.0 g, 16.3 mmol) was dissolved in 1,2-dichloroethane (40 mL), to which was added boron trichloride (19.2 mL, 19.2 mmol) dropwise at 0 °C, and then p-nitrobenzonitrile (2.8 g, 19.2 mmol) was added slowly. After addition, the reaction solution was heated to 80 °C and stirred overnight. The reaction solution was cooled to room temperature, to which was added hydrochloric acid (40 mL, 2M), and then heated to 80 °C and stirred for 30 min. After the reaction was completed by detection with LCMS, water (80 mL) was added to the reaction solution, and the resultant solution was extracted with dichloromethane (100 mL x 3). The combined organic phase was dried over anhydrous sodium sulfate, filtered, and solvent was evaporated to dryness under reduced pressure. The crude product was purified by silica gel column chromatography (PE:EA = 10:1), to provide the target compound (A1.18B, 2.0 g, yield 46.5%).

LCMS (ESI) [M+H]⁺ = 275.1;
¹H NMR (400 MHz, DMSO) δ 8.34 (d, *J =* 8.6 Hz, 2H), 7.77 (d, *J =* 8.6 Hz, 2H), 7.39 (s, 2H), 7.11 (d, *J =* 8.8 Hz, 1H), 6.63 (d, *J =* 12.4 Hz, 1H), 2.00 (s, 3H)_{∘}

### Step 2:

Compound A1.18B (620 mg, 2.28 mmol), (S)-4-ethyl-4-hydroxy-7,8-dihydro-1H-pyrano[3,4-f]indolizin-3,6,10(4H)-trione (600 mg, 2.28 mmol), p-toluenesulfonic acid (560 mg, 2.96 mmol) were added to the reaction flask, and thoroughly dissolved with dichloromethane. Dichloromethane was removed by rotatory evaporation. The mixture was vacuumized, heated to 120 °C under vacuumfor 6 hours. After the reaction was completed by LCMS detection, methanol (10 mL) was added to the system, followed by addition of water (80 mL), and a large amount of precipitation was precipitated. The precipitation was filtered and dried to obtain the target compound (A1.18C, 800 mg, yield 80.0%) as yellow solid.

LCMS (ESI) [M+H]⁺ = 502.2;
¹H NMR (400 MHz, DMSO) δ 8.52 (d, *J =* 8.2 Hz, 2H), 8.13 - 7.89 (m, 3H), 7.64 (d, *J =* 8.4 Hz, 1H), 7.37 (d, *J =* 13.4 Hz, 1H), 6.54 (s, 1H), 5.41 (s, 2H), 5.06 (m, 2H), 2.40 (s, 3H), 1.94 - 1.84 (m, 2H), 0.87 (m, 3H).

### Step 3:

Raney Ni (609 mg, 10.5 mmol) was added to a solution of compound A1.18C (1.0 g, 2.1 mmol) in the mixed solvent of methanol (25 mL) and tetrahydrofuran (50 mL), and the reaction solution was stirred at room temperature under hydrogen atmosphere for 5 hours. LCMS indicated that the reaction was complete. The reaction solution was filtered, and the filtrate was rotatory evaporated to dryness, to obtain the target compound (S)-4-ethyl-8-fluoro-4-hydroxy-11-(4-aminophenyl)-9-methyl-1,12-dihydro-14H-pyrano[3',4':6,7]indolizino[1,2-b]quinolin-3,14(4H)-dione (A1.18, 650 mg, yield 67%) as yellow solid.

LCMS (ESI) [M+H]⁺ = 472.3;
¹H NMR (400 MHz, DMSO) δ 7.93 - 7.81 (m, 2H), 7.40 - 7.25 (m, 3H), 6.81 (d, *J =* 8.2 Hz, 2H), 6.51 (s, 1H), 5.62 (s, 2H), 5.41 (s, 2H), 5.10 (s, 2H), 2.41 (s, 3H), 1.93 - 1.80 (m, 2H), 0.88 (t, *J =* 7.2 Hz, 3H).

### B. Synthesis of intermediates containing bioactive molecular fragments

### Example B1.1: Synthesis of (S)-2-amino-N-((4-(4-ethyl-8-fluoro-4-hydroxy-9-methyl-3,14-dioxo- 3,4,12,14-tetrahydro-1H-pyrano[3',4':6,7]indolizino[1,2-b]quinolin-11-yl)butoxy)methyl)acetamide (B1.1)

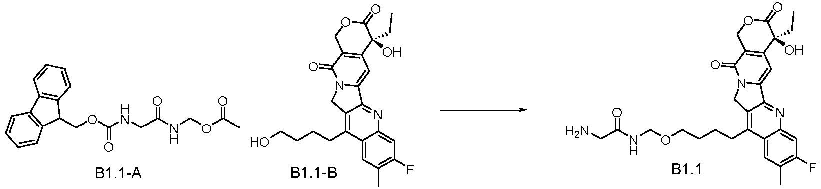

Compound (B1.1-A) (368 mg), compound (B1.1-B) (452 mg) and pyridinium p-toluenesulfonate (PPTS) (25 mg) were refluxed in dichloromethane (20 ml) for 20 h, and then washed with the aqueous sodium bicarbonate solution and the aqueous hydrochloric acid, respectively. The organic solvent was removed under reduced pressure, and the residue was dissolved in DMF (5ml), to which was added piperidine (1 ml). The mixture was stirred for 20 min, and then most of the low-boiling components was removed under reduced pressure. The residue was separated by preparative HPLC to provide the target product (S)-2-amino-N-((4-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)butoxy)methyl)acetamide (B1.1).

ESI-MS (m/z): 539 [M+H]⁺.

### Example B1.2: Synthesis of (S)-2-((2-aminoacetamido)methoxy)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide (B1.2)

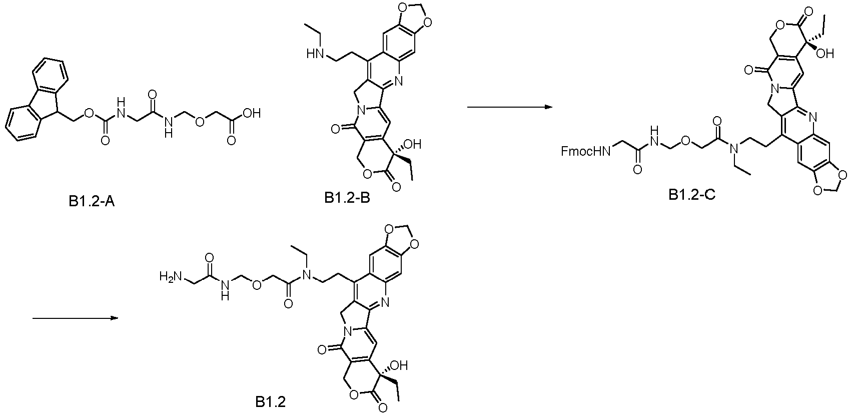

### Step 1:

Compound B1.2-A (274 mg), diisopropylethylamine (334 mg) and HBTU (369 mg) were sequentially added to N,N-dimethylformamide (10 mL), followed by addition of compound B1.2-B (300 mg). The reaction solution was stirred at room temperature for 2 h. Ethyl acetate (50 mL) was added to the reaction solution, and the resultant solution was washed with saturated brine (30 mL×3), dried over anhydrous sodium sulfate, and filtered. The filtrate was concentrated to a solid under reduced pressure, and then the crude product was separated and purified by column chromatography (dichloromethane:methanol=10/1) to obtain the target compound B1.2-C (300 mg).

LCMS (ESI) [M+H]⁺: 830.2;
¹H NMR (400 MHz, DMSO-d6) δ 8.74 (s, 1H), 7.91 - 7.84 (m, 2H), 7.80 - 7.67 (m, 2H), 7.65 - 7.54 (m, 2H), 7.53 - 7.46 (m, 1H), 7.45 - 7.37 (m, 2H), 7.36 - 7.27 (m, 2H), 7.25 - 7.23 (m, 1H), 6.57 - 6.44 (m, 1H), 6.29 (s, 2H), 5.50 - 5.19 (m, 4H), 4.71 - 4.57 (m, 2H), 4.32 - 3.97 (m, 7H), 3.80 - 3.53 (m, 4H), 3.20 - 3.14 (m, 2H), 1.92 - 1.80 (m, 2H), 1.28 - 1.22 (m, 3H), 0.87 - 0.82 (m, 3H).

### Step 2:

To a solution of compound B1.2-C (300 mg) in N,N-dimethylformamide (4 mL), was added piperidine (1 mL), and the reaction solution was stirred at room temperature for 20 min. The target product was obtained after removing low boiling point components from the reaction solution, and used directly in the next step synthesis.

LCMS (ESI) [M+H]⁺ = 608.0.

### Example B1.3: Synthesis of (S)-2-amino-N-((2-(((4-ethyl-8-fluoro-4-hydroxy-9-methyl-3,14-dioxo-3,4,12,14-tetrahydro-1H-pyrano[3',4':6,7]indolizino[1,2-b]quinolin-11-yl)methyl)amino)-2-oxoethoxy)methyl)acetamide (B1.3)

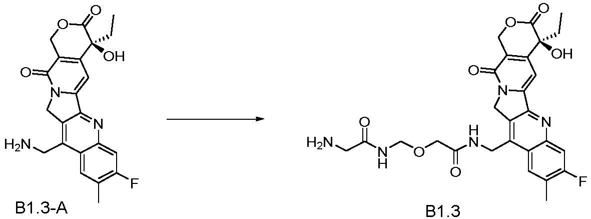

Using the same method and reaction conditions as in Example B1.1, compound (B1.2-B) was replaced by compound (B1.3-A), to obtain the target product (S)-2-amino-N-((2-(((4-ethyl-8-fluoro-4-hydroxy-9-methyl-3,14-dioxo-3,4,12,14-tetrahydro-1H-pyrano[3',4':6,7] indolizino[1,2-b]quinolin-11-yl)methyl)amino)-2-oxoethoxy)methyl)acetamide (B1.3).

ESI-MS (m/z): 554 [M+H]⁺.

### Example B1.4: Synthesis of (S)-2-amino-N-((4-ethyl-8-fluoro-4-hydroxy-9-methyl-3,14-dioxo-3,4,12,14-tetrahydro-1H-pyrano[3',4':6,7]indolizino[1,2-b]quinolin-11-yl)methyl)acetamide (B1.4)

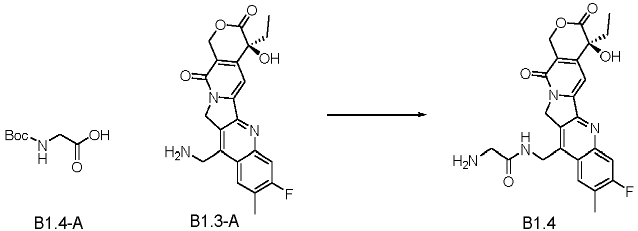

To a solution of compound (B1.4-A) (175 mg) and compound (B1.3-A) (409 mg) in DMF (5 ml), were added DIPEA (200 µl) and HBTU (420 mg). The mixture was stirred at room temperature for 20 hours, to which was then added ethyl acetate (100 ml). The resultant mixture was washed with water (100 ml x 3), and the organic solvent was removed under reduced pressure. Then, 1:1 DCM/TFA (10 ml) was added to the residue, and the mixture was placed at room temperature for 20 minutes. The low boiling point components were removed under reduced pressure, and the residue was separated by preparative HPLC to obtain the target product (S)-2-amino-N-((4-ethyl-8-fluoro-4-hydroxy-9-methyl-3,14-dioxo-3,4,12,14-tetrahydro-1H-pyrano[3',4':6,7] indolizino[1,2-b]quinolin-11-yl)methyl)acetamide (B1.4).

ESI-MS (m/z): 467 [M+H]⁺.

### Example B1.5: Synthesis of (S)-2-amino-N-((7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)acetamide (B1.4)

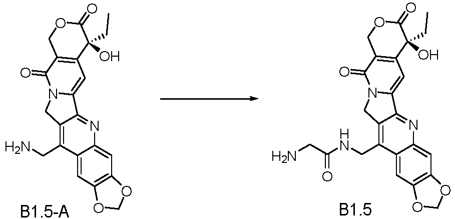

B1.5-A (200 mg) was dissolved in N,N-dimethylformamide (5 mL), to which were successively added N,N-diisopropylethylamine (154.80 mg) and 2,5-dioxopyrrolidin-1-yl (tert-butoxycarbonyl)glycinate (157 mg), and then the reaction solution was stirred at 25° C for 60 min. Water (30 mL) was added to the reaction solution, and then the resultant solution was filtered. The filter cake was dried to obtain a white solid product (ESI-MS (m/z): 579.4 [M+H]⁺). The above white solid product was dissolved in a mixed solution of trifluoroacetic acid and dichloromethane (volume ratio 1:3; 4 mL), and then the reaction solution was stirred at room temperature for 1 h. Then, the reaction solution was concentrated to obtain a crude product, which was separated and purified by C18 reversed phase column (acetonitrile/0.05% formic acid in water: 5% to 50%) to obtain the target product (S)-2-amino-N-((7-ethyl-7-hydroxy-8,11-dioxo-7,8,11,13-tetrahydro-10H-[1,3] dioxolo[4,5-g]pyrano[3',4':6,7]indolizino[1,2-b]quinolin-14-yl)methyl)acetamide (B1.5, 110 mg).

ESI-MS (m/z): 479.3 [M+H]⁺.

### Example B1.6: Synthesis of (S)-2-amino-N-((4-(7-ethyl-7-hydroxy-8,11-dioxo-7,8,11,13-tetrahydro-10H-[1,3] dioxolo[4,5-g]pyrano[3',4':6,7]indolizino[1,2-b]quinolin-14-yl)-1H-pyrazol-1-yl)methyl)acetamide (B1.6)

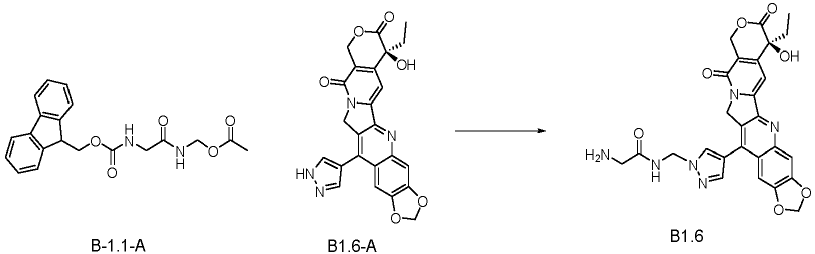

Using the same method and reaction conditions as Example B1.1, compound (B1.1-B) was replaced by compound (B1.6-A), to obtain the target product (S)-2-amino-N-((4-(7-ethyl-7-hydroxy-8,11-dioxo-7,8,11,13-tetrahydro-10H-[1,3]dioxolo[4,5-g]pyrano[3',4':6,7] indolizino[1,2-b]quinolin-14-yl)-1H-pyrazole-1-yl)methyl)acetamide (B1.6).

ESI-MS (m/z): 545 [M+H]⁺.

### Example B1.7: Synthesis of (S)-2-amino-N-((2-(((7-ethyl-7-hydroxy-8,11-dioxo-7,8,11,13-tetrahydro-10H-[1,3] dioxolo[4,5-g]pyrano[3',4':6,7]indolizino[1,2-b]quinolin-14-yl)methyl)amino)-2-oxoethoxy)methyl)acetamide (B1.7)

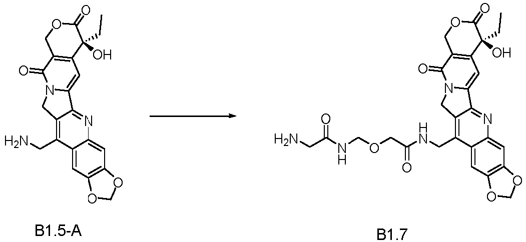

Using the same method and reaction conditions as Example B1.2, compound (B1.2-B) was replaced by compound (B1.5-A), to obtain the target product (S)-2-amino-N-((2-(((7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethoxy)methyl) acetamide (B1.7) as a mixture, which was directly used in the following synthesis reaction.

ESI-MS (m/z): 566 [M+H]⁺.

### Example B1.8: Synthesis of (S)-2-amino-N-(3-(7-ethyl-7-hydroxy-8,11-dioxo-7,8,11,13-tetrahydro-10H-[1,3] dioxolo[4,5-g]pyrano[3',4':6,7]indolizino[1,2-b]quinolin-14-yl)phenyl)acetamide (B1.8)

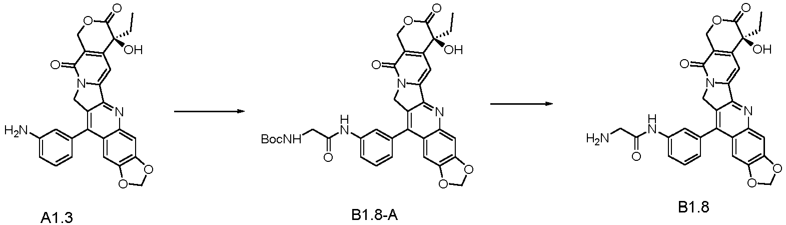

Using the same method and reaction conditions as Example B1.4, compound (B1.3-A) was replaced by compound (A1.3), to obtain the target product (S)-2-amino-N-(3-(7-ethyl-7-hydroxy-8,11-dioxo-7,8,11,13-tetrahydro- 10H-[1,3]-dioxolo[4,5-g]pyrano[3',4':6,7]indolizino[1,2-b]quinolin-14-yl)phenyl)acetamide (B1.8).

ESI-MS (m/z): 541 [M+H]⁺.

### Example B1.9: Synthesis of (S)-2-amino-N-(4-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)phenyl)acetamide (B1.9)

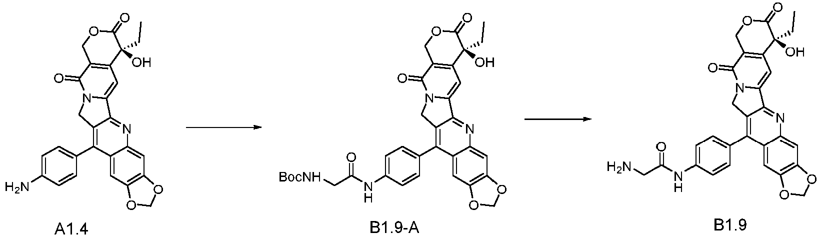

Using the same method and reaction conditions as Example B1.4, compound (B1.3-A) was replaced by compound (A1.4), to obtain the target product (S)-2-amino-N-(4-(7-ethyl-7-hydroxy-8,11-dioxo-7,8,11,13-tetrahydro- 10H-[1,3]-dioxolo[4,5-g]pyrano[3',4':6,7]indolizino[1,2-b]quinolin-14-yl)phenyl)acetamide (B1.9). Alternatively, the following reaction conditions can be used to obtain the target product.

Compound A1.4 (60 mg, 0.12 mmol) was dissolved in N,N-dimethylformamide (3 mL), to which were successively added (tert-butoxycarbonyl)glycine (26 mg, 0.15 mmol), HATU (56 mg, 0.15 mmol), and N,N-diisopropylethylamine (48 mg, 0.37 mmol), and then the mixture was stirred at room temperature for 1 h. TLC indicated completion of the reaction. TFA (1.0 mL) was then added directly to the above reaction solution. The reaction was continually stirred at room temperature for 1 h, and LCMS indicated that the reaction was completed. The reaction solution was concentrated to remove trifluoroacetic acid and obtain a crude product, which was purified by preparative chromatography (0.01% trifluoroacetic acid aqueous solution, acetonitrile) to obtain the target product (S)-2-amino-N-(4-(7-ethyl-7-hydroxy-8,11-dioxo-7,8,11,13-tetrahydro-10H-[1,3]- dioxolo[4,5-g]pyrano[3',4':6,7]indolizino[1,2-b]quinolin-14-yl)phenyl)acetamide (B1.9) (26.3 mg, yield 38%) as yellow solid.

ESI-MS (m/z): 541 [M+H]⁺;
¹H NMR (400 MHz, DMSO) δ 10.71 (s, 1H), 8.16 (s, 2H), 7.86 (d, *J =* 8.6 Hz, 2H), 7.67-7.58 (m, 3H), 7.29 (s, 1H), 7.04 (s, 1H), 6.50 (s, 1H), 6.28 (s, 2H), 5.40 (s, 2H), 5.05 (s, 2H), 3.87 (s, 2H), 1.93-1.81 (m, 2H), 0.88 (t, *J =* 7.3 Hz, 3H).

### Example B1.10: Synthesis of (S)-2-amino-N-(3-(7-ethyl-7-hydroxy-8,11-dioxo-7,8,11,13-tetrahydro-10H- [1,3]-dioxolo[4,5-g]pyrano[3',4':6,7]indolizino[1,2-b]quinolin-14-yl)propyl)acetamide (B1.10)

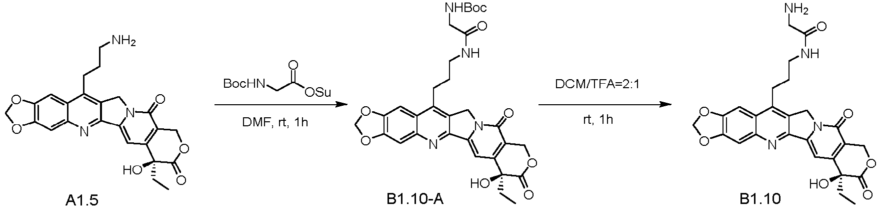

### Step 1:

At room temperature, to a solution of compound A1.5 (200 mg) in N,N-dimethylformamide (5 mL), were successively added triethylamine (67 mg) and 2,5-dioxopyrrolidin-1-yl (tert-butoxycarbonyl)glycinate (200 mg) and then the mixture was allowed to react for 1 h at room temperature. The reaction solution was diluted with water (30 mL) and then extracted with ethyl acetate (30 mL × 2). The organic phase was washed with saturated brine, dried over anhydrous sodium sulfate, and concentrated to obtain the crude product, which was further purified by C18 column (acetonitrile/0.05% formic acid aqueous solution: 5%-60%) to obtain the target compound (B1.10-A, 100 mg).

LCMS (ESI) [M+H]⁺: 607.

### Step 2:

To a solution of compound B1.10-A (100 mg) in dichloromethane (4 mL), was added trifluoroacetic acid (2 mL), and the reaction was stirred at room temperature for 1 h. The reaction solution was concentrated, and then N,N-dimethylformamide solution (3 mL) was added to the crude product, and further purified by C18 column (acetonitrile/0.05% formic acid in water: 5%-60%), to provide the target compound (S)-2-amino-N-(3-(7-ethyl-7-hydroxy-8,11-dioxo-7,8,11,13-tetrahydro-10H-[1,3]-dioxolo[4,5-g]pyrano[3',4':6,7] indolizino[1,2-b]quinolin-14-yl)propyl)acetamide (B1.10, 80 mg).

LCMS (ESI) [M+H]⁺: 507;
¹H NMR (400 MHz, DMSO-d6) δ 8.26 (s, 1H), 7.64 (s, 1H), 7.51 (s, 1H), 7.24 (s, 1H), 6.51 (s, 1H), 6.29 (s, 2H), 5.42 (s, 2H), 5.23 (s, 2H), 3.31 (s, 2H), 3.30 - 3.27 (m, 2H), 3.13 - 3.07 (m, 2H), 2.04 - 1.76 (m, 4H), 0.87 (t, *J =* 7.3 Hz, 3H).

### Example B1.11: Synthesis of (S)-2-((2-aminoacetamido)methoxy)-N-isopropyl-N-(2-(7-ethyl-7- hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide (B1.11)

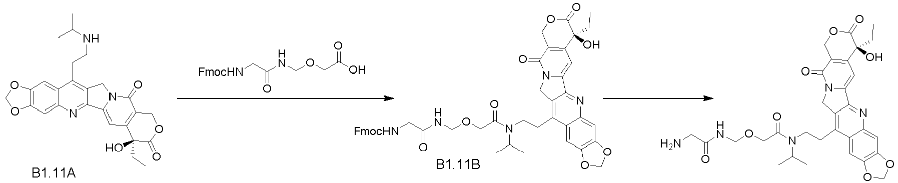

### Step 1:

Compound 1-(9H-fluoren-9-yl)-3,6-dioxo-2,9-dioxo-4,7-diazacyclo-11-carboxylic acid (242 mg), diisopropylethylamine (330 mg) and N,N,N',N'-tetramethylurea hexafluorophosphate (359 mg) were dissolved in N,N-dimethylformamide (10 mL), to which was then added (S)-7-ethyl-7-hydroxy- 14-(2-(isopropylamino)ethyl)-10,13-dihydro-11H-[1,3]dioxolo[4,5-g]pyrano[3',4':6,7]indolizino[1,2-b]quinolin-8,11(7H)-dione (300 mg). The reaction was stirred at room temperature for 2 h. Ethyl acetate (50 mL) was added to the reaction solution, and the resultant solution was washed with saturated brine (30 mL × 3), dried over anhydrous sodium sulfate, filtered . The filtrate was concentrated under reduced pressure to a solid, and the crude product was separated and purified by column chromatography (dichloromethane : methanol = 10/1) to obtain the target compound B1.11B (220 mg).

LCMS (ESI) [M+H]⁺: 844.0.

### Step 2: Preparation of (S)-2-((2-aminoacetamido)methoxy)-N-isopropyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide

To a solution of compound B1.11B (220 mg, 0.261 mmol) in N,N-dimethylformamide (4 mL), was added piperidine (1 mL), and the reaction solution was stirred at room temperature for 20 min. LCMS indicated completion of the reaction. The reaction solution was concentrated under reduced pressure to obtain the target compound (220 mg) as a brown solid, and the above product was directly used in the next step without purification.

LCMS (ESI) [M+H]⁺: 622.1.

### Example B1.12: Synthesis of (S)-2-amino-N-((4-(4-ethyl-8-fluoro-4-hydroxy-9-methyl-3,14-dioxo- 3,4,12,14-tetrahydro-1H-pyrano[3',4':6,7]indolizino[1,2-b]quinolin-11-yl)propoxy)methyl)acetamide (B1.12)

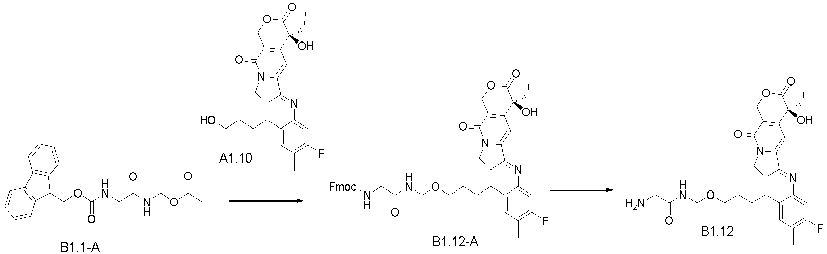

### Step 1:

Compound A1.10 (160 mg, 0.365 mmol) was dissolved in N,N-dimethylformamide (3 mL), to which was added (2-((((9H-fluoren-9-yl)methoxy)carbonyl)amino)acetamido)methyl acetate ((B1.1-A, 672 mg, 1.83 mmol), and then HCl-ethyl acetate (0.073 mL, 3M) was added to the reaction solution. The reaction solution was stirred at room temperature overnight. LCMS detection showed the reaction was complete . The reaction solution was directly purified by reversed phase chromatography (acetonitrile/0.05% FA aqueous solution: 5% to 50%) to obtain the target compound (80 mg, yield 29.0%) as a white solid.

LCMS (ESI) [M+H]⁺ = 747.4;
¹H NMR (400 MHz, DMSO-d6) δ 8.74 (t, *J =* 6.4 Hz, 1H), 8.28 - 8.17 (m, 1H), 7.99 - 7.88 (m, 3H), 7.73 (d, *J =* 7.3 Hz, 2H), 7.63 (t, *J =* 5.7 Hz, 1H), 7.44 (t, *J =* 7.4 Hz, 2H), 7.35 (t, *J =* 7.2 Hz, 3H), 6.58 (s, 1H), 5.48 (s, 2H), 5.29 (s, 2H), 4.66 (d, *J =* 6.3 Hz, 2H), 4.32 (d, *J =* 6.9 Hz, 2H), 4.26 (d, *J =* 6.1 Hz, 1H), 3.71 (d, *J =* 5.8 Hz, 2H), 3.57 (t, *J =* 5.7 Hz, 2H), 3.29 - 3.20 (m, 2H), 2.55 (s, 3H), 2.00 - 1.86 (m, 4H), 0.93 (t, *J =* 7.2 Hz, 3H).

Step 2: B1.12-A (240 mg) was dissolved in DMF (5 ml), to which was added piperidine (1 ml), and then the compounds were stirred for 20 min. The reaction solution was evaporated under reduced pressure to remove low-boiling components, and the residue was directly used in the next step.

ESI-MS (m/z): 525.2 [M+H]⁺.

A small amount of crude product was purified by reversed-phase chromatography (acetonitrile/0.05% FA in water: 5% to 50%) to give the target compound.

ESI-MS (m/z): 525.1 [M+H]⁺;
¹H NMR (400 MHz, DMSO) δ 9.13 (t, *J =* 6.6 Hz, 1H), 8.21 (d, *J =* 8.1 Hz, 1H), 8.02 (brs, 2H), 7.89 (d, *J =* 10.8 Hz, 1H), 7.32 (s, 1H), 6.54 (s, 1H), 5.44 (s, 2H), 5.28 (s, 2H), 4.66 (d, *J =* 6.5 Hz, 2H), 3.64 (s, 2H), 3.53 (t, *J* = 6.1 Hz, 2H), 3.25-3.18 (m, 2H), 2.52 (s, 3H), 1.98-1.84 (m, 4H), 0.88 (t, *J =* 7.3 Hz, 3H).

### Example B1.13: Synthesis of (S)-2-amino-N-(3-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)acetamide (B1.13)

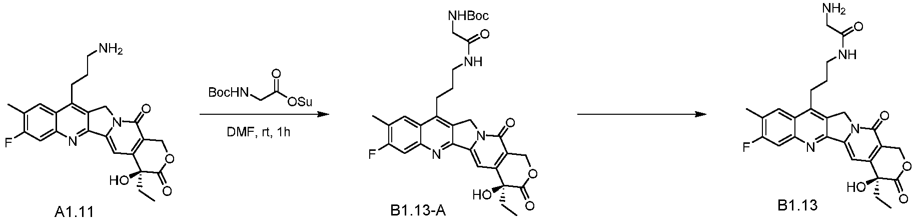

### Step 1:

At room temperature, to a solution of compound A1.11 (200 mg) in N,N-dimethylformamide (5 mL), were successively added triethylamine (67 mg) and 2,5-dioxopyrrolidin-1-yl (tert-butoxycarbonyl)glycinate (182 mg) and then the mixture was allowed to react for 1 h at room temperature. The reaction solution was diluted with water (30 mL) and then extracted with ethyl acetate (30 mL × 2). The organic phase was washed with saturated brine, dried over anhydrous sodium sulfate, and concentrated to obtain the crude product, which was further purified by C18 column (acetonitrile/0.05% formic acid aqueous solution: 5%-60%) to obtain the target compound (B1.13-A, 104 mg).

LCMS (ESI) [M+H]⁺: 595.

### Step 2:

To a solution of compound B1.13-A (100 mg) in dichloromethane (4 mL), was added trifluoroacetic acid (2 mL), and then the mixture was stirred at room temperature for 1 h. The reaction solution was concentrated, and N,N-dimethylformamide solution (3mL) was added to the crude product, which was further purified by C18 column (acetonitrile/0.05% formic acid aqueous solution: 5%-60%) to obtain the target compound (S)-2-amino-N-(3-(4-ethyl-8-fluoro-4-hydroxy-9-methyl-3,14-dioxo-3,4,12,14-tetrahydro-1H-pyrano[3',4':6,7] indolizino[1,2-b]quinolin-11-yl)propyl)acetamide (B1.13, 88 mg).

LCMS (ESI) [M+H]⁺: 495.

### Example B1.14: Synthesis of (S)-2-amino-N-((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)acetamide (B1.14)

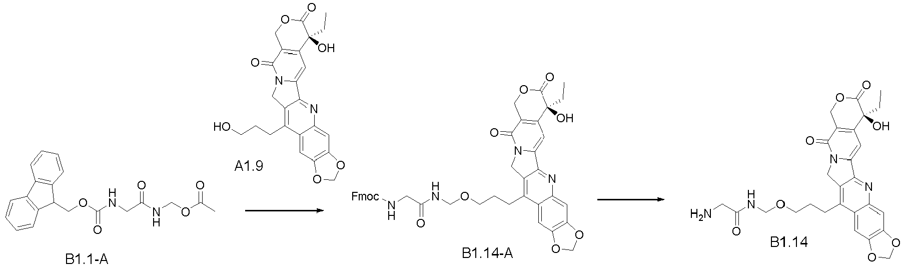

Step 1: Compound (B1.1-A) (368 mg), compound (A1.9) (440 mg) and pyridinium p-toluenesulfonic acid (PPTS) (25 mg) were refluxed in dichloromethane (20 ml) for 20 h, and then the reaction solution was washed with aqueous sodium bicarbonate solution and aqueous hydrochloric acid solution, respectively. The organic solvent was removed under reduced pressure to obtain a crude product, which was separated and purified by column chromatography (dichloromethane:methanol = 10/1) to obtain the target compound B1.14A (240 mg).

LCMS (ESI) [M+H]⁺: 747.

Step 2: B1.14-A (240 mg) was dissolved in DMF (5 ml), to which was added piperidine (1ml), and then the mixture was stirred for 20 min. The low boiling point components were removed under reduced pressure, and the residue was directly used in the next synthesis. A small amount of the crude product was purified by reversed phase chromatography (acetonitrile/0.05% FA aqueous solution: 5% to 50%) to obtain the target compound.

ESI-MS (m/z): 525 [M+H]⁺;
¹H NMR (400 MHz, DMSO-d6) δ 9.13 (t, 1H), 8.04 (br, 2H), 7.58 (s, 1H), 7.51 (s, 1H), 7.25 (s, 1H), 6.29 (s, 2H), 5.43 (S, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

### Example B1.15: (S,E)-2-amino-N-(3-(7-ethyl-7-hydroxy-8,11-dioxo-7,8,11,13-tetrahydro- 10H-[1,3]dioxolo[4,5-g]pyrano[3',4':6,7]indolizino[1,2-b]quinolin-14-yl)allyl)acetamide

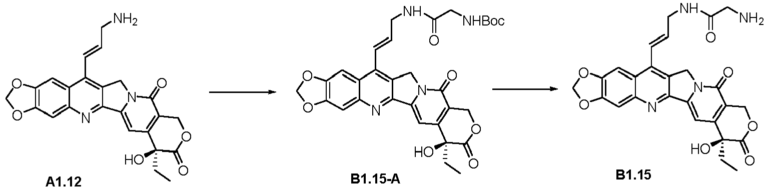

### Step 1:

At room temperature, to a solution of compound A1.12 (200 mg, 0.447 mmol) in N,N-dimethylformamide (5 mL), were added triethylamine (135 mg, 1.341 mmol) and N-hydroxy-2,5-dioxopyrrolidine tert-butoxycarbonylglycine ester (183 mg, 0.671 mmol), and then the mixture was allowed to react at room temperature for 1 h. LCMS showed the reaction has completed. Water (20 mL) was added to the reaction solution, and the resultant mixture was extracted with ethyl acetate (20 mL×3). The organic phase was washed with saturated brine, dried over anhydrous sodium sulfate, and concentrated to obtain the target compound B1.15A (130 mg, yield: 48%) as a brown solid.

LCMS (ESI) [M+H]⁺ = 605.6.

### Step 2:

At room temperature, compound B1.15-A (130 mg, 0.215 mmol) was dissolved in 1,4-dioxane HCl solution (5 mL) and then reacted at room temperature for 30 min. LCMS showed the reaction was complete. The reaction solution was purified by reversed phase column chromatography, to obtain the target compound (60 mg, yield: 52%) as a brown solid.

LCMS (ESI) [M+H]⁺ =505.2;
¹H NMR (400 MHz, DMSO-d6) δ 8.85 (s, 1H), 8.15 (s, 2H), 7.70 (s, 1H), 7.54 (s, 1H), 7.26 (s, 1H), 7.21 (d, *J* = 16.2 Hz, 1H), 6.51 (d, *J =* 16.2 Hz, 1H), 6.31 (s, 2H), 5.42 (s, 2H), 5.28 (s, 2H), 4.19 (s, 2H), 1.86 - 1.82 (m, 4H), 0.87 (t, *J =* 7.3 Hz, 3H).

### Example B1.16: (S,E)-2-amino-N-(((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)acetamide

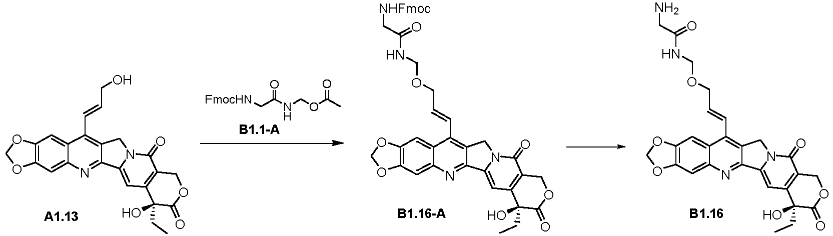

### Step 1:

Under the protection of nitrogen, the starting material A1.13 (150 mg, 0.335 mmol) was dissolved in toluene (3 mL), to which was added (2-((((9H-fluoren-9-yl)methoxy)carbonyl)amino)acetamido)methyl acetate (B1.1-A, 308 mg, 0.84 mmol), and then zinc acetate (123 mg, 0.67 mmol) was added to the reaction solution. The reaction solution was stirred at 100 °C overnight. The LCMS detection indicated the reaction was complete. The reaction solution was purified directly by reversed-phase chromatography (acetonitrile /0.05% formic acid aqueous solution: 5% to 50%), to obtain the target compound (9H-fluoren-9-yl)methyl(S,E)-(2-(((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)carbamate (B1.16-A, 80 mg, yield 31%) as white solid.

LCMS (ESI) [M+H]⁺ = 757.4.

### Step 2:

B1.16-A (80 mg, 0.11 mmol) was dissolved in N,N-dimethylformamide (2 ml), to which was added piperidine (0.05 mL), and then the mixture was stirred at room temperature for 1 h. LCMS indicated completion of the reaction. The reaction solution was concentrated to obtain a crude product, which was separated and purified by C18 reversed phase column (acetonitrile/0.05% aqueous ammonia solution: 5% to 50%) to obtain the target compound (35 mg, yield 62%) as a brown solid.

LCMS (ESI) [M+H]⁺ = 535.2, tR = 1.070 min.

### Example B1.17: (S,E)-2-amino-N-(((3-(4-ethyl-8-fluoro-4-hydroxy-9-methyl-3,14-oxo-3,4,12,14-tetrahydro- 1H-pyrano[3',4':6,7|indolizino[1,2-b]quinolin-11-yl)allyl)oxy)methyl)acetamide

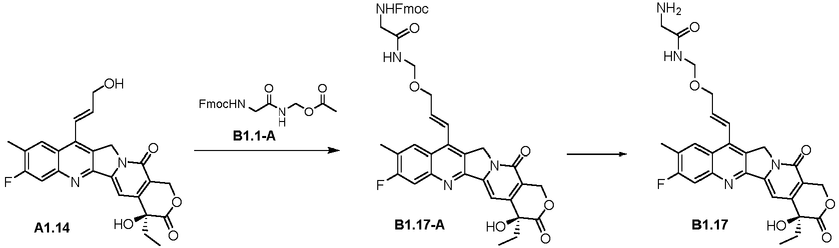

### Step 1:

Compound A1.14 (80 mg, 0.183 mmol) and compound (2-((((9H-fluoren-9-yl)methoxy)carbonyl)amino)acetamido)methyl acetate (B1.1-A, 135 mg, 0.367 mmol) were dissolved in toluene (4 mL), to which was added zinc acetate (190 mg, 1.03 mmol), and then the reaction solution was stirred at 100 °C for 48 h. The reaction solution was cooled to room temperature, and water (10 mL) was added. The resultant mixture was extracted with ethyl acetate (10 mL×3). The combined organic phases were dried over anhydrous sodium sulfate, filtered, and concentrated to give the crude product, which was purified by reversed phase chromatography (0.05% formic acid in water/acetonitrile: 5% to 100%) to give the target compound (S,E)-2-(Fmoc amino)-N-(((3-(4-ethyl-8-fluoro-4-hydroxy-9-methyl-3,14-oxo-3,4,12,14-tetrahydro-1H-pyrano[3',4':6,7]indolizino[1,2-b]quinolin-11-yl)allyl)oxy)methyl)acetamide (B1.17-A, 30 mg) as white solid.

LCMS (ESI) [M+H]⁺ = 745.2;
¹H NMR (400 MHz, DMSO) δ 8.82 (m 1H), 8.21 (m, 1H), 7.89 (m, 2H), 7.84 (d, *J =* 7.8 Hz, 2H), 7.68 (m, 1H), 7.61 (m, 1H), 7.42 (m, 1H), 7.37 (m, 1H), 7.33 - 7.28 (m, 4H), 6.72 - 6.55 (m, 1H), 6.53 (s, 1H), 5.42 (s, 2H), 5.28 (s, 2H), 4.75 (m, 2H), 4.34 (m, 2H), 4.28 - 4.24 (m, 2H), 4.20 (m, 1H), 3.70 (d, *J =* 6.1 Hz, 2H), 2.47 (s, 3H), 1.90 - 1.82 (m, 2H), 0.88 (t, *J =* 7.3 Hz, 3H).

### Step 2:

Compound 4 (25 mg, 0.034 mmol) was dissolved in N,N-dimethylformamide (2 mL), to which was then added diethylamine (0.2 mL), and after completion of addition, the reaction solution was stirred at room temperature for 1 h. LCMS indicated completion of the reaction, and then the reaction solution was directly evaporated to dryness under reduced pressure to obtain the crude target compound (S,E)-2-amino-N-(((3-(4-ethyl-8-fluoro-4-hydroxy-9-methyl-3,14-oxo-3,4,12,14-tetrahydro-1H-pyrano[3',4':6,7] indolizino[1,2-b]quinolin-11-yl)allyl)oxy)methyl)acetamide as yellow sticky product.

LCMS (ESI) [M+H]+ = 523.2;
LCMS (ESI) [M+H]⁺ = 523.1, t_{R} = 0.446 min, 1.311 min.

¹H NMR (400 MHz, DMSO) δ 9.26 (t, *J =* 6.5 Hz, 1H), 8.23 *(d, J* = 8.2 Hz, 1H), 8.06 (s, 2H), 7.91 (m, 1H), 7.40 (d, *J =* 16.3 Hz, 1H), 7.34 (s, 1H), 6.70 - 6.60 (m, 1H), 6.54 (s, 1H), 5.44 (s, 2H), 5.35 (s, 2H), 4.80 (d, *J =* 6.6 Hz, 2H), 4.38 (d, *J =* 3.9 Hz, 2H), 3.68 (d, 2H), 2.52 (s, 3H), 1.90 - 1.84 (m, 2H), 0.88 (m, 3H).

### C. Synthesis of molecular fragment intermediates containing coupling linking segments

### Example C1.1: N⁶-(tert-butoxycarbonyl)-N²-((1-(2-(methylthio)pyrimidin-5-yl)-1-oxo-5,8,11,14,17,20,23,26,29-nonaoxa-2-aza-hentriacontanoyl)-L-valinyl)-L-lysine (compound C1.1)

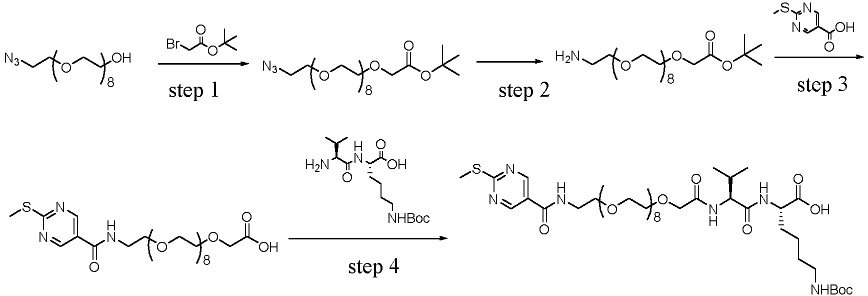

### Step 1: tert-butyl 29-azido-3,6,9,12,15,18,21,24,27-nonaoxa-nonacosanoate

To a solution of 26-azido-3,6,9,12,15,18,21,24-octaoxa-hexacosan-1-ol (4.39g) in DMF (40ml), was added NaH (0.6 g, 60%). After the above mixture was stirred for 30 min, tert-butyl bromoacetate (2.4 g) was added. The mixture was stirred under dry conditions for 20 h. Then, ethyl acetate (200 ml) and water (200 ml, added slowly at the beginning) were added to the mixture. The organic phase was washed with water (100 ml x 3), and then dried over anhydrous sodium sulfate. The solvent was removed under reduced pressure, and the residue was separated by silica gel column chromatography to get the target product tert-butyl 29-azido-3,6,9,12,15,18,21,24,27-nonaoxa-nonacosanoate.

ESI-MS *(m*/*z):* 554 [M + H]⁺.

### Step 2: tert-butyl 29-amino-3,6,9,12,15,18,21,24,27-nonaoxa-nonacosanoate

To a solution of tert-butyl 29-azido-3,6,9,12,15,18,21,24,27-nonaoxa-nonacosanoate (1.16 g) in ethyl acetate (20 ml), was added Pd/C catalyst (Pd/C,10%, 100 mg). The solution was stirred under hydrogen atmosphere for 5 h, and then filtered to remove Pd/C. The solvent was removed under reduced pressure to obtain the target product.

ESI-MS *(m*/*z):* 528 [M + H]⁺.

### Step 3: 1-(2-(methylthio)pyrimidin-5-yl)-1-oxo-5,8,11,14,17,20,23,26,29-nonaoxa-2-aza-hentriacontanoic acid

tert-Butyl 29-amino-3,6,9,12,15,18,21,24,27-nonaoxa-nonacosanoate (527 mg) and 2-methylthio-pyrimidine-5-carboxylic acid (170 mg) were added to dry DMF (10 ml), to which were then added DIPEA (0.2 ml) and HBTU (420 mg) successively under an ice bath. The above mixture was stirred at room temperature for 20 h. Then, the reaction solution was diluted with ethyl acetate (100 ml), and washed with water (100 ml x 4). The organic phase was dried over anhydrous sodium sulfate, and the organic solvent was removed under reduced pressure. The residue was dissolved in DCM (10 ml), to which was then added TFA (10 ml), and then the mixture was stirred at room temperature for 1 h. The low-boiling components were removed under reduced pressure, and the residue was separated by preparative HPLC to obtain 1-(2-(methylthio)pyrimidin-5-yl)-1-oxo-5,8,11,14,17,20,23,26,29-nonaoxa-2-aza-hentriacontanoic acid.

ESI-MS *(m*/*z):* 624 [M + H]⁺.

### Step 4: N⁶-(tert-butoxycarbonyl)-N²-((1-(2-(methylthio)pyrimidin-5-yl)-1-oxo-5,8,11,14,17,20,23,26,29-nonaoxa-2-aza-hentriacontanoyl)-L-valinyl)-L-lysine

1-(2-(Methylthio)pyrimidin-5-yl)-1-oxo-5,8,11,14,17,20,23,26,29-nonaoxa-2-aza-hentriacontanoic acid (623 mg) was dissolved in DMF (10 ml), to which were added DIPEA (300 µl) and HBTU (420 mg) at 0 °C. The mixture was stirred for 30 min, and then a valinyl-(Boc)lysine dipeptide compound (345 mg) was added. The reaction was stirred for 20 h, and then ethyl acetate (100 ml) was added. The resultant solution was washed with dilute hydrochloric acid (20 ml x 3). The organic phase was dried, and the organic solvent was removed under reduced pressure. The crude product was separated by preparative HPLC to obtain the target compound N⁶-(tert-butoxycarbonyl)-N²-((1-(2-(methylthio)pyrimidin-5-yl)-1-oxo-5,8,11,14,17,20,23,26,29-nonaoxa-2-aza-hentriacontanoyl)-L-valinyl)-L-lysine (compound C1.1).

ESI-MS *(m*/*z):* 951 [M + H]⁺.

### Example C1.2 : N⁶-(tert-butoxycarbonyl)-N²-((29-(4-(2-(methylthio)pyrimidin-5-yl)-1H-1,2,3-triazol-1-yl)-3,6,9,12,15,18,21,24,27-nonaoxa-nonacosanoyl)-L-valinyl)-L-lysine (compound C1.2)

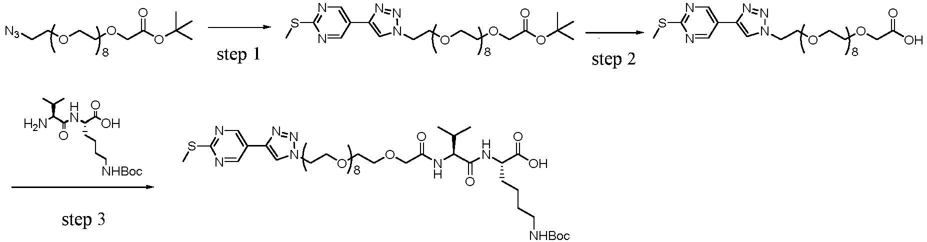

### Step 1: tert-Butyl 29-(4-(2-(methylthio)pyrimidin-5-yl)-1H-1,2,3-triazol-1-yl)-3,6,9,12,15,18,21,24,27-nonaoxa-nonacosanoate

tert-Butyl 29-azido-3,6,9,12,15,18,21,24,27-nonaoxa-nonacosanoate (1.7 g) and 2-methylthio-5-ethynylpyrimidine (450 mg) were dissolved in DMSO-H₂O (20 ml, 4:1), to which was added cuprous bromide (50 mg). The reaction solution was stirred at room temperature for 2 h, and then ethyl acetate (100 ml) was added. The resultant solution was washed with water (100 ml x 3) and dried, and then the organic solvent was removed under reduced pressure. The crude product was separated by silica gel column chromatography to obtain the target product tert-butyl 29-(4-(2-(methylthio)pyrimidin-5-yl)-1H-1,2,3-triazol-1-yl)-3,6,9,12,15,18,21,24,27-nonaoxa-nonacosanoate.

MS (*m*/*z*)*:* 704 [M + H]⁺.

### Step 2: 29-(4-(2-(methylthio)pyrimidin-5-yl)-1H-1,2,3-triazol-1-yl)-3,6,9,12,15,18,21,24,27-nonaoxa-nonacosanoic acid

tert-Butyl 29-(4-(2-(methylthio)pyrimidin-5-yl)-1H-1,2,3-triazol-1-yl)-3,6,9,12,15,18,21,24,27-nonaoxa-nonacosanoate (1g) was dissolved in dichloromethane (10 ml), to which was then added TFA (5 ml). The mixture was allowed to stand at room temperature for 1 h, and then the low-boiling components were removed under reduced pressure to obtain the target product 29-(4-(2-(methylthio)pyrimidin-5-yl)-1H-1,2,3-triazol-1-yl)-3,6,9,12,15,18,21,24,27-nonaoxa-nonacosanoic acid.

MS (*m*/*z*)*:* 648 [M + H]⁺.

### Step 3: N⁶-(tert-butoxycarbonyl)-N²-((29-(4-(2-(methylthio)pyrimidin-5-yl)-1H-1,2,3-triazol-1-yl)-3,6,9,12,15,18,21,24,27-nonaoxa-nonacosanoyl)-L-valinyl)-L-lysine

29-(4-(2-(Methylthio)pyrimidin-5-yl)-1H-1,2,3-triazol-1-yl)-3,6,9,12,15,18,21,24,27-nonaoxa-nonacosanoic acid (647 mg) was dissolved in DMF (10 ml), to which were added DIPEA (300 µl) and HBTU (420 mg) at 0 °C. The mixture was stirred for 30 min, to which was then added valinyl-lysine dipeptide compound (345 mg). The reaction was stirred for 20 h, and then ethyl acetate (100 ml) was added. The resultant solution was washed with dilute hydrochloric acid (20 ml x 3). After the organic phase was dried, the organic solvent was removed under reduced pressure, and the crude product was separated by preparative HPLC to obtain the target compound N⁶-(tert-butoxycarbonyl)-N²-((29-(4-(2-(methylthio)pyrimidin-5-yl)-1H-1,2,3-triazol-1-yl)-3,6,9,12,15,18,21,24,27-nonaoxa-nonacosanoyl)-L-valinyl)-L-lysine (compound C1.2).

ESI-MS (*m*/*z*)*:* 975 [M + H]⁺.

### Example C1.3: (1-(2-(methylthio)pyrimidin-5-yl)-1-oxo-5,8,11,14,17,20,23,26,29-nonaoxa-2-azahentriacontanoyl)-glycyl-glycyl-L-phenylalanine (compound C1.3)

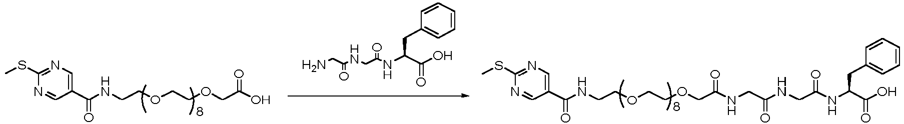

By the same method and reaction conditions as step 4 in Example C1.1, corresponding raw materials were used to obtain the target product (1-(2-(methylthio)pyrimidin-5-yl)-1-oxo-5,8,11,14,17,20,23,26,29-nonaoxa-2-aza-hentriacontanoyl)-glycyl-glycyl-L-phenylalanine (compound C1.3).

ESI-MS (m/z): 885 [M+H]⁺.

### Example C1.4 : (36-(2-(methylthio)pyrimidin-5-yl)-31-oxo-3,6,9,12,15,18,21,24,27-nonaoxa-30-aza-hexatriacontan-(35-yne)-oyl)glycylglycylphenylalanine (compound C1.4)

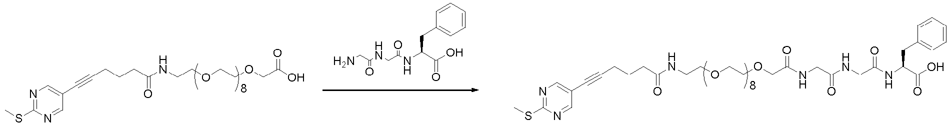

By the same method and reaction conditions as step 4 in Example C1.1, the raw materials shown in the reaction scheme were used to obtain the target product (36-(2-(methylthio)pyrimidin-5-yl)-31-oxo-3,6,9,12,15,18,21,24,27-nonaoxa-30-aza-hexatriacontan-(35-yne)-oyl)glycylglycylphenylalanine (compound C1.4).

ESI-MS (m/z): 951 [M+H]⁺.

### Example C1.5: N⁶-(tert-butoxycarbonyl)-N²-((36-(2-(methylthio)pyrimidin-5-yl)-31-oxo-3,6,9,12,15,18,21,24,27-nonaoxa-30-aza-hexatriacont-(35-yn)-oyl)-L-valinyl)-L-lysine (C1.5)

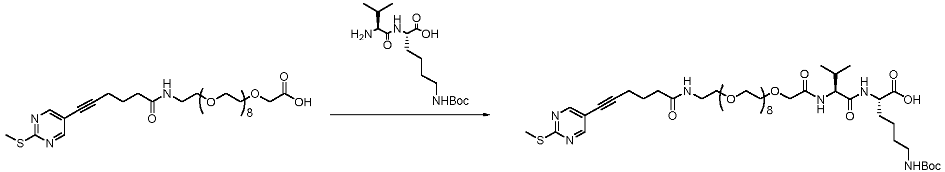

By the same method and reaction conditions as step 4 in Example C1.1, the raw materials shown in the reaction scheme were used to obtain the target product N⁶-(tert-butoxycarbonyl)-N²-((36-(2-(methylthio)pyrimidin-5-yl)-31-oxo-3,6,9,12,15,18,21,24,27-nonaoxa-30-aza-hexatriacont-(35-yn)-oyl)-L-valinyl)-L-lysine (compound C1.5).

ESI-MS (m/z): 1017 [M+H]⁺.

### Example C1.6: N⁶-(tert-butoxycarbonyl)-N²-((6-(2-(methylthio)pyrimidin-5-yl)-hexan-5-ynoyl)-L-valyl)- L-lysine (compound C1.6)

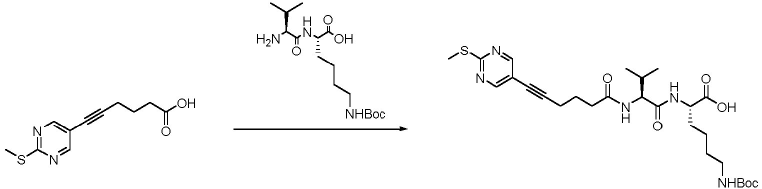

6-(2-(Methylthio)pyrimidin-5-yl)hex-5-ynoic acid (920 mg) and N-hydroxysuccinimide (537 mg) were dissolved in dichloromethane (50 mL), to which was then added dicyclohexylcarbodiimide (963 mg). The reaction solution was stirred at room temperature for 1 h, and then dichloromethane was removed under reduced pressure. The residue was dissolved in N,N-dimethylformamide (50 mL), to which was then added N²-L-valinyl-N⁶-(Boc)-L-lysine (1.5 g). The reaction solution was stirred at room temperature for 16 h, poured into 200 mL of water, and then adjusted to pH 11 with saturated sodium bicarbonate solution. The above aqueous solution was extracted twice with ethyl acetate, and the organic phase was discarded. The aqueous phase was adjusted to pH 4-5 with citric acid, and then extracted three times with ethyl acetate (100 mL x 3). The organic phase was washed with saturated brine (100 mL), dried over anhydrous sodium sulfate, filtered, and rotary evaporated to obtain 2.8 g of crude product, which was separated and purified by flash chromatography (DCM:MeOH = 30:1) to obtain the target compound N⁶-(tert-butoxycarbonyl)-N²-((6-(2-(methylthio)pyrimidin- 5-yl)-hexan-5-ynoyl)-L-valyl)-L-lysine (1.0 g) (compound C1.6).

ESI-MS (m/z): 564.3 [M+H]⁺.

¹H NMR (400 MHz, DMSO-*d*₆) δ 12.45 (s, 1H), 8.68 (s, 2H), 8.12 (d, *J =* 7.3 Hz, 1H), 7.88 (d, *J =* 8.9 Hz, 1H), 6.76 (s, 1H), 4.27 - 4.19 (m, 1H), 4.11 (d, *J =* 4.9 Hz, 1H), 2.88 (d, *J =* 7.9 Hz, 3H), 2.73 (s, 1H), 2.59 (s, 1H), 2.52 (s, 4H), 2.40 - 2.24 (m, 3H), 2.03 - 1.88 (m, 2H), 1.77 (d, *J =* 3.2 Hz, 2H), 1.68 (d, *J =* 7.0 Hz, 1H), 1.60 - 1.49 (m, 1H), 1.36 (s, 15H), 0.85 (dd, *J =* 14.8, 6.7 Hz, 7H).

### Example C1.7: N⁶-(tert-butoxycarbonyl)-N²-((6-(2-(methanesulfonyl)pyrimidin-5-yl)-hexan-5-ynoyl)-L- valinyl)-L-lysine (compound C1.7)

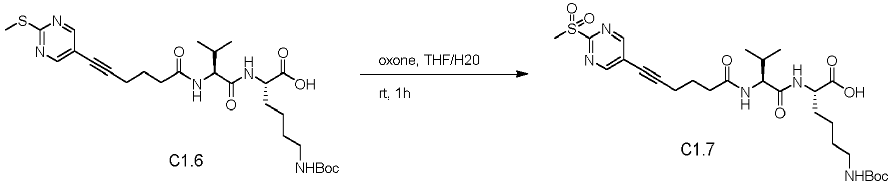

To a solution of compound C1.6 (260 mg) in mixed solvent of tetrahydrofuran (3 mL) and water (3 mL), was added potassium peroxymonosulfonate (1.414 g), and then the mixture was allowed to react under stirring at room temperature for 1 h. The reaction solution was filtered, and the filtrate was purified by C18 column (acetonitrile/0.05% formic acid in water: 5%-60%) to obtain the target compound N⁶-(tert-butoxycarbonyl)-N²-((6-(2-(methanesulfonyl)pyrimidin-5-yl)-hexan-5-ynoyl)-L-valinyl)-L-lysine (compound C1.7) (120 mg).

LCMS (ESI) [M+H]⁺: 596;

¹H NMR (400 MHz, DMSO-d6) δ 12.48 (s, 1H), 9.13 (s, 2H), 8.14 (d, *J =* 7.0 Hz, 1H), 7.90 (d, *J =* 9.1 Hz, 1H), 6.77 (s, 1H), 4.24 (t, *J =* 7.7 Hz, 1H), 4.12 (br s, 1H), 3.41 (s, 3H), 2.89 (d, *J =* 6.0 Hz, 2H), 2.43 - 2.29 (m, 2H), 2.03 - 1.93 (m, 2H), 1.82 (br s, 2H), 1.63 - 1.60 (m, 4H), 1.37 (s, 12H), 0.88 - 0.83 (m, 6H).

### Example C1.8: N⁶-(tert-butoxycarbonyl)-N²-((6-(2-(methylthio)pyrimidin-5-formamido)hexanoyl)-L- valinyl)-L-lysine (compound C1.8)

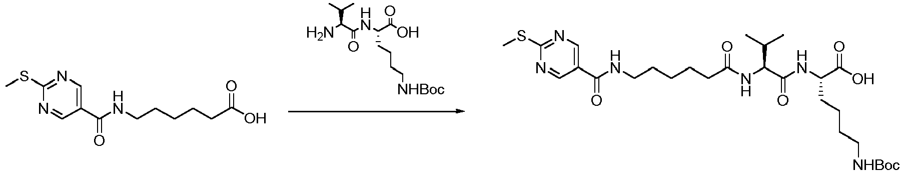

By the same method and reaction conditions as Example C1.6, the raw materials shown in the reaction scheme were used to obtain the target product N⁶-(tert-butoxycarbonyl)-N²-((6-(2-(methylthio)pyrimidin-5-formamido)hexanoyl)-L-valinyl)-L-lysine (compound C1.8).

ESI-MS (m/z): 611 [M+H]⁺.

### Example C1.9: N⁶-(tert-butoxycarbonyl)-N²-((6-(4-(2-(methylthio)pyrimidin-5-yl)-1H- 1,2,3-triazol-1-yl)hexanoyl)-L-valinyl)-L-lysine (compound C1.9)

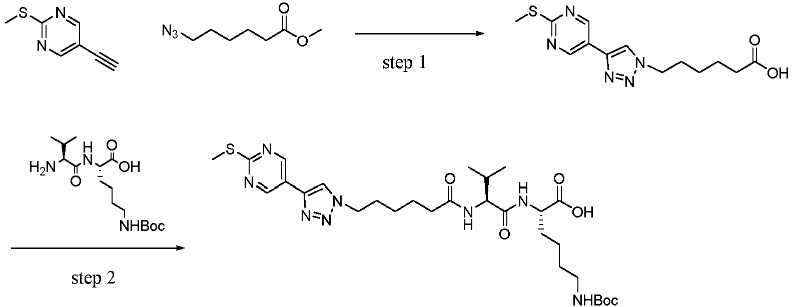

### Step 1: 6-(4-(2-(methylthio)pyrimidin-5-yl)-1H-1,2,3-triazol-1-yl)hexanoic acid

At room temperature, 2-methylthio-5-ethynylpyrimidine (1.5 g) and methyl 6-azidohexanoate (1.71g) were dissolved in mixed solvent of tert-butanol and water (20 mL/25 mL), to which were added sodium vitamin C (5.7 g) and cuprous bromide (2.9g), and then the mixture was allowed to react for 10 h under stirring. Ethyl acetate (200 ml) was added to the reaction solution, and then the resultant solution was washed with water (100 ml x 5). After drying, the organic solvent was removed, and the residue was subjected to silica gel column chromatography to obtain methyl 6-(4-(2-(methylthio)pyrimidin-5-yl)-1H-1,2,3-triazol-1-yl)hexanoate (2.8 g), ESI-MS (m/z): 321.9 [M+H]⁺. The above product was dissolved in tetrahydrofuran (100 ml), to which was added lithium hydroxide (1 M, 20 ml). The mixture was stirred for 3 h, and then hydrochloric acid was added to the reaction solution (pH 2), followed by addition of ethyl acetate (200 ml). The reaction solution was washed with water (100 ml x 3), dried, and the organic solvent was removed to obtain the target product 6-(4-(2-(methylthio)pyrimidin-5-yl)-1H-1,2,3-triazol-1-yl)hexanoic acid. ESI-MS (m/z): 308.4 [M+H]⁺.

### Step 2: N⁶-(tert-butoxycarbonyl)-N²-((6-(4-(2-(methylthio)pyrimidin-5-yl)-1H-1,2,3-triazol-1-yl)hexanoyl)- L-valinyl)-L-lysine

Compound 6-(4-(2-(methylthio)pyrimidin-5-yl)-1H-1,2,3-triazol-1-yl)hexanoic acid (370 mg) and N-hydroxysuccinimide (152 mg) were dissolved in dichloromethane (5 mL) under stirring, to which was then added dicyclohexylcarbodiimide (273 mg), and the reaction solution was then stirred at room temperature for 1 h. 10 mL of water was added to the reaction solution, and the resultant solution was extracted with ethyl acetate (20 mL x 2). The organic phase was washed with saturated brine, dried over anhydrous sodium sulfate, and then filtered. The filtrate was concentrated under reduced pressure to obtain the crude product, which was dissolved in N,N-dimethylformamide (10 mL) under stirring, and then the compound N²-L-valinyl-N⁶-(Boc)-L-lysine was added. The reaction solution was allowed to react at room temperature for 16 hours, and citric acid was slowly added to the reaction solution, so that the pH value was adjusted to be about 5, followed by addition of water. The resultant solution was extracted with ethyl acetate (3 x 10 mL). The organic phase was washed with saturated brine, dried over anhydrous sodium sulfate, and concentrated to remove ethyl acetate and obtain the target product N⁶-(tert-butoxycarbonyl)-N²-((6-(4-(2-(methylthio)pyrimidin-5-yl)-1H-1,2,3-triazol-1-yl)hexanoyl)-L-valinyl)-L- lysine (compound C1.9, 400 mg).

ESI-MS (m/z): 635.3 [M+H]⁺;
¹H NMR (400 MHz, DMSO-d6) δ 12.46 (s, 1H), 9.06 (s, 2H), 9.06 (s, 1H), 8.70 (s, 1H), 8.07 (d, *J =* 7.4 Hz, 1H), 7.77 (d*, J* = 9.0 Hz, 1H), 6.76 (t, *J =* 5.6 Hz, 1H), 4.41 (t, *J =* 7.0 Hz, 2H), 4.19 (dd, *J =* 8.9, 7.0 Hz, 1H), 4.14 - 4.05 (m, 1H), 2.92 - 2.85 (m, 2H), 2.56 (s, 3H), 2.24 - 2.10 (m, 2H), 1.97 - 1.81 (m, 3H), 1.71 - 1.64 (m, 1H), 1.61 - 1.50 (m, 3H), 1.36 (s, 9H), 1.28 - 1.22 (m, 6H), 0.88 - 0.76 (m, 6H).

### Example C1.10: (6-(2-(methylthio)pyrimidin-5-yl)hex--(5-yn)-oyl)glycinylglycinyl-L-phenylalanine (compound C1.10)

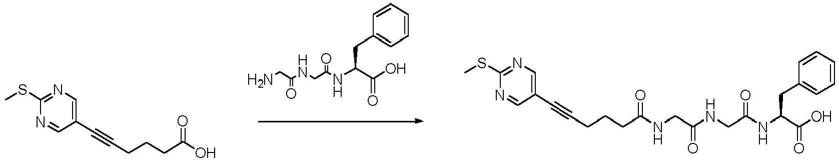

By the same method and reaction conditions as Example C1.6, the raw materials shown in the reaction scheme were used to obtain the target product (6-(2-(methylthio)pyrimidin-5-yl)hex-5-ynoyl) glycinylglycinyl-L-phenylalanine (compound C1.10). ESI-MS (m/z): 498 [M+H]⁺.

By the same method and reaction conditions as Example C1.6, using different reaction raw materials, the target products in the following table were obtained.

| Compound | Chemical structure | Chemical name | ESI-MS (*m*/*z*): [M + H]⁺ |
|---|---|---|---|
| C1.11 | 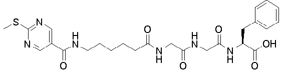 | (6-(2-(methylthio)pyrimidin-5-formamido)hexanoyl)glycinylglyci nyl-L-phenylalanine | 545 |
| C1.12 | 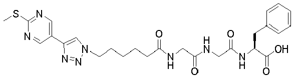 | (6-(4-(2-(methylthio)pyrimidin-5-yl)-1H-1,2,3-triazol-1-yl)hexanoyl) glycinylglycinyl-L-phenylalanine | 569 |

### Example C1.13: (29-(4-(2-(methylthio)pyrimidin-5-yl)-1H-1,2,3-triazol-1-yl)-3,6,9,12,15,18,21,24,27-nonaoxanonacosanoyl) glycinylglycinyl-L-phenylalanine (compound C1.13)

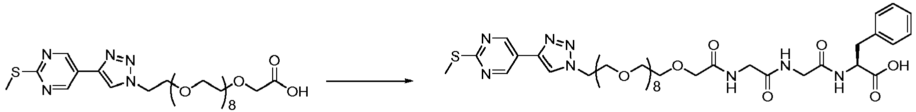

By the same method and reaction conditions as step 4 in Example C1.1, known raw materials were used to obtain target product (29-(4-(2-(methylthio)pyrimidin-5-yl)-1H-1,2,3-triazol-1-yl)-3,6,9,12,15,18,21,24,27-nonaoxanonacosanoyl)glycinylglycinyl-L-phenylalanine (compound C1.13).

ESI-MS (m/z): 909 [M+H]⁺.

Example C1.14: N⁶-(tert-butoxycarbonyl)-N²-((39-(2-(methylthio)pyrimidin-5-yl)-5,34-dioxo-3,9,12,15,18,21,24,27,30-nonaoxa-6,33-diazanonatriacont-38-ynoyl)-L-valinyl)-L-lysine (C1.14)

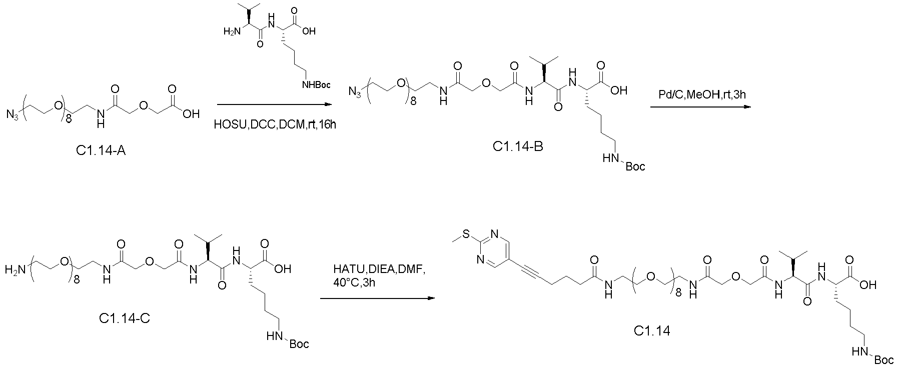

### Step 1:

Compound C1.14-A (336 mg) was dissolved in dichloromethane (5 mL), to which were added N-hydroxysuccinimide (98 mg, 0.85 mmol) and dicyclohexylcarbodiimide (175 mg, 0.85 mmol), and the mixture were allowed to react for 1 h under stirring at room temperature. Then, compound N²-L-valinyl-N⁶-(Boc)-L-lysine (230 mg) was added to the reaction solution, and the mixture was stirred and reacted at room temperature overnight. The reaction solution was concentrated to remove the solvent and obtain a crude product, which was separated and purified by C18 reversed phase column (acetonitrile/0.01% FA aqueous solution: 5%-50%) to obtain a colorless oily target compound (C1.14B, 290 mg).

LCMS (ESI) [M+H]⁺ = 882.3.

### Step 2:

Compound C1.14-B (300 mg) was dissolved in anhydrous methanol (5 mL), to which was added Pd/C (10%, 60 mg), and then hydrogen was purged three times. The reaction was stirred at room temperature overnight, filtered, and concentrated to obtain the target compound C1.14-C (293 mg) as colorless oil.

LCMS (ESI) [M+H]⁺: 856.1.

### Step 3:

6-(2-(Methylthio)pyrimidin-5-yl)hex-5-ynoic acid (84 mg) was dissolved in N,N-dimethylformamide (3 mL), to which were successively added N,N-diisopropylethylamine (61 mg) and HATU (108 mg). The reaction solution was stirred at room temperature for 20 min, and then compound C1.14-C (196 mg) was added. The reaction was stirred at 40 °C for 3 h. The crude product was separated and purified by reversed-phase C18 column (acetonitrile/0.01% FA aqueous solution: 5%-65%) to obtain the target compound N⁶-(tert-butoxycarbonyl)-N²-((39-(2-(methylthio)pyrimidin-5-yl)-5,34-dioxo-3,9,12,15,18,21,24,27,30-nonaoxa-6,33-diazanonatriacont-38-ynoyl)-L-valinyl)-L-lysine (C1.14) (170 mg).

LCMS (ESI) [M+H]⁺: 1074.0;
¹H NMR (400 MHz, DMSO-d6) δ 8.68 (s, 2H), 8.23 (t, *J =* 5.7 Hz, 1H), 8.13 (d, *J =* 8.9 Hz, 1H), 7.99 (t, *J =* 5.6 Hz, 1H), 7.43 (d*, J* = 6.5 Hz, 1H), 6.69 (s, 1H), 4.09 (dd, *J =* 8.8, 6.5 Hz, 1H), 4.02 (s, 2H), 3.98 (d, *J =* 2.9 Hz, 2H), 3.74 - 3.70 (m, 1H), 3.50 (s, 30H), 3.45 (d, *J =* 6.2 Hz, 2H), 3.39 (d, *J =* 5.8 Hz, 2H), 3.26 (d, *J =* 6.1 Hz, 2H), 3.20 (q*, J* = 5.9 Hz, 2H), 2.81 (t, *J =* 6.6 Hz, 2H), 2.52 (s, 3H), 2.24 (t, *J =* 7.4 Hz, 2H), 2.12 - 2.06 (m, 1H), 1.79 - 1.74 (m, 2H), 1.67 - 1.59 (m, 1H), 1.50 (d, *J =* 5.3 Hz, 1H), 1.36 (s, 9H), 1.24 (s, 2H), 1.20 - 1.09 (m, 2H), 0.85 (t, *J =* 6.3 Hz, 6H).

### Example C1.15: N⁶-(tert-butoxycarbonyl)-N²-((32-(4-(2-(methylthio)pyrimidin-5-yl)-1H-1,2,3-triazol-1- yl)-5-oxo-3,9,12,15,18,21,24,27,30-nonaoxa-6-azadotriacontanoyl)-L-valinyl)-L-lysine (C1.15)

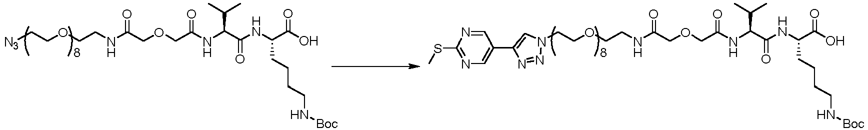

Compound C1.14-B (130 mg), 5-ethynyl-2-methylthiopyrimidine (44 mg), sodium vitamin C (3 mg) and copper sulfate (5 mg) were added to mixed solvent of tert-butanol (2 mL) and water (2 mL), and then the mixture was allowed to react under nitrogen at room temperature for 3 h. Water (10 mL) was added to the reaction solution, and then the resultant solution was extracted with dichloromethane (30 mL×3). The organic phase was washed with saturated brine, dried over anhydrous sodium sulfate, filtered, and concentrated to obtain a crude product, which was separated and purified by TLC (dichloromethane: methanol = 10 to 1) to obtain the target compound N⁶-(tert-butoxycarbonyl)-N²-((32-(4-(2-(methylthio)pyrimidin-5-yl)-1H-1,2,3-triazol-1-yl)- 5-oxo-3,9,12,15,18,21,24,27,30-nonaoxa-6-azadotriacontanoyl)-L-valinyl)-L-lysine (C1.15) (90 mg).

LCMS (ESI) [M+H]⁺ =1032.3.

### Example C1.16: N⁶,N⁶-dimethyl-N²-((6-(2-(methylthio)pyrimidin-5-yl)hexan-5-ynoyl)-L-valinyl)-L-lysine

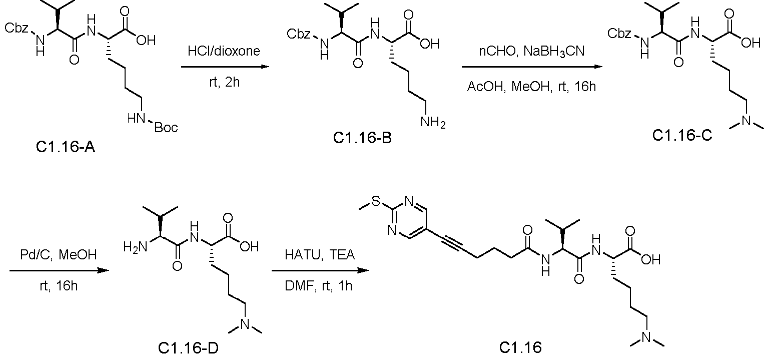

### Step 1:

To compound C1.16-A (10.0 g), was added HCl-dioxane solution (100 mL), and then reacted at room temperature for 2 h. The reaction solution was concentrated under reduced pressure to obtain the target compound C1.16-B (8.0 g) as a white solid.

LCMS (ESI) [M+H]⁺: 380.1.

¹H NMR (400 MHz, DMSO) δ 8.18 (d, *J =* 7.4 Hz, 1H), 7.40 - 7.30 (m, 5H), 7.26 (d, *J =* 8.8 Hz, 1H), 5.08 - 4.99 (m, 2H), 4.23 - 4.11 (m, 1H), 3.94 - 3.88 (m, 1H), 2.78 - 2.73 (m, 2H), 2.03 - 1.94 (m, 1H), 1.77 - 1.51 (m, 4H), 1.44 - 1.31 (m, 2H), 0.87 (dd, *J =* 17.3, 6.6 Hz, 6H).

### Step 2:

Compound C1.16-B (3.0 g) and sodium acetate (1.90 g) were dissolved in methanol (100 mL) and then reacted at room temperature for 10 min. Paraformaldehyde (2.8 g) was added to the reaction solution, and then the reaction was stirred at room temperature for 30 min. Then, sodium cyanoborohydride (1.0 g) was added to the reaction solution, and the reaction was stirred at room temperature for 16 h. After the reaction solution was filtered, the filtrate was separated and purified by reversed-phase chromatography over C18 column (acetonitrile to 0.05% formic acid aqueous solution: 5% - 55%) to obtain the target compound C1.16-C (1.70 g).

LCMS (ESI) [M+H]⁺: 408.1;
¹H NMR (400 MHz, DMSO) δ 7.86 (d, *J =* 7.3 Hz, 1H), 7.40 - 7.26 (m, 6H), 5.03 (s, 2H), 4.08 - 4.06 (m, 1H), 3.90 - 3.85 (m, 1H), 2.50 - 2.45 (m, 2H), 2.35 (s, 6H), 2.05 - 1.93 (m, 1H), 1.73 - 1.55 (m, 2H), 1.51 - 1.40 (m, 2H), 1.33 - 1.22 (m, 2H), 0.85 (dd, *J =* 16.5, 6.8 Hz, 6H).

### Step 3:

At room temperature, compound C1.16-C (1.6 g) was dissolved in methanol (80 mL), to which was then added Pd/C (10%, 0.16 g), and the reaction was stirred at room temperature under hydrogen atmosphere for 12 h. The reaction solution was filtered, and the filtrate was concentrated under reduced pressure to obtain the target compound C1.16-D (680 mg).

LCMS (ESI) [M+H]⁺: 274.2.

### Step 4:

6-(2-(Methylthio)pyrimidin-5-yl)hex-5-ynoic acid (944 mg) was dissolved in N,N-dimethylformamide (30 mL), to which were then added N,N-diisopropylethylamine (1.3 g) and 2-(7-azobenzotriazole)-N,N,N',N'-tetramethylurea hexafluorophosphate (HBTU, 1.8 g) sequentially. The reaction solution was stirred at room temperature for 20 min. Then, compound C1.16-D (1.1 g) was added, and the reaction was stirred at 40 °C for 3 h. The crude product was separated and purified by reversed-phase C18 column (acetonitrile/0.01% formic acid in water: 5%-65%) to obtain the target compound N⁶,N⁶-dimethyl-N²-((6-(2-(methylthio)pyrimidin-5-yl)hexan-5-ynoyl)-L-valinyl)-L-lysine C1.16 (1.2 g) as a white solid.

LCMS (ESI) [M+H]⁺: 492.1;
¹H NMR (400 MHz, DMSO) δ 8.68 (s, 2H), 8.00 (d, *J =* 7.6 Hz, 1H), 7.91 (d, *J =* 9.0 Hz, 1H), 4.20 (t, 1H), 4.08 (dd, *J =* 12.7, 7.7 Hz, 1H), 2.47 - 2.40 (m, 4H), 2.37 - 2.31 (m, 2H), 2.30 (s, 6H), 2.01 - 1.92 (m, 1H), 1.82 - 1.73 (m, 2H), 1.71 - 1.56 (m, 2H), 1.49 - 1.37 (m, 2H), 1.33 - 1.23 (m, 2H), 0.88 - 0.82 (m, 6H).

### Example C1.17: N⁶,N⁶-dimethyl-N²-((6-(2-(methanesulfonyl)pyrimidin-5-yl)hexan-5-ynoyl)-L-valinyl)-L-lysine C1.17

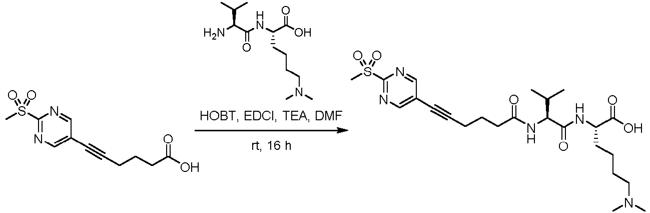

6-(2-(Methanesulfonyl)pyrimidin-5-yl)hex-5-ynoic acid (268 mg), compound C1.16-D (328 mg), and triethylamine (322 mg) were dissolved in N,N-dimethylformamide (5 mL). Then, 1-hydroxybenzotriazole (HOBT, 162 mg) and 1-(3-dimethylaminopropyl)-3-ethylcarbodiimide hydrochloride (EDCI, 229 mg) were added, and the reaction solution was stirred at room temperature for 16 h. The reaction solution was directly purified over reversed-phase C18 column (acetonitrile and 0.05% formic acid aqueous solution system) to obtain the target compound N⁶,N⁶-dimethyl-N²-((6-(2-(methanesulfonyl)pyrimidin-5-yl)hexan-5-ynoyl)-L-valinyl)-L-lysine (C1.17, white solid, 327 mg).

LCMS (ESI) [M+H]⁺: 524.4.

¹H NMR (400 MHz) δ 9.13 (s, 2H), 7.95 (t, *J =* 8.8 Hz, 2H), 4.21 (dd, *J =* 8.8, 6.9 Hz, 1H), 4.08 - 4.03 (m, 1H), 3.41 (s, 3H), 2.55 (t, *J =* 7.0 Hz, 2H), 2.42 - 2.32 (m, 4H), 2.27 (s, 6H), 1.98 (dd, *J =* 13.6, 6.8 Hz, 1H), 1.86 - 1.77 (m, 2H), 1.74 - 1.55 (m, 2H), 1.47 - 1.37 (m, 2H), 1.31 - 1.23 (m, 2H), 0.85 (dd, *J =* 12.8, 6.8 Hz, 6H).

### Example C1.18: N²-(tert-butoxycarbonyl)-N⁶-((6-(2-(methanesulfonyl)pyrimidin-5-yl)hexan-5-ynoyl)- L-valinyl)-L-lysine

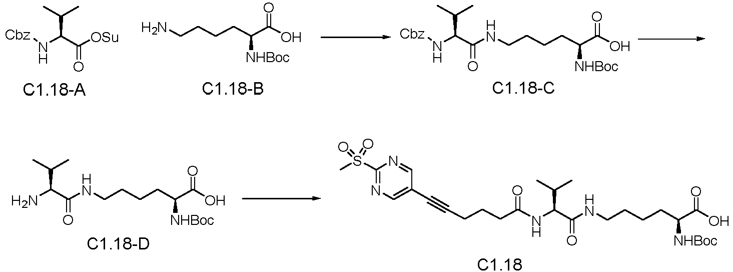

### Step 1:

Compound C1.18-B (3 g) was dissolved in dichloromethane (30 ml), to which was added DIPEA (4 ml), followed by addition of compound C1.18-A (3.48 g). The mixture was allowed to react at room temperature under stirring for 20 h. Ethyl acetate (200 ml) was added to the reaction solution, and the mixture was washed successively with hydrochloric acid (0.1 M, 30 ml x 3) and water (30 ml x 3), dried over anhydrous sodium sulfate, and concentrated under reduced pressure to obtain the target mixture C1.18-C (4.7 g).

### Step 2:

At room temperature, compound C1.18-C (4.7 g) was dissolved in methanol (80 mL), to which was then added Pd/C (10%, 0.16 g), and the reaction was stirred at room temperature under hydrogen atmosphere for 12 h. The reaction solution was filtered, and the filtrate was concentrated under reduced pressure to obtain the target compound C1.18-D (3.4 g). LCMS (ESI) [M+H]⁺: 346.2.

### Step 3:

6-(2-(Methanesulfonyl)pyrimidin-5-yl)hex-5-ynoic acid (2.68 g) was dissolved in N,N-dimethylformamide (50 mL), to which were then added triethylamine (3 ml) and HBTU (3.8 g), and then the mixture was stirred at room temperature for 10 min. Then, ethyl acetate (200 ml) was added. The mixture was successively washed with saturated sodium bicarbonate (20 ml x 2), hydrochloric acid (0.1 M, 50 ml x 2), and water (50 ml x 2), and then dried over anhydrous sodium sulfate, and concentrated under reduced pressure to obtain the crude intermediate. The above crude product was dissolved in DMF (30 ml), to which were successively added DIPEA (1.6 ml) and C1.18-D (3.4 g). The reaction solution was stirred at room temperature for 3 h, and then ethyl acetate (200 ml) was added. The resultant solution was successively washed with hydrochloric acid (0.1 M, 50 ml x 2) and water (50 ml x 2), dried over anhydrous sodium sulfate, and concentrated under reduced pressure to give crude product, which was separated and purified over silica gel column chromatography to obtain the target compound N²-(tert-butoxycarbonyl)-N⁶-((6-(2-(methanesulfonyl)pyrimidin-5-yl)hexan-5-ynoyl)- L-valinyl)-L-lysine (C1.18, white solid, 3.8 g).

LCMS (ESI) [M+H]⁺: 596.4.

### Example C1.19: N⁶,N⁶-dimethyl-N²-((6-(4-(2-(methanesulfonyl)pyrimidin-5-yl)-1H-1,2,3-triazol-1-yl)hexanoyl)-L-valinyl)-L-lysine

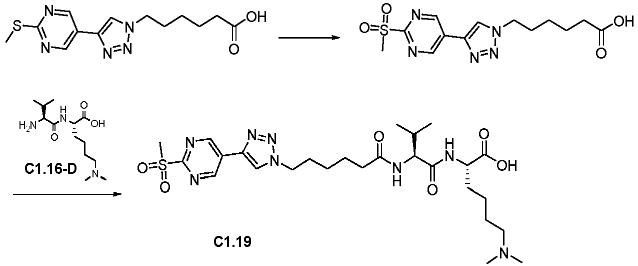

### Step 1:

Compound 6-(4-(2-(methylthio)pyrimidin-5-yl)-1H-1,2,3-triazol-1-yl)hexanoic acid (1.0 g, 3.3mmol) was dissolved in mixed solvent of tetrahydrofuran and water (40 mL, 3:1), to which was added potassium peroxymonosulfonate (10.0 g, 16.3mmol). The reaction was stirred for 3 h at room temperature, and was completed as shown by LCMS detection. The reaction solution was filtered. The cake was washed with DMSO, and the filtrate was combined and subjected to reversed phase purification (C18, acetonitrile: 0.1% formic acid = 5%-55%), to afford 6-(4-(2-(methanesulfonyl)pyrimidin-5-yl)-1H-1,2,3-triazol-1-yl)hexanoic acid (750 mg, yield 67.9%) as white solid.

LCMS (ESI) [M+H]⁺ = 340.1;
¹H NMR (400 MHz, DMSO-d6) δ 9.48 (s, 2H), 8.95 (s, 1H), 4.49 (t, *J =* 7.0 Hz, 2H), 3.45 (s, 3H), 2.22 (t, *J =* 7.3 Hz, 2H), 1.95 - 1.84 (m, 2H), 1.61 - 1.50 (m, 2H), 1.36 - 1.26 (m, 2H).

### Step 2:

Compound 6-(4-(2-(methanesulfonyl)pyrimidin-5-yl)-1H-1,2,3-triazol-1-yl)hexanoic acid (300 mg, 0.88 mmol) was dissolved in N,N-dimethylformamide (6 mL), to which were added HATU (337 mg, 0.88mmol) and DIPEA (286 mg, 2.21 mmol), and the reaction was stirred at room temperature for 30 min. Then, dipeptide C1.16-D (243 mg, 0.88 mmol) was added, and the reaction was stirred at room temperature for 2 h. LCMS indicated completion of the reaction, and the reaction solution was separated and purified over C18 column (acetonitrile/0.01% FA aqueous solution: 5%-50%) to obtain the target compound N⁶,N⁶-dimethyl-N²-((6-(4-(2-(methanesulfonyl)pyrimidin-5-yl)-1H-1,2,3-triazol-1-yl)hexanoyl)-L-valinyl)- L-lysine (300 mg, yield 57%) as white solid.

LCMS (ESI) [M+H]⁺ = 595.5, t_{R} = 2.024 min.

¹H NMR (400 MHz, DMSO-d6) δ 9.49 (s, 2H), 9.01 (s, 1H), 7.85 (br s, 1H), 7.84 (d, *J =* 8.9 Hz, 1H), 4.48 (t, *J* = 6.8 Hz, 2H), 4.17 - 4.15 (m, 1H), 4.02 (br s, 1H), 3.44 (s, 3H), 2.42 (br s, 2H), 2.30 (s, 6H), 2.18 - 2.14 (m, 2H), 1.99 - 1.96 (m, 1H), 1.88 (dd, *J =* 14.6, 7.1 Hz, 2H), 1.67 (br s, 1H), 1.59 - 1.53 (m, 2H), 1.43 (br s, 2H), 1.28 - 1.26 (m, 5H), 0.83 - 0.89 (m, 6H).

### Example C1.20: N⁶,N⁶-diethyl-N²-((6-(2-(methanesulfonyl)pyrimidin-5-yl)hexan-5-ynoyl)-L-valinyl)-L-lysine

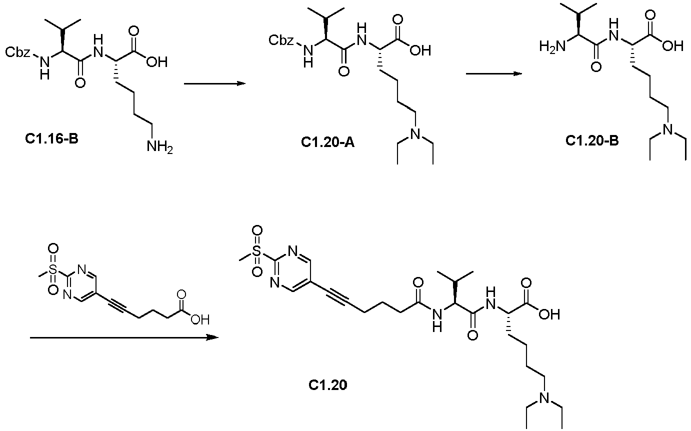

### Step 1:

Compound C1.16-B (5.0 g, 12.05 mmol) was dissolved in dichloromethane (100 mL), to which was added acetaldehyde (3.2 g, 72.3 mmol), and the reaction was stirred for 10 min at room temperature. Then, sodium triacetoxyborohydride (12.8 g, 60.25 mmol) was added to the reaction solution, and the reaction was stirred for 1 h at room temperature. LCMS showed completion of the reaction. Saturated aqueous solution of ammonium chloride was added to the reaction solution, and then stirred for 1 h. The reaction solution was rotatory evaporated to dryness. The crude product was separated and purified over reversed-phase C18 column (acetonitrile to 0.05% formic acid aqueous solution: 5% to 55%) to obtain the target compound C1.20-A (4.57 g, yield 82.0%) as white solid.

LCMS (ESI) [M+H]⁺= 436.4;
¹H NMR (400 MHz, DMSO-d₆) δ 7.70 (d, *J =* 7.0 Hz, 1H), 7.41 (d, *J =* 9.0 Hz, 1H), 7.38 - 7.26 (m, 5H), 5.08 - 4.99 (m, 2H), 4.00 (dd, *J =* 12.6, 6.5 Hz, 1H), 3.86 (dd, *J =* 8.6, 6.8 Hz, 1H), 2.74 (dd, *J =* 14.0, 6.9 Hz, 4H), 2.64 - 2.54 (m, 2H), 2.05 - 1.94 (m, 1H), 1.72 - 1.52 (m, 2H), 1.52 - 1.38 (m, 2H), 1.38 - 1.18 (m, 2H), 1.04 (t, *J* = 7.1 Hz, 6H), 0.87 - 0.81 (m, 6H).

### Step 2:

At room temperature, compound C1.20-A(1.6 g, 3.68 mmol) was dissolved in methanol (80 mL), to which was then added Pd/C (0.16 g), and the reaction was stirred at room temperature under hydrogen atmosphere for 12 h. LCMS indicated completion of the reaction. The reaction solution was filtered, and the filtrate was concentrated under reduced pressure to obtain the target compound C1.20-B (900 mg, yield 82%) as off-white solid.

¹H NMR (400 MHz, DMSO-d6) δ 8.04 (br s, 1H), 4.02 - 3.99 (m, 1H), 3.10 (d, *J =* 4.5 Hz, 1H), 2.65 (q, *J =* 7.1 Hz, 4H), 2.55 - 2.51 (m, 2H), 2.06 - 1.93 (m, 1H), 1.73 - 1.54 (m, 2H), 1.47 - 1.38 (m, 2H), 1.30 - 1.21 (m, 2H), 1.01 (t*, J =* 7.1 Hz, 6H), 0.89 (d, *J =* 6.9 Hz, 3H), 0.79 (d, *J =* 6.8 Hz, 3H).

### Step 3:

Compound 6-(2-(methanesulfonyl)pyrimidin-5-yl)hex-5-ynoic acid (268 mg, 1 mmol) was dissolved in DMF (8 mL), to which were successively added HATU (380 mg, 1 mmol) and triethylamine (322 mg, 2.5 mmol), and then stirred at room temperature for 20 min, followed by addition of compound C1.20-B (301 mg, 1 mmol). The reaction solution was further stirred for 30 min at room temperature. After completion of the reaction by LCMS, the reaction solution was directly purified over reversed phase C18 column (acetonitrile and 0.05% formic acid aqueous solution system) to obtain the target compound (280 mg, yield 51%) as a white solid.

LCMS (ESI) [M+H]⁺ = 552.3;
¹H NMR (400 MHz, DMSO-d6) δ 9.13 (s, 2H), 7.95 (d, *J =* 8.9 Hz, 1H), 7.86 (d, *J =* 7.2 Hz, 1H), 4.18 (dd, *J* = 8.8, 6.8 Hz, 1H), 4.02 (dd, *J =* 12.8, 7.2 Hz, 1H), 3.41 (s, 3H), 2.74 - 2.69 (m, 4H), 2.62 - 2.52 (m, 4H), 2.44 - 2.29 (m, 2H), 2.04 - 1.94 (m, 1H), 1.86 - 1.77 (m, 2H), 1.72 - 1.54 (m, 2H), 1.51 - 1.39 (m, 2H), 1.33 - 1.23 (m, 2H), 1.02 (t, *J=* 7.2 Hz, 6H), 0.87 - 0.82 (m, 6H).

### Example C1.21: N⁶,N⁶-diethyl-N²-((6-(4-(2-(methanesulfonyl)pyrimidin-5-yl)-1H-1,2,3-triazol-1-yl)hexanoyl)-L-valinyl)-L-lysine

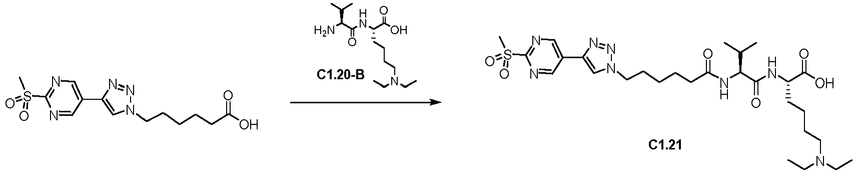

Compound 6-(4-(2-(methanesulfonyl)pyrimidin-5-yl)-1H-1,2,3-triazol-1-yl)hexanoic acid (500 mg, 1.475 mmol) was dissolved in N,N-dimethylformamide (10 mL), to which were then added HATU (560 mg, 1.475 mmol) and N,N-diisopropylethylamine (476 mg, 3.688 mmol), and then stirred at room temperature for 30 min, followed by addition of compound C1.20-B (444 mg, 1.475 mmol). The reaction solution was stirred for 1 h at room temperature. After completion by detecting with LCMS, the reaction solution was directly purified over reversed phase C18 column (acetonitrile and 0.05% formic acid aqueous solution system) to obtain the target compound N⁶,N⁶-diethyl-N²-((6-(4-(2-(methanesulfonyl)pyrimidin-5-yl)-1H-1,2,3-triazol-1-yl)hexanoyl)- L-valinyl)-L-lysine (330 mg, yield 34%) as a white solid.

LCMS (ESI) [M+H]⁺ = 623.4;
¹H NMR (400 MHz, DMSO) δ 9.49 (s, 2H), 9.04 (s, 1H), 7.84 (d, *J =* 8.9 Hz, 1H), 7.80 (d, *J =* 7.1 Hz, 1H), 4.47 (t, *J =* 7.0 Hz, 2H), 4.14 (dd, *J =* 8.8, 6.6 Hz, 1H), 4.06 - 3.95 (m, 1H), 3.44 (s, 3H), 2.67 - 2.64 (m, 4H), 2.55 (t, *J* = 7.4 Hz, 2H), 2.21 - 2.10 (m, 2H), 2.02 - 1.94 (m, 1H), 1.92 - 1.84 (m, 2H), 1.72 - 1.63 (m, 1H), 1.72 - 1.63 (m, 3H), 1.59 - 1.54 (m, 2H), 1.32 - 1.21 (m, 4H), 1.01 (t, *J =* 7.1 Hz, 6H), 0.81 (dd, *J =* 9.6, 6.8 Hz, 6H).

### Example C1.22: N⁶,N⁶-dipropyl-N²-((6-(2-(methanesulfonyl)pyrimidin-5-yl)hexan-5-ynoyl)-L-valinyl)-L-lysine

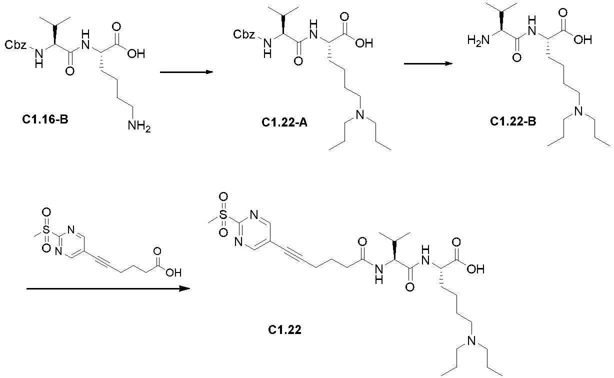

### Step 1:

Compound C1.16-B (5.0 g, 12 mmol) was dissolved in dichloromethane (100 mL), to which was added n-propionaldehyde (4.2 g, 72.3 mmol), and then the reaction was stirred at room temperature for 10 min, followed by addition of sodium triacetoxyborohydride (12.8 g, 60.25 mmol). The reaction was stirred at room temperature for 1 h. LCMS showed the reaction was completed. To the reaction solution, was added saturated aqueous solution of ammonium chloride, and then the solution was stirred for 1 h and rotatory evaporated to dryness. After filtration, the filtrate was separated and purified over reversed phase C18 column (acetonitrile to 0.05% formic acid aqueous solution: 5% to 55%), to obtain the target compound C1.22-A (4.57 g, yield 82.0%) as a white solid.

LCMS (ESI) [M+H]⁺ = 464.0;
¹H NMR (400 MHz, DMSO-d6) δ 7.81 (d, *J =* 7.3 Hz, 1H), 7.38 - 7.28 (m, 5H), 5.04 (d, *J =* 1.7 Hz, 2H), 4.12 - 4.02 (m, 1H), 3.94 - 3.82 (m, 1H), 2.65 - 2.52 (m, 6H), 2.06 - 1.94 (m, 1H), 1.76 - 1.64 (m, 1H), 1.64 - 1.53 (m, 1H), 1.52 - 1.40 (m, 6H), 1.34 - 1.18 (m, 2H), 0.92 - 0.80 (m, 12H).

### Step 2:

At room temperature, compound C1.22-A (2.0 g, 4.32 mmol) was dissolved in methanol (80 mL), to which was then added Pd/C (0.16 g), and the reaction was stirred at room temperature under hydrogen atmosphere for 12 h. LCMS indicated completion of the reaction. The reaction solution was filtered, and the filtrate was concentrated under reduced pressure to obtain the target compound C1.22-B (1.2 g, yield 85.5%) as a white solid.

### Step 3:

Compound 6-(2-(methanesulfonyl)pyrimidin-5-yl)hex-5-ynoic acid (100 mg, 0.373 mmol) was dissolved in N,N-dimethylformamide (1 mL), to which were then added HATU (142 mg, 0.373 mmol) and N,N-diisopropylethylamine (120 mg, 0.93 mmol), and the reaction was stirred for 30 min. Then, compound C1.22-B (122 mg, 0.371 mmol) was added, and the reaction solution was stirred at room temperature for 1 h. After LCMS indicated that the reaction was completed, the reaction solution was directly purified over a reversed-phase C18 column (acetonitrile and 0.05% formic acid aqueous solution), to obtain the target compound N⁶,N⁶-dipropyl-N²-((6-(2-(methanesulfonyl)pyrimidin-5-yl)hexan-5-ynoyl)-L-valinyl)-L-lysine(50 mg, yield 28%) as a pale yellow solid.

LCMS (ESI) [M+H]⁺ = 580.0;
¹H NMR (400 MHz, DMSO-d₆) δ 8.24 (s, 2H), 7.98 - 7.93 (m, 2H), 4.24 - 4.16 (m, 1H), 4.10 (d, *J =* 5.2 Hz, 1H), 3.41 (s, 3H), 2.79 - 2.64 (m, 6H), 2.55 (t, *J =* 7.1 Hz, 2H), 2.45 - 2.26 (m, 2H), 2.06 - 1.91 (m, 1H), 1.89 - 1.78 (m, 2H), 1.76 - 1.66 (m, 1H), 1.64 - 1.57 (m, 1H), 1.57 - 1.42 (m, 6H), 1.37 - 1.24 (m, 2H), 0.93 - 0.78 (m, 12H).

### Example C1.23: N⁶,N⁶-dipropyl-N²-((6-(4-(2-(methanesulfonyl)pyrimidin-5-yl)-1H-1,2,3-triazol-1-yl)hexanoyl)-L-valinyl)-L-lysine

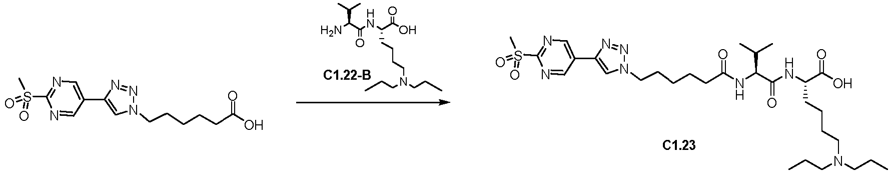

Compound 6-(4-(2-(methanesulfonyl)pyrimidin-5-yl)-1H-1,2,3-triazol-1-yl)hexanoic acid (500 mg, 1.475 mmol) was dissolved in N,N-dimethylformamide (10 mL), to which were then added HATU (560 mg, 1.475 mmol) and N,N-diisopropylethylamine (476 mg, 3.688 mmol), and the reaction was stirred for 30 min, followed by addition of compound C1.22-B (485 mg, 1.475 mmol). The reaction solution was stirred at room temperature for 1 h. After the reaction was completed as shown by LCMS, the reaction solution was directly purified over a reversed-phase C18 column (acetonitrile and 0.05% formic acid aqueous solution), to obtain the target compound N⁶,N⁶-dipropyl-N²-((6-(4-(2-(methanesulfonyl)pyrimidin-5-yl)-1H-1,2,3-triazol-1-yl)hexanoyl)-L-valinyl)- L-lysine (320 mg, yield 33%) as a white solid.

LCMS (ESI) [M+H]⁺ = 651.5;
¹H NMR (400 MHz, DMSO) δ 9.49 (s, 2H), 9.00 (s, 1H), 7.95 (d, *J =* 7.2 Hz, 1H), 7.79 (d, *J =* 8.9 Hz, 1H), 4.47 (t*, J* = 6.9 Hz, 2H), 4.21 - 4.11 (m, 1H), 4.09 - 4.01 (m, 1H), 3.44 (s, 3H), 2.45 - 2.35 (m, 6H), 2.23 - 2.09 (m, 2H), 1.97 - 1.86 (m, 3H), 1.72 - 1.64 (m, 1H), 1.61 - 1.50 (m, 3H), 1.43 - 1.34 (m, 6H), 1.32 - 1.22 (m, 4H), 0.86 - 0.77 (m, 12H).

### Example C1.24: tert-butyl (S)-(6-((4-(hydroxymethyl)phenyl)amino)-5-(6-(2-(methylthio)pyrimidin-5-yl)hex-5-ynamido)-6-oxohexyl)carbamate

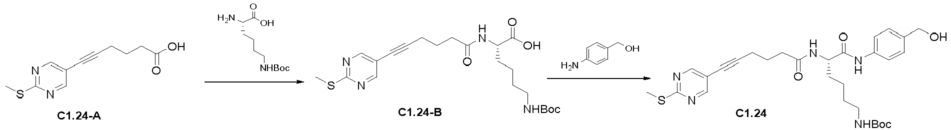

### Step 1:

Compound C1.24-A (3.70 g, 15.7 mmol), diisopropylethylamine (11.0 mL, 62.8 mmol) and HBTU (8.90 g, 23.6 mmol) were dissolved in N,N-dimethylformamide (30 mL), and then the mixture was allowed to react at room temperature for 30 min. Subsequently, the compound Boc-protected lysine (3.86 g, 15.7 mmol) was added, and the mixture was further reacted at room temperature for 2 h. The reaction was completed by LCMS detection. The aqueous citric acid solution (30 mL) was added to the reaction solution, and the pH value was adjusted to 5. The aqueous solution was extracted with dichloromethane (50 mL X 3). The organic phase was combined, dried over anhydrous sodium sulfate, filtered. The filtrate was concentrated under reduced pressure to provide the target compound C1.24-B (7.0g, crude product) as yellow oil. LCMS (ESI) [M+H]⁺ = 465.1, t_{R} = 1.751 min.

### Step 2:

Compound C1.24-B (7.00 g, 5.60 mmol), diisopropylethylamine (10.7 mL, 60.4 mmol) and HBTU (8.60 g, 22.7 mmol) were dissolved in N,N-dimethylformamide (100 mL), to which was then added p-aminobenzyl alcohol (3.71 g, 30.2 mmol), and the mixture was allowed to react at room temperature for 2 h. The reaction was completed as shown by detecting with LCMS. Ethyl acetate (300 mL) was added to the reaction solution. The resultant solution was washed with saturated brine (100 mL×3), dried over anhydrous sodium sulfate, filtered. The filtrate was concentrated under reduced pressure to a solid, and the crude product was separated and purified by column chromatography (dichloromethane: methanol = 10/1) to obtain the target compound tert-butyl (S)-(6-((4-(hydroxymethyl)phenyl)amino)-5-(6-(2-(methylthio)pyrimidin-5-yl)hex-5-ynamido)-6-oxohexyl) carbamate (6.00 g, yield 69.9%) as yellow oil.

LCMS (ESI) [M+H]⁺ = 570.1;
¹H NMR (400 MHz, CDCl₃) δ 9.24 (s, 1H), 8.46 (s, 2H), 7.51 - 7.47 (m, 2H), 7.31 - 7.23 (m, 3H), 7.02 - 6.95 (m, 1H), 4.83 (br s, 1H), 4.63 - 4.60 (m, 2H), 3.19 - 2.96 (m, 4H), 2.57 (s, 3H), 2.52 - 2.48 (m, 2H), 2.47 - 2.41 (m, 2H), 1.99 - 1.92 (m, 2H), 1.87 - 1.77 (m, 2H), 1.76 - 1.63 (m, 2H), 1.43 (s, 9H).

### Example C1.25: tert-butyl (S)-(6-((4-(hydroxymethyl)phenyl)amino)-5-(6-(2-(methylthio)pyrimidin-5-yl)-1H-1,2,3-triazol-1-yl)hexanamido)-6-oxohexyl)carbamate

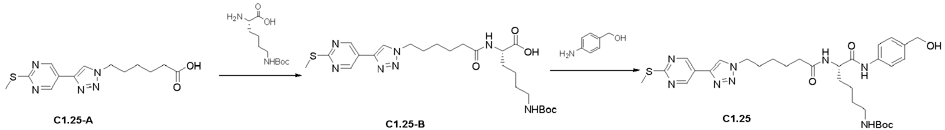

### Step 1:

Compound C1.25-A (1.60 g, 5.21 mmol), diisopropylethylamine (2.68 mL, 20.8 mmol) and HBTU (2.97 g, 7.82 mmol) were dissolved in N,N-dimethylformamide (30 mL), and then the mixture was allowed to react at room temperature for 30 min. Subsequently, the compound Boc-protected lysine (1.28 g, 5.21 mmol) was added, and the mixture was further reacted at room temperature for 2 h. The reaction was completed as shown by LCMS. The aqueous citric acid solution (30 mL) was added to the reaction solution, and the pH value was adjusted to be 5. The aqueous solution was extracted with dichloromethane (50 mL X 3). The organic phase was combined, dried over anhydrous sodium sulfate, filtered. The filtrate was concentrated under reduced pressure to provide the target compound C1.25-B (3.0 g, crude product) as yellow oil.

LCMS (ESI) [M+H]⁺ = 536.0;

### Step 2:

Compound C1.25-B (3.00 g, 5.60 mmol), diisopropylethylamine (2.89 g, 22.4 mmol) and HBTU(3.19 g, 8.40 mmol) were dissolved in N,N-dimethylformamide (30 mL), to which was then added p-aminobenzyl alcohol (1.38 g, 11.2 mmol), and the mixture was allowed to react at room temperature for 2 h. The reaction was completed as shown by detecting with LCMS. Ethyl acetate (200 mL) was added to the reaction solution. The resultant solution was washed with saturated brine (80 mL×3), dried over anhydrous sodium sulfate, filtered. The filtrate was concentrated under reduced pressure to a solid, and the crude product was separated and purified by column chromatography (dichloromethane: methanol = 10/1) to obtain the target compound

### tert-butyl (S)-(6-((4-(hydroxymethyl)phenyl)amino)-5-(6-(2-(methylthio)pyrimidin-5-yl)-1H- 1,2,3-triazol-1-yl)hexanamido)-6-oxohexyl)carbamate

(2.90 g) as yellow oil.

LCMS (ESI) [M+H]⁺ = 641.1;
¹H NMR (400 MHz, CDCl₃) δ 9.13 (s, 1H), 8.95 (s, 2H), 7.94 (s, 1H), 7.47 (d*, J =* 8.4 Hz, 2H), 7.24 (d, *J* = 8.4 Hz, 2H), 6.83 - 6.77 (m, 1H), 4.93 - 4.80 (m, 1H), 4.64 - 4.58 (m, 2H), 4.40 - 4.29 (m, 2H), 3.12 - 2.97 (m, 4H), 2.60 (s, 3H), 2.31 - 2.23 (m, 2H), 1.96 - 1.83 (m, 4H), 1.76 - 1.61 (m, 4H), 1.41 (s, 9H), 1.37 - 1.29 (m, 4H).

### Example C1.26: N⁶,N⁶-dibutyl-N²-((6-(2-(methanesulfonyl)pyrimidin-5-yl)hexan-5-ynoyl)-L-valinyl)-L-lysine

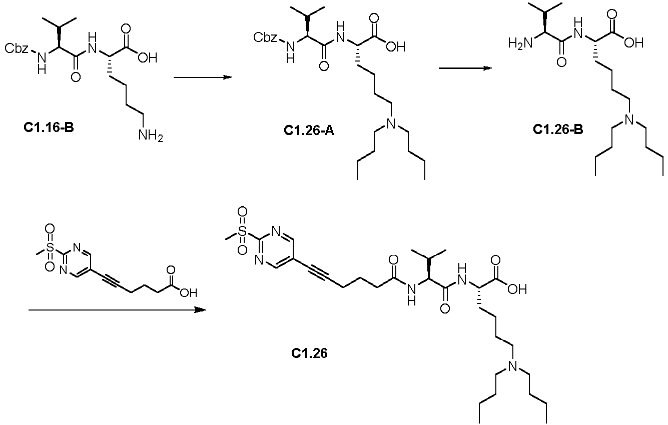

### Step 1:

Compound C1.16-B (10.0 g) was dissolved in dichloromethane (200 mL), to which was added n-butyraldehyde (10.4 g), and then the reaction was stirred at room temperature for 10 min, followed by addition of sodium triacetoxyborohydride (25.6 g) in batches. The reaction was stirred at room temperature for 1 h. LCMS showed the reaction was completed. To the reaction solution, was added saturated aqueous solution of ammonium chloride, and then the solution was stirred for 1 h and rotatory evaporated to dryness. After filtration, the filtrate was separated and purified over reversed phase C18 column (acetonitrile to 0.05% formic acid aqueous solution: 5% to 55%), to obtain the target compound C1.26-A (4.9 g) as a white solid.

LCMS (ESI) [M+H]⁺ = 492.7;
¹H NMR (400 MHz, CDCl₃) δ 7.35 - 7.30 (m, 5H), 5.14 - 5.08 (m, 2H), 4.33 - 4.30 (m, 1H), 4.14 - 4.12 (m, 1H), 2.94 - 2.80 (m, 6H), 2.13 - 2.11 (m, 1H), 1.85 - 1.82 (m, 2H), 1.58 - 1.55 (m, 4H), 1.40 - 1.22 (m, 8H), 0.98 - 0.87 (m, 12H).

### Step 2:

At room temperature, compound C1.26-A (5.0 g) was dissolved in methanol (100 mL), to which was then added Pd/C (10%, 1 g), and the reaction was stirred at room temperature under hydrogen atmosphere for 12 h. LCMS indicated completion of the reaction. The reaction solution was filtered, and the filtrate was concentrated under reduced pressure to obtain the target compound C1.26-B (3.68 g) as a white solid.

LCMS (ESI) [M+H]⁺ = 358.3.

### Step 3:

Compound 6-(2-(methanesulfonyl)pyrimidin-5-yl)hex-5-ynoic acid (1.0 g) was dissolved in N,N-dimethylformamide (15 mL), to which were then added N,N-diisopropylethylamine (1.2 g) and HATU (1.4 g), and the reaction was stirred for 30 min. Then, compound C1.26-B (1.3 g) was added, and the reaction solution was stirred at room temperature for 1 h. After the reaction was completed as shown by LCMS, the reaction solution was directly purified over preparative HPLC (0.01% trifluoroacetic acid aqueous solution, acetonitrile), to obtain the target compound N⁶,N⁶-dibutyl-N²-((6-(2-(methanesulfonyl)pyrimidin-5-yl)hexan-5-ynoyl)-L-valinyl)-L-lysine (C1.26, 500 mg) as a yellow solid.

LCMS (ESI) [M+H]⁺ = 608.7;
1H NMR (400 MHz, DMSO) δ 9.37 (s, 1H), 9.13 (s, 2H), 8.19 (d, *J =* 7.3 Hz, 1H), 7.92 (d, *J =* 8.8 Hz, 1H), 4.25 - 4.20 (m, 1H), 4.20 - 4.13 (m, 1H), 3.42 (s, 3H), 3.06 - 2.99 (m, 6H), 2.57 - 2.53 (m, 2H), 2.41 - 2.30 (m, 2H), 1.99 - 1.95 (m, 2H), 1.87 - 1.79 (m, 2H), 1.78 - 1.71 (m, 1H), 1.62 - 1.56 (m, 4H), 1.34 - 1.32 (m, 8H), 0.94 - 0.85 (m, 12H).

### Example C1.27: (S)-1-ethyl-4-(3-methyl-2-(6-(2-(methanesulfonyl)pyrimidin-5-yl)hex-5-ynamido)butanamido) piperidin-4-carboxylic acid

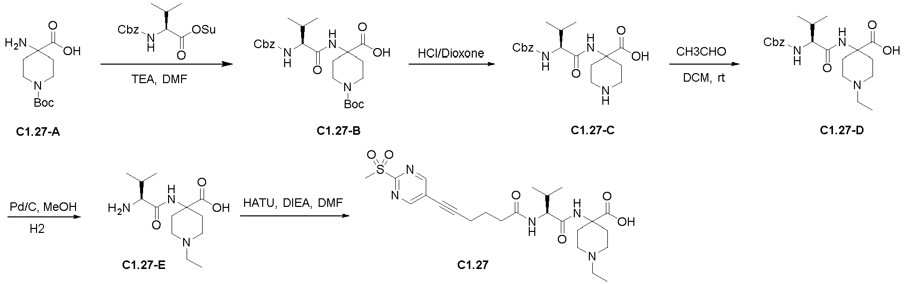

### Step 1:

Compound C1.27-A (5.0 g, 20.49 mmol) was dissolved in DMF (100 mL), to which was then added N-((benzyloxy)carbonyl)-L-valinyl 2,5-dioxopyrrolidine-N-hydroxy ester (7.13 g, 20.49 mmol), and then the reaction was stirred at 100 °C for 48 h. LCMS indicated completion of the reaction. The reaction solution was separated and purified over reversed phase C18 column (acetonitrile to 0.05% aqueous formic acid solution: 5% to 55%) to give the target compound C1.27-B (1.3g, yield 13.3%) as yellow solid.

LCMS (ESI) [M-boc]⁺ = 378.2;
¹H NMR (400 MHz, DMSO) δ 7.38 - 7.33 (m, 5H), 7.32 - 7.27 (m, 1H), 7.20 (d, *J =* 9.0 Hz, 1H), 5.14 - 5.02 (m, 2H), 3.97 - 3.87 (m, 1H), 3.70 - 3.59 (m, 2H), 3.08 - 2.95 (m, 2H), 2.04 - 1.97 (m, 1H), 1.95 - 1.87 (m, 2H), 1.76 - 1.69 (m, 2H), 1.40 (s, 9H), 0.91 - 0.84 (m, 6H).

### Step 2:

At room temperature, compound C1.27-B (1.3 g, 2.71 mmol) was dissolved in ethyl acetate (10 mL), to which was then added dioxane-HCl (3N, 10 mL), and the reaction was stirred at room temperature for 1 h. LCMS showed the reaction was completed. The reaction solution was concentrated under reduced pressure to obtain the target compound C1.27-C (1.06 g, yield 94.4%) as a yellow solid.

LCMS (ESI) [M+H]⁺ = 378.4.

### Step 3:

Compound C1.27-C (1.06 g, 2.81 mmol) was dissolved in dichloromethane (10 mL), to which was added acetaldehyde (0.75 g, 16.87 mmol), and then the reaction was stirred at room temperature for 10 min, followed by addition of sodium triacetoxyborohydride (3 g, 14.05 mmol) in batches. The reaction was stirred at room temperature for 1 h. LCMS showed the reaction was completed. To the reaction solution, was added saturated aqueous solution of ammonium chloride, and then the solution was stirred for 1 h and evaporated under reduced pressure to remove dichloromethane. The residual aqueous phase was separated over reversed phase column chromatography (acetonitrile to 0.05% aqueous formic acid solution: 5% to 55%) to give the target compound C1.27-D (1.13 g, yield 99.23%) as a white solid.

LCMS (ESI) [M+H]⁺ = 406.1;
1H NMR (400 MHz, MeOD) δ 7.42 - 7.25 (m, 5H), 5.22 - 5.06 (m, 2H), 3.91 (d, *J =* 7.0 Hz, 1H), 3.52 - 3.36 (m, 2H), 3.25 - 2.93 (m, 4H), 2.66 - 2.54 (m, 1H), 2.47 - 2.36 (m, 1H), 2.27 - 2.14 (m, 2H), 2.13 - 2.06 (m, 1H), 1.35 - 1.29 (m, 4H), 0.98 - 0.90 (m, 6H).

### Step 4:

At room temperature, compound C1.27-D (1.13 g, 2.78 mmol) was dissolved in methanol (20 mL), to which was then added Pd/C (1.5 g), and the reaction was stirred at room temperature under hydrogen atmosphere for 12 h. LCMS indicated completion of the reaction. The reaction solution was filtered, and the filtrate was concentrated under reduced pressure to obtain the target compound C1.27-E (0.58 g, yield 76.18%) as a white solid. LCMS (ESI) [M+H]⁺ = 272.4.

### Step 5:

Compound 6-(2-(methanesulfonyl)pyrimidin-5-yl)hex-5-ynoic acid (1 g, 3.73 mmol) was dissolved in N, N-dimethylformamide (5 mL), to which were added N,N-diisopropylethylamine (1.2 g, 9.32 mmol) and HATU (1.42 g, 3.73 mmol) successively, and the reacion solution was stirred for 30 min, followed by addition of compound C1.27-E (1.01 g, 3.73 mmol). The reaction solution was stirred at room temperature for 1 h. After the reaction was completed as shown by detecting with LCMS, the reaction solution was directly purified over preparative HPLC (0.01% trifluoroacetic acid aqueous solution, acetonitrile), to obtain the target compound (S)-1-ethyl-4-(3-methyl-2-(6-(2-(methanesulfonyl)pyrimidin-5-yl)hex-5-ynamido)butanamido)piperidin-4- carboxylic acid (C1.27, 500 mg, yield 28.7%) as a yellow solid.

LCMS (ESI) [M+H]⁺ = 522.5;
¹H NMR (400 MHz, DMSO) δ 9.12 (s, 2H), 8.08 (s, 1H), 7.88 (d, *J =* 9.0 Hz, 1H), 4.28 - 4.25 (m, 1H), 3.41 (s, 3H), 2.89 - 2.78 (m, 2H), 2.57 - 2.52 (m, 4H), 2.45 - 2.30 (m, 4H), 2.07 - 2.01 (m, 2H), 1.99 - 1.96 (m, 1H), 1.95 - 1.88 (m, 2H), 1.85 - 1.79 (m, 2H), 1.04 (t, *J =* 7.1 Hz, 3H), 0.88 - 0.83 (m, 6H).

### II. Synthesis of drug-linker compound

### Example 2.1: N-((S)-1-(((S)-6-amino-1-((2-((((S)-7-ethyl-7-hydroxy-8,11-dioxo-7,8,11,13-tetrahydro-10H-[1,3] dioxolo[4,5-g]pyrano[3',4':6,7]indolizino[1,2-b]quinolin-14-yl)methyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (DL-001)

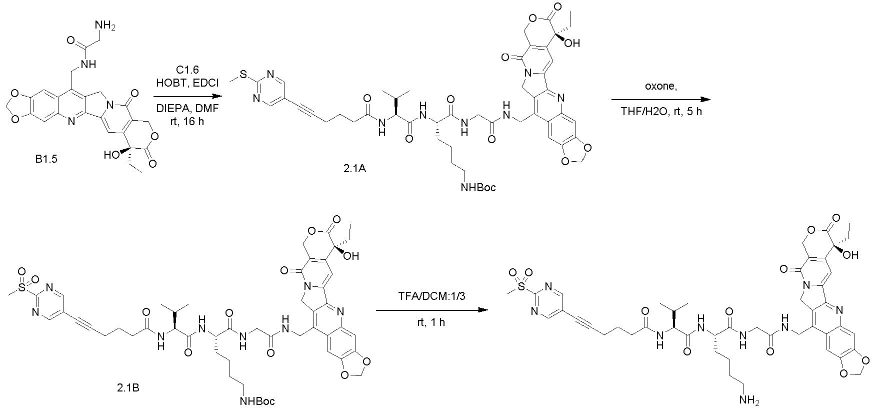

### Step 1:

Compound C1.6 (50 mg, 0.10 mol), compound B1.5 (71 mg, 0.12 mol), and N,N-diisopropylethylamine (39 mg, 0.30 mol) were dissolved in DMF (5 mL), to which were then added 1-hydroxybenzotriazole (16 mg, 0.12 mmol) and 1-(3-dimethylaminopropyl)-3-ethylcarbodiimide hydrochloride (23 mg, 0.12 mmol), and the reaction solution was stirred at room temperature for 16 h, and diluted with 50 mL of ethyl acetate. The organic phase was washed with water for three times (25 mL x 3), dried over anhydrous sodium sulfate, and then concentrated to removed ethyl acetate to obtain crude product, which was separated and purified by flash silica gel column chromatography (DCM:MeOH = 100:0 to 90:10) to obtain the target compound 2.1A (62 mg, yellow solid, yield 58%).

LCMS (ESI) [M+H]⁺: 1024.8.

### Step 2:

Compound 2.1A (40 mg, 0.04 mmol) was dissolved in mixed solution of tetrahydrofuran and water (volume ratio 1/1; 4 mL), to which was added potassium peroxymonosulfonate (246 mg, 0.4 mmol), and the reaction solution was stirred at room temperature for 5 h, and diluted with 30 mL of ethyl acetate. The organic phase was washed three times with water (25 mL x 3), dried over anhydrous sodium sulfate, and concentrated to remove ethyl acetate and obtain the target compound 2.1B (30 mg, yellow solid, yield 74%).

LCMS (ESI) [M+H]⁺ = 1055.9.

### Step 3:

Compound 2.1B (30 mg, 0.03 mmol) was dissolved in a mixed solution of trifluoroacetic acid and dichloromethane (volume ratio 1:3; 4 mL), and then the reaction solution was stirred at room temperature for 1 h. Then, the reaction solution was concentrated under reduced pressure to provide the crude product, which was purified by preparative chromatography (0.01% TFA in water, MeCN) to obtain the target compound N-((S)-1-(((S)-6-amino-1-((2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)amino)-1-oxohexan-2-yl)amino)-3 - methyl-1-oxobutan-2-yl)-6-(2-(methanesulfonyl)pyrimidin-5-yl)hex-5-ynamide (9 mg, white solid, yield 33%).

LCMS (ESI) [M+H]⁺: 955.8;
¹H NMR (400 MHz, DMSO-d6) δ 9.09 (s, 2H), 8.54 (s, 1H), 8.15 (m, 2H), 7.84 (m, 1H), 7.80 (s, 1H), 7.64 (br s, 3H), 7.53 (s, 1H), 7.26 (s, 1H), 6.51 (s, 1H), 6.30 (d, *J =* 2.9 Hz, 2H), 5.59 - 5.46 (m, 2H), 5.43 (s, 2H), 4.87 - 4.78 (m, 1H), 4.70 - 4.60 (m, 1H), 4.17 - 4.03 (m, 2H), 3.82 - 3.58 (m, 2H), 3.41 (s, 3H), 2.80 - 2.70 (m, 2H), 2.40 - 2.22 (m, 2H), 1.93 - 1.68 (m, 6H), 1.65 - 1.57 (m, 1H), 1.56 - 1.45 (m, 3H), 1.36 - 1.22 (m, 2H), 0.87 (t, *J* = 7.3 Hz, 3H), 0.80 (d, *J =* 5.2 Hz, 6H).

Using the same method and reaction conditions as Example 2.1, as well as different starting materials as shown, the target products shown in Examples 2.2 to 2.8 in the following table were obtained.

| Example | Compound | Name | (m/z): [M+H]⁺ |
|---|---|---|---|
| Example 2.2 | 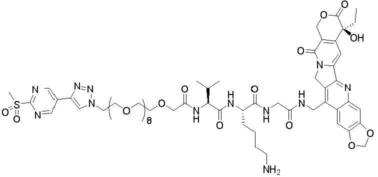 | N-((S)-1-(((S)-6-amino-1-((2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin- 14-yl)methyl)amino)-2-oxoethyl)amino)-1-oxo-hexan-2-yl)amino)-3-methyl-1-oxo-butan-2-yl)-29-(4-(2-(methanesulfonyl)pyrimidin-5-yl)-1H-1,2,3-triazol-1-yl)-3,6,9,12,15,18,21,24,27-nonaoxa nonacosamide | 1366.9 |
| | DL-002 | | |
| | Starting materials: C1.2, B1.5 | | |
| Example 2.3 | 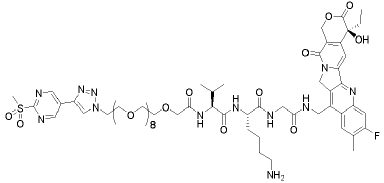 | N-((S)-1-(((S)-6-amino-1-((2-((((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)methyl)amino)-2-oxoethyl)amino)-1- oxohexan-2-yl)amino)-3-methyl-1-oxobutan-2-yl)-29-(4-(2-(methanesulfonyl)pyrimidin-5-yl)-1H-1,2,3-triazol-1-yl)-3,6,9,12,15,18,21,24,27-nonaoxanonacosamide | 1354.9 |
| | DL-003 | | |
| | Starting materials: C1.2, B1.4 | | |
| Example 2.4 | 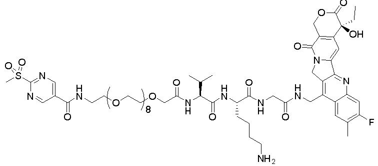 DL-004 | N-((7S,10S)-7-(4-aminobutyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-10-isopropyl-3,6,9,12-tetraoxo-14,17,20,23,26,29,32,35,38-nonaoxa-2,5,8,11-tetraazatetracontan-40-yl)-2-(methanesulfonyl)pyrimidin-5-carboxamide | 1330.9 |
| | Starting materials: C1.1, B1.4 | | |
| Example 2.5 | 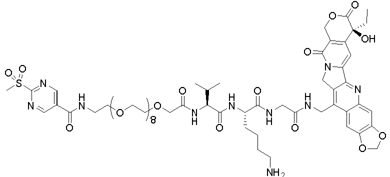 DL-005 | N-((7S,10S)-7-(4-aminobutyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-10-isopropyl-3,6,9,12-tetraoxo-14,17,20,23,26,29,32,35,38-nonaoxa-2,5,8,11-tetraazatetracontan-40-yl)-2-(methanesulfonyl)pyrimidin-5-carboxamide | 1342.8 |
| | Starting materials: C1.1, B1.5 | | |
| Example 2.6 | 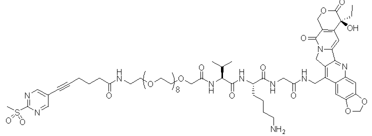 DL-006 | N-((S)-1-(((S)-6-amino-1-((2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)amino)-1- oxohexan-2-yl)amino)-3-methyl-1-oxobutan-2-yl)-29-(6-(2-(methanesulfonyl)pyrimidin-5-yl)hex-5-ynamido)-3,6,9,12,15,18,21,24,27-nonaoxanonacosamide | 1408.5 |
| | Starting materials: C1.5, B1.5 | | |
| Example 2.7 | 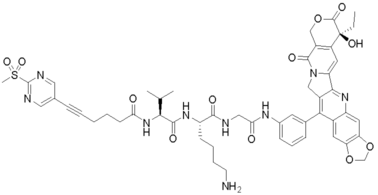 DL-007 | N-((S)-1-(((S)-6-amino-1-((2-((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin- 14-yl)phenyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide | 1017.9 |
| | Starting materials: C1.7, B1.8 | | |
| Example 2.8 | 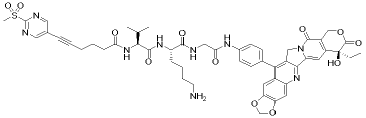 DL-008 | N-((S)-1-(((S)-6-amino-1-((2-((4-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin- 14-yl)phenyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide | 1017.8 |
| | Starting materials: C1.7, B1.9 | | |

### Example 2.9 : (S)-6-amino-N-(2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-009)

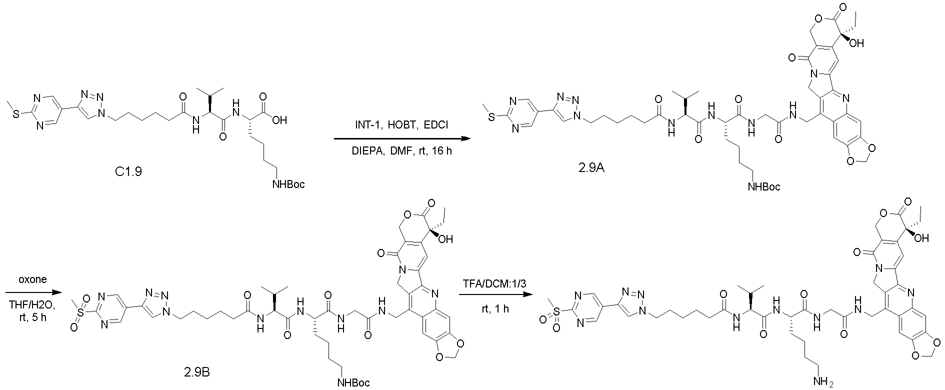

### Step 1:

Compound B1.5(30 mg), compound C1.9 (48 mg), and N,N-diisopropylethylamine (23 mg) were dissolved in DMF (5 mL), to which were then added 1-hydroxybenzotriazole (9 mg) and 1-(3-dimethylaminopropyl)-3-ethylcarbodiimide hydrochloride (13 mg), and the reaction solution was stirred at room temperature for 16 h, and diluted with 50 mL of ethyl acetate. The organic phase was washed with water three times (25 mL x 3), dried over anhydrous sodium sulfate, and then concentrated to remove ethyl acetate and obtain the crude product, which was separated and purified by flash silica gel column chromatography (DCM:MeOH = 100:0 to 90:10) to obtain the target compound 2.9A (35 mg, pale yellow solid, yield 43%).

LCMS (ESI) [M+H]⁺: 1095.8

### Step 2:

Compound 2.9A (35 mg) was dissolved in mixed solution of tetrahydrofuran and water (volume ratio 1/1; 4 mL), to which was added potassium peroxymonosulfonate (184 mg), and the reaction solution was stirred at room temperature for 5 h, and diluted with 30 mL of ethyl acetate. The organic phase was washed three times with water (25 mL x 3), dried over anhydrous sodium sulfate, and concentrated to remove ethyl acetate and obtain the target compound 2.9B (25 mg, pale yellow solid, yield 71%).

LCMS (ESI) [M+H]⁺: 1126.9.

### Step 3:

Compound 2.9B (25 mg) was dissolved in a mixed solution of trifluoroacetic acid and dichloromethane (volume ratio 1:3; 4 mL), and then the reaction solution was stirred at room temperature for 1 h. Then, the reaction solution was concentrated under reduced pressure to provide the crude product, which was purified by prep-HPLC (0.01% TFA in water, MeCN) to obtain the target compound (S)-6-amino-N-(2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide trifluoroacetate (5.1 mg).

LCMS (ESI) [M+H]⁺: 1026.9;
¹H NMR (400 MHz, DMSO) δ 9.46 (s, 2H), 8.91 (s, 1H), 8.53 (s, 1H), 8.18 (s, 1H), 8.11 - 8.10 (m, 1H), 7.79 (s, 1H), 7.73 - 7.72 (m, 1H), 7.63 (s, 3H), 7.53 (s, 1H), 7.26 (s, 1H), 6.52 (s, 1H), 6.29 (m, 2H), 5.61 - 5.46 (m, 2H), 5.43 (s, 2H), 4.85 - 4.83 (m, 1H), 4.63 - 4.60 (m 1H), 4.44 (s, 2H), 4.06 - 4.03 (m, 3H), 2.75 (s, 2H), 2.12 - 2.10 (m, 2H), 1.89 - 1.85 (m, 6H), 1.56 - 1.53 (m, 8H), 1.24 (br s, 4H), 0.87 - 0.84 (m, 3H), 0.77 (d, *J =* 6.1 Hz, 6H).

### Example 2.10: N-((7S,10S)-7-(4-aminobutyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-10-isopropyl-3,6,9,12,16-pentaoxo-14,20,23,26,29,32,35,38,41-nonaoxa-2,5,8,11,17-pentaazatritetracontan-43-yl)-6-(2-(methanesulfonyl)pyrimidin-5-yl)hex-5-ynamide (DL-010)

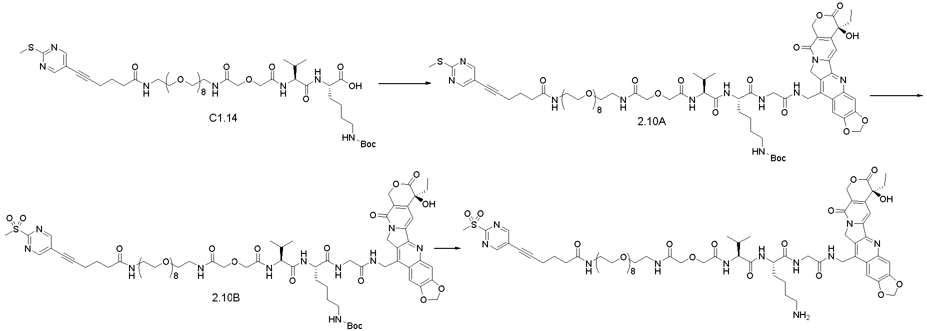

### Step 1:

Compound C1.14 (112 mg) was dissolved in N,N-dimethylformamide (2 mL), to which were successively added triethylamine (32 mg, 0.31 mmol), 1-hydroxybenzotriazole (17 mg, 0.13 mmol) and 1-(3-dimethylaminopropyl)-3-ethylcarbodiimide hydrochloride (24 mg, 0.13 mmol), and then the mixture were stirred for 20 min. Then, compound B1.5 (50 mg) was added to the reaction solution, and the reaction solution was stirred at 40 °C overnight. 10 mL of water was added to the reaction solution, and the resultant solution was extracted with dichloromethane, dried over anhydrous sodium sulfate, filtered, and rotatory evaporated to dryness, to obtain the crude product, which was separated and purified over reversed-phase C18 column (acetonitrile/0.01% FA aqueous solution: 0%-55%) to obtain the target compound 2.10A (75 mg, yield 46%) as a white solid.

LCMS (ESI) [M+H]⁺: 1534.0.

### Step 2:

Compound 2.10A (75 mg) was dissolved in a solution of tetrahydrofuran and water (8 mL, 1:1), to which was added potassium peroxymonosulfonate (211 mg), and then the reaction was stirred at room temperature for 10 h. 10 mL of dichloromethane was added to the reaction solution, followed by extraction and separation of phases. The organic phase was dried over anhydrous sodium sulfate, filtered and rotatory evaporated to dryness, to obtain the target product 2.10B (50 mg, yield 65%).

LCMS (ESI) [M+H]⁺: 1566.2.

### Step 3:

Compound 2.10B (50 mg) was dissolved in a mixed solvent of trifluoroacetic acid and dichloromethane (volume ratio 1:2; 4 mL), and then the reaction solution was stirred at room temperature for 1 h. The reaction solution was concentrated to obtain a crude product, which was subjected to preparative chromatography (0.01% TFA in water, MeCN), to obtain the target compound N-((7S,10S)-7-(4-aminobutyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-10-isopropyl-3,6,9,12,16-pentaoxo-14,20,23,26,29,32,35,38,41-nonaoxa-2,5,8,11,17-pentaazatritetracontan-43-yl)-6-(2-(methanesulfonyl)pyrimidin-5-yl)hex-5-ynamide (10.5 mg, yield 21%).

LCMS (ESI) [M+H]⁺: 1466.9;
¹H NMR (400 MHz, DMSO-d6) δ 9.12 (s, 2H), 8.62 (t, *J =* 5.7 Hz, 1H), 8.26 - 8.11 (m, 2H), 8.03 (t, *J =* 5.8 Hz, 1H), 7.93 (t, *J =* 5.3 Hz, 1H), 7.85 (d, *J =* 8.7 Hz, 1H), 7.79 (d, *J =* 5.6 Hz, 1H), 7.61 (s, 3H), 7.54 (s, 1H), 7.26 (s, 1H), 6.50 (s, 1H), 6.31 (d, *J =* 1.7 Hz, 2H), 5.46 *(d, J* = 17.2 Hz, 4H), 4.76 (dt, *J =* 14.8, 8.7 Hz, 2H), 4.20 - 4.15 (m, 4H), 4.00 (s, 3H), 3.95 (d, *J =* 5.5 Hz, 3H), 3.72 - 3.67 (m, 2H), 3.50 (d, *J =* 3.9 Hz, 30H), 3.41 (s, 3H), 3.25 - 3.19 (m, 4H), 2.76 (d, *J =* 5.6 Hz, 2H), 2.56 (t, *J* = 4.9 Hz, 2H), 2.27 (t, *J =* 7.3 Hz, 2H), 1.90 (dd, *J* = 16.1, 7.4 Hz, 2H), 1.84 - 1.78 (m, 2H), 1.63 (s, 1H), 1.53 - 1.48 (m, 2H), 1.30 (d, *J =* 7.7 Hz, 2H), 0.87 - 0.84 (m, 4H), 0.78 - 0.75 (m, 6H).

### Example 2.11: N-((S)-1-(((S)-6-amino-1-((2-((3-((S)-7-ethyl-7-hydroxy-8,11-dioxo-7,8,11,13-tetrahydro-10H-[1,3] dioxolo[4,5-g]pyrano[3',4':6,7]indolizino[1,2-b]quinolin-14-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (DL-011)

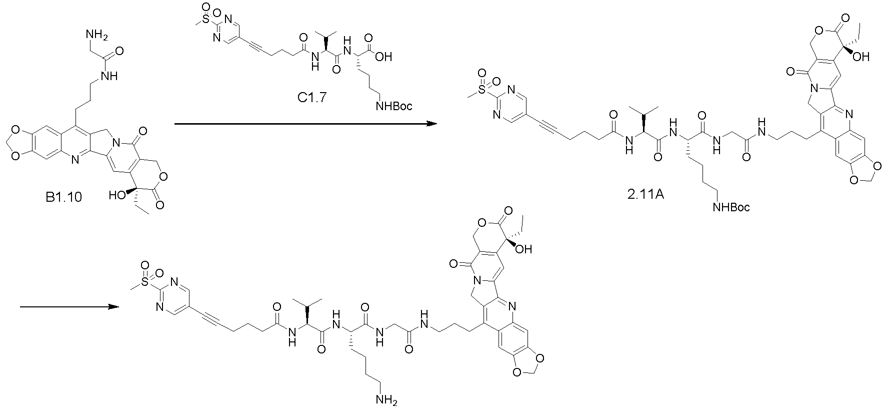

### Step 1:

To a solution of compound B1.10 (75 mg) in N,N-dimethylformamide (3 mL), were successively added compound C1.7 (107 mg), triethylamine (45 mg), HOBT (25 mg) and EDCI (35 mg), and then the mixture was allowed to react at 35 °C for 4 h. Water (30 mL) was added to the reaction solution, and the resultant solution was extracted with ethyl acetate (30 mL×2). The organic phase was dried over anhydrous sodium sulfate, and concentrated to obtain the target compound 2.11A (60 mg, yield: 37%).

LCMS (ESI) [M+H]⁺: 1084;

### Step 2:

To a solution of compound 2.11A (60 mg) in dichloromethane (4 mL), was added a mixed solvent of trifluoroacetic acid and dichloromethane (volume ratio 1:2; 4 mL), and the reaction was stirred at room temperature for 1 h. The reaction solution was concentrated, and the crude product was purified by prep-HPLC (acetonitrile/0.05% formic acid in water) to obtain the target compound N-((S)-1-(((S)-6-amino-1-((2-((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyl)amino)-2-oxoethyl)amino)-1-oxohexan-2-yl)amino)-3- methyl-1-oxobutan-2-yl)-6-(2-(methanesulfonyl)pyrimidin-5-yl)hex-5-ynamide (9 mg, yield 18%).

LCMS (ESI) [M+H]⁺: 983.9;
¹H NMR (400 MHz, DMSO-d6) δ 9.08 (s, 2H), 8.34 (s, 1H), 8.24 (s, 1H), 8.19 (d, *J =* 7.1 Hz, 1H), 8.00 (d, *J* = 8.3 Hz, 1H), 7.95 (s, 1H), 7.63 (s, 1H), 7.49 (d, *J =* 2.8 Hz, 1H), 7.24 (s, 1H), 6.50 (s, 1H), 6.28 (d, *J =* 1.9 Hz, 2H), 5.42 (s, 2H), 5.24 (s, 2H), 4.25 - 4.07 (m, 2H), 3.74 - 3.70 (m, 3H), 3.25 (d, *J =* 12.4 Hz, 3H), 3.08 (d, *J =* 7.6 Hz, 2H), 2.73 (s, 2H), 2.40 - 2.19 (m, 3H), 2.01 - 1.64 (m, 10H), 1.52 (br s, 4H), 1.34 (br s, 2H), 0.87 (t, *J =* 7.3 Hz, 3H), 0.83 - 0.75 (m, 6H).

### Example 2.12 : (S)-6-amino-N-(2-((((S)-7-ethyl-7-hydroxy-8,11-dioxo-7,8,11,13-tetrahydro-10H-[1,3] dioxolo[4,5-g]pyrano[3',4':6,7]indolizino[1,2-b]quinolin-14-yl)methyl)amino)-2-oxoethyl)-2-((S)-2-isopropyl-35-(4-(2-(methanesulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (DL-012)

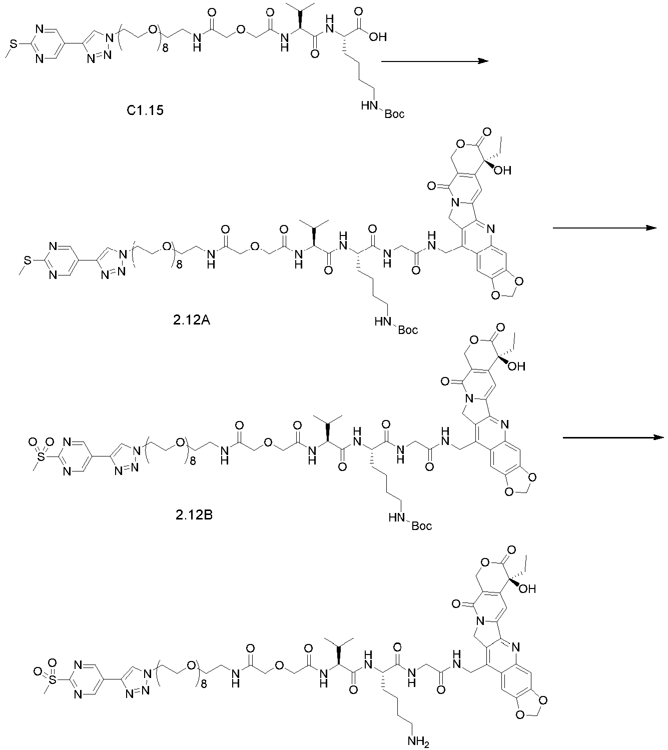

### Step 1:

Compound C1.15 (80 mg), compound B1.5 (37 mg), and triethylamine (23 mg, 0.231 mol) were dissolved in DMF (5 mL), to which were then added 1-hydroxybenzotriazole (12 mg) and 1-(3-dimethylaminopropyl)-3-ethylcarbodiimide hydrochloride (17 mg), and the reaction solution was stirred at room temperature for 16 h, and diluted with 50 mL of ethyl acetate. The organic phase was washed with water three times (25 mL x 3), dried over anhydrous sodium sulfate, and then concentrated to removed ethyl acetate to obtain a crude product, which was separated and purified by flash chromatography (DCM:MeOH = 100:0 to 90:10) to obtain the target compound 2.12A (75 mg, yield 65%).

LCMS (ESI) [M+H]⁺: 1492.0.

### Step 2:

Compound 2.12A (50 mg) was dissolved in a mixed solution of tetrahydrofuran and water (volume ratio 1/1; 4 mL), to which was added potassium peroxymonosulfonate (202 mg), and the reaction solution was stirred at room temperature for 5 h, and diluted with 30 mL of ethyl acetate. The organic phase was washed three times with water (25 mL x 3), dried over anhydrous sodium sulfate, and concentrated to remove ethyl acetate and obtain the target compound 2.12B (40 mg, yield 80%).

LCMS (ESI) [M+H]⁺: 1524.1.

### Step 3:

Compound 2.12B (30 mg) was dissolved in a mixed solution of trifluoroacetic acid and dichloromethane (volume ratio 1:3; 4 mL), and then the reaction solution was stirred at room temperature for 1 h. Then, the reaction solution was concentrated to provide the crude product, which was purified by prep-HPLC (0.01% TFA in water, MeCN) to obtain the target compound (S)-6-amino-N-(2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)-2-((S)-2-isopropyl-35-(4-(2-(methanesulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (9 mg, yield 30%).

LCMS (ESI) [M+H]⁺: 1423.9.

### Example 2.13 : N-((13S,16S)-13-(4-(dimethylamino)butyl)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9, 12,15-tetraoxo-6-oxa-3,8,11,14-tetraazaoctadecan-16-yl)-6-(2-(methanesulfonyl)pyrimidin-5-yl)hex-5-ynamide (DL-013)

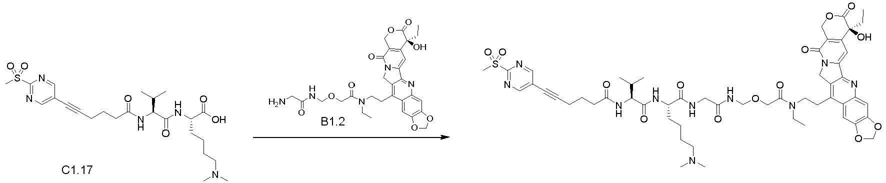

Compound C1.17 (50 mg), compound B1.2 (58 mg), and triethylamine (24 mg) were dissolved in N, N-dimethylformamide (2 mL), to which were then added 1-hydroxybenzotriazole (16 mg) and 1-(3-dimethylaminopropyl)-3-ethylcarbodiimide hydrochloride (22 mg), and the reaction solution was stirred at room temperature for 16 h. The reaction solution was directly purified by preparative chromatography (0.01% aqueous formic acid solution, acetonitrile) to obtain the target compound (21 mg, yield 10%).

LCMS (ESI) [M+H]⁺: 1113.0;
¹H NMR (400 MHz, MeOD) δ 8.93 (s, 2H), 8.40 - 8.33 (m, 1H), 8.30 - 8.25 (m, 1H), 8.25 - 8.21 (m, 1H), 7.65 (s, 1H), 7.48 (s, 1H), 7.34 (s, 1H), 6.22 (s, 2H), 5.47 (dd, *J =* 78.6, 16.4 Hz, 2H), 5.28 - 5.13 (m, 2H), 4.73 - 4.59 (m, 2H), 4.30 - 4.05 (m, 4H), 3.94 - 3.82 (m, 2H), 3.70 - 3.57 (m, 2H), 3.54 - 3.44 (m, 1H), 3.34 (s, 6H), 3.16 - 3.08 (m, 2H), 2.90 (s, 6H), 2.58 - 2.40 (m, 4H), 2.04 - 1.83 (m, 6H), 1.80 - 1.68 (m, 3H), 1.54 - 1.36 (m, 2H), 1.24 - 1.15 (m, 3H), 1.03 - 0.86 (m, 9H).

### Example 2.14: N-((13S,16S)-13-(4-(N-oxo-N,N-dimethylamino)butyl)-3-ethyl-1-((S)-7-ethyl-7-hydroxy- 8,11-dioxo-7,8,11,13-tetrahydro-10H-[1,3]dioxolo[4,5-g]pyrano[3',4':6,7]indolizino[1,2-b]quinolin-14-yl)-17- methyl-4,9,12,15-tetraoxo-6-oxa-3,8,11,14-tetraazaoctadecan-16-yl)-6-(2-(methanesulfonyl)pyrimidin-5-yl)hex-5-ynamide (DL-014)

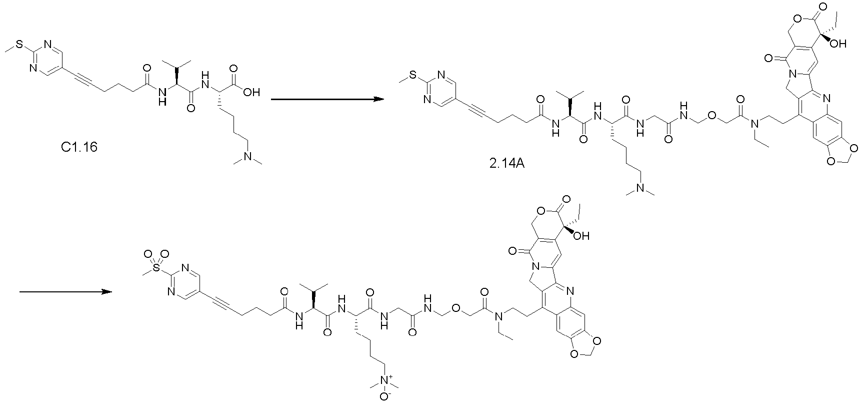

### Step 1:

Compound C1.16 (49 mg), compound B1.2 (71 mg), and N,N-diisopropylethylamine (39 mg) were dissolved in DMF (5 mL), to which were then added 1-hydroxybenzotriazole (16 mg) and 1-(3-dimethylaminopropyl)-3-ethylcarbodiimide hydrochloride (23 mg), and then the reaction solution was stirred at room temperature for 16 h, and diluted with 50 mL of ethyl acetate. The organic phase was washed three times with water (25 mL x 3), dried over anhydrous sodium sulfate, and then concentrated to remove ethyl acetate and obtain the crude product, which was separated and purified by flash silica gel column chromatography (DCM:MeOH = 100:0 to 90:10) to obtain the target compound 2.14A (61 mg, yield 60%).

LCMS (ESI) [M+H]⁺: 1081.6;

### Step 2:

Compound 2.14A (40 mg) was dissolved in a mixed solvent of tetrahydrofuran and water (volume ratio 1/1; 4 mL), to which was added potassium peroxymonosulfonate (227 mg, 0.37 mmol), and the reaction solution was stirred at room temperature for 5 h. LCMS indicated completion of the reaction, and then the reaction solution was diluted with 30 mL of ethyl acetate. The organic phase was washed three times with water (25 mL x 3), dried over anhydrous sodium sulfate, and concentrated to remove ethyl acetate and obtain a crude compound, which was purified by preparative chromatography (0.01% formic acid aqueous solution, acetonitrile) to obtain the target compound N-((13S,16S)-13-(4-(N-oxo-N,N-dimethylamino)butyl)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9, 12,15-tetraoxo-6-oxa-3,8,11,14-tetraazaoctadecan-16-yl)-6-(2-(methanesulfonyl)pyrimidin-5-yl)hex-5-ynamide (9 mg, yield 33%).

LCMS (ESI) [M+H]⁺ = 1129.0.

### Example 2.15: N-((13S,16S)-13-(4-(N,N-dimethylamino)butyl)-3-isopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6-oxa-3,8,11,14-tetraazaoctadecan-16-yl)-6-(2-(methanesulfonyl)pyrimidin-5-yl)hex-5-ynamide (DL-015)

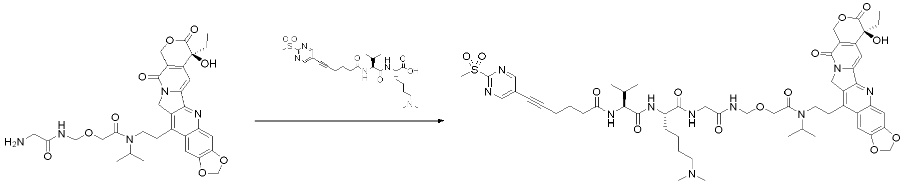

### To a solution of compound N⁶,N⁶-dimethyl-N²-((6-(2-(methanesulfonyl)pyrimidin-5-yl)hexan-5-ynoyl)- L-valinyl)-L-lysine (42 mg, C1.17) and compound (S)-2-((2-aminoacetamido)methoxy)-N-isopropyl-N- (2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide (50 mg, B1.11) in N,N-dimethylformamide (10 mL), were successively added HBTU (31 mg) and diisopropylethylamine (21 mg), and then the reaction was stirred at room temperature for 2 h. After filtration, the reaction solution was purified by preparative HPLC (acetonitrile/water containing 0.05% trifluoroacetic acid) to obtain the target compound N-((13S,16S)-13-(4-(N,N-dimethylamino)butyl)-3-isopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6- oxa-3,8,11,14-tetraazaoctadecan-16-yl)-6-(2-(methanesulfonyl)pyrimidin-5-yl)hex-5-ynamide (15.2 mg).

LCMS (ESI) [M+H]⁺ = 1127.6;
¹H NMR (400 MHz, DMSO-d6) δ 9.10 (s, 2H), 8.73 (t, *J =* 8.7 Hz, 1H), 8.23 - 8.17 (m, 1H), 8.10 (d, *J =* 7.5 Hz, 1H), 7.97 - 7.93 (m, 2H), 7.52 (s, 1H), 7.25 (s, 1H), 6.50 (s, 1H), 6.30 (s, 2H), 5.42 (s, 2H), 5.36 (s, 2H), 4.66 (d, *J =* 6.6 Hz, 2H), 4.33 - 4.21 (m, 4H), 4.20 - 4.13 (m, 2H), 3.76 - 3.74 (m, 2H), 3.40 (s, 3H), 3.40 - 3.38 (m, 1H), 3.30 - 3.21 (m, 2H), 3.04 - 2.95 (m, 2H), 2.76 (s, 3H), 2.74 (s, 3H), 2.39 - 2.31 (m, 2H), 2.00 - 1.74 (m, 6H), 1.68 - 1.49 (m, 4H), 1.37 - 1.27 (m, 2H), 1.23 - 1.16 (m, 6H), 0.89 - 0.82 (m, 9H).

### Example 2.16: N-((10S,13S)-10-(4-(N,N-dimethylamino)butyl)-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-14-methyl-1,6,9,12-tetraoxo-3-oxa-5,8,11-triazapentadecan-13-yl)-6-(2-(methanesulfonyl)pyrimidin-5-yl)hex-5-ynamide (DL-016)

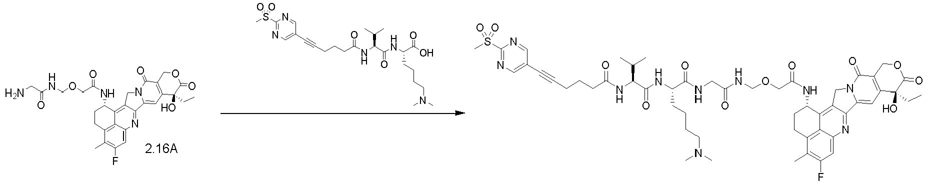

Compound N⁶,N⁶-dimethyl-N²-((6-(2-(methanesulfonyl)pyrimidin-5-yl)hexadecyl-5-ynoyl)-L-propionyl)-L-lysine (C1.17, 52.3 mg) was dissolved in N,N-dimethylformamide (2 mL), to which was then added HBTU(38 mg, 0.10 mmol) and N,N-diisopropylethylamine (26 mg, 0.20 mmol) sequentially, and after addition, the reaction solution was stirred for 30 min at room temperature. Then, compound 2-amino-N-((2-(((1S,9S)-9-ethyl-5-fluoro- 9-hydroxy-4-methyl-10,13-dioxo-2,3,9,10,13,15-hexahydro-1H,12H-benzo[de]pyrano[3',4':6,7]indolo[1,2-b]quinolin-1-yl)amino)-2-oxoethoxy)methyl)acetamide (2.16A, prepared according to the same method reported on pages 147-148 in CN104755494A, 63 mg) was added. LCMS detection indicated completion of the reaction, and then the reaction solution was directly purified by preparative chromatography (0.01% trifluoroacetic acid aqueous solution, acetonitrile) to obtain the target compound N-((10S,13S)-10-(4-(N,N-dimethylamino)butyl)-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-14-methyl-1,6,9,12-tetraoxo-3-oxa-5,8,11-triazapentadecan-13-yl)-6-(2-(methanesulfonyl)pyrimidin-5-yl)hex-5-ynamide (8.1 mg, yield 7%).

LCMS (ESI) [M+H]⁺ = 1085.5;
¹H NMR (400 MHz, DMSO-d6) δ 9.31 (s, 1H), 9.11 (s, 2H), 8.72 (t, *J =* 6.8 Hz, 1H), 8.51 (d, *J =* 8.8 Hz, 1H), 8.19 (t, *J =* 6.8 Hz, 1H), 8.10 (d, *J =* 7.1 Hz, 1H), 7.91 (d, *J =* 8.6 Hz, 1H), 7.79 (d, *J =* 10.9 Hz, 1H), 7.32 (s, 1H), 6.53 (s, 1H), 5.63 - 5.54 (m, 1H), 5.42 (s, 2H), 5.20 (s, 2H), 4.68 - 4.58 (m, 2H), 4.26 - 4.12 (m, 2H), 4.01 (s, 2H), 3.73 (d, *J =* 5.4 Hz, 2H), 3.20 - 3.10 (m, 2H), 3.05 - 2.95 (m, 2H), 2.76 (d, *J =* 4.7 Hz, 6H), 2.55 - 2.53 (m, 2H), 2.42 - 2.28 (m, 6H), 2.21 - 2.13 (m, 2H), 2.02 - 1.77 (m, 6H), 1.76 - 1.62 (m, 2H), 1.62 - 1.51 (m, 3H), 1.37 - 1.21 (m, 2H), 0.90 - 0.80 (m, 9H).

### Example 2.17: tert-butyl ((S)-3-ethyl-1-((S)-7-ethyl-7-hydroxy-8,11-dioxo-7,8,11,13-tetrahydro-10H-[1,3]dioxolo [4,5-g]pyrano[3',4':6,7]indolizino[1,2-b]quinolin-14-yl)-13-((S)-3-methyl-2-(6-(2-(methanesulfonyl)pyrimidin-5-yl)hex-5-ynamido)butanamido)-4,9,12-trioxo-6-oxa-3,8,11-triazaheptadecan-17-yl)carbamate (DL-017)

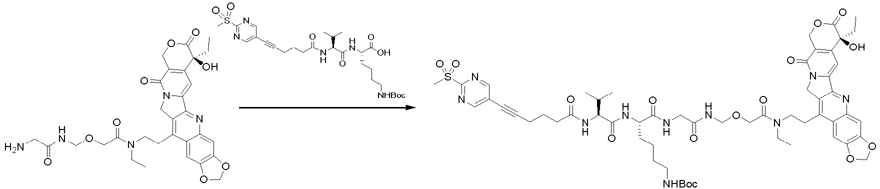

Compound C1.7 (90.0 mg), diisopropylethylamine (68.0 mg) and HATU (100 mg) were dissolved in N,N-dimethylformamide (5 mL), to which was then added compound B1.2 (80 mg), and the mixture was allowed to react at room temperature for 2 h. LCMS detection indicated completion of the reaction. Ethyl acetate (50 mL) was added to the reaction solution, and the resultant solution was washed with saturated brine (30 mL×3), dried over anhydrous sodium sulfate, filtered. The filtrate was concentrated under reduced pressure to a solid, and the crude product was purified by preparative HPLC ( acetonitrile/water containing 0.1% formic acid) to obtain a yellow solid compound tert-butyl ((S)-3-ethyl-1-((S)-7-ethyl-7-hydroxy- 8,11-dioxo-7,8,11,13-tetrahydro-10H-[1,3]dioxolo[4,5-g]pyrano[3',4':6,7]indolizino[1,2-b]quinolin-14-yl)-13-((S)-3-methyl-2-(6-(2-(methanesulfonyl)pyrimidin-5-yl)hex-5-ynamido)butanamido)-4,9,12-trioxo-6-oxa-3,8,11-triazaheptadecan-17-yl)carbamate (2.06 mg, yiled 10.6%).

LCMS (ESI) [M+H]⁺: 1184.9.

### Example 2.18: N-((11S,14S)-11-(4-(dimethylamino)butyl)-1-((S)-4-ethyl-8-fluoro-4-hydroxy-9-methyl- 3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide (DL-018)

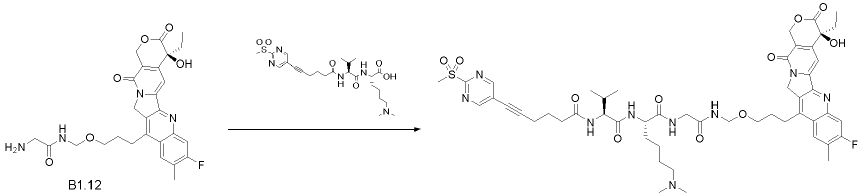

Compound N⁶,N⁶-dimethyl-N²-((6-(2-(methanesulfonyl)pyrimidin-5-yl)hexan-5-ynoyl)-L-valinyl)-L-lysine (126 mg) (C1.17), B1.12 (150 mg), 1-hydroxybenzotriazole (49 mg), 1-(3-dimethylaminopropyl)-3-ethylcarbodiimide hydrochloride (69 mg) and diisopropylethylamine (156 mg, 1.21 mmol) were dissolved in N,N-dimethylformamide (10 mL), and then the reaction was stirred at 40 °C for 12 h. LCMS indicated completion of the reaction. The reaction solution was filtered and purified by preparative HPLC (acetonitrile/water containing 0.05% formic acid) to obtain compound N-((11S,14S)-11-(4-(dimethylamino)butyl)-1-((S)-4-ethyl- 8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide (DL-018) (12 mg, yield 6.0%) as a yellow solid.

LCMS (ESI) [M+H]⁺ = 1030.6;
¹H NMR (400 MHz, DMSO-d6) δ 9.10 (s, 2H), 8.62 (t, *J =* 6.4 Hz, 1H), 8.23 - 8.13 (m, 2H), 8.07 (d, *J* = 7.2 Hz, 1H), 7.95 - 7.83 (m, 2H), 7.31 (s, 1H), 6.53 (s, 1H), 5.44 (s, 2H), 5.29 (s, 2H), 4.64 - 4.53 (m, 2H), 4.28 - 4.20 (m, 1H), 4.19 - 4.11 (m, 1H), 3.76 - 3.69 (m, 2H), 3.50 (t, *J =* 5.7 Hz, 2H), 3.41 (s, 3H), 3.23 - 3.19 (m, 2H), 2.67 - 2.62 (m, 2H), 2.57 - 2.54 (m, 2H), 2.54 (s, 3H), 2.42 - 2.26 (m, 3H), 2.02 - 1.77 (m, 6H), 1.68 - 1.66 (m, 1H), 1.58 - 1.45 (m, 3H), 1.36 - 1.25 (m, 2H), 0.88 (t, *J =* 7.4 Hz, 3H), 0.82 (t, *J =* 6.3 Hz, 6H).

### Example 2.19: N-((11S,14S)-11-(4-(dimethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methanesulfonyl)pyrimidin-5-yl)hex-5-ynamide (DL-019)

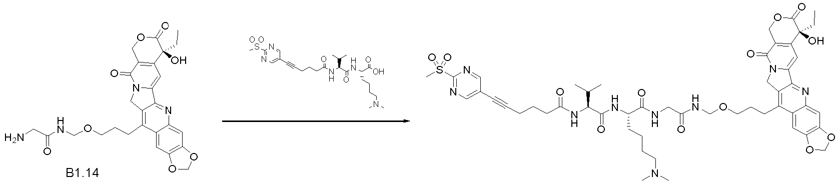

Compound B1.14 (100 mg, 0.186 mmol) and compound N⁶,N⁶-dimethyl-N²-((6-(2-(methanesulfonyl)pyrimidin-5-yl)hexan-5-ynoyl)-L-valinyl)-L-lysine (100 mg, 0.191 mmol) were dissolved in DMF (2 mL), to which were then added 1-hydroxybenzotriazole (38 mg, 0.280 mmol) and 1-(3-dimethylaminopropyl)-3-ethylcarbodiimide hydrochloride (53 mg, 0.280 mmol), followed by addition of triethylamine (56 mg, 0.559 mmol). The reaction was stirred at room temperature for 16 h. LCMS indicated completion of the reaction. The reaction solution was purified by preparative chromatography (0.01% TFA in water, MeCN) to obtain the target compound N-((11S,14S)-11-(4-(dimethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methanesulfonyl)pyrimidin-5-yl)hex-5-ynamide (20 mg, yield 10%) as a yellow solid.

LCMS (ESI) [M+H]⁺ = 1042.5;
¹H NMR (400 MHz, DMSO-d6) δ 9.28 (s, 1H, TFA), 9.10 (s, 2H), 8.64 (br s, 1H), 8.19 (br s, 1H), 8.09 (d, *J* = 6.4 Hz, 1H), 7.92 (d, *J =* 7.8 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.67 - 4.52 (m, 2H), 4.30 - 4.10 (m, 2H), 3.74 (br s, 2H), 3.50 (br s, 2H), 3.41 (s, 3H), 3.17 - 3.07 (m, 2H), 3.04 - 2.91 (m, 2H), 2.75 (s, 6H), 2.46 - 2.24 (m, 3H), 2.04 - 1.66 (m, 9H), 1.63 - 1.46 (m, 3H), 1.32 - 1.29 (m, 2H), 0.87 - 0.82 (m, 9H).

### Example 2.20: N-((S)-1-(((S)-6-amino-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (DL-020)

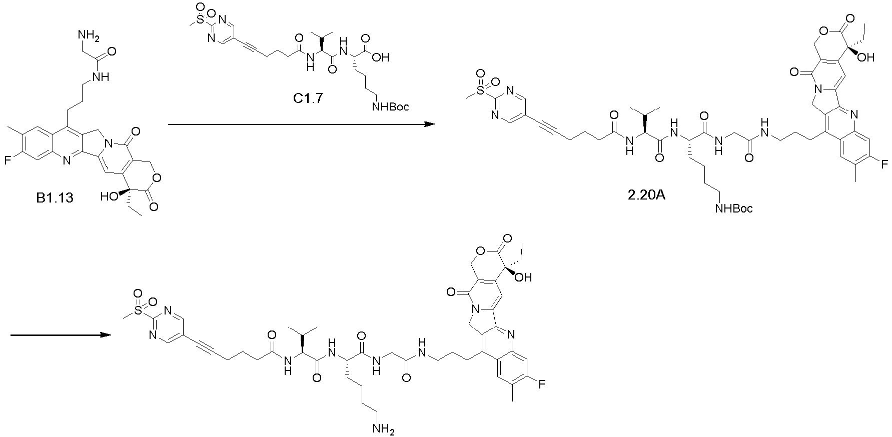

### Step 1:

To a solution of compound B1.13 (75 mg) in N,N-dimethylformamide (3 mL), were successively added compound C1.7 (107 mg), triethylamine (45 mg), HOBT (25 mg) and EDCI (35 mg), and the mixture was allowed to react at 35°C for 4 h. Water (30 mL) was added to the reaction solution, and the resultant solution was extracted with ethyl acetate (30 mL×2). The organic phase was dried over anhydrous sodium sulfate, and concentrated to obtain the target compound 2.20A (76 mg).

LCMS (ESI) [M+H]⁺: 1072.5;

### Step 2:

To a solution of compound 2.20A (60 mg, 0.06 mmol) in dichloromethane (2 mL), was added trifluoroacetic acid (1 mL), and then the mixture was stirred at room temperature for 1 h. LCMS showed completion of the reaction. The reaction solution was concentrated and purified by preparative chromatography (0.01% TFA in water, MeCN) to obtain the target compound N-((S)-1-(((S)-6-amino-1-((2-((3-((S)-4-ethyl-8-fluoro-4-hydroxy-9-methyl- 3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (43 mg, yield 80%) as a yellow solid.

LCMS (ESI) [M+H]⁺: 972.9;
¹H NMR (400 MHz, DMSO-d6) δ 9.09 (s, 2H), 8.24 - 8.17 (m, 2H), 8.12 (d, *J* = 7.0 Hz, 1H), 7.99 - 7.93 (m, 1H), 7.88 - 7.84 (m, 2H), 7.64 (s, 3H, NH2-TFA), 7.31 (s, 1H), 6.52 (s, 1H), 5.44 (s, 2H), 5.30 (s, 2H), 4.25 - 4.18 (m, 1H), 4.15 - 4.11 (m, 1H), 3.76 - 3.71 (m, 2H), 3.41 (s, 3H), 3.32 - 3.26 (m, 2H), 3.21 - 3.17 (m, 1H), 2.79 - 2.70 (m, 2H), 2.52 (s, 3H), 2.40 - 2.24 (m, 3H), 1.94 - 1.82 (m, 7H), 1.80 - 1.73 (m, 2H), 1.58 - 1.49 (m, 3H), 1.36 - 1.31 (m, 1H), 1.18 - 1.14 (m, 1H), 0.87 (t, *J =* 7.4 Hz, 3H), 0.80 - 0.76 (m, 6H).

### Example 2.21: N-((S)-1-(((S)-5-amino-6-((2-((3-((S)-7-ethyl-7-hydroxy-8,11-dioxo-7,8,11,13-tetrahydro- 10H-[1,3]dioxolo[4,5-g]pyrano[3',4':6,7]indolizino[1,2-b]quinolin-14-yl)propyl)amino)-2-oxoethyl)amino)-6-oxohexyl)amino)-3-methyl-1-oxobutan-2-yl)-6-(2-(methanesulfonyl)pyrimidin-5-yl)hex-5-ynamide

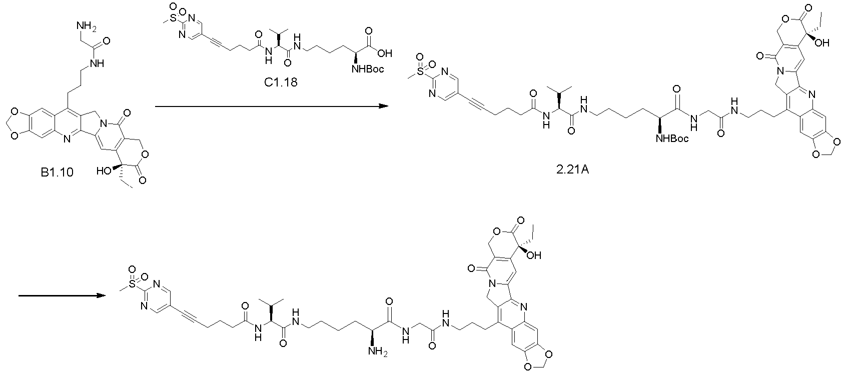

### Step 1:

Compound C1.18 (430 mg) was dissolve in N,N-dimethylformamide (5 mL), to which were then added EDCI (160 mg), HOBT (115 mg) and triethylamine (214 mg). After addition, the reaction solution was stirred at room temperature for 20 min, and then compound B1.10 (314 mg) was added. The reaction was stirred at room temperature for 3 h. LCMS indicated completion of the reaction. The reaction solution was diluted with water, and then extracted with ethyl acetate. The organic phase was concentrated to provide crude product 2.21A (350 mg ).

LCMS (ESI) [M+H]⁺: 1084.3;

### Step 2:

To a solution of compound 2.21A (350 mg) in dichloromethane (4 mL), was added a mixted solvent of trifluoroacetic acid and dichloromethane (volume ratio 1:2; 4 mL), and then the reaction was stirred for 1 h at room temperature. The reaction solution was concentrated, and the crude product was purified by preparative HPLC (acetonitrile/0.05% formic acid aqueous solution) to obtain the target compound N-((S)-1-(((S)-5-amino-6-((2-((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyl)amino)-2-oxoethyl)amino)-6-oxohexyl)amino)-3-methyl-1-oxobutan-2-yl)-6-(2-(methanesulfonyl)pyrimidin-5-yl)hex-5-ynamide (100 mg).

LCMS (ESI) [M+H]⁺: 984.4;
¹H NMR (400 MHz, DMSO-d6) δ 9.10 (s, 2H), 8.69 (t, *J* = 6.1 Hz, 1H), 8.14 (t, *J* = 5.4 Hz, 1H), 8.08 (s, 3H, NH2-TFA), 7.95 (t, *J* = 4.9 Hz, 1H), 7.89 (d, *J =* 8.9 Hz, 1H), 7.66 (s, 1H), 7.51 (s, 1H), 7.25 (s, 1H), 6.48 (s, 1H), 6.29 (s, 2H), 5.42 (s, 2H), 5.25 (s, 2H), 4.09 (t, *J =* 8.1 Hz, 1H), 3.83 (d, *J =* 5.7 Hz, 2H), 3.40 (s, 3H), 3.33 - 3.27 (m, 2H), 3.15 - 3.06 (m, 3H), 2.97 - 2.94 (m, 1H), 2.57 - 2.53 (m, 2H), 2.43 - 2.28 (m, 3H), 1.95 - 1.68 (m, 9H), 1.46 - 1.28 (m, 4H), 0.88 (t, *J =* 7.3 Hz, 3H), 0.82 (d, *J =* 6.7 Hz, 6H).

### Example 2.22: N-((S)-1-(((S)-5-amino-6-((2-((3-((S)-4-ethyl-8-fluoro-4-hydroxy-9-methyl-3,14-dioxo- 3,4,12,14-tetrahydro-1H-pyrano[3',4':6,7]indolizino[1,2-b]quinolin-11-yl)propyl)amino)-2-oxoethyl)amino)-6-oxohexyl)amino)-3-methyl-1-oxobutan-2-yl)-6-(2-(methanesulfonyl)pyrimidin-5-yl)hex-5-ynamide (DL-022)

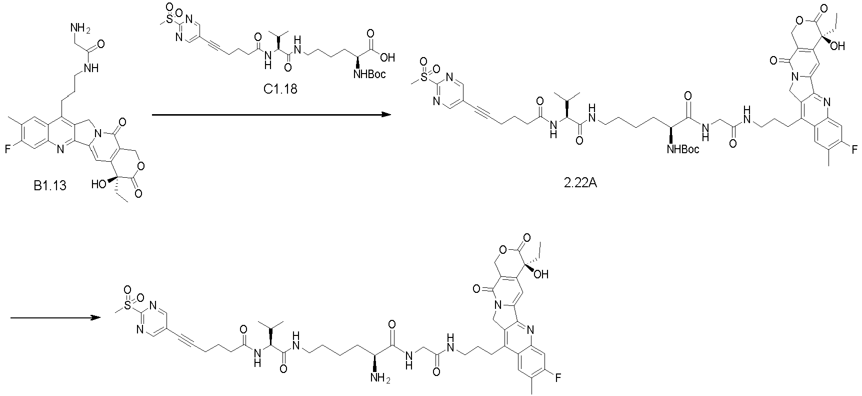

### Step 1:

Compound C1.18 (136 mg, 0.23 mmol) was dissolved in N,N-dimethylformamide (5 mL), to which were then added EDCI (48 mg, 0.25 mmol), HOBT (34 mg, 0.25 mmol) and triethylamine (64 mg, 0.63 mmol). After addition, the reaction solution was stirred at room temperature for 20 min, and then compound B1.13 (3105 mg, 0.19 mmol) was added. The reaction was stirred at room temperature for 3 h. LCMS indicated completion of the reaction. The reaction solution was diluted with water, and then extracted with ethyl acetate. The organic phase was concentrated to provide the crude product. The obtained compound was dissolved in dichloromethane (4 mL), and then trifluoroacetic acid (2 mL) was added. The mixture was allowed to react for 1 h, then concentrated, and the crude product was purified to obtain the target compound N-((S)-1-(((S)-5-amino-6-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-6-oxohexyl)amino)-3-methyl-1-oxobutan-2-yl)-6-(2-(methanesulfonyl)pyrimidin-5-yl)hex-5-ynamide (DL-022) (88 mg).

LCMS (ESI) [M+H]⁺ = 972.9;
¹H NMR (400 MHz, DMSO-d6) δ 9.10 (s, 2H), 8.72 (t, *J =* 5.5 Hz, 1H), 8.23 (d, *J =* 8.4 Hz, 1H), 8.17 (t, *J =* 5.4 Hz, 1H), 8.11 (s, 3H, NH2-TFA), 7.97 (t*, J* = 5.6 Hz, 1H), 7.93 - 7.86 (m, 2H), 7.32 (s, 1H), 6.53 (s, 1H), 5.44 (s, 2H), 5.29 (s, 2H), 4.14 - 4.02 (m, 2H), 3.85 - 3.83 (m, 2H), 3.41 (s, 3H), 3.32 (d, *J =* 6.2 Hz, 2H), 3.24 - 3.16 (m, 2H), 3.11 - 2.94 (m, 2H), 2.55 (s, 3H), 2.35 - 2.30 (m, 3H), 1.92 - 1.69 (m, 10H), 1.43 - 1.29 (m, 4H), 0.88 (t, *J=* 7.3 Hz, 3H), 0.82 (d, *J =* 6.7 Hz, 6H).

### Example 2.23 : (S)-6-amino-N-(2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)-2-((S)-2-isopropyl-35-(4-(2-(methanesulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (DL-023)

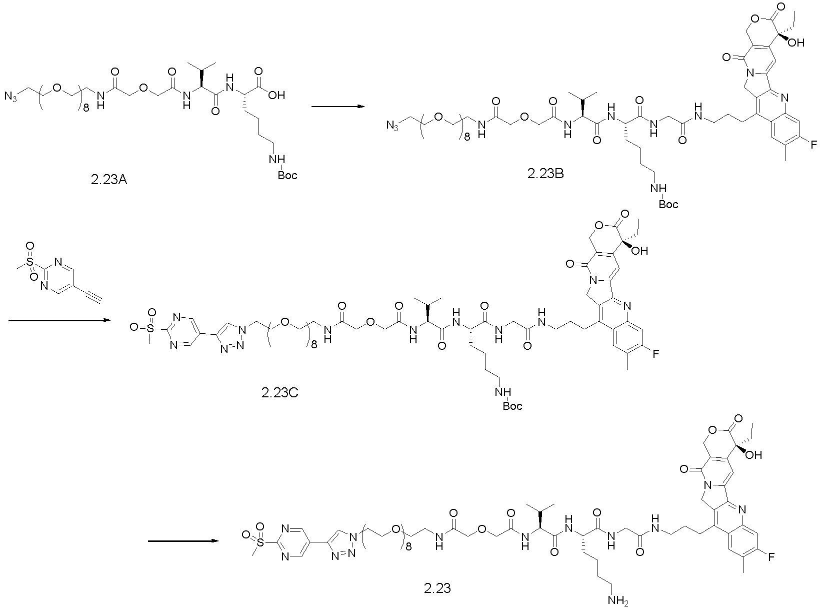

### Step 1:

To a solution of compound B1.13 (46 mg) in N,N-dimethylformamide (3 mL), were successively added compound 2.23A (100 mg), triethylamine (50 µl), HOBT (16 mg) and EDCI (23 mg), and then the mixture was allowed to react at 35°C for 4 h. Water (30 mL) was added to the reaction solution, and then extracted with ethyl acetate (30 mL×2). The organic phase was dried over anhydrous sodium sulfate, and concentrated to obtain the target compound 2.23B (105 mg).

LCMS (ESI) [M+H]⁺: 1358.4;

### Step 2:

Compound 2.23B (105 mg) and 2-methanesulfonyl-5-ethynylpyrimidine (45 mg) were dissolved in DMSO-water (2 ml, 4:1), to which was added cupprous bromide (50 mg). The reaction solution was stirred at room temperature for 2 h, and then ethyl acetate (100ml) was added. The resultant solution was washed with water (100 ml x 3) and dried, and then the organic solvent was removed under reduced pressure. The crude product was separated by silica gel column chromatography to obtain the target product 2.23C (84 mg), MS (*m*/*z*): 1540.8 [M + H]⁺;

### Step 3:

Compound 2.23C was dissolved in a mixed solution of trifluoroacetic acid and dichloromethane (volume ratio: 1:2; 4 mL), and then the reaction solution was stirred at room temperature for 30 min. LCMS indicated completion of the reaction. The reaction solution was concentrated to obtain a crude product, which was purified over preparative chromatography (0.01% TFA in water, MeCN) to provide the target compound (S)-6-amino-N-(2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)-2-((S)-2-isopropyl-35-(4-(2-(methanesulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (DL-023, 8 mg, trifluoroacetate, yield 5%).

LCMS (ESI) [M+H]⁺ = 1440.8;
¹H NMR (400 MHz, DMSO-d6) δ 9.49 (s, 2H), 8.91 (s, 1H), 8.24 - 8.18 (m, 3H), 8.01 (t, *J =* 5.6 Hz, 1H), 7.96 (t, *J =* 5.5 Hz, 1H), 7.90 - 7.84 (m, 2H), 7.63 (s, 3H, TFA salt), 7.32 (s, 1H), 5.44 (s, 2H), 5.30 (s, 2H), 4.66 (t, *J* = 5.1 Hz, 2H), 4.21 (dd, *J =* 15.2, 7.9 Hz, 2H), 3.97 (s, 2H), 3.93 (s, 2H), 3.89 (t, *J =* 5.1 Hz, 2H), 3.75 - 3.72 (m, 2H), 3.56 - 3.54 (m, 2H), 3.54 - 3.38 (m, 30H), 3.33 - 3.13 (m, 5H), 2.75 (d, *J* = 7.1 Hz, 2H), 2.52 (s, 3H), 2.02 - 1.94 (m, 1H), 1.91 - 1.81 (m, 3H), 1.71 - 1.65 (m, 1H), 1.58 - 1.46 (m, 3H), 1.38 - 1.29 (m, 2H), 0.88 (t, *J =* 7.3 Hz, 3H), 0.81 - 0.75 (m, 6H).

### Example 2.24: N-((S)-1-(((S)-6-(dimethylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (DL-024)

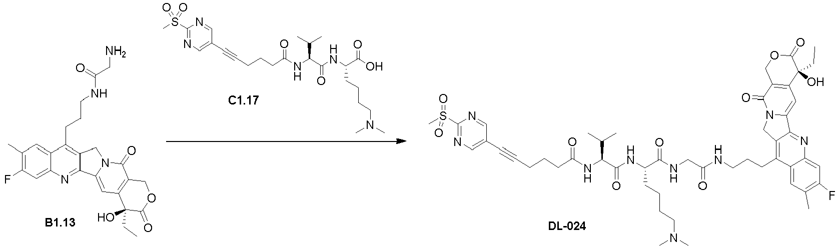

To a solution of compound B1.13 (40 mg, 0.081 mmol) and compound C1.17 (42 mg, 0.081 mmol) in N,N-dimethylformamide (2 mL), were successively added PyBOP (41 mg, 0.081) and DIPEA (20 mg, 0.16 mmol), and then the reaction solution was stirred for 2 h at room temperature. LCMS indicated completion of the reaction. The reaction solution was purified by preparative HPLC (acetonitrile/0.05% trifluoroacetic acid in water), to provide compound N-((S)-1-(((S)-6-(dimethylamino)-1-((2-((3-((S)-4-ethyl-8-fluoro-4-hydroxy-9-methyl- 3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (11.54 mg, yield 14%) as a yellow solid.

LCMS (ESI) [M+H]⁺ = 1000.6;
¹H NMR (400 MHz, DMSO-d6) δ 9.42 (s, 1H), 9.08 (s, 2H), 8.27 - 8.18 (m, 2H), 8.14 (d, *J* = 6.9 Hz, 1H), 7.96 (t, *J =* 6.9 Hz, 1H), 7.91 - 7.77 (m, 2H), 7.31 (s, 1H), 5.44 (s, 2H), 5.29 (s, 2H), 4.15 - 4.12 (m, 1H), 3.81 - 3.65 (m, 3H), 3.41 (s, 3H), 3.33 - 3.25 (m, 2H), 3.22 - 3.15 (m, 2H), 3.03 - 2.95 (m, 2H), 2.77 - 2.71 (m, 6H), 2.38 - 2.23 (m, 3H), 1.92 - 1.81 (m, 7H), 1.78 - 1.73 (m, 2H), 1.62 - 1.56 (m, 3H), 1.38 - 1.29 (m, 2H), 0.92 - 0.84 (m, 3H), 0.84 - 0.72 (m, 6H).

### Example 2.25: N-((S)-1-(((S)-6-(diethylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (DL-025)

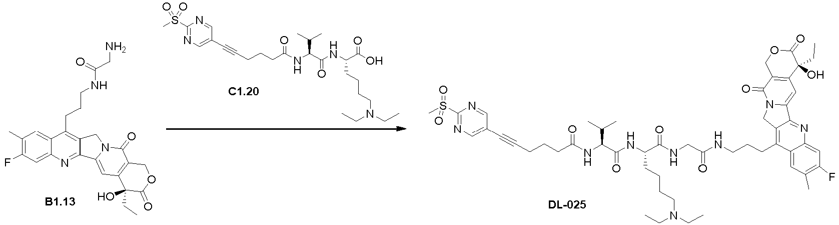

To a solution of compound C1.20 (30 mg, 0.054 mmol) and compound B1.13 (27 mg, 0.054 mmol) in N,N-dimethylformamide (2 mL), were successively added HBTU(22 mg, 0.054 mmol) and N,N-diisopropylethylamine (17 mg, 0.135 mmol). After addition, the reaction solution was stirred for 30 min at room temperature. LCMS indicated completion of the reaction. The reaction solution was directly purified by preparative chromatography (0.01% trifluoroacetic acid in water, acetonitrile), to provide the target compound N-((S)-1-(((S)-6-(diethylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (23 mg, yield 42%) as a yellow solid.

LCMS (ESI) [M+H]⁺ = 1028.5;
¹H NMR (400 MHz, DMSO-d6) δ 9.08 (s, 2H), 9.02 (s, 1H), 8.21 (d, *J =* 7.4 Hz, 2H), 8.13 (d, *J =* 7.1 Hz, 1H), 7.97 (t, *J =* 5.4 Hz, 1H), 7.87 (d, *J =* 10.6 Hz, 2H), 7.31 (s, 1H), 6.52 (s, 1H), 5.44 (s, 2H), 5.30 (s, 2H), 4.28 - 4.18 (m, 1H), 4.14 (t, *J =* 7.7 Hz, 1H), 3.80 - 3.65 (m, 2H), 3.41 (s, 3H), 3.31 - 3.24 (m, 2H), 3.22 - 3.15 (m, 2H), 3.12 - 3.06 (m, 4H), 3.02 - 2.91 (m, 2H), 2.36 - 2.23 (m, 2H), 1.95 - 1.68 (m, 9H), 1.68 - 1.49 (m, 4H), 1.38 - 1.28 (m, 2H), 1.13 (t, *J* = 7.2 Hz, 6H), 0.86 (t, *J =* 7.2 Hz, 3H),0.79 - 0.76 (m, 6H).

### Example 2.26: N-((S)-1-(((S)-6-(dipropylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (DL-026)

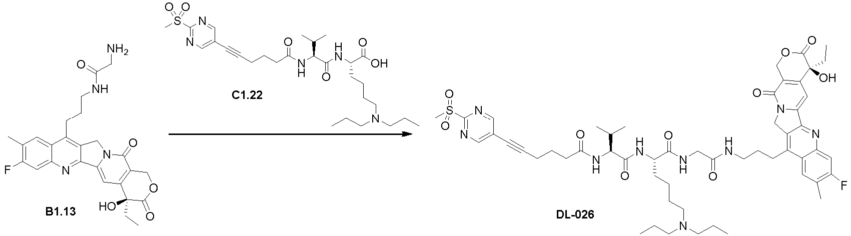

Compound C1.22 (31 mg, 0.054 mmol) and compound B1.13 (27 mg, 0.054 mmol) were dissolved in N,N-dimethylformamide (2 mL), to which were then added HBTU (22 mg, 0.054 mmol) and N,N-diisopropylethylamine (17 mg, 0.135 mmol) sequentially. The reaction solution was stirred for 30 min at room temperature. LCMS indicated completion of the reaction. The reaction solution was directly purified by preparative chromatography (0.01% trifluoroacetic acid in water, acetonitrile), to provide the target compound N-((S)-1-(((S)-6-(dipropylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (25 mg, yield 45%) as a yellow solid.

LCMS (ESI) [M+H]⁺ = 1056.6;
H NMR (400 MHz, DMSO-d6) δ 9.09 (s, 2H), 8.27 - 8.19 (m, 2H), 8.13 (d, *J =* 7.3 Hz, 1H), 7.97 (t, *J =* 5.3 Hz, 1H), 7.91 - 7.84 (m, 2H), 7.31 (s, 1H), 6.53 (s, 1H), 5.44 (s, 2H), 5.29 (s, 2H), 4.24 (d, *J =* 6.7 Hz, 1H), 4.13 (t, *J* = 7.8 Hz, 1H), 3.73 (dd, *J* = 14.7, 5.9 Hz, 2H), 3.41 (s, 3H), 3.28 (d, *J* = 6.4 Hz, 2H), 3.19 (t, *J* = 7.6 Hz, 2H), 2.99 (br, 6H), 2.33 - 2.27 (m, 2H), 1.95 - 1.70 (m, 9H), 1.62 - 1.57 (m, 8H), 1.33 - 1.30 (m, 2H), 0.96 - 0.82 (m, 9H), 0.82 - 0.73 (m, 6H).

### Example 2.27: N-((S)-1-(((S)-6-(diethylamino)-1-((2-(((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide

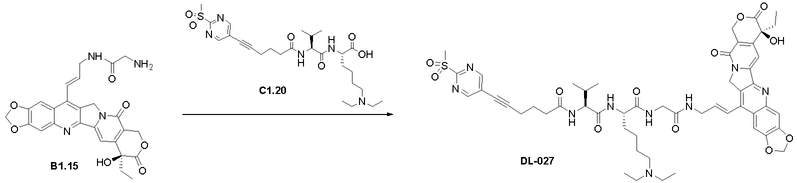

To a solution of compound B1.15 (40 mg, 0.074 mmol) and compound C1.20 (40 mg, 0.072 mmol) in N,N-dimethylformamide (4 mL), were sequentially added HBTU (30 mg, 0.079 mmol) and DIPEA (26 mg, 0.2 mmol). The reaction solution was stirred for 1 h at room temperature. LCMS indicated completion of the reaction. The reaction solution was purified by preparative HPLC (acetonitrile/0.05% trifluoroacetic acid in water), to provide compound N-((S)-1-(((S)-6-(diethylamino)-1-((2-(((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (11.1 mg, yield 14%).

LCMS (ESI) [M+H]⁺ =1038.6;
¹H NMR (400 MHz, DMSO-d6) δ 9.10 (s, 2H), 9.02 (s, 1H, TFA), 8.33 - 8.25 (m, 2H), 8.12 (d, *J =* 7.3 Hz, 1H), 7.92 (d, *J* = 8.4 Hz, 1H), 7.68 (d, *J* = 4.1 Hz, 1H), 7.52 (d, *J* = 2.8 Hz, 1H), 7.26 (s, 1H), 7.15 (d, *J* = 16.2 Hz, 1H), 6.52 - 6.50 (m, 2H), 6.30 (s, 2H), 5.43 (s, 2H), 5.28 - 5.24 (m, 2H), 4.27 (d, *J =* 6.2 Hz, 2H), 4.12 (br s, 2H), 3.81 (br s, 2H), 3.41 (s, 3H), 3.11 - 3.07 (m, 6H), 2.97 (br s, 2H), 2.53 - 2.50 (m, 1H), 2.39 - 2.24 (m, 4H), 1.85 - 1.79 (m, 8H), 1.16 (t, *J =* 6.4 Hz, 6H), 0.88 (t, *J =* 6.4 Hz, 3H), 0.78 (d, *J =* 6.4 Hz, 6H).

### Example 2.28: (S)-6-(dimethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide

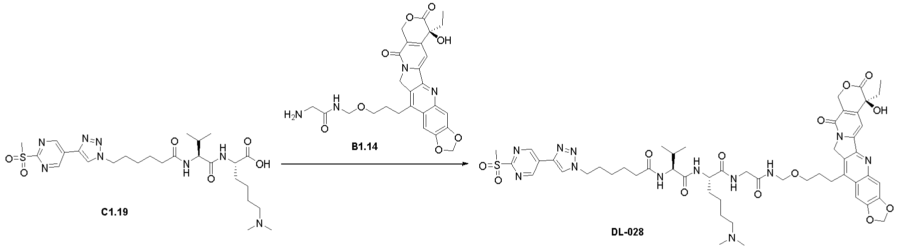

Compound C1.19 (40 mg, 0.06 mmol) was dissolved in N,N-dimethylformamide (1.5 mL), to which were successively added HBTU (26 mg, 0.06 mmol), B1.14 (36 mg, 0.06mmol) and DIPEA (66 mg, 0.17 mmol), and then the reaction was stirred for 1 h at room temperature. LCMS indicated completion of the reaction. The reaction solution was separated and purified by preparative chromatography (acetonitrile/0.05% trifluoroacetic acid in water), to provide the target compound (S)-6-(dimethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (13.88 mg, TFA salt, yield 16%) as a maize-yellow solid.

LCMS (ESI) [M+H]⁺ = 1113.7;
¹H NMR (400 MHz, DMSO-d6) δ 9.46 (s, 2H), 9.29 (s, 1H, TFA salt), 8.92 (s, 1H), 8.63 (t, *J* = 6.6 Hz, 1H), 8.18 (br s, 1H), 8.03 (d, *J =* 7.6 Hz, 1H), 7.81 (d, *J =* 8.3 Hz, 1H), 7.59 (s, 1H), 7.50 (s, 1H), 7.24 (s, 1H), 6.48 (s, 1H), 6.28 (s, 2H), 5.42 (s, 2H), 5.24 (s, 2H), 4.64 - 4.55 (m, 2H), 4.46 (t, *J =* 7.0 Hz, 2H), 4.28 - 4.20 (m, 1H), 4.15 - 4.09 (m, 1H), 3.76 - 3.71 (m, 4H), 3.53 - 3.48 (m, 4H), 3.44 (s, 3H), 3.10 - 3.08 (m, 2H), 2.97 - 2.95 (m, 2H), 2.75 (d, *J =* 4.9 Hz, 6H), 2.20 - 2.09 (m, 2H), 1.92 - 1.83 (m, 5H), 1.68 - 1.65 (m, 1H), 1.63 - 1.50 (m, 5H), 0.90 - 0.74 (m, 9H).

### Example 2.29 : (S)-6-(diethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide

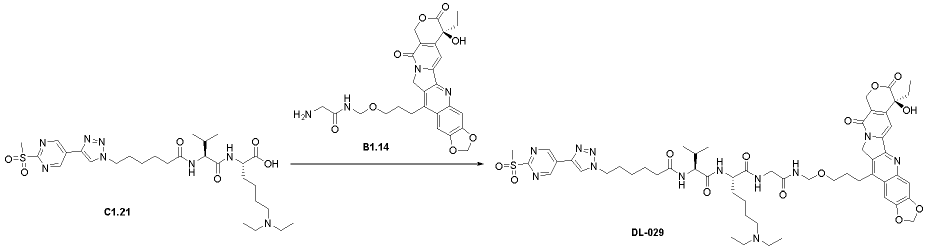

Compound C1.21 (58 mg, 0.09 mmol) and compound B1.14 (50 mg, 0.09 mmol) were dissolved in DMF (1 mL), to which were then added HBTU (35 mg, 0.09 mmol) and N,N-diisopropylethylamine (30 mg, 0.23 mmol). After addition, the reaction solution was stirred for 1 h at room temperature. LCMS indicated completion of the reaction. The reaction solution was purified by preparative chromatography (0.01% TFA in water, MeCN), to provide the target compound (S)-6-(diethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (14 mg, yield 13%) as a yellow solid.

LCMS (ESI) [M+H]⁺ = 1141.7;
1H NMR (400 MHz, DMSO) δ 9.46 (s, 2H), 8.98 (s, 1H, TFA), 8.93 (s, 1H), 8.64 (t, *J =* 6.5 Hz, 1H), 8.18 (d, *J=* 5.7 Hz, 1H), 8.03 (d, *J =* 7.4 Hz, 1H), 7.81 (d, *J =* 8.5 Hz, 1H), 7.58 (s, 1H), 7.50 (d, *J =* 3.1 Hz, 1H), 7.24 (s, 1H), 6.52 (s, 1H), 6.28 (s, 2H), 5.42 (s, 2H), 5.23 (s, 2H), 4.62 - 4.55 (m, 2H), 4.46 (t, *J =* 6.9 Hz, 2H), 4.27 - 4.23 (m, 1H), 4.11 - 4.09 (m, 1H), 3.74 (d, *J =* 5.8 Hz, 2H), 3.49 (t, *J =* 5.8 Hz, 2H), 3.44 (s, 3H), 3.13 - 3.06 (m, 6H), 3.01 - 2.93 (m, 2H), 2.22 - 2.07 (m, 2H), 1.91 - 1.83 (m, 6H), 1.70- 1.51 (m, 5H), 1.33 - 1.21 (m, 6H), 1.16 (t, *J =* 7.2 Hz, 6H), 0.87 (t, *J* = 7.4 Hz, 3H), 0.82 - 0.76 (m, 6H).

### Example 2.30: (S)-6-(dipropylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide

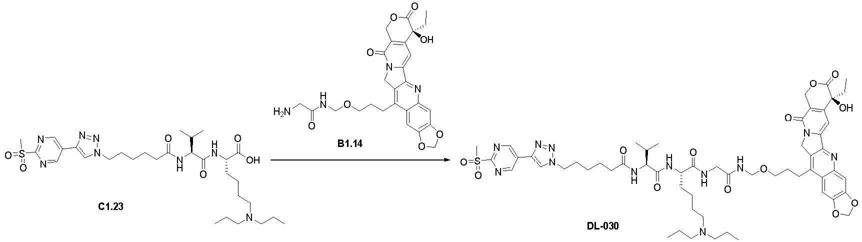

Compound C1.23 (61 mg, 0.09 mmol) and compound B1.14 (50 mg, 0.09 mmol) were dissolved in DMF (1 mL), to which were then added HBTU (35 mg, 0.09 mmol) and N,N-diisopropylethylamine (30 mg, 0.23 mmol). After addition, the reaction solution was stirred for 1 h at room temperature. LCMS indicated completion of the reaction. The reaction solution was concentrated to afford the crude product, which was purified by preparative chromatography (0.01% TFA in water, MeCN), to provide the target compound (S)-6-(dipropylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (21 mg, yield 19%) as a yellow solid.

LCMS (ESI) [M+H]⁺ = 1169.8;
¹H NMR (400 MHz, DMSO) δ 9.46 (s, 2H), 9.01 (s, 1H, TFA), 8.93 (s, 1H), 8.64 (t, *J =* 6.4 Hz, 1H), 8.19 (t, *J*= 5.4 Hz, 1H), 8.02 (d, *J =* 7.3 Hz, 1H), 7.81 (d, *J =* 8.3 Hz, 1H), 7.58 (s, 1H), 7.51 (s, 1H), 7.24 (s, 1H), 6.50 (s, 1H), 6.28 (s, 2H), 5.42 (s, 2H), 5.23 (s, 2H), 4.60 - 4.58 (m, 2H), 4.46 (t, *J =* 6.9 Hz, 2H), 4.24 (d, *J =* 6.0 Hz, 1H), 4.13 - 4.09 (m, 1H), 3.74 (d, *J =* 5.7 Hz, 2H), 3.49 (t, *J =* 5.8 Hz, 2H), 3.44 (s, 3H), 3.10 (br s, 2H), 3.02 - 2.95 (m, 6H), 2.19 - 2.15 (m, 2H), 1.88 - 1.86 (m, 6H), 1.71 - 1.69 (m, 1H), 1.61 - 1.56 (m, 8H), 1.28 - 1.26 (m, 6H), 0.88 - 0.83 (m, 9H), 0.81 - 0.78 (m, 6H).

### Example 2.31: (S)-6-(dimethylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide

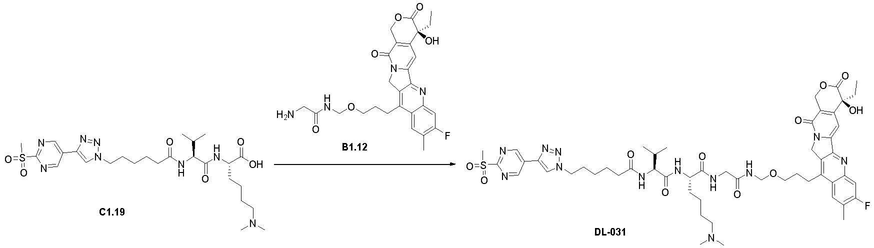

Compound C1.19 (113 mg, 0.19 mmol) and compound B1.12 (100 mg, 0.19 mmol) were dissolved in DMF (1 mL), to which were then added HBTU (72 mg, 0.19 mmol) and N,N-diisopropylethylamine (61 mg, 0.48 mmol). After addition, the reaction solution was stirred for 1 h at room temperature. LCMS indicated completion of the reaction. The reaction solution was purified by preparative chromatography (0.01% TFA in water, MeCN), to provide the target compound (S)-6-(dimethylamino)-N-(2-(((3-((S)-4-ethyl-8-fluoro-4-hydroxy-9-methyl- 3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (16 mg, yield 8%) as a white solid.

LCMS (ESI) [M+H]⁺ = 1101.6;
¹H NMR (400 MHz, DMSO) δ 9.46 (s, 2H), 8.93 (s, 1H), 8.63 (t, *J =* 6.3 Hz, 1H), 8.20 - 8.28 (m, 2H), 8.04 (d, *J* = 7.2 Hz, 1H), 7.88 (d, *J =* 10.8 Hz, 1H), 7.81 (d, *J* = 8.3 Hz, 1H), 7.31 (s, 1H), 6.53 (s, 1H), 5.44 (s, 2H), 5.29 (s, 2H), 4.63 - 4.54 (m, 2H), 4.46 (t, *J =* 7.0 Hz, 2H), 4.29 - 4.19 (m, 1H), 4.15 - 4.08 (m, 1H), 3.73 (d, *J =* 6.9 Hz, 2H), 3.50 (t, *J =* 5.8 Hz, 2H), 3.44 (s, 3H), 3.24 - 3.14 (m, 2H), 3.01 - 2.91 (m, 2H), 2.72 (s, 6H), 2.53 (s, 3H), 2.23 - 2.07 (m, 2H), 1.93 - 1.84 (m, 6H), 1.72 - 1.64 (m, 1H), 1.61 - 1.45 (m, 6H), 1.34 - 1.22 (m, 4H), 0.87 (t, *J* = 7.4 Hz, 3H), 0.82 - 0.76 (m, 6H).

### Example 2.32: (S)-6-(diethylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide

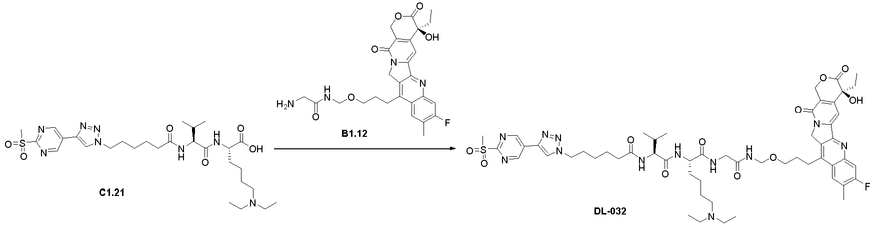

Compound C1.21 (59 mg, 0.10 mmol) and compound B1.12 (50 mg, 0.10 mmol) were dissolved in DMF (1 mL), to which were then added HBTU (36 mg, 0.10 mmol) and N,N-diisopropylethylamine (31 mg, 0.24 mmol). After addition, the reaction solution was stirred for 1 h at room temperature. LCMS indicated completion of the reaction. The reaction solution was purified by preparative chromatography (0.01% aqueous formic acid solution, MeCN), to provide the target compound (S)-6-(diethylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (33.8 mg, yield 32%) as a white solid.

LCMS (ESI) [M+H]⁺ = 1129.7;
¹H NMR (400 MHz, DMSO) δ 9.46 (s, 2H), 8.93 (s, 1H), 8.61 (t, *J =* 6.4 Hz, 1H), 8.29 (s, 1H), 8.24 - 8.15 (m, 2H), 8.00 (d, *J =* 7.2 Hz, 1H), 7.85 (d, *J =* 10.5 Hz, 2H), 7.31 (s, 1H), 5.44 (s, 2H), 5.26 (s, 2H), 4.63 - 4.55 (m, 2H), 4.46 (t, *J =* 6.8 Hz, 2H), 4.23 - 4.18 (m, 1H), 4.11 (d, *J =* 7.9 Hz, 1H), 3.77 - 3.69 (m, 2H), 3.50 (t, *J =* 5.9 Hz, 2H), 3.44 (s, 3H), 3.17 - 3.15 (m, 3H), 2.61 (q, *J =* 6.7 Hz, 4H), 2.22 - 2.10 (m, 2H), 1.92 - 1.84 (m, 6H), 1.68 (d, *J =* 6.7 Hz, 1H), 1.57 - 1.48 (m, 3H), 1.39 (d, *J* = 5.9 Hz, 2H), 1.30 - 1.22 (m, 4H), 0.97 (t, *J =* 6.9 Hz, 6H), 0.88 (t, *J =* 7.3 Hz, 3H), 0.82 - 0.77 (m, 6H).

### Example 2.33: (S)-6-(dipropylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide

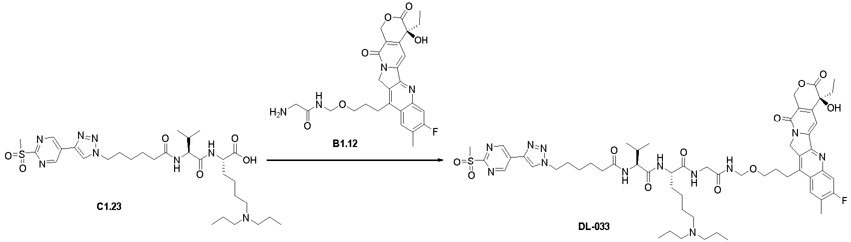

Compound C1.23 (62 mg, 0.10 mmol) and compound B1.12 (50 mg, 0.10 mmol) were dissolved in DMF (1 mL), to which were then added HBTU (36 mg, 0.10 mmol) and N,N-diisopropylethylamine (31 mg, 0.24 mmol). After addition, the reaction solution was stirred for 1 h at room temperature. LCMS indicated completion of the reaction. The reaction solution was purified by preparative chromatography (0.01% TFA in water, MeCN), to provide the target compound (S)-6-(dipropylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (35.2 mg, yield 32%) as a yellow solid.

LCMS (ESI) [M+H]⁺ = 1157.8;
¹H NMR (400 MHz, DMSO) δ 9.46 (s, 2H), 9.05 (s, 1H, TFA), 8.93 (s, 1H), 8.64 (t, *J* = 6.7 Hz, 1H), 8.20 (d, *J* = 7.4 Hz, 2H), 8.03 (d, *J =* 7.2 Hz, 1H), 7.88 (d, *J =* 10.8 Hz, 1H), 7.81 (d, *J* = 8.4 Hz, 1H), 7.32 (s, 1H), 6.54 (s, 1H), 5.44 (s, 2H), 5.28 (s, 2H), 4.62 - 4.55 (m, 2H), 4.46 (t, *J =* 7.0 Hz, 2H), 4.24 (m, 1H), 4.13 - 4.07 (m, 1H), 3.74 (m, 2H), 3.50 (m, 2H), 3.44 (s, 3H), 3.23 - 3.16 (m, 2H), 3.01 - 2.95 (m, 6H), 2.50 (s, 3H), 2.22 - 2.09 (m, 2H), 1.95 - 1.84 (m, 8H), 1.67 - 1.56 (m, 9H), 1.33 - 1.23 (m, 6H), 0.91 - 0.86 (m, 9H), 0.82 - 0.77 (m, 6H).

### Example 2.34 : N-((11S,14S)-11-(4-(diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide (DL-034)

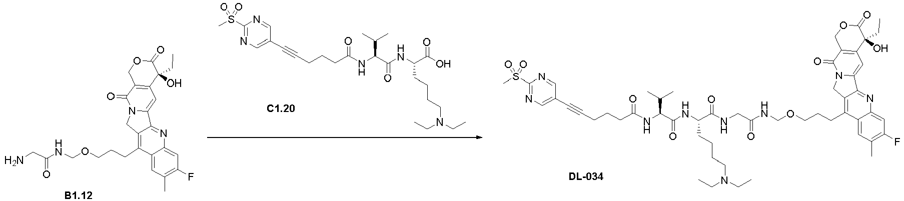

Compound (S)-2-amino-N-((3-(4-ethyl-8-fluoro-4-hydroxy-9-methyl-3,14-dioxy-3,4,12,14-tetrahydro-1H-pyrano[3',4':6,7]indolo[1,2-b]quinolin-11-yl)propoxy)methyl)acetamide (B1.12, 30 mg, 0.057 mmol) and N⁶,N⁶-diethyl-N²-((6-(2-(methanesulfonyl)pyrimidin-5-yl)hexadecyl-5-ynoyl)-L-propionyl)-L-lysine (C1.20, 31 mg, 0.057 mmol) were dissolved in DMF (1 mL), to which were then successively added HBTU(22 mg, 0.057 mmol) and N,N-diisopropylethylamine (18 mg, 0.143 mmol). After addition, the reaction solution was stirred for 1 h at room temperature. LCMS indicated completion of the reaction. The reaction solution was purified by preparative chromatography (0.01% TFA in water, MeCN), to provide the target compound N-((11S,14S)-11-(4-(diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide trifluoroacetate (3.18 mg, yield 5%) as a yellow solid.

LCMS (ESI) [M+H]⁺ = 1058.7;
1H NMR (400 MHz, DMSO-d6) δ 9.10 (s, 2H), 8.65 (t, *J* = 6.5 Hz, 1H), 8.21 - 8.19 (m, 2H), 8.09 (d, *J =* 7.3 Hz, 1H), 7.92 (d, *J =* 8.6 Hz, 1H), 7.88 (d, *J* = 10.8 Hz, 1H), 7.32 (s, 1H), 6.53 (s, 1H), 5.44 (s, 2H), 5.28 (s, 2H), 4.64 - 4.56 (m, 2H), 4.29 - 4.22 (m, 1H), 4.15 (t, *J =* 7.4 Hz, 1H), 3.74 (d, *J =* 4.6 Hz, 2H), 3.51 - 3.49 (m, 2H), 3.24 - 3.18 (m, 2H), 3.13 - 3.07 (m, 4H), 3.01 - 2.95 (m, 2H), 2.55 (s, 3H), 2.40 - 2.31 (m, 3H), 1.99 - 1.74 (m, 8H), 1.65 - 1.49 (m, 4H), 1.38 - 1.25 (m, 2H), 1.16 (t, *J =* 7.2 Hz, 6H), 0.89 - 0.79 (m, 9H).

### Example 2.35: N-((11S,14S)-11-(4-(dipropylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide (DL-035)

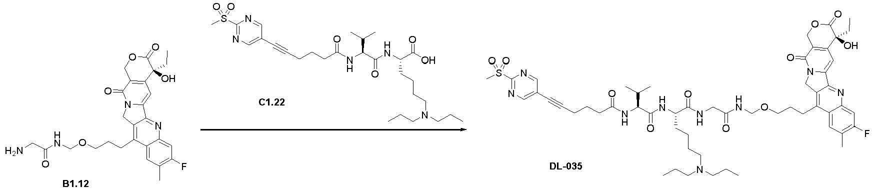

Compound (S)-2-amino-N-((3-(4-ethyl-8-fluoro-4-hydroxy-9-methyl-3,14-dioxy-3,4,12,14-tetrahydro-1H-pyrano[3',4':6,7]indolo[1,2-b]quinolin-11-yl)propoxy)methyl)acetamide (B1.12, 30 mg, 0.057 mmol) and N2-((6-(2-(methanesulfonyl)pyrimidin-5-yl)hexan-5-ynoyl)-L-propionyl)-N6,N6-dipropyl-L-lysine (C1.22, 33 mg, 0.057 mmol) were dissolved in DMF (1 mL), to which were then successively added HBTU(22 mg, 0.057 mmol) and N,N-diisopropylethylamine (18 mg, 0.143 mmol). After addition, the reaction solution was stirred for 1 h at room temperature. LCMS indicated completion of the reaction. The reaction solution was purified by preparative chromatography (0.01% TFA in water, MeCN), to provide the target compound N-((11S,14S)-11-(4-(dipropylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide (DL-035) (10.65 mg, yield 16%) as a yellow solid.

LCMS (ESI) [M+H]⁺ = 1086.7;
¹H NMR (400 MHz, DMSO-d6) δ 9.10 (s, 2H), 8.65 (t, *J* = 6.2 Hz, 1H), 8.28 - 8.16 (m, 2H), 8.08 (d, *J* = 6.8 Hz, 1H), 7.92 (d, *J* = 8.1 Hz, 1H), 7.88 (d, *J* = 10.9 Hz, 1H), 7.32 (s, 1H), 6.53 (s, 1H), 5.44 (s, 2H), 5.29 (s, 2H), 4.63 - 4.52 (m, 2H), 4.31 - 4.19 (m, 1H), 4.19 - 4.08 (m, 1H), 3.74 (d, *J =* 4.9 Hz, 2H), 3.50 (t, *J* = 5.2 Hz, 2H), 3.41 (s, 3H), 3.24 - 3.18 (m, 2H), 3.03 - 2.94 (m, 6H), 2.55 (s, 3H), 2.46 - 2.24 (m, 3H), 2.05 - 1.67 (m, 8H), 1.66 - 1.51 (m, 8H), 1.36 - 1.22 (m, 4H), 0.91 - 0.80 (m, 15H).

### Example 2.36: N-((11S,14S)-11-(4-(diethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-036)

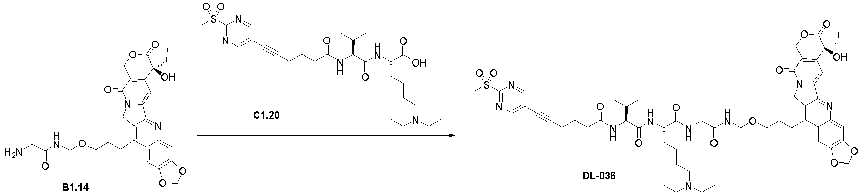

Compound C1.20 (40 mg, 0.075 mmol) and compound B1.14 (41 mg, 0.075 mmol) were dissolved in DMF (1 mL), to which were then added HOBt (15.2 mg, 0.113 mmol) and 1-(3-dimethylaminopropyl)-3-ethylcarbodiimide hydrochloride (21.5 mg, 0.113 mmol), followed by addition of triethylamine (23 mg, 0.225 mmol). After addition, the reaction solution was stirred for 16 h at room temperature. LCMS detection indicated completion of the reaction. The reaction solution was concentrated to obtain a crude product, which was purified by preparative chromatography (0.01% TFA in water, MeCN) to obtain the target compound N-((11S,14S)-11-(4-(diethylamino)butyl)-1-((S)-7-ethyl-7-hydroxy-8,11-dioxo-7,8,11,13-tetrahydro -10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methanesulfonyl)pyrimidin-5-yl)hex-5-ynamide (16 mg, yield 20%) as a yellow solid.

LCMS (ESI) [M+H]⁺ = 1070.6;
¹H NMR (400 MHz, DMSO-d6) δ 9.13 - 9.08 (m, 2H), 9.01 (s, 1H), 8.64 (t, *J =* 6.5 Hz, 1H), 8.20 (t, *J =* 5.7 Hz, 1H), 8.09 (d, *J* = 7.4 Hz, 1H), 7.92 (d, *J =* 8.4 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.65 - 4.53 (m, 2H), 4.26 (d, *J =* 6.5 Hz, 1H), 4.20 - 4.10 (m, 1H), 3.74 (d, *J* = 5.5 Hz, 2H), 3.50 (t, *J* = 5.8 Hz, 2H), 3.41 (s, 3H), 3.14 - 3.07 (m, 6H), 2.98 (s, 2H), 2.55 (d, *J* = 7.3 Hz, 2H), 2.41 - 2.29 (m, 2H), 2.03 - 1.89 (m, 2H), 1.89 - 1.77 (m, 7H), 1.61 - 1.56 (m, 2H), 1.32 (s, 2H), 1.16 (t, *J =* 7.2 Hz, 6H), 0.91 - 0.78 (m, 9H).

### Example 2.37 : N-((11S,14S)-11-(4-(di-n-propylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methanesulfonyl)pyrimidin-5-yl)hex-5-ynamide (DL-037)

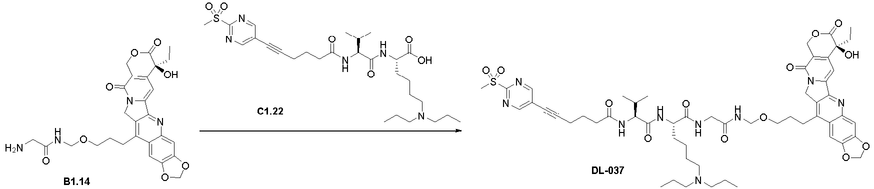

Compound C1.22 (43 mg, 0.074 mmol) and compound B1.14 (40 mg, 0.075 mmol) were dissolved in N,N-dimethylformamide (1 mL), to which were then added HBTU (28 mg, 0.075 mmol) and N,N-diisopropylethylamine (24 mg, 0.187 mmol). After addition, the reaction solution was stirred for 1 h at room temperature. LCMS detection indicated completion of the reaction. The reaction solution was directly purified by preparative chromatography (0.01% TFA in water, MeCN), to provide the trifluoroacetate of the target compound (8.5 mg, yield 10%) as a yellow solid.

LCMS (ESI) [M+H]⁺ = 1098.6;
¹H NMR (400 MHz, DMSO-d6) δ 9.10 (s, 2H), 9.03 (s, 1H), 8.64 (t, *J =* 6.4 Hz, 1H), 8.19 (t, *J =* 5.9 Hz, 1H), 8.08 (d, *J =* 7.4 Hz, 1H), 7.92 (d, *J =* 8.5 Hz, 1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66 - 4.52 (m, 2H), 4.31 - 4.21 (m, 1H), 4.19 - 4.10 (m, 1H), 3.74 (d, *J =* 5.5 Hz, 2H), 3.49 - 3.48 (m, 2H), 3.40 (s, 3H), 3.15 - 3.06 (m, 2H), 3.02 - 2.95 (m, 6H), 2.59 - 2.52 (m, 3H), 2.41 - 2.29 (m, 2H), 2.05 - 1.90 (m, 2H), 1.91 - 1.77 (m, 6H), 1.63 - 1.57 (m, 6H), 1.31 - 1.29 (m, 2H), 0.92 - 0.80 (m, 15H).

Using the same procedure in this Example, the reaction solution was directly purified by preparative chromatography (0.1% formic acid aqueous solution, acetonitrile) and lyophilized to obtain the formate of the target compound (32.6 mg, yield 39%) as a white solid.

LCMS (ESI) [M+H]⁺ = 1098.6;
¹H NMR (400 MHz, DMSO-d₆) δ 9.09 (s, 2H), 8.56 (t, *J* = 6.5 Hz, 1H), 8.17 (s, 1H), 8.14 (t, *J* = 5.7 Hz, 1H), 7.97 (d, *J* = 7.3 Hz, 1H), 7.89 (d, *J* = 8.6 Hz, 1H), 7.58 (s, 1H), 7.50 (s, 1H), 7.24 (s, 1H), 6.45 (s, 1H), 6.28 (s, 2H), 5.48 - 5.35 (m, 2H), 5.24 (s, 2H), 4.64 - 4.54 (m, 2H), 4.23 - 4.07 (m, 2H), 3.79 - 3.66 (m, 2H), 3.50 (t, *J* = 5.9 Hz, 2H), 3.40 (s, 3H), 3.14 - 3.08 (t, *J* = 8.2 Hz, 2H), 2.59 - 2.52 (m, 2H), 2.36 - 2.27 (m, 8H), 2.00 - 1.76 (m, 7H), 1.72- 1.62 (m, 1H), 1.58- 1.48 (m, 1H), 1.40 - 1.20 (m, 8H), 0.88 - 0.77 (m, 15H).

DL-037 formate (16mg) was purified by HPLC (0.1% ammonium bicarbonate aqueous solution, acetonitrile) and lyophilized to obtain a free target compound (DL-037, 10 mg, yield 65%) as a white solid.

LCMS (ESI) [M+H]⁺ = 1098.6;
¹H NMR (400 MHz, DMSO-d6) δ 9.10 (s, 2H), 8.61 (t, *J* = 6.4 Hz, 1H), 8.21 (t, *J* = 5.6 Hz, 1H), 8.04 (d, J = 7.2 Hz, 1H), 7.95 (d, *J* = 8.5 Hz, 1H), 7.53 (s, 1H), 7.48 (s, 1H), 7.22 (s, 1H), 6.50 (s, 1H), 6.26 (s, 2H), 5.48 - 5.35 (m, 2H), 5.16 (s, 2H), 4.63 - 4.51 (m, 2H), 4.25 - 4.10 (m, 2H), 3.82 - 3.63 (m, 2H), 3.49 (t, *J* = 5.8 Hz, 2H), 3.40 (s, 3H), 3.06 (t, *J* = 8.0 Hz, 2H), 2.56 - 2.51 (m, 2H), 2.41 - 2.23 (m, 8H), 1.96 - 1.76 (m, 7H), 1.71 - 1.57 (m, 1H), 1.57 - 1.48 (m, 1H), 1.37 - 1.21 (m, 8H), 0.88 - 0.78 (m, 15H).

### Example 2.38 : (S)-6-(dipropylamino)-N-(2-(((((E)-3-((S)-7-ethyl-7-hydroxy-8,11-dioxo-7,8,11,13-tetrahydro-10H -[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide

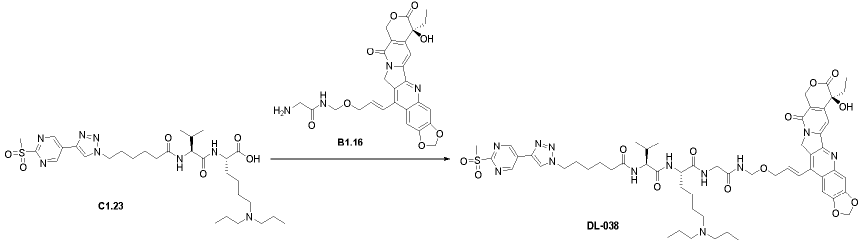

Compound B1.16 (30 mg, 0.06 mmol) and compound C1.23 (37 mg, 0.06 mmol) were dissolved in DMF (1 mL), to which were then added HBTU (21 mg, 0.06 mmol) and N,N-diisopropylethylamine (16 mg, 0.12 mmol). After addition, the reaction solution was stirred for 1 h at room temperature. LCMS detection indicated completion of the reaction. The reaction solution was purified by preparative chromatography (0.01% TFA in water, MeCN) to obtain the target compound (S)-6-(dipropylamino)-N-(2-(((((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (4 mg, yield 6%) as a yellow solid.

LCMS (ESI) [M+H]⁺ = 1167.6;
¹H NMR (400 MHz, DMSO) δ 9.45 (s, 2H), 8.98 (s, 1H, TFA), 8.92 (s, 1H), 8.74 (t, *J =* 6.8 Hz, 1H), 8.25 (t, *J* = 5.8 Hz, 1H), 8.05 (d, *J* = 7.3 Hz, 1H), 7.82 (d, *J* = 8.4 Hz, 1H), 7.63 (s, 1H), 7.53 (s, 1H), 7.26 (d, *J =* 12.0 Hz, 1H), 7.26 (s, 1H), 6.58 - 6.52 (m, 1H), 6.50 (s, 1H), 6.30 (s, 2H), 5.42 (s, 2H), 5.31 (s, 2H), 4.74 - 4.67 (m, 2H), 4.46 (m, 2H), 4.30 (m, 1H), 4.10 (m, 1H), 3.77 (m, 2H), 3.44 (s, 3H), 3.01 - 2.95 (m, 8H), 2.23 - 2.18 (m, 1H), 2.13 (m, 1H), 2.00 (m, 2H), 1.90 - 1.84 (m, 6H), 1.63 - 1.57 (m, 9H), 0.88 (m, 9H), 0.81 - 0.78 (m, 6H).

### Example 2.39: 4-((S)-6-amino-2-(6-(2-(methanesulfonyl)pyrimidin-5-yl)hex-5-ynamido)hexanamido)benzyl N-ethyl-N-(2-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)carbamate

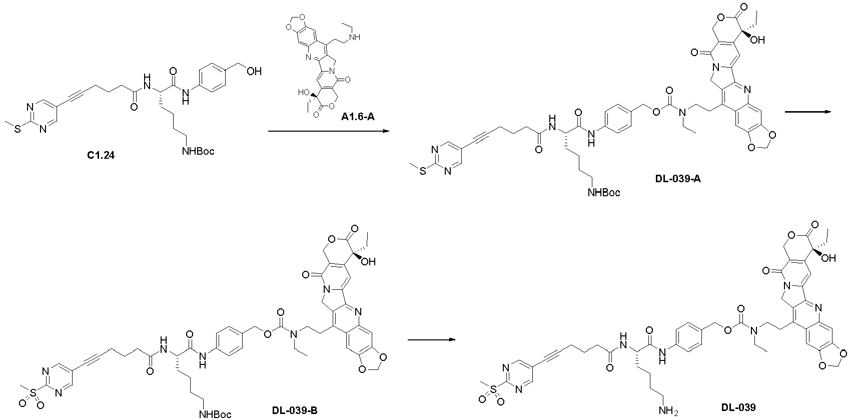

### Step 1:

At 0 °C, to a solution of C1.24 (150 mg, 0.264 mmol) and triethylamine (89 mg, 0.88 mmol) in dichloromethane (10 mL), was added N,N'-disuccinimidyl carbonate (DSC, 90 mg, 0.352 mmol) in an ice bath, and the mixture was allowed to react at 0 °C under nitrogen atmosphere for 30 min. Then, compound A1.6-A (82 mg, 0.176 mmol) was added dropwise, and the mixture was allowed to react at room temperature for 12 h. The reaction was completed as monitored by LCMS. The reaction solution was concentrated under reduced pressure to obtain a crude product, which was purified by preparative HPLC (acetonitrile/water containing 0.05% formic acid) to obtain a yellow oily compound DL-039-A (27 mg, yield: 14.4%) as a yellow oil.

LCMS (ESI) [M+H]⁺ = 1059.2;

### Step 2:

To a solution of compound DL-039-A (60 mg, 0.057 mmol) in a mixed solvent of tetrahydrofuran (5 mL) and water (1 mL), was added potassium peroxymonosulfonate (Oxone, 349 mg, 0.567 mmol), and the reaction was stirred at room temperature for 12 h . The reaction was completed as detected by TLC. Water (10 mL) was added to the reaction solution, and the resultant solution was extracted with dichloromethane (10 mL×3). The organic phase was washed with saturated brine, dried over anhydrous sodium sulfate, filtered, and concentrated under reduced pressure to obtain the target compound DL-039-B (60 mg, crude product) as a yellow solid.

LCMS (ESI) [M+H]⁺ = 1091.0;

### Step 3:

Compound DL-039-B (60 mg, 0.055 mmol) was dissolved in dichloromethane (1 mL), and then dichloroacetic acid (1 mL) was added to the reaction solution at 0°C. The reaction was stirred at room temperature for 3 h. LCMS showed that the reaction was completed. The filtrate was concentrated under reduced pressure to a crude product, which was purified by preparative HPLC (acetonitrile /water containing 0.05% trifluoroacetic acid) to obtain compound 4-((S)-6-amino-2-(6-(2-(methanesulfonyl)pyrimidin-5-yl)hex-5-ynamide)hexanamido)benzyl N-ethyl-N-(2-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)carbamate (2.44 mg, yield 3.69%) as a yellow solid.

LCMS (ESI) [1/2M+H]⁺ = 496.3, t_{R} = 2.252 min.

### Example 2.40: 4-((S)-6-amino-2-(6-(4-(2-(methanesulfonyl)pyrimidin-5-yl)-1H-1,2,3-triazol- 1-yl)hexanamido)hexanamido)benzyl N-ethyl-N-(2-((S)-7-ethyl-7-hydroxy-8,11-dioxo-7,8,11,13- tetrahydro-10H-[1,3]dioxolo[4,5-g]pyrano[3',4':6,7]indolizino[1,2-b]quinolin-14-yl)ethyl)carbamate (DL-040)

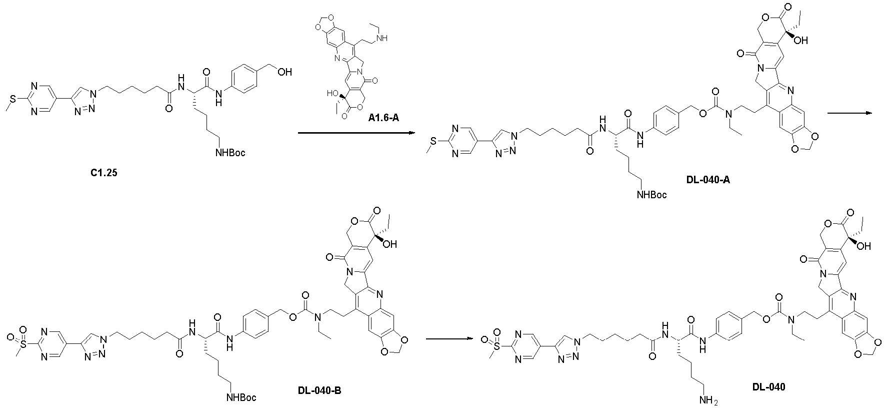

### Step 1:

At 0 °C, to a solution of C1.25 (300 mg, 0.469 mmol) and triethylamine (126 mg, 1.25 mmol) in dichloromethane (10 mL), was added N,N'-disuccinimidyl carbonate (DSC, 160 mg, 0.626 mmol) in an ice bath, and the mixture was allowed to react at 0 °C for 30 min. Then, compound A1.6-A (145 mg, 0.313 mmol) was added dropwise, and the mixture was allowed to react at room temperature for 12 h. The reaction was completed as monitored by LCMS. The reaction solution was concentrated under reduced pressure to obtain a crude product, which was purified by preparative HPLC (acetonitrile/water containing 0.05% formic acid) to obtain a yellow oily compound DL-040-A (70 mg, yield: 19.8%). LCMS (ESI) [M+H]⁺ = 1130.1.

### Step 2:

To a solution of compound DL-040-A (70 mg, 0.062 mmol) in a mixed solvent of tetrahydrofuran (5 mL) and water (1 mL), was added potassium peroxymonosulfonate (Oxone, 381 mg, 0.619 mmol), and the reaction was stirred at room temperature for 12 h . The reaction was completed as detected by TLC. Water (10 mL) was added to the reaction solution, and the resultant solution was extracted with dichloromethane (10 mL×3). The organic phase was washed with saturated brine, dried over anhydrous sodium sulfate, filtered, and concentrated under reduced pressure to obtain the target compound DL-040-B (70 mg, crude product) as a yellow solid. LCMS (ESI) [M+H]⁺ = 1162.6, t_{R} = 1.710 min.

### Step 3:

Compound DL-040-B (70 mg, 0.060 mmol) was dissolved in dichloromethane (1 mL), and then dichloroacetic acid (1 mL) was added to the reaction solution at 0°C. The reaction was stirred at room temperature for 3 h. LCMS showed that the reaction was completed. The filtrate was concentrated under reduced pressure to a crude product, which was purified by preparative HPLC (acetonitrile /water containing 0.05% trifluoroacetic acid) to obtain compound 4-((S)-6-amino-2-(6-(4-(2-(methanesulfonyl)pyrimidin-5-yl)-1H-1,2,3-triazol-1-yl)hexanamido)hexanamido)benzyl N-ethyl-N-(2-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)carbamate (5.54 mg, yield 8.66%) as a yellow solid.

LCMS (ESI) [1/2M+H]⁺ = 1062.5, t_{R} = 4.052 min;
¹H NMR (400 MHz, DMSO-d6) δ 10.22 (br s, 1H), 9.46 (s, 2H), 8.93 (s, 1H), 8.22 - 8.03 (m, 1H), 7.85 (br s, 3H, NH2-TFA), 7.61 - 7.45 (m, 3H), 7.32 - 7.26 (m, 1H), 7.26 - 7.17 (m, 2H), 6.54 (s, 1H), 6.35 (s, 2H), 5.91 (s, 2H), 5.47 (s, 2H), 5.37 - 5.30 (m, 1H), 5.27 - 5.20 (m, 1H), 5.10 - 5.00 (m, 1H), 4.97 - 4.78 (m, 1H), 4.52 - 4.26 (m, 3H), 3.56 - 3.46 (m, 2H), 3.44 (s, 3H), 2.83 - 2.71 (m, 2H), 2.21 - 2.11 (m, 2H), 1.98 - 1.78 (m, 4H), 1.67 - 1.48 (m, 6H), 1.37 - 1.22 (m, 4H), 1.09 - 0.96 (m, 3H), 0.93 - 0.78 (m, 3H).

### Example 2.41: (S)-6-(dimethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-041)

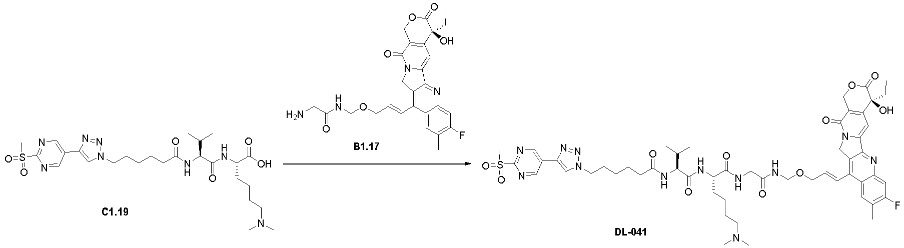

Compound B1.17 (30 mg) and compound C1.19 (37 mg) were dissolved in DMF (1 mL), to which were then added HBTU (21 mg) and N,N-diisopropylethylamine (16 mg). After addition, the reaction solution was stirred for 1 h at room temperature. LCMS detection indicated completion of the reaction. The reaction solution was purified by preparative chromatography (0.01% TFA in water, MeCN) to obtain the target compound (S)-6-(dimethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (11 mg).

LCMS (ESI) [M+H]⁺ = 1099.5.

### Example 2.42: (S)-6-(diethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-042)

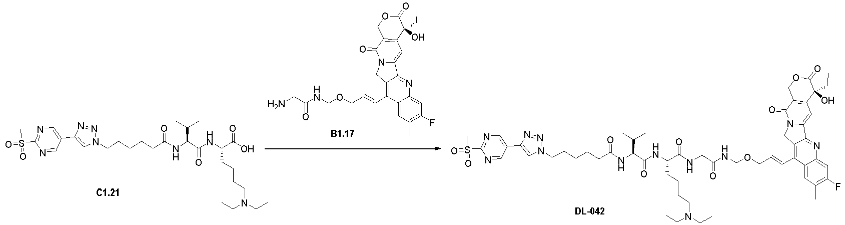

Compound B1.17 (30 mg) and compound C1.21 (37 mg) were dissolved in DMF (1 mL), to which were then added HBTU (21 mg) and N,N-diisopropylethylamine (16 mg). After addition, the reaction solution was stirred for 1 h at room temperature. LCMS detection indicated completion of the reaction. The reaction solution was purified by preparative chromatography (0.01% TFA in water, MeCN) to obtain the target compound (S)-6-(diethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (7 mg).

LCMS (ESI) [M+H]⁺ = 1127.5.

### Example 2.43: (S)-6-(dipropylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-043)

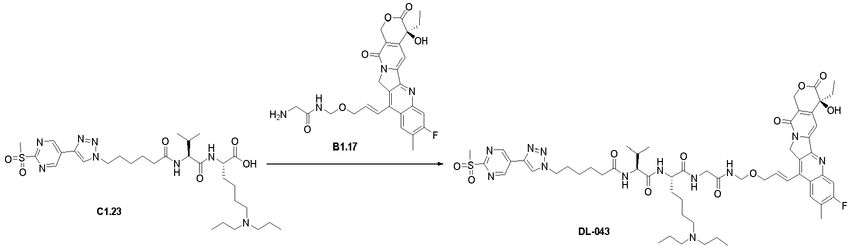

Compound B1.17 (17.5 mg, 0.033 mmol) and compound C1.23 (22 mg, 0.034 mmol) were dissolved in N,N-dimethylformamide (2 mL), to which were then successively added HBTU (7 mg, 0.051 mmol), EDCI (10 mg, 0.051mmol) and DIPEA (9 mg, 0.068 mmol). After addition, the reaction solution was stirred for 1 h at room temperature. The reaction solution was directly purified by preparative HPLC (0.01% TFA in water, MeCN), and the collected eluent was lyophilized to obtain the target compound (S)-6-(diethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (7 mg, yield 18%) as a pale yellow solid product.

LCMS (ESI) [M+H]⁺ = 1155.5.

### Example 2.44: N-((11S,14S,E)-11-(4-(dimethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-1-en-14-yl)-6-(2-(methanesulfonyl)pyrimidin-5-yl)hex-5-ynamide (DL-044)

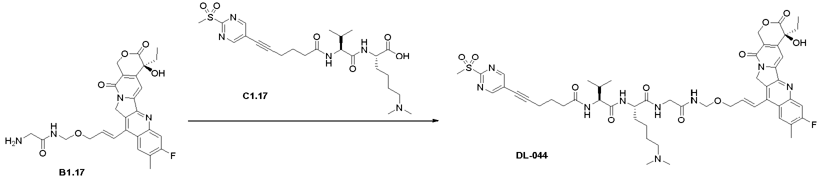

Compound B1.17 (60 mg) and compound C1.17 (80 mg) were dissolved in DMF (2 mL), to which were then added HBTU (45 mg) and N,N-diisopropylethylamine (35 mg). After addition, the reaction solution was stirred for 1 h at room temperature. LCMS detection indicated completion of the reaction. The reaction solution was purified by preparative chromatography (0.01% TFA in water, MeCN) to obtain the target compound N-((11S,14S,E)-11-(4-(dimethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-1-en-14-yl)-6-(2-(methanesulfonyl)pyrimidin-5-yl)hex-5-ynamide (21 mg).

LCMS (ESI) [M+H]⁺ = 1028.5.

### Example 2.45: N-((11S,14S,E)-11-(4-(diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-1-en-14-yl)-6-(2-(methanesulfonyl)pyrimidin-5-yl)hex-5-ynamide (DL-045)

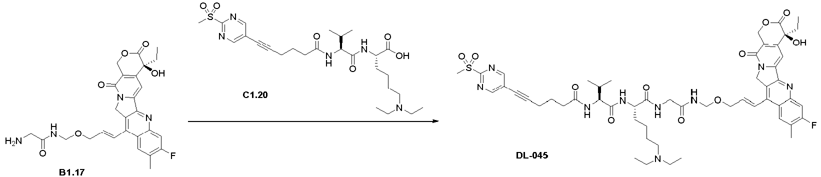

Compound B1.17 (60 mg) and compound C1.20 (80 mg) were dissolved in DMF (2 mL), to which were then added HBTU (45 mg) and N,N-diisopropylethylamine (35 mg). After addition, the reaction solution was stirred for 1 h at room temperature. LCMS detection indicated completion of the reaction. The reaction solution was purified by preparative chromatography (0.01% TFA in water, MeCN) to obtain the target compound N-((11S,14S,E)-11-(4-(diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-1-en-14-yl)-6-(2-(methanesulfonyl)pyrimidin-5-yl)hex-5-ynamide (29 mg).

LCMS (ESI) [M+H]⁺ = 1056.5.

### Example 2.46: N-((11S,14S,E)-11-(4-(dipropylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-1-en-14-yl)-6-(2-(methanesulfonyl)pyrimidin-5-yl)hex-5-ynamide (DL-046)

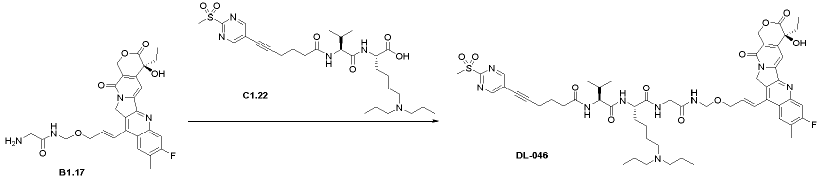

Compound B1.17 (60 mg) and compound C1.22 (80 mg) were dissolved in DMF (2 mL), to which were then added HBTU (45 mg) and N,N-diisopropylethylamine (35 mg). After addition, the reaction solution was stirred for 1 h at room temperature. LCMS detection indicated completion of the reaction. The reaction solution was purified by preparative chromatography (0.01% TFA in water, MeCN) to obtain the target compound N-((11S,14S,E)-11-(4-(diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-1-en-14-yl)-6-(2-(methanesulfonyl)pyrimidin-5-yl)hex-5-ynamide (30 mg).

LCMS (ESI) [M+H]⁺ = 1084.2;
¹H NMR (400 MHz, DMSO) δ 9.09 (s, 2H), 8.73 (d, *J =* 6.3 Hz, 1H), 8.30 - 8.24 (m, 2H), 8.05 (m, 1H), 7.94 - 7.88 (m, 2H), 7.38 - 7.33 (m, 2H), 6.67 - 6.60 (m, 1H), 6.53 (s, 1H), 5.44 (s, 2H), 5.37 (s, 2H), 4.73 (t, *J =* 6.2 Hz, 2H), 4.34 (d, *J =* 4.2 Hz, 2H), 4.26 - 4.20 (m, 1H), 4.17 - 4.13 (m, 1H), 3.86 - 3.69 (m, 4H), 3.40 (s, 3H), 2.41 - 2.30 (m, 8H), 2.02 - 1.94 (m, 2H), 1.89 - 1.85 (m, 2H), 1.82 - 1.77 (m, 2H), 1.74 - 1.63 (m, 2H), 1.60 - 1.50 (m, 2H), 1.47 - 1.39 (m, 8H), 0.90 - 0.86 (m, 6H), 0.85 - 0.80 (m, 9H).

### Example 2.47: N-((11S,14S,E)-11-(4-(dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-oxo-4-oxa-6,9,12-triazahexadecan-1-en-14-yl)-6-(2-(methanesulfonyl)pyrimidin-5-yl)hex-5-ynamide (DL-047)

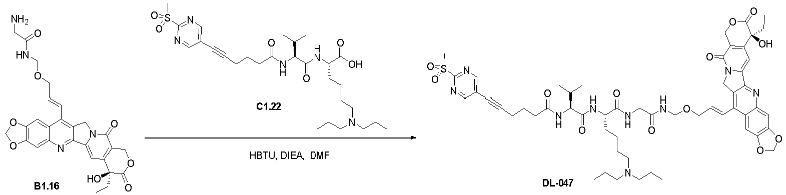

Compound B1.16 (45 mg, 0.08 mmol) and compound C1.22 (49 mg, 0.08 mmol) were dissolved in DMF (1 mL), to which were then added HBTU (32 mg, 0.08 mmol) and N,N-diisopropylethylamine (27 mg, 0.2 mmol). After addition, the reaction solution was stirred for 1 h at room temperature. LCMS detection indicated completion of the reaction. The reaction solution was purified by preparative chromatography (0.01% TFA in water, MeCN) to obtain the target compound N-((11S,14S,E)-11-(4-(dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-oxo-4-oxa-6,9,12-triazahexadecan-1-en-14-yl)-6-(2-(methanesulfonyl)pyrimidin-5-yl)hex-5-ynamide (DL-047, 10 mg) as a yellow solid.

LCMS (ESI) [M+H]⁺ = 1096.6;
¹H NMR (400 MHz, DMSO) δ 9.10 (s, 2H), 9.05 (s, 1H, TFA), 8.75 (t, *J =* 5.3 Hz, 1H), 8.26 (t, *J =* 6.4 Hz, 1H), 8.11 (d, *J =* 7.6 Hz, 1H), 7.93 (d, *J =* 8.4 Hz, 1H), 7.63 (s, 1H), 7.53 (s, 1H), 7.26 - 2.24 (m, 2H), 6.65 - 6.43 (m, 2H), 6.30 (s, 2H), 5.42 (s, 2H), 5.30 (s, 2H), 4.74 - 4.69 (m, 1H), 4.31 - 4.29 (m, 2H), 4.26 - 424 (m, 1H), 4.19 - 4.12 (m, 1H), 3.79 - 2.76 (m, 1H), 3.40 (s, 3H), 3.02 - 2.95 (m, 8H), 2.55 (m, 2H), 2.39 - 2.32 (m, 2H), 2.01 - 1.93 (m, 2H), 1.92 - 1.77 (m, 5H), 1.62 - 1.58 (m, 6H), 1.36 - 1.30 (m, 2H), 0.91 - 0.83 (m, 15H).

### Example 2.48: N-((11S,14S)-11-(4-(dibutylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide (DL-048)

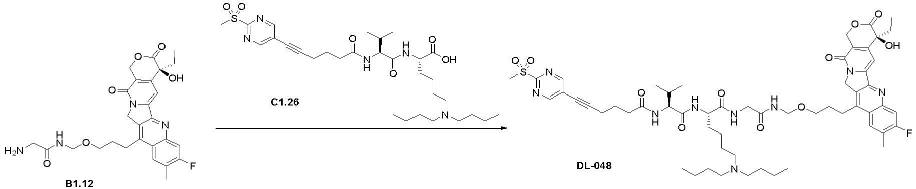

Compound C1.26 (50 mg, 0.08 mmol) and compound B1.12 (43 mg, 0.08 mmol) were dissolved in N,N-dimethylformamide (1.5 mL), to which were then added DIPEA (26 mg, 0.21 mmol) and HBTU (31 mg, 0.08 mmol). The reaction was stirred for 1 h at room temperature. LCMS detection indicated completion of the reaction. The reaction solution was separated and purified by preparative HPLC (0.01% TFA in water, MeCN) to obtain the target compound (13.57mg) as a yellow solid.

LCMS (ESI) [M+H]⁺ = 1114.6;
¹H NMR (400 MHz, DMSO) δ 9.09 (s, 2H), 9.05 (s, 1H, TFA), 8.64 (t, *J =* 7.2 Hz, 1H), 8.23 - 8.16 (m, 2H), 8.07 (d, *J* = 7.3 Hz, 1H), 7.93 - 7.85 (m, 2H), 7.32 (s, 1H), 6.52 (s, 1H), 5.44 (s, 2H), 5.29 (s, 2H), 4.61 - 4.59 (m, 2H), 4.24 - 4.22 (m, 1H), 4.19 - 4.10 (m, 1H), 3.73 - 3.70 (m, 2H), 3.50 (s, 3H), 3.21 - 3.18 (m, 6H), 3.00 - 2.98 (m, 8H), 2.54 (s, 3H), 1.97 - 1.78 (m, 7H), 1.56 - 1.52 (m, 8H), 1.36 - 1.25 (m, 6H), 0.93 - 0.78 (m, 15H);

### Example 2.49: (S)-6-(dimethylamino)-N-(2-(((((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-049)

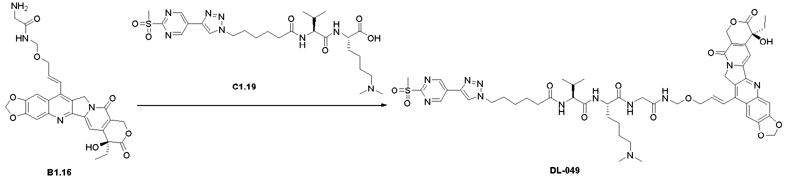

Compound B1.16 (45 mg, 0.08 mmol) and compound C1.19 (50 mg, 0.08 mmol) were dissolved in DMF (1 mL), to which were then added N,N-diisopropylethylamine (27 mg, 0.2 mmol) and HBTU (32 mg, 0.08 mmol). After addition, the reaction solution was stirred for 1 h at room temperature. LCMS detection indicated completion of the reaction. The reaction solution was directly purified by preparative chromatography (0.01% formic acid in water, acetonitrile) to obtain the target compound (S)-6-(dimethylamino)-N-(2-(((((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-049, 3 mg) as a white solid.

LCMS (ESI) [M+H]⁺ = 1111.1;
¹H NMR (400 MHz, DMSO) δ 9.45 (s, 2H), 8.92 (s, 1H), 8.69 (t, *J* = 6.6 Hz, 1H), 8.25 (t, *J* = 5.9 Hz, 1H), 8.19 (s, 1H, FA), 7.99 (d, *J* = 7.0 Hz, 1H), 7.83 (d, *J* = 8.5 Hz, 1H), 7.64 (s, 1H), 7.52 (s, 1H), 7.27 (m, 2H), 6.59 - 6.48 (m, 2H), 6.29 (s, 2H), 5.42 (s, 2H), 5.31 (s, 2H), 4.74 - 4.67 (m, 2H), 4.44 (m, 2H), 4.30 (d, *J* = 4.8 Hz, 2H), 4.21 (m, 1H), 4.12 (m, 1H), 3.83 - 3.70 (m, 4H), 3.43 (s, 3H), 2.25 (m, 2H), 2.15 (s, 6H), 2.04 - 1.97 (m, 2H), 1.90 - 1.85 (m, 4H), 1.55 - 1.51 (m, 3H), 1.39 - 1.34 (m, 6H), 0.90 - 0.83 (m, 3H), 0.80 (t, *J=* 7.2 Hz, 6H).

### Example 2.50: 1-ethyl-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-4-((S)-3-methyl-2-(6-(2-(methanesulfonyl)pyrimidin-5-yl)hex-5-ynamido)butanamido)piperidine-4-carboxamide (DL-050)

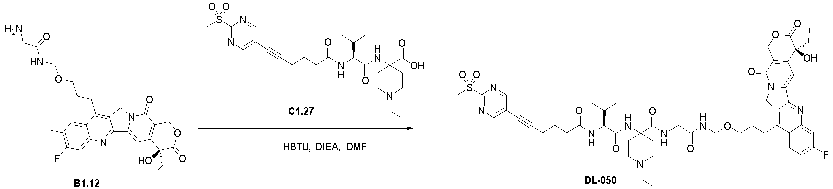

Compound B1.12 (50 mg, 0.10 mmol) and compound C1.27 (50 mg, 0.10 mmol) were dissolved in DMF (1 mL), to which were then added N,N-diisopropylethylamine (30 mg, 0.24 mmol) and HBTU (36 mg, 0.10 mmol). After addition, the reaction solution was stirred for 1 h at room temperature. LCMS detection indicated completion of the reaction. The reaction solution was purified by preparative chromatography (0.01% FA in water, MeCN) to obtain the target compound 1-ethyl-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-4-((S)-3-methyl-2-(6-(2-(methanesulfonyl)pyrimidin-5-yl)hex-5-ynamido)butanamido)piperidine-4-carboxamide (DL-050, 10 mg) as a white solid.

LCMS (ESI) [M+H]⁺ = 1028.6;
¹H NMR (400 MHz, DMSO) δ 9.08 (s, 2H), 8.42 (s, 1H, FA), 8.18 - 8.15 (m, 4H), 7.98 (t, *J* = 5.6 Hz, 1H), 7.86 (d, *J* = 10.9 Hz, 1H), 7.30 (s, 1H), 6.51 (s, 1H), 5.43 (s, 2H), 5.28 (s, 2H), 4.72 - 4.63 (m, 1H), 4.53 - 4.45 (m, 1H), 4.07 - 4.06 (m, 1H), 3.79 - 3.69 (m, 1H), 3.58 - 3.55 (m, 2H), 3.55 - 3.48 (m, 5H), 3.21 - 3.16 (m, 3H), 2.55 (s, 3H), 2.66 - 2.64 (m, 2H), 2.40 - 2.26 (m, 5H), 1.99 - 1.71 (m, 12H), 0.96 (t, *J* = 7.1 Hz, 3H), 0.93 - 0.85 (m, 9H).

### Example 2.51: (R)-6-(dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methanesulfonyl)pyrimidin-5-yl)-1H-1,2,3-triazol-1-yl)hexanamido)butanamido)hexanamide (DL-051) and (S)-6-(dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methanesulfonyl)pyrimidin-5-yl)-1H-1,2,3-triazol-1-yl)hexanamido)butanamido)hexanamide (DL-052)

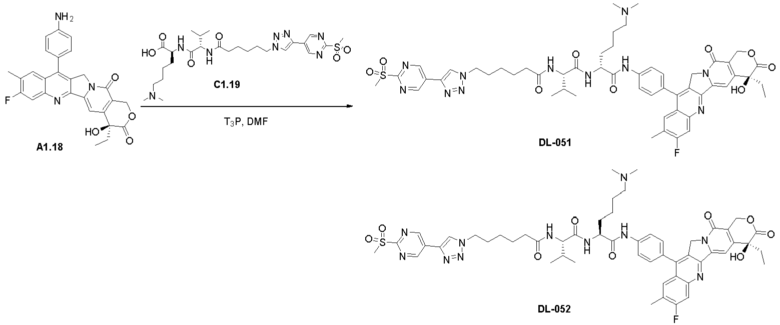

Compounds A1.18 (40 mg, 0.08 mmol) and C1.19 (71 mg, 0.12 mmol) were dissolved in N,N-dimethylformamide (3 mL), to which was added T₃P (254 mg, 0.40 mmol, 50% w/w mixture in ethyl acetate). The reaction solution was stirred for 1 h at room temperature. LCMS detection indicated completion of the reaction. The reaction solution was directly purified by preparative chromatography (0.01% trifluoroacetic acid in water, acetonitrile) to obtain the target compound (R)-6-(dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methanesulfonyl)pyrimidin-5-yl)-1H-1,2,3-triazol-1-yl)hexanamido)butanamido)hexanamide (DL-051, 8.1 mg) and (S)-6-(dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methanesulfonyl)pyrimidin-5-yl)-1H-1,2,3-triazol-1-yl)hexanamido)butanamido)hexanamide (DL-052, 8.8 mg), as white solids for both compounds.
DL-051:
   LCMS (ESI) [M+H]⁺ = 1048.2, t_{R} = 1.258 min;
   ¹H NMR (400 MHz, DMSO) δ 10.12 (s, 1H), 9.40 (s, 2H), 8.84 (s, 1H), 8.55 (d, *J* = 7.4 Hz, 1H), 8.26 (s, 1H), 8.05 (d, *J* = 7.8 Hz, 1H), 7.99 - 7.89 (m, 3H), 7.73 (d, *J* = 8.4 Hz, 1H), 7.57 (d, *J* = 8.7 Hz, 2H), 7.36 (s, 1H), 5.41 (s, 2H), 5.10 (s, 2H), 4.40 (m, 3H), 4.22 - 4.10 (m, 1H), 3.44 (s, 3H), 2.40 (s, 3H), 2.30 - 2.25 (m, 2H), 2.24 - 2.20 (m, 2H), 2.19 - 2.15 (m, 6H), 2.03 - 1.93 (m, 2H), 1.92 - 1.78 (m, 6H), 1.72 - 1.66 (m, 1H), 1.61 - 1.54 (m, 2H), 1.48 - 1.42 (m, 2H), 1.30 - 1.26 (m, 2H), 0.91 - 0.86 (m, 9H).
DL-052:
   LCMS (ESI) [M+H]⁺ = 1048.2, t_{R} = 1.283 min;
   ¹H NMR (400 MHz, DMSO) δ 10.34 (s, 1H), 9.45 (s, 2H), 8.94 (s, 1H), 8.20 (s, 1H), 7.99 - 7.85 (m, 4H), 7.75 (d, *J* = 7.4 Hz, 1H), 7.59 (m, 2H), 7.36 (s, 1H), 6.55 (s, 1H), 5.42 (s, 2H), 5.10 (s, 2H), 4.52 - 4.42 (m, 3H), 4.25 - 4.17 (m, 1H), 3.44 (s, 3H), 2.41 (s, 3H), 2.30 - 2.13 (m, 8H), 2.05 - 1.97 (m, 2H), 1.95 - 1.81 (m, 7H), 1.60 - 1.55 (m, 2H), 1.50 - 1.44 (m, 2H), 1.33 - 1.28 (m, 4H), 0.95 - 0.83 (m, 9H).

### III. Antibody preparation and identification

### Example 3.1 Preparation of B7H3 antigen and anti-B7H3 antibodies B7H3-C1 and B7H3-C2

The nucleic acid sequence for amino acids at positions 29-245 (NCBI protein number NP_001316557.1) of human 2Ig B7H3 was selected, in which a 6×His detection tag was added to the C-terminus and named as human B7H3-2IgG-his; the nucleic acid sequence for amino acids at positions 27-461 (Uniport protein number Q5ZPR3-1) of human 4Ig B7H3 was selected, in which a 6×His purification tag was added to the C-terminus and named as human B7H3-4IgG-his; the nucleic acid sequence for amino acids at positions 27-465 (Uniport protein number F7G5V3) of monkey B7H3 4Ig was selected, in which a 6x His purification tag was added to the C-terminus and named as monkey B7H3-4IgG-his; for B7H3-C1, see antibody M30-H1-L4 in CN 103687945B; for B7H3-C2, see the antibody of mAb-C-DUBA in CN109069633A.

The genes of the above three B7H3 antigens were synthesized, as well as the genes of antibodies B7H3-C1 and B7H3-C2 were synthesized by codon-optimization (Sangon Biotech (Shanghai) Co., Ltd.), and then the genes were constructed into PTT5 expression vector, and a large number of plasmids were extracted. The extracted and prepared expression vectors were transiently transfected into and expressed in HEK293F for 7 days, and the expression supernatants were prepared by Protein A to obtain antigen protein, B7H3-C1 antibody and B7H3-C2 antibody.

### Example 3.2 Preparation of full human phage library

10 healthy male and 10 healthy female volunteers at the age of about 20-30 were recruited, and then about 50 mL of fresh blood was collected. Fresh PBMC cells were extracted by Ficol, and then mRNA was extracted by Trizol for reverse transcription to obtain cDNA. The heavy and light chains of an antibody were amplified from cDNA by VH and VK primer pairs. ScFv fragments were further obtained by Overlap PCR, ligated to pTT-1 vector by SfiI enzyme digestion, and thus full human ScFv phage library was obtained by electroporation, with a library capacity of about 5.2*10⁹.

1 mL of the library was added to 1.0 L of fresh medium 2YT + 100 µg/ml Amp + 2% glucose, with an initial OD600 of the bacterial liquid being < 0.1, and then cultured at 37 °C and 220 rpm. When the OD600 was about 0.6, M13K07 phage was added at the ratio of cell:phage = 1:20 to assist phagemid infection, and cultured at 37 °C for 30 min, and then cultured at 37 °C and 200 rpm for 30 min, and finally centrifuged under a condition of 4000 rpm for 10 min, then resuspended with 1.0 L equal volume of 2YT + 100 µg/ml Amp + 50 µg/ml Kana, and cultured at 30 °C for 16 h for expression. After the expression culture was completed, the bacterial solution was centrifuged at 8000 rpm for 30 min at 4°C, and the supernatant was removed, followed by high-speed centrifugation to remove bacterial body. 1/5 volume of PEG/NACL was added to the supernatant, and Phage in the supernatant was precipitated. After centrifugation, Phage was dissolved in PBS, and the phage titer was determined to be about 2 * 10¹¹.

### Example 3.3 Screening of anti-B7H3 antibodies from phage library

### 3.3.1 hB7H3-his/cynoB7H3-his/MCF-7 affinity panning

The first round of affinity panning: hB7H3-his antigen (Sino Biological Inc) was diluted using carbonate buffer with pH value of 9.6 to a final concentration of 5 µg/mL, and then added to enzyme labelling wells at 100 µL/well, and 8 wells were coated overnight at 4 °C; the second round of affinity panning: cynoB7H3-his antigen (Sino Biological) was diluted using carbonate buffer with pH value of 9.6 to a final concentration of 2 µg/mL, and then added to enzyme labelling wells at 100 µL/well, and 4 wells were coated overnight at 4 °C. The third round of affinity panning: MCF-7 cells were digested by trypsin, resuspended by DMEM+10% FBS, plated on 96-well cell plates at 50000 cells/wells, and incubated overnight at 37 °C in a 5% CO₂ incubator. The coating solution or cell culture solution was discarded, and the plate was washed with PBS for three times. 300 µL of 3% OVA-PBS blocking solution was added to each well and blocked for 1 hour at 37 °C; the plate was washed three times with PBS, and then 100 µL of phage library was added, followed by incubating for 0.5-1 hour at 37 °C; unbound phages were aspirated, and then the plate was washed 6-8 times with PBST and 2 times with PBS; 100µL of Gly-HCl eluate was added, and then the plate was incubated at 37°C for 8 minutes to elute the specifically bound phages; the eluate was transferred to a 1.5 mL sterile centrifuge tube, and quickly neutralized with 10 µL of Tris-HCl neutralization buffer; 10 µL of solution was taken out for gradient dilution, and the titer was determined, to calculate the recovery rate of panning. The remaining eluates were mixed for amplification and purification and used for the next round of affinity panning, and the panning conditions were changed. The conditions for each round of panning are listed in Table 1.

**Table 1. Affinity panning conditions.**

| Round | Antigen coating concentration (µg/mL) | Blocking solution | Library input amount (cfu) | Binding time (h) | PBST concentration | PBST wash times |
|---|---|---|---|---|---|---|
| 1 | 5 | OVA-PBS | 2×10¹¹ | 1 | 0.1% | 6 |
| 2 | 2 | OVA-PBS | 1×10¹¹ | 0.75 | 0.25% | 8 |
| 3 | MCF-7 50000 cells/well | OVA-PBS | 1×10¹¹ | 0.5 | 0.5% | 8 |

### 3.3.2 Phage rescue

From the plate of panning eluate titer, 96 clones were randomly selected from each plate of the third round of titer determination with a sterile toothpick, inoculated in 1 mL of 2×YT-A, and incubated for 8 h at 37 °C and 230 r/min under shaking. 100 µL of the above culture was taken out, and M13K07 phages were added at the ratio of cell: phage = 1:20. The culture was allowed to stand at 37 °C for 15 min, and cultured with shaking at 220 r/min for 45 min. and then supplemented with 800 µL of 2×YT-AK, followed by incubating overnight at 30°C with vigorous shaking. The next day, the cells were centrifuged at 12,000 rpm for 2 min, and the supernatant was taken out for monoclonal ELISA identification.

### 3.3.3 Identification of positive phage clones

Human B7H3-4IgG-his was diluted with carbonate buffer (pH 9.6) to a final concentration of 1µg/mL, and 2% OVA-PBS was added to enzyme labeling wells at 100 µL/well, and coated overnight at 4 °C; the coating solution was discarded, and the plate was washed 3 times with PBST. Each well was added with 300 µL of 5% skim milk, and blocked at 37 °C for 1 hour; the plate was washed once with PBST. 50 µL of supernatant of phage culture solution and 50µL of 5% skim milk were added to each well and incubated for 1 h at 37 °C. The plate was washed 5 times with PBST, and then horseradish peroxidase-labeled anti-M13 antibodies (diluted in 1:10000 with PBS) was added at 100µL/well, and reacted at 37 °C for 1 h. The plate was washed 6 times with PBST. TMB chromogenic solution was added at 100µL/well and 37°C and incubated for 7min, and then the reaction was terminated by adding a stopping solution at 50µL/well. The absorbance was measured at 450 nm, and a total of 9 anti-B7H3 scFv antibodies were screened with unique sequences, in which 1D1 sequence of scFv antibody that binds the most to tumor cells was set forth in SEQ ID NO:1, and 2E3 sequence was set forth in SEQ ID NO:2.

### Example 3.4 Expression of anti-B7H3 antibody

The VH amino acid sequences SEQ ID NO: 3 and SEQ ID NO: 23 of the ScFv of 1D1 and 2E3 were respectively added with the amino acid sequence of the constant region of IgG1 (SEQ ID NO: 43) by codon optimization and gene synthesis (Sangon Biotech (Shanghai) Co., Ltd.), and then constructed into PTT5 vector, named PTT5-01-CH and PPT5-02-CH respectively; the VL amino acid sequences SEQ ID NO: 13 and SEQ ID NO: 33 of the ScFvs of 1D1 and 2E3 were added with the Kappa constant region sequence (SEQ ID NO: 44) by codon optimization and gene synthesis (Sangon Biotech (Shanghai) Co., Ltd.), and then constructed into PTT5 vector, named PTT5-01-CL and PTT5-01-CL, respectively; anti-B7H3 antibody expression plasmids PTT5-01-CH/PTT5-01-CL and PTT5-02-CH/PTT5-02-CL were co-transfected into HEK293F cells by PEI max reagent and expressed in a 5% CO₂ shaker at 37 °C for 7 days. Supernatants were collected and purified by ProA magnetic beads to obtain anti-B7H3 antibodies named 1D1-01 and 2E3-02, respectively.

### Example 3.5 Protein affinity detection of anti-B7H3 antibody

The affinity of B7H3 full human antibody to human B7H3-4IgG-his and monkey B7H3-4IgG-his proteins was tested by ELISA. Specific experimental procedures: human B7H3-4IgG-his or monkey B7H3-4IgG-his proteins were diluted with carbonate buffer (pH 9.6) to a final concentration of 1µg/mL and added into 96-well enzyme labelling wells at 100 µL/well, and coated overnight at 4 °C; the coating solution was discarded, and the plate was washed once with PBST, then 100 µL of PBS (containing 2% BSA) was added to each well, and blocked at 37 °C for 1 h; then, 100 µL of B7H3 full human antibody diluted with PBS (containing 2% BSA) (starting at 10µg, 3 folds serial dilution in duplicate wells) was added to each well, and then incubated at 37 °C for 2h; the plate was washed three times with PBST, and horseradish peroxidase-labeled anti-human Fc antibody (diluted at 1:10000 with PBS) was added at 100µL/well, then reacted for 1h at 37 °C. The plate was washed 5 times with PBST. TMB chromogenic solution was added at 100µL/well and incubated at 37 °C for 7 min to develop color, and then the stop solution was added at 50µL/well to terminate the reaction. The absorbance was measured at 450 nm, and EC₅₀ values were calculated from original data.

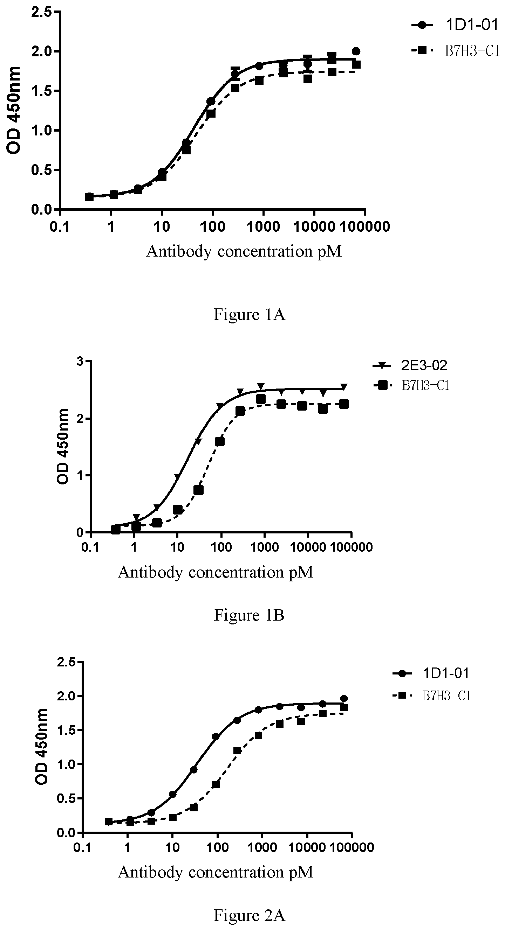

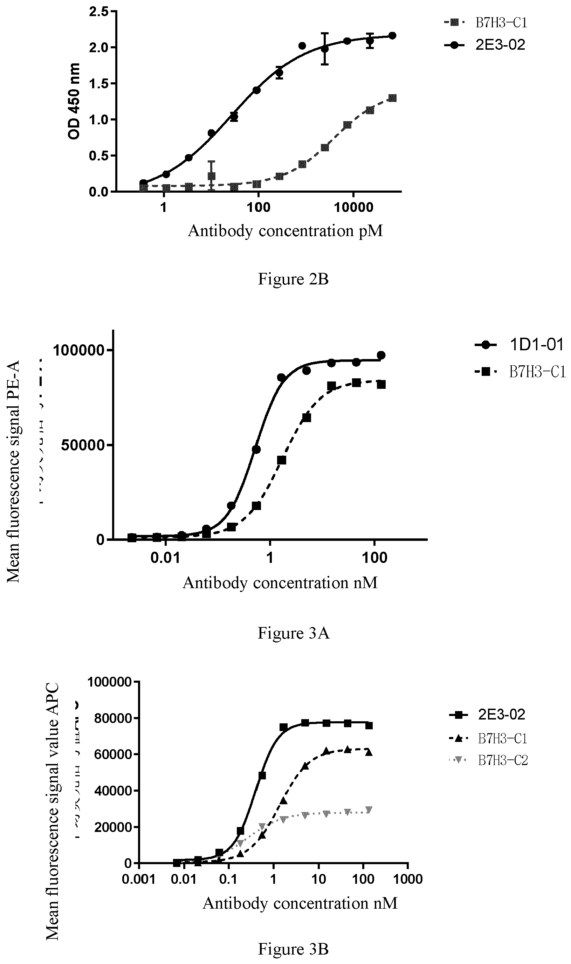

The results are shown in Figures 1A, 1B, 2A and 2B, and the detailed results are listed in Tables 1-1 and 1-2. 1D1-01 and 2E3-02 both have higher affinity with human B7H3-4IgG, which are 43.56pM and 17.54pM, respectively, in which 2E3-02 had a stronger affinity compared with B7H3-C1 with 51.85pM; 1D1-01 and 2E3-02 had higher binding ability to monkey B7H3-4IgG with 35.82pM and 27.72pM, respectively.

**Table 1-1. Test results for protein affinity of antibody1D1-01.**

| Antibody | Human B7H3-4IgG-His | Monkey B7H3-4IgG-His |
|---|---|---|
| | EC50 (pM) | EC50 (pM) |
| 1D1-01 | 43.56 | 35.82 |
| B7H3-C1 | 48.03 | 166.52 |

**Table 1-2. Test results for protein affinity of antibody 2E3-02.**

| Antibody | Human B7H3-4IgG-His | Monkey B7H3-4IgG-His |
|---|---|---|
| | EC50 (pM) | EC50 (pM) |
| 2E3-02 | 17.54 | 27.72 |
| B7H3-C1 | 51.85 | 3921 |

### Example 3.6 Detection of cell affinity of anti-B7H3 antibody

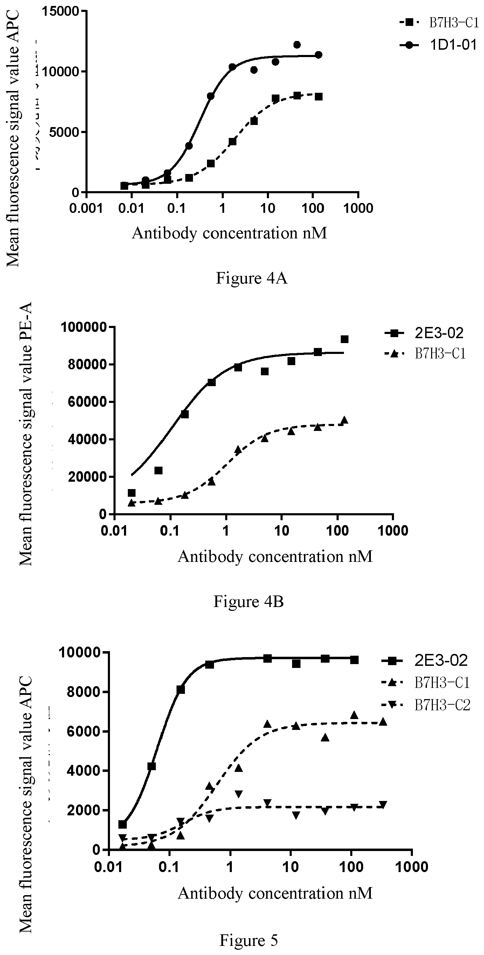

B7H3 protein is highly expressed in a variety of solid tumor cells. The affinity of B7H3 full human antibody to tumor cells was detected by FACS assay. Specific experimental procedures: MCF-7 breast cancer cells, A549 human non-small cell lung cancer cells and PC3 human prostate cancer cells grown to logarithmic phase were digested by trypsin, resuspended to 5*10^⁵ cells/ml in PBS buffer containing 2% BSA. 100µL cell suspension was added to 96-well plate. B7H3-C1, B7H3-C2,1D1-01 and 2E3-02 were diluted in PBS buffer containing 2% BSA, with 3 folds serial dilution at a starting concentration at 40µg/mL. Then 100µL antibody suspension was added into 96-well plate and mixed; the plate was incubated at 4 °C for 1 hour, washed twice with pre-chilled PBS at 300µL/well for each time, and then centrifuged at 500 g for 5 min; 1:50 fluorescent secondary antibody APC anti-Human IgG or PE anti-human IgG (purchased from Biolegend) was added at 100µL/well and mixed. The plate was incubated at 4 °C for 0.5 h, washed twice with pre-chilled PBS at 300µL/well for each time, centrifuged at 500 g for 5 minutes, and then rescreened with 500µl PBS. The average fluorescence signal value was measured by Beckman flow cytometry. The EC₅₀ value was calculated from the average fluorescence signal value, and the results are shown in Figures 3A, 3B, 4A, 4B and Figure 5. Specific data are listed in Tables 2-1 and 2-2.

**Table 2-1. Test results for cell affinity of 1D1-01 antibody.**

| Antibody | MCF-7 tumor cells | | A549 tumor cells | |
|---|---|---|---|---|
| | EC₅₀ (nM) | Maximum MFI | EC₅₀ (nM) | Maximum MFI |
| 1D1-01 | 0.524 | 94465 | 0.322 | 11288 |
| B7H3-C1 | 1.709 | 83992 | 1.865 | 8273 |

**Table 2-2. Test results for cell affinity of 2E3-02 antibody.**

| Antibody | MCF-7 tumor cells | | A549 tumor cells | | PC-3 tumor cells | |
|---|---|---|---|---|---|---|
| | EC₅₀ (nM) | Maximum MFI | EC₅₀ (nM) | Mximum MFI | EC₅₀ (nM) | Maximum MFI |
| 2E3-02 | 0.398 | 77732 | 0.150 | 84806 | 0.063 | 9729 |
| B7H3-C1 | 1.360 | 63194 | 1.054 | 47956 | 0.618 | 6436 |
| B7H3-C2 | 0.278 | 27925 | Not detected | Not detected | 0.167 | 2170 |

As shown in Tables 2-1 and 2-2, for high B7H3-expressing tumor cells MCF-7, the affinity of 1D1-01 and 2E3-02 was 0.524 nM and 0.398 nM, respectively, which were much higher than that of B7H3-C1, and the maximum fluorescence signal values were 12% and 23% higher than that of B7H3-C1, respectively, and the maximum fluorescence signal value of 2E3-02 was 2.78 times higher than that of control antibody 2. For middle B7H3-expressing tumor cells A549, the affinities of 1D1-01 and 2E3-02 were 0.322nM and 0.150nM, respectively, which were much higher than that of B7H3-C1, and the maximum fluorescence signal value was 36% and 77% higher than that of B7H3-C1, respectively. For low B7H3-expressing PC-3 tumor cells, the affinity of 2E3-02 with 0.06nM was much higher than that of B7H3-C1 with 0.618nM, and also higher than that of B7H3-C1 with 0.167nM, and the maximum fluorescence signal values were 1.51 times and 4.48 times higher than that of B7H3-C1 and B7H3-C2 respectively. In conclusion, anti-B7H3 antibodies 1D1-01 and 2E3-02 had better affinity to tumor cell than that of B7H3-C1 and B7H3-C1.

### Example 3.7 Dynamic affinity detection of anti-B7H3 antibody

Fortibio is a commonly used dynamic affinity detection device by which the dynamic affinity of anti-B7H3 antibodies to B7H3 proteins was detected. The method was briefly described as follows: serial dilution of human B7H3-4IgG-His, human B7H3-2IgG-His or monkey B7H3-4IgG-His was performed using PBST, to obtain 200 nM, 100 nM, 50 nM, 25 nM, 12.5 µM, 6.25 nM, 3.125 nM, 1.5625nM and 0 nM. The ProA biosensor (Pall Life Sciences) was pre-humidified with PBST buffer prior to use. Anti-B7H3 antibody was diluted to 5 µg/mL with PBST and immobilized on the ProA sensor. The antibody-immobilized sensors were then equilibrated in PBST buffer for 60 s to obtain a baseline, then transferred to the antigen diluent for binding for 60 s, followed by dissociation in PBST for 180 s. After one analytical cycle, the sensor was regenerated with 10 mM Gly (pH 1.5). The association (Ka) and dissociation (Kd) rate constants were determined using Date analysis with a 1:1 model and used to calculate the dissociation equilibrium constant (KD).

As shown in Table 3, the dynamic affinity of 1D1-01 and 2E3-02 to human B7H3-4Ig-his is 4.3 E-11M and 2.4E-10M, which is much higher than that of antibody B7H3-C1 and antibody B7H3-C2. Meanwhile, the dynamic affinity of 1D1-01 and 2E3-02 to monkey B7H3-4Ig-his is E-10M and has good monkey crossing. The dynamic affinity folds of 1D1-01 and 2E3-02 to human B7H3-4Ig-his/human B7H3-2Ig-his were 9.5 and 32.5 respectively.

**Table 3. Dynamic affinity analysis of anti-B7H3 antibody molecules.**

| Antibody | Human B7H3-4Ig-his affinity | Human B7H3-2Ig-his affinity | Monkey B7H4-4Ig-his affinity |
|---|---|---|---|
| | KD (M) | KD (M) | KD (M) |
| 1D1-01 | 4.3E-11 | 4.1E-10 | 2.5E-10 |
| 2E3-02 | 2.4E-10 | 7.8E-09 | 4.7E-10 |
| B7H3-C1 | 5.4E-10 | 4.8 E-08 | 2.0 E-10 |
| B7H3-C2 | 1.8E-09 | 7.1E-08 | 2.5 E-09 |

### Example 3.8 Specificity detection of anti-B7H3 antibody

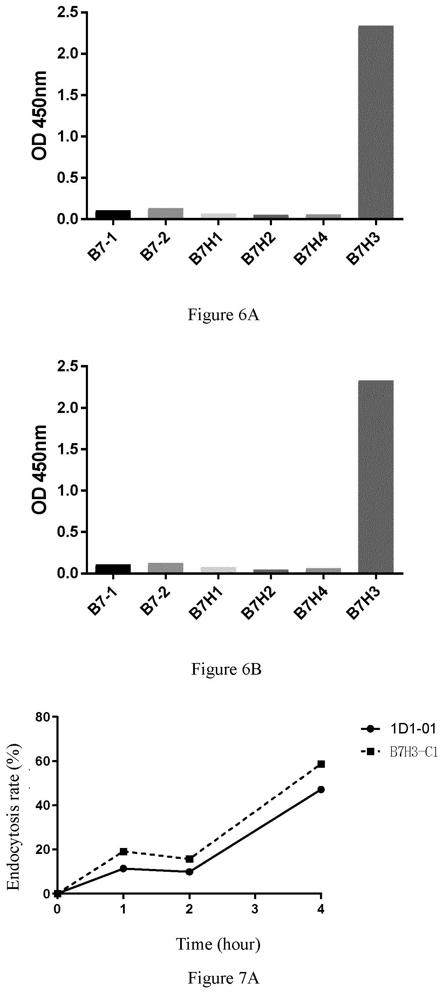

The specificity of anti-B7H3 antibody binding to B7H3 was determined by ELISA method. Specific experimental procedures: human B7-1, human B7-2, human B7H1-his, human B7H2-his, human B7H3-his and human B7H4-his proteins (purchased from Sino Biological Inc.) are diluted with carbonate buffer (pH 9.6) to a final concentration of 1 µg/mL, respectively, and added into 96-well enzyme labelling wells at 100 µL/well, and coated overnight at 4 °C; the coating solution was discarded, and the plate was washed once with PBST, then 100 µL PBS (containing 2% BSA) was added to each well, and the plate was blocked at 37 °C for 1h; then, 100 µL of B7H3 full human antibody diluted with PBS (containing 2% BSA) was added to each well at 10 µg/mL, and the plate was incubated at 37°C for 2h; the plate was washed 3 times with PBST, and horseradish peroxidase-labeled anti-human Fc antibody ( (1:10000 dilution with PBS) was added at 100 µL/well, then incubated at 37°C for 1 h; the plate was washed 5 times with PBST. TMB chromogenic solution for color development was added at 100 µL/well, incubated at 37°C for 7 min, and then the stop solution was added to terminate the reaction at 50 µL/well, and the optical density was measured at 450 nm. The results of 1D1-01 and 2E3-02 are shown in Figures 6A and 6B, respectively. As shown by the results, 1D1-01 and 2E3-02 specifically bound to human B7H3 protein, but did not bind to human B7-1, human B7-2, human B7H1, human B7H2, and human B7H4 proteins.

### Example 3.9 Detection of endocytic activity of anti-B7H3 antibody

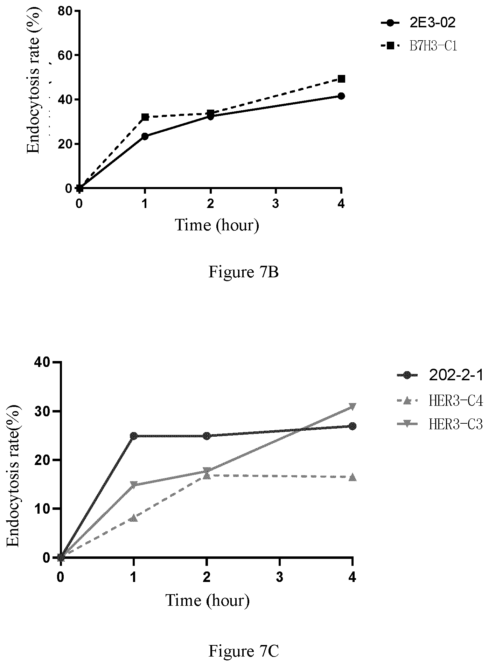

The endocytosis of anti-B7H3 antibody by A549 human non-small cell lung cancer cells was detected by FACS. The A549 cells grown to the logarithmic growth phase were digested with trypsin, and then the cells were collected by centrifugation and washed three times with pre- chilled PBS; the cells were resuspended in PBS (containing 1% BSA), to which was added the anti-B7H3 antibody to be tested at a concentration of 10 µg/mL, followed by incubation at 4 °C for 1 h; cells were collected by centrifugation, washed three times with PBS, resuspended in DMEM+10% FBS, divided into 4 parts, and incubated at 37 °C for 0, 1, 2, and 4 hours respectively; after completion of the incubation phase, the cells were collected by centrifugation, washed three times with pre-cooled PBS, resuspended in 50µL of 1% BSA (in PBS), and then 1µL of fluorescent secondary antibody APC anti-Human IgG (Biolegend) was added to each well, mixed well, and incubated at 4°C for 0.5 h; cells were collected by centrifugation, washed three times with pre-chilled PBS, resuspended in 200µL of PBS, and tested on the apparatus. According to the formula: endocytosis ratio (%) = [1-(the average fluorescence value of the detection sample at this time point - the average fluorescence value of the negative control sample at this time point)/ (the average fluorescence value of the detection sample at 0 h - the average fluorescence value of the negative control sample at 0 h)] *100. The results of 1D1-01 and 2E3-02 are shown in Figures 7A and 7B, respectively, and anti-B7H3 antibodies 1D1-01 and 2E3-02 have strong endocytosis activities for A549 tumor cells, reaching about 40% at 4 hours, which is comparable to antibody B7H3-C1.

### Example 3.10 Competitive detection of anti-B7H3 antibody epitopes

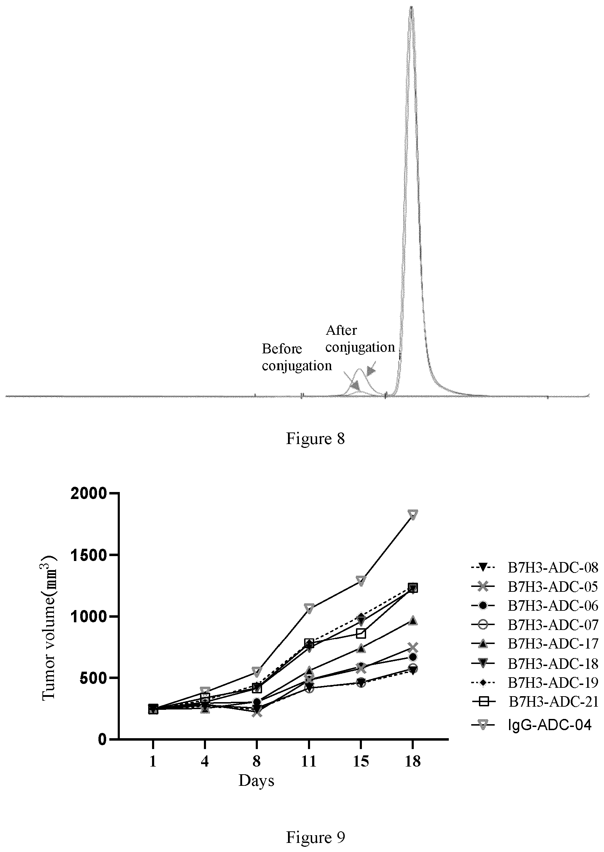

A competitive ELISA was used to determine whether anti-B7H3 antibodies 1D1-01 and 2E3-02 competed with the antibody B7H3-C1 for epitopes. The specific steps were as follows: human B7H3-4Ig-his antigen was coated with CBS coating solution at 100 ng/well, and then the plate was incubated at 4 °C overnight; the coating solution was removed, and the wells were washed once with 300µL of PBS, then each well was blocked with PBS (containing 2% BSA) solution at 100µL/well, followed by incubation at 37 °C for 2 h; the blocking solution was discarded, and antibodies 1D1-01 and 2E3-02 were diluted with 2 folds dilution starting from 40µg/mL with a total of 11 dilution points, but the 12th point was replaced by the diluent; the antibody was added to the wells at 50µL/well in duplicate; biotin-labeled B7H3-C1 diluted to 50 ng/mL was added to each well at 50µL/well, and then incubated at 25 °C for 2 h; the wells were washed with 300µL of PBST, repeated 3 times, and then added with HRP anti-biotin secondary antibody diluted with PBS (containing 2% BSA) at a ratio of 1:5000 at 100µL/well, followed by incubating at 25 °C for 1 h; the wells were washed with 300µL of PBST, repeated 5 times; 100µL of TMB chromogenic solution (Huzhou InnoReagents, Anhui) was added to each well, and after 5 minutes of color development at room temperature, 50µL of H₂SO₄ was added to each well to stop the reaction, and the OD450nm was read immediately on a microplate reader. The original data were imported into Graph-prism to calculate EC50 values, and the results are shown in Figures 8A and 8B. The results showed that anti-B7H3 antibody 1D1-01 competed with antibody B7H3-C1 for binding epitopes; anti-B7H3 antibody 2E3-02 did not compete with antibody B7H3-C1 for binding epitopes.

### Example 3.11 Preparation of Her3 antigen

The nucleic acid sequence for amino acids at positions 1-643 of human Her3(NCBI: NP_001973.2) was selected, in which a 6×His detection tag was added to the C-terminal and named as human Her3-his. The nucleic acid sequence of monkey Her3 was selected, in which a 6×His detection tag was added to the C-terminal and named as monkey Her3-his. The nucleic acid sequence of rat Her3 was selected, in which a 6x His detection tag was added to the C-terminal and named as monkey Her3-his.

### Example 3.12 Expression of anti-Her3 antibody

The VH (SEQ ID NO:49) of antibody 202-2-1 was added with the constant region of heavy chain IgG1 (SEQ NO: 43), and the VL (SEQ ID NO:50) of antibody 202-2-1 were added with the Kappa constant region sequence (SEQ ID NO: 44). Antibody Her3-C3 (patritumab) was from Daiichi Sankyo Company Limited, TYO, with the sequence from IMGT database IMGT/mAb-DB ID: 964, INN number: 11093, and the antibody Her3-C4 was from MediaPharma company patent CN 103189392 B (heavy chain SEQ ID NO: 10, light chain SEQ ID NO: 14), and then by codon optimization and gene synthesis, said antibodies were constructed into PTT5 vectors; the expression plasmids of anti-Her3 antibody heavy chain and light chain were co-transfected into HEK293F cells by PEI max reagent and expressed in a 5% CO₂ shaker at 37 °C for 7 days. Supernatants were collected and purified by ProA magnetic beads to obtain anti-Her3 antibodies named as 202-2-1 (whose heavy chain sequence is SEQ ID NO 51, and whose light chain sequence is SEQ ID NO 52) and Her3-C3, respectively.

### Example 3.13 Protein affinity detection of anti-Her3 antibody

The affinity of anti-Her3 antibody to Her3 protein was detected by ELISA experimental method. Specific experimental procedures: human Her3-his, monkey Her3-his protein and rat Her3-his was diluted with carbonate buffer with a pH value of 9.6 to a final concentration of 1µg/mL, and then added to 96-well enzyme labelling wells at 100µL/well, and coated overnight at 4°C; the coating solution was discarded, and the plate was washed once with PBST, then 100 µL of PBS (containing 2% BSA) was added to each well, and blocked at 37 °C for 1 h; then, 100 µL of anti-Her3 antibody diluted with PBS (containing 2% BSA) ((starting at 1 µg, 3 folds serial dilution, in duplicate wells) was added to each well, and then incubated at 37 °C for 2h; the plate was washed three times with PBST, and horseradish peroxidase-labeled anti-human Fc antibody (diluted at 1:10000 with PBS) was added at 100µL/well, then reacted for 1 h at 37 °C. The plate was washed 5 times with PBST. TMB chromogenic solution was added at 100µL/well and incubated at 37°C for 7 min to develop color, and then the stop solution was added at 50µL/well to terminate the reaction. The absorbance was measured at 450 nm, and EC₅₀ values were calculated from original data imported into Graph-prism. The results are shown in Table 4 in detail. 202-2-1 has strong binding to human Her3 protein and monkey Her3 protein, which is comparable to antibody Her3-C4. Both of them do not bind to rat Her3 protein.

**Table 4. Detection results for protein affinity of anti-Her3 antibody.**

| Antibody name | Human Her3-his EC50 (pM) | Monkey Her3-his EC50 (pM) | Rat Her3-his EC50 (pM) |
|---|---|---|---|
| 202-2-1 | 20.97 | 15.91 | Not binding |
| Her3-C4 | 27.72 | 9.275 | Not binding |

### Example 3.14 Detection of endocytic activity of anti-Her3 antibody

The endocytosis of anti-Her3 antibody by A549-human Her3 (non-small cell lung cancer cells) was detected by FACS. The A549-human Her3 cells grown to the logarithmic growth phase were digested with trypsin, and then the cells were collected by centrifugation and washed three times with pre-chilled PBS; the cells were resuspended in PBS (containing 1% BSA), to which was added the anti-Her3 antibody to be tested at a concentration of 10µg/mL, followed by incubation at 4 °C for 1 h; cells were collected by centrifugation, washed three times with PBS, resuspended in DMEM+10% FBS, divided into 4 parts, and incubated at 37 °C for 0, 1, 2, and 4 hours respectively; after completion of the incubation phase, the cells were collected by centrifugation, washed three times with pre-chilled PBS, resuspended in 50µL of 1% BSA (in PBS), and then 1µL of fluorescent secondary antibody APC anti-Human IgG (Biolegend) was added to each well, mixed well, and incubated at 4°C for 0.5 h; cells were collected by centrifugation, washed three times with pre-chilled PBS, resuspended in 200µL of PBS, and tested on the flow cytometer (Beckman, Version CytoFlex). According to the formula: endocytosis ratio (%) = [1-(the average fluorescence value of the detection sample at this time point - the average fluorescence value of the negative control sample at this time point)/(the average fluorescence value of the detection sample at 0 h - the average fluorescence value of the negative control sample at 0 h)]*100. The results are shown in Figures 7C, and anti-HER3 antibody 202-2-1 has strong endocytosis activities for A549 tumor cells, reaching about 26.9% at 4 hours, which is 16.6% stronger than antibody Her3-C4 (16.6%) and comparable to antibody Her3-C3. However, the endocytosis of anti-Her3 antibody 202-2-1 reached the maximum endocytosis rate before Her3-C4/Her3-C3, and had a faster endocytosis rate than Her3-C4/Her3-C3.

### IV. Conjugation of compounds containing cellular bioactive molecules and linkers with antibodies

### Example 4.1: Preparation of B7H3-ADC

### Example 4.1.1: Preparation of B7H3-ADC-01

0.3 mL of anti-B7H3 antibody (2E3-02 antibody, 33.5 mg/mL) was taken and diluted with 0.25 mL of 20 mM PB + 150 mM NaCl + 20 mM sodium edetate solution (pH 7.6), to which was then added 0.45 mL of 20 mM PB + 150 mM NaCl solution (pH 7.6), and mixed homogeneously, whose pH was adjusted to 7.4 with 1M K₂HPO₄ solution, followed by addition of 10 mM TCEP (tris(2-carboxyethyl)phosphine, 0.033 ml, 0.33 µmol) solution. The mixture was mixed homogeneously, and left at room temperature for 30 min. A solution of DL001 (1.35 mg, 20-folds molar amount of antibody substance) in dimethyl sulfoxide (0.1 mL) was added to the above solution, and the resultant solution was mixed homogeneously, allowed to stand for 2 h at room temperature, and after that, 6.1 µl of 100 mM cysteine was added to stop the reaction. Finally, the buffer solution was replaced with 20 mM of PB buffer solution at pH 6.44 using a G-25 gel column. The coupling product B7H3-ADC-01 of DL-001 and anti-B7H3 antibody was obtained. The DAR value was measured to be 7.8 by mass spectrometry.

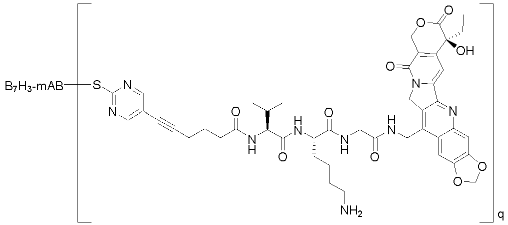

### Example 4.1.2: Preparation of B7H3-ADC-02

Compound DL-001 in Example 4.1.1 was replaced by compound DL-011 to obtain the coupling product B7H3-ADC-02 of DL-011 and anti-B7H3 antibody. The DAR value was measured to be 7.8 by mass spectrometry.

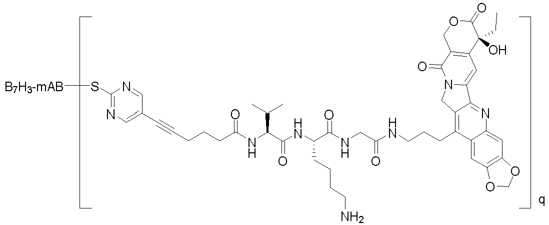

### Example 4.1.3: Preparation of B7H3-ADC-03

Compound DL-001 in Example 4.1.1 was replaced by compound DL-020 to obtain the coupling product B7H3-ADC-03 of DL-020 and anti-B7H3 antibody. The DAR value was measured to be 7.8 by mass spectrometry.

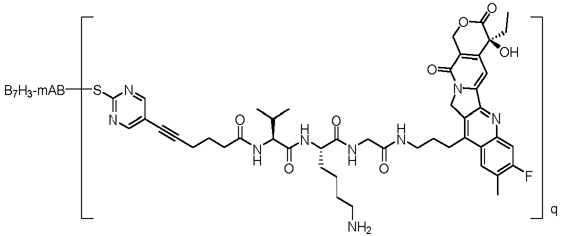

### Example 4.1.4: Preparation of B7H3-ADC-04

Compound DL-001 in Example 4.1.1 was replaced by compound DL-019 to obtain the coupling product B7H3-ADC-04 of DL-019 and anti-B7H3 antibody. The DAR value was measured to be 7.8 by mass spectrometry.

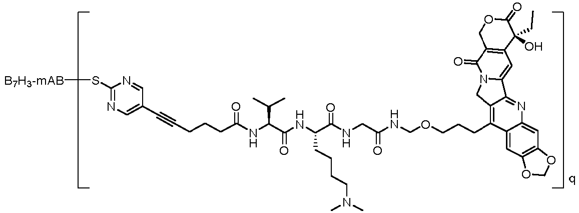

### Example 4.1.5.1: Preparation of B7H3-ADC-05(DAR8)

Compound DL-001 in Example 4.1.1 was replaced by compound DL-018 to obtain the coupling product B7H3-ADC-05 of DL-018 and anti-B7H3 antibody. The DAR value was measured to be 7.8 by mass spectrometry.

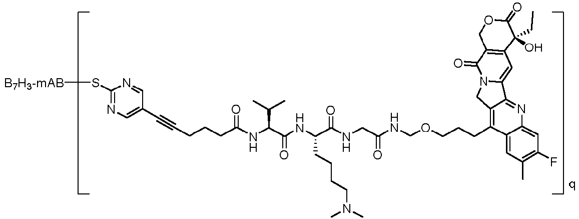

### Example 4.1.5.2: Preparation of B7H3-ADC-05(DAR4)

Compound DL-037 in Example 4.1.7.3 was replaced by compound DL-018 to obtain the coupling product B7H3-ADC-05(DAR4) of DL-018 and anti-B7H3 antibody. The DAR value was determined by mass spectrometry.

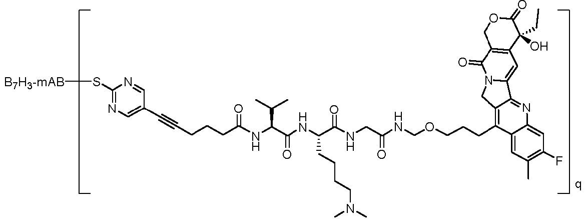

### Example 4.1.5.3: Preparation of B7H3-C1-ADC-05(DAR4)

Compound DL-037 in Example 4.1.7.3 was replaced by compound DL-018, and antibody 2E3-02 was replaced by antibody B7H3-C1, to obtain the coupling product B7H3-C1-ADC-05 (DAR4) of DL-018 and anti-B7H3 antibody. The DAR value was determined by mass spectrometry.

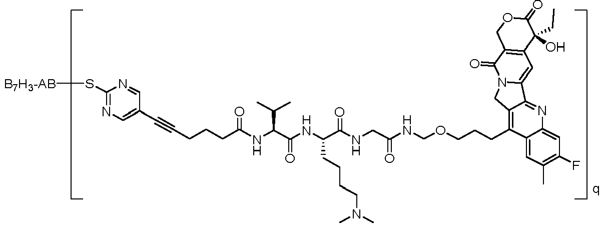

### Example 4.1.6: Preparation of B7H3-ADC-06

Compound DL-001 in Example 4.1.1 was replaced by compound DL-036 to obtain the coupling product B7H3-ADC-06 of DL-036 and anti-B7H3 antibody. The DAR value was measured to be 7.7 by mass spectrometry.

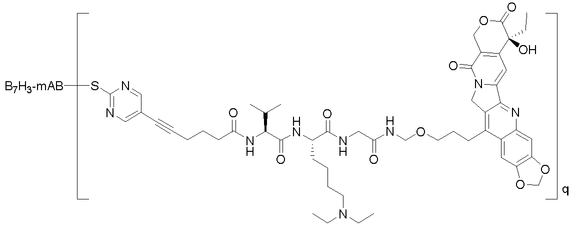

### Example 4.1.7: Preparation of B7H3-ADC-07

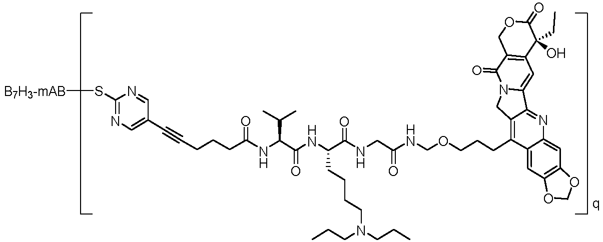

### 4.1.7.1 Preparation of sample B7H3-ADC-07(DAR8)

Preparation method A: compound DL-001 in Example 4.1.1 was replaced by compound DL-037 to obtain the coupling product of B7H3-ADC-07(DAR8) of DL-037 and anti-B7H3 antibody. The DAR value was measured to be 7.7 by mass spectrometry.

Preparation method B: 25 ml of antibody 2E3-02 (anti-B7H3, concentration 28.5 mg/ml, 20 mM acetate buffer), was taken, to which was added 25 ml of 20 mM acetate buffer for dilution, and then 1 ml of 0.25 M EDTA aqueous solution was added and mixed homogeneously. The pH of the sample was adjusted to 7.6 with 0.5 M disodium hydrogen phosphate aqueous solution, and then 20 mM TCEP (tris(2-carboxyethyl)phosphine hydrochloride) solution was added at 4.5-fold equivalents relative to the antibody and mixed homogeneously. The mixture was allowed to react at room temperature for 90 min. Finally, 10-fold equivalents (relative to the antibody) of DL-037 dissolved in DMSO was added, and the reaction was continued for 2 h at room temperature after mixing homogeneously. After completion of the reaction, a 30KDa ultrafiltration tube was used to transfer the sample into a 10 mM histidine buffer (pH 5.5), and remove low molecular weight substances. Finally, the sample was concentrated to obtain a solution containing the anti-B7H3 antibody ADC composition B7H3-ADC-07 (DAR8), whose DAR value was measured to be 7.98 by mass spectrometry.

### 4.1.7.2 Preparation of sample B7H3-ADC-07(DAR2)

5 ml of antibody 2E3-02 (anti-B7H3, concentration 28.5 mg/ml, 20 mM acetate buffer) was taken, to which was added 5 ml of 20 mM acetate buffer for dilution, and then 0.2 ml of 0.25 M EDTA aqueous solution was added and mixed homogeneously. The pH of the sample was adjusted to 7.6 with 0.5 M disodium hydrogen phosphate aqueous solution, and then 20 mM TCEP (tris(2-carboxyethyl)phosphine hydrochloride) solution was added at 4.5-fold equivalent relative to the antibody and mixed homogeneously. The mixture was allowed to react at room temperature for 90 min. Finally, 2.4-fold equivalent (relative to the antibody) of DL-037 dissolved in DMSO was added, and the reaction was continued for 2 h at room temperature after mixing homogeneously. After completion of the reaction, a 30KDa ultrafiltration tube was used to transfer the sample into a 10 mM histidine buffer solution (pH 5.5), and remove low molecular weight substances. Finally, the sample was concentrated to obtain a solution containing the anti-B7H3 antibody ADC composition B7H3-ADC-07 (DAR2), DAR value of which was measured to be 2.3 by mass spectrometry.
4.1.7.3

### (1) Preparation of sample B7H3-ADC-07(DAR4)

5 ml of antibody 2E3-02 (anti-B7H3, concentration 28.5 mg/ml, 20 mM acetate buffer) was taken, to which was added 5 ml of 20 mM acetate buffer for dilution, and then 0.2 ml of 0.25 M EDTA aqueous solution was added and mixed homogeneously. The pH of the sample was adjusted to 7.6 with 0.5 M disodium hydrogen phosphate aqueous solution, and then 20 mM TCEP (tris(2-carboxyethyl)phosphine hydrochloride) solution was added at 4.5-fold equivalent relative to the antibody and mixed homogeneously. The mixture was allowed to react at room temperature for 90 min. Finally, 4.8-fold equivalent (relative to the antibody) of DL-037 dissolved in DMSO was added, and the reaction was continued for 2 h at room temperature after mixing homogeneously. After completion of the reaction, a 30KDa ultrafiltration tube was used to transfer the sample into a 10 mM histidine buffer (pH 5.5), and remove low molecular weight substances. Finally, the sample was concentrated to obtain a solution containing the anti-B7H3 antibody ADC composition B7H3-ADC-07 (DAR4), whose DAR value was measured to be 4.1 by mass spectrometry.

### (2) Preparation of sample B7H3-C1-ADC-07(DAR4)

Using the method for preparing B7H3-ADC-07(DAR4), antibody 2E3-02 was replaced by antibody B7H3-Cl, to obtain the solution of antibody B7H3-C1 ADC composition B7H3-C1-ADC-07(DAR4), and the DAR value was measured to be 4.9 by mass spectrometry.

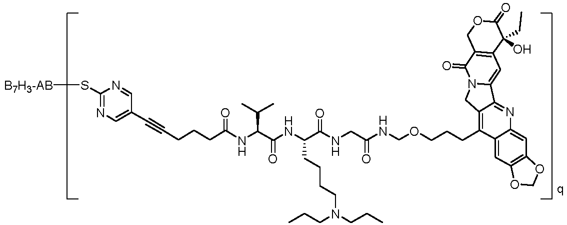

### 4.1.7.4 Preparation of sample B7H3-ADC-07(DAR6)

5 ml of antibody 2E3-02 (anti-B7H3, concentration 28.5 mg/ml, 20 mM acetate buffer) was taken, to which was added 5 ml of 20 mM acetate buffer for dilution, and then 0.2 ml of 0.25 M EDTA aqueous solution was added and mixed homogeneously. The pH of the sample was adjusted to 7.6 with 0.5 M disodium hydrogen phosphate aqueous solution, and then 20 mM TCEP (tris(2-carboxyethyl)phosphine hydrochloride) solution was added at 4.5-fold equivalent relative to the antibody and mixed homogeneously. The mixture was allowed to react at room temperature for 90 min. Finally, 7.2-fold equivalent (relative to the antibody) of DL-037 dissolved in DMSO was added, and the reaction was continued for 2 h at room temperature after mixing homogeneously. After completion of the reaction, a 30KDa ultrafiltration tube was used to transfer the sample into a 10 mM histidine buffer (pH 5.5), and remove low molecular weight substances. Finally, the sample was concentrated to obtain a solution containing the anti-B7H3 antibody ADC composition B7H3-ADC-07 (DAR6), DAR value of which was measured to be 5.4 by mass spectrometry.

### Example 4.1.8: Preparation of B7H3-ADC-08

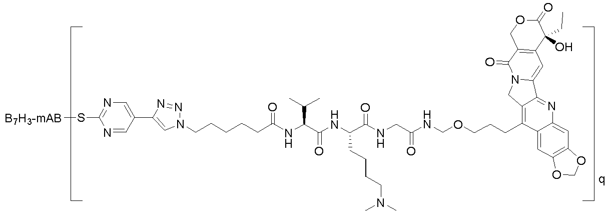

### 4.1.8.1 Preparation of sample B7H3-ADC-08(DAR8)

Compound DL-001 in Example 4.1.1 was replaced by compound DL-028 to obtain the coupling product B7H3-ADC-08(DAR8) of DL-028 and antibody B7H3. The DAR value was measured to be 7.3 by mass spectrometry.

Meanwhile, compound DL-037 in preparation method B of Example 4.1.7 was replaced by compound DL-028 to obtain the coupling product B7H3-ADC-08(DAR8) of DL-028 and antibody B7H3. The DAR value was determined by mass spectrometry, and results were shown as follows:

| Peptide chain | Theoretical value | Experimental value | Area | Ratio | DAR |
|---|---|---|---|---|---|
| LC | 23335 | Not detected | 0 | 0 | |
| LC+DAR1 | 24368.4 | 23247.05 | 70520268 | 1 | 7.321946 |
| HC | 50735 | 50371.9 | 409745 | 0.068972 | |
| HC+DAR1 | 51768.4 | 51405.98 | 151226 | 0.025456 | |
| HC+DAR2 | 52801.8 | 52438.75 | 482382 | 0.081199 | |
| HC+DAR3 | 53835.2 | 53471.3 | 4897381 | 0.824373 | |

4.1.8.2

### (1) Preparation of sample B7H3-ADC-08(DAR4)

Compound DL-037 in the preparation of B7H3-ADC-07(DAR4) of Example 4.1.7.3 was replaced by compound DL-028 to obtain the coupling product B7H3-ADC-08(DAR4) of DL-028 and anti-B7H3 antibody. The DAR value was measured to be 4.7 by mass spectrometry.

### (2) B7H3-C1-ADC-08(DAR4)

Compound DL-037 in the preparation of B7H3-C1-ADC-07(DAR4) of Example 4.1.7.3 was replaced by compound DL-028 to obtain the coupling product B7H3-C1-ADC-08 of DL-028 and anti-B7H3 antibody. The DAR value was measured to be 4.7 by mass spectrometry.

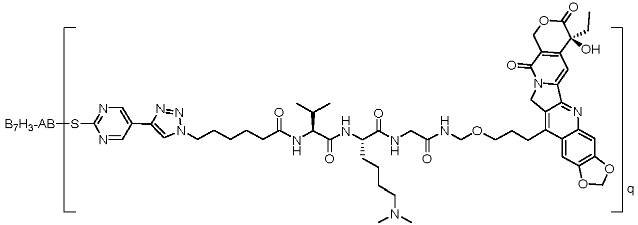

### Example 4.1.9: Preparation of B7H3-ADC-09

Compound DL-001 in Example 4.1.1 was replaced by compound DL-029 to obtain the coupling product of B7H3-ADC-09 of DL-029 and anti-B7H3 antibody. The DAR value was measured to be 7.6 by mass spectrometry.

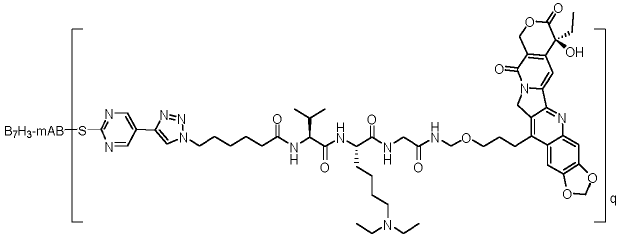

Meanwhile, compound DL-037 in the preparation method B of Example 4.1.7 was replaced by compound DL-029 to obtain the coupling product B7H3-ADC-09 of DL-029 and anti-B7H3 antibody. The DAR value was determined by mass spectrometry, and the results were as follows:

| Peptide chain | Theoretical value | Experimental value | Area | Ratio | DAR |
|---|---|---|---|---|---|
| LC | 23245 | 23247.03 | 6274384 | 0.094179 | |
| LC+DAR1 | 24306 | 24308.41 | 60347328 | 0.905821 | 7.811641 |
| HC | 50367 | Not detected | 0 | 0 | |
| HC+DAR1 | 51428 | Not detected | 0 | 0 | |
| HC+DAR2 | 52489 | Not detected | 0 | 0 | |
| HC+DAR3 | 53550 | 53555.46 | 10285189 | 1 | |

### Example 4.1.10: preparation of B7H3-ADC-10

Compound DL-001 in Example 4.1.1 was replaced by compound DL-030 to obtain the coupling product B7H3-ADC-10 of DL-030 and anti-B7H3 antibody. The DAR value was measured to be 7.6 by mass spectrometry.

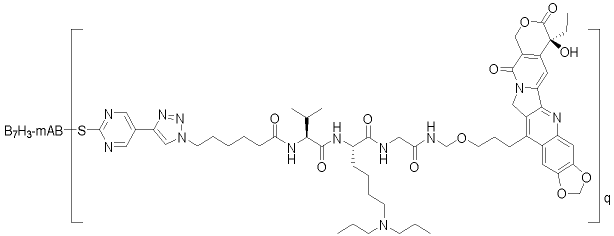

Meanwhile, compound DL-037 in the preparation method B of Example 4.1.7 was replaced by compound DL-030 to obtain the coupling product B7H3-ADC-10 of DL-030 and antibody B7H3. The DAR value was determined by mass spectrometry, and the results were as follows:

| Peptide chain | Theoretical value | Experimental value | Area | Ratio | DAR |
|---|---|---|---|---|---|
| LC | 23245 | Not detected | 0 | 0 | |
| LC+DAR1 | 24194.4 | 24336.45 | 66512554 | 1 | 8 |
| HC | 50367 | Not detected | 0 | 0 | |
| HC+DAR1 | 51316.4 | Not detected | 0 | 0 | |
| HC+DAR2 | 52165.8 | Not detected | 0 | 0 | |
| HC+DAR3 | 53215.2 | Not detected | 11529758 | 1 | |

### Example 4.1.11: Preparation of B7H3-ADC-11

Compound DL-001 in Example 4.1.1 was replaced by compound DL-045 to obtain the coupling product B7H3-ADC-11 of DL-045 and antibody B7H3. The DAR value was measured to be 7.5 by mass spectrometry.

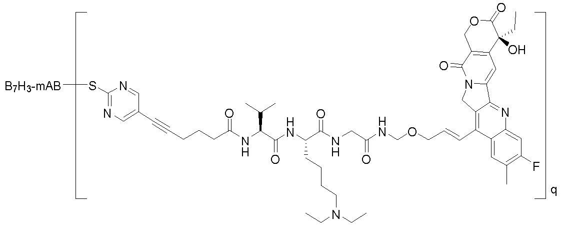

### Example 4.1.12:

Compound DL-001 in Example 4.1.1 was replaced by compound DL-046 to obtain the coupling product B7H3-ADC-12 of DL-046 and anti-B7H3 antibody. The DAR value was measured to be 7.6 by mass spectrometry.

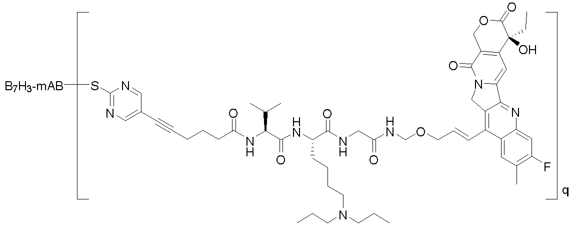

### Example 4.1.13: Preparation of B7H3-ADC-13

Compound DL-001 in Example 4.1.1 was replaced by compound DL-041 to obtain the coupling product B7H3-ADC-13 of DL-041 and anti-B7H3 antibody. The DAR value was measured to be 7.8 by mass spectrometry.

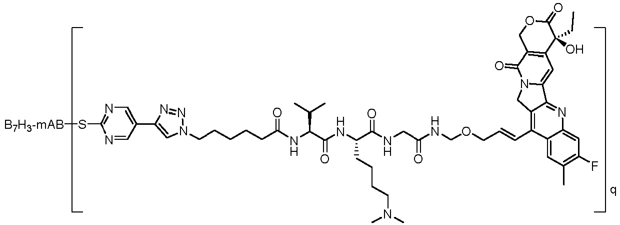

### Example 4.1.14: Preparation of B7H3-ADC-14

Compound DL-001 in Example 4.1.1 was replaced by compound DL-042 to obtain the coupling product B7H3-ADC-14 of DL-042 and anti-B7H3 antibody. The DAR value was measured to be 7.9 by mass spectrometry.

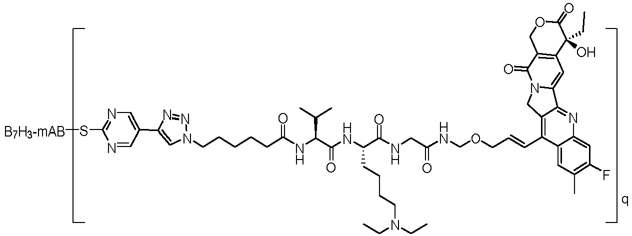

### Example 4.1.15: Preparation of B7H3-ADC-15

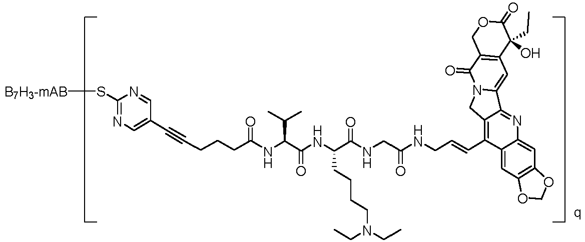

### 4.1.15.1 Preparation of sample B7H3-ADC-15(DAR8)

Compound DL-001 in Example 4.1.1 was replaced by compound DL-027 to obtain the coupling product B7H3-ADC-15(DAR8) of DL-027 and anti-B7H3 antibody. The DAR value was measured to be 7.8 by mass spectrometry.

### 4.1.15.2 Preparation of B7H3-ADC-15(DAR4)

Compound DL-037 in the preparation of B7H3-ADC-07(DAR4) of Example 4.1.7.3 was replaced by compound DL-027 to obtain the coupling product B7H3-ADC-15(DAR4) of DL-027 and anti-B7H3 antibody. The DAR value was measured to be 3.4 by mass spectrometry.

### Example 4.1.16: Preparation of B7H3-ADC-16

Compound DL-001 in Example 4.1.1 was replaced by compound DL-043 to obtain the coupling product B7H3-ADC-16 of DL-043 and anti-B7H3 antibody. The DAR value was measured to be 7.4 by mass spectrometry.

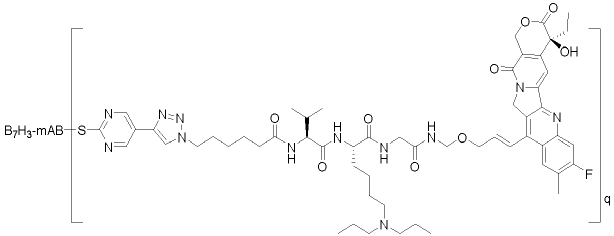

### Example 4.1.17: Preparation of B7H3-ADC-17

Compound DL-037 in the preparation method B of Example 4.1.7 was replaced by compound DL-024 to obtain the coupling product B7H3-ADC-17 of DL-024 and anti-B7H3 antibody. The DAR value was determined by mass spectrometry.

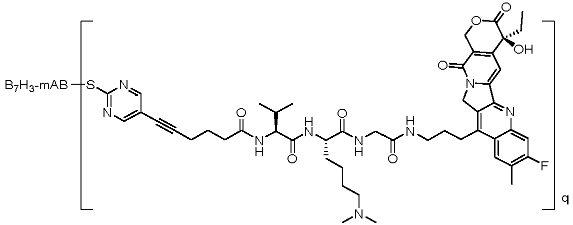

### Example 4.1.18: preparation of B7H3-ADC-18

Compound DL-037 in the preparation method B of Example 4.1.7 was replaced by compound DL-025 to obtain the coupling product B7H3-ADC-18 of DL-025 and anti-B7H3 antibody. The DAR value was measured to be 8.0 by mass spectrometry.

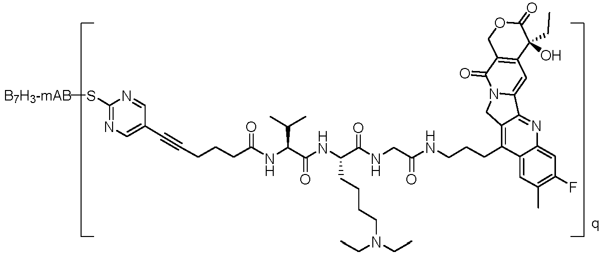

### Example 4.1.19: Preparation of B7H3-ADC-19

Compound DL-037 in the preparation method B of Example 4.1.7 was replaced by compound DL-026 to obtain the coupling product B7H3-ADC-19 of DL-026 and anti-B7H3 antibody. The DAR value was measured to be 8.0 by mass spectrometry.

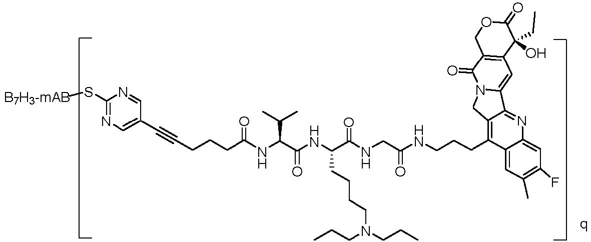

### Example 4.1.20: Preparation of B7H3-ADC-20

Compound DL-037 in the preparation method B of Example 4.1.7 was replaced by compound DL-038 to obtain the coupling product B7H3-ADC-20 of DL-038 and anti-B7H3 antibody. The DAR value was measured to be 7.3 by mass spectrometry.

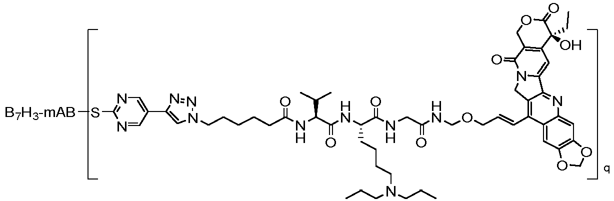

| Peptide chain | Theoretical value | Experimental value | Area | Ratio | DAR |
|---|---|---|---|---|---|
| LC | 23245 | 23247.17 | 29131808 | 0.323854 | |
| LC+DAR1 | 24331.5 | 24334.63 | 60821684 | 0.676146 | 7.306055 |
| HC | 50375 | Not detected | 0 | 0 | |
| HC+DAR1 | 51461 | Not detected | 0 | 0 | |
| HC+DAR2 | 52547.83 | 52547.83 | 159238 | 0.023119 | |
| HC+DAR3 | 53633.84 | 53633.84 | 6728635 | 0.976881 | |

### Example 4.1.21: Preparation of B7H3-ADC-21

With reference to CN104755494B, the following drug-linker compound was prepared:

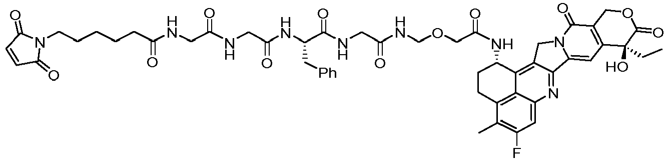

Compound DL-037 in the preparation method B of Example 4.1.7.1 was replaced by the above drug-linker compound, to obtain B7H3-ADC-21(DAR8), the structure of which was as follows, and the DAR value was measured to be 7.8 by mass spectrometry.

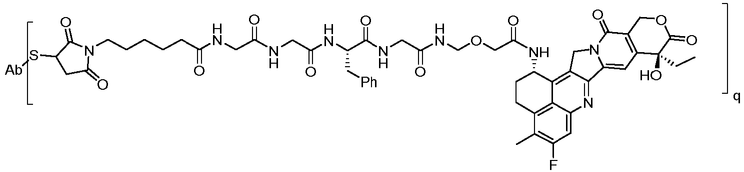

Compound DL-037 in Example 4.1.7.2 was replaced by the above drug-linker compound, to obtain B7H3-ADC-21(DAR2), the structure of which was as follows, and the DAR value was measured to be 2.0 by mass spectrometry.

Compound DL-037 in Example 4.1.7.3 was replaced by the above drug-linker compound, and antibody 2E3-02 was replaced by the control antibody 1, to obtain B7H3-C1-ADC-21(DAR4), the structure of which was as follows, and the DAR value was measured to be 4.5 by mass spectrometry.

Compound DL-037 in Example 4.1.7.4 was replaced by the above drug-linker compound, to obtain B7H3-ADC-21(DAR6), the structure of which was as follows, and the DAR value was measured to be 5.4 by mass spectrometry.

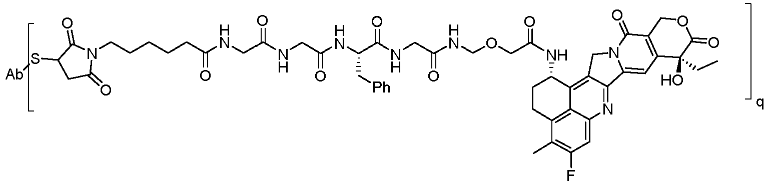

### Example 4.1.22: Preparation of B7H3-ADC-22

Compound DL-037 in the preparation method B of Example 4.1.7 was replaced by compound DL-048 to obtain the coupling product B7H3-ADC-22 of DL-048 and anti-B7H3 antibody. The DAR value was measured to be 8.0 by mass spectrometry.

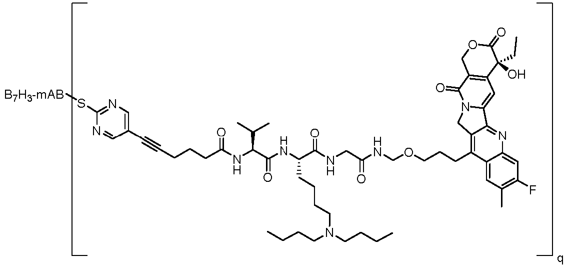

### Example 4.1.23: Preparation of B7H3-ADC-23

Compound DL-037 in the preparation method B of Example 4.1.7 was replaced by compound DL-031 to obtain the coupling product B7H3-ADC-23 of DL-031 and anti-B7H3 antibody. The DAR value was measured to be 8.0 by mass spectrometry.

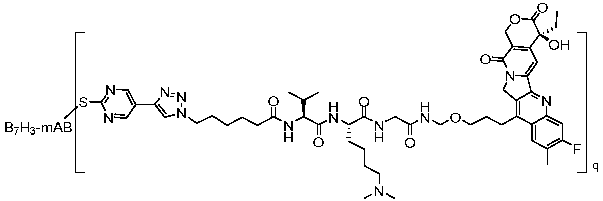

### Example 4.1.24: Preparation of B7H3-ADC-24

Compound DL-037 in the preparation method B of Example 4.1.7 was replaced by compound DL-032 to obtain the coupling product B7H3-ADC-24 of DL-032 and anti-B7H3 antibody. The DAR value was measured to be 8.0 by mass spectrometry.

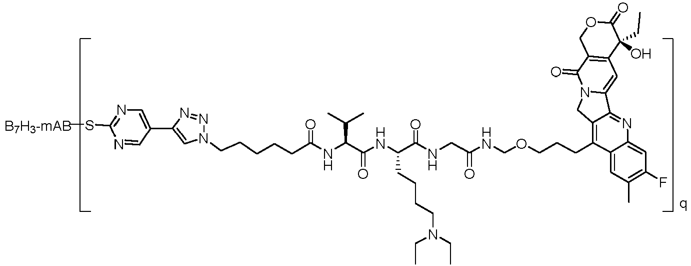

### Example 4.1.25: Preparation of B7H3-ADC-25

Compound DL-037 in the preparation method B of Example 4.1.7 was replaced by compound DL-033 to obtain the coupling product B7H3-ADC-25 of DL-033 and anti-B7H3 antibody. The DAR value was measured to be 8.0 by mass spectrometry.

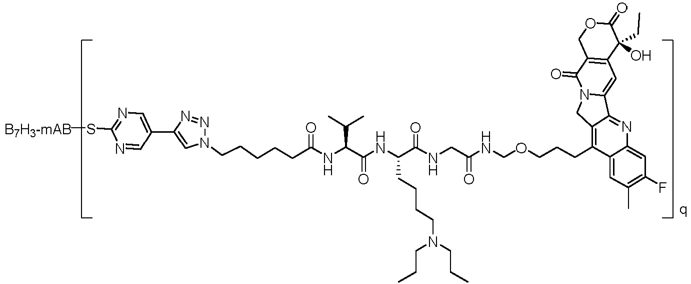

### Example 4.1.27: Preparation of B7H3-ADC-27

Compound DL-037 in the preparation method B of Example 4.1.7 was replaced by compound DL-050 to obtain the coupling product B7H3-ADC-27 of DL-050 and anti-B7H3 antibody. The DAR value was measured to be 8.0 by mass spectrometry.

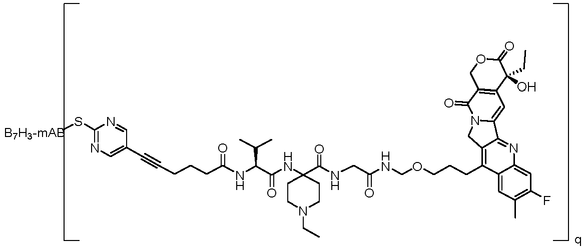

### Example 4.1.28: Preparation of B7H3-ADC-28

Compound DL-037 in the preparation method B of Example 4.1.7 was replaced by compound DL-021 to obtain the coupling product B7H3-ADC-28 of DL-021 and anti-B7H3 antibody. The DAR value was measured to be 8.0 by mass spectrometry.

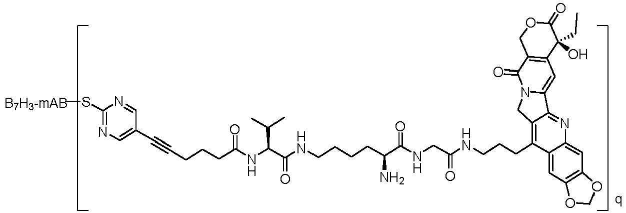

### Example 4.1.29: Preparation of B7H3-ADC-32

With reference to Example 16 of WO2020219287, the following drug-linker compound was prepared,

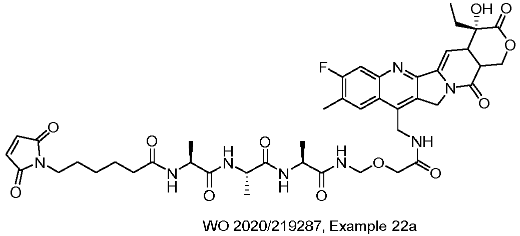

Compound DL-037 in the preparation method B of Example 4.1.7 was replaced by the above drug-linker compound to obtain B7H3-ADC-32. The DAR value was measured to be 7.3 by mass spectrometry.

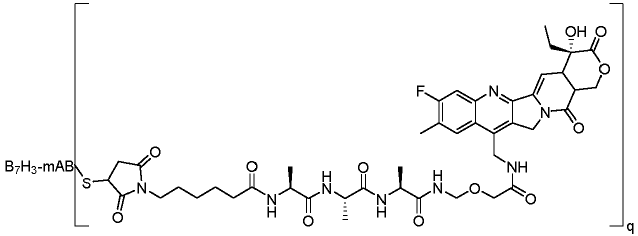

### Example 4.1.30: Preparation of B7H3-ADC-33

With reference to Example 4-1 of WO2019195665, the following drug-linker compound was prepared,

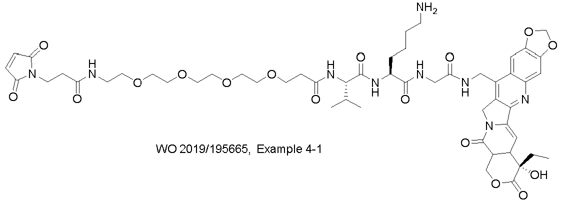

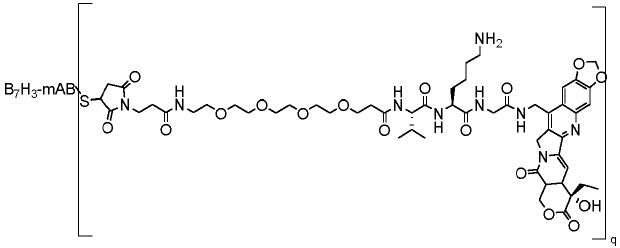

### 4.1.30.1 Preparation of sample B7H3-ADC-33(DAR8)

Compound DL-037 in the preparation method B of Example 4.1.7 was replaced by the above drug-linker compound to obtain B7H3-ADC-33(DAR8). The DAR value was measured to be 7.4 by mass spectrometry.

### 4.1.30.2 Preparation of sample B7H3-ADC-33(DAR4)

Compound DL-037 in the preparation of B7H3-ADC-07(DAR4) of Example 4.1.7.3 was replaced by the above drug-linker compound to obtain B7H3-ADC-33(DAR4). The DAR value was measured to be 4.9 by mass spectrometry.

Note: B7H3-mAB was antibody 2E3-02, B7H3-AB is B7H3-C1.

### 4.2: Preparation of Trop2-ADC

### Example 4.2.1 Preparation of Trop2-ADC-01(DAR8)

0.3 ml of antibody Sacituzumab (anti-Trop-2, 33.5 mg/mL) was taken and diluted with 0.25 mL of 20 mM PB + 150 mM NaCl + 20 mM edetate sodium solution (pH 7.6), and then 0.45 ml of 20 mM PB + 150 mM NaCl solution (pH 7.6) was added and mixed homogeneously. The pH of the sample was adjusted to 7.4 with 1M K₂HPO₄ solution, and then 10 mM TCEP (tris(2-carboxyethyl)phosphine, 0.033 ml, 0.33 µmol) solution was added and mixed homogeneously. The mixture was allowed to stand at room temperature for 30 min. To the above solution, was added a solution of DL001 (1.35 mg, 20-fold molar amount relative to the antibody) in dimethyl sulfoxide (0.1mL), and then the mixture was mixed homogeneously and allowed to stand for 2 h at room temperature. After completion of the reaction, the reaction was stopped by adding 6.1µl of 100 mM cysteine. Finally, the buffer was replaced with 20 mM PB buffer at pH 6.44 using a G-25 gel column. The coupling product Trop2-ADC-01 (DAR8) of DL-001 and Sacituzumab antibody was obtained. The DAR value was measured to be 7.8 by mass spectrometry.

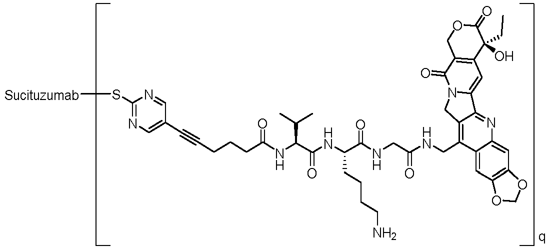

### Example 4.2.2: Preparation of Trop2-ADC-02(DAR8)

Compound DL-001 in Example 4.2.1 was replaced by compound DL-009 to obtain the coupling product Trop2-ADC-02(DAR8) of DL-009 and Sacituzumab. The DAR value was measured to be 7.9 by mass spectrometry.

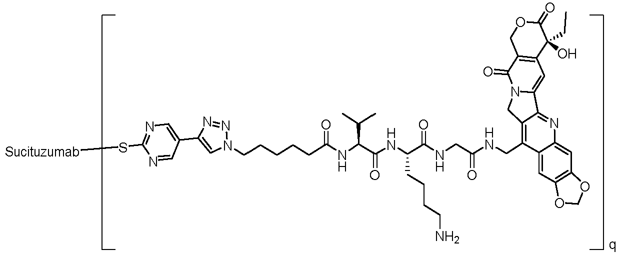

### Example 4.2.3: Preparation of Trop2-ADC-03(DAR8)

Compound DL-001 in Example 4.2.1 was replaced by compound DL-011 to obtain the coupling product Trop2-ADC-03(DAR8) of DL-011 and Sacituzumab. The DAR value was measured to be 7.8 by mass spectrometry.

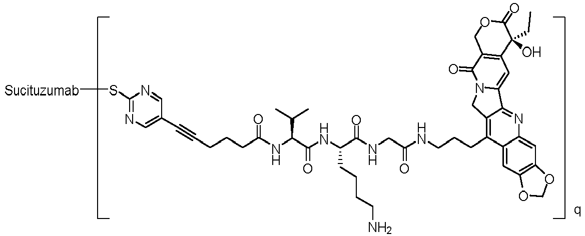

### Example 4.2.4: Preparation of Trop2-ADC-04(DAR8)

Compound DL-001 in Example 4.2.1 was replaced by compound DL-015 to obtain the coupling product Trop2-ADC-04(DAR8) of DL-015 and Sacituzumab. The DAR value was measured to be 7.9 by mass spectrometry.

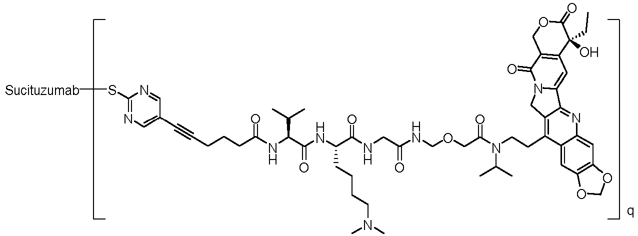

### Example 4.2.5: Preparation of Trop2-ADC-05(DAR8)

Compound DL-001 in Example 4.2.1 was replaced by compound DL-018 to obtain the coupling product Trop2-ADC-05(DAR8) of DL-018 and Sacituzumab. The DAR value was measured to be 7.8 by mass spectrometry.

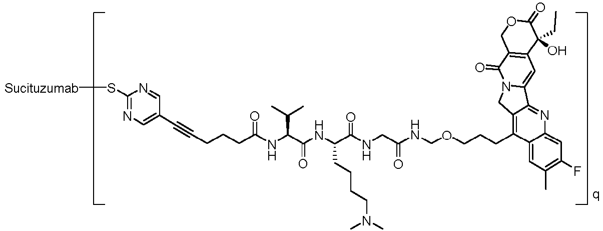

### Example 4.2.6: Preparation of Trop2-ADC-06(DAR8)

Compound DL-001 in Example 4.2.1 was replaced by compound DL-036 to obtain the coupling product Trop2-ADC-06(DAR8) of DL-036 and Sacituzumab. The DAR value was measured to be 7.7 by mass spectrometry.

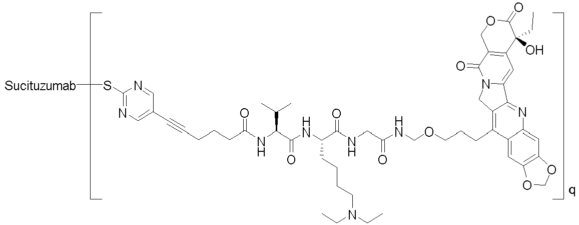

### Example 4.2.7: Preparation of Trop2-ADC-07(DAR8)

Compound DL-001 in Example 4.2.1 was replaced by compound DL-037 to obtain the coupling product Trop2-ADC-07(DAR8) of DL-037 and antibody Sacituzumab. The DAR value was measured to be 7.7 by mass spectrometry.

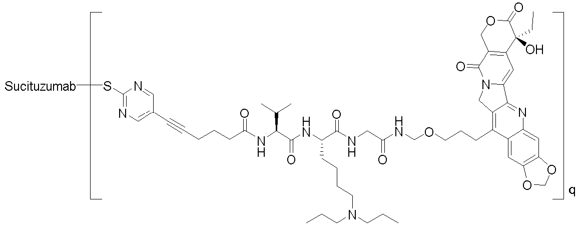

### Example 4.2.8: Preparation of Trop2-ADC-08(DAR8)

Compound DL-001 in Example 4.2.1 was replaced by compound DL-028 to obtain the coupling product Trop2-ADC-08(DAR8) of DL-028 and antibody Sacituzumab. The DAR value was measured to be 7.3 by mass spectrometry.

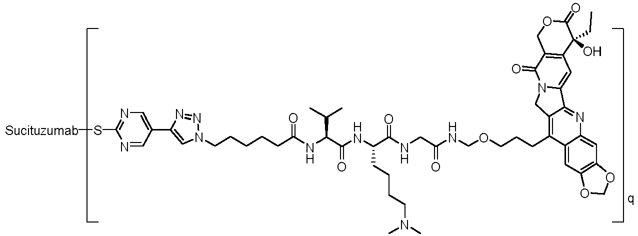

### Example 4.2.9: Preparation of Trop2-ADC-09(DAR8)

Compound DL-001 in Example 4.2.1 was replaced by compound DL-029 to obtain the coupling product Trop2-ADC-09(DAR8) of DL-029 and antibody Sacituzumab. The DAR value was measured to be 7.6 by mass spectrometry.

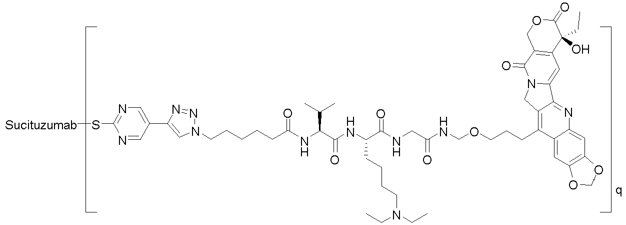

### Example 4.2.10: Preparation of Trop2-ADC-10(DAR8)

Compound DL-001 in Example 4.2.1 was replaced by compound DL-030 to obtain the coupling product Trop2-ADC-10(DAR8) of DL-030 and antibody Sacituzumab. The DAR value was measured to be 7.6 by mass spectrometry.

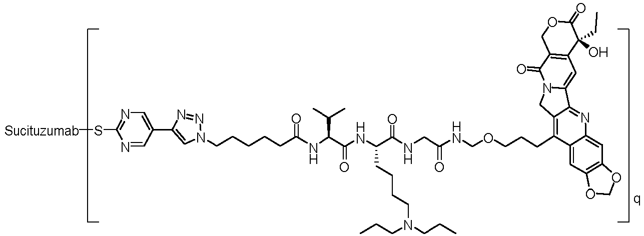

### 4.3: Preparation of Her2-ADC

### Example 4.3.1: Preparation of Her2-ADC-01(DAR8)

At 37 °C, TCEP (10 mM, tris(2-carboxyethyl)phosphine, 0.033 ml, 0.33 µmol) was added to Trastuzumab in PBS buffer (pH = 6.5, 1 ml, 10.05 mg/ml, 0.0677 µmol), and the solution was mixed homogeneously, then allowed to stand at 37 °C for 3 h. A solution of DL001 (1.35 mg, 20-fold molar amount of the antibody) in dimethyl sulfoxide (0.1 mL) was added to the above solution, and the solution was mixed homogeneously and allowed to stand at room temperature for 3 h. After that, 6.1 µl of 100 mM cysteine was added to stop the reaction. Finally, the buffer was replaced by 20 mM PB buffer at pH 6.44 using a G-25 gel column. The coupling product Her2-ADC-01(DAR8) of DL-001 and Trastuzumab antibody was obtained. The DAR value was measured to be 7.9 by mass spectrometry.

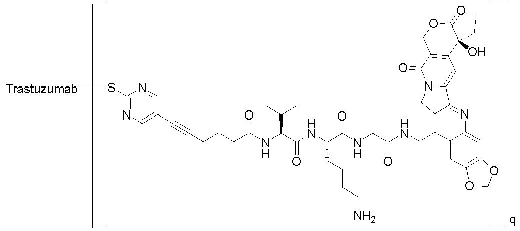

### Example 4.3.2: Preparation of Her2-ADC-02(DAR8)

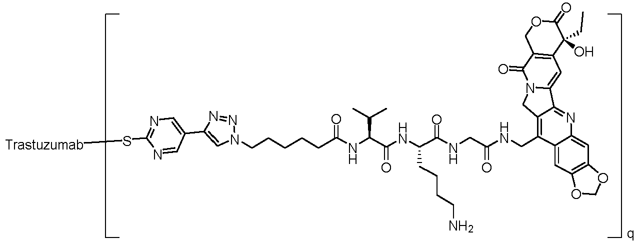

Compound DL-001 in Example 4.3.1 was replaced by compound DL-009 to obtain the coupling product Her2-ADC-02(DAR8) of DL-009 and antibody Trastuzumab. The DAR value was measured to be 7.9 by mass spectrometry.

### Example 4.3.3: Preparation of Her2-ADC-03(DAR8)

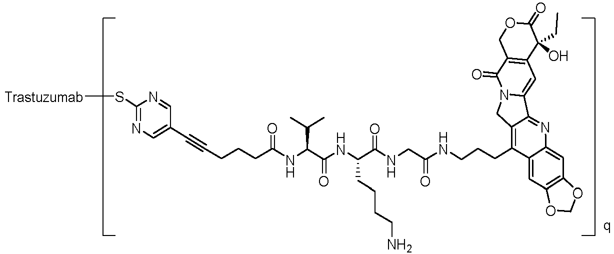

Compound DL-001 in Example 4.3.1 was replaced by compound DL-011 to obtain the coupling product Her2-ADC-03(DAR8) of DL-011 and antibody Trastuzumab. The DAR value was measured to be 7.8 by mass spectrometry.

### Example 4.3.4: Preparation of Her2-ADC-04(DAR8)

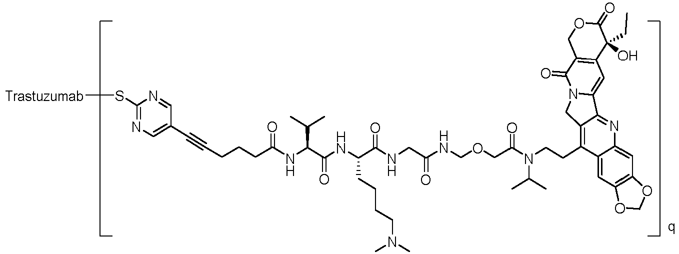

Compound DL-001 in Example 4.3.1 was replaced by compound DL-015 to obtain the coupling product Her2-ADC-04(DAR8) of DL-015 and antibody Trastuzumab. The DAR value was measured to be 7.9 by mass spectrometry.

### Example 4.3.5: Preparation of Her2-ADC-05(DAR8)

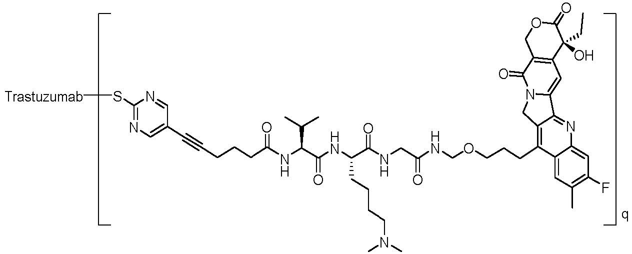

Compound DL-001 in Example 4.3.1 was replaced by compound DL-018 to obtain the coupling product Her2-ADC-05(DAR8) of DL-018 and antibody Trastuzumab. The DAR value was measured to be 7.9 by mass spectrometry.

### Example 4.3.6: Preparation of Her2-ADC-06(DAR8)

Compound DL-001 in Example 4.3.1 was replaced by compound DL-036 to obtain the coupling product Her2-ADC-06(DAR8) of DL-036 and antibody Trastuzumab. The DAR value was measured to be 7.7 by mass spectrometry.

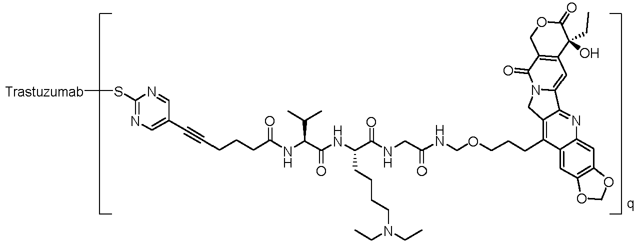

### Example 4.3.7: Preparation of Her2-ADC-07(DAR8)

Compound DL-001 in Example 4.3.1 was replaced by compound DL-037 to obtain the coupling product Her2-ADC-07(DAR8) of DL-037 and antibody Trastuzumab. The DAR value was measured to be 7.7 by mass spectrometry.

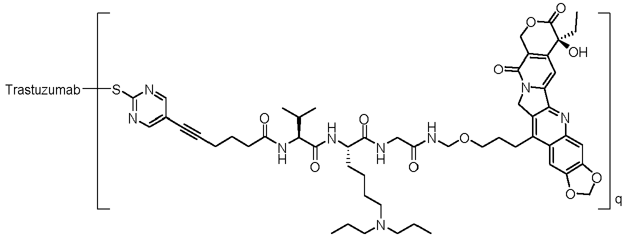

### Example 4.3.8: Preparation of Her2-ADC-08(DAR8)

Compound DL-001 in Example 4.3.1 was replaced by compound DL-028 to obtain the coupling product Her2-ADC-08(DAR8) of DL-028 and antibody Trastuzumab. The DAR value was measured to be 7.3 by mass spectrometry.

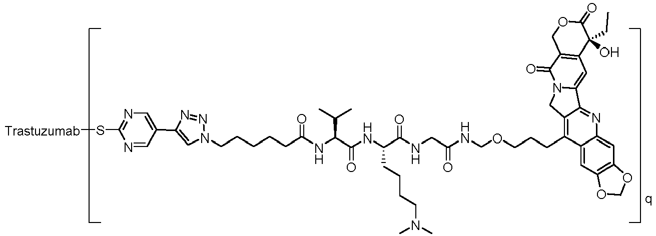

### Example 4.3.9: Preparation of Her2-ADC-09(DAR8)

Compound DL-001 in Example 4.3.1 was replaced by compound DL-029 to obtain the coupling product Her2-ADC-09(DAR8) of DL-029 and antibody Trastuzumab. The DAR value was measured to be 7.6 by mass spectrometry.

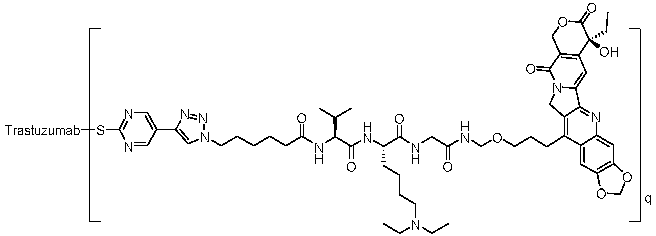

### Example 4.3.10: Preparation of Her2-ADC-10(DAR8)

Compound DL-001 in Example 4.3.1 was replaced by compound DL-030 to obtain the coupling product Her2-ADC-10(DAR8) of DL-030 and antibody Trastuzumab. The DAR value was measured to be 7.6 by mass spectrometry.

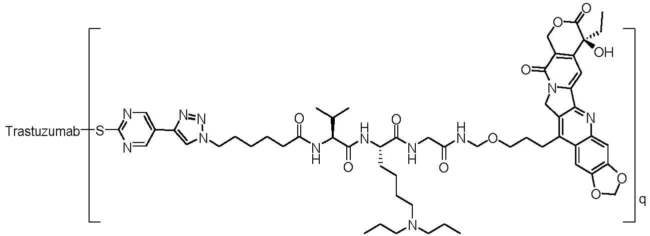

### 4.4: Preparation of Her3-ADC

### Example 4.4.1: Preparation of Her3-ADC-07

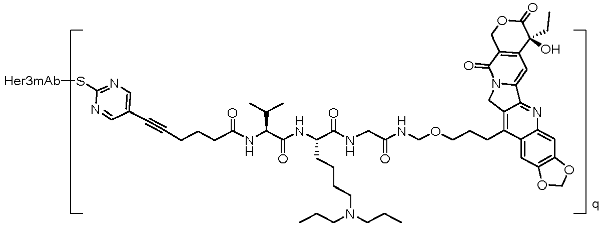

### 4.4.1.1 Preparation of Her3-ADC-07(DAR8)

30 mg of anti-Her3 antibody 202-2-1 was diluted with diluent (20 mM PB + 105 mM NaCl, pH 7.7), to which was added sodium edetate solution at a final concentration of 5 mM, and then the solution was mixed homogeneously; TCEP solution was added at 4.5-fold equivalent of the antibody, and then the solution was mixed homogeneously and allowed to stand at room temperature for 30 min; to the above solution, was added 10-fold equivalent (relative to the antibody) of DL-037 dissolved in dimethyl sulfoxide, and then the solution was mixed homogeneously, and allowed to stand at room temperature for 2 h to obtain a conjugated sample. After completion of the reaction, a 30KDa ultrafiltration tube was used to transfer the sample into a 10 mM histidine buffer with a pH of 5.5 and remove low molecular weight substances, and finally the sample was concentrated to obtain a solution containing antibody 202-2-1 ADC composition Her3-ADC-07 (DAR8). The DAR value was measured to be 7.98 by mass spectrometry.

### 4.4.1.2 Preparation of sample Her3-ADC-07(DAR4)

30 mg of anti-Her3 antibody 202-2-1 was diluted with diluent (20 mM PB + 105 mM NaCl, pH 7.7), to which was added sodium edetate solution at a final concentration of 5 mM, and then the solution was mixed homogeneously; TCEP solution was added at 4.5-fold equivalent of the antibody, and then the solution was mixed homogeneously and allowed to stand at room temperature for 30 min; to the above solution, was added 4.8-fold equivalent (relative to the antibody) of DL-037 dissolved in dimethyl sulfoxide, and then the solution was mixed homogeneously, and allowed to stand at room temperature for 2h to obtain a conjugated sample. After completion of the reaction, a 30KDa ultrafiltration tube was used to transfer the sample into a 10 mM histidine buffer with a pH of 5.5 and remove low molecular weight substances, and finally the sample was concentrated to obtain a solution containing antibody 202-2-1 ADC composition Her3-ADC-07 (DAR4). The DAR value was measured to be 4.5 by mass spectrometry.

### 4.4.1.3 Preparation of Her3-ADC-07(DAR6)

30 mg of anti-Her3 antibody 202-2-1 was diluted with diluent (20 mM PB + 105 mM NaCl, pH 7.7), to which was added sodium edetate solution at a final concentration of 5 mM, and then the solution was mixed homogeneously; TCEP solution was added at 4.5-fold equivalent of the antibody, and then the solution was mixed homogeneously and allowed to stand at room temperature for 30 min; to the above solution, was added 7.2-fold equivalent (relative to the antibody) of DL-037 dissolved in dimethyl sulfoxide, and then the solution was mixed homogeneously, and allowed to stand at room temperature for 2h to obtain a conjugated sample. After completion of the reaction, a 30KDa ultrafiltration tube was used to transfer the sample into a 10 mM histidine buffer with a pH of 5.5 and remove low molecular weight substances, and finally the sample was concentrated to obtain a solution containing antibody 202-2-1 ADC composition Her3-ADC-07 (DAR6). The DAR value was measured to be 5.8 by mass spectrometry.

### Example 4.4.2: Preparation of Her3-ADC-05

### 4.4.2.1 Preparation of sample Her3-ADC-05(DAR4)

Compound DL-037 in Example 4.4.1.2 was replaced by compound DL-018 to obtain the coupling product Her2-ADC-05(DAR4) of DL-018 and antibody 202-2-1. The DAR value was measured to be 4.0 by mass spectrometry.

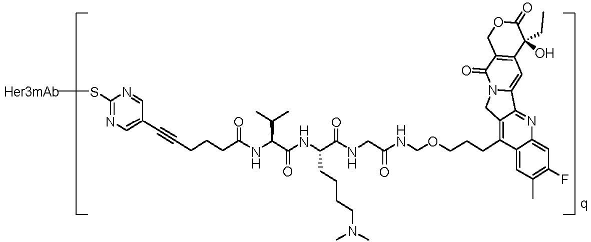

### 4.4.2.2 Preparation of sample Her3-C3-ADC-05(DAR4) (DAR4)

Compound DL-037 in Example 4.4.1.2 was replaced by compound DL-018, and anti-Her3 antibody 202-2-1 in Example 4.4.1.2 was replaced by antibody Her3-C3, to obtain the coupling product Her3-C3-ADC-05(DAR4) of DL-018 and antibody Her3-C3. The DAR value was measured to be 4.7 by mass spectrometry.

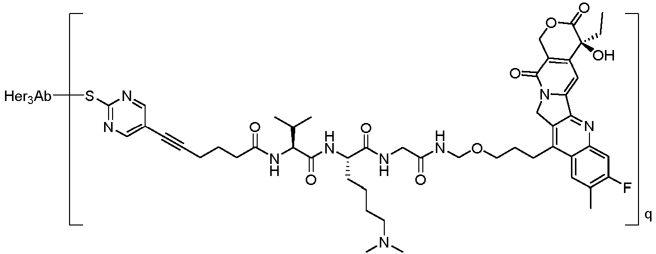

### Example 4.4.3: Preparation of Her3-ADC-15

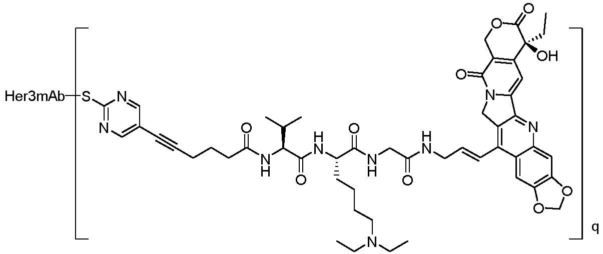

### 4.4.3.1 Preparation of sample Her3-ADC-15(DAR8)

Compound DL-037 in Example 4.4.1.1 was replaced by compound DL-027 to obtain the coupling product Her2-ADC-15(DAR8) of DL-027 and antibody 202-2-1. The DAR value was measured to be 7.9 by mass spectrometry.

### 4.4.3.2 Preparation of sample Her3-ADC-15(DAR2)

Compound DL-037 in Example 4.1.7.2 was replaced by compound DL-027, and antibody 2E3-02 in Example 4.1.7.2 was replaced by anti-Her3 antibody 202-2-1, to obtain the coupling product Her3-ADC-15(DAR2) of DL-027 and antibody 202-2-1. The DAR value was measured to be 2 by mass spectrometry.

### Example 4.4.4: Preparation of Her3-ADC-21

With reference to CN104755494B, the following drug-linker compound was prepared,

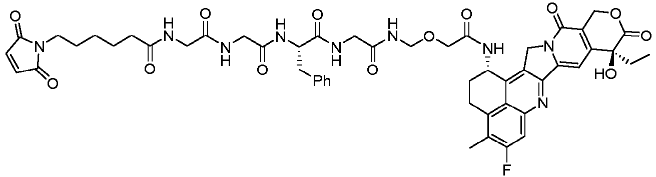

### 4.4.4.1 Preparation of sample Her3-C3-ADC-21(DAR4)

Compound DL-037 in Example 4.4.1.2 was replaced by the above drug-linker compound, anti-Her3 antibody 202-2-1 in Example 4.4.1.2 was replaced by antibody Her3-C3, to obtain Her3-C3-ADC-21(DAR4). The DAR value was measured to be 4.4 by mass spectrometry.

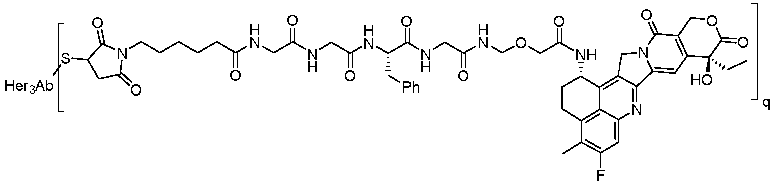

### 4.4.4.2 Preparation of sample Her3-ADC-21(DAR4)

Compound DL-037 in Example 4.4.1.2 was replaced by the above drug-linker compound, to obtain the coupling product Her3-ADC-21 (DAR4) of the above drug-linker compound and antibody 202-2-1. The DAR value was measured to be 4.1 by mass spectrometry.

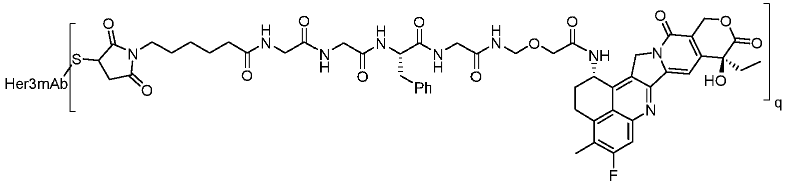

### 4.4.4.3 Preparation of sample Her3-C3-ADC-21(DAR8)

Compound DL-037 in Example 4.4.1.1 was replaced by the above drug-linker compound, anti-Her3 antibody 202-2-1 in Example 4.4.1.1 was replaced by antibody Her3-C3, to obtain Her3-C3-ADC-21(DAR8). The DAR value was measured to be 9.5 by mass spectrometry.

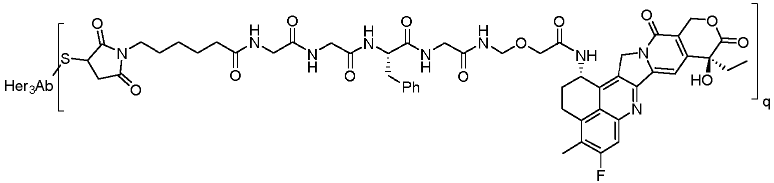

### Example 4.4.5: Preparation of Her3-ADC-23

### 4.4.5.1 Preparation of sample Her3-ADC-23(DAR4)

Compound DL-037 in Example 4.4.1.2 was replaced by DL-031 to obtain the coupling product Her2-ADC-23(DAR4) of DL-031 and antibody 202-2-1. The DAR value was measured to be 4.2 by mass spectrometry.

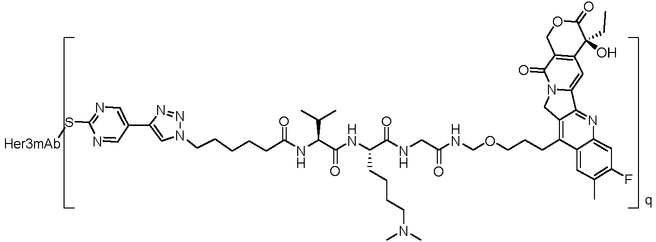

### 4.4.5.2 Preparation of sample Her3-C3-ADC-23(DAR4)

Compound DL-037 in Example 4.4.1.2 was replaced by DL-031, anti-Her3 antibody 202-2-1 in Example 4.4.1.2 was replaced by antibody Her3-C3, to obtain Her3-C3-ADC-23(DAR4) of DL-031 and antibody Her3-C3. The DAR value was measured to be 4.9 by mass spectrometry.

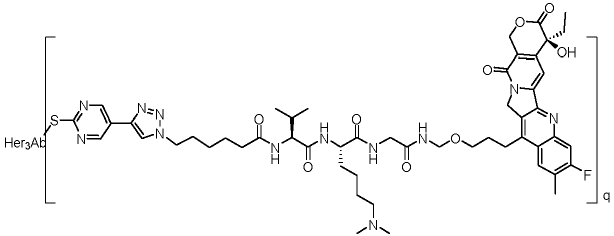

### Example 4.4.6: Preparation of Her3-ADC-33

With reference to Example 4-1 in WO2019195665, the following drug-linker compound was prepared,

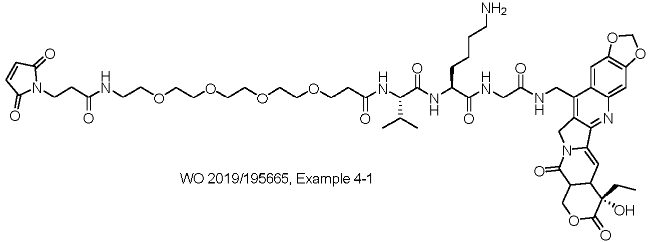

Compound DL-037 in Example 4.4.1.2 was replaced by the above drug-linker compound, to obtain the coupling product Her3-ADC-33(DAR4) of the above drug-linker compound and antibody 202-2-1. The DAR value was measured to be 5.6 by mass spectrometry.

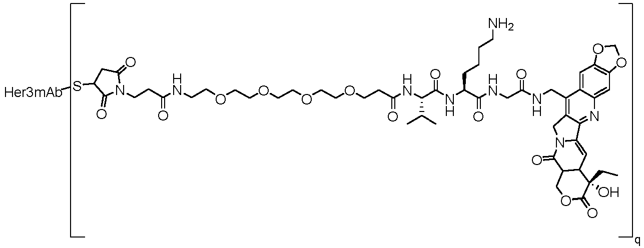

Note: Her3mAb was antibody 202-2-1, and Her3Ab was antibody Her3-C3_{∘}

### 4.5: Preparation of EGFR-ADC

### 4.5.1 Preparation of EGFR-ADC-07

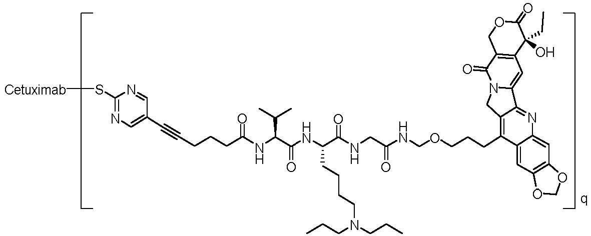

### 4.5.1.1 Preparation of sample EGFR-ADC-07(DAR8)

Antibody 2E3-02 in preparation method B of Example 4.1.7 was replaced by antibody Cetuximab, to obtain the coupling product EGFR-ADC-07(DAR8) of DL-037 and antibody Cetuximab. The DAR value was measured to be 7.8 by mass spectrometry.

### 4.5.1.2 Preparation of sample EGFR-ADC-07(DAR4)

Antibody 2E3-02 in preparation method of B7H3-ADC-07(DAR4) of Example 4.1.7.3 was replaced by antibody Cetuximab, to obtain the coupling product EGFR-ADC-07(DAR4) of DL-037 and antibody Cetuximab. The DAR value was measured to be 5.0 by mass spectrometry.

### 4.5.2 Preparation of EGFR-ADC-08

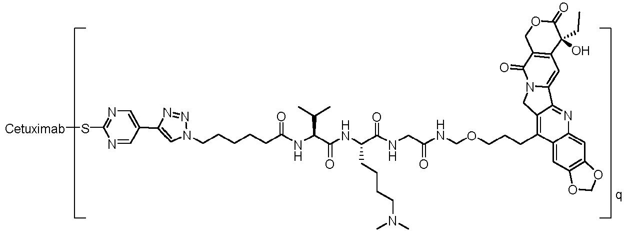

### 4.5.2.1 Preparation of sample EGFR-ADC-08(DAR8)

Antibody 2E3-02 in preparation method B of Example 4.1.7 was replaced by antibody Cetuximab, and compound DL-037 in preparation method B of Example 4.1.7 was replaced by DL-028, to obtain the coupling product EGFR-ADC-08(DAR8) of DL-028 and antibody Cetuximab. The DAR value was measured to be 7.3 by mass spectrometry.

### 4.5.2.2 Preparation of sample EGFR-ADC-08(DAR4)

Antibody 2E3-02 in preparation method of B7H3-ADC-07(DAR4) of Example 4.1.7.3 was replaced by antibody Cetuximab, and compound DL-037 in preparation method B of Example 4.1.7 was replaced by DL-028, to obtain the coupling product EGFR-ADC-08(DAR4) of DL-028 and antibody Cetuximab. The DAR value was measured to be 4.3 by mass spectrometry.

### 4.5.3 Preparation of sample EGFR-ADC-32(DAR4)

With reference to Example 16 in WO2020219287, the following drug-linker compound was prepared,

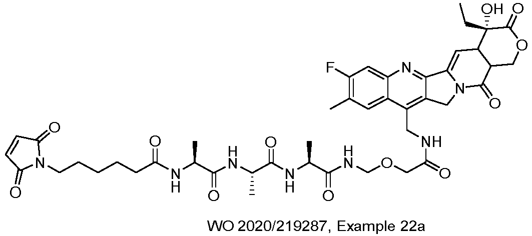

Antibody 2E3-02 in preparation method of B7H3-ADC-07(DAR4) of Example 4.1.7.3 was replaced by antibody Cetuximab, and compound DL-037 in preparation method B of Example 4.1.7 was replaced by the above drug-linker compound, to obtain the coupling product EGFR-ADC-32(DAR4) of the above drug-linker compound and antibody Cetuximab. The DAR value was measured to be 4.9 by mass spectrometry.

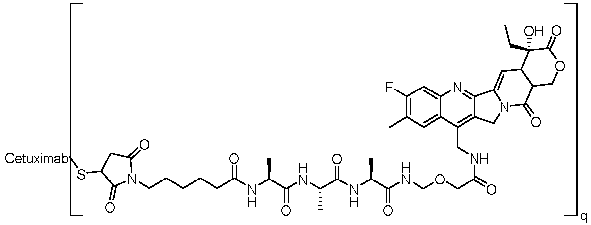

### 4.6: Preparation of IgG-ADC

### Example 4.6.1: Preparation of IgG1-ADC-07

Antibody 2E3-02 in preparation method B of Example 4.1.7 was replaced by IgG1 isotype antibody (anti-chicken lysozyme antibody, prepared by MediLink Therapeutics (Suzhou) Co., Ltd. ), to obtain the coupling product IgG1-ADC-07 of DL-037 and antibody IgG1. The DAR value was measured to be 8.0 by mass spectrometry.

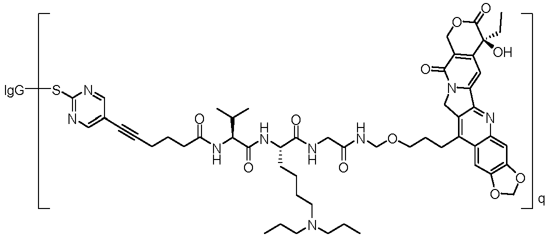

### Example 4.6.2: Preparation of IgG1-ADC-04

Antibody 2E3-02 in preparation method B of Example 4.1.7 was replaced by IgG1 isotype antibody (anti-chicken lysozyme antibody, prepared by MediLink Therapeutics (Suzhou) Co., Ltd. ), and compound DL-037 in preparation method B of Example 4.1.7 was replaced by compound DL-019, to obtain the coupling product IgG1-ADC-04 of DL-019 and antibody IgG1. The DAR value was measured to be 7.9 by mass spectrometry.

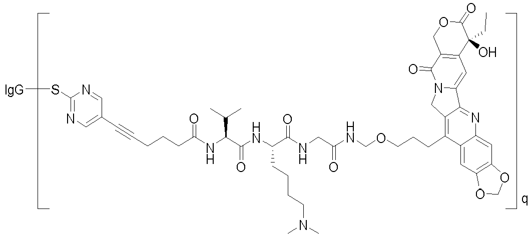

### V. ADC Characterization

### Example 5.1. SEC-HPLC determination

Conjugation reactions were monitored by SEC-HPLC, and SEC detection of antibody conjugates was performed.
Instrument model: Agilent 1200
Chromatographic conditions:
   Column: TSKgel G3000SWXL, 7.8×300 mm, 5µm
   Mobile phase: 60 mmol/L Na₂HPO₄, 40 mmol/L NaH₂PO₄, 200 mmol/L NaCl.
   Column temperature: room temperature; sample chamber temperature: 8°C; flow rate: 0.8 ml/min
   Injection amount: 200µg, Detection wavelength: 280 nm.
   Sample treatment: the sample B7H3-ADC-07 (DAR8) was diluted to 10 mg/ml with ultrapure water, and the injection volume was 20 µl.

The SEC chromatograms before and after antibody conjugation are shown in Figure 8. Before and after antibody conjugation, the molecular weight of the main peak was about 150 kD, indicating that the conjugated product still maintained the complete structure of the antibody.

### Example 5.2. Determination of DAR values of conjugated samples by mass spectrometry (I)

### Chromatographic conditions:

Column: PLRP-S, 2.1*50 mm, 5 µm;
Mobile phase A: 0.1% FA /H2O; mobile phase B: 0.1% FA/ACN
Column temperature: 30°C; sample chamber temperature: 8°C; flow rate: 0.6 ml/min; injection volume: 2 µl

| Time (min) | 1 | 5 | 5.1 | 7 | 10 |
|---|---|---|---|---|---|
| Mobile phase A | 90 | 40 | 10 | 90 | 90 |
| Mobile phase B | 10 | 60 | 90 | 10 | 10 |

Sample treatment: to 50 µg of sample B7H3-ADC-07, was added 1M DTT (2 µl), to which was added ultrapure water to 50 µl, and the solution was diluted to a concentration of about 1.0 mg/ml, then mixed homogeneously, followed by reducing at room temperature for 30 min.
LC/MS model: Agilent 1290-6545XT Q-TOF
MS conditions: Gas temp: 320°C, Drying Gas: Nebulizer: 35 psi; Sheath Gas Temp: 350°C; sheath Gas flow: 11 l/min: m/z 500~3000.

The results were shown in the following:

| Peptide chain | | mAb | DAR1 | DAR2 | DAR3 |
|---|---|---|---|---|---|
| LC | Theoretical value | 23245 | 24262.4 | 25279.8 | 26297.2 |
| | Experimental value | 23247.41 | 24265.49 | Not detected | Not detected |
| HC | Theoretical value | 50367 | 51384.4 | 52401.8 | 53419.2 |
| | Experimental value | Not detected | Not detected | Not detected | 53426.64 |

In the table, mAb represents an unconjugated antibody; LC represents a light chain of an antibody; HC represents a heavy chain of an antibody; DAR1 represents a conjugate containing a light or heavy chain conjugated with one toxin molecule; DAR2 represents a conjugate containing a light or heavy chain conjugated with 2 toxin molecules; DAR3 represents a conjugate containing a light or heavy chain conjugated with 3 toxin molecule; where the theoretical molecular weight of the monoclonal antibody is calculated as G0F glycoform. Hereinafter, mAb, LC, HC, DAR1, DAR2, and DAR3 are defined as above.

The light chain of 2E3-02 antibody was measured to be conjugated with 0~1 toxin molecule (LC and DAR1 percentage were 1.0% and 99.0%, respectively), while the heavy chain was measured to be conjugated with 0~3 toxin molecules (mAb, DAR1, DAR2, DAR3 percentages were 0%, 0%, 0%, 100%, respectively), and thus the antibody-drug coupling ratio (DAR value) of B7H3-ADC-07(DAR8) was calculated to be 7.98.

### Example 5.3 Determination of DAR value of conjugated samples by mass spectrometry (II)

Using the same experimental conditions of 5.2, the molecular weights of samples B7H3-ADC-07(DAR2), B7H3-ADC-07(DAR4) and B7H3-ADC-07(DAR6) were determined, and the results are as follows:

| Sample name | Peptide chain | | mAb | DAR1 | DAR2 | DAR3 |
|---|---|---|---|---|---|---|
| B7H3-ADC-07(DAR2) | LC | Theoretical value | 23245 | 24262.4 | 25279.8 | 26297.2 |
| | | Experimental value | 23247 | Not detected | Not detected | Not detected |
| | HC | Theoretical value | 50367 | 51384.4 | 52401.8 | 53419.2 |
| | | Experimental value | 50372 | 51389 | 52408 | 53426 |
| B7H3-ADC-07(DAR4) | LC | Theoretical value | 23245 | 24262.4 | 25279.8 | 26297.2 |
| | | Experimental value | 23247 | 24265 | Not detected | Not detected |
| | HC | Theoretical value | 50367 | 51384.4 | 52401.8 | 53419.2 |
| | | Experimental value | 50372 | 51389 | 52408 | 53426 |
| B7H3-ADC-07(DAR6) | LC | Theoretical value | 23245 | 24262.4 | 25279.8 | 26297.2 |
| | | Experimental value | 23247 | 24265 | Not detected | Not detected |
| | HC | Theoretical value | 50367 | 51384.4 | 52401.8 | 53419.2 |
| | | Experimental value | 50372 | Not detected | 52408 | 53426 |

Moreover, the results of DAR values were calculated as follows: the DAR value of B7H3-ADC-07(DAR2) was 2.3; the DAR value of B7H3-ADC-07(DAR4) was 4.1; and the DAR value of B7H3-ADC-07(DAR6) was 5.4.

### Example 5.4 Determination of the molecular weight and drug-antibody ratio (DAR) of Trop2-ADC-01 by LC-MS

The molecular weight analysis of Trop2-ADC-01 was performed by LCMS.
Chromatographic determination conditions:
LC column: ACQUITU UPLC^{®} Protein BEH C4 1.7 µm, 2.1 mm x 100 mm;
Mobile phase A: 0.1% FA/98% H₂O/2% ACN; mobile phase B: 0.1% FA/2% H₂O/98% ACN;
Flow rate: 0.25 mL/min; sample chamber temperature: 8 °C; column temperature: 60 °C; injection amount: 1 µg;

| Time (min) | 1 | 7 | 8 | 9 | 13 |
|---|---|---|---|---|---|
| Mobile phase A (volume%) | 90 | 20 | 20 | 90 | 90 |
| Mobile phase B (volume%) | 10 | 80 | 80 | 10 | 10 |

MS determination conditions:
MS model: Triple TOF 5600+;
GS1 60; GS2 60; CUR30; TEM600; ISVF5000; DP300; CE10 m/z 600-5000.
Theoretical molecular weight and experimental molecular weight of Trop2-ADC-01

| Glycoform | | mAb | DAR1 | DAR2 | DAR3 | DAR4 |
|---|---|---|---|---|---|---|
| LC | Theoretical value | 23334.0 | 24209.7 | 25085.4 | | |
| | Experimental value | Not detected | 24209.9 | Not detected | Not detected | Not detected |
| HC-G0F | Theoretical value | 50734.0 | 51609.7 | 52485.4 | 53361.1 | 56024.7 |
| | Experimental value | Not detected | Not detected | 52485.3 | 53361.2 | Not detected |

In the table, mAb represents a monoclonal antibody; LC represents a light chain of an antibody; HC represents a heavy chain of an antibody; DAR1 represents a conjugate containing one light/heavy chain of an antibody and one cell bioactive molecule; DAR2 represents a conjugate containing one light/heavy chain of an antibody and two cell bioactive molecules; DAR3 represents a conjugate containing one light/heavy chain of an antibody and three cell bioactive molecules; DAR4 represents a conjugate containing one light/heavy chain of an antibody and four cell bioactive molecules; glycoforms indicate sugar chain structures on two heavy chains: G0F indicates fucosylation and no galactose.

The molecular weight of light and heavy chains changed after an antibody was conjugated with compound DL-001, in which the light chain conjugated with one cell bioactive molecule, and the heavy chain conjugated with 2.9 cell bioactive molecules. Thus, the coupling ratio (DAR) of the full antibody and cell bioactive molecules was 7.8.

### Example 5.5 Determination of Her3-ADC molecular weight and drug-antibody ratio (DAR) by LC-MS

Detection: the molecular weight and DAR value of Her3-ADC-07 (DAR8) were analyzed by LC-MS under the following conditions:

### Chromatographic conditions:

Column: PLRP-S, 2.1*50mm,5µm;
Mobile phase A: 0.1%FA /H2O; Mobile phase B: 0.1%FA/ACN column temperature: 30 °C; sample chamber temperature: 8 °C; flow rate: 0.6 mL/min; injection volume: 2 µl

| Time (min) | 1 | 5 | 5.1 | 7 | 10 |
|---|---|---|---|---|---|
| Mobile phase A | 90 | 40 | 10 | 90 | 90 |
| Mobile phase B | 10 | 60 | 90 | 10 | 10 |

Sample treatment: to 50 µg of sample, was added 1M DTT (2 µl), to which was added ultrapure water to 50 µl, and the solution was diluted to a concentration of about 1.0 mg/ml, then mixed homogeneously, followed by reducing at room temperature for 30 min.
LC/MS model: Agilent 1290-6545XT Q-TOF
MS conditions: Gas temp: 320°C, Drying Gas: Nebulizer: 35 psi; Sheath Gas Temp: 350°C; sheath Gas flow: 11 l/min: m/z 500~3000.

The results were shown in the following:

| Peptide chain | | mAb | DAR1 | DAR2 | DAR3 |
|---|---|---|---|---|---|
| LC | Theoretical value | 23243.8 | 24261.2 | 25278.6 | 26296 |
| | Experimental value | Not detected | 24258 | Not detected | Not detected |
| HC | Theoretical value | 50598.6 | 51616 | 52633.4 | 53650.8 |
| | Experimental value | Not detected | Not detected | Not detected | 53646 |

In above table, mAb represents an unconjugated monoclonal antibody; LC represents a light chain of an antibody; HC represents a heavy chain of an antibody; DAR1 represents a conjugate containing a light or heavy chain conjugated with one toxin molecule; DAR2 represents a conjugate containing a light heavy or chain conjugated with 2 toxin molecules; DAR3 represents a conjugate containing a light or heavy chain conjugated with 3 toxin molecules; where the theoretical molecular weight of the monoclonal antibody is calculated as G0F glycoform. Hereinafter, mAb, LC, HC, DAR1, DAR2, and DAR3 are defined as above.

According to the test results, the light chain of antibody in Her3-ADC-07(DAR8) was conjugated with 0~1 toxin molecule (LC and DAR1 percentage were 0% and 100%, respectively), while the heavy chain was conjugated with 0~3 toxin molecules (mAb, DAR1, DAR2, DAR3 percentage were 0%, 0%, 0%, 100%, respectively), and thus the drug-to-antibody coupling ratio (DAR value) of Her3-ADC-07(DAR8) was calculated to be 8.0.

It can be understood that the molecular weight and drug-antibody ratio (DAR) of the ADCs of the present disclosure can all be determined by the same or similar methods.

### VI. Assays of biological activity of bioactive molecules and antibody-drug conjugates

### Example 6.1: Assay I of inhibitory activity of bioactive molecules on cell viability in vitro

Tumor cells were first cultured; the bioactive molecules of the present disclosure were co-cultured with tumor cells, and then CCK8 reagent (Dojindo Laboratories (Shanghai), Cat: CK04, Lot: JJ744) was added, and the activity of dehydrogenase in mitochondria was detected by reading (detection wavelength 450 nm) on a microplate reader (manufacturer: Molecular Devices, model: SpectraMax M2), to evaluate the inhibitory effect of bioactive molecules on cell proliferation. The source of tumor cells is shown in Table 5.

**Table 5.**

| Cell name | Tumor type | source |
|---|---|---|
| NCl-H358 | human non-small cell lung cancer | ATCC, CRL-5807 |
| HT29 | human colon cancer cells | ATCC, HTB-38 |
| LS174T | human colon adenocarcinoma cells | ATCC, CL-187 |
| NCl-H322M | human non-small cell lung cancer | ATCC |
| PANC-1 | Human pancreatic cancer tumor cells | ATCC, CRL-1469 |
| HCC1806 | breast cancer | Nanjing Cobioer Biosciences Co., Ltd. |

*In vitro* cell viability assay method: bioactive molecules (12 concentration gradients) were diluted with corresponding assay medium (containing 2% FBS). The tumor cells were digested by conventional methods using trypsin, and cells in tube were collected and counted, then resuspended in the corresponding assay media (containing 2% FBS). The diluted bioactive molecules were added to a 96-well plate, followed by the cells. Then, 20µL of CCK8 reagent was added to each well, and the reaction was carried out for 4 h, then the plate was read on a microplate reader (detection wavelength was 450 nm). The experimental results are shown in Tables 6.1, 6.2, 6.3, 6.4, and 6.5.

DXD was prepared with reference to CN104755494B, and the specific structure was as follows,

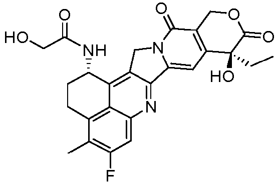

**Table 6.1. Killing results of bioactive molecules on NCl-H358 cells.**

| Name | Cell name | EC₅₀(nM) |
|---|---|---|
| A1.10 | NCl-H358 | 8.11 |
| DXD | | 39.02 |
| | | |

| Name | Cell name | EC₅₀(nM) |
|---|---|---|
| A1.8D | NCl-H358 | 7.919 |
| A1.15a | | 3.882 |
| DXD | | 21.43 |
| | | |

| Name | Cell name | EC₅₀(nM) |
|---|---|---|
| A1.4 | NCl-H358 | 29.91 |
| A1.8 | | 296.4 |
| A1.9 | | 60.79 |
| A1.11 | | 202.9 |
| A1.12 | | 167.1 |
| A1.13 | | 49.19 |
| A1.14 | | 122.6 |
| DXD | | 148.45±10.87 (n=4) |

**Talbe 6.2. Killing results of bioactive molecules on HT29 cells.**

| Name | Cell name | EC₅₀(nM) |
|---|---|---|
| A1.10 | HT29 | 5.965 |
| DXD | | 49.47 |
| | | |

| Name | Cell name | EC₅₀(nM) |
|---|---|---|
| A1.15a | HT29 | 3.574 |
| A1.15b | | 47.52 |
| DXD | | 64.84 |
| | | |

| Name | Cell name | EC₅₀(nM) |
|---|---|---|
| A1.4 | HT29 | 29.45 |
| A1.8 | | 367.1 |
| A1.9 | | 57.5 |
| A1.11 | | 284.5 |
| A1.12 | | 211.8 |
| A1.13 | | 96.85 |
| A1.14 | | 155.9 |
| DXD | | 301.875±69.00 (n=4) |

**Table 6.3. Killing results of bioactive molecules on LS174T cells.**

| Name | Cell name | EC₅₀(nM) |
|---|---|---|
| A1.15a | | 19.49 |
| A1.15b | LS174T | 44.62 |
| DXD | | 82.05 |

**Table 6.4. Killing results of bioactive molecules on NCl-H322M cells.**

| Name | Cell name | EC₅₀(nM) |
|---|---|---|
| A1.9 | NCl-H322M | 30.45 |
| DXD | | 76.89 |

**Table 6.5. Killing results of bioactive molecules on PANC-1 cells.**

| Name | Cell name | EC₅₀(nM) |
|---|---|---|
| A1.9 | PANC-1 | 94.54 |
| DXD | | 966 |

**Table 6.6. Killing results of bioactive molecules on HCC1806 cells.**

| Name | Cell name | EC₅₀(nM) |
|---|---|---|
| A1.9 | HCC1806 (7500 cells/well, 3 days) | 1.9 |
| Dxd | | 37 |

Test results showed that the bioactive molecules of the present disclosure had a killing effect on tumor cells.

### Example 6.2: Assay II of inhibitory activity of bioactive molecules on cell viability in vitro

Tumor cells NUCG-4, PC-9, HT29, NCI-H358, KYSE520, A431 and A549 were first cultured; the bioactive molecules or fragments of the present disclosure were co-cultured with tumor cells, and then CCK8 reagent (Dojindo Laboratories (Shanghai), Cat: CK04, Lot: JJ744) was added, and the activity of dehydrogenase in mitochondria was detected by reading (detection wavelength 450 nm) on a microplate reader (manufacturer: Molecular Devices, model: SpectraMax M2), to evaluate the inhibitory effect of bioactive molecules on cell proliferation. The source of tumor cells is shown in Table 7.

**Table 7. Tumor cell source.**

| Cell name | Tumor type | Source |
|---|---|---|
| NUCG-4 | Gastric cancer | Nanjing Cobioer |
| PC-9 | Lung adenocarcinoma | ATCC |
| HT29 | Colon cancer | ATCC, HTB-38 |
| NCI-H358 | Non-small cell lung cancer | ATCC, CRL-5807 |
| KYSE520 | Esophageal cancer | ATCC |
| A431 | Human Skin Squamous Carcinoma (Human Epidermal Carcinoma) Cells | ATCC |
| A549 | Non-small cell lung cancer | ATCC |

*In vitro* cell viability assay method: the tumor cells were digested by conventional methods using trypsin, and cells in tube were collected and counted, and then resuspended in the corresponding assay media (containing 2% FBS), which was added to a 96-well plate at 5000-10000 cells/well. Bioactive molecules were diluted with DMSO, and then 100µL was added to a 96-well plate, the initial concentration was 10µM, followed by 3-fold serial dilution (12 concentration gradients). The plate was incubated for 3-4 days at 37 °C under 5% CO₂. Then, 20µL of CCK8 reagent was added to each well, and the reaction was carried out for 2-6 h. The plate was read on a microplate reader (detection wavelength was 450 nm). The bioactive molecules and their detection results are shown in Table 8.

**Table 8. Activity assays of A1.9 and B1.14 .**

| Cells | A1.9 EC₅₀(nM) | B1.14 EC₅₀(nM) | DXD EC₅₀ (nM) |
|---|---|---|---|
| NUCG-4 | 8.73 | 184.8 | 43.32 |
| PC-9 | 2.06 | 34.32 | 16.69 |
| HT29 | 20.32 | 367.9 | 210.5 |
| NCI-H358 | 46.2 | 581.2 | 261.6 |
| A431 | 5.71 | 67.97 | 25.08 |
| A549 | 65.86 | 252.3 | 262.2 |

The experimental results showed that the bioactive molecules A1.9 and B1.14 had killing effects on tumor cells, in which A1.9 had a stronger tumor killing effect than Dxd against several tumor cells. In the *in vitro* inhibitory assay against KYSE520, A1.9 had an EC50 value of 49.62nM, thereby had killing effects on KYSE520.

### Example 6.3: Assay III of inhibitory activity of bioactive molecules on cell viability in vitro

Tumor cells HT29 (having a same source as the above) were first cultured. The bioactive molecules or fragments of the present disclosure were co-cultured with tumor cells, and then CCK8 reagent (Dojindo Laboratories (Shanghai), Cat: CK04, Lot: JJ744) was added, and the activity of dehydrogenase in mitochondria was detected by reading (detection wavelength 450 nm) on a microplate reader (manufacturer: Molecular Devices, model: SpectraMax M2), to evaluate the inhibitory effect of bioactive molecules on cell proliferation.

*In vitro* cell viability assay method: the tumor cells were digested by conventional methods using trypsin, and cells in tube were collected and counted, and then resuspended in the corresponding assay media (containing 2% FBS), which was added to a 96-well plate at 5000-10000 cells/well. Bioactive molecules were diluted with DMSO, and then added to a 96-well plate at 100µL, the initial concentration was 10µM, followed by 3-fold serial dilution (12 concentration gradients). The plate was incubated for 3-4 days at 37 °C under 5% CO₂. Then, 20µL of CCK8 reagent was added to each well, and the reaction was carried out for 2-6 h. The plate was read on a microplate reader (detection wavelength was 450 nm). The bioactive molecules and their detection results are shown in Table 9.

**Table 9. Killing results of bioactive molecules on HT29 cells.**

| Name | Cell name | EC₅₀(nM) |
|---|---|---|
| B1.9 | | 262.2 |
| B1.12 | HT29 | 85.87 |
| B1.17 | | 204.8 |
| DXD | | 22.89 |

The experimental results showed that the bioactive molecules B1.9, B1.12 and B1.17 have killing effects on tumor cells.

### Example 6.4: Assay of inhibitory activity of ADC on cell viability in vitro.

HT29 and NCI-H358 tumor cells were digested by conventional methods using trypsin, and cells in tube were collected and counted, and then resuspended in the corresponding assay media (containing 2% FBS), which was added to a 96-well plate at 2000-5000 cells/well. 100µL ADC, diluted with the media containing 2% FBS, was added to the 96-well plate, the initial concentration was 150µg/ml, followed by 3-fold serial dilution (12 concentration gradients). The plate was cultured for 7 days at 37 °C under 5% CO₂. Then, 20µL of CCK8 reagent was added to each well, and the reaction was carried out for 2-6 h. The plate was read on a microplate reader (detection wavelength was 450 nm). ADCs and their detection results are shown in Tables 10 and 11.

**Table 10. Activity assay of some ADCs.**

| ADC | HT29 (EC₅₀, ng/mL) | NCI-H358 (EC₅₀, ng/mL) |
|---|---|---|
| B7H3-ADC-07(DAR8) | 23555 | 21131 |
| B7H3-ADC-05(DAR8) | 33135 | 33490 |
| B7H3-ADC-04(DAR8) | 28793 | 28493 |
| B7H3-ADC-22(DAR8) | 24418 | 20825 |
| B7H3-ADC-12(DAR8) | 5032 | 4068 |
| B7H3-ADC-27(DAR8) | 28510 | 29155 |
| B7H3-ADC-08(DAR8) | 31669 | 34149 |
| B7H3-ADC-23(DAR8) | 21074 | 19578 |
| B7H3-ADC-20(DAR8) | 1348 | 1325 |
| B7H3-ADC-09(DAR8) | 22479 | 20751 |
| 2E3-02 antibody | weak | weak |
| B7H3-ADC-32(DAR8) | 32472 | 30903 |

The experimental results showed that the ADC molecules of the present disclosure had killing effects on tumor cells, in which most of the ADCs had stronger killing effects on tumor cells than B7H3-ADC-32(DAR8).

**Table 11. Activity assay of some ADCs.**

| ADC | HT29 (EC₅₀, ng/mL) | NCI-H358 (EC₅₀, ng/mL) |
|---|---|---|
| B7H3-ADC-33(DAR8) | 18380 | 20354 |
| B7H3-ADC-15(DAR8) | 1181 | 1015 |

The experimental results showed that the ADC molecules of the present disclosure had killing effects on tumor cells, in which B7H3-ADC-15(DAR8) had stronger killing effects on tumor cells than B7H3-ADC-33(DAR8).

Meanwhile, the ADC molecule B7H3-ADC-07(DAR8) of the present disclosure was assayed against B7H3 negative cells Calu-3, and the results indicated that the killing effect was weak (IC50>10⁷ng/ml), suggesting that for B7H3 positive and negative tumor cells, the ADC molecules of the present disclosure had excellent selective targeting killability *in vitro.*

### Example 6.5: Assay of the in vitro inhibitory activities of antibody-drug conjugates having multiple targets on various tumor cells

### A. Inhibitory activity assays of B7H3 ADC against various tumors

ADC sample: ADC sample was prepared according to the examples.

*In vitro* cell viability assay method: various tumor cells were digested by conventional methods using trypsin, and cells in tube were collected and counted, and then resuspended in the corresponding assay media (containing 2% FBS), which was added to a 96-well plate at 2000-5000 cells/well. 100µL ADC diluted with the media containing 2% FBS (see table 12) was added to the 96-well plate, the initial concentration was 150µg/ml, followed by 3-fold serial dilution (11 concentration gradients). The plate was incubated for 7 days at 37 °C under 5% CO₂. Then, 20µL of CCK8 reagent was added to each well, and the reaction was carried out for 2-6 h. The plate was read on a microplate reader (detection wavelength was 450 nm). The test cells and the detection results are shown in Table 12.

**Table 12**

| Cells | B7H3-ADC-07 (DAR8) | B7H3-ADC-21 (DAR8) |
|---|---|---|
| PC-9 (EC50, ng/mL) | 3618 | 2377 |
| HT29 (EC50, ng/mL) | 10433 | 7442 |
| KYSE520 (EC50, ng/mL) | 5845 | 2842 |
| NCI-H358 (EC50, ng/mL) | 15800 | 8060 |
| NUCG-4 (EC50, ng/mL) | 4855 | 4158 |
| A431 (EC50, ng/mL) | 7624 | 2478 |

The experimental results indicated that ADCs in Table 12 had killing effects on various tumor cells.

### B. Assay of the inhibitory activities of Her3 ADC on various tumor cells

ADC sample: ADC sample Her3-ADC-07(DAR8) was prepared according to the example.

*In vitro* cell viability assay method: PC-9 and NCI-H358 tumor cells were digested by conventional methods using trypsin, and cells in tube were collected and counted, and then resuspended in the corresponding assay media (containing 2% FBS), which was added to a 96-well plate at 2000-5000 cells/well. 100µL ADC, diluted with the media containing 2% FBS, was added to the 96-well plate, the initial concentration was 150µg/ml, followed by 3-fold serial dilution (11 concentration gradients). The plate was incubated for 7 days at 37 °C under 5% CO₂. Then, 20µL of CCK8 reagent was added to each well, and the reaction was carried out for 2-6 h. The plate was read on a microplate reader (detection wavelength was 450 nm). The test cells and the test results are shown in Table 13.

**Table 13.**

| Cells | Her3-ADC-07(DAR8) |
|---|---|
| PC-9 (EC₅₀, nM/L) | 49.26 |
| NCI-H358 (EC₅₀, nM/L) | 183.3 |

The experimental results indicated that Her3-ADC-07(DAR8) had killing effects on tumor cells.

### C. Assay of the inhibitory activities of EGFR ADC on tumors

KYSE520 tumor cells were digested by conventional methods using trypsin, and cells in tube were collected and counted, and then resuspended in the corresponding assay media (containing 2% FBS), which was added to a 96-well plate at 2000-5000 cells/well. 100µL ADC diluted with the media containing 2% FBS (as shown in table 14) was added to the 96-well plate, the initial concentration was 50µg/ml, followed by 3-fold serial dilution (11 concentration gradients). The plate was incubated for 5 days at 37 °C under 5% CO₂. Then, 20µL of CCK8 reagent was added to each well, and the reaction was carried out for 1-4 h. The plate was read on a microplate reader (detection wavelength was 450 nm). The test cells and the experimental results are shown in Table 14.

**Table 14.**

| Test group No. | ADC sample | KYSE520 (EC₅₀, ng/mL) |
|---|---|---|
| 1 | EGFR-ADC-07 (DAR8) | 11.56 |
| | EGFR-ADC-07 (DAR4) | 18.17 |
| | EGFR-ADC-32 (DAR4) | 29.21 |
| 2 | Her3-ADC-05 (DAR4) | 19.01 |
| | Her3-C3-ADC-05 (DAR4) | 32.94 |

The experimental results indicated that EGFR-ADC-07, Her3-ADC-05 and Her3-C3-ADC-05 all had strong killing effects on tumor cells.

Based on the above experimental results, the ADCs with various targets (e.g., B7H3, Her3, EGFR) disclosed in the present application all had a killing effect on various tumor cells.

### D. Inhibitory effect of Trop-2 antibody-drug conjugates on cell viability in vitro

Tumor cells HCC1806 (Trop-2 positive cell lines) and NCI-H23 (Trop-2 negative cell lines) were first cultured; the bioactive molecules and ADC molecules of the present disclosure were co-cultured with tumor cells, and then CCK8 reagent (Dojindo Laboratories (Shanghai), Cat: CK04, Lot: JJ744) was added, and the activity of dehydrogenase in mitochondria was detected by reading (detection wavelength 450 nm) on a microplate reader (manufacturer: Molecular Devices, model: SpectraMax M2), to evaluate the inhibitory effect of ADCs on cell proliferation. The source of tumor cells is shown in Table 15.

**Table 15.**

| Cell name | Tumor type | Source |
|---|---|---|
| HCC1806 | Breast cancer | Nanjing Cobioer Biosciences Co., Ltd. |
| NCI-H23 | non-small cell lung cancer | Concortis |

*In vitro* cell viability assay: bioactive molecules or ADCs were diluted with the corresponding detection media (containing 2% FBS) (12 concentration gradients). Tumor cells were digested by conventional methods using trypsin, and cells in tube were collected and counted, and then resuspended in the corresponding assay media (containing 2% FBS). The diluted bioactive molecules or ADCs were added to a 96-well plate, followed by addition of cells. Then, 20µL of CCK8 reagent was added to each well, and the reaction was carried out for 4h. The plate was read on a microplate reader (detection wavelength was 450 nm). The experimental conditions and the test results are shown in Table 16.

**Table 16. Killing results of the conjugates on Trop-2 positive and negative cell lines.**

| Name | Cell name | EC₅₀(nM) |
|---|---|---|
| Trop2-ADC-01(DAR8) | HCC1806 (7500 cells/well, 3 days) | 2.1 |
| | NCI-H23 (10000 cells/well, 3 days) | 10000 |
| Trop2-ADC-05(DAR8) | HCC1806 (7500 cells/well, 3 days) | 3.4 |
| | NCI-H23 (10000 cells/well, 3 days) | 10000 |

Experimental results indicated that the ADC molecules of the present disclosure had killing effects on tumor cells. Meanwhile, good targeting was displayed between Trop-2 positive and negative cells.

### Example 6.6: Inhibitory effects of antibody drug conjugates with different DAR values on cell activity in vitro

ADC sample: ADC samples were prepared according to the examples.

*In vitro* cell viability assay: NCI-H358 tumor cells were digested by conventional methods using trypsin, and cells in tube were collected and counted, and then resuspended in the corresponding assay media (containing 2% FBS), which was added to a 96-well plate at 2000-5000 cells/well. 100µL ADC, diluted with the media containing 2% FBS, was added to the 96-well plate, the initial concentration was 150µg/ml, followed by 3-fold serial dilution (11 concentration gradients). The plate was incubated for 7 days at 37 °C under 5% CO₂. Then, 20µL of CCK8 reagent was added to each well, and the reaction was carried out for 2-6 h. The plate was read on a microplate reader (detection wavelength was 450 nm). Each group of samples and the experimental results are shown in Table 17.

**Table 17.**

| Test group No. | ADC sample | NCI-H358 (EC₅₀, ng/mL) |
|---|---|---|
| 1 | B7H3-ADC-07(DAR4) | 30428 |
| | B7H3-C1-ADC-07(DAR4) | 22174 |
| | B7H3-C1-ADC-21(DAR4) | 31057 |
| 2 | Her3-ADC-05(DAR4) | 47742 |
| | Her3-C3-ADC-05(DAR4) | 65109 |
| | Her3-C3-ADC-21(DAR4) | 76365 |
| 3 | B7H3-ADC-07(DAR6) | 27858 |
| | B7H3-ADC-21(DAR6) | 20629 |
| 4 | B7H3-ADC-08(DAR4) | 83363 |
| | B7H3-C1-ADC-08(DAR4) | 101649 |
| 5 | Her3-ADC-23(DAR4) | 49330 |
| | Her3-C3-ADC-23(DAR4) | weak |
| 6 | Her3-ADC-15(DAR2) | 5572 |
| | Her3-ADC-15(DAR8) | 2562 |
| | Her3-ADC-33(DAR4) | 50774 |
| 7 | B7H3-ADC-15(DAR4) | 2931 |
| | B7H3-ADC-33(DAR4) | 42071 |
| | 2E3-02 antibody | weak |

The experimental results of this example and Example 6.5 C indicated that the ADCs with various DAR values (e.g., 4, 6, and 8) disclosed in the present application all had killing effects on tumor cells.

### Example 6.7: In vivo activity assay of anti-B7H3 antibody drug conjugates

### 6.7.1 Efficacy Assay I of B7H3 antibody-drug conjugates on NCI-HT29 xenografts

### 1. Experimental materials

Test compounds: B7H3-ADC-21 (DAR8), B7H3-ADC-07 (DAR8), B7H3-ADC-08 (DAR8), B7H3-ADC-05 (DAR8), B7H3-ADC-06 (DAR8), B7H3-ADC-17 (DAR8), B7H3-ADC-18 (DAR8), B7H3-ADC-19 (DAR8), IgG1-ADC-04 (DAR8).

Test cells: NCI-HT29 cells, purchased from ATCC;
Experimental animals: Balb/c nu nude mice, female, 5-6 weeks age, purchased from Charles River Laboratory Animal Co., Ltd.

### 2. Experimental method

### 2.1. Cell treatment

NCI-HT29 cells were cultured in 1640 media (containing 10% FBS) in a petri dish (15 cm diameter), and digested by trypsin-EDTA when achieving about 80-90% confluence, washed twice with PBS, and then centrifuged. The cells were resuspended in pre-cooled PBS and counted on a cell counter. The cells were diluted with PBS to a cell concentration of 5×10⁷/ml.

### 2.2. tumor cell transplantation

After adapting to the laboratory environment for 2-5 days, Balb/c nu mice were subcutaneously inoculated with NCI-HT29 cells in the right rib, with an inoculation amount of 5×10⁶ cells/mouse and an inoculation volume of 0.2 ml (containing 50% Matrigel). The experiment was carried out when the tumor grew to about 250 mm³.

### 2.3. Administration of animals and detection

The tumor-bearing nude mice were administrated according to the following regimens:

**Table 18**

| No. | Groups | Animal numbers | Dosage | Administration route and cycle |
|---|---|---|---|---|
| 1 | B7H3-ADC-21(DAR8) | 5 | 3 mg/kg | i.v., QW×3 |
| 2 | B7H3-ADC-07(DAR8) | 5 | 3 mg/kg | i.v., QW×3 |
| 3 | B7H3-ADC-08(DAR8) | 5 | 3 mg/kg | i.v., QW×3 |
| 4 | B7H3-ADC-05(DAR8) | 5 | 3 mg/kg | i.v., QW×3 |
| 5 | B7H3-ADC-06(DAR8) | 5 | 3 mg/kg | i.v., QW×3 |
| 6 | B7H3-ADC-17(DAR8) | 5 | 3 mg/kg | i.v., QW×3 |
| 7 | B7H3-ADC-18(DAR8) | 5 | 3 mg/kg | i.v., QW×3 |
| 8 | B7H3-ADC-19(DAR8) | 5 | 3 mg/kg | i.v., QW×3 |
| 9 | IgG1-ADC-04(DAR8) (control group) | 5 | 3 mg/kg | i.v., QW×3 |

After the end of the administration cycle, mice were continually observed for 1-2 weeks. After the last measurement of tumor volume, the tumor was isolated, and the tumor weight was accurately weighed and photographed for recording.

### 2.4. Determination of tumor volume and body weight:

Tumor volume and body weight were measured twice a week, and TGI% and tumor inhibition rate (TGI)(%) were calculated according to the formula: relative tumor proliferation rate T/C(%) = TRTV/CRTV × 100% (TRTV: average RTV in treatment group; CRTV: average RTV in control group; RTV = Vt/V0, V0 is the tumor volume of the animal at the time of grouping, Vt is the tumor volume of the animal after treatment); the relative tumor inhibition rate TGI% = (1-T/C)× 100% (T and C are the relative tumor volume (RTV) at a specific time point in the treatment groups and control groups, respectively).

The tumor volume (V) was calculated according to the formula: V = 1/2×L_{long}×Lₛₕₒᵣₜ², wherein L_{long} and Lₛₕₒᵣₜ represent the long diameter and short diameter of the tumor, respectively.

### 3. Experimental results

The specific results are shown in Table 19 and Figure 9. The ADCs of the present application all showed an inhibitory effect on tumor growth. In particular, B7H3-ADC-05, B7H3-ADC-06, B7H3-ADC-07, B7H3-ADC-08 and B7H3-ADC-17 have a very strong inhibitory effect on tumors. B7H3-ADC-18, B7H3-ADC-19 and B7H3-ADC-21 have similar inhibitory effects on tumors. There was no significant weight loss and drug toxicity in each group during the administration period.

**Table 19. NCI-HT29 xenograft model data.**

| | Tumor volume mm³ | | | | | | T/C (%) | TGI (%) |
|---|---|---|---|---|---|---|---|---|
| Sample | Day 1 of administra tion | Day 4 of administra tion | Day 8 of administr ation | Day 11 of administra tion | Day 15 of administra tion | Day 18 of administra tion | | |
| IgG1-ADC-04 (DAR8) | 244.4 | 381.0 | 543.6 | 1061.2 | 1285.6 | 1821.8 | - | - |
| B7H3-ADC-21 (DAR8) | 244.4 | 335.4 | 414.4 | 781.4 | 862.2 | 1233.4 | 62.70 | 37.30 |
| B7H3-ADC-08 (DAR8) | 244.4 | 268.6 | 243 | 418.4 | 456.6 | 555.4 | 19.72 | 80.28 |
| B7H3-ADC-05 (DAR8) | 244.4 | 286.2 | 221.8 | 479.6 | 574 | 745.2 | 31.75 | 68.25 |
| B7H3-ADC-06 (DAR8) | 245.8 | 293.6 | 302.8 | 482.6 | 592.2 | 669 | 26.83 | 73.17 |
| B7H3-ADC-07 (DAR8) | 243.6 | 276 | 253.2 | 414.8 | 461.8 | 576.4 | 21.10 | 78.90 |
| B7H3-ADC-17 (DAR8) | 245.4 | 250.2 | 304.2 | 559.2 | 741 | 970 | 45.94 | 54.06 |
| B7H3-ADC-18 (DAR8) | 245.2 | 302.2 | 413.4 | 741.4 | 958.8 | 1218.6 | 61.71 | 38.29 |
| B7H3-ADC-19 (DAR8) | 245.2 | 318.6 | 441.4 | 784.6 | 1003.6 | 1244.6 | 63.36 | 36.64 |

### 6.7.2 Efficacy assay II of B7H3 antibody-drug conjugates on NCI-HT29 xenografts

The efficacy of B7H3-ADC-21(DAR8), B7H3-ADC-08(DAR8) and B7H3-ADC-07(DAR8) on xenografts was determined using the same method as that of 5.3.1. The specific grouping and administration schedule are shown in Table 20.

**Table 20. Administration schedule.**

| Group No. | Group | Animal number | Dosage | Administration route | Administration frequency | Dosing volume |
|---|---|---|---|---|---|---|
| 1 | Normal saline | 5 | - | i.v. | QW×3 w | 10 mL/kg |
| 2 | B7H3-ADC-21(DAR8) | 5 | 10 mg/kg | i.v. | QW×3 w | 10 mL/kg |
| 3 | B7H3-ADC-21(DAR8) | 5 | 3 mg/kg | i.v. | QW×3 w | 10 mL/kg |
| 4 | B7H3-ADC-08(DAR8) | 5 | 3 mg/kg | i.v. | QW×3 w | 10 mL/kg |
| 5 | B7H3-ADC-07(DAR8) | 5 | 10 mg/kg | i.v. | QW×3 w | 10 mL/kg |
| 6 | B7H3-ADC-07(DAR8) | 5 | 3 mg/kg | i.v. | QW×3 w | 10 mL/kg |

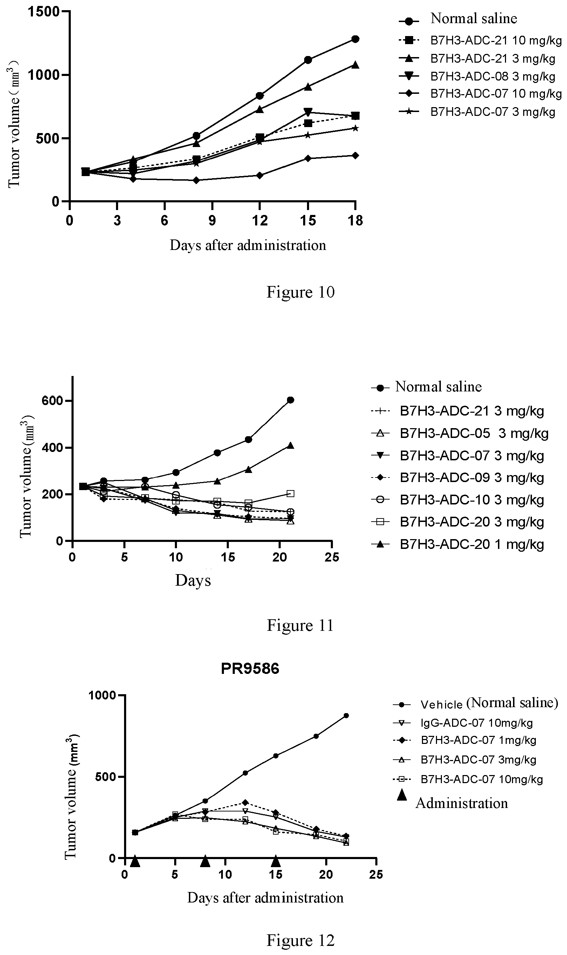

The results obtained are shown in Table 21 and Figure 10.

**Table 21. NCI-HT29 xenografts model data.**

| Group | Mean tumor volume (mm³) D1 | Mean tumor volume (mm³) D18 | T/C (%) D18 | Tumor inhibition rate TGI (%) D18 |
|---|---|---|---|---|
| Normal saline | 232.2±35.1 | 1281.8±170.0 | - | - |
| B7H3-ADC-21(DAR8) 10 mg/kg | 231.8±49.6 | 679.2±141.7*** | 53.1 | 46.92 |
| B7H3-ADC-21(DAR8) 3 mg/kg | 232.2±41.4 | 1081.6±209.7 | 84.4 | 15.62 |
| B7H3-ADC-08(DAR8) 3 mg/kg | 232.6±37.5 | 675.8±190.0*** | 52.6 | 47.37 |
| B7H3-ADC-07 (DAR8) 10 mg/kg | 231.6±45.9 | 363.8±162.4*** | 28.5 | 71.54 |
| B7H3-ADC-07 (DAR8) 3 mg/kg | 231.8±65.0 | 579.2±281.8*** | 45.3 | 54.74 |

| | | | | |
|---|---|---|---|---|
| D1: The first day of first administration; *P < 0.05, **P < 0.01, ***P < 0.001. | | | | |

The results of tumor volume indicated that by the end of the experiment (D18), the tumor volumes of the animals in groups B7H3-ADC-21 (10 mg/kg), B7H3-ADC-08 (3 mg/kg), B7H3-ADC-07 (10 mg/kg and 3 mg/kg) were significantly lower than that of the vehicle control group (P < 0.001), and the tumor inhibition rates of above groups were 46.92%, 47.37%, 71.54%, and 54.74%, respectively. Compared with the vehicle group, the tumor volume of animals in B7H3-ADC-21 (3 mg/kg) group did not show a statistical difference. The results of body weight showed that by the end of the administration (D18), the body weight of all test dose groups increased during the administration period, indicating that each dose group was well tolerated.

### 6.7.3 Efficacy assay of B7H3 antibody-drug conjugates on NCI-H358 xenografts

### 1. Research objective

The antitumor effect of test drugs in the subcutaneous xenograft female Balb/c Nude mouse model of human non-small cell lung cancer NCI-H358 was evaluated.

### 2. Test drug information

B7H3-ADC-21(DAR8), B7H3-ADC-05(DAR8), B7H3-ADC-07(DAR8), B7H3-ADC-09(DAR8), B7H3-ADC-10(DAR8), B7H3-ADC-20(DAR8).

### 3. Experimental content

NCl-H358 (ATCC, CRL-5807) cell treatment and tumor cell transplantation were performed with reference to Example 6.7.1.

### 3.1 Administrating and grouping

The number of animals in each group and the detailed administration routes, doses and schedules are shown in Table 22 below:

**Table 22. Dosage regimen.**

| No. | Test group | Animal number | Dosage (mg/kg) | Administration route and cycle |
|---|---|---|---|---|
| 1 | Normal saline | 5 | 3 | i.v., QW×3 |
| 2 | B7H3-ADC-21(DAR8) | 5 | 3 | i.v., QW×3 |
| 3 | B7H3-ADC-05(DAR8) | 5 | 3 | i.v., QW×3 |
| 4 | B7H3-ADC-07(DAR8) | 5 | 3 | i.v., QW×3 |
| 5 | B7H3-ADC-09(DAR8) | 5 | 3 | i.v., QW×3 |
| 6 | B7H3-ADC-10(DAR8) | 5 | 3 | i.v., QW×3 |
| 7 | B7H3-ADC-20(DAR8) | 5 | 3 | i.v., QW×3 |
| 8 | B7H3-ADC-20(DAR8) | 5 | 1 | i.v., QW×3 |

### 3.2 Test indicators

For the measurement of the tumor volume and body weight, both of the tumor volume (mm³) and the body weight were measured twice a week, and the relative tumor proliferation rate T/C(%) and the relative tumor inhibition rate TGI(%) were calculated.

The tumor volume (V) was calculated as the formula:$V = 1 / 2 \times {L}_{long} \times {{L}_{short}}^{2} ;$
Relative tumor proliferation rate T/C(%) = TRTV/CRTV × 100% (TRTV: the mean RTV of treatment group; CRTV: the mean RTV of control group; RTV = Vt/V0, V0 is the tumor volume of the animal at the time of grouping, Vt is the tumor volume of the animal after treatment);
Relative tumor inhibition rate TGI% = (1-T/C)× 100% (T and C are the relative tumor volume (RTV) of the treatment group and the control group at a specific time point, respectively).

### 3.3 Experimental end point

After confirming the end of the administration period, animals were continually observed for 1-2 weeks, and then the tumor was removed, weighed and photographed.

### 3. Experimental results

The specific results are shown in Table 23 and Figure 11. The ADCs of the present application all showed an inhibitory effect on tumor growth, and for ADC-20, the tumor-inhibition effect of 3mg/kg group was better than 1 mg/kg group. During the administration period, there was no obvious body weight loss and obvious drug toxicity in animals of each group.

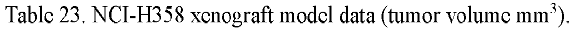

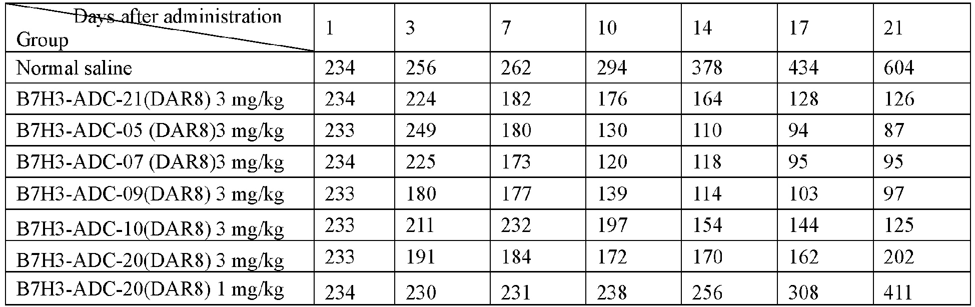

### 6.7.4 Efficacy assay of B7H3 antibody-drug conjugates in a subcutaneous xenograft PDX model of prostate cancer

### 1. Research objective

The efficacy of B7H3 antibody-drug conjugates in the subcutaneous xenograft NPG male mouse model of HuPrime^{®} prostate cancer PR9586 was evaluated.

### 2. Test compound

B7H3-ADC-07(DAR8), IgG1-ADC-07(DAR8)

### 3. Experimental content

### 3.1 Experimental mice information

NPG PR9586 model mice, 6-9 weeks of age (estimated weeks of age for mice at the time of tumor cell inoculation), male, 100 mice in total, purchased from Beijing Vitalstar Biotechnology Co.,Ltd.

### 3.2 Construction of tumor-bearing mouse model

Tumor tissues were collected from HuPrime^{®} prostate cancer PR9586 (race: Western) tumor-bearing mice, cut into tumor blocks of 2-3 mm diameter, and then inoculated into NPG right anterior scapular subcutaneously.

When the average tumor volume of tumor-bearing mice reached about 100-200 (~150) mm³, the mice were randomly grouped according to the table in "3.3 Grouping and administration". The coefficient of variation (CV) of tumor volume within each group was calculated according to the formula CV = SD/MTV×100%, and should be less than 40%. The day of grouping was defined as day 1.

### 3.3. Grouping and administration

The number of animals in each group and detailed administration routes, doses and schedules are shown in Table 24 below.

**Table 24. Grouping of experimental animals.**

| Group | Animal number | Treatment group | Dosage (mg/kg) | Administration route | Administration cycle |
|---|---|---|---|---|---|
| 1 | 8 | Vehicle(Normal saline) | / | *i.v.* | QW×3weeks |
| 2 | 8 | IgG1-ADC-07(DAR8) | 10 | *i.v.* | QW×3weeks |
| 3 | 8 | B7H3-ADC-07(DAR8) | 1 | *i.v.* | QW×3weeks |
| 4 | 8 | B7H3-ADC-07(DAR8) | 3 | *i.v.* | QW×3weeks |
| 5 | 8 | B7H3-ADC-07(DAR8) | 10 | *i.v.* | QW×3weeks |

| | | | | | |
|---|---|---|---|---|---|
| Note: 1. The volume of administration is 10 µL/g; 2. The day of grouping is defined as the first day (day 1), and the drug is administered on day 1, day 8 and day 15; 3. Protect from light during administration; 4. On the day of grouping, the tumor of one remaining mouse was collected for FACS detection to analyze the expression of tumor cell surface antigens, and flow cytometry results indicated that B7H3 was significantly expressed in PR9586 model. | | | | | |

### 3.4 Experimental observation and data collection

After tumor cell inoculation, routine monitoring included tumor growth and the effect of treatment on the normal behavior of animals, and the specific items comprised the activity of experimental animals, food and water intake, weight gain or loss, as well as eyes, fur and other abnormalities. Clinical symptoms observed during the experimental procedures were recorded in the raw data. After the start of administration, the body weight and tumor size of mice were measured twice a week. Tumor volume calculation formula: tumor volume (mm³) = 1/2 × (a × b²) (where a represents the long diameter, and b represents the short diameter). In the experiment, StudyDirector^{™} (version number 3.1.399.19, supplier Studylog System, Inc.) software was used to collect data, including the measurement of long and short diameters of tumors as well as the weighing of animal body weight. The raw data is directly imported into the software after being measured by the balance and vernier caliper. Any changes of the data will be recorded in this software.

The entire process including drug administration, tumor measurement, and body weight weighing, etc., was performed in a biological safety cabinet or a clean bench.

### 3.5 Data analysis

The main observation indicators of this experiment were:
the relative tumor proliferation rate, T/C(%), is the percentage of the relative tumor volume or tumor weight of the treatment group to that of the control group at a certain time point.

The calculation formula is: $T / C % = {T}_{RTV} / {C}_{RTV} \times 100 %$ (T_{RTV}: the mean RTV of the treatment group; C_{RTV}: the mean RTV of the control group; RTV = Vₜ/V₀, V₀ is the tumor volume of the animal at the time of grouping, Vₜ is the tumor volume of the animal after treatment);
or T/C % = T_{TW}/C_{TW} × 100% (T_{TW}: the average tumor weight of the treatment group at the end of the experiment; C_{TW}: the average tumor weight of the control group at the end of the experiment).

The relative tumor inhibition rate, TGI (%), was calculated as: $TGI % = \left(1-T/C\right) \times 100 %$ (T and C are the relative tumor volume (RTV) or tumor weight (TW) of the treatment group and the control group at a specific time point, respectively).

### 4. Experimental results

The efficacy results of B7H3-ADC-07(DAR8) and IgG1-ADC-07(DAR8) in PR9586 model are shown in Figure 12, Table 25, and Table 26:

**Table 25. Tumor volume analysis table for each group in the HuPrime^{®} prostate cancer PR9586 model.**

| Experimental group | Day 22 after grouping | | | |
|---|---|---|---|---|
| | Tumor volume (*x̅* ±S) | TGI (%) | T/C (%) | P value |
| Group 1 Vehicle | 874.80 ± 135.11(8) | -- | -- | -- |
| Group 2 IgG1-ADC-07(DAR8) 10 mg/kg | 129.30 ± 49.16(8) | 85.73 | 14.27 | <0.001 |
| Group 3 B7H3-ADC-07(DAR8) 1 mg/kg | 135.42 ± 39.36(8) | 84.00 | 16.00 | 0.009 |
| Group 4 B7H3-ADC-07(DAR8) 3 mg/kg | 92.61 ± 36.73(8) | 88.96 | 11.04 | <0.001 |
| Group 5 B7H3-ADC-07(DAR8) 10 mg/kg | 104.38 ± 33.65(8) | 87.7 | 12.30 | <0.001 |

| | | | | |
|---|---|---|---|---|
| Note: 1. Data are expressed as "mean ± standard error"; 2. T/C% = T_{RTV}/C_{RTV} × 100%; TGI% = (1-T/C) × 100%; | | | | |

**Table 26. Tumor weight analysis table of each group in the HuPrime^{®} prostate cancer PR9586 model.**

| Experimental Group | Tumor weight (mg)(*×̅* ±s) Day 22 after grouping | TGI(%) | T/C (%) | P value |
|---|---|---|---|---|
| Group 1 Vehicle | 473.29 ± 91.75(8) | | | |
| Group 2 IgG1-ADC-07(DAR8) 10 mg/kg | 52.96 ± 22.85(8) | 88.81 | 11.19 | 0.002 |
| Group 3 B7H3-ADC-07(DAR8) 1 mg/kg | 52.64 ± 19.72(8) | 88.88 | 11.12 | 0.006 |
| Group 4 B7H3-ADC-07(DAR8) 3 mg/kg | 27.90 ± 14.59(8) | 94.11 | 5.89 | <0.001 |
| Group 5 B7H3-ADC-07(DAR8) 10 mg/kg | 46.40 ± 24.35(8) | 90.20 | 9.80 | 0.001 |

| | | | | |
|---|---|---|---|---|
| Note: 1. Data are expressed as "mean ± standard error"; 2. T/C% = T_{TW}/C_{TW} × 100%; TGI% = (1-T/C) × 100%; | | | | |

### 5. Conclusion

In PR9586 model, the results of tumor volume measurement showed that at the end of the experiment (Day 22), the tumor volume of the animals in B7H3-ADC-07(DAR8) 3 mg/kg and 10 mg/kg groups was significantly lower (p<0.001) than that in the vehicle (normal saline) group. The tumor volume inhibition rates (TGI) of B7H3-ADC-07(DAR8) 1 mg/kg, 3 mg/kg and 10 mg/kg groups were 84.00%, 88.96% and 88.7%, respectively. The results of animal body weight measurement showed that at the end of the experiment (Day 22), the body weight of the animals in each dose group of B7H3-ADC-07(DAR8) all increased, which didn't have significant difference from the control group, suggesting the doses of 1 mg/kg, 3 mg/kg, and 10 mg/kg were well tolerated. The results of tumor weight measurement showed that at the end of the experiment (Day 22), the mean tumor weight of the animals in B7H3-ADC-07(DAR8) 3 mg/kg group was significantly lower than that in the vehicle group, with statistical significance (P < 0.001). The tumor weight TGI of B7H3-ADC-07(DAR8) 1 mg/kg, 3 mg/kg and 10 mg/kg groups were 88.88%, 94.11% (P < 0.001) and 90.20%, respectively. Regardless of tumor volume or tumor weight, IgG1-ADC-07(DAR8) group had an excellent tumor inhibition effect, with TGI values of 85.7% and 88.8%, respectively. The results showed that the antibody-drug conjugate and its drug-linker (toxin-linker) provided by the present disclosure did not require the endocytosis of the antibody, nor the positive expression of the tumor cell surface antigens to achieve anti-tumor effect. At the same time, the body weight of each experimental group was not significantly different from that of the control group, and the antibody drug conjugate of the present disclosure had good safety.

### 6.7.5 Efficacy of B7H3 antibody-drug conjugates in subcutaneous xenograft PDX model of esophageal squamous cell carcinoma

### 1. Research objective

The efficacy of B7H3-ADC-07(DAR8) and IgG1-ADC-07(DAR8) in a BALB/c female nude mouse subcutaneous xenograft model of esophageal squamous cell carcinoma ES0204 was investigated.

### 2. Test compounds

B7H3-ADC-07(DAR8), IgG1-ADC-07(DAR8)

### 3. Experimental content

### 3.1 Experimental mouse information

BALB/c nude for ES0204 model mice, 6-9 weeks (estimated ages of week for mice at the time of tumor cell inoculation), female, 100 mice in total (40 mice, plus 150% surplus), purchased from Jiangsu Gempharmatech Co., Ltd.

### 3.2 Construction of tumor-bearing mouse model

Tumor tissue was collected from esophageal squamous cell carcinoma ES0204 (subtype: ECC, race: Asian) tumor-bearing mice, cut into tumor pieces with a diameter of 2-3 mm, and inoculated subcutaneously at the right anterior scapula of Balb/c nude mice.

When the average tumor volume of tumor-bearing mice reached about 100-200 (~150) mm³, the mice were randomly grouped according to the table in "3.3 Grouping and administration". The coefficient of variation (CV) of tumor volume within each group was calculated as the formula CV = SD/MTV×100%, and should be less than 40%. The day of grouping was defined as day 1.

### 3.3. Grouping and administration

The number of animals in each group and detailed administration routes, doses and schedules are shown in Table 27 below.

**Table 27. Grouping of experimental animals.**

| Groups | Animal numbers | Test groups | Dosage (mg/kg) | Administration route | Administration cycle |
|---|---|---|---|---|---|
| 1 | 8 | Vehicle(Normal saline) | / | *i.v.* | QW×3weeks |
| 2 | 8 | IgG1-ADC-07(DAR8) | 10 | *i.v.* | QW×3weeks |
| 3 | 8 | B7H3-ADC-07(DAR8) | 1 | *i.v.* | QW×3weeks |
| 4 | 8 | B7H3-ADC-07(DAR8) | 3 | *i.v.* | QW×3weeks |
| 5 | 8 | B7H3-ADC-07(DAR8) | 10 | *i.v.* | QW×3weeks |

| | | | | | |
|---|---|---|---|---|---|
| Note: 1. The volume of administration was 10µL/g; 2. The day of grouping was defined as the first day (day 1), and the drug was administered on day 1, day 8 and day 15; 3. Protect from light during administration; 4. On the day of grouping, the tumor of one remaining mouse was collected for FACS detection to analyze the expression of tumor cell surface antigens, and flow cytometry results indicated that B7H3 was significantly expressed in ES0204 model. | | | | | |

### 3.4 Experimental observation and data collection

After tumor cell inoculation, routine monitoring included tumor growth and the effect of treatment on the normal behavior of animals, and the specific items comprised the activity of experimental animals, food and water intake, weight gain or loss, as well as eyes, fur and other abnormalities. Clinical symptoms observed during the experimental procedures were recorded in the raw data. After the start of dosing, the body weight and tumor size of mice were measured twice a week. Tumor volume calculation formula: tumor volume (mm³) = 1/2 × (a × b²) (where a represents the long diameter, and b represents the short diameter). In the experiment, StudyDirector^{™} (version number 3.1.399.19, supplier Studylog System, Inc.) software was used to collect data, including the measurement of long and short diameters of tumors as well as the weighing of animal body weight. The raw data were measured by a balance and a vernier caliper, and then directly imported into the software. Any changes in the data will be recorded in this software.

The entire process including drug administration, tumor measurement, and body weight weighing, etc., was performed in a biological safety cabinet or a clean bench.

### 3.5 Data analysis

The main observation indicators of this experiment were:
The relative tumor proliferation rate, T/C(%), is the percentage of the relative tumor volume or tumor weight of the treatment group to that of the control group at a certain time point.

The calculation formula is: $T / C % = {T}_{RTV} / {C}_{RTV} \times 100 %$ (T_{RTV}: the mean RTV of the treatment group; C_{RTV}: the mean RTV of the control group; RTV = Vₜ/V₀, V₀ is the tumor volume of the animal at the time of grouping, Vₜ is the tumor volume of the animal after treatment);
or T/C% = T_{TW}/C_{TW} × 100% (T_{TW}: the average tumor weight of the treatment group at the end of the experiment; C_{TW}: the average tumor weight of the control group at the end of the experiment).

The relative tumor inhibition rate, TGI (%), was calculated as: $TGI % = \left(1-T/C\right) \times 100 %$ (T and C are the relative tumor volume (RTV) or tumor weight (TW) of the treatment group and the control group at a specific time point, respectively).

### 4. Experimental results

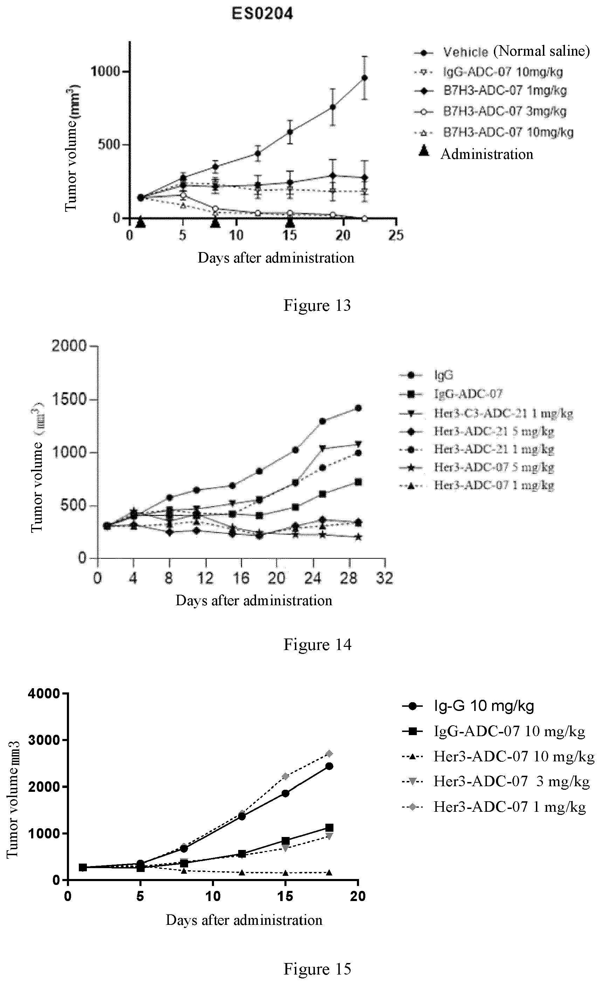

The efficacy results of B7H3-ADC-07(DAR8) and IgG1-ADC-07(DAR8) in ES0204 model are shown in Figure 13, Table 28, and Table 29:

**Table 28. Analysis table for tumor volume of each group in the HuPrime^{®} esophageal squamous cell carcinoma ES0204 model.**

| Experimental group | 22 days after grouping | | | |
|---|---|---|---|---|
| | Tumor volume (*x̅* ±S) | TGI (%) | T/C (%) | P value |
| Group 1 Vehicle | 959.89 ± 146.45(8) | - | - | - |
| Group 2 IgG1-ADC-07(DAR8)10 mg/kg | 182.05 ± 66.12(8) | 81.28 | 18.72 | <0.001 |
| Group 3 B7H3-ADC-07(DAR8) 1 mg/kg | 277.49 ± 115.05(8) | 75.71 | 24.29 | <0.001 |
| Group 4 B7H3-ADC-07(DAR8) 3 mg/kg | 0.00 ± 0.00(8) | 100.00 | 0.00 | <0.001 |
| Group 5 B7H3-ADC-07(DAR8)10 mg/kg | 0.00 ± 0.00(8) | 100.00 | 0.00 | <0.001 |

| | | | | |
|---|---|---|---|---|
| Note: 1. Data are expressed as "mean ± standard error"; 2. T/C% = T_{RTV}/C_{RTV} × 100%; TGI% = (1-T/C) × 100%; | | | | |

**Table 29. Analysis table for tumor weight of each group in the HuPrime^{®} esophageal squamous cell carcinoma ES0204 model.**

| Experimental group | Tumor weight (mg) (*x̅* ±s) | TGI | T/C (%) | P value (compared with the control group) |
|---|---|---|---|---|
| | Day 22 after grouping | | | |
| Group 1 Vehicle | 794.41 ± 143.85(8) | -- | -- | -- |
| Group 2 IgG1-ADC-07(DAR8)10 mg/kg | 38.14 ± 13.37(8) | 95.20 | 4.80 | <0.001 |
| Group 3 B7H3-ADC-07(DAR8) 1 mg/kg | 193.65 ± 90.66(8) | 75.62 | 24.38 | <0.001 |
| Group 4 B7H3-ADC-07(DAR8) 3 mg/kg | 0.00 ± 0.00(8) | 100.00 | 0.00 | <0.001 |
| Group 5 B7H3-ADC-07(DAR8) 10 mg/kg | 0.00 ± 0.00(8) | 100.00 | 0.00 | <0.001 |

| | | | | |
|---|---|---|---|---|
| Note: 1. Data are expressed as "mean ± standard error"; 2. T/C% = T_{TW}/C_{TW} × 100%; TGI% = (1-T/C) × 100%; | | | | |

### 5. Conclusion

In ES0204 model, the results of tumor volume measurement showed that at the end of the experiment (Day 22), the tumor volumes of animals in B7H3-ADC-07(DAR8) 1 mg/kg, 3 mg/kg, and 10 mg/kg groups were significantly lower than that in vehicle (normal saline) group (P < 0.001), and the tumor volume inhibition rate (TGI) of B7H3-ADC-07(DAR8) 1 mg/kg, 3 mg/kg, and 10 mg/kg dose groups were 75.71%, 100.00%, and 100.00%, respectively, which is positively correlated with dose. The results of animal body weight measurement showed that at the end of the experiment (Day 22), during the administration, animal body weight for each dose group of B7H3-ADC-07(DAR8) increased and was not significantly different from that of the control group, indicating that the animals well tolerated the doses of 1 mg/kg, 3 mg/kg and 10 mg/kg of B7H3-ADC-07(DAR8). The results of tumor weight measurement showed that at the end of the experiment (Day 22), the mean tumor weights of the animals in the B7H3-ADC-07(DAR8) 1 mg/kg, 3 mg/kg, and 10 mg/kg groups were significantly lower than those in the Vehicle group, with statistical significance (P < 0.001), and the tumor weight TGI values were 75.62%, 100.00% and 100.00%, respectively; IgG1-ADC-07 (DAR8) group also had an excellent tumor inhibition effect, and TGI values were 81.3% (tumor volume) and 95.2% (tumor weight), respectively. The results showed that the antibody-drug conjugate and its drug-linker (toxin-linker) provided by the present disclosure can achieve anti-tumor effect without the endocytosis of the antibody, and without the positive expression of the tumor cell surface antigens. At the same time, the body weight of each experimental group was not significantly different from that of the control group, and the antibody drug conjugate of the present disclosure had good safety.

### Example 6.8: In vivo activity test of anti-Her3 antibody drug conjugate and IgG1-ADC

### 6.8.1 Efficacy assay of anti-Her3 antibody drug conjugate on NCI-H358 xenograft tumor

The present disclosure demonstrated the *in vivo* efficacy by evaluating the antitumor effect of an anti-Her3 antibody ADC drug in a Balb/c Nude mouse subcutaneously transplanted tumor model of human non-small cell lung cancer cell line NCI-H358 cells (available from ATCC, about 10% of Her3 expression positive rate).

The specific steps are as follows: Balb/c nu nude mice, female, 5-6 weeks of age, were purchased from Vital River Laboratory Animal Technology Co., Ltd.. NCI-H358 cells were cultured in a 15 cm diameter petri dish with 1640 medium (containing 10% FBS), digested with trypsin-EDTA when the confluency reached about 80-90%, washed twice with PBS, and then centrifuged and resuspended in pre-cooled in PBS. Cells were counted by a cell counter, and then the cells were diluted with PBS to a concentration of 5×10⁷ cells/ml. After adapting to the laboratory environment for 7 days, Balb/c nu mice were subcutaneously inoculated with NCI-H358 cells in the right rib, with an inoculation amount of 5×10⁶ cells/mouse and an inoculation volume of 0.2 ml (containing 50% Matrigel). When the tumor grew to about 300 mm³, mice were randomly grouped according to the tumor size, 5 mice for each group: anti-chicken lysozyme human IgG1 isotype antibody (negative control, prepared by MediLink Therapeutics (Suzhou) Co., Ltd.) group, IgG1-ADC-07(DAR8), Her3-C3-ADC-21(DAR4), Her3-ADC-21(DAR4) and Her3-ADC-07(DAR8) dose groups. All samples were injected into the tail vein, once a week, for a total of 3 weeks. Tumor volume and body weight of mice were observed and measured periodically after administration. The relative tumor proliferation rate, T/C(%), is the percentage of the relative tumor volume or tumor weight of the treatment group to those of control group at a specific time point. Relative tumor inhibition rate, TGI(%), was calculated as the formula: TGI% = (1-T/C) × 100% (T and C are the relative tumor volume (RTV) or tumor weight (TW) of the treatment and control groups at a specific time point, respectively).

The results are shown in Table 30 and Figure 14.

**Table 30. Effect of Her3-ADC-07 on subcutaneously xenograft of human non-small cell lung cancer NCI-H358 in nude mice.**

| Group | Mean tumor volume (mm³) | Mean tumor volume (mm³) | T/C (%) | Tumor inhibition rate TGI(%) | *P* value (D29) |
|---|---|---|---|---|---|
| | D1 | D29 | D29 | D29 | |
| IgG1 5 mg/kg | 309.0±72.0 | 1425.0±241.2 | - | - | - |
| IgG1-ADC-07(DAR8) 5 mg/kg | 309.4±53.4 | 725.2±415.1 | 50.83 | 49.17 | 0.203 |
| Her3-C3-ADC-21(DAR4) 1 mg/kg | 308.8±67.7 | 1080.8±741.8 | 75.89 | 24.11 | 0.996 |
| Her3-ADC-21(DAR4) 5 mg/kg | 309.6±60.6 | 346.4±123.8** | 24.26 | 75.74 | 0.002 |
| Her3-ADC-21(DAR4) 1 mg/kg | 309.6±58.6 | 1000.6±184.1 | 70.08 | 29.92 | 0.214 |
| Her3-ADC-07(DAR8) 5 mg/kg | 309.4±43.4 | 205.4±56.7** | 14.40 | 85.60 | 0.003 |
| Her3-ADC-07(DAR8) 1 mg/kg | 309.6±47.1 | 339.0±95.8** | 23.74 | 76.26 | 0.003 |

Based on the experimental results, compared with IgG1 group, both 5 mg/kg and 1 mg/kg of Her3-ADC-07(DAR8) significantly inhibited the tumor growth of NCI-H358 human non-small cell lung cancer xenograft model, and presented a dose gradient trend. The TGI value of Her3-ADC-07 1mg/kg group was 76.26%, slightly greater than the TGI value 75.74% of Her3-ADC-21 5 mg/kg group. The TGI value of Her3-ADC-21 1 mg/kg group was 29.92%, slightly greater than the TGI value 24.11% of Her3-C3-ADC-21 1 mg/kg group.

Surprisingly, compared with IgG1 group, IgG1-ADC-07 also had significant tumor-inhibiting effect, indicating that the resulting conjugates (ADCs) using the linker of the present disclosure have better tumor tissue targeting and enhanced the exposure of the overall ADC in the tumor environment regardless with or without antigen-binding activity or endocytosis activity. The ADC of current disclosure had better tumor inhibition effects whether with or without tumor antigen expression.

In addition, the results of animal body weight showed that the animals in each test group tolerated well during the administration period and the recovery period.

### 6.8.2 Efficacy assay I of anti-Her3 antibody drug conjugate on SW480 xenograft tumor

The present disclosure demonstrated the *in vivo* efficacy by evaluating the antitumor effect of an anti-Her3 antibody ADC drug in subcutaneously transplanted tumor model of human colon cancer cell line SW480 cells (available from ATCC, about 30% of Her3 expression positive rate) of Balb/c Nude mice. The specific steps are as follows: 36 Balb/c nu nude mice, female, 5-6 weeks of age, were purchased from Vital River Laboratory Animal Technology Co., Ltd.. SW480 cells were cultured in a 15 cm diameter petri dish with 1640 medium (containing 10% FBS), digested with trypsin-EDTA when the confluency reached about 80-90%, washed twice with PBS, and then centrifuged and resuspended in pre-cooled PBS. Cells were counted by a cell counter, and then the cells were diluted with PBS to a concentration of 5×10⁷ cells/ml. After adapting to the laboratory environment for 7 days, Balb/c nu mice were subcutaneously inoculated with SW480 cells in the right rib, with an inoculation amount of 5×10⁶ cells/mouse and an inoculation volume of 0.2 ml (containing 50% Matrigel). When the tumor grew to about 250 mm³, mice were randomly grouped according to the tumor size, 5 mice for each group: anti-chicken lysozyme human IgG1 isotype antibody (negative control, prepared by MediLink Therapeutics (Suzhou) Co., Ltd.) group, as well as IgG1-ADC-07 and Her3-ADC-07 dose groups. All samples were injected into the tail vein, once a week, for a total of 3 weeks. Tumor volume and body weight of mice were observed and measured periodically after administration.
1) The relative tumor proliferation rate, T/C(%), is the percentage of the relative tumor volume or tumor weight of the treatment group to those of control group at a specific time point.
2) Relative tumor inhibition rate, TGI(%), was calculated as the formula: TGI% = (1-T/C) × 100% (T and C are the relative tumor volume (RTV) or tumor weight (TW) of the treatment and control groups at a specific time point, respectively).

The specific results are shown in Table 32 and Figure 15. According to the experimental results, both 10 mg/kg and 3 mg/kg doses of Her3-ADC-07 had a significant inhibitory effect on the tumor growth of SW480 human colorectal cancer xenograft model, and presented a dose gradient trend.

**Table 31: Efficacy results of SW480 + Balb/c Nude mouse model.**

| Group | Mean tumor volume (mm³) | Mean tumor volume (mm³) | T/C (%) | Tumor inhibition rate TGI(%) | *P* value (D18) |
|---|---|---|---|---|---|
| | D1 | D18 | D18 | D18 | |
| Ig-G1 10 mg/kg | 269.80±32.42 | 2437.80±378.82 | - | - | - |
| IgG1-ADC-07(DAR8) 10 mg/kg | 270.60±33.13 | 1129.40±856.17** | 46.2 | 53.8 | 0.007 |
| Her3-ADC-07(DAR8) 10 mg/kg | 269.20±31.17 | 164.80±39.81*** | 6.8 | 93.2 | 0.000 |
| Her3-ADC-07(DAR8) 3 mg/kg | 270.20±31.40 | 939.00±744.80** | 38.5 | 61.5 | 0.002 |
| Her3-ADC-07 (DAR8) 1 mg/kg | 271.60±38.35 | 2708.80±535.14 | 110.4 | -10.4 | 0.876 |

### 6.8.3. Efficacy assay II of anti-Her3 antibody drug conjugate on SW480 xenograft tumor

The antitumor effects of IgG1, IgG1-ADC-07(DAR8), Her3-ADC-07(DAR8) and Her3-C3-ADC-21(DAR8) in a transplanted tumor model of human colon cancer cell lines SW480 (available from ATCC, about 30% of Her3 expression positive rate) were determined by referring to the same model and method as Example 6.8.2..

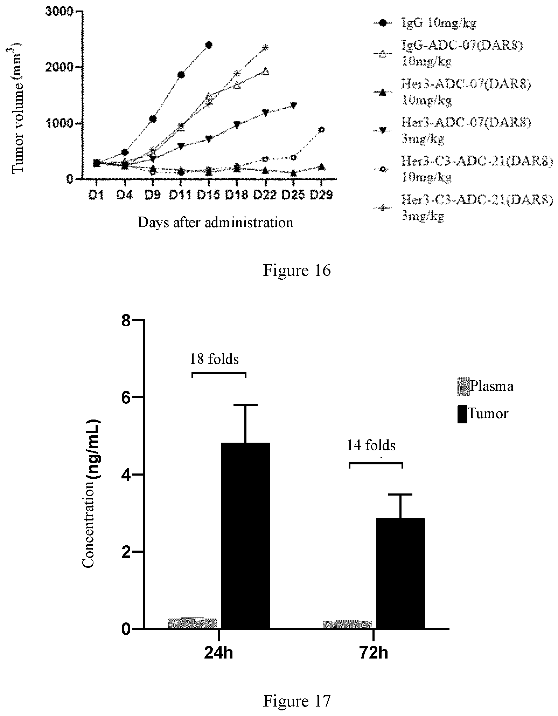

The specific results are shown in Table 32 and Figure 16.

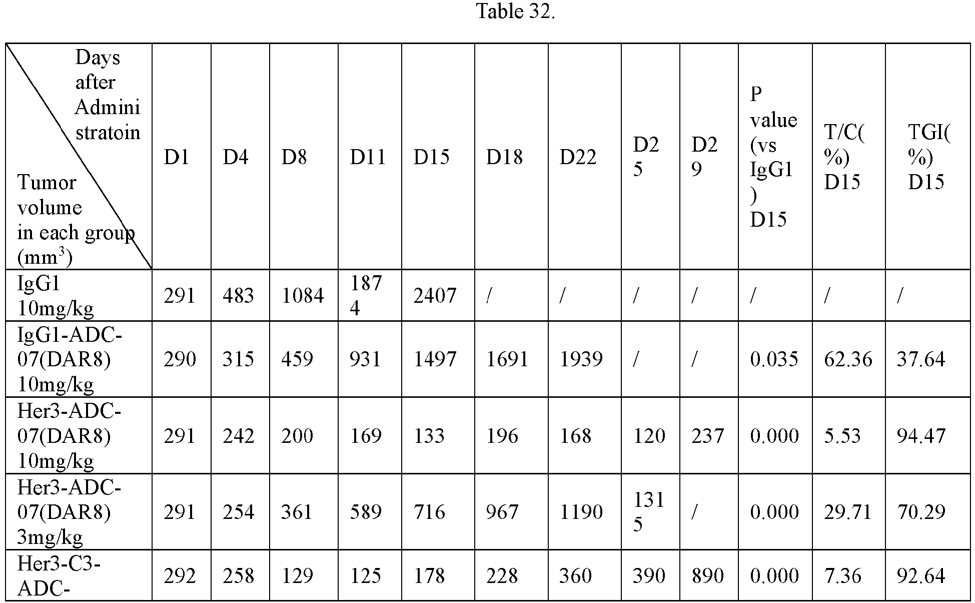

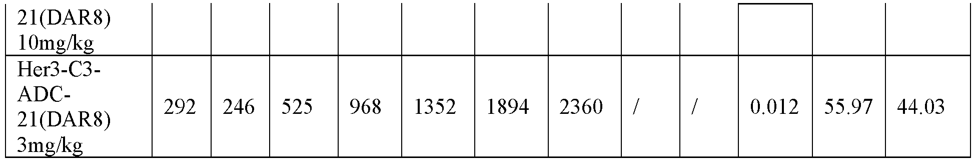

According to the experimental results, both 10 mg/kg and 3 mg/kg doses of Her3-ADC-07 had a significant inhibitory effect on the tumor growth of SW480 human colorectal cancer xenograft model, and presented a dose gradient trend.

Unexpectedly, the tumor-inhibitory effect of Her3-ADC-07(DAR8) 3 mg/kg group was significantly stronger than that of Her3-C3-ADC-21(DAR8) 3 mg/kg group, and the tumor-inhibitory effect of Her3-ADC-07(DAR8) 10 mg/kg group was significantly stronger than that of Her3-C3-ADC-21(DAR8) 10 mg/kg group.

Surprisingly, compared with IgG1 group, IgG1-ADC-07 also had a significant tumor-inhibitory effect in SW480 human colorectal cancer xenograft model and had better tumor-inhibitory effect than Her3-C3-ADC-21(DAR8) 3 mg/kg group, indicating that the resulting conjugates (ADC) using the linker of the present disclosure has better tumor tissue targeting and increased the exposure of the entire ADC in the tumor environment regardless with or without antigen-binding activity or endocytosis activity of the antibody., And thus the ADC had a better tumor inhibition effects whether with or without tumor antigen expression.

In addition, the results of animal body weight showed that the animals in each test group tolerated well during the administration period and the recovery period.

### Example 6.9 ADC plasma stability test

The stability of B7H3-ADC-07(DAR8) in human plasma was evaluated by measuring the release of bioactive molecule A1.9 in human plasma in which B7H3-ADC-07(DAR8) was incubated, as well as Dxd release in human plasma in which B7H3-C1-ADC-21(DAR4) was incubated.

### Samples and groups

**Table 33**

| Group | Test substance | Incubation concentration | System | Analyte | Detection time point |
|---|---|---|---|---|---|
| 1 | B7H3-ADC-07(DAR8) | 200 µg/mL | human plasma | A1.9 | 0 h, 4h, 24h, 2d, 3d, 7d, 10d, 14d, 17d, 21d, 28d |
| 5 | B7H3-C1-ADC-21 (DAR4) | 200 µg/mL | human plasma | Dxd | |

Preparation of solution: B7H3-ADC-07(DAR8) and B7H3-C1-ADC-21(DAR4) stock solutions were diluted with PBS into a working solution at a concentration of 2 mg/mL. The appropriate concentration of internal standard stock solution was prepared with DMSO, and then acetonitrile or other reagents were used to prepare the appropriate concentration of the internal standard-containing precipitant.

Incubation of sample: the bacteriostatic agent ProClin was added to the plasma of each genera (human, cynomolgus monkey, ICR mouse) and PBS containing 1% BSA, and the final concentration of ProClin was 0.1%, Sterile operations were adopted. 40 µL working solution of test substance and 360 µL of human plasma were mixed to prepare 200 µg/mL of incubation sample. The plate was sealed with a film, and then incubated at 37 °C under 5% CO₂ in an incubator, with a shaking speed of 80 times/min. At 0 h, 4 h, 24 h, 2d, 3d, 7d, 10d, 14d, 17d, 21d and 28d, 20 µL of plasma sample was transferred, to which was added 200 µL of precipitant (containing internal standard), and completely mixed to precipitate the proteins.

All samples were subject to protein precipitating, and then centrifuged to collect the supernatant, which was diluted with water, and analyzed by LC-MS/MS. The analytical conditions were:

**Table 34. Analytical conditions for plasma stability testing.**

| | | | | | | | | |
|---|---|---|---|---|---|---|---|---|
| Analyte | A1.9 | | | | Dxd | | | |
| Detection method | LC-MS/MS-6500-001 | | | | LC-MS/MS-6500-001 | | | |
| Internal standard | Tolbutamide | | | | Tolbutamide | | | |
| MS conditions | Positive ion, ESI | | | | Positive ion, ESI | | | |
| Mobile phase | A: 0.1%FA in H₂O | | | | A: 0.1%FA in H₂O | | | |
| | B: 0.1%FA in ACN | | | | B: 0.1%FA in ACN | | | |
| Column | Waters, BEH C18, 1.7 µm, 2.1*50 mm | | | | Aglient, ZORBAX XDB-C18, 3.5 µm, 2.1*50 mm | | | |
| Flow rate | 0.5 mL/min | | | | 0.5 mL/min | | | |
| Liquid chromatograp hy conditions | Time (min) | | Pump B (%) | | Time (min) | | Pump B (%) | |
| | 0.4 | | 5 | | 0.4 | | 10 | |
| | 1.8 | | 95 | | 1.8 | | 95 | |
| | 2.2 | | 95 | | 2.2 | | 95 | |
| | 2.21 | | 5 | | 2.21 | | 10 | |
| | 2.5 | | termination | | 2.5 | | termination | |
| Detection | Compound | m/z | De-clustering voltage (v) | collision energy (v) | Compound | m/z | De-clustering voltage (v) | collision energy (v) |
| | Tolbutamide | 271.0-172.0 | 50 | 30 | DXD | 494.2-375.1 | 100 | 45 |
| | A1.9 | 451.1-407.2 | 50 | 25 | | | | |

The results obtained are shown in Table 35 below:

**Table 35**

| Time (day) | Release ratio (%) | |
|---|---|---|
| | A1.9 | DXD |
| 0 | 0.14 | 0.348 |
| 0.17 | 0.10 | 1.786 |
| 1 | 0.11 | 2.765 |
| 2 | 0.16 | 3.603 |
| 3 | 0.14 | 3.763 |
| 7 | 0.19 | 4.116 |
| 10 | 0.24 | 5.126 |
| 14 | 0.35 | 5.786 |
| 17 | 0.40 | 6.446 |
| 21 | 0.46 | 7.494 |
| 28 | 0.82 | 12.659 |

B7H3-ADC-07(DAR8) had good stability in human plasma, and after 28 days of incubation, the released bioactive molecules (A1.9) accounted for less than 2% of the total bioactive molecules. In contrast, B7H3-C1-ADC-21(DAR4) released significantly more Dxd in human plasma.

### Example 6.10 Test experiment of tumor tissue distribution of antibody drug conjugates in HT 29 Xenografts

### 6.10.1 Experimental content

### Step 1. Cell treatment

Human colon cancer HT29 cells were purchased from ATCC. HT29 cells were adherently cultured in a 15 cm² culture dish, and the culture conditions were 1640 media containing 10% fetal bovine serum, and the cells were cultured at 37 °C in an incubator containing 5% CO₂. When cells were in exponential growth phase, cells were digested with trypsin, harvested, counted, and inoculated.

### Step 2. Transplantation of tumor cells

After adapting to the laboratory environment for 2-5 days, BALB/c Nude mice were subcutaneously inoculated with HT29 cells in the right rib, with an inoculation amount of 5×10⁶ cells/mouse and an inoculation volume of 0.2 ml (containing 50% Matrigel). The experiment was carried out when the tumor grew to about 200-300 mm³.

### Step 3. Administration and sampling

The number of animals in each group and the detailed route of administration, dosage and protocol are shown in the table below.

**Table 36**

| Group | Animal number | Test group | Dosage (mg/kg ) | Administ ration volume (µL/g) | Administration route | Administration cycle | Collecting samples | Sampling time point |
|---|---|---|---|---|---|---|---|---|
| 1 | 3 | Normal saline | / | 10 | i.v. | Single | Tumor and plasma | 72 h after administration |
| 2 | 3 | B7H3-ADC-07 (DAR8) | 10 | 10 | i.v. | Single | Tumor and plasma | 24 h after administration |
| 3 | 3 | B7H3-ADC-07 (DAR8) | 10 | 10 | i.v. | Single | Tumor and plasma | 72 h after administration |

### Step 4. Tumor tissue homogenate

The tumor tissue pieces were sheared into small size on ice, weighed and transferred to a grinding tube. The tissue homogenate was prepared by grinding them with normal saline solution at 1:5 (w/v); tissue homogenate supernatant was prepared by centrifugation for 10 min at 2~8 °C and 1800 × g, and then stored at -15~-25 °C for use. Before use, it was taken out and left at 15-25 °C, naturally warmed to room temperature, and then used.

### Step 5. Analysis of biological samples

For the analysis method of A1.9, refer to Example 6.9.

### Analysis of B7H3-ADC-07(DAR8)

(1) Coating: the anti-toxin antibody was diluted with coating solution (0.05M CBS) to 1 µg/mL, and added to the microtiter plate at 100 µL/well, covered with film, and allowed to stand at 2~8 °C overnight (more than 12 h).
(2) Washing the plate: the solution in the well was discarded, and the plate was washed three times with 0.05% PBST at 300 µL/well, and pat dry.
(3) Blocking: blocking solution (2%BSA-1×PBST) was added at 300 µL/well, and then the plate was covered with film, and incubated at 37 °C for 2 h±20 min.
(4) Washing the plate: the solution in the well was discarded, and the plate was washed three times with 0.05% PBST at 300 µL/well, and pat dry.
(5) Adding sample: to the enzyme labelling plate, were added the matrix solution, the standard curve sample, the quality control sample and the test sample, 100 µL/well, and then the plate was covered with film, and incubated at 37°C for 2 h±5 min. each sample was tested in duplicate.
(6) Washing the plate: the solution in the well was discarded, and the plate was washed three times with 0.05% PBST at 300 µL/well, and pat dry.
(7) Adding the secondary antibody: Goat Anti-Human IgG Fc-HRP was diluted with diluent (2%BSA-1×PBST) at a ratio of 1:40000 (16 ng/ml), 100 µL/well. The plate was covered with film, and then incubated at 37 °C for 1 h± 5 min.
(8) Washing the plate: the solution in the well was discarded, and the plate was washed three times with 0.05% PBST at 300 µL/well, and pat dry.
(9) Color development: the color development solution for TMB substrate was added at 100 µL/well, and then the plate was covered with film, and placed at 15~25 °C for 2~15 min in the dark for color development.
(10) stopping: the reaction was terminated by adding a stopping solution (0.5 M H₂SO₄) at 100 µL /well.
(11) Detection: the OD value of each well was read at the detection wavelength of 450 nm (reference wavelength of 620 nm) with a reader.

### Experimental results

The distribution results of A1.9 were shown in Figure 17. The distribution of A1.9 in tumors have up to 18-fold distribution difference compared with the distribution in plasma, and A1.9 in the tumor tissue accounted for the vast majority.

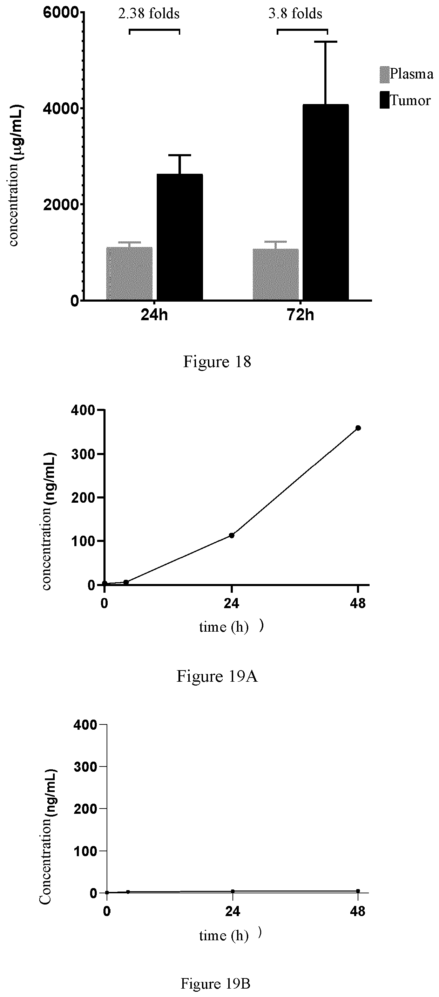

The distribution results of ADC are shown in Figure 18. In tumor/serum, ADC molecules had obvious differences in the distribution ratio and were time-dependent, which proved that ADC molecules of the present disclosure or ADCs containing linkers of the present disclosure were more likely to be distributed in tumor tissues. This resulted in the exposure of the entire ADC molecule in a relatively acidic tumor environment, and thus the ADCs had better tumor tissue targeting which increased the concentration ratio of the bioactive molecules in tumor to that in blood, and reduced the mechanism-related toxicity of ADC molecules (or also known as "on-target safety"). The formula for calculating the relative on-target safety between different ADCs was:
(*In vitro* EC50 inhibitory concentration of the first ADC against tumor cells/*In vivo* administration dosage in the same tumor cell model of the first ADC)/(*In vitro* EC50 inhibitory concentration of the second ADC against tumor cells/*In vivo* administration dosage in the same tumor cell model of the second ADC under the circumstance it has an *in vivo* effect close to that of the first ADC)

Specifically, combined with the data in the above examples, the effects of B7H3-ADC-07 3 mg/kg group and B7H3-ADC-21 10 mg/kg group were close, as shown in Figure 10. The relative on-target safety of B7H3-ADC-07 against HT29 cells compared with B7H3-ADC-21 was: $\left(10433 ng/mL/150KDa/3mg/kg\right) / \left(7442 ng/mL/150 KDa/10mg/kg\right) = 4.66$

Therefore, B7H3-ADC-07 had a 4.66-fold relative on-target safety compared with B7H3-ADC-21 against HT29 cells. In other words, due to the better tumor tissue targeting of the ADC of the present disclosure, the administration dose to achieve the same *in vivo* tumor inhibitory effect was lower (e.g. in Example 6.7.2, the TGI values for both B7H3-ADC-08 3 mg/kg group and B7H3-ADC-07 3 mg/kg group were all close to that of B7H3-ADC-21 10 mg/kg group).

### Example 6.11 Stability test of antibody-drug conjugates in tumor tissue homogenate and muscle tissue homogenate

### Research objective:

The research objective of this experiment was to simulate the cleavage of B7H3-ADC-07(DAR8) in tumor tissue/tumor microenvironment, to investigate the *in vitro* stability of B7H3-ADC-07 in tumor tissue homogenates of 22RV1 Balb/c nu nude mouse, in muscle tissue homogenates of Balb/c mouse, and in homogenate matrix normal saline, and to determine A1.9 in the incubation system of B7H3-ADC-07(DAR8). The release efficiency of toxin molecules in tumor tissue/tumor microenvironment was evaluated by comparing tumor tissue homogenate results with plasma stability results.

### Groups:

**Table 37**

| | Test substance | Incubation concentration | System | Analyte | Detection time point |
|---|---|---|---|---|---|
| 1 | B7H3-ADC-07(DAR8) | 100 µg/mL | Tumor tissue homogenates of 22RV1 Balb/c nu nude mouse | A1.9 | |
| 2 | B7H3-ADC-07(DAR8) | 100 µg/mL | Muscle tissue homogenates of Balb/c mouse | A1.9 | 0 h, 4 h, 24 h, 48h |
| 3 | B7H3-ADC-07(DAR8) | 100 µg/mL | Homogenate matrix (Normal saline) | A1.9 | |

### Experimental steps:

### 1. Preparation of normal saline (0.85%)

0.85 g sodium chloride added 100 ml of distilled water, that is, 85 mg sodium chloride was added into 10 ml of water.

### 2. Preparation of B7H3-ADC-07

100 µL of B7H3-ADC-07 (DAR8) stock solution was diluted at a ratio of 1:4, to obtain a working solution at a concentration of 5 mg/ml, namely 5 µg/µl.

### 3. Dilution of tissue homogenate

1) The original homogenate was 1:4, which was diluted with normal saline at a ratio of 1:2 for use (200 µL of homogenate + 400 µL of normal saline).
2) After a further 1:9 dilution, the protein concentration was determined, the muscle homogenate was 0.8 mg/ml, and the tumor homogenate was 1.2 mg/ml. This step is only for detection and not for subsequent experiments.
3) Adjusting the protein concentration in the tumor homogenate to be the same as that of the muscle homogenate: the tumor homogenate obtained in 1) was further diluted at a ratio of 2:1 (600 µL of homogenate + 300 µL of normal saline), and then the protein concentration of the homogenate in the final incubation system were both 8 mg/ml, the muscle was diluted by 15-fold, and the tumor was diluted by 22.5-fold.
4. Incubation system 200 µL,

**Table 38**

| | Test substance | Incubation concentration | System | Calculation |
|---|---|---|---|---|
| 1 | B7H3-ADC-07(DAR8) 5 µg/µl | 100 µg/mL | B7H3-ADC-07(DAR8) work solution (4 µL) was added with 196 µL of homogenate or Normal saline | 5 µg/µl×4µL÷0.2ml |

5. Incubating at 37 °C, and then collecting 30 µL and adding 300 µL of precipitant at 0 h, 4 h, 24 h and 48h.

### Results and discussion:

The results are shown in Figures 19A and 19B. From the release of the toxin molecule A1.9, B7H3-ADC-07(DAR8) could release a large amount of A1.9 in the homogenate of tumor tissue, but there was no obvious release of A1.9 in the homogenate of normal tissue. It was suggested that the toxin linker of B7H3-ADC-07(DAR8) has the characteristics of tumor tissue-specific release of toxin molecules.

Thus, the ADC of the present disclosure has a broader therapeutic index and therapeutic effect. Although specific embodiments of the present disclosure had been described in detail, persons skilled in the art would appreciate that in light of all the teachings that had been published, various modifications and changes in details can be made and they all will be within the scope of the present disclosure. The claimed scope of the present disclosure was given by the appended claims and any equivalents thereof.

The following numbered clauses contain further statements of various aspects of the present invention:-
1. A ligand drug conjugate of formula XV, 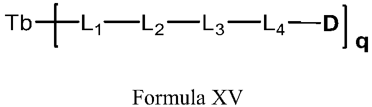
   or a stereoisomer of said ligand drug conjugate, a prodrug thereof, a pharmaceutically acceptable salt thereof, or a pharmaceutically acceptable solvate thereof,
   wherein:
      Tb is a ligand or a targeting moiety that binds to a target;
      q is a drug-to-ligand coupling ratio;
      D is a bioactive molecule fragment;
      L₁ is an extension unit;
      L₂ is absent or a linking unit;
      L₃ is selected from an amino acid residue or short peptides consisting of 2-10 amino acid residues;
      L₄ is absent or present, and when L₄ is present, L₄ is selected from 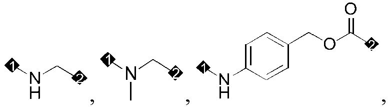 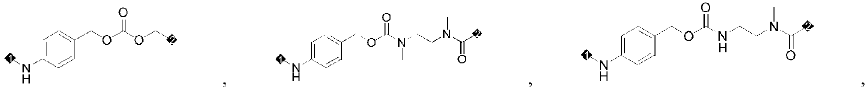 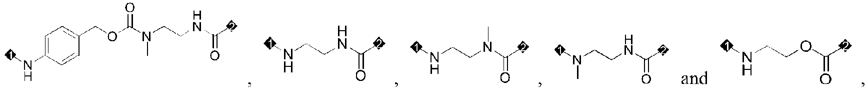 wherein position 1 is attached to L₃ and position 2 is attached to D.
2. The ligand drug conjugate of clause 1, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) Tb is an antibody or antigen-binding fragment thereof;
   (2) q is selected from any numerical value between 0.1 and 16.0;
   (3) D is a bioactive molecular fragment with antitumor bioactivity;
   (4) L₁ is selected from: 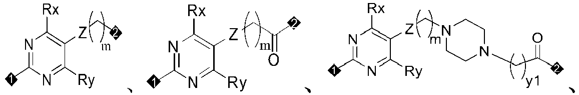 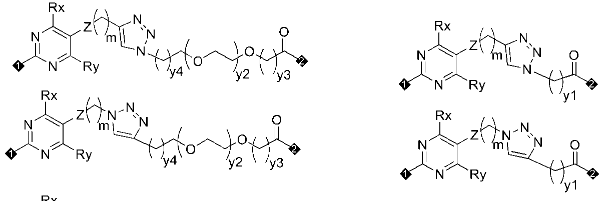 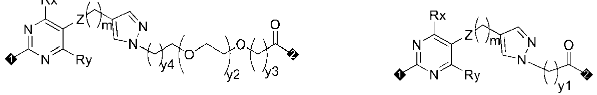 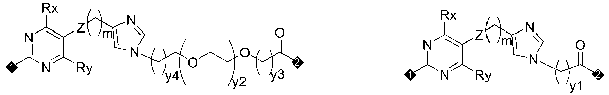 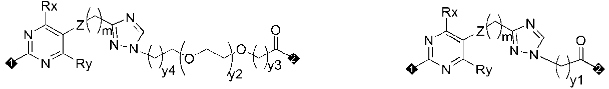 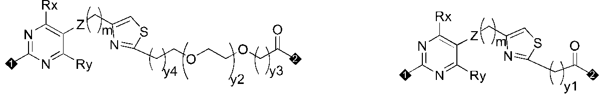 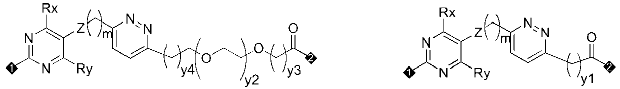 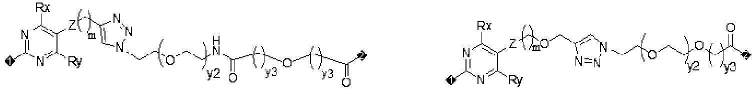 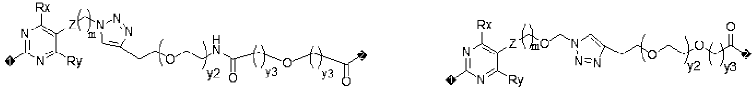 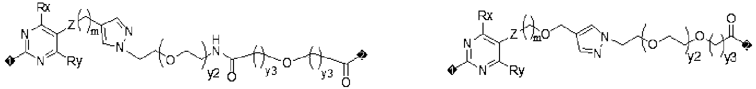 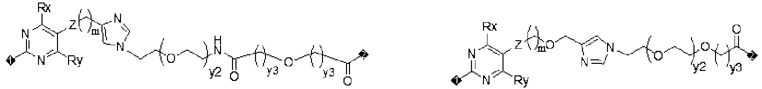 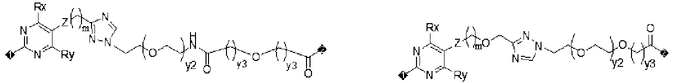 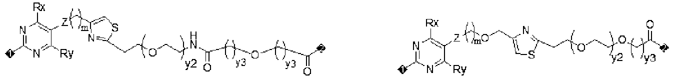 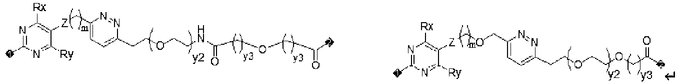 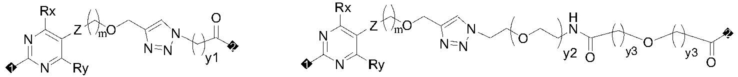 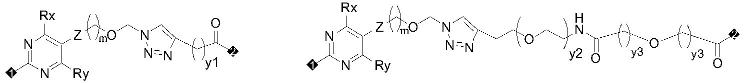 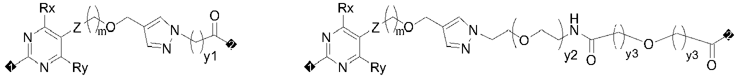 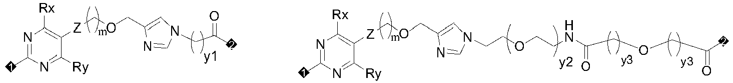 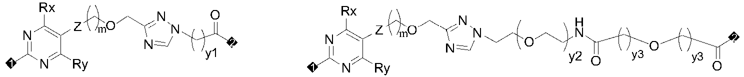 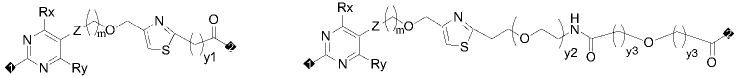 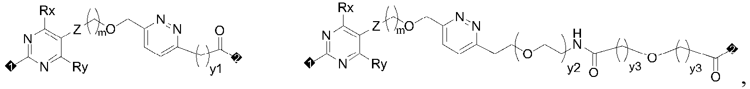
      each Z is independently selected from a direct bond, a carbon-carbon triple bond, a carbon-carbon double bond, C6-10 aryl, 5-10 membered heteroaryl, amido, sulfonamido, imino, and CF₂;
      Rx and Ry are each independently selected from H and C1-4 alkyl;
      each m is independently selected from 0, 1, 2, 3, 4, 5 and 6;
      y1, y2, y3, and y4 are each independently selected from any integer between 0 and 20;
      position 1 is attached to Tb via an S atom, and position 2 is attached to L₂ or L₃;
   (5) L₂ is absent or present, and when L₂ is present, L₂ is selected from: 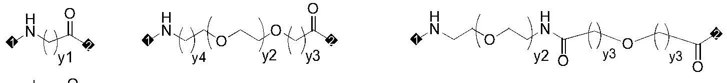 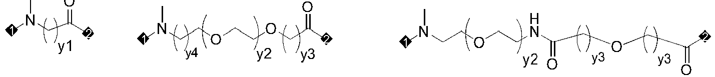 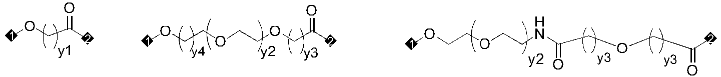 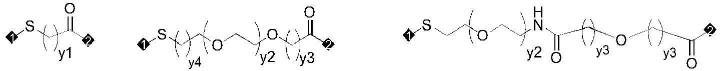 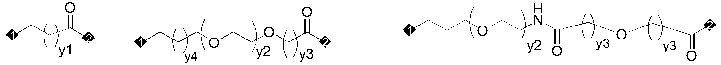 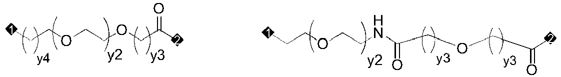 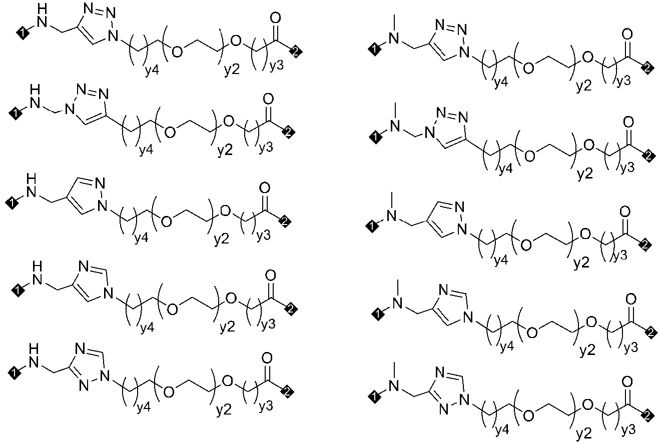 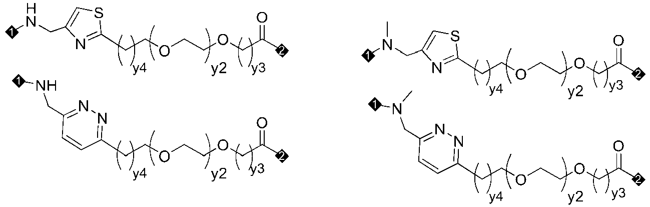 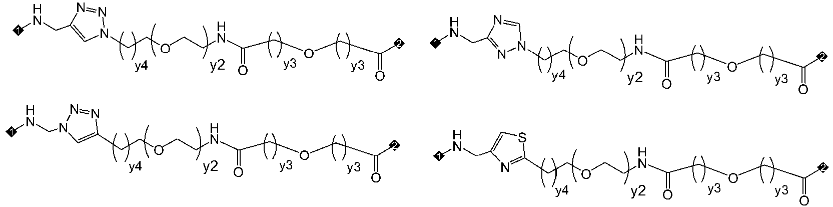 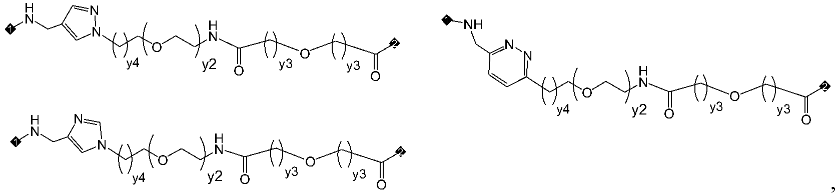 y1, y2, y3, and y4 are each independently selected from any integer between 0 and 20, position 1 is attached to L₁, position 2 is attached to L₃;
   (6) L₃ is selected from an amino acid residue or short peptides consisting of 2-10 amino acid residues; the amino acid residues are selected from natural amino acid residues, non-natural amino acid residues, or selected from amino acid residues represented by AA¹ or stereoisomer thereof;
      the structure of the amino acid residues represented by AA¹ is shown below, 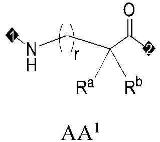
      wherein: R^{a} and R^{b} are each independently selected from H, 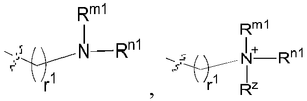 , and 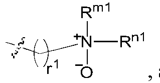 and R^{a} and R^{b} are not both H;
      or, R^{a} and R^{b} together with the carbon atom to which they are both attached form a 4-10 membered heterocyclic ring, said 4-10 membered heterocyclic ring is optionally substituted with one or more R⁰;
      r, r¹ are each independently selected from any integer from 0 to 20;
      R^{m1}, Rⁿ¹ are each independently selected from H, C1-6 alkyl, C3-6 cycloalkyl and-COOR^{x1};
      R^{x1} is selected from C1-6 alkyl;
      or, R^{m1} and Rⁿ¹ together with the nitrogen atom to which they are both attached form a 4-10 membered heterocyclic ring, said 4-10 membered heterocyclic ring is optionally substituted with one or more R^{0'};
      R^{z} is selected from C1-6 alkyl;
      R⁰, R^{0'} are each independently selected from C1-6 alkyl, C3-6 cycloallcyl, -NR^{m2}Rⁿ² and 4-10 membered heterocyclyl optionally substituted with C1-6 alkyl;
      R^{m2}, Rⁿ² are each independently selected from H and C1-6 alkyl;
   (7) L₄ is absent or present, and when L₄ is present, L₄ is selected from 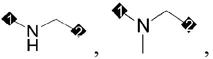 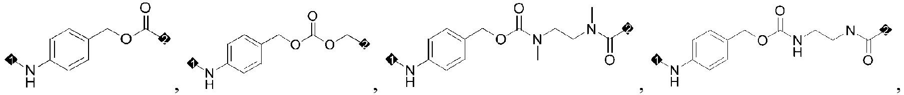 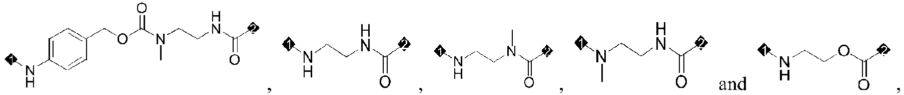 position 1 is attached to L₃ and position 2 is attached to D.
3. The ligand drug conjugate of clause 2, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) Tb is an antibody or antigen-binding fragment thereof that meets one or more of the following conditions:
      (i) The antibody or antigen binding fragment thereof comprises a Fab, Fab', F(ab')2, Fd, Fv, dAb, complementarity determining region fragment, non-human antibody, humanized antibody, chimeric antibody, fully human antibody, probody, monoclonal antibody, bispecific antibody, or multi-specific antibody;
      (ii) Tb is a monoclonal antibody or antigen-binding fragment thereof;
      (iii) Tb is an antibody or antigen-binding fragment thereof with endocytosis, without endocytosis, or with reduced endocytosis;
      (iv) Tb is an antibody or antigen-binding fragment thereof having the activity of binding to a free antigen in tumor tissue and/or to a tumor cell surface antigen;
      (v) Tb is an antibody or antigen-binding fragment thereof that does not have the activity of binding to a free antigen in tumor tissue and/or to a tumor cell surface antigen;
   (2) q is selected from 0.1, 1, 2, 3, 4, 5, 6, 7, 8, 9, 10, 11, 12 and any numerical value therebetween;
   (3) in the bioactive molecule fragment, the bioactive molecule is a DNA topoisomerase inhibitor or a tubulin inhibitor;
   (4) L₁ is selected from 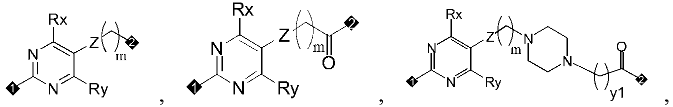 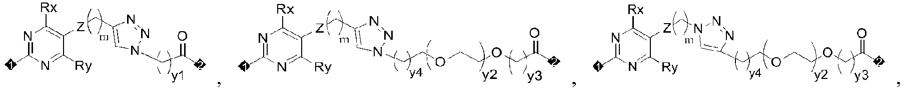 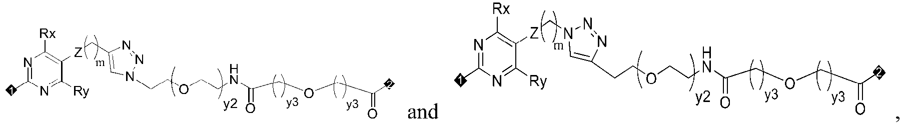 each Z is independently selected from a direct bond, a carbon-carbon triple bond, a carbon-carbon double bond, C6-10 aryl, 5-10 membered heteroaryl, and amido; Rx, Ry are each independently selected from H and C1-4 alkyl; each m is independently selected from 0, 1, 2, 3, 4, 5 and 6; y1 is selected from any integer between 1 and 6; each y2 is independently selected from any integer between 0 and 15; each y3 is independently selected from 1, 2, and 3; each y4 is independently selected from 0 and 1; position 1 is attached to Tb via an S atom, and position 2 is attached to L₂ or L₃;
   (5) L₂ is absent or present, and when L₂ is present, L₂ is selected from 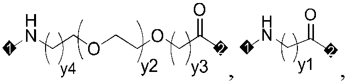 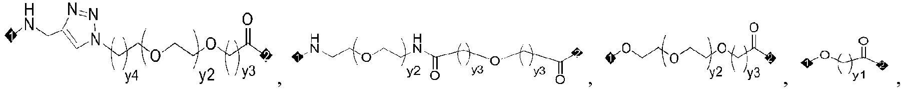 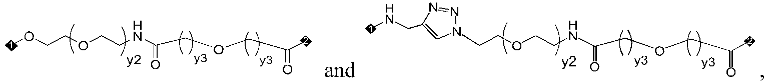 y1 is selected from any integer between 1 and 6; each y2 is independently selected from any integer between 0 and 10; each y3 is independently selected from 1 and 2; each y4 is independently selected from 0 and 1; position 1 is attached to L₁ and position 2 is attached to L₃;
   (6) L₃ is selected from amino acid residues Val, D-Val, Cit, Phe, Lys, Lys (Ac), Leu, Gly, Ala, Asn, Asp, Arg, and AA¹, or short peptides consisting of 2-10 amino acid residues selected from Val, Cit, Phe, Lys, D-Val, Leu, Gly, Ala, Asn, Asp, and AA¹;
   (7) in the amino acid residues of AA¹, wherein one of R^{a} and R^{b} is H, and the other is selected from 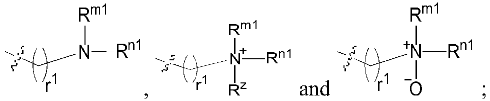 or, in the amino acid residues of AA¹, R^{a} and R^{b} together with the carbon atom to which they are both attached form a 5-6 membered heterocyclic ring substituted with R⁰;
   (8) in the amino acid residues of AA¹, r and r¹ are each independently selected from 0, 1, 2, 3, 4 and 5;
   (9) In the amino acid residues of AA¹, R^{m1} and Rⁿ¹ are each independently selected from H, methyl, ethyl, n-propyl, n-butyl, -COOCH3, -COOCH2CH3, -COOCH2CH2CH3, -COOCH(CH3)2, -COOC(CH3)3 and-COOCH2CH2CH2CH3; or R^{m1} and Rⁿ¹ together with the nitrogen atom to which they are both attached form a 5-6 membered heterocyclic ring substituted with R^{0'};
   (10) in the amino acid residues of AA¹, R^{z} is methyl;
   (11) in the amino acid residues of AA¹, R⁰ and R^{0'} are each independently selected from C1-6 alkyl, -NR^{m2}Rⁿ² and 5-6 membered heterocyclyl optionally substituted with C1-6 alkyl;
   (12) L₄ is absent or present, and when L₄ is present, L₄ is selected from 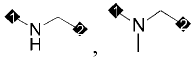 and 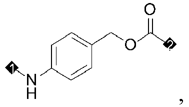 wherein position 1 is attached to L₃ and position 2 is attached to D.
4. The ligand drug conjugate of clause 3, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) Tb is an anti-B7H3 antibody or antigen-binding fragment thereof, an anti-Trop-2 antibody or antigen-binding fragment thereof, an anti-Her2 antibody or antigen-binding fragment thereof, an anti-Her3 antibody or antigen-binding fragment thereof, an anti-EGFR antibody or antigen-binding fragment thereof, or an IgG1 isotype antibody anti-chicken lysozyme antibody;
   (2) q is selected from 0.1, 1, 2, 3, 4, 5, 6, 7, 8 and any numerical value therebetween;
   (3) when the bioactive molecule in the bioactive molecule fragment is a DNA topoisomerase inhibitor, the DNA topoisomerase inhibitor is camptothecin-type bioactive molecule;
   (4) when the bioactive molecule in the bioactive molecule fragment is a tubulin inhibitor, the tubulin inhibitor is an MMAFs tubulin inhibitor or an MMAEs tubulin inhibitor;
   (5) in L₁, each Z is independently selected from a direct bond, a carbon-carbon triple bond, and a carbon-carbon double bond;
   (6) in L₁, y1 is 4, 5 or 6;
   (7) in L₁, each y2 is independently selected from any integer between 6 and10;
   (8) L₂ is absent or present, and when L₂ is present, L₂ is selected from 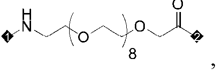 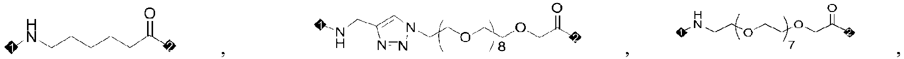 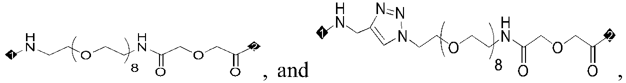 wherein position 1 is attached to L₁ and position 2 is attached to L₃,
   (9) L₃ is selected from Val, Cit, Phe, Lys, D-Val, Leu, Gly, Ala, Asn, AA¹, Val-Cit, Cit-Val, Cit-Ala, Val-Ala, Lys-Val, Val-Lys(Ac), Phe-Lys, Phe-Lys(Ac), Ala-Ala, Val-AA¹, Ala-AA¹, Gly-AA¹, AA¹-Gly, Ala-Ala-Ala, Ala-Ala-Asn, Ala-Ala-Asp, Val-AA¹-Gly, Ala-AA¹-Gly, Gly-AA¹-Gly, Lys-Ala-Ala-Asn, Lys-Ala-Ala-Asp, Gly-Phe-Gly, Gly-Gly-Phe-Gly, D-Val-Leu-Lys, Gly-Gly-Arg, Ala-Ala-Asn, Gly-Gly-Phe, Val-Lys-Gly, Val-Lys-Gly-Gly, Val-Lys, and Lys-Ala-Asn;
   (10) in the amino acid residues represented by AA¹, one of R^{a} and R^{b} is H, the other is selected from 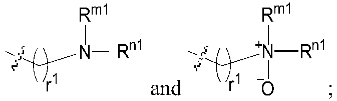 or R^{a} and R^{b} together with the carbon atom to which they are both attached form a 5-6 membered heterocyclic ring substituted with R⁰, preferably a piperidine ring or a piperazine ring substituted with R⁰;
   (11) in the amino acid residues of AA¹, r and r¹ are each independently selected from 0 and 4;
   (12) in the amino acid residues of AA¹, R^{m1} and Rⁿ¹ are each independently selected from H, C1-6 alkyl, C3-6 cycloalkyl and t-butoxycarbonyl; or R^{m1} and Rⁿ¹ together with the nitrogen atom to which they are both attached form a piperidine or piperazine ring substituted with R^{0'};
   (13) in the amino acid residues of AA¹, R⁰ is selected from C1-6 alkyl and 5-6 membered heterocyclyl substituted with C1-6 alkyl, said 5-6 membered heterocyclyl is selected from piperidinyl and piperazinyl;
   (14) in the amino acid residues of AA¹, R^{0'} is selected from C1-6 alkyl and-NR^{m2}Rⁿ²;
   (15) in the amino acid residues of AA¹, R^{m2} and Rⁿ² are methyl;
   (16) L₄ is absent or present, and when L₄ is present, L₄ is 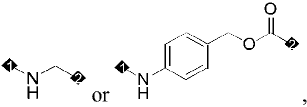 wherein position 1 is attached to L₃ and position 2 is attached to D.
5. The ligand drug conjugate according to any one of clauses 1 to 4, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) q is selected from 2, 3, 4, 5, 6, 7, 8 and any numerical value therebetween;
   (2) Tb is any of the following:
      (i) Tb is an anti-Her2 antibody or antigen-binding fragment thereof, said anti-Her2 antibody is anbenitamab, coprelotamab, disitamab, gancotamab, margetuximab, pertuzumab, timigutuzumab, zanidatamab, Trastuzumab, Pertuzumab, or an antigen-binding fragment thereof;
      (ii) Tb is an anti-Trop-2 antibody or an antigen-binding fragment thereof, and the anti-Trop-2 antibody is datopotamab, Sacituzumab or an antigen-binding fragment thereof; or
      (iii) Tb is an anti-EGFR antibody or antigen-binding fragment thereof, the anti-EGFR antibody is demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, Cetuximab, or an antigen-binding fragment thereof; or
      (iv) Tb is an anti-B7H3 antibody or antigen-binding fragment thereof, wherein the anti-B7H3 antibody is enoblituzumab, mirzotamab, omburtamab, 1D1-01, 2E3-02 antibody or antigen-binding fragment thereof; or
      (v) Tb is an anti-B7H3 antibody or antigen-binding fragment thereof, wherein the anti-B7H3 antibody or antigen-binding fragment thereof comprises the Complementarity Determining Regions (CDRs) as shown below, wherein the CDRs are defined by the Kabat numbering system:
         (a) HCDR1 consisting of the sequence of SEQ ID NO: 7, HCDR2 consisting of the sequence of SEQ ID NO: 8, HCDR3 consisting of the sequence of SEQ ID NO: 9; and/or,
         LCDR1 consisting of the sequence of SEQ ID NO: 17, LCDR2 consisting of the sequence of SEQ ID NO: 18, and LCDR3 consisting of the sequence of SEQ ID NO: 19;
         (b) HCDR1 consisting of the sequence of SEQ ID NO: 27, HCDR2 consisting of the sequence of SEQ ID NO: 28, HCDR3 consisting of the sequence of SEQ ID NO: 29; and/or,
         LCDR1 consisting of the sequence of SEQ ID NO: 37, LCDR2 consisting of the sequence of SEQ ID NO: 38, and LCDR3 consisting of the sequence of SEQ ID NO: 39;
         (c) HCDR1 consisting of the sequence of SEQ ID NO: 7, HCDR2 consisting of the sequence of SEQ ID NO: 8, HCDR3 consisting of the sequence of SEQ ID NO: 9; and/or
         LCDR1 consisting of the sequence of SEQ ID NO: 37, LCDR2 consisting of the sequence of SEQ ID NO: 38, and LCDR3 consisting of the sequence of SEQ ID NO: 39;
         or (d) HCDR1 consisting of the sequence of SEQ ID NO: 27, HCDR2 consisting of the sequence of SEQ ID NO: 28, HCDR3 consisting of the sequence of SEQ ID NO: 29; and/or
         LCDR1 consisting of the sequence of SEQ ID NO: 17, LCDR2 consisting of the sequence of SEQ ID NO: 18, and LCDR3 consisting of the sequence of SEQ ID NO: 19; or
      (vi) Tb is an anti-Her3 antibody or antigen binding fragment thereof, wherein said anti-Her3 antibody is barecetamab, duligotuzumab, elgemtumab, lumretuzumab, patritumab, seribantumab, 202-2-1 or antigen binding fragment thereof, or
      (vii) Tb is an anti-Her3 antibody or antigen-binding fragment thereof, wherein the anti-Her3 antibody or antigen-binding fragment thereof comprises Complementarity Determining Regions (CDRs) as shown below, wherein the CDRs are defined by the Kabat numbering system:
         HCDR1 consisting of the sequence of SEQ ID NO: 53, HCDR2 consisting of the sequence of SEQ ID NO: 54, HCDR3 consisting of the sequence of SEQ ID NO: 55; and/or,
         LCDR1 consisting of the sequence of SEQ ID NO: 59, LCDR2 consisting of the sequence of SEQ ID NO: 60, LCDR3 consisting of the sequence of SEQ ID NO: 21;
      (viii) an antibody or antigen-binding fragment thereof, which has tumor cell endocytosis activity and has the activity of binding to free antigen in tumor tissue and/or to tumor cell surface antigen;
      (ix) an antibody or an antigen-binding fragment thereof, which has weak or no tumor cells endocytosis activity and has the activity of binding to free antigen in tumor tissues and/or tumor cell surface antigen;
      (x) an antibody or antigen binding fragment thereof, which has weak or no tumor cells endocytosis activity and has no activity of binding to free antigen in tumor tissues and/or tumor cell surface antigen; for example, Tb is an anti-chicken lysozyme human IgG1 isotype antibody;
      (xi) an antibody or antigen-binding fragment thereof that binds to a non-endocytosed antigen; such as ALCAM/CD 166;
   (3) L₁ is selected from 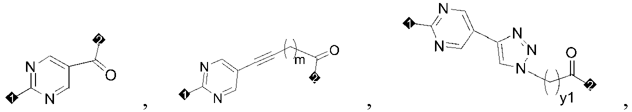 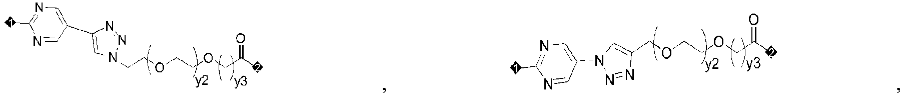 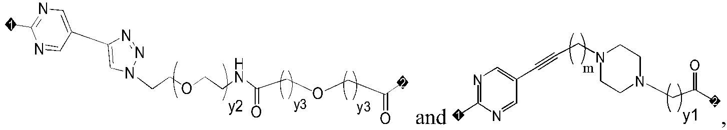 m is selected from 2, 3 and 4, y1 is selected from any integer between 1 and 6, each y2 is independently selected from any integer between 0 and 10, each y3 is independently selected from 1 or 2, position 1 is attached to Tb via an S atom, position 2 is attached to L₂ or L₃;
   (4) L₂ is absent or present, and when L₂ is present, L₂ is selected from 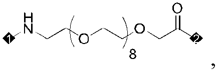 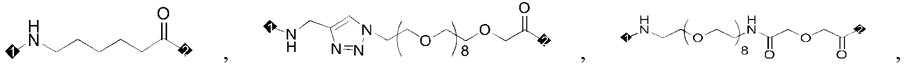 and 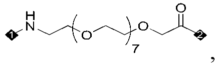 wherein position 1 is attached to L₁ and position 2 is attached to L₃ ;
   (5) L₃ is selected from AA¹, AA¹ -Gly, Val-Cit, Val-AA¹-Gly, AA¹-Ala-Asn, and Gly-Gly-Phe-Gly;
   (6) in the amino acid residues represented by AA¹, R^{a} and R^{b} together with the carbon atom to which they are both attached form a 5-6-membered heterocyclic ring substituted by R⁰, and preferably a piperidine ring substituted by R⁰;
   (7) in the amino acid residues of AA¹, one of r and r¹ is 0, and the other is 4;
   (8) in the amino acid residues of AA¹, R^{m1} and Rⁿ¹ are each independently selected from H and C1-6 alkyl; or, R^{m1} and Rⁿ¹ together with the nitrogen atom to which they are both attached form a piperidine ring substituted with R^{0'};
   (10) in the amino acid residues of AA¹, R⁰ is selected from methyl, ethyl and 5-6 membered heterocyclyl substituted with methyl, said 5-6 membered heterocyclyl is piperidinyl;
   (11) in the amino acid residues of AA¹, R^{0'} is selected from methyl and -NR^{m2}Rⁿ²;
   (12) when the bioactive molecule in the bioactive molecule fragment is a DNA topoisomerase inhibitor and the DNA topoisomerase inhibitor is a camptothecin-type bioactive molecule, the camptothecin-type bioactive molecule is camptothecin, DXD, a substituent-modified camptothecin or a substituent-modified DXD.
6. The ligand drug conjugate according to any one of clauses 1 to 4, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) q is selected from 3, 4, 5, 6, 7, 8 and any numerical value therebetween;
   (2) Tb is any of the following embodiments:
      (vi) Tb is an anti-B7H3 antibody or antigen binding fragment thereof, wherein the anti-B7H3 antibody or antigen binding fragment thereof comprises Complementarity Determining Regions (CDRs) as shown below, wherein the CDRs are defined by the Kabat numbering system:
         (a) HCDR1 consisting of the sequence of SEQ ID NO: 7, HCDR2 consisting of the sequence of SEQ ID NO: 8, HCDR3 consisting of the sequence of SEQ ID NO: 9; and/or
         LCDR1 consisting of the sequence of SEQ ID NO: 17, LCDR2 consisting of the sequence of SEQ ID NO: 18, LCDR3 consisting of the sequence of SEQ ID NO: 19;
         (b) HCDR1 consisting of the sequence of SEQ ID NO: 27, HCDR2 consisting of the sequence of SEQ ID NO: 28, HCDR3 consisting of the sequence of SEQ ID NO: 29; and/or,
         LCDR1 consisting of the sequence of SEQ ID NO: 37, LCDR2 consisting of the sequence of SEQ ID NO: 38, LCDR3 consisting of the sequence of SEQ ID NO: 39;
         (c) HCDR1 consisting of the sequence of SEQ ID NO: 7, HCDR2 consisting of the sequence of SEQ ID NO: 8, HCDR3 consisting of the sequence of SEQ ID NO: 9; and/or,
         LCDR1 consisting of the sequence of SEQ ID NO: 37, LCDR2 consisting of the sequence of SEQ ID NO: 38, LCDR3 consisting of the sequence of SEQ ID NO: 39;
         or (d) HCDR1 consisting of the sequence of SEQ ID NO: 27, HCDR2 consisting of the sequence of SEQ ID NO: 28, HCDR3 consisting of the sequence of SEQ ID NO: 29; and/or,
         LCDR1 consisting of the sequence of SEQ ID NO: 17, LCDR2 consisting of the sequence of SEQ ID NO: 18, LCDR3 consisting of the sequence of SEQ ID NO: 19;
         and the anti-B7H3 antibody or antigen-binding fragment thereof comprises a VH comprising a set of heavy chain CDRs in the above (a) - (d) and a VL comprising a set of light chain CDRs in (a) - (d);
      (ii) Tb is an anti-Her3 antibody or antigen binding fragment thereof,
      wherein the anti-Her3 antibody or antigen binding fragment thereof comprises Complementarity Determining Regions (CDRs) as shown below, wherein the CDRs are defined by the Kabat numbering system:
         HCDR1 consisting of the sequence of SEQ ID NO: 53, HCDR2 consisting of the sequence of SEQ ID NO: 54, HCDR3 consisting of the sequence of SEQ ID NO: 55; and/or
         LCDR1 consisting of the sequence of SEQ ID NO: 59, LCDR2 consisting of the sequence of SEQ ID NO: 60, LCDR3 consisting of the sequence of SEQ ID NO: 21;
         the anti-Her3 antibody or antigen-binding fragment thereof comprises a VH comprising the above set of heavy chain CDRs and a VL comprising the above set of light chain CDRs;
   (3) L₁ is selected from 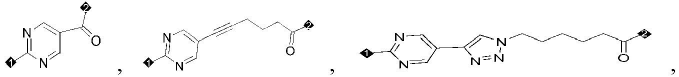 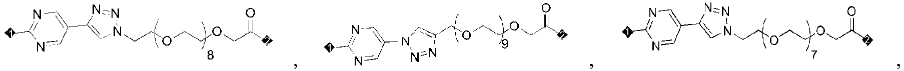 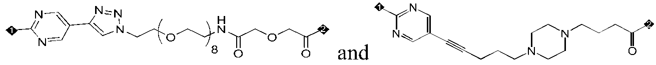 , wherein position 1 is attached to Tb via an S atom, position 2 is attached to L₂ or L₃ ;
   (4) L₂ is absent or present, and when L₂ is present, L₂ is selected from 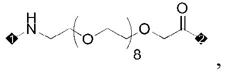 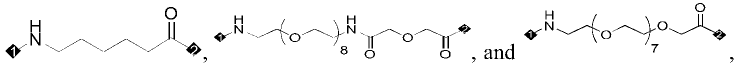 wherein position 1 is attached to L₁ and position 2 is attached to L₃;
   (5) L₃ is selected from AA¹, Val-AA¹-Gly;
   (6) in the amino acid residues of AA¹, R⁰ is selected from methyl, ethyl and 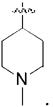
7. The ligand drug conjugate of any one of clauses 1 to 4, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) q is selected from 4, 5, 6, 7, 8 and any numerical value therebetween;
   (2) Tb is any of the following embodiments:
      (i) Tb is an anti-B7H3 antibody or antigen-binding fragment thereof, wherein the anti-B7H3 antibody or antigen-binding fragment thereof comprises: a VH set forth in SEQ ID NO: 3 or 23, and/or a VL set forth in SEQ ID NO: 13 or 33;
      (ii) Tb is an anti-Her3 antibody or antigen-binding fragment thereof, wherein the anti-Her3 antibody or antigen-binding fragment thereof comprises: VH set forth in SEQ ID NO: 49, and/or VL set forth in SEQ ID NO: 50;
   (3) L₁ is selected from 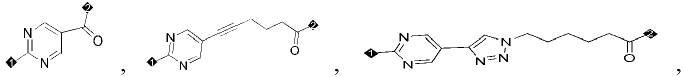 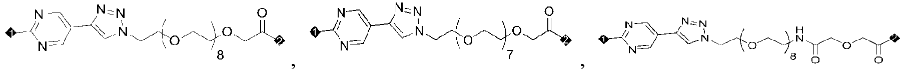 and 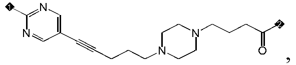 wherein position 1 is attached to Tb via an S atom, position 2 is attached to L₂ or L₃ ;
   (4) L₂ is absent or 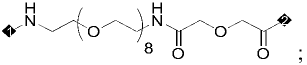
   (5) L₃ is selected from Val-AA¹ -Gly;
   (6) in the amino acid residues represented by AA¹, R^{a} and R^{b} together with the carbon atom to which they are both attached form 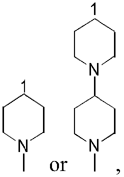 and the carbon atom marked with number 1 is the carbon atom to which R^{a} and R^{b} are both attached;
   (7) for r and r¹, when r is 4 and r¹ is 0, R^{m1} and Rⁿ¹ are each independently selected from H and C1-6 alkyl; when r is 0 and r¹ is 4, R^{m1} and Rⁿ¹ are each independently selected from C1-6 alkyl, or R^{m1} and Rⁿ¹ together with the nitrogen atom to which they are both attached form 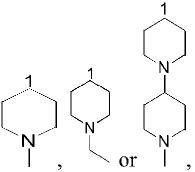 carbon atom marked with number 1 is the carbon atom to which R^{a} and R^{b} are both attached.
8. The ligand drug conjugate according to clause 7, or a stereoisomer of said ligand drug conjugate, a prodrug thereof, a pharmaceutically acceptable salt thereof or a pharmaceutically acceptable solvate thereof,
   (1) q is selected from 6, 7, 8 and any numerical value therebetween;
   (2) Tb is any of the following embodiments:
      (i) Tb is an anti-B7H3 antibody or antigen-binding fragment thereof, wherein the anti-B7H3 antibody or antigen-binding fragment thereof comprises: a VH set forth in SEQ ID NO: 3 or 23, and/or a VL set forth in SEQ ID NO: 13 or 33;
         further comprises:
         (a) a heavy chain constant region (CH) of a human immunoglobulin, or a variant thereof; and/or
         (b) a light chain constant region (CL) of a human immunoglobulin, or a variant thereof,
      (ii) Tb is an anti-Her3 antibody or antigen-binding fragment thereof, wherein the anti-Her3 antibody or antigen-binding fragment thereof comprises: a VH set forth in SEQ ID NO: 49, and/or a VL set forth in SEQ ID NO: 50;
         further comprises:
         (a) a heavy chain constant region (CH) of a human immunoglobulin, or a variant thereof; and/or
         (b) a light chain constant region (CL) of a human immunoglobulin, or a variant thereof;
   (3) For r and r¹, when r is 4 and r¹ is 0, R^{m1} and Rⁿ¹ are each independently selected from H and C1-6 alkyl; when r is 0 and r¹ is 4, R^{m1} and Rⁿ¹ are each independently selected from C2-6 alkyl;
   (4) L₁ is selected from 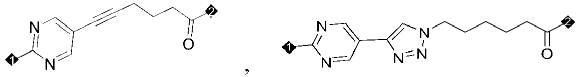 and 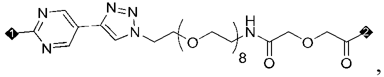 wherein position 1 is attached to Tb via an S atom, position 2 is attached to L₂ or L₃ ; for example, L₁ is selected from 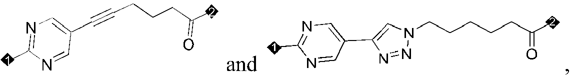 wherein position 1 is attached to Tb via an S atom and position 2 is attached to L₂ or L₃.
9. The ligand drug conjugate according to clause 8, or a stereoisomer of said ligand drug conjugate, a prodrug thereof, a pharmaceutically acceptable salt thereof or a pharmaceutically acceptable solvate thereof, characterized in that:
   (1) Tb is any of the following embodiments:
      (i) Tb is an anti-B7H3 antibody or antigen-binding fragment thereof, wherein the anti-B7H3 antibody or antigen-binding fragment thereof comprises:
         a VH set forth in SEQ ID NO: 3, a CH set forth in SEQ ID NO: 43 or a variant thereof, and/or a VL set forth in SEQ ID NO: 13, a CL set forth in SEQ ID NO: 44 or a variant thereof;
         a VH set forth in SEQ ID NO: 23, a CH set forth in SEQ ID NO: 43 or variant thereof, and/or a VL set forth in SEQ ID NO: 33, a CL set forth in SEQ ID NO: 44 or variant thereof;
         preferably, the anti-B7H3 antibody or antigen binding fragment thereof comprises:
            a heavy chain set forth in SEQ ID NO: 45, and/or a light chain set forth in SEQ ID NO: 46;
            a heavy chain set forth in SEQ ID NO: 47, and/or a light chain set forth in SEQ ID NO: 48;
      (ii) Tb is an anti-Her3 antibody or antigen-binding fragment thereof, wherein the anti-Her3 antibody or antigen-binding fragment thereof comprises:
         a VH set forth in SEQ ID NO: 49, a CH set forth in SEQ ID NO: 43 or variant thereof, and/or a VL set forth in SEQ ID NO: 50, a CL set forth in SEQ ID NO: 44 or variant thereof;
         preferably, the anti-Her3 antibody or antigen binding fragment thereof comprises:
            a heavy chain set forth in SEQ ID NO: 51, and/or a light chain set forth in SEQ ID NO: 52;
   (2) R^{m1} and Rⁿ¹ are each independently selected from C1-6 alkyl, and said C1-6 alkyl is selected from methyl, ethyl and n-propyl; or R^{m1} and Rⁿ¹ are each independently selected from C2-6 alkyl, and the C2-6 alkyl is selected from ethyl and n-propyl.
10. The ligand drug conjugate according to any one of clauses 1 to 9, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) Tb is an antibody or an antigen-binding fragment thereof, and when the antibody or the antigen-binding fragment thereof comprises a single-chain antibody, the single-chain antibody is scFv;
   (2) Tb is an antibody or antigen-binding fragment thereof having endocytic activity;
   (3) the amino acid residue represented by AA¹ is selected from 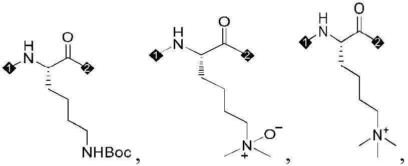 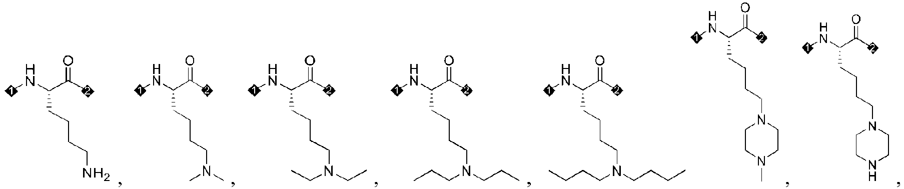 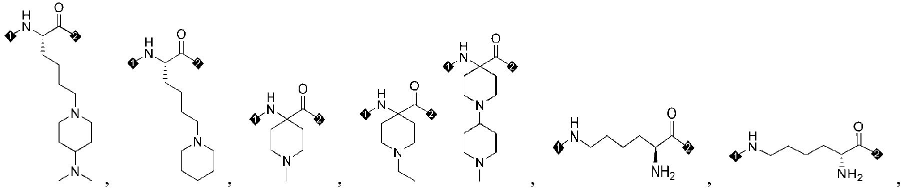 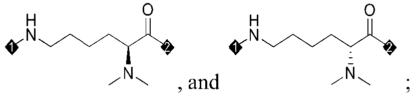 preferably, the amino acid residue represented by AA¹ is selected from 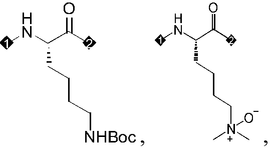 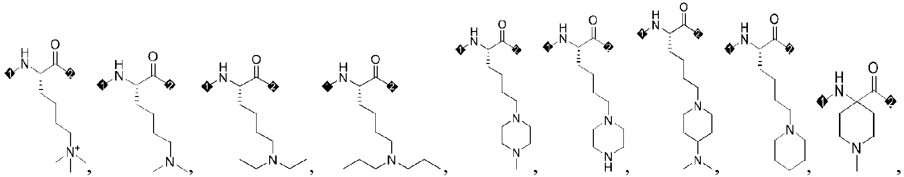 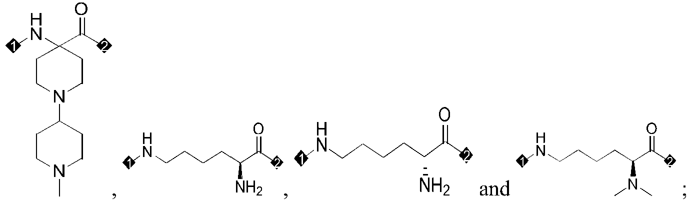 further preferably, the amino acid residue represented by AA¹ is selected from 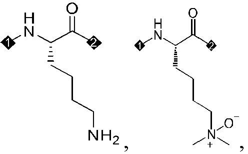 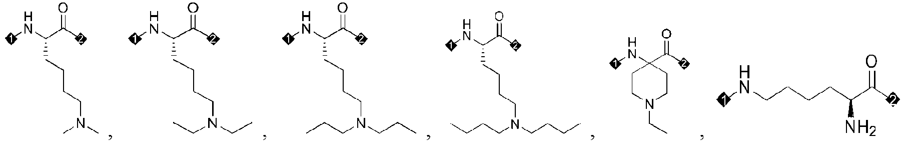 and 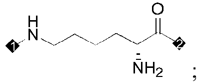 most preferably, the amino acid residue represented by AA¹ is selected from 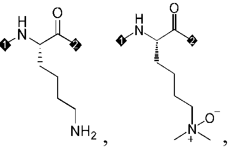 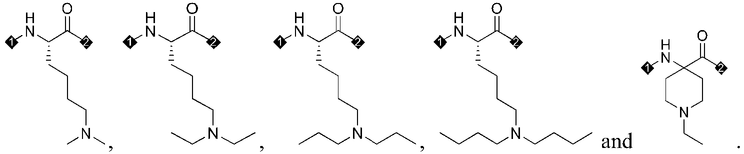
11. The ligand drug conjugate according to any one of clauses 1 to 10, or a stereoisomer of said ligand drug conjugate, a prodrug thereof, a pharmaceutically acceptable salt thereof or a pharmaceutically acceptable solvate thereof, characterized in that
   L₃ is selected from 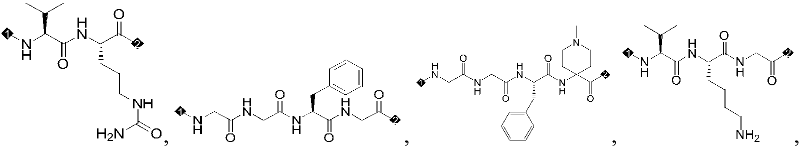 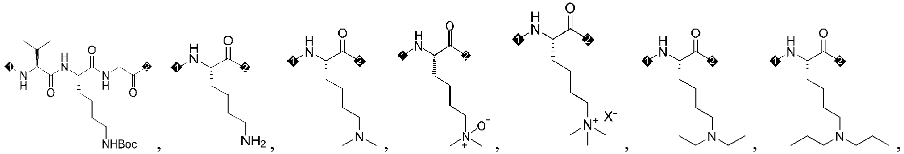 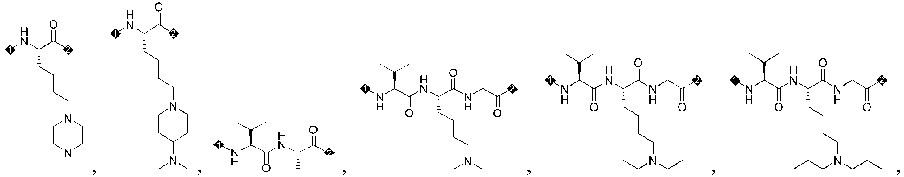 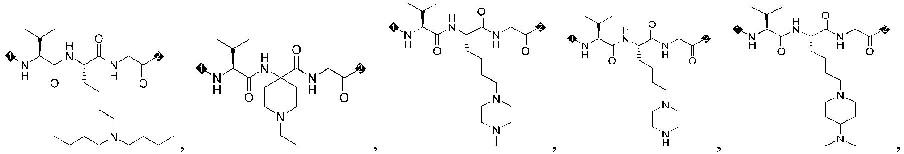 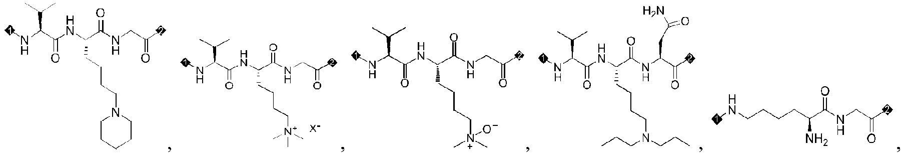 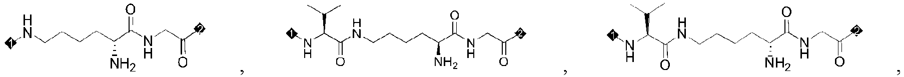 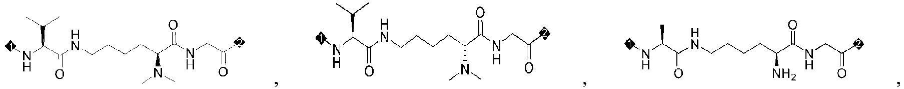 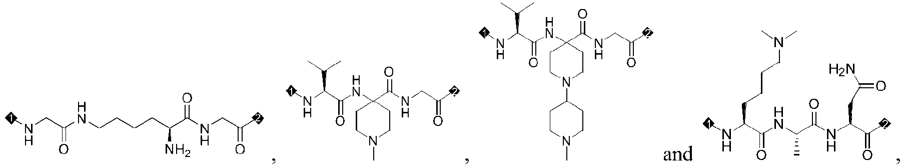 X ⁻ is selected from halide ion, carboxylate ion, sulfate ion, hydrogen sulfate ion and OH⁻, position 1 is attached to L₁ or L₂, position 2 is attached to L₄ or D;
   preferably, L₃ is selected from 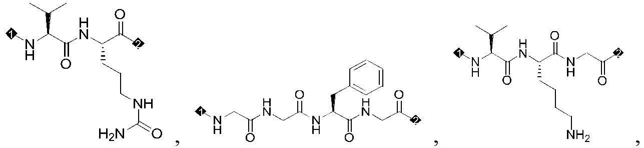 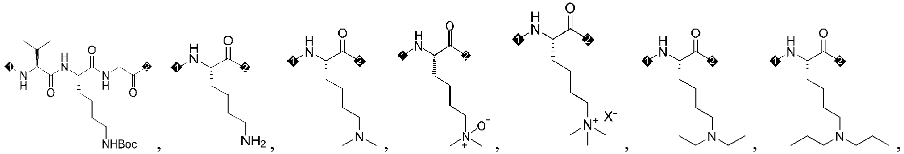 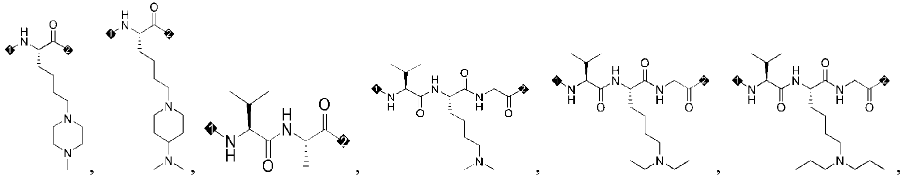 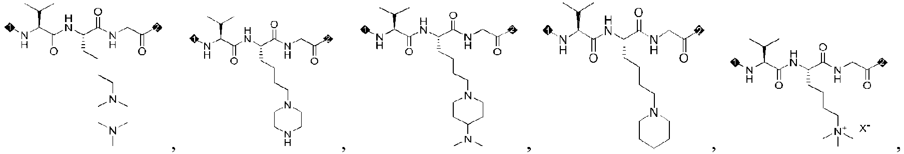 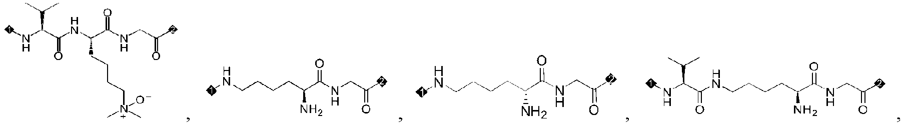 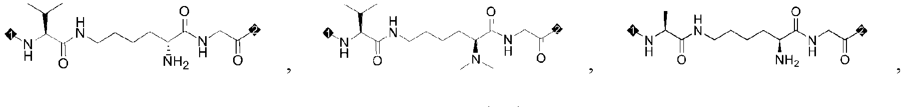 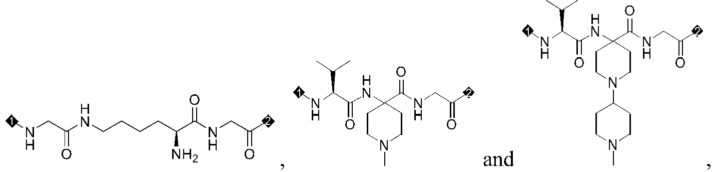 X⁻ is selected from halide ion, carboxylate ion, sulfate ion, hydrogen sulfate ion, and OH⁻, position 1 is attached to L₁ or L₂, position 2 is attached to L₄ or D;
   further preferably, L₃ is selected from 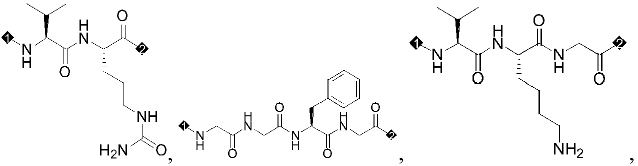 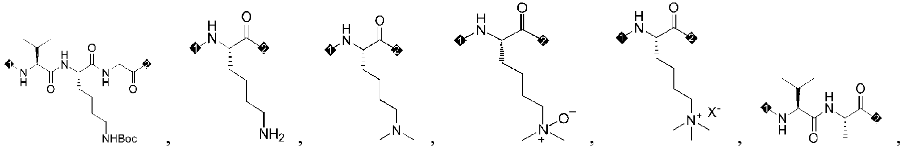 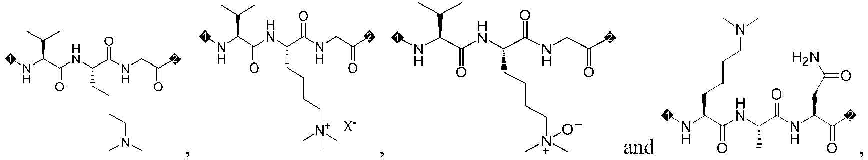 X ⁻ is selected from is halide ion, carboxylate ion, sulfate ion, hydrogen sulfate ion and OH⁻, position 1 is attached to L₁ or L₂, position 2 is attached to L₄ or D;
   also preferably, L₃ is selected from 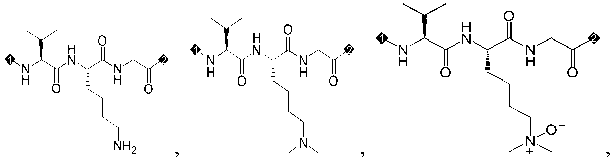 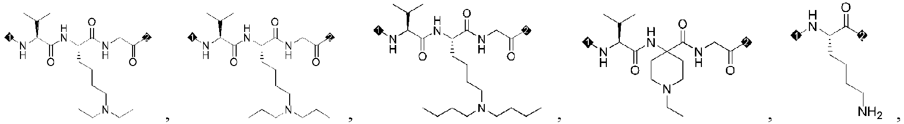 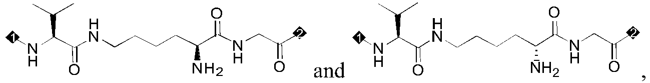 position 1 is attached to L₁ or L₂ and position 2 is attached to L₄ or D;
   most preferably, L₃ is selected from 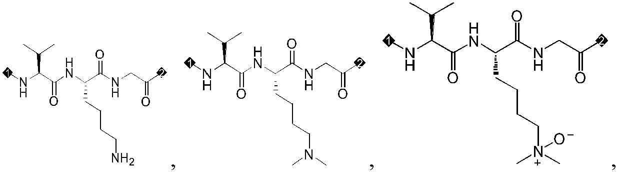 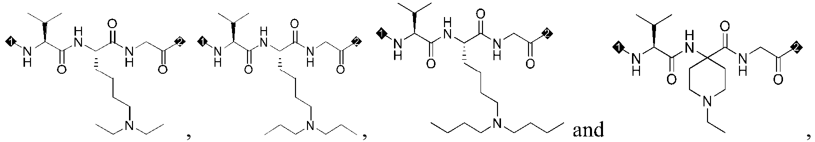 position 1 is attached to L₁ or L₂, position 2 is attached to L₄ or D;
   for example, 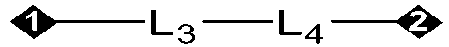 is selected from the following structural fragments: 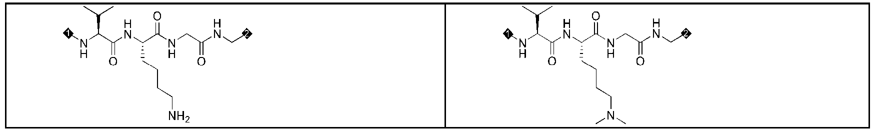 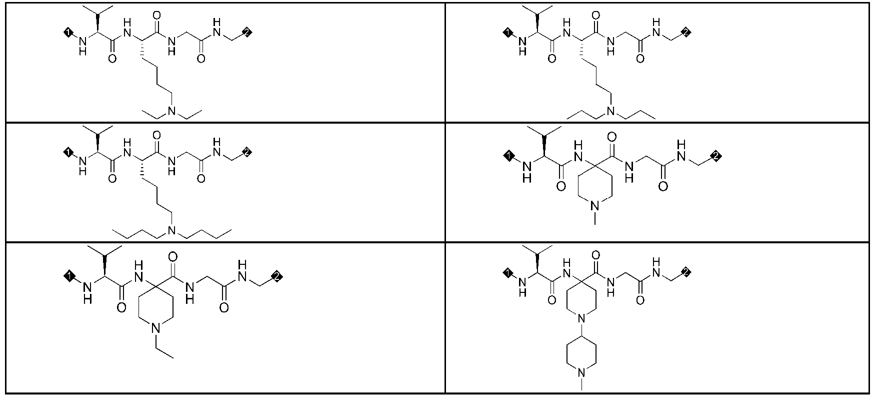
12. The ligand drug conjugate according to any one of clauses 1 to 11, or a stereoisomer of said ligand drug conjugate, a prodrug thereof, a pharmaceutically acceptable salt thereof or a pharmaceutically acceptable solvate thereof, characterized in that
   wherein 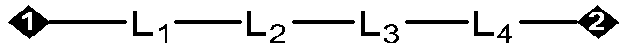 has a structure selected from: 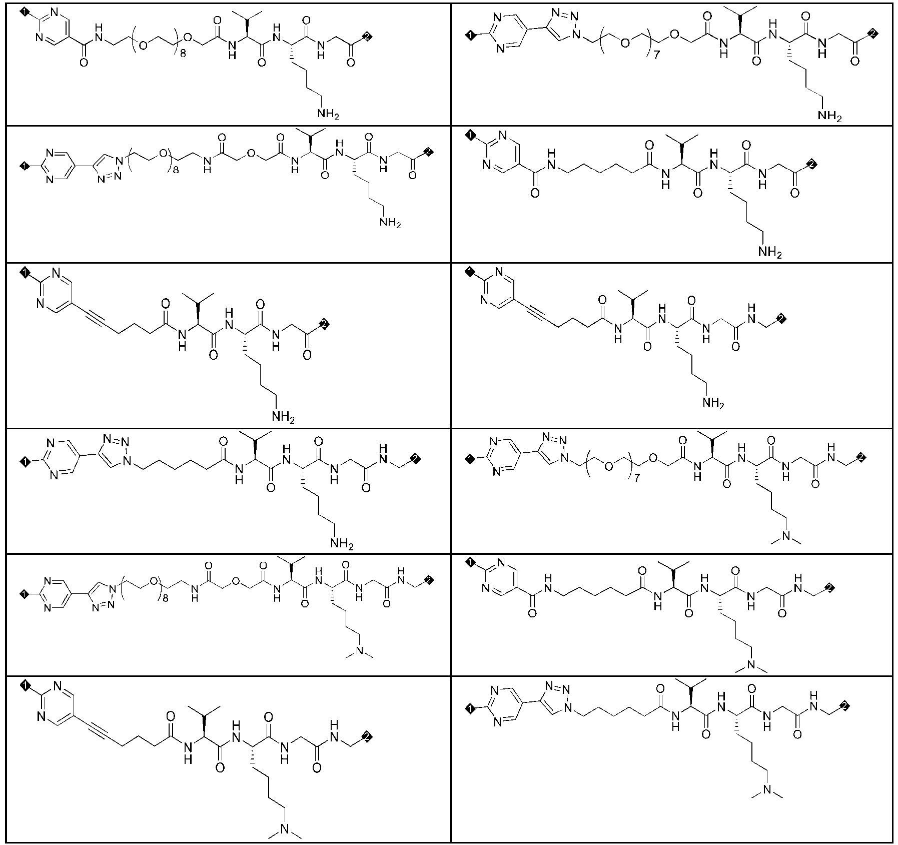 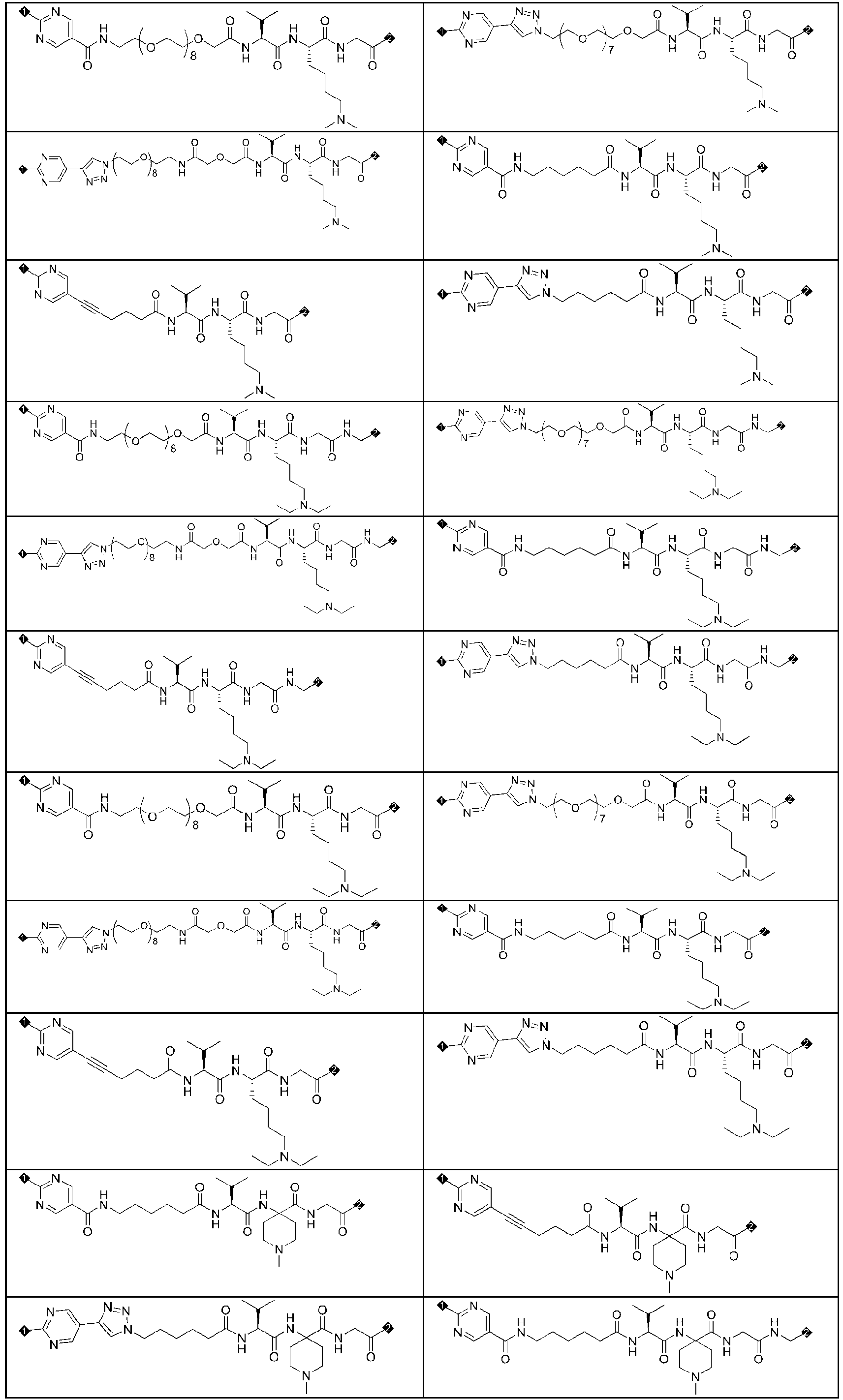 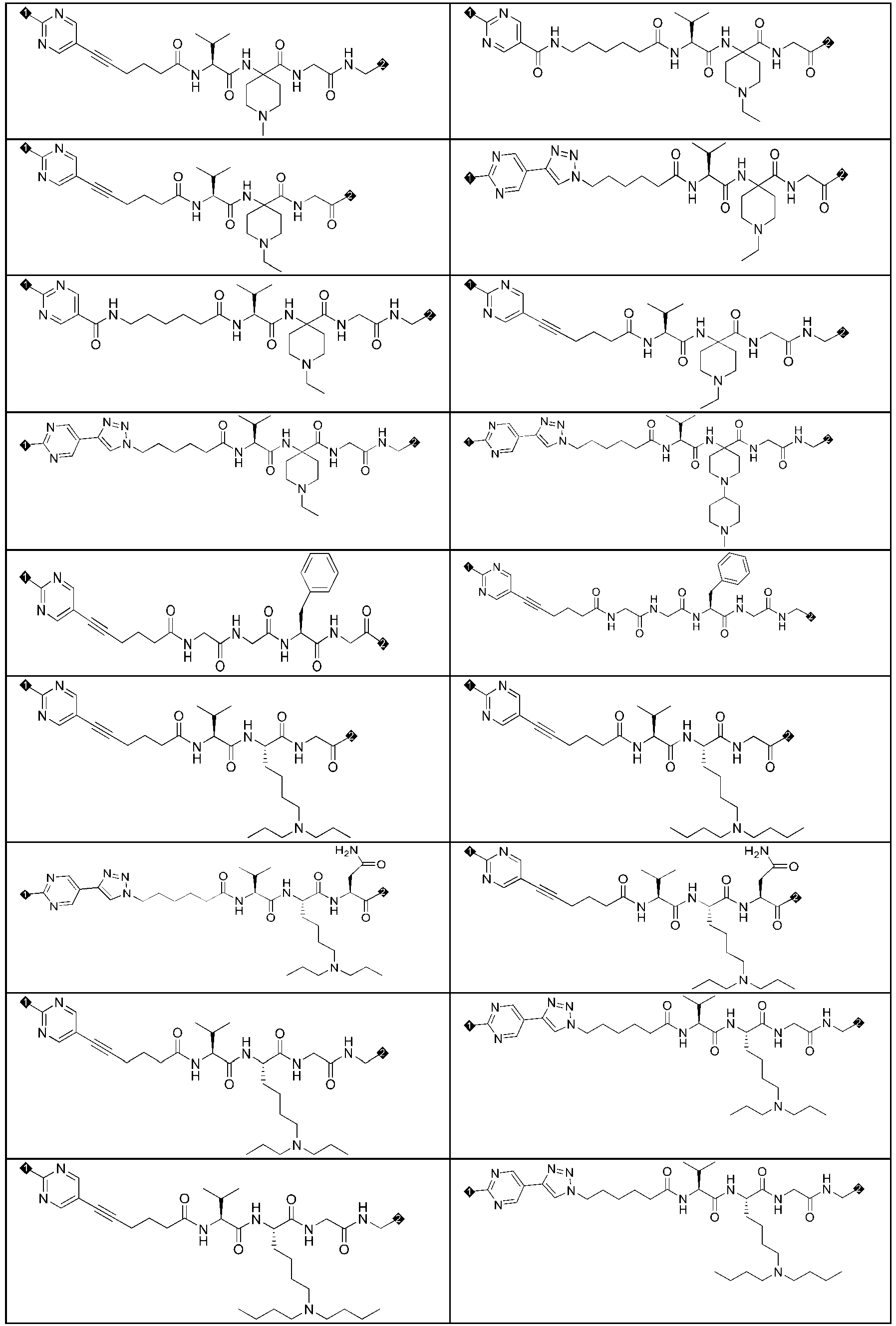
   wherein, position 1 is attached to Tb, and position 2 is attached to D.
13. The ligand drug conjugate according to any one of clauses 1 to 12, or a stereoisomer of the ligand drug conjugate, a prodrug thereof, a pharmaceutically acceptable salt thereof or a pharmaceutically acceptable solvate thereof, characterized in that
   wherein the ligand drug conjugate has a structure represented by formula I: 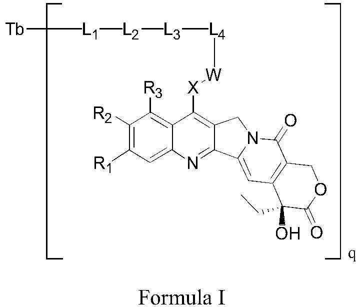
   wherein: Tb, L₁, L₂, L₃, L₄ and q are as defined in any one of clauses 1 to 12;
   R₁, R₂ are each independently selected from H, halogen, -OH, optionally substituted C1-6 alkyl, and optionally substituted C1-6 alkoxy, or,
   R₁ and R₂ together with the carbon atoms to which they are attached form a 5-7 membered carbocyclic ring or a 5-7 membered heterocyclic ring containing one or more O, S, N, carbonyl, sulfoxide group or sulfone group or any combination thereof;
   R₃ is selected from H, halogen, -OH, - NH₂, optionally substituted C1-6 alkyl and optionally substituted C1-6 alkoxy, or,
   R₃ and X, together with the carbon atoms to which they are attached, form a 5-7 membered carbocyclic ring or a 5-7 membered heterocyclic ring containing one or more O, S, N, carbonyl, sulfoxide or sulfone groups or any combination thereof, or,
   R₃ and R₂, together with the carbon atoms to which they are attached, form a 5-7 membered carbocyclic ring or a 5-7 membered heterocyclic ring containing one or more O, S, N, carbonyl, sulfoxide group or sulfone group or any combination thereof;
   W is absent or present, and when W is present, W is selected from-O-, -S-, -NR₄ -, 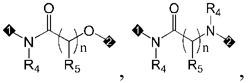 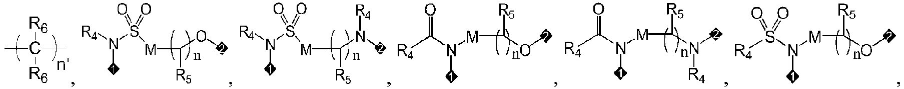 and 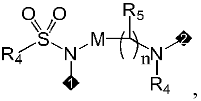 position 1 is attached to X, and position 2 is attached to L₄ or L₃ ;
   X is selected from a direct bond, optionally substituted -O-(CH₂)ₙ₃-, -NR₄-(CH₂)ₙ₃-, -S-(CH₂)ₙ₃-, carbonyl-(CH2)ₙ₃-, -SO₂-(CH2)ₙ₃-, -(CH₂)ₙ₁-, 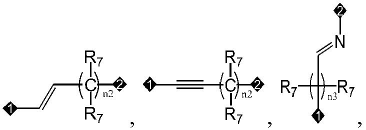 C3-6 cycloalkyl, C6-10 aryl, 5-10 membered heteroaryl and 4-10 membered heterocyclyl, position 1 is attached to the parent ring and position 2 is attached to W or L₄ ; the substituents are selected from one or more C1-4 alkyl groups, C3-6 cycloalkyl groups, or said more C1-4 alkyl groups together with the carbon atom to which they are both attached form a C3-6 cycloallcyl group;
   each M is independently selected from a direct bond and-CR^{5a}R^{5b}-;
   R₄, R₅, R^{5a}, R^{5b}, R₆, R₇ are each independently selected from H, optionally substituted C1-4 alkyl, optionally substituted C1-4 alkoxy, and optionally substituted C3-6 cycloallcyl;
   n, n', n1, n2, n3 are each independently selected from any integer between 0 and 6;
   position 1 is attached to the parent core of camptothecin, and 2 position is attached to W or L₄.
14. The ligand drug conjugate according to clause 13, or a stereoisomer of said ligand drug conjugate, a prodrug thereof, a pharmaceutically acceptable salt thereof, or a pharmaceutically acceptable solvate thereof, characterized in that one or more of the following conditions are met:
   (1) R₁ and R₂ are each independently selected from H, halogen, C1-4 alkyl; or, R₁ and R₂, together with the carbon atoms to which they are attached, form a 5-6 membered heterocyclic ring containing 1, 2 or 3 O, S or N or any combination thereof;
   (2) R₃ is selected from H, C1-4 alkyl; or, R₃ and X, together with the carbon atoms to which they are attached, form a 5-6 membered carbocyclic ring;
   (3) W is absent or present, and when W is present, W is selected from -O-, -S-, -NR₄ -, 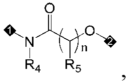 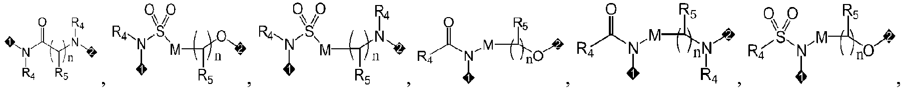 and 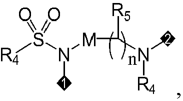 position 1 is attached to X, and position 2 is attached to L₄ or L₃ ;
   (4) X is selected from optionally substituted -(CH₂)ₙ₁-, 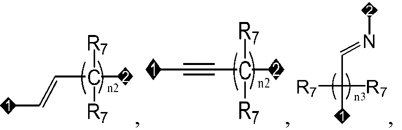 C6-10 aryl, 5-10 membered heteroaryl and 4-10 membered heterocyclyl, at position 1 is attached to the parent ring and position 2 is attached to W or L₄ ; the substituents are selected from 1 or 2 C1-4 alkyl groups, or 2 C1-4 alkyl groups together with the carbon atom to which they are both attached form a C3-6 cycloalkyl group;
   (5) R₄, R₅ are each independently selected from H, C1-4 alkyl and C3-6 cycloalkyl;
   (6) R^{5a}, R^{5b} are each independently selected from H and C1-4 alkyl;
   (7) each R₇ is independently selected from H and C1-4 alkyl;
   (8) n is selected from 1, 2 and 3;
   (9) n1 is selected from 1, 2, 3 and 4;
   (10) n2 is 1;
   (10) n3 is 0.
15. The ligand drug conjugate according to clause 13 or 14, or a stereoisomer of said ligand drug conjugate, a prodrug thereof, a pharmaceutically acceptable salt thereof or a pharmaceutically acceptable solvate thereof, characterized in that one or more of the following conditions are met:
   (1) R₁ is selected from H and halogen, R₂ is selected from H and C1-4 alkyl; or, R₁ and R₂, together with the carbon atoms to which they are attached, form 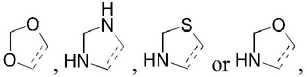 the dotted line indicates where the heterocyclic ring is fused to the benzene ring;
   (2) R₃ is H; or, R₃ and X together with the carbon atoms to which they are attached form 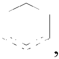 the dotted line indicates where the carbocyclic ring is fused to the benzene ring and pyridine ring;
   (3) W is absent or present, and when W is present, W is selected from -O-, -S-, -NR₄ -, 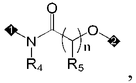 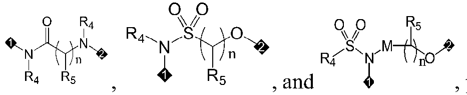 position 1 is attached to X, and position 2 is attached to L₄ or L₃;
   (4) each R₄ is independently selected from H, C1-4 alkyl and C3-6 cycloalkyl, R₅ is H;
   (5) R^{5a}, R^{5b} are each independently selected from H and methyl;
   (6) R₇ is H;
   (7) n is 1.
16. The ligand drug conjugate according to any one of clauses 13 to 15, or a stereoisomer of the ligand drug conjugate, a prodrug thereof, a pharmaceutically acceptable salt thereof or a pharmaceutically acceptable solvate thereof, characterized in that one or more of the following conditions are met:
   (1) R₁ is H or F, and R₂ is H or methyl; or, R₁ and R₂ together with the carbon atoms to which they are attached form 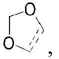 the dotted line indicates where the heterocyclic ring is fused to the benzene ring;
   (2) W is selected from -O-, -NR₄- and 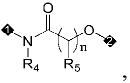 position 1 is attached to X and position 2 is attached to L₄ or L₃;
   (3) each R₄ is independently selected from H, methyl, ethyl, n-propyl, isopropyl, t-butyl, and cyclopropyl, and R₅ is H;
   (4) X is selected from optionally substituted 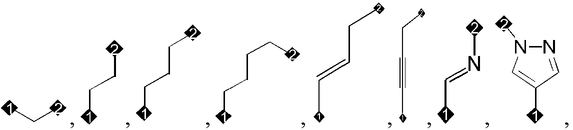 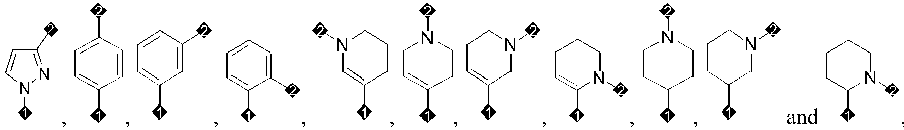 , position 1 is attached to the parent ring and position 2 is attached to W or L₄; the substituents are selected from 1 or 2 C1-4 alkyl groups, or 2 C1-4 alkyl groups together with the carbon atom to which they are both attached form a C3-6 cycloalkyl group; said C1-4 alkyl group is for example methyl; said C3-6 cycloalkyl is for example cyclopropyl.
17. The ligand drug conjugate according to any one of clauses 13 to 16, or a stereoisomer of said ligand drug conjugate, a prodrug thereof, a pharmaceutically acceptable salt thereof or a pharmaceutically acceptable solvate thereof, characterized in that
   (1) W is selected from -O- and -NR₄ -;
   (2) X is selected from 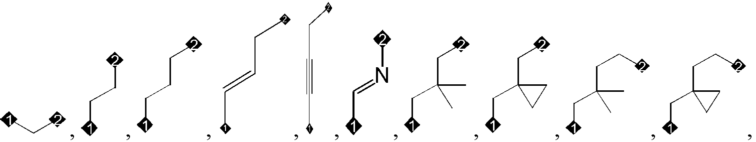 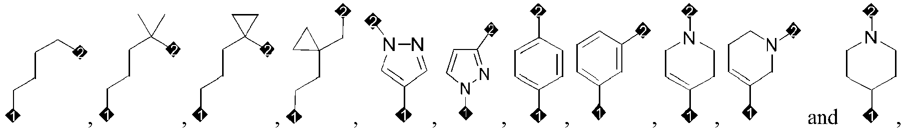 position 1 is attached to the parent ring, and position 2 is attached to W or L₄ .
18. The ligand drug conjugate according to any one of clauses 13 to 17, or a stereoisomer of said ligand drug conjugate, a prodrug thereof, a pharmaceutically acceptable salt thereof or a pharmaceutically acceptable solvate thereof, characterized in that when W is absent, X is selected from 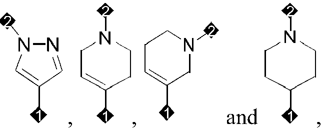 position 1 is attached to the parent ring and position 2 is attached to L₄; when W is present, X is selected from 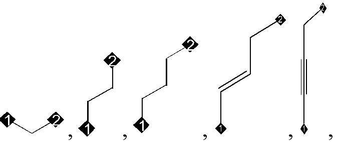 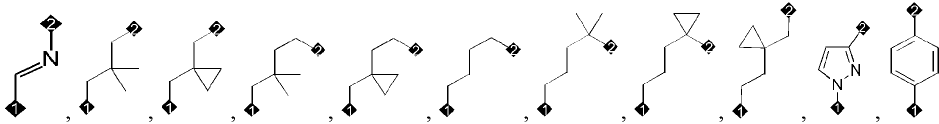 and 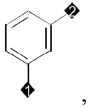 position 1 is attached to the parent ring and position 2 is attached to W.
19. The ligand drug conjugate according to any one of clauses 13 to 18, or a stereoisomer of said ligand drug conjugate, a prodrug thereof, a pharmaceutically acceptable salt thereof or a pharmaceutically acceptable solvate thereof, characterized in that 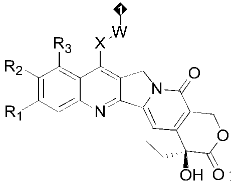 is selected from the following: 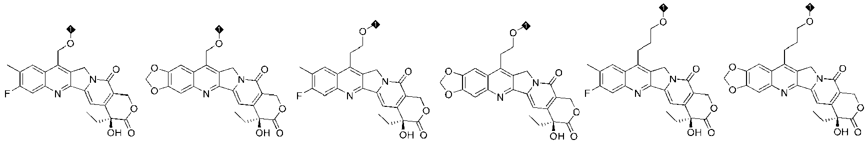 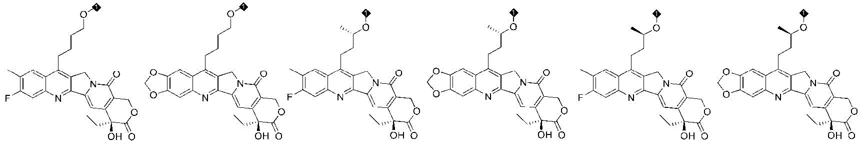 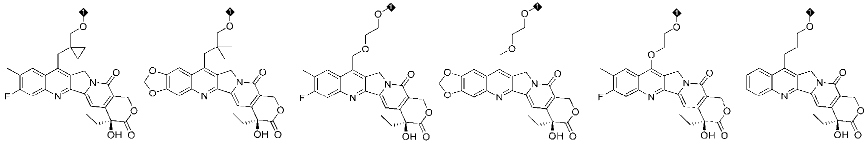 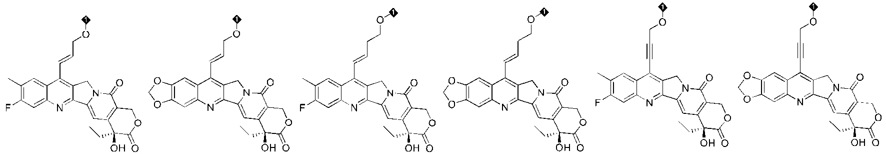 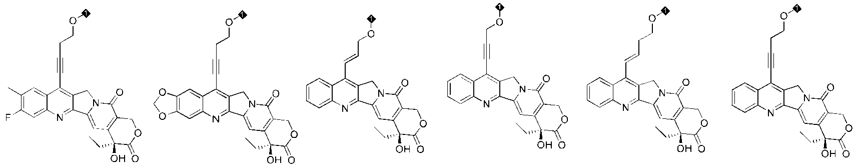 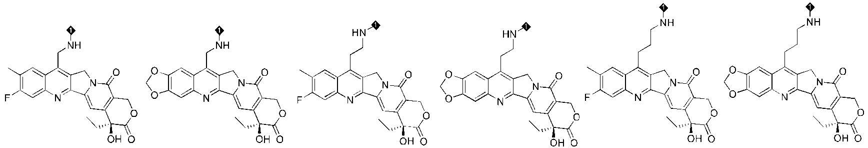 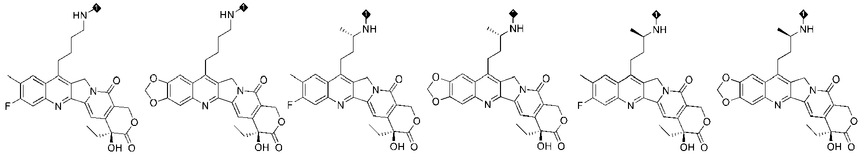 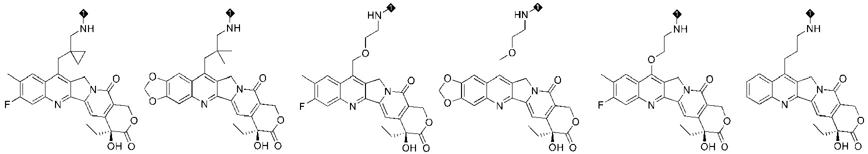 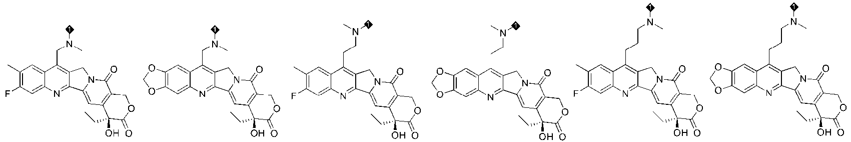 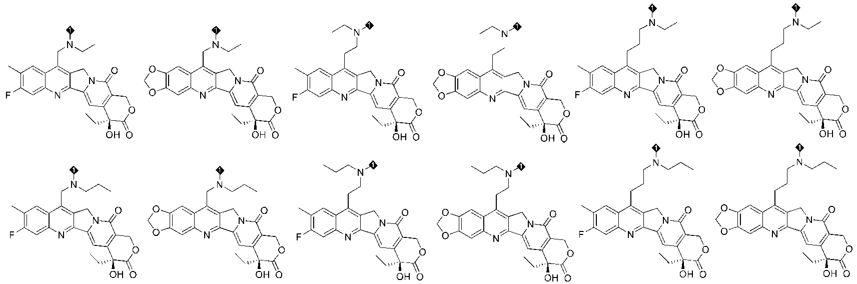 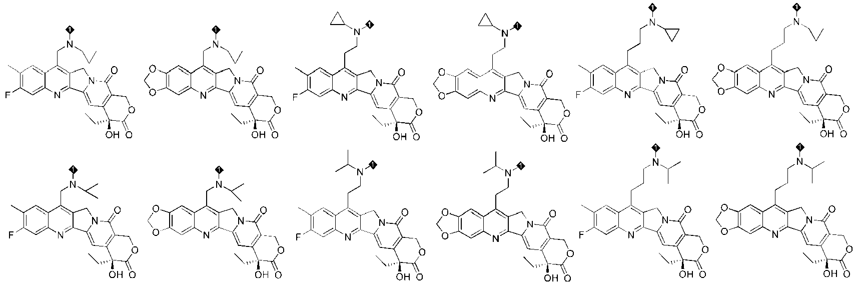 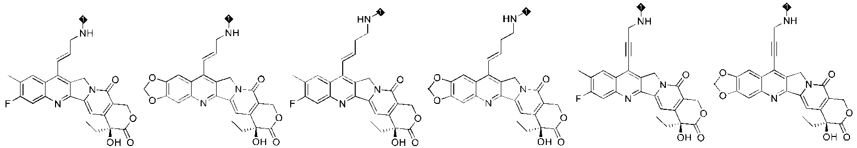 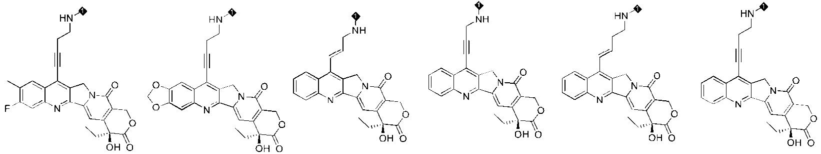 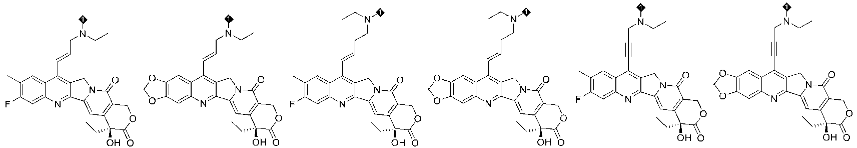 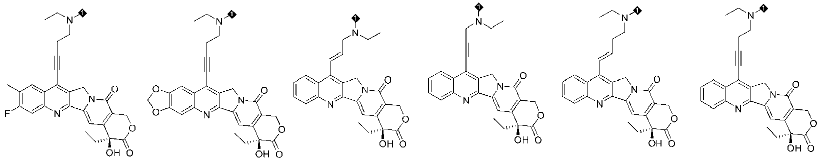 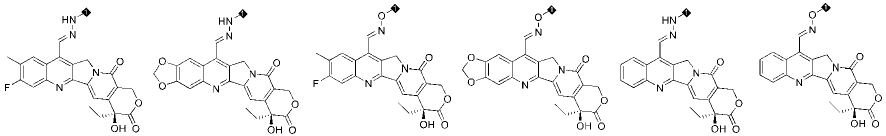 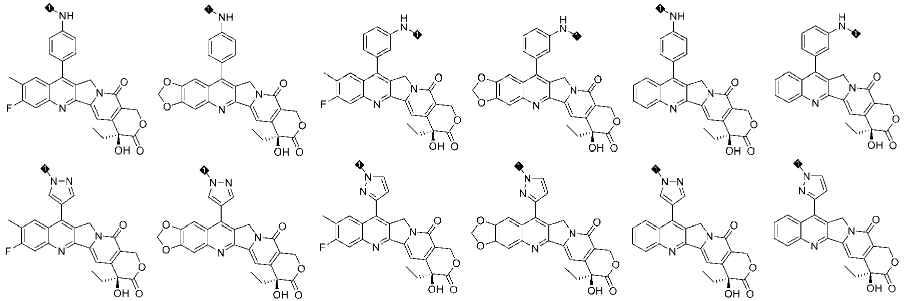 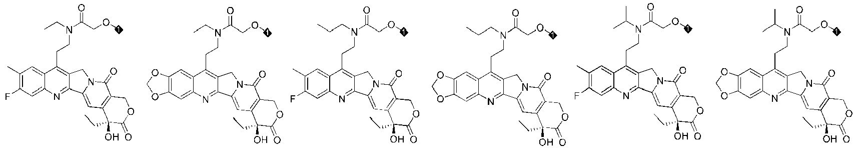 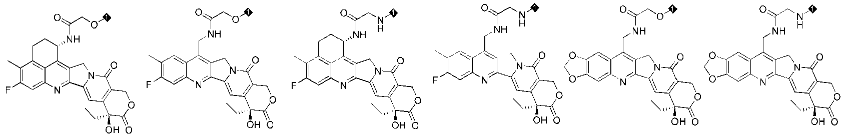 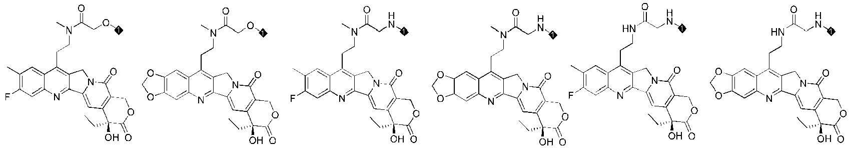 wherein position 1 is attached to L₄; when L₄ is absent, position 1 is attached to L₃.
20. The ligand drug conjugate according to any one of clauses 13 to 19, or a stereoisomer of said ligand drug conjugate, a prodrug thereof, a pharmaceutically acceptable salt thereof, or a pharmaceutically acceptable solvate thereof, characterized in that said ligand drug conjugate has a structure according to formula I-1, formula I-2, or formula I-3: 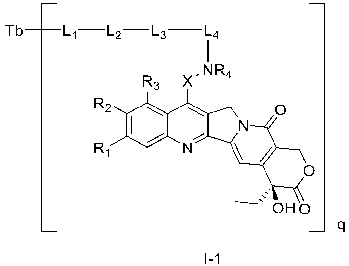
   Wherein Tb, L₁, L₂, L₃, L₄, X, R₁, R₂, R₃, R₄, and q are as defined in any one of clauses 13 to 19; 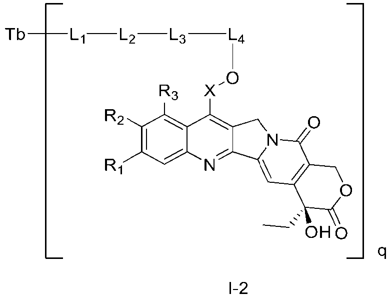
   Wherein Tb, L₁, L₂, L₃, L₄, X, R₁, R₂, R₃, and q are as defined in any one of clauses 13 to 19; 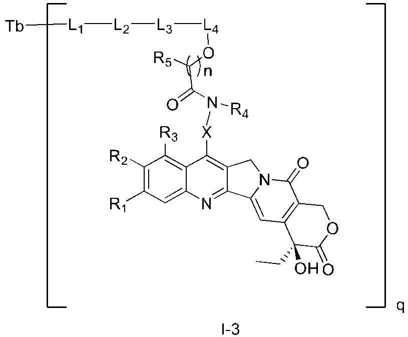
   Wherein Tb, L₁, L₂, L₃, L₄, X, R₁, R₂, R₃, R₄, R₅, n, and q are as defined in any one of clauses 13 to 19.
21. The ligand drug conjugate according to any one of clauses 13 to 20, or a stereoisomer of said ligand drug conjugate, a prodrug thereof, a pharmaceutically acceptable salt thereof, or a pharmaceutically acceptable solvate thereof, characterized in that said ligand drug conjugate has the structure of formula I-1A, formula I-1B, formula I-2A, formula I-2B, formula I-3A, or formula I-3B: 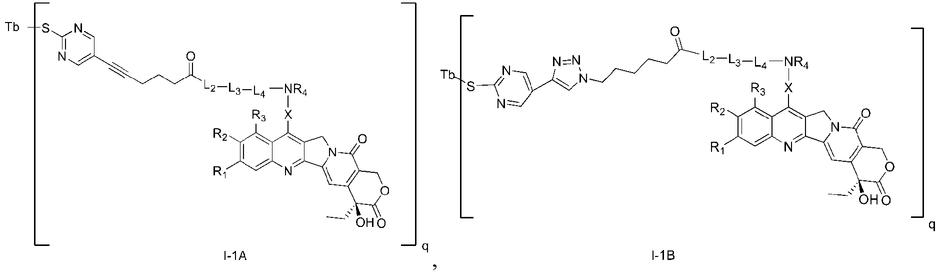
   wherein Tb, L₂, L₃, L₄, X, R₁, R₂, R₃, R₄, and q are as defined in any one of clauses 13 to 19; 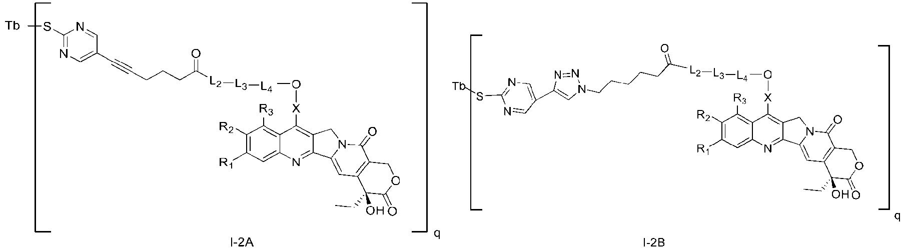
   wherein Tb, L₂, L₃, L₄, X, R₁, R₂, R₃, and q are as defined in any one of clauses 13 to 19; 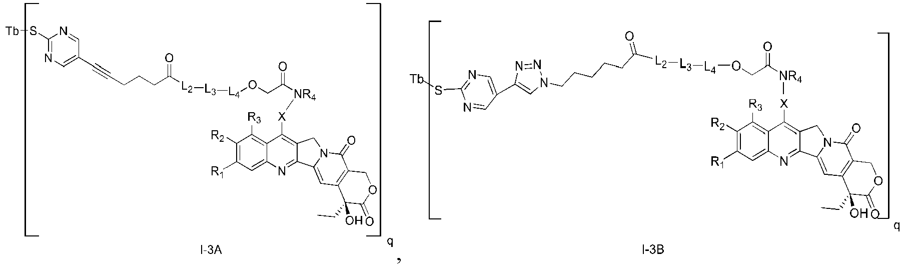
   wherein Tb, L₂, L₃, L₄, X, R₁, R₂, R₃, R₄, and q are as defined in any one of clauses 13 to 19.
22. The ligand drug conjugate according to any one of clauses 13 to 21, or a stereoisomer of said ligand drug conjugate, a prodrug thereof, a pharmaceutically acceptable salt thereof, or a pharmaceutically acceptable solvate thereof, characterized in that said ligand drug conjugate has the structure of formula I-A or formula I-B: 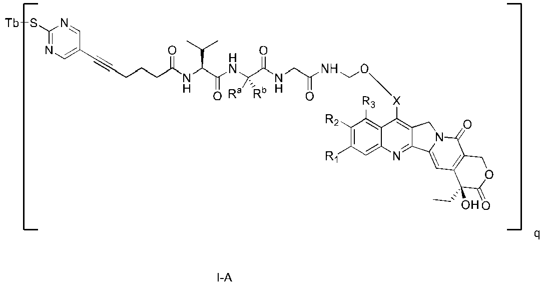
   wherein Tb, X, R₁, R₂, R₃, R^{a}, R^{b}, and q are as defined in any one of clauses 13 to 19; 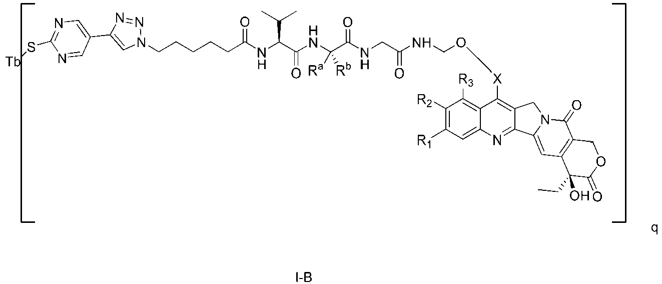
   wherein Tb, X, R₁, R₂, R₃, R^{a}, R^{b}, and q are as defined in any one of clauses 13 to 19.
23. The ligand drug conjugate according to any one of clauses 1 to 22, or a stereoisomer of said ligand drug conjugate, a prodrug thereof, a pharmaceutically acceptable salt thereof, or a pharmaceutically acceptable solvate thereof, characterized in that, wherein said ligand drug conjugate is selected from: 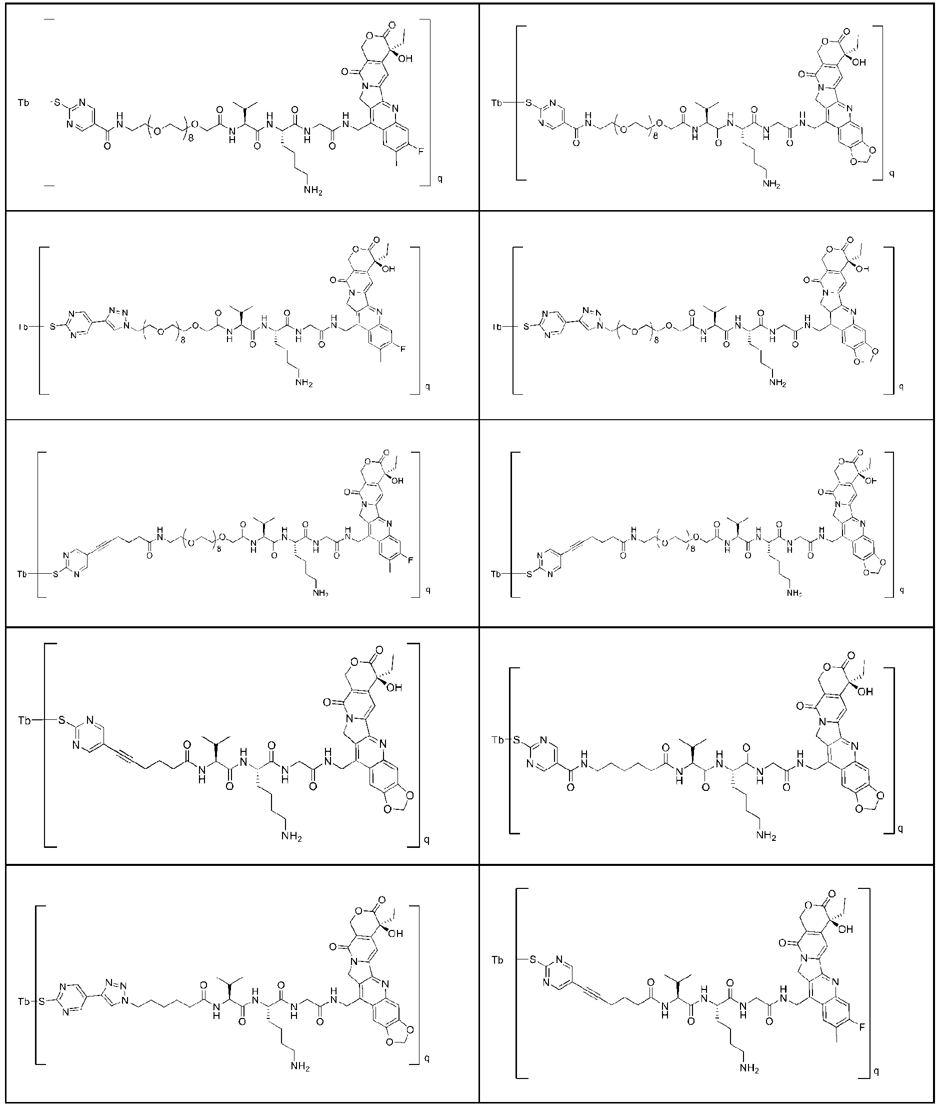 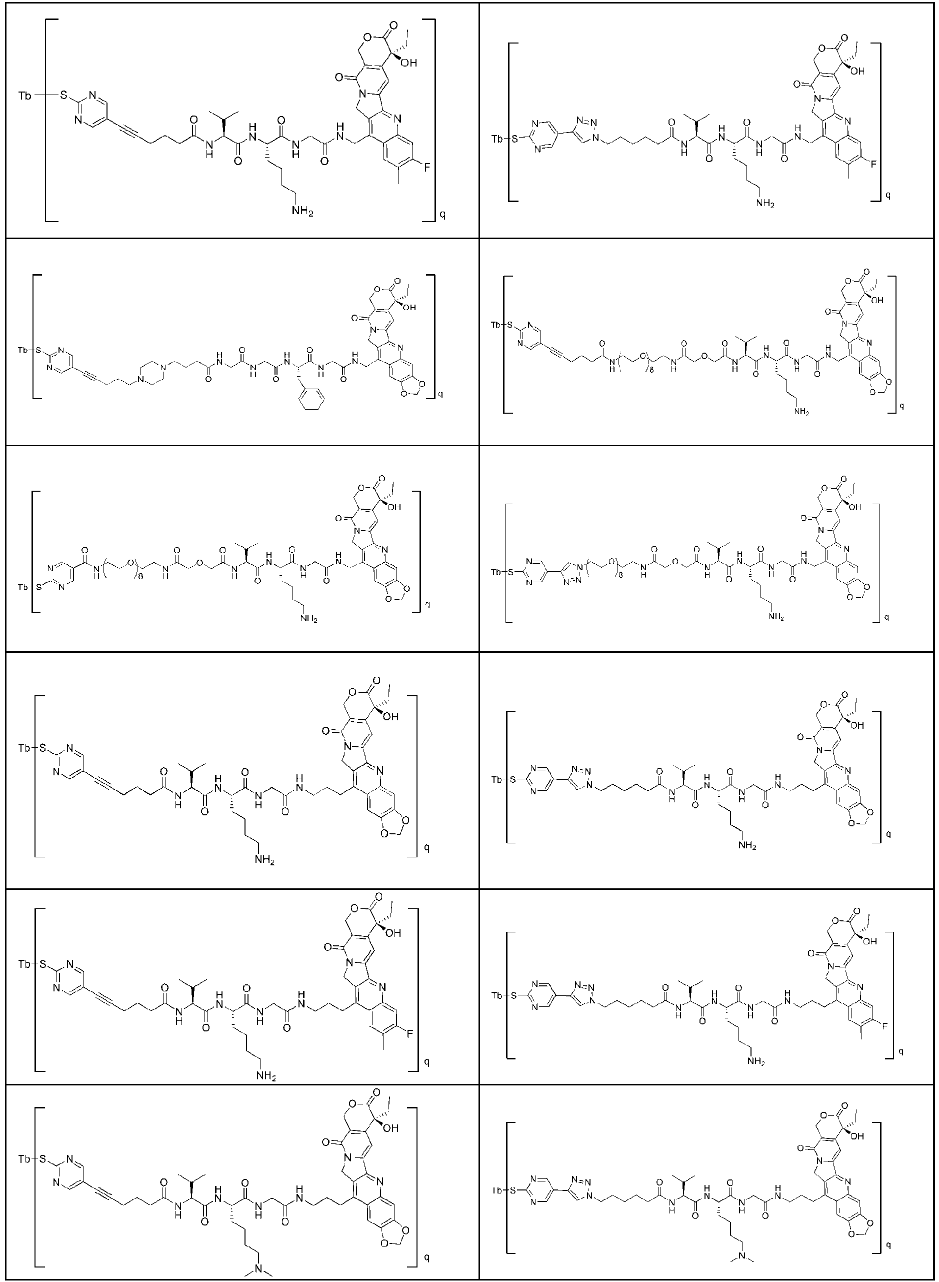 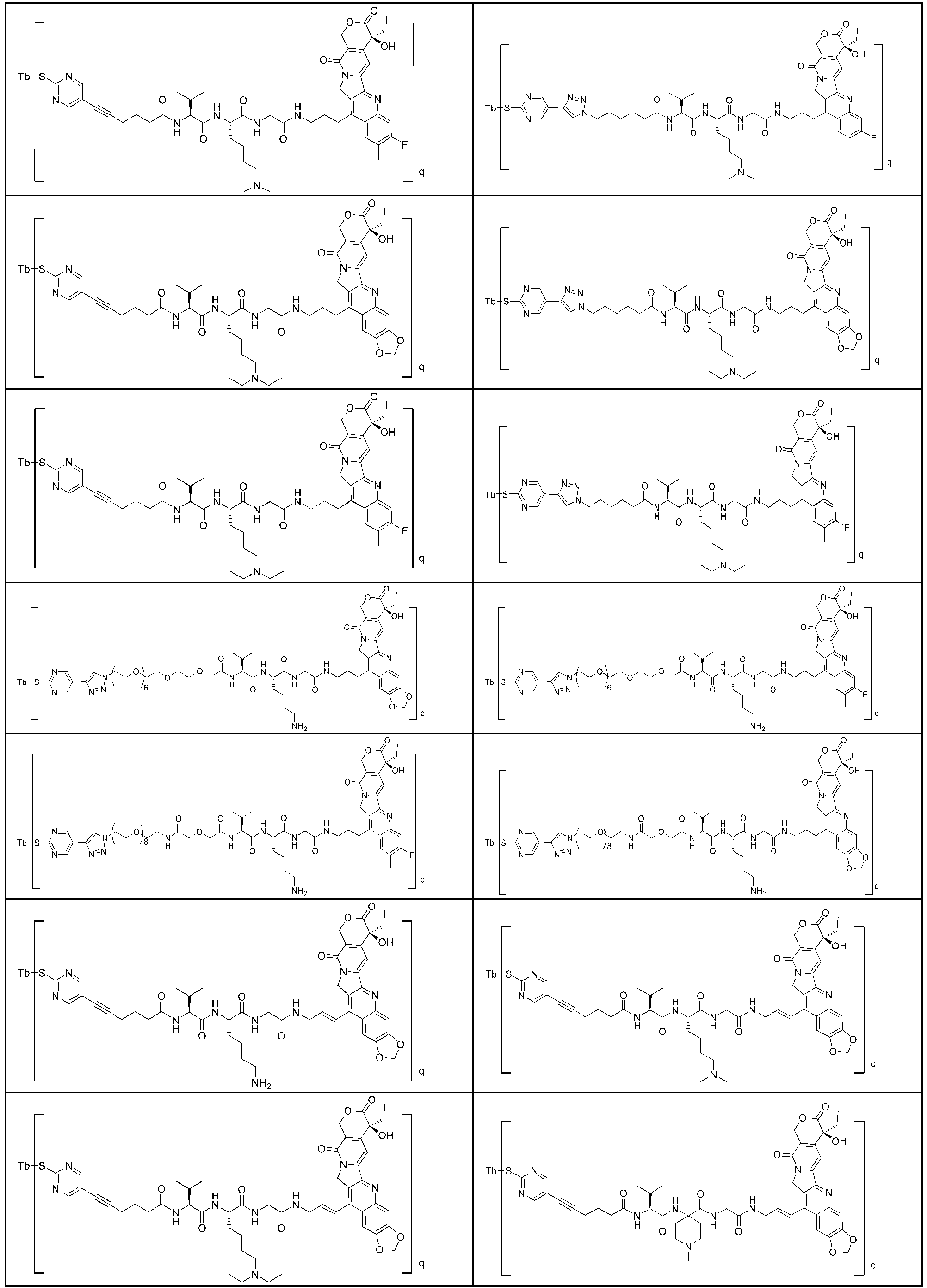 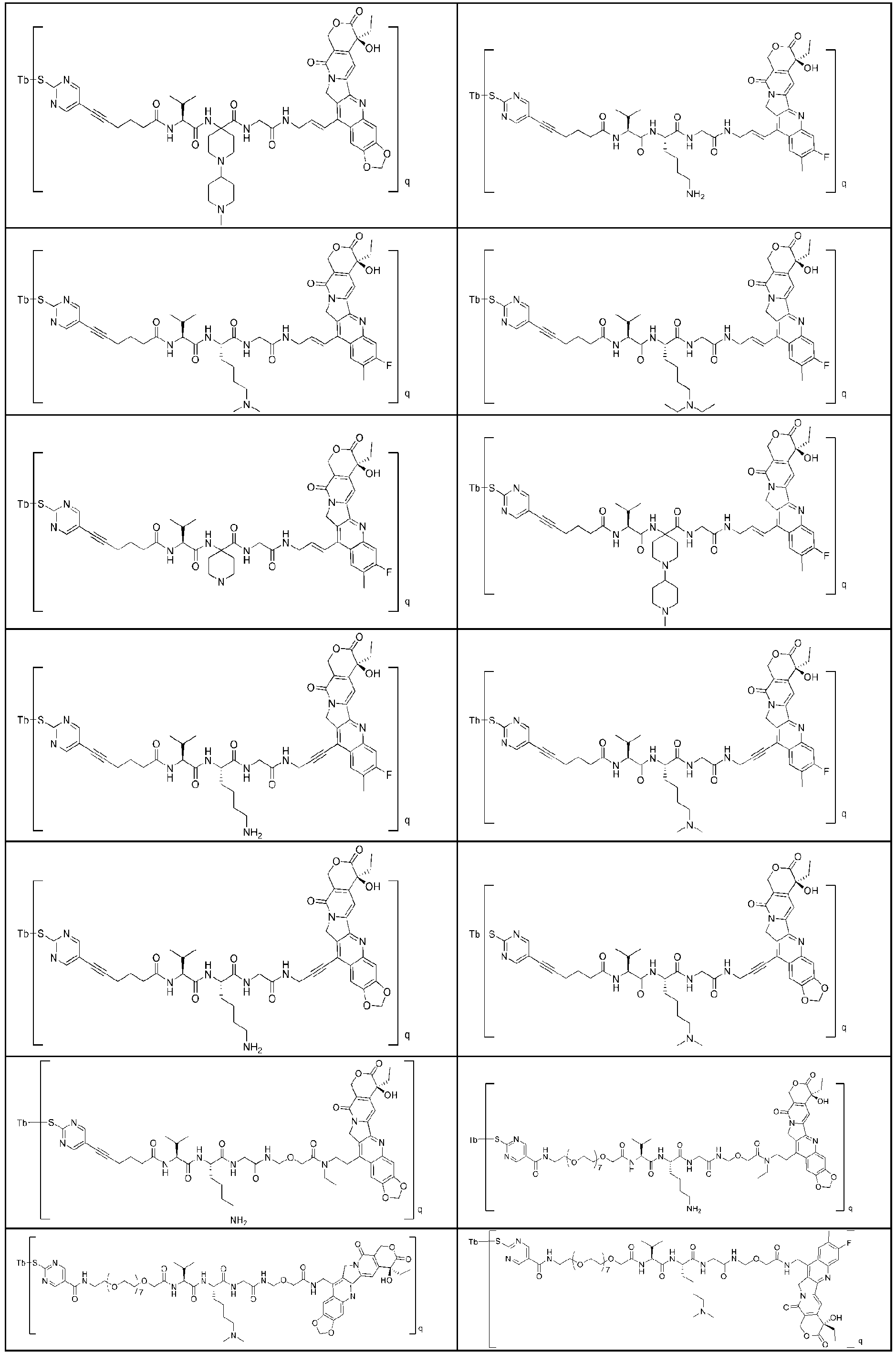 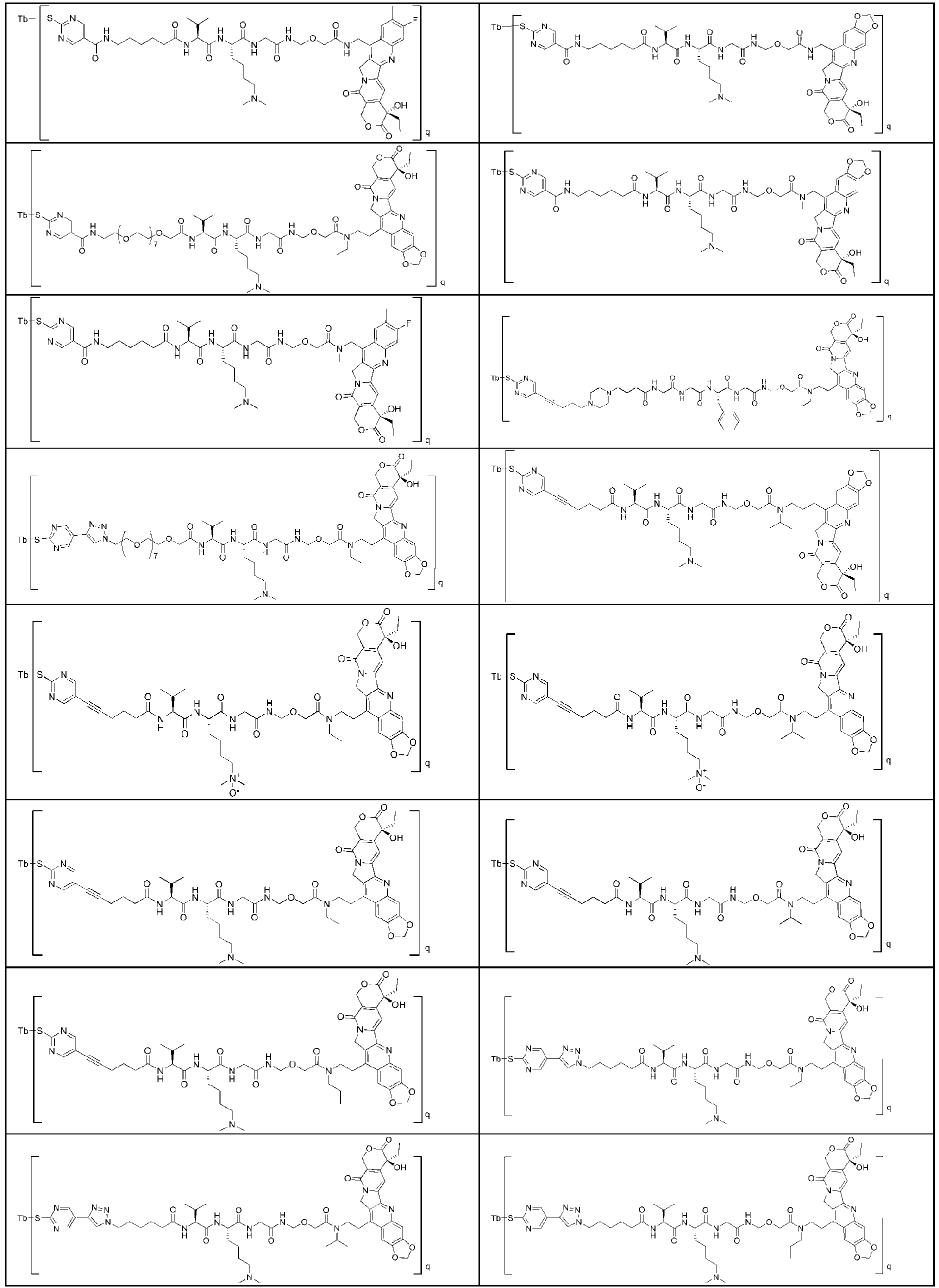 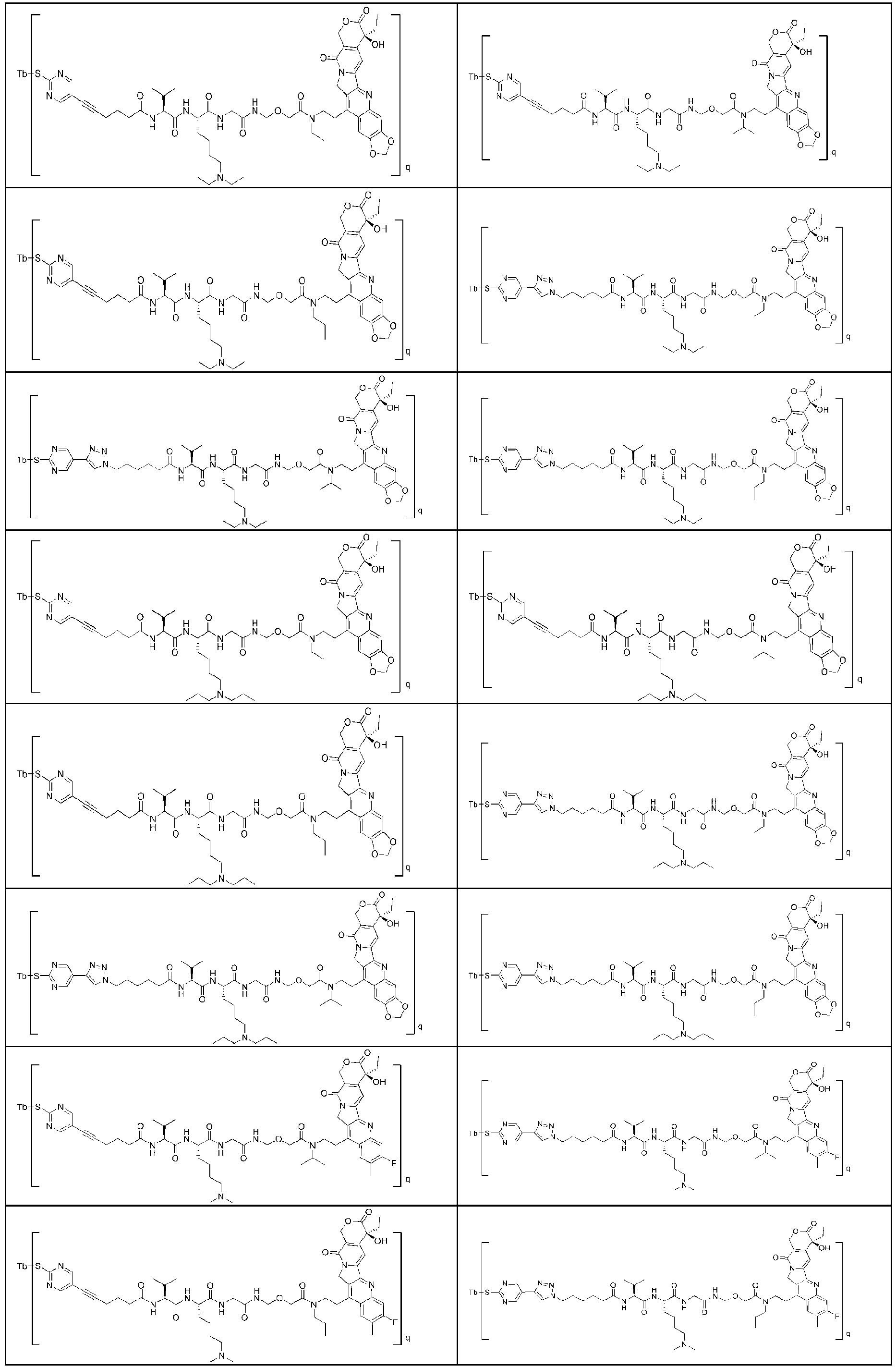 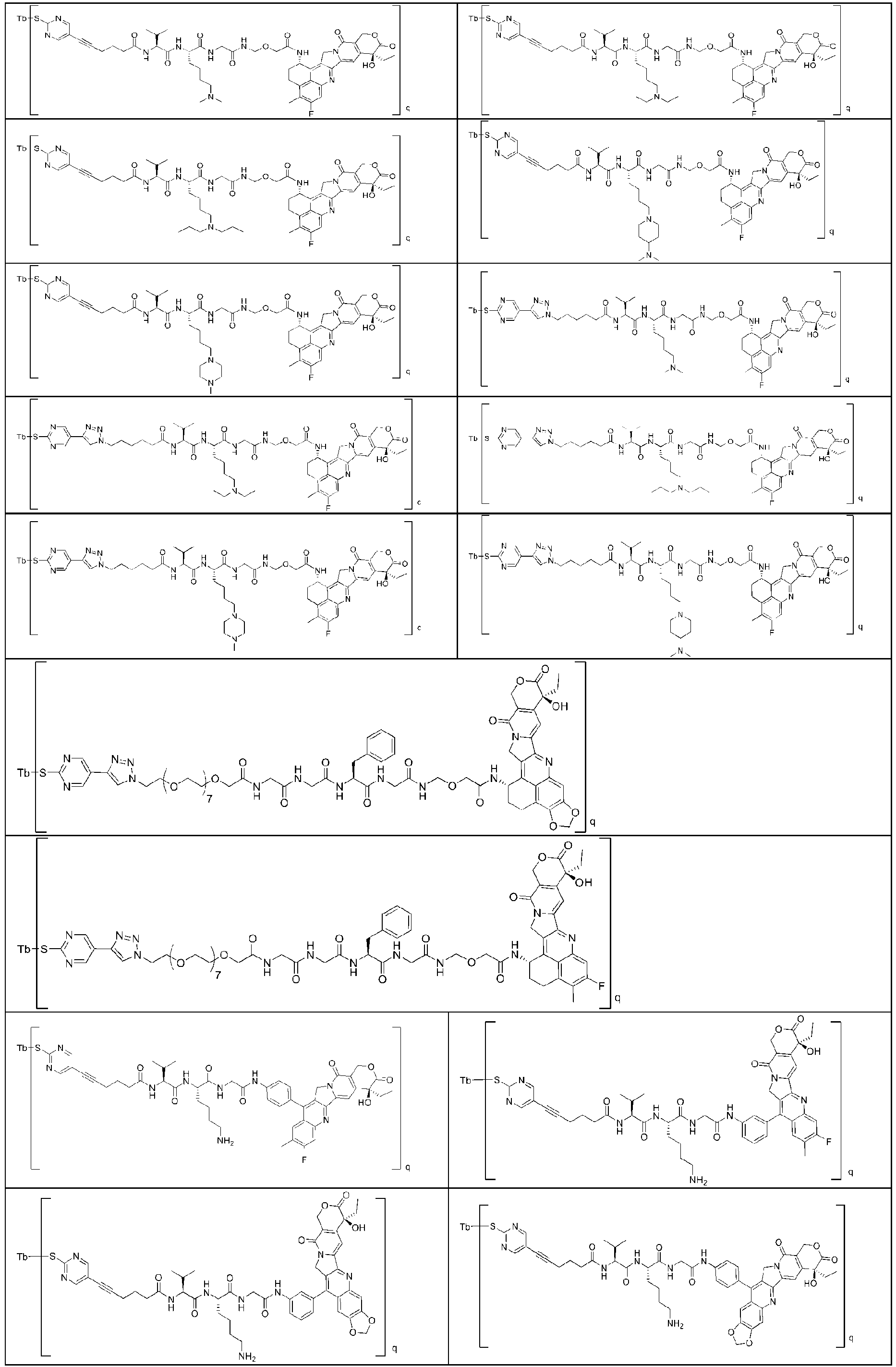 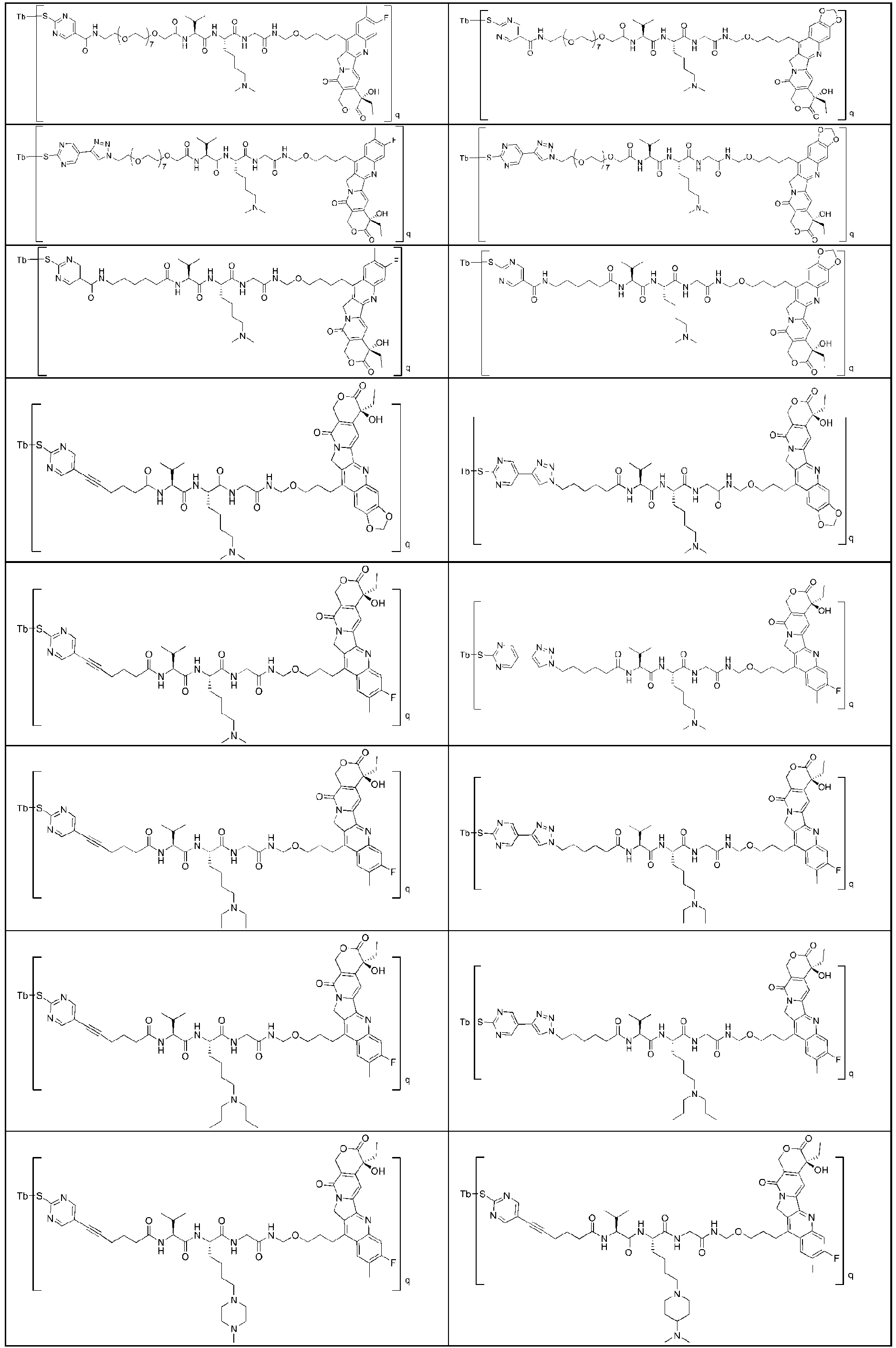 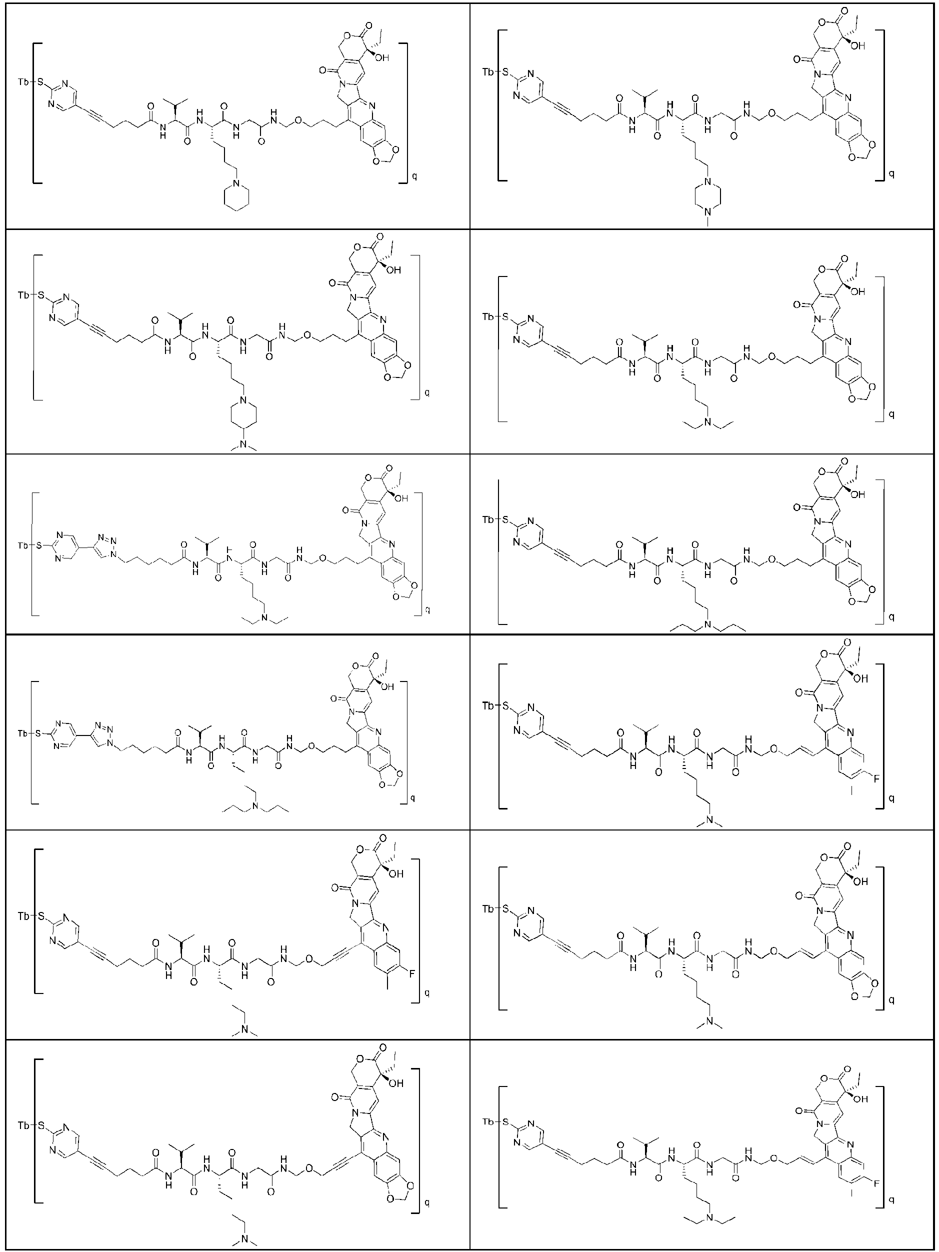 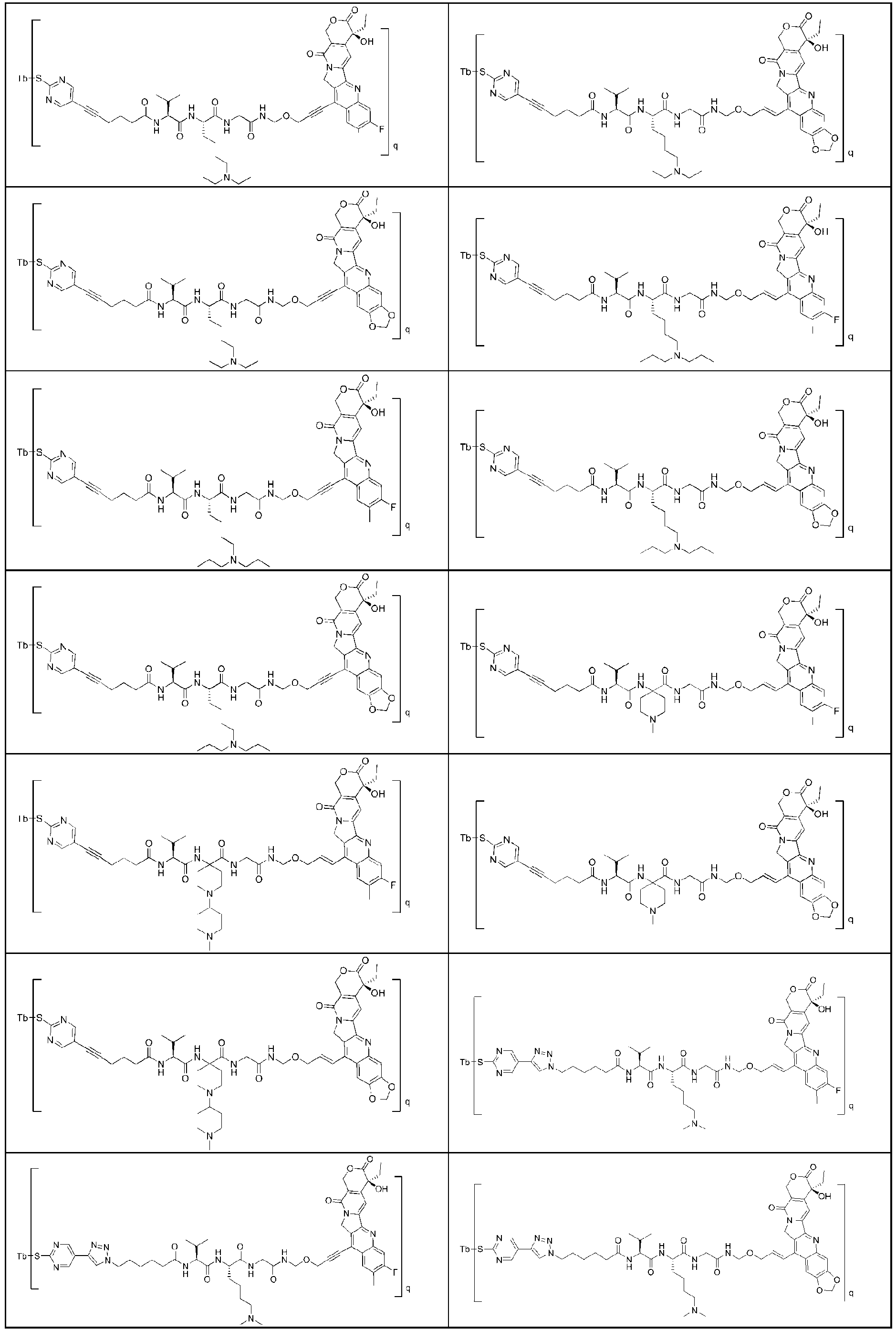 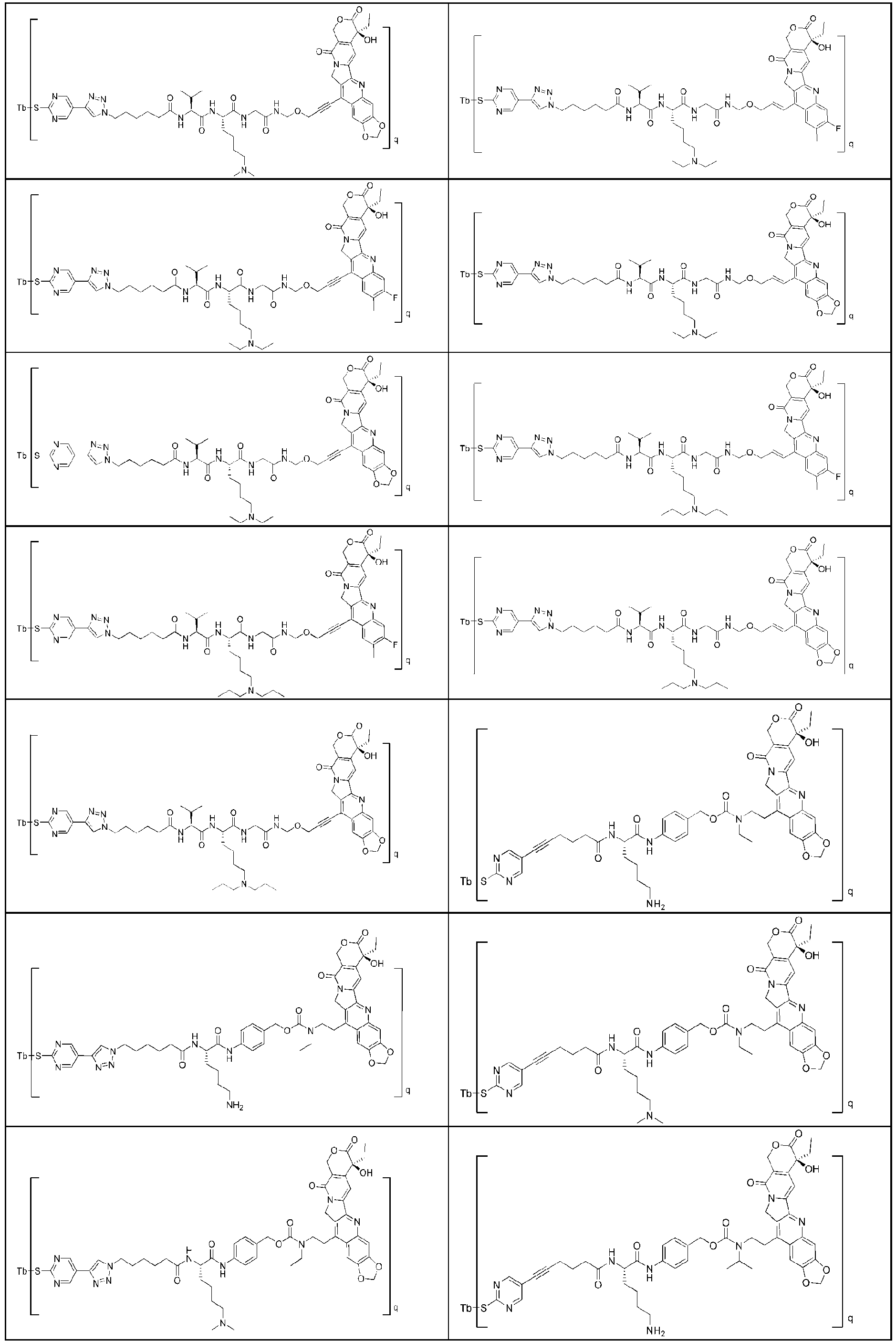 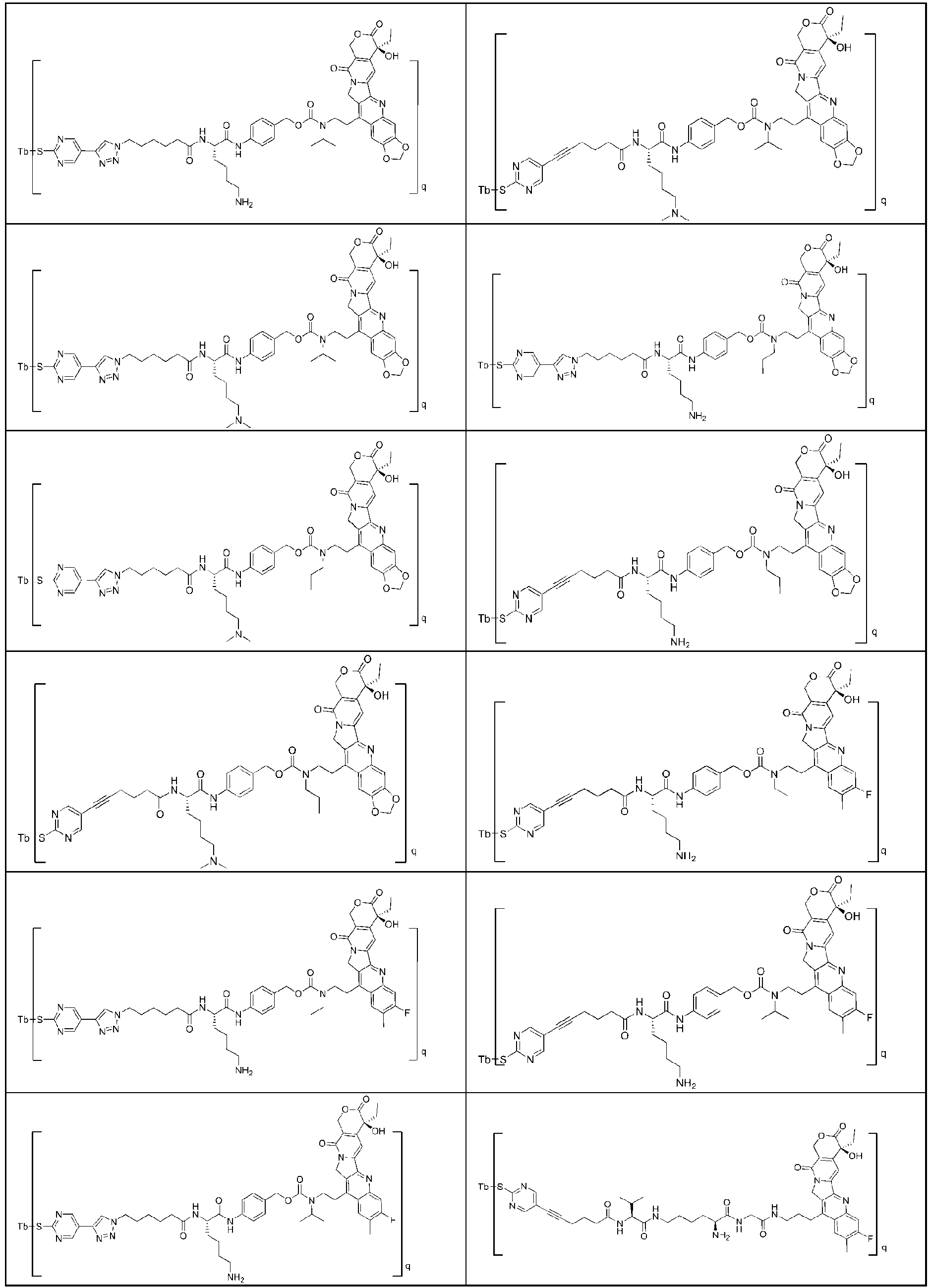 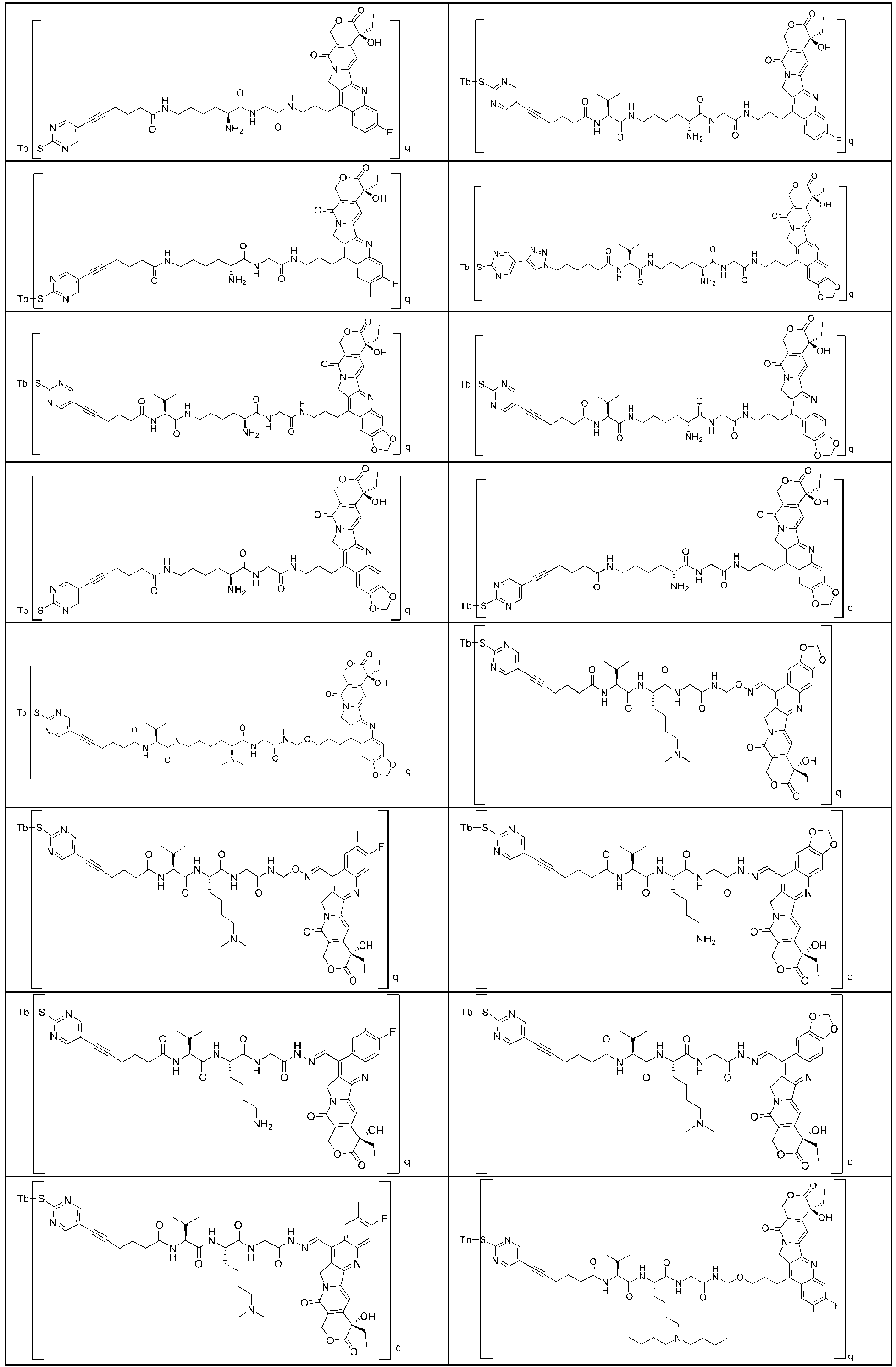 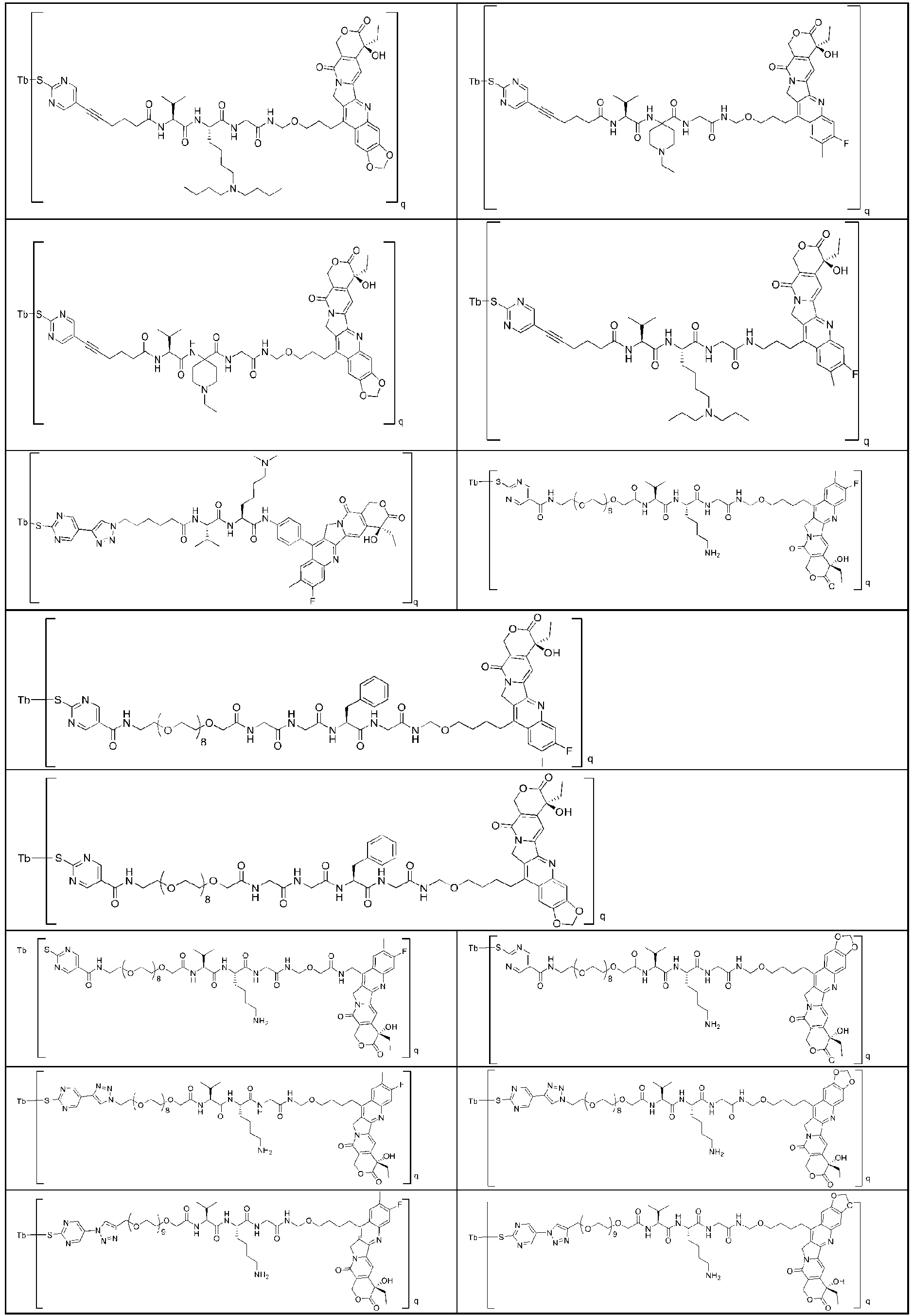 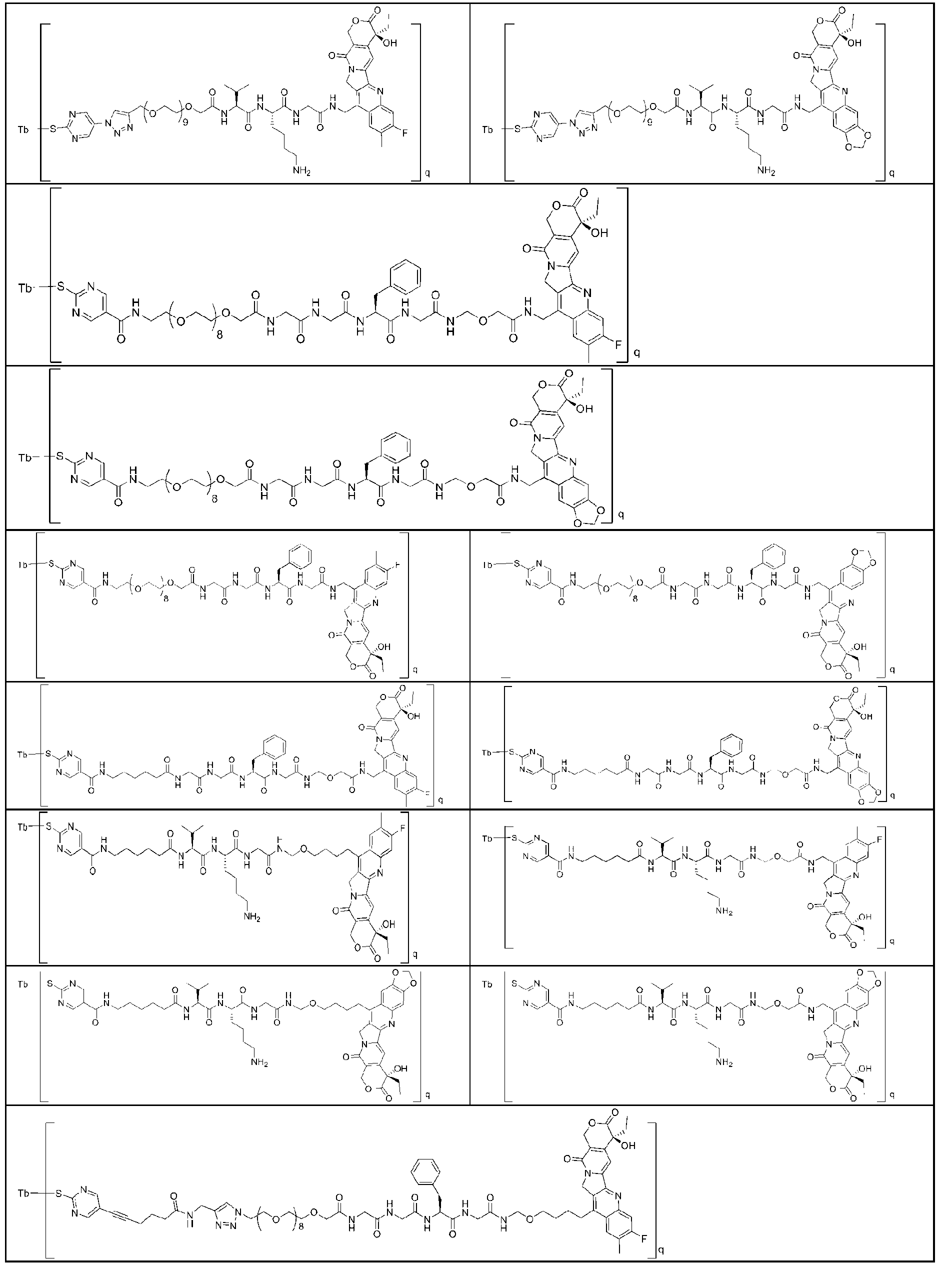 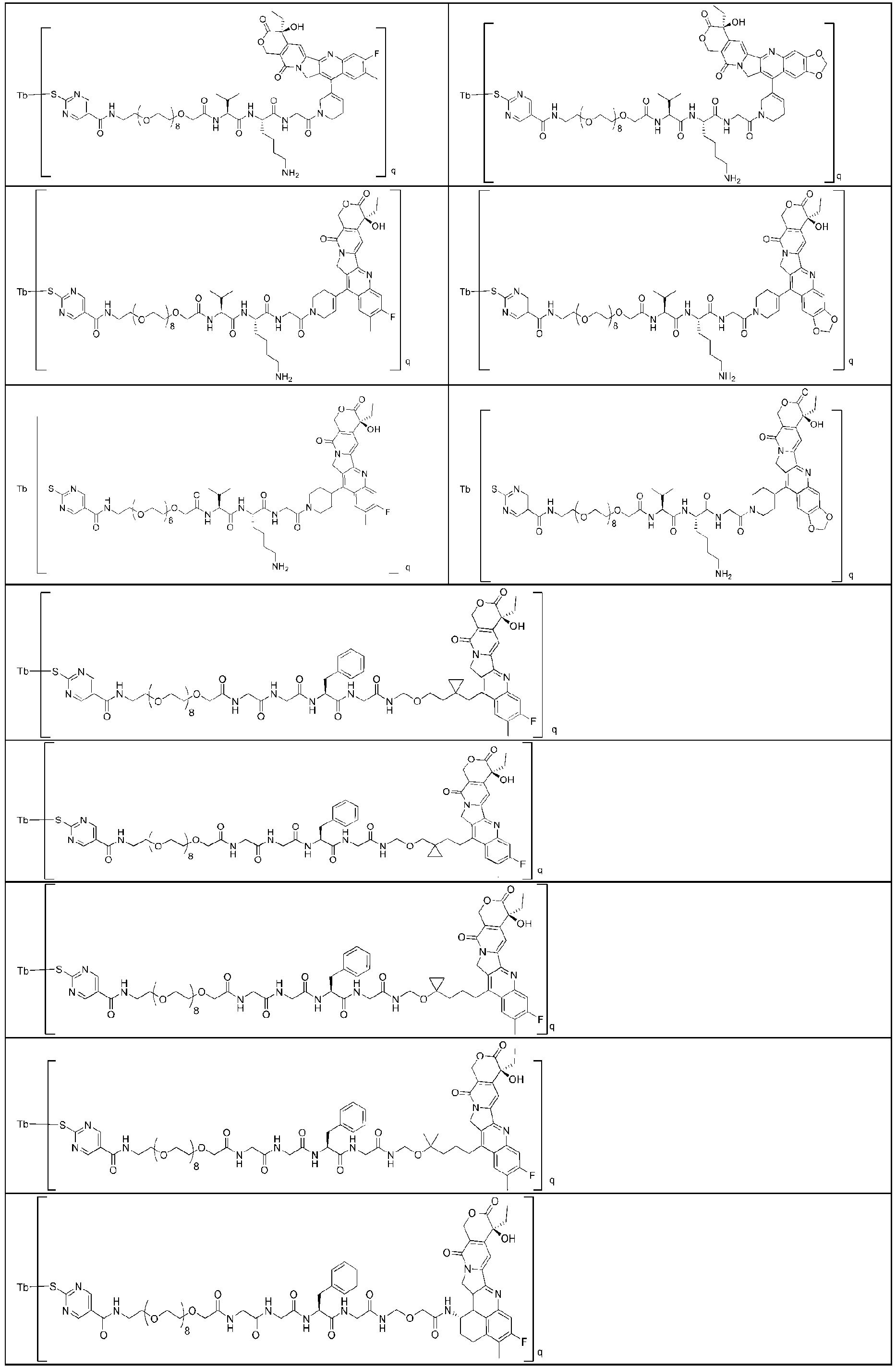 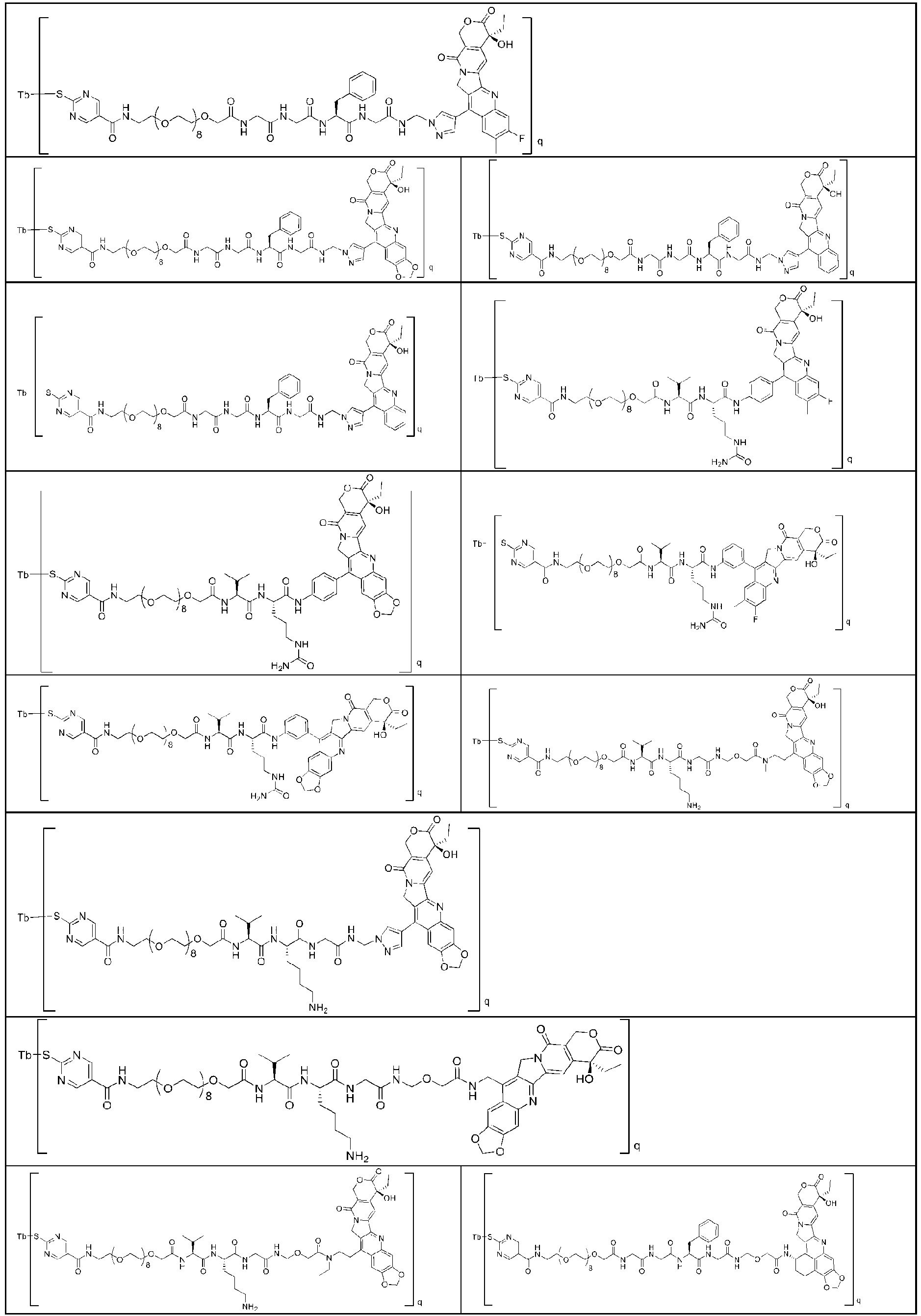 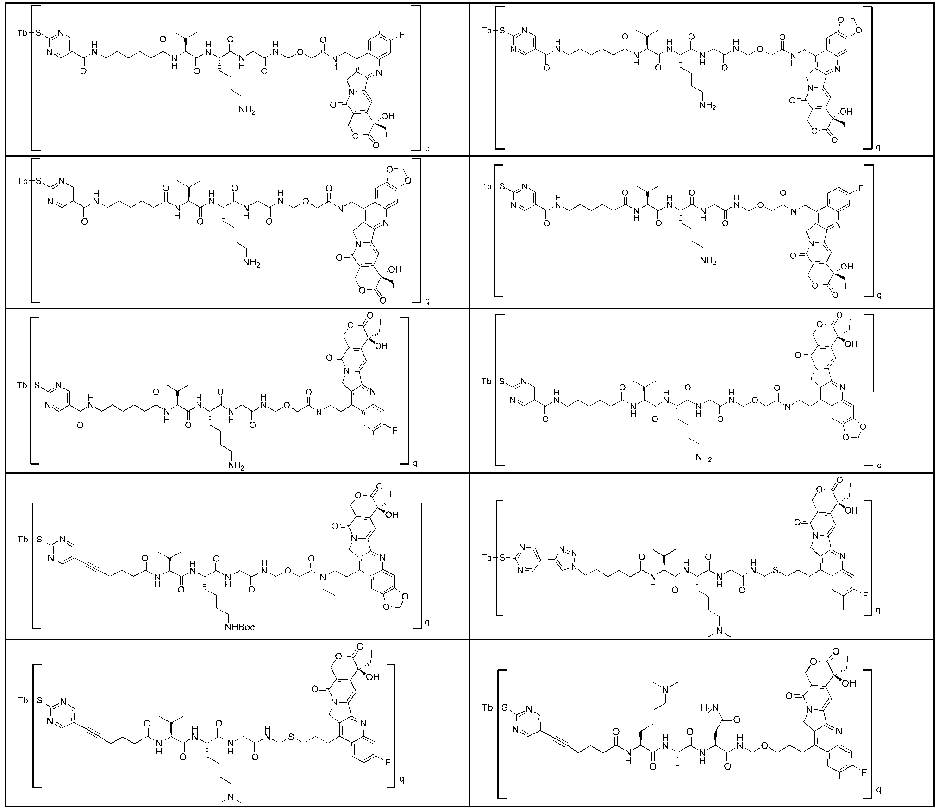
24. The ligand drug conjugate according to any one of clauses 1 to 23, or a stereoisomer of said ligand drug conjugate, a prodrug thereof, a pharmaceutically acceptable salt thereof, or a pharmaceutically acceptable solvate thereof, characterized in, wherein, said ligand drug conjugate is selected from: 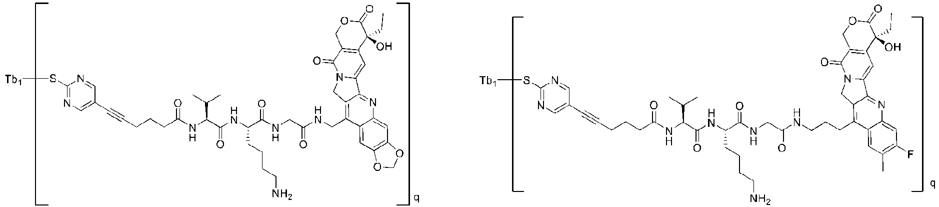 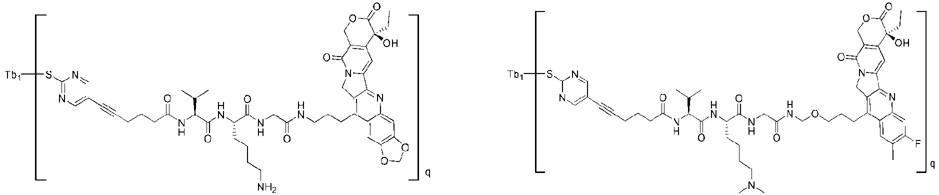 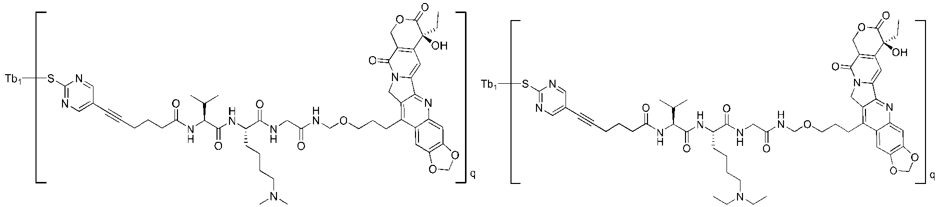 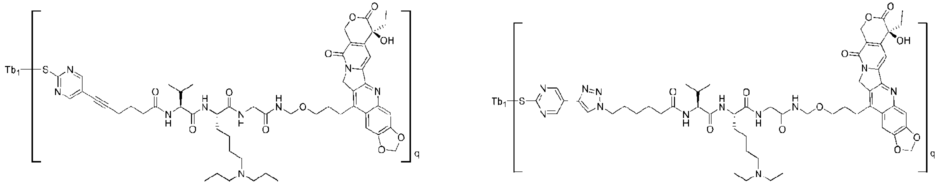 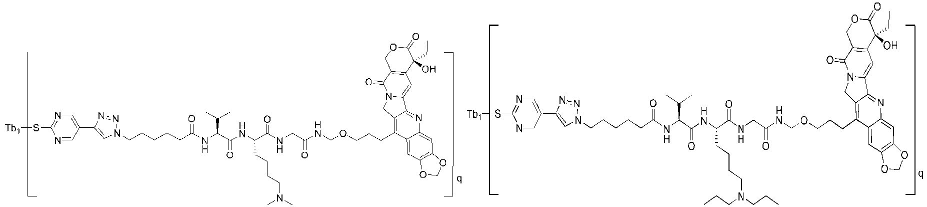 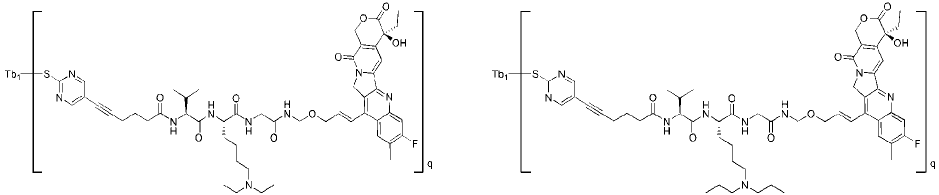 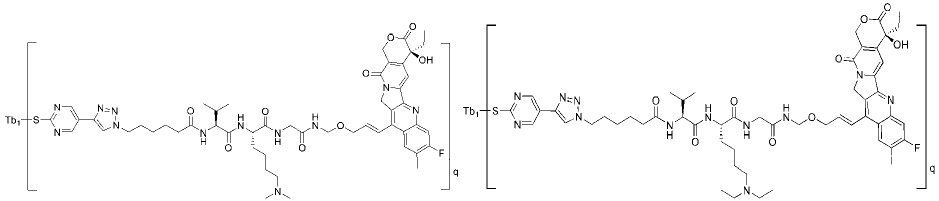 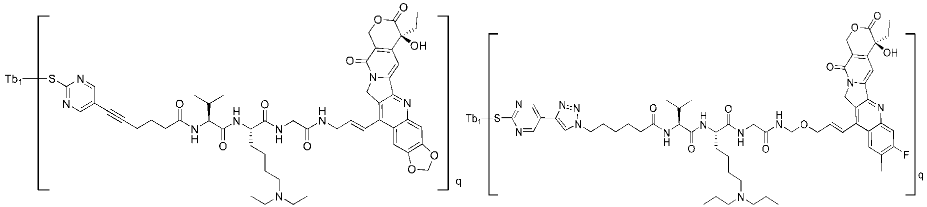 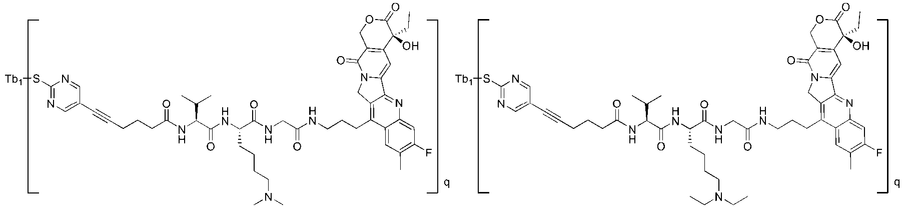 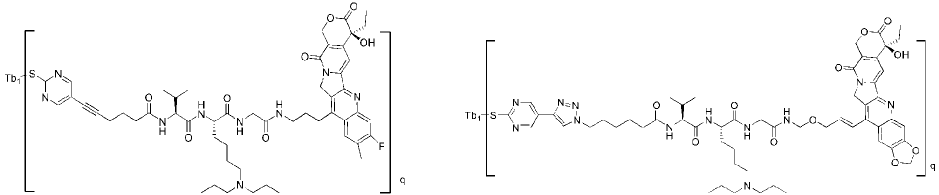 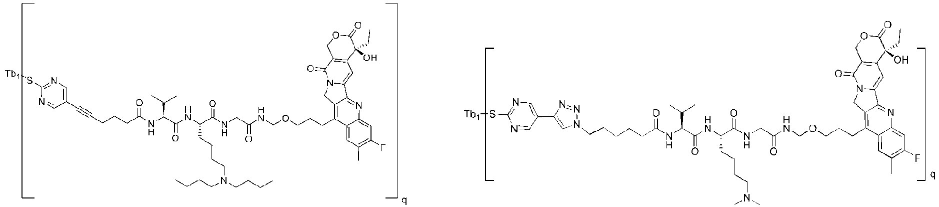 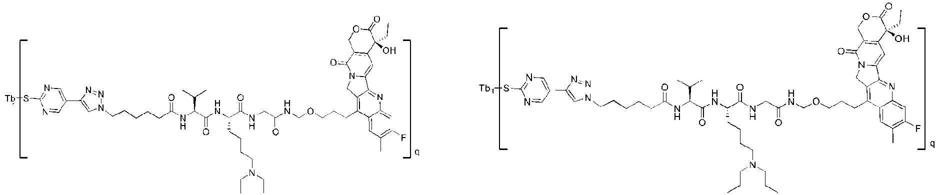 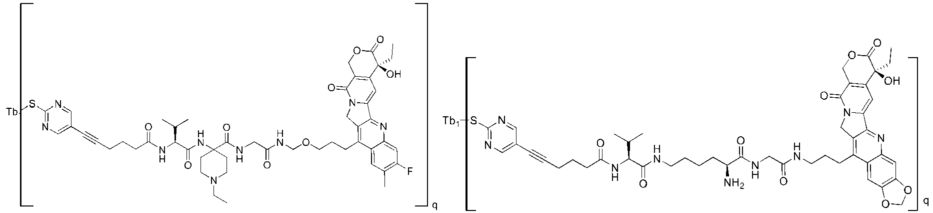
   wherein Tb₁ is an anti-B7H3 antibody or antigen binding fragment thereof, such as enoblituzumab, mirzotamab, omburtamab, 1D1-01, 2E3-02 antibody; q is selected from any numerical value between 0.1 and 16.0, preferably from any numerical value between 2 and 8, more preferably q is 2, 4, 6 or 8; 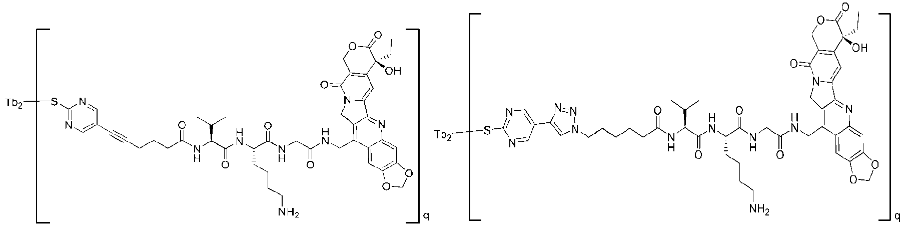 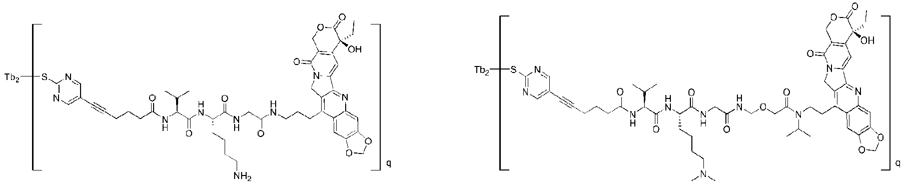 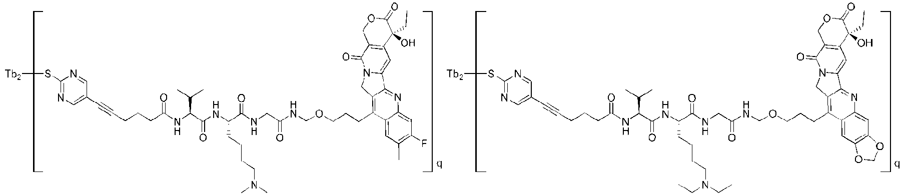 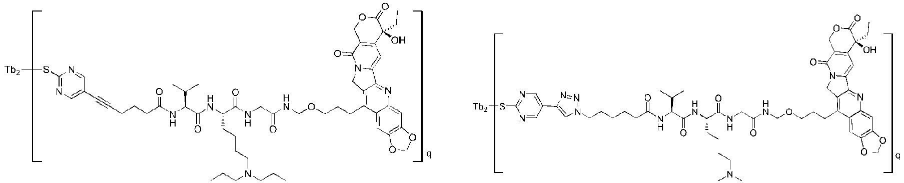 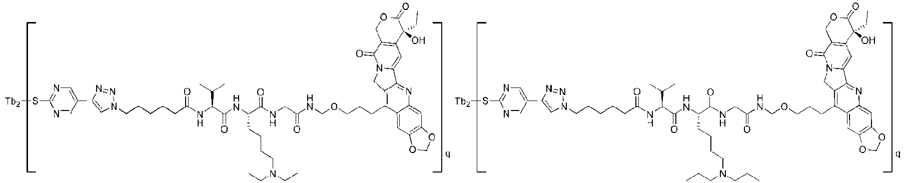
   wherein Tb₂ is an anti-Trop-2 antibody or antigen binding fragment thereof, e.g., datopotamab, sacituzumab; q is selected from any numerical value between 0.1 and 16.0, preferably from any numerical value between 2 and 8, more preferably q is 2, 4, 6 or 8; 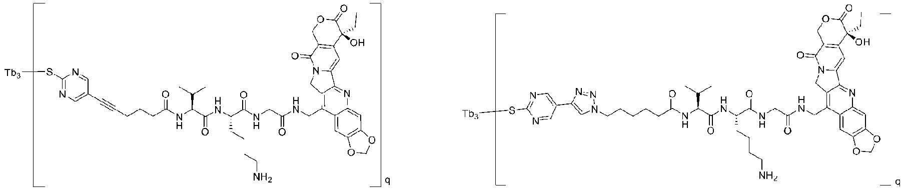 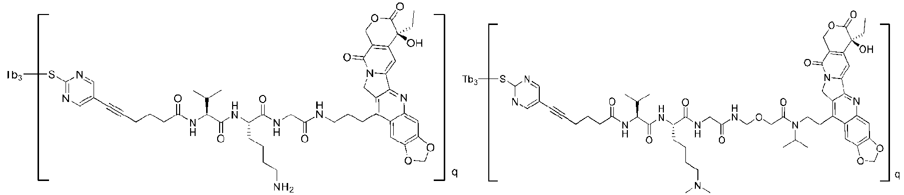 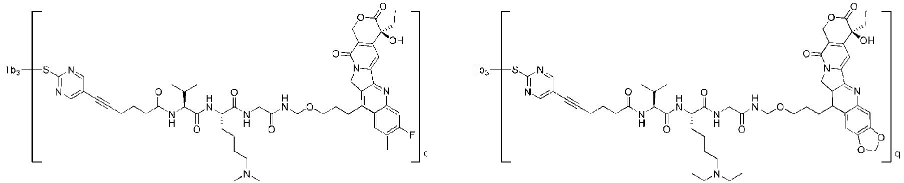 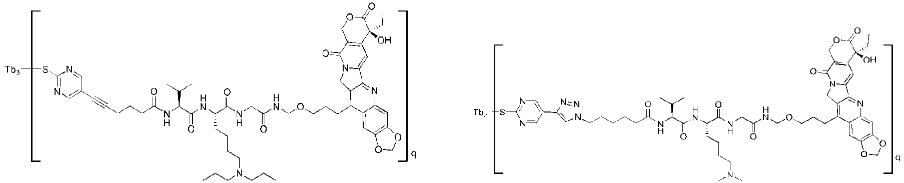 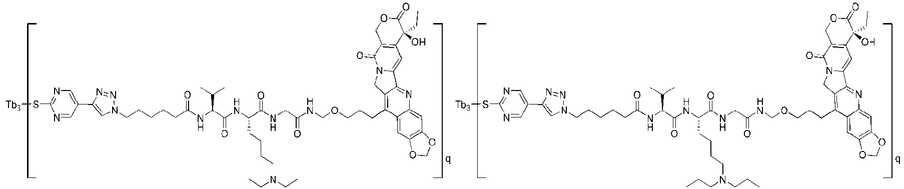
   wherein Tb₃ is an anti-Her2 antibody or antigen binding fragment thereof, such as anbenitamab, coprelotamab, disitamab, gancotamab, margetuximab, pertuzumab, timigutuzumab, zanidatamab, Trastuzumab; q is selected from any numerical value between 0.1 and 16.0, preferably from any numerical value between 2 and 8, more preferably q is 2, 4, 6 or 8; 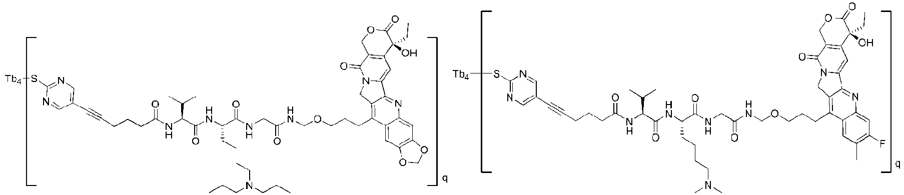 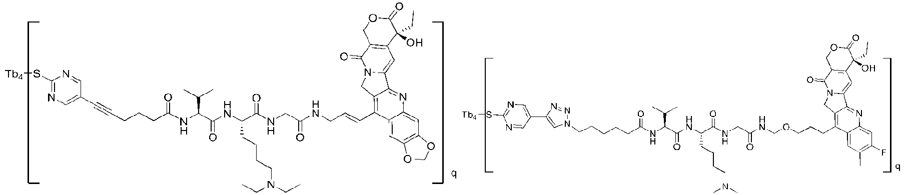
   wherein Tb₄ is an anti-Her3 antibody or antigen binding fragment thereof, such as, barecetamab, duligotuzumab, elgemtumab, lumretuzumab, patritumab, seribantumab, an antibody represented by IMGT/mAb-DB ID: 546; q is selected from any numerical value between 0.1 and 16.0, preferably from any numerical value between 2 and 8, more preferably q is 2, 4, 6 or 8; 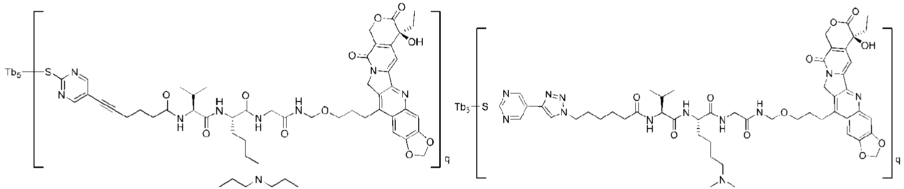
   wherein Tb₅ is an anti-EGFR antibody or antigen binding fragment thereof, such as demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, Cetuximab; q is selected from any numerical value between 0.1 and 16.0, preferably from any numerical value between 2 and 8, more preferably q is 2, 4, 6 or 8; 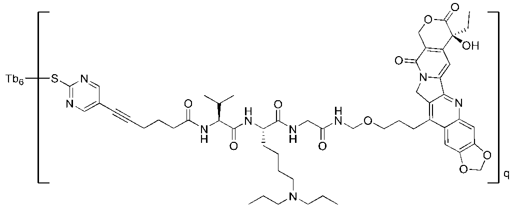
   wherein Tb₆ is an antibody that has no tumor cell endocytosis activity and has no tumor cell surface antigen binding activity, or an antibody that has binding activity to a non-endocytic antigen (e.g., ALCAM/CD 166), such as, antibodies of the IgG isotype which do not have the corresponding cell surface antigen in humans, anti-CD 166 antibodies; more specifically, anti-chicken lysozyme human IgG1 isotype antibody; q is selected from any numerical value between 0.1 and 16.0, preferably from any numerical value between 2 and 8, more preferably q is 2, 4, 6 or 8; 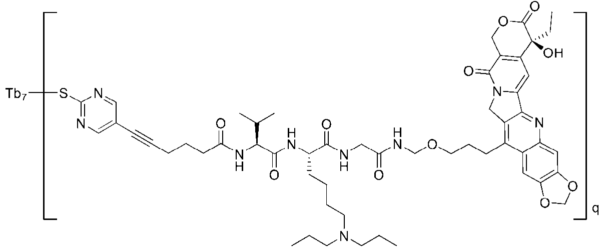
   wherein Tb₇ is an antibody with weak or no tumor cell endocytosis activity but with tumor cell surface antigen binding activity; for example, the antibodies are antibodies that have activity binding to B7H3, Her3, GD-2, Trop-2, EGFR, CD19, CD30, GPNMB, CD20, CD79b, and BCMA antigens but have no endocytic activity; q is selected from any numerical value between 0.1 and 16.0, preferably from any numerical value between 2 and 8, more preferably q is 2, 4, 6 or 8; 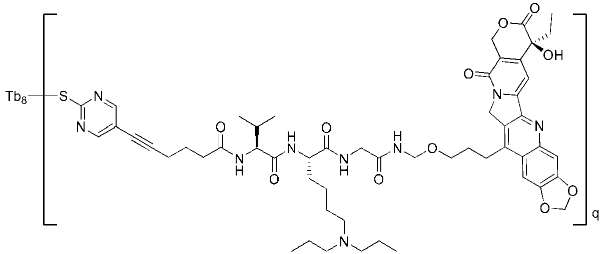
   wherein Tb₈ is an antibody having tumor cell endocytosis activity and tumor cell surface antigen binding activity, such as 1D1-01, 2E3-02 antibody, sacituzumab, pertuzumab, Trastuzumab, or Cetuximab antibody; q is selected from any numerical value between 0.1 and 16.0, preferably from any numerical value between 2 and 8, more preferably, q is 2, 4, 6 or 8.
25. A linker in a ligand drug conjugate, comprising the following fragments: 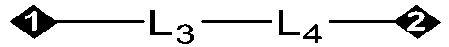
   wherein position 2 is attached to the bioactive molecular fragment;
   L₃, L₄ are defined as in any one of clauses 1 to 24;
   preferably, the structure thereof is as follows: 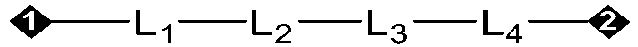
   wherein position 1 is attached to a ligand or targeting moiety that binds to a target, and position 2 is attached to a bioactive molecule fragment;
   L₃, L₄ are defined as in any one of clauses 1 to 24;
   preferably, said ligand or targeting moiety that binds to a target and said bioactive molecule fragment are defined as for Tb and D in any one of clauses 1 to 24, respectively.
26. A compound represented by formula II: 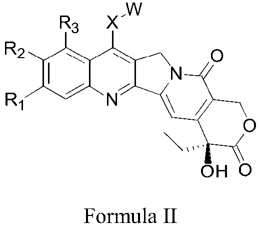
   or a stereoisomer of said compound, a prodrug thereof, a pharmaceutically acceptable salt thereof, a pharmaceutically acceptable solvate thereof, or a ligand drug conjugate thereof,
   wherein R₁, R₂, R₃ and X are as defined in any one of clauses 13 to 24;
   W is absent or present, and when W is present, W is selected from -OH, -SH, -NHR₄, 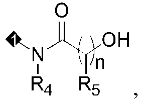 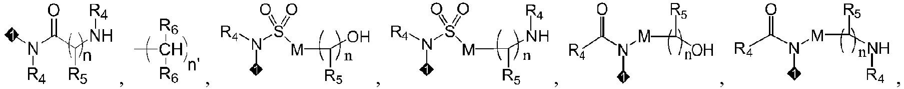 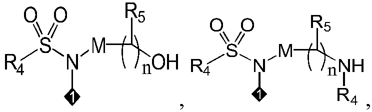 position 1 is attached to X;
   preferably, W is absent or present, and when W is present, W is selected from -OH, -SH, -NHR₄, 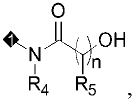 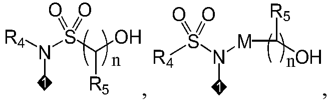 and position 1 is attached to X;
   and when W is absent, X is attached to H; wherein when R₁ and R₂ are both H, and X is -(CH₂)ₙ₁-, and n1 is 1, 2, 3, 4, then W is not -OH or-NHR₄; and the compound of formula II does not comprise 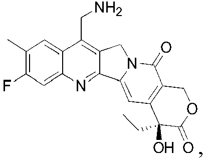 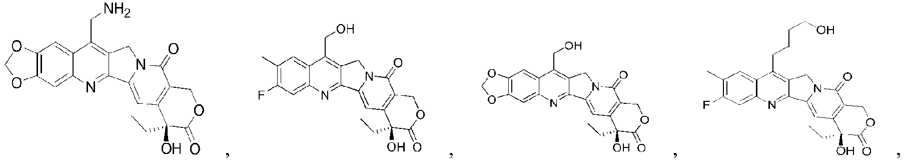 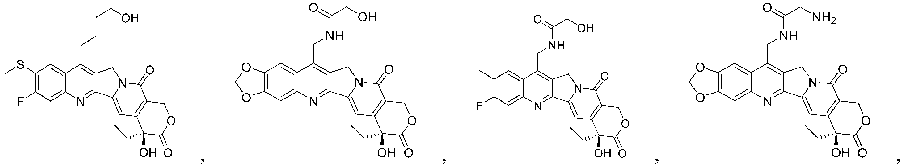 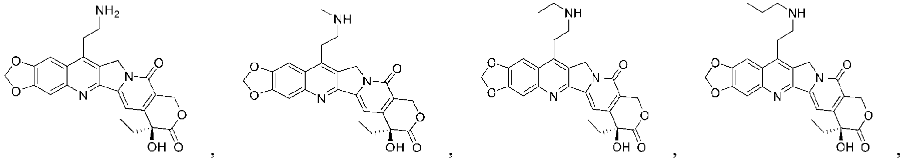 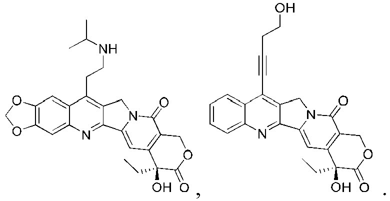
27. The compound of clause 26, wherein the compound of formula II is selected from: 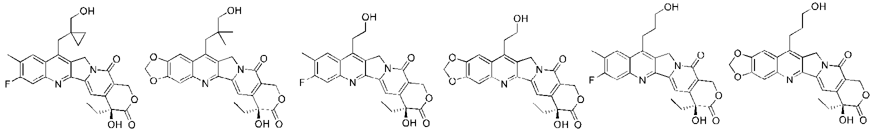 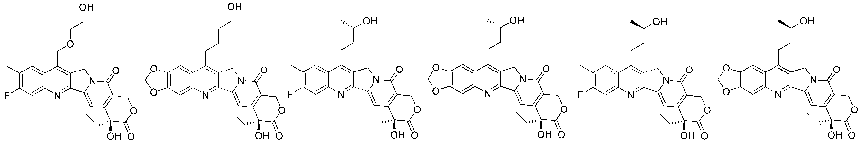 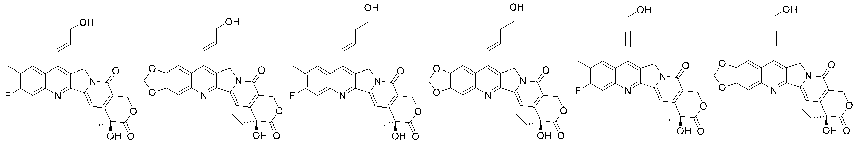 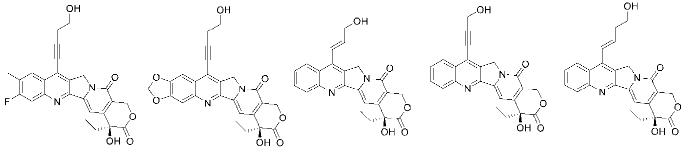 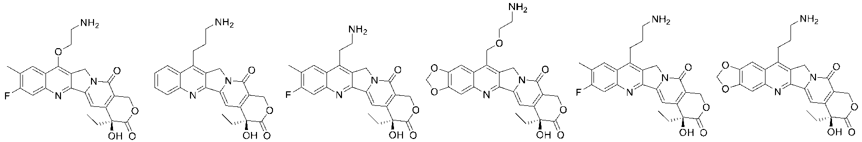 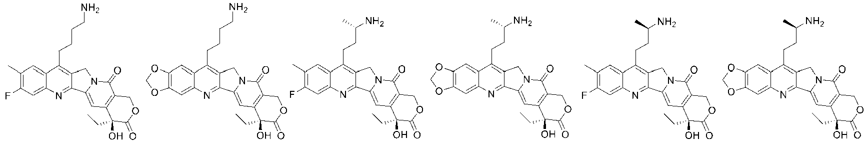 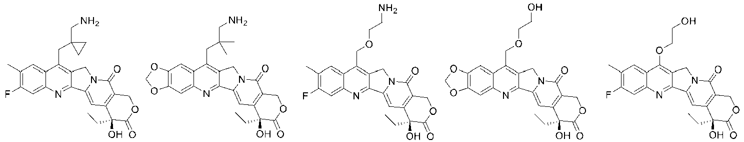 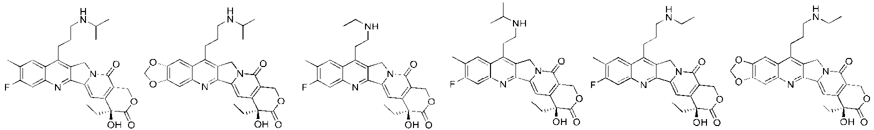 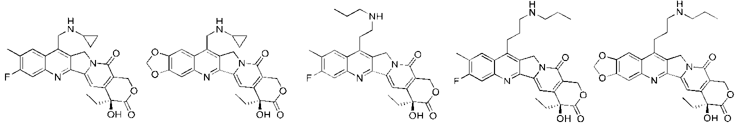 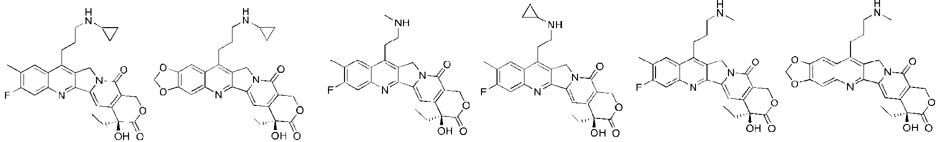 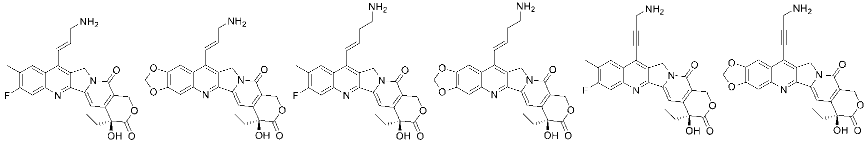 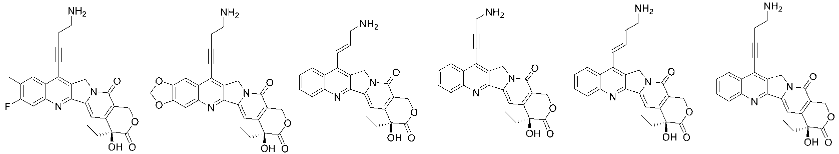 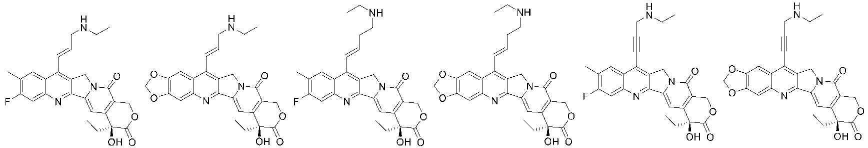 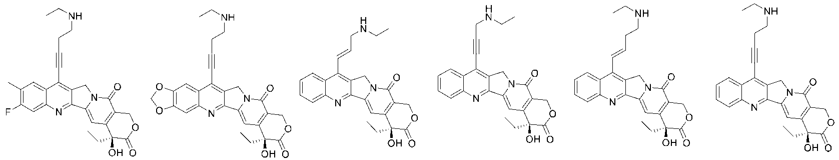 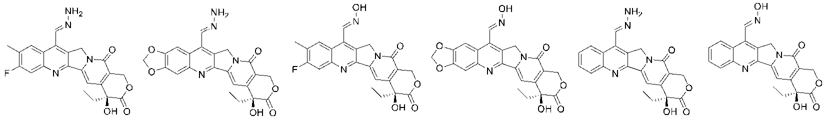 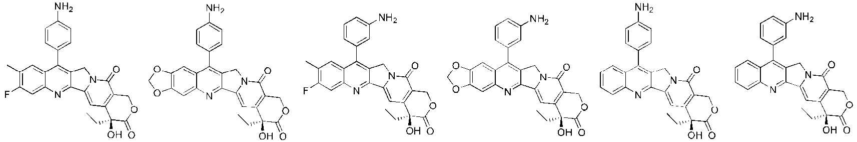 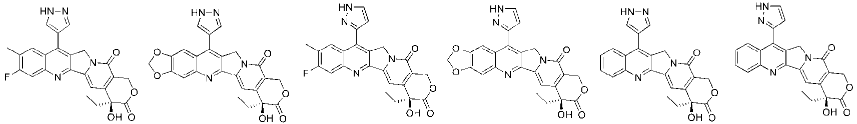 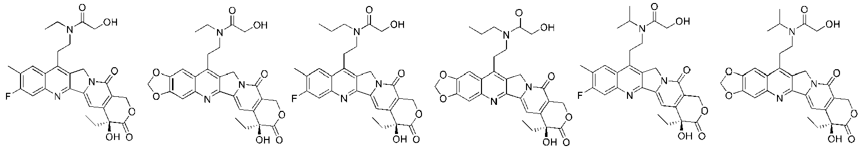 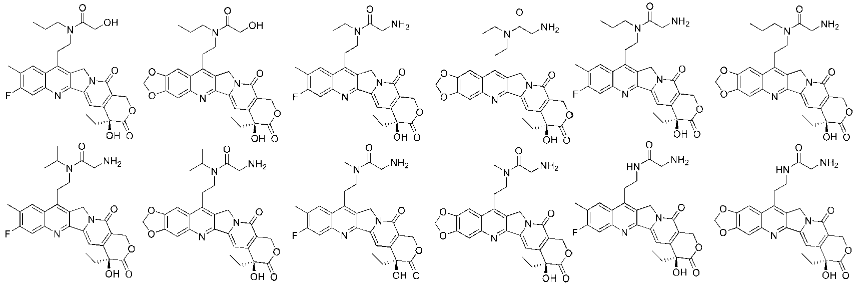 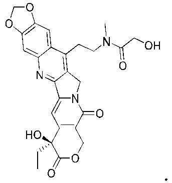
28. A drug linker conjugate of formula III, 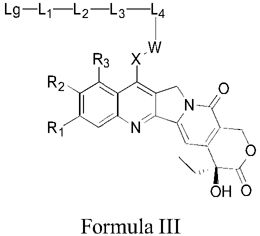
   or a stereoisomer of said drug linker conjugate, a prodrug thereof, a pharmaceutically acceptable salt thereof, or a pharmaceutically acceptable solvate thereof,
   wherein:
      R₁, R₂, R₃, X, L₁, L₂, L₃, and L₄ are as defined in any one of clauses 1 to 24; W is as defined in any one of clauses 13 to 24; position 1 of L1 is attached to Lg;
      Lg is a leaving group and is selected from halogen, sulfone group, tertiary amine salt group (Me₃N⁺, Et₃N⁺), diazonium salt group, -OMs, MeSO₂-, and CF₃SO₃-;
      preferably, Lg is selected from F, Cl and MeSO₂-; the tertiary amine salt group is selected from Me₃N⁺ and Et₃N⁺;
      more preferably, Lg is selected from F and MeSO₂-.
29. The drug linker conjugate according to clause 28, or a stereoisomer of said drug linker conjugate, a prodrug thereof, a pharmaceutically acceptable salt thereof, or a pharmaceutically acceptable solvate thereof, characterized in that it has a structure of formula III-(1), formula III-(2), or formula III-(3): 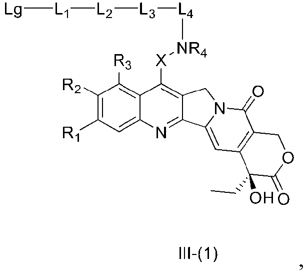
   wherein L₁, L₂, L₃, L₄, X, R₁, R₂, R₃, R₄, and Lg are as defined in clause 28; 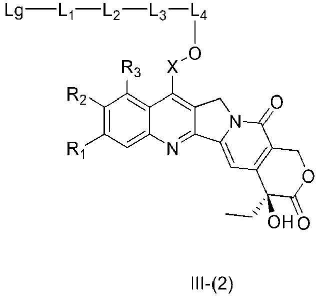
   wherein L₁, L₂, L₃, L₄, X, R₁, R₂, R₃, and Lg are as defined in clause 28, 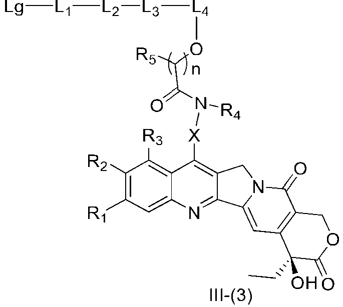
   wherein L₁, L₂, L₃, L₄, X, R₁, R₂, R₃, R₄, R₅, n, and Lg are as defined in clause 28.
30. The drug linker conjugate according to clause 28 or 29, or a stereoisomer of said drug linker conjugate, a prodrug thereof, a pharmaceutically acceptable salt thereof, or a pharmaceutically acceptable solvate thereof, characterized in that it has a structure of formula III-(1A), III-(1B), III-(2A), III-(2B), III-(3A), or III-(3B);
   the structure of formula III-(1A) or III-(1B): 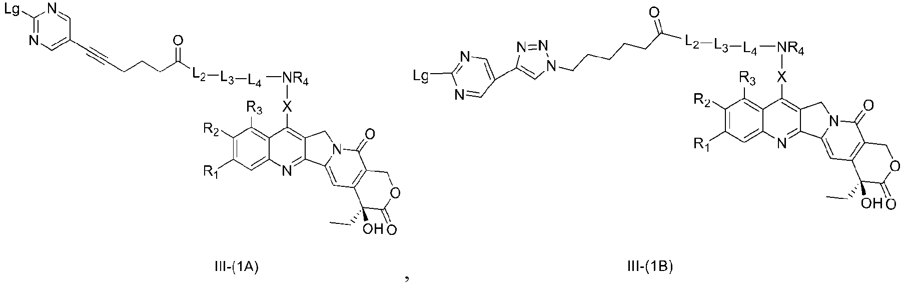
   Wherein L₂, L₃, L₄, X, R₁, R₂, R₃, R₄, and Lg are as defined in clause 28 or 29;
   the structure of formula III-(2A) or III-(2B): 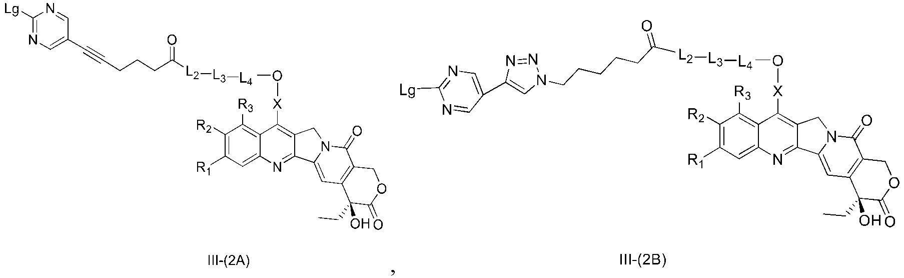
   wherein L₂, L₃, L₄, X, R₁, R₂, R₃, and Lg are as defined in clause 28 or 29;
   the structure of formula III-(3A) or III-(3B): 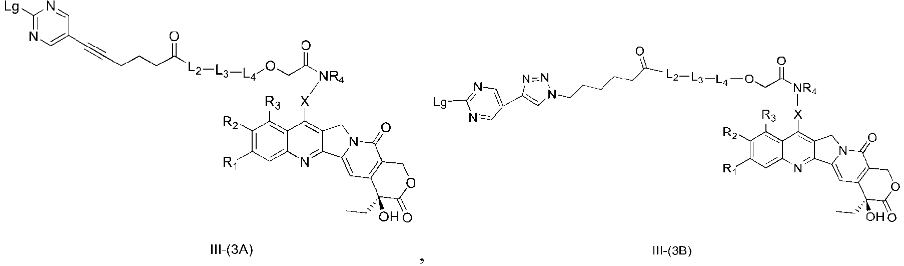
   wherein L₂, L₃, L₄, X, R₁, R₂, R₃, R₄, and Lg are as defined in clause 28 or 29.
31. The drug linker conjugate according to any one of clauses 28 to 30, or a stereoisomer of said drug linker conjugate, a prodrug thereof, a pharmaceutically acceptable salt thereof, or a pharmaceutically acceptable solvate thereof, characterized in that the drug linker conjugate has a structure of formula III-A or formula III-B: 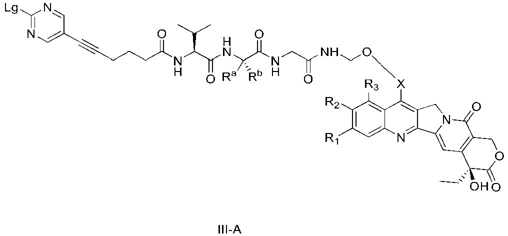
   wherein Lg, X, R₁, R₂, R₃, R^{a}, R^{b}, and q are as defined in any one of clauses 28 to 30; 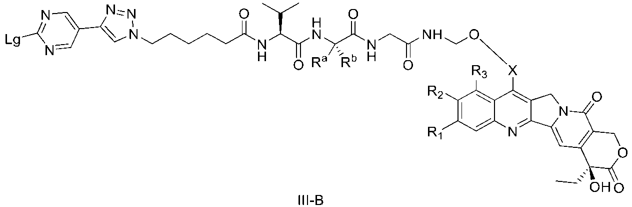
   wherein Lg, X, R₁, R₂, R₃, R^{a}, R^{b}, and q are as defined in any one of clauses 28 to 30.
32. The drug linker conjugate according to any one of clauses 28 to 31, or a stereoisomer of said drug linker conjugate, a prodrug thereof, a pharmaceutically acceptable salt thereof, or a pharmaceutically acceptable solvate thereof, characterized in, wherein, the drug linker conjugate is selected from the following: 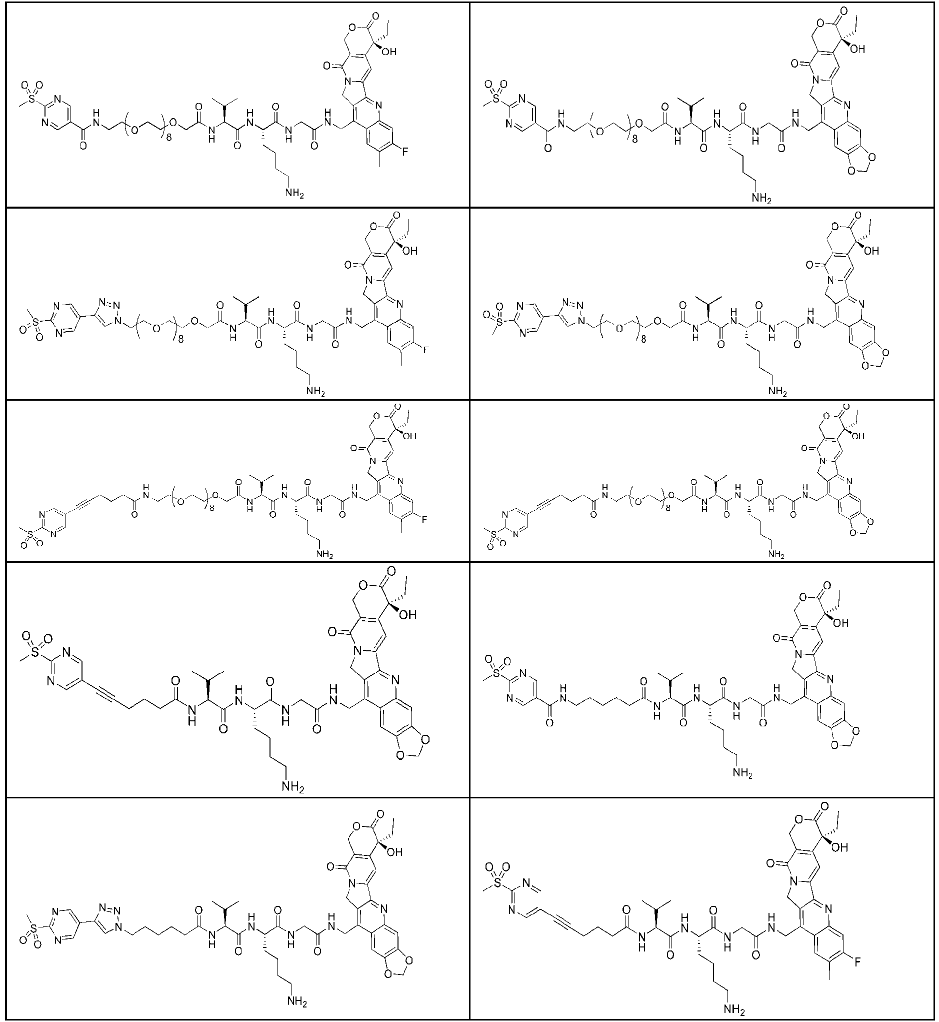 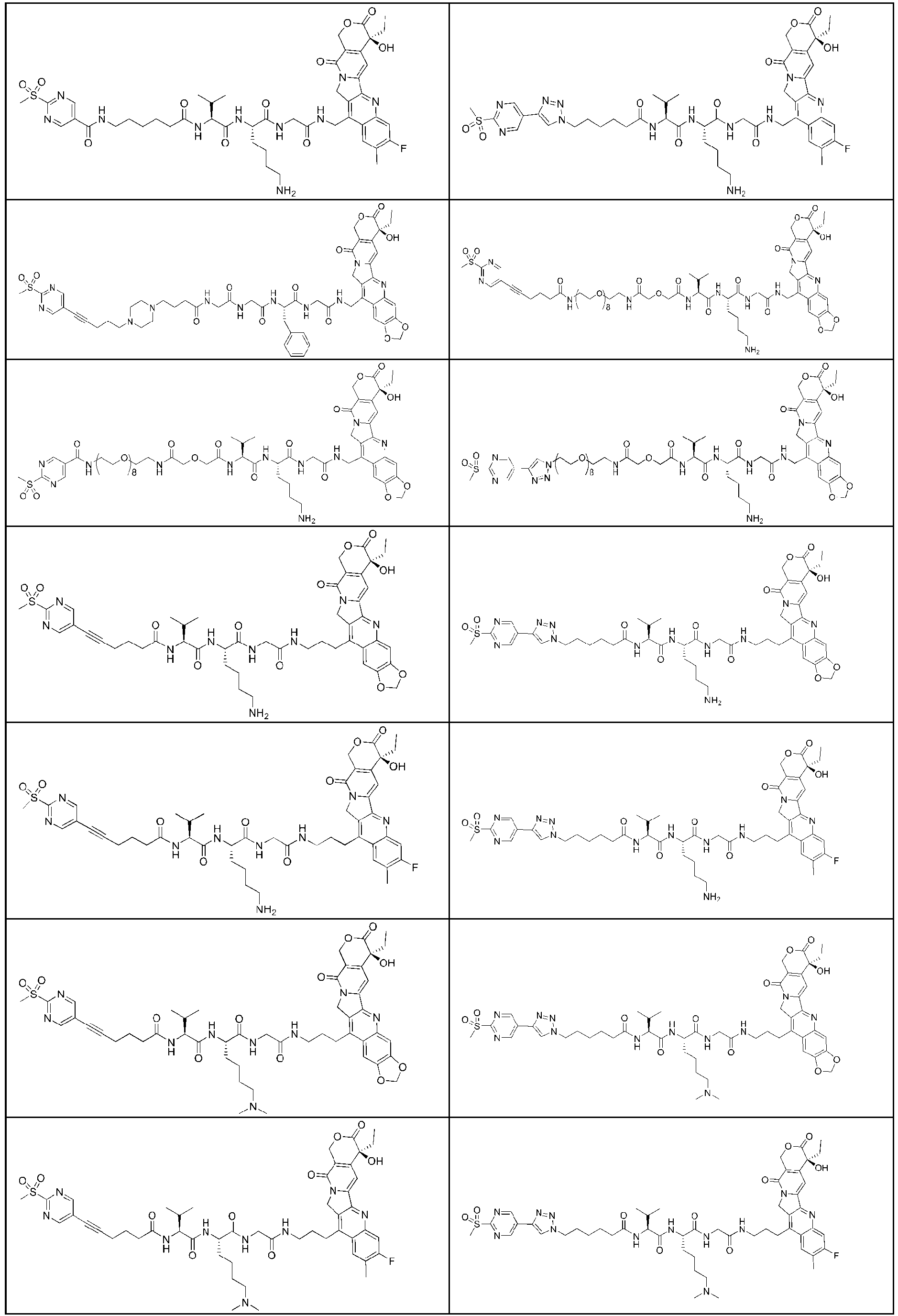 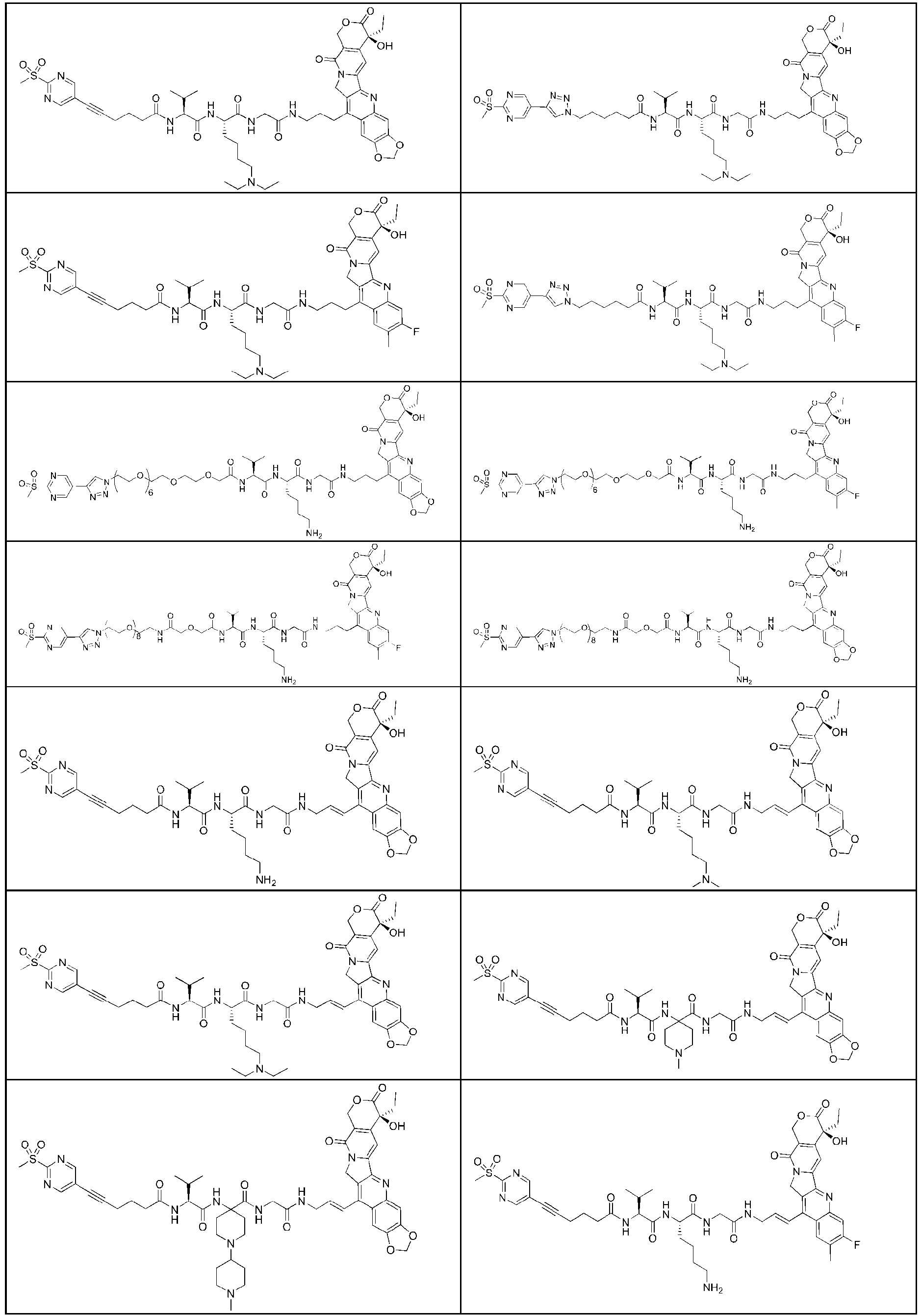 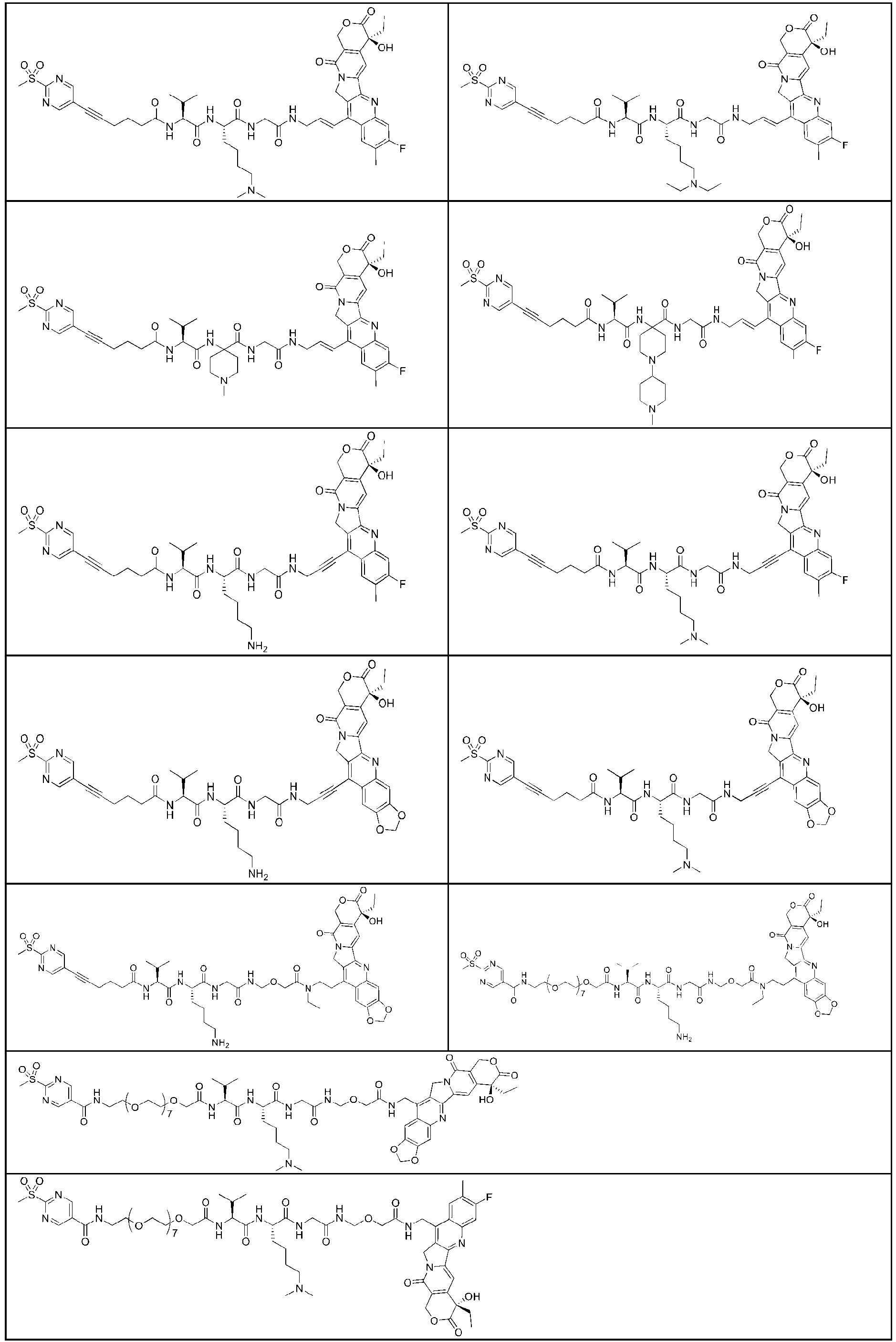 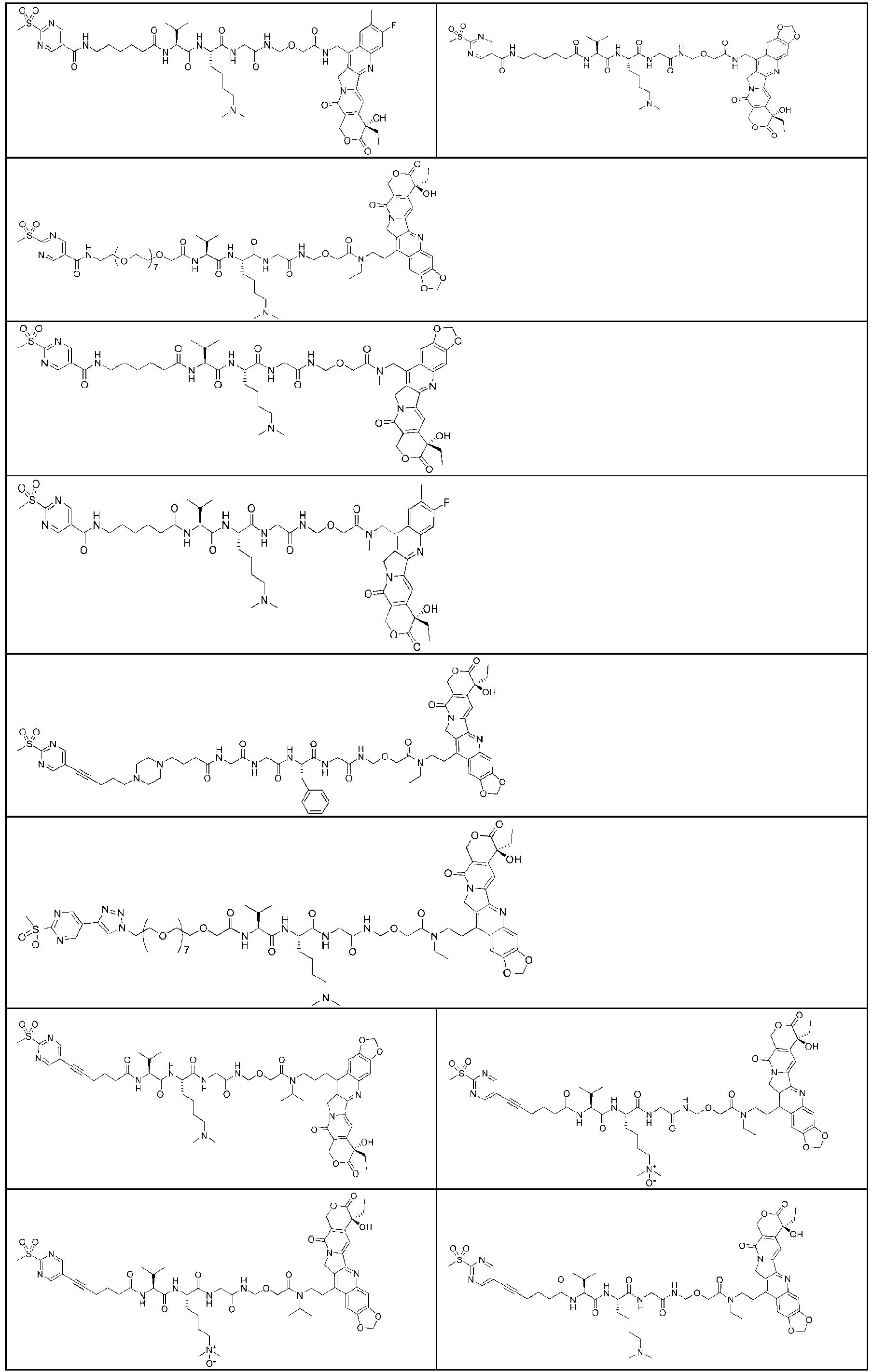 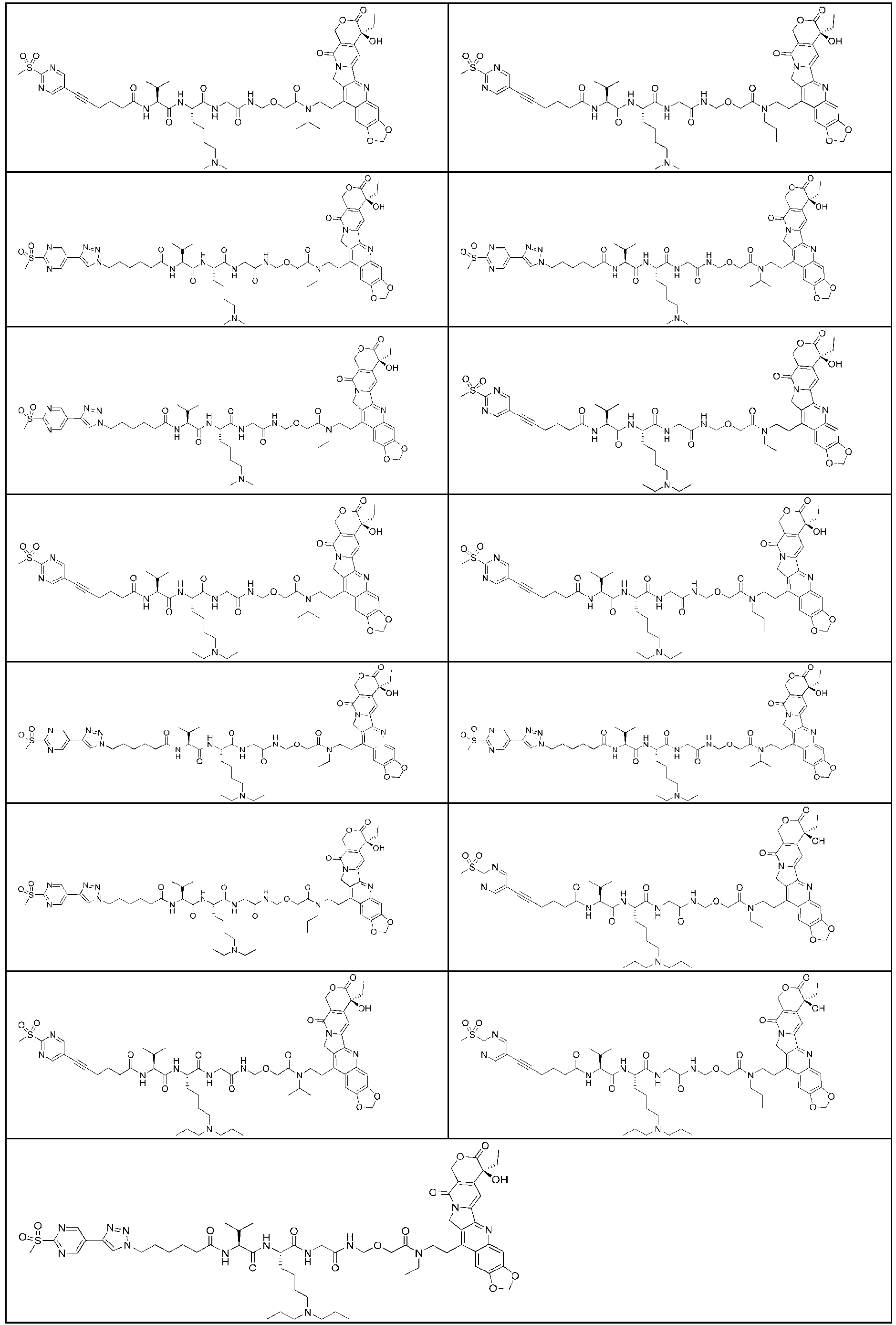 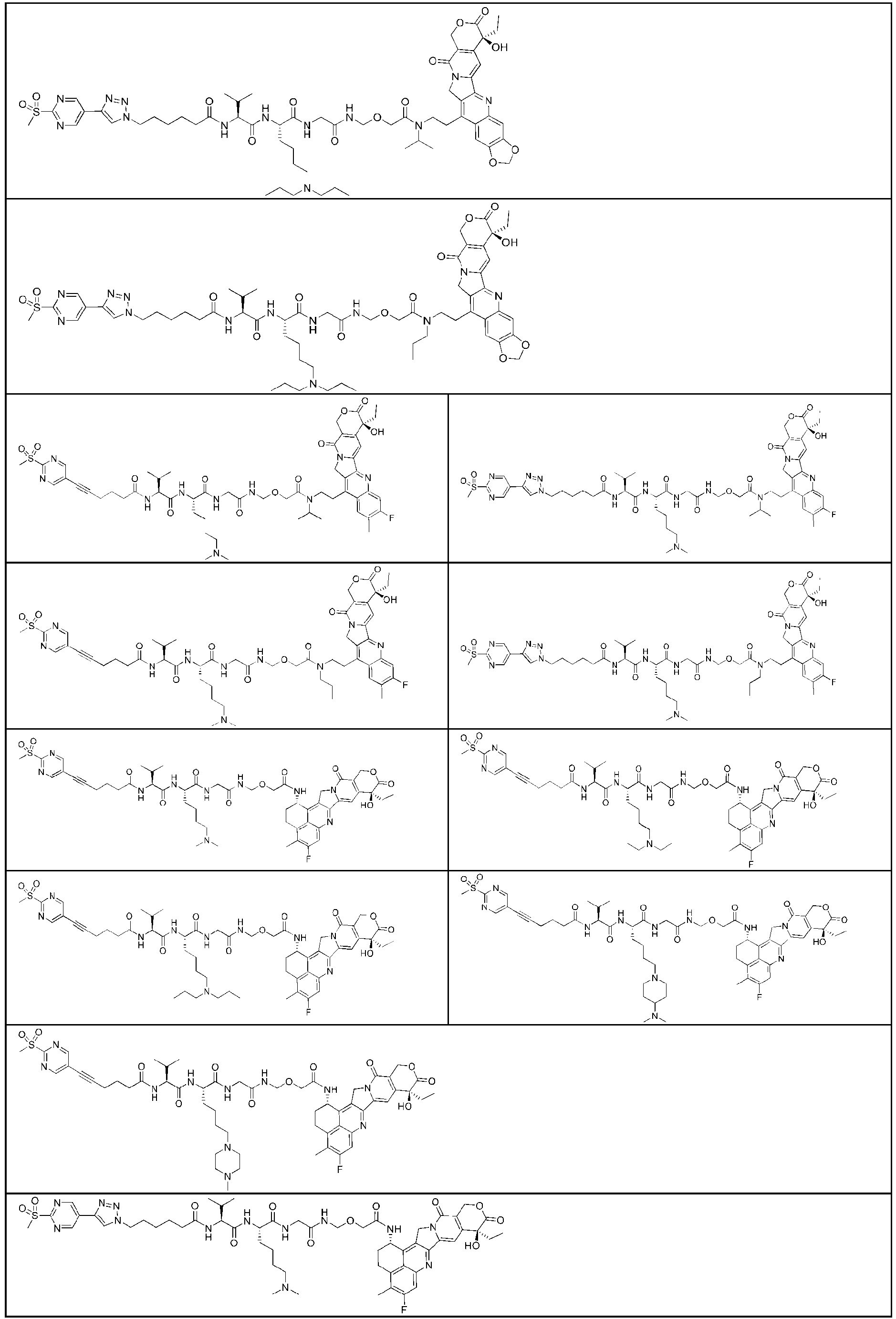 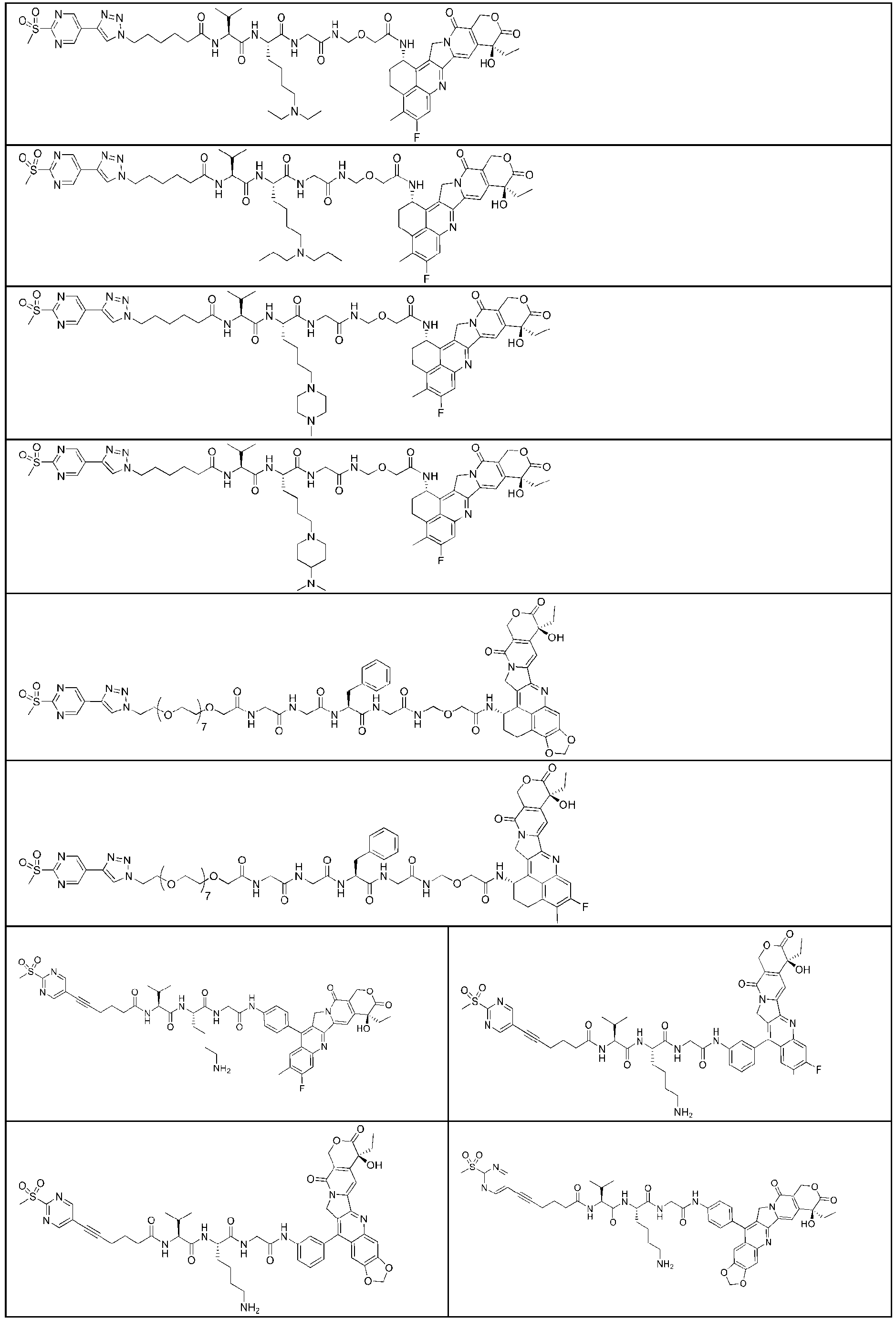 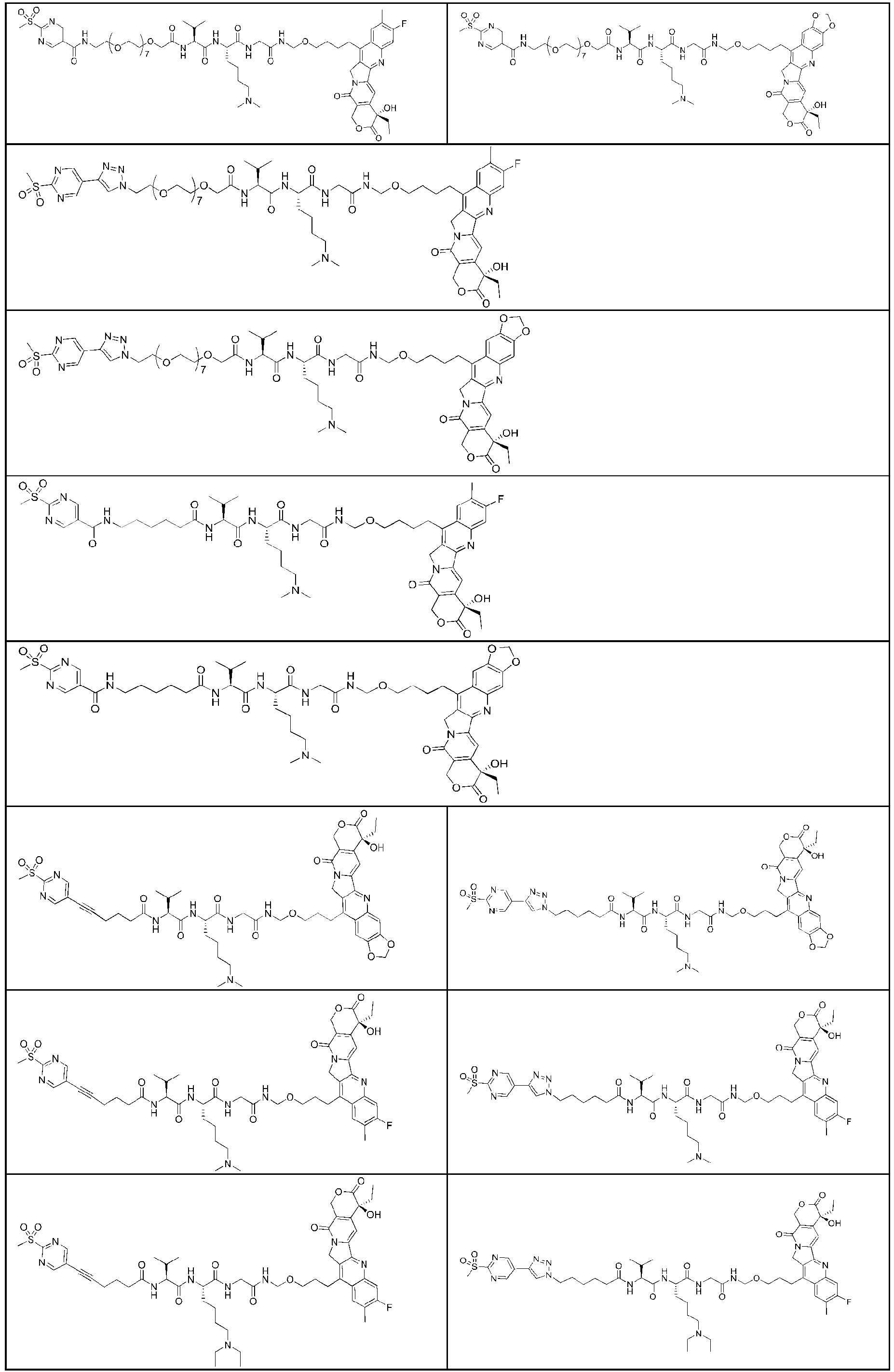 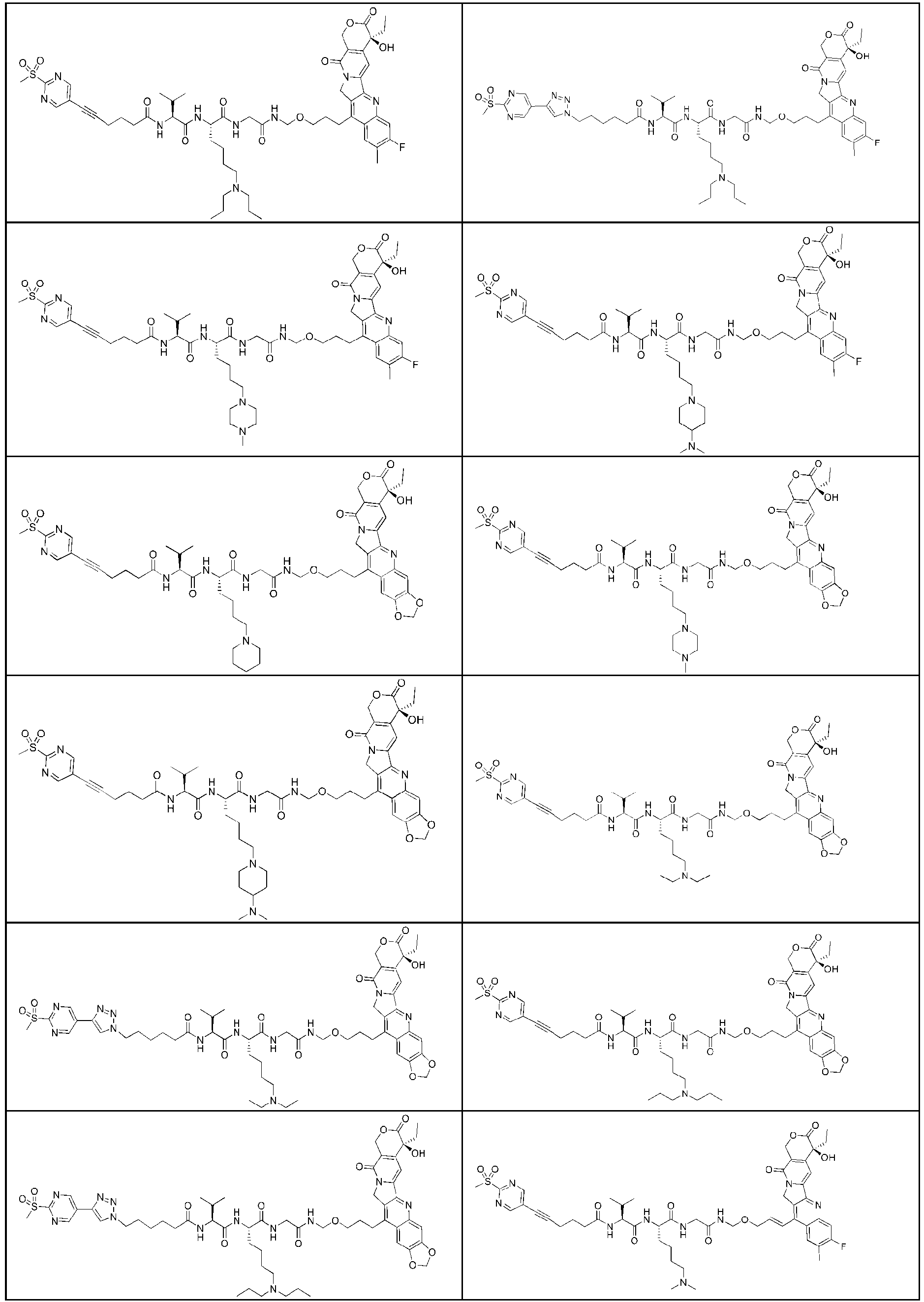 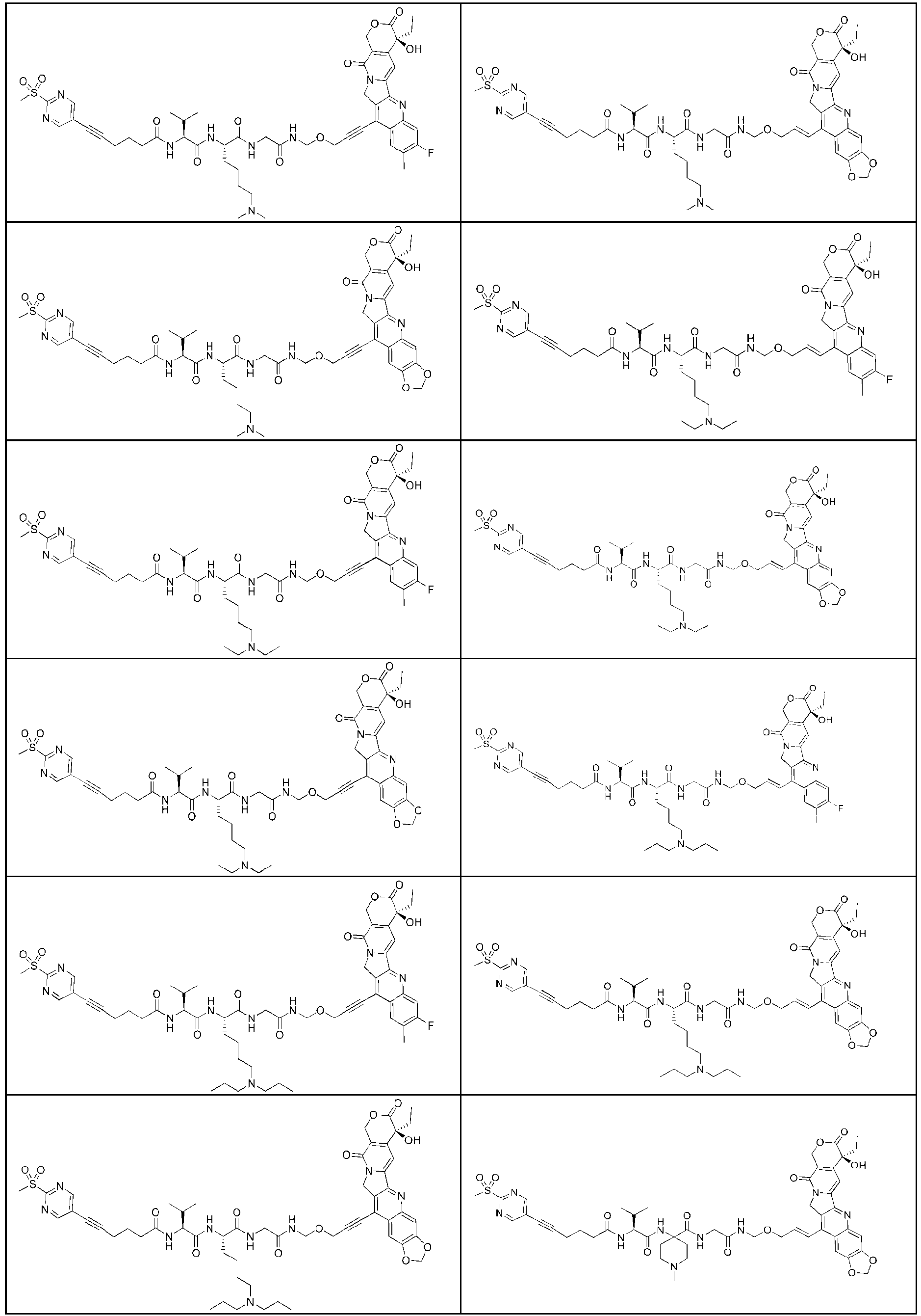 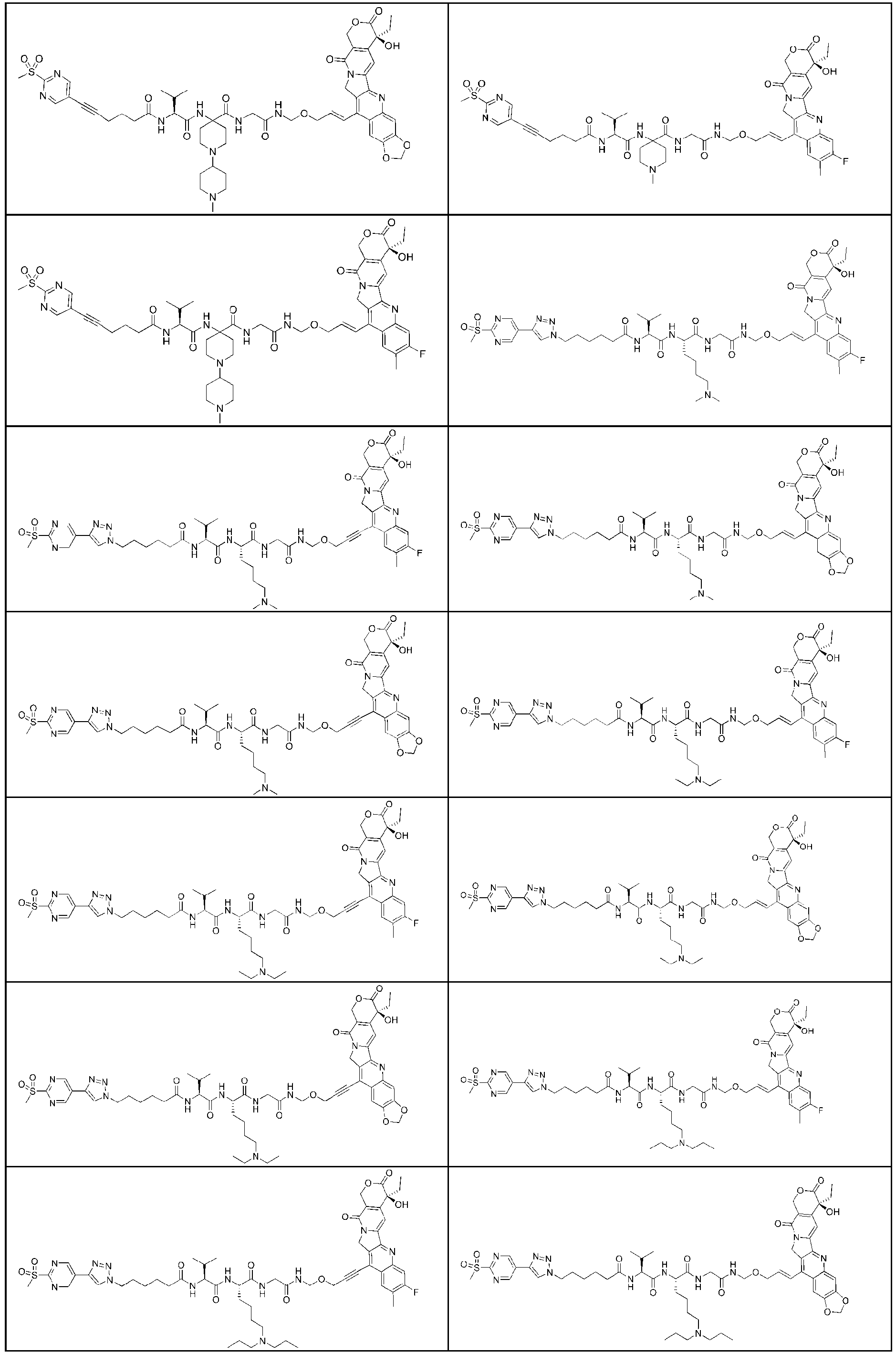 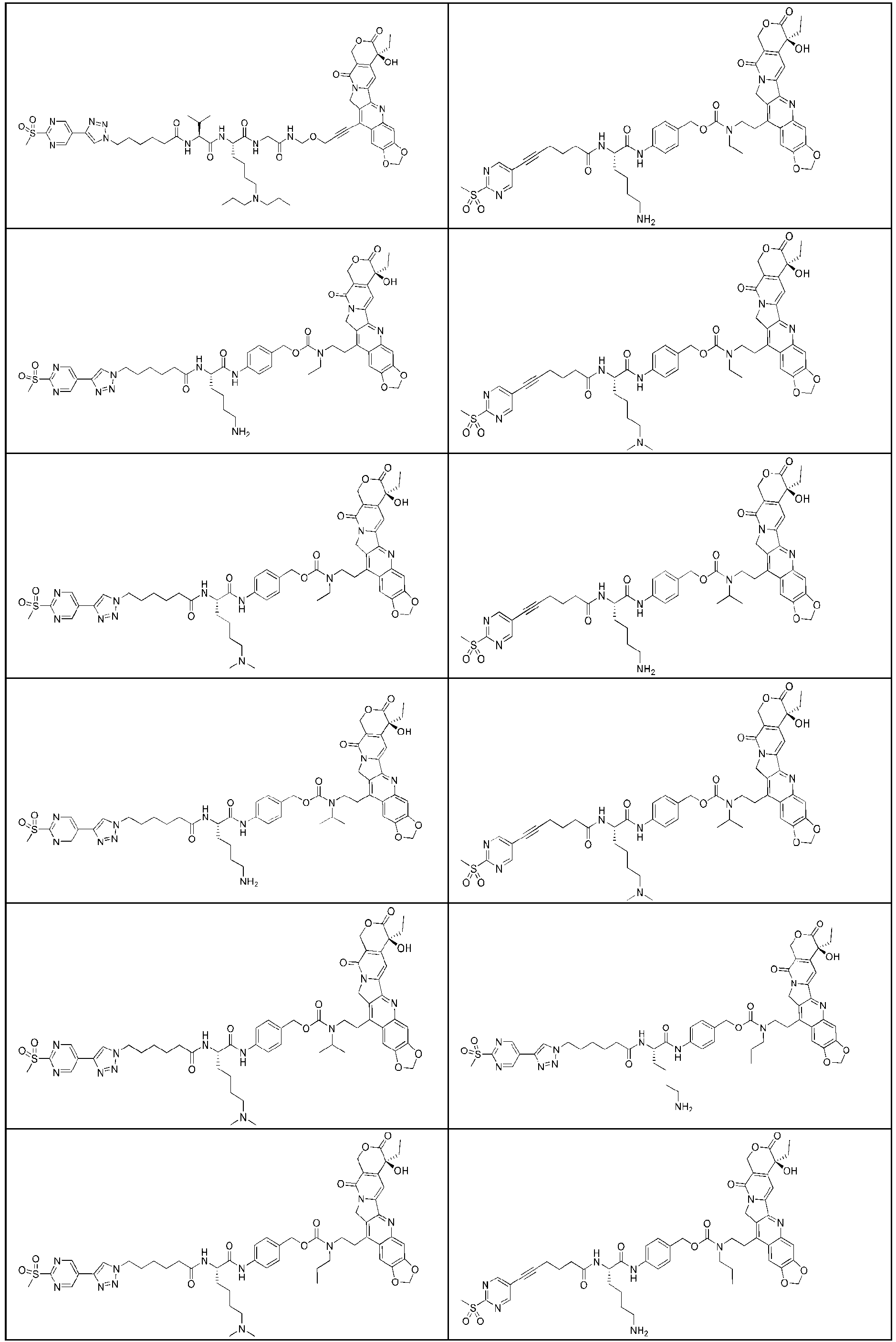 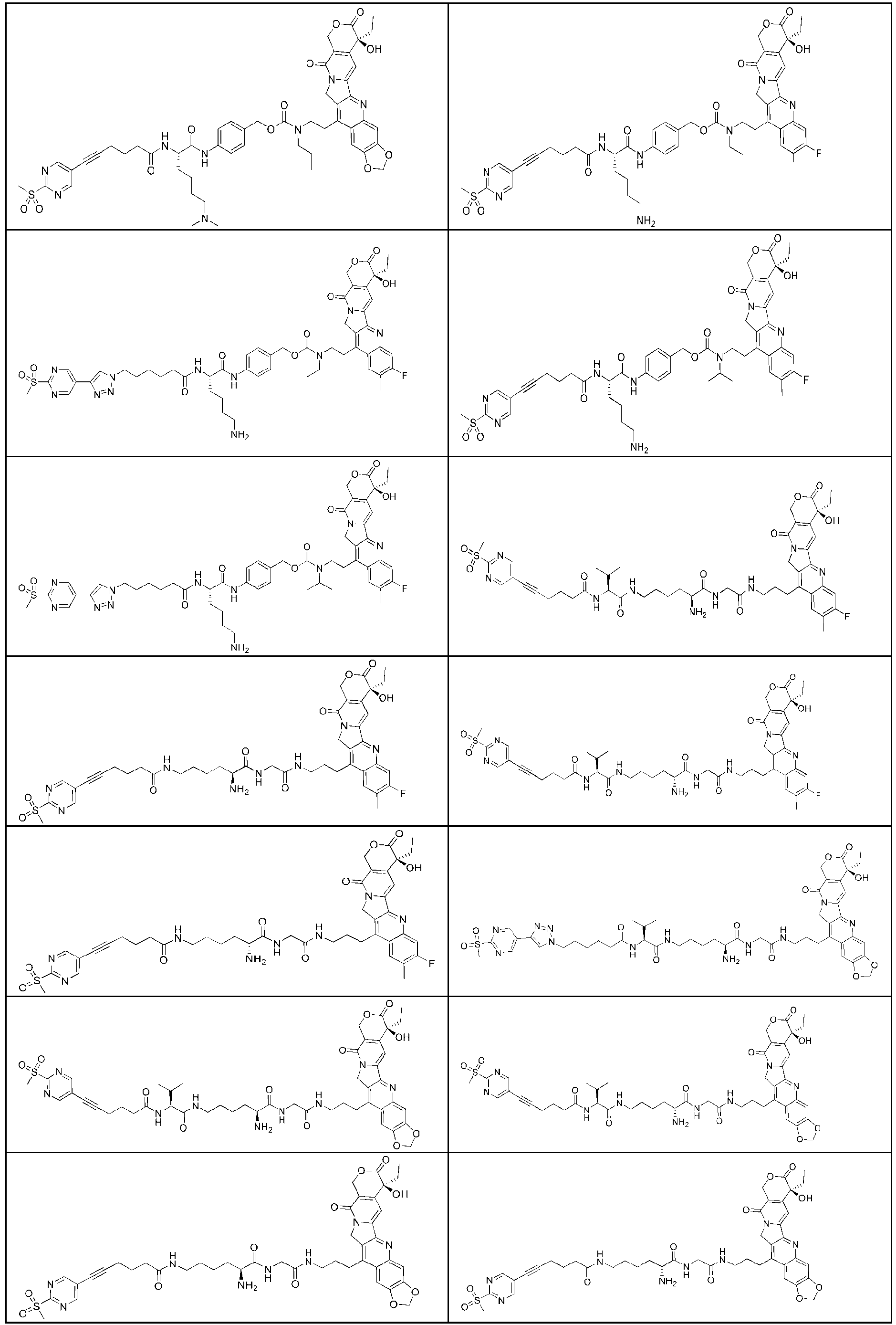 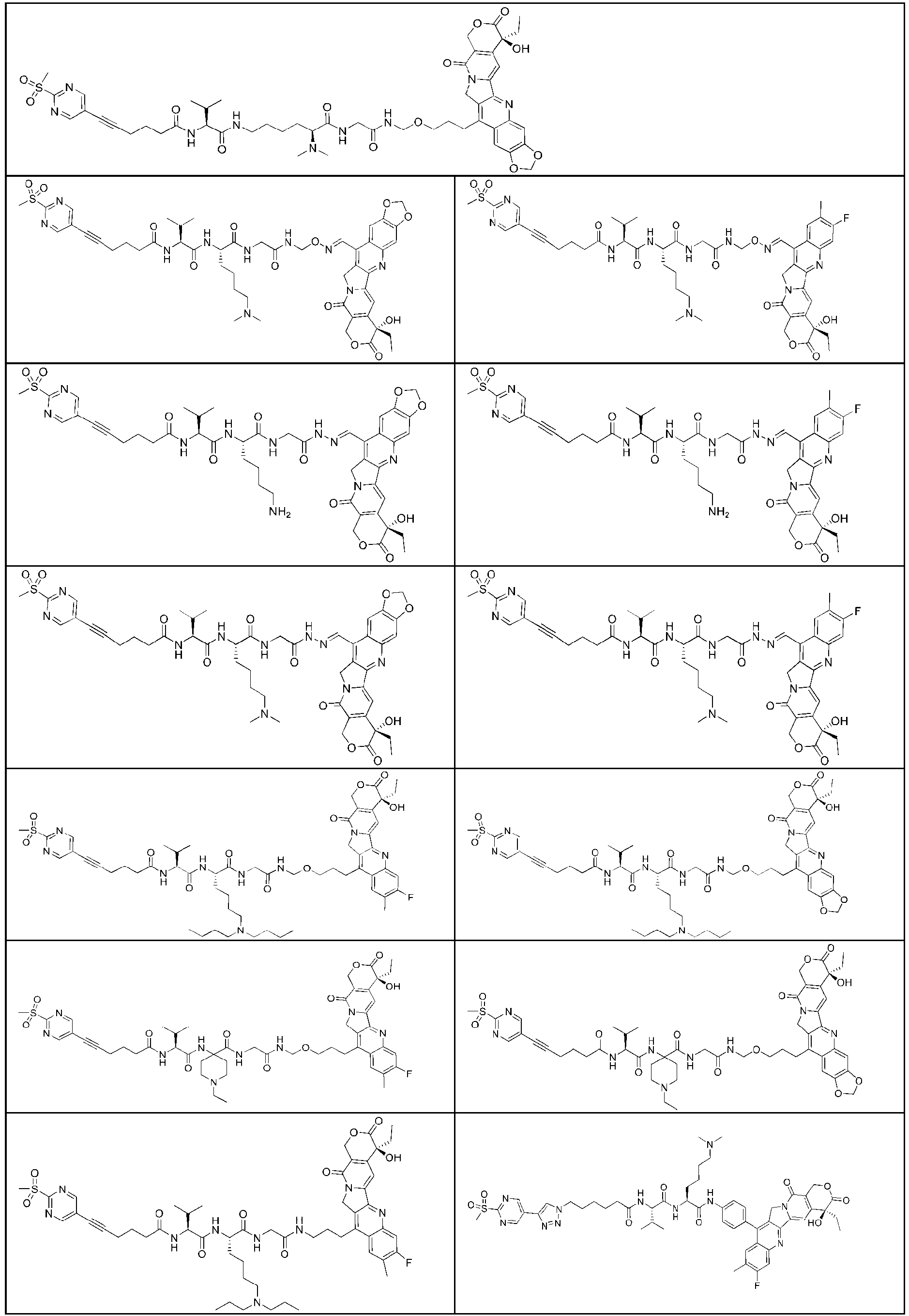 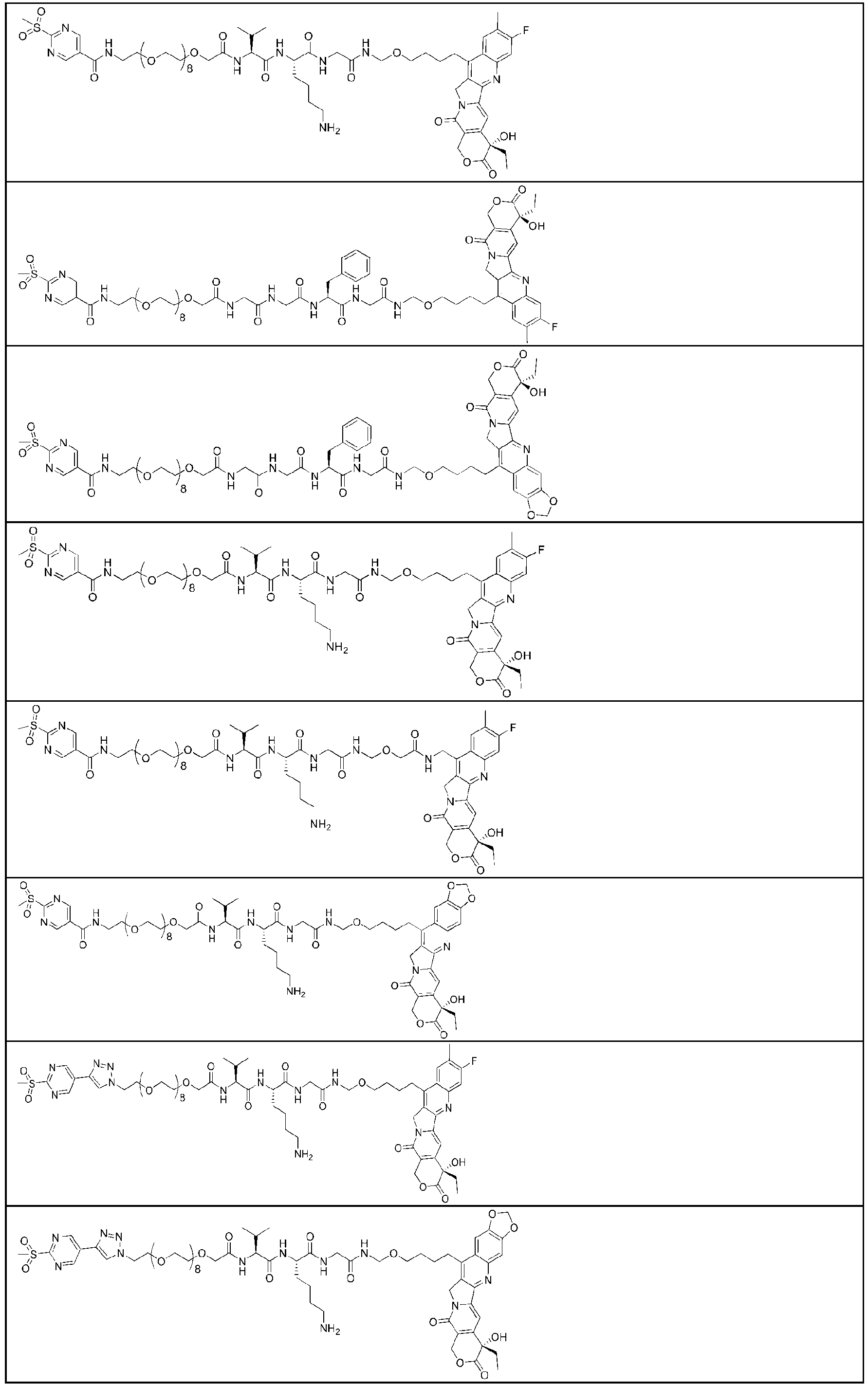 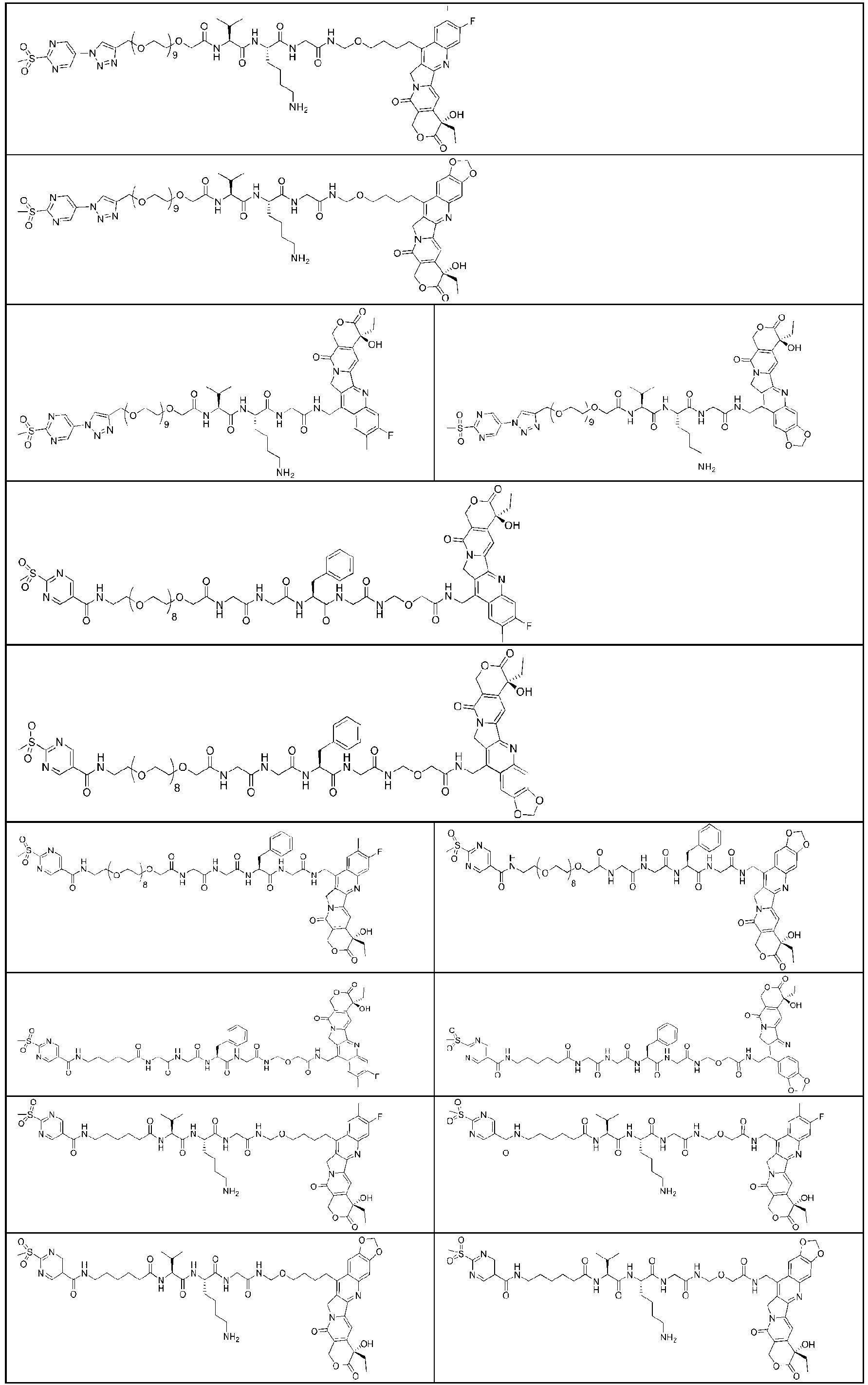 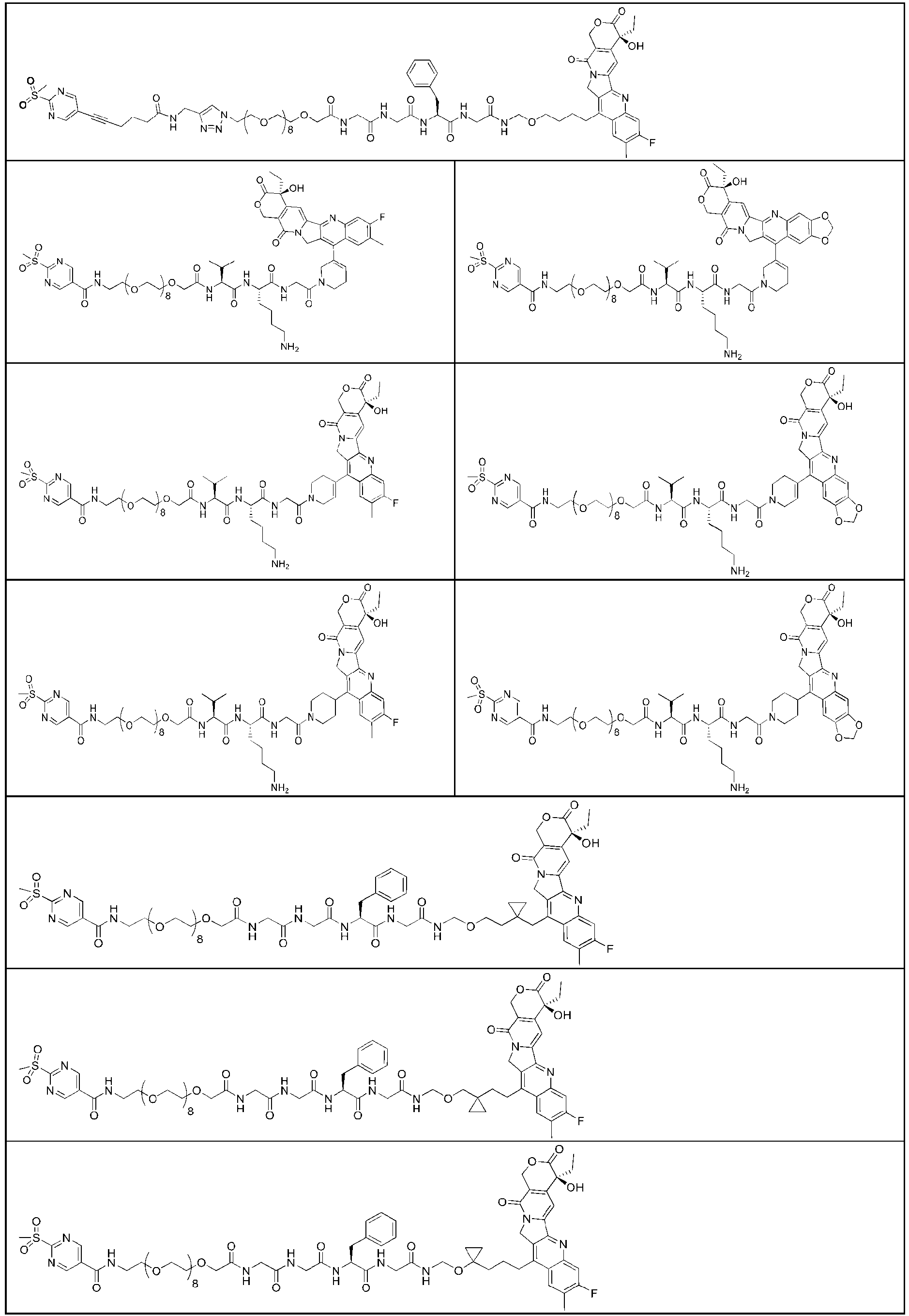 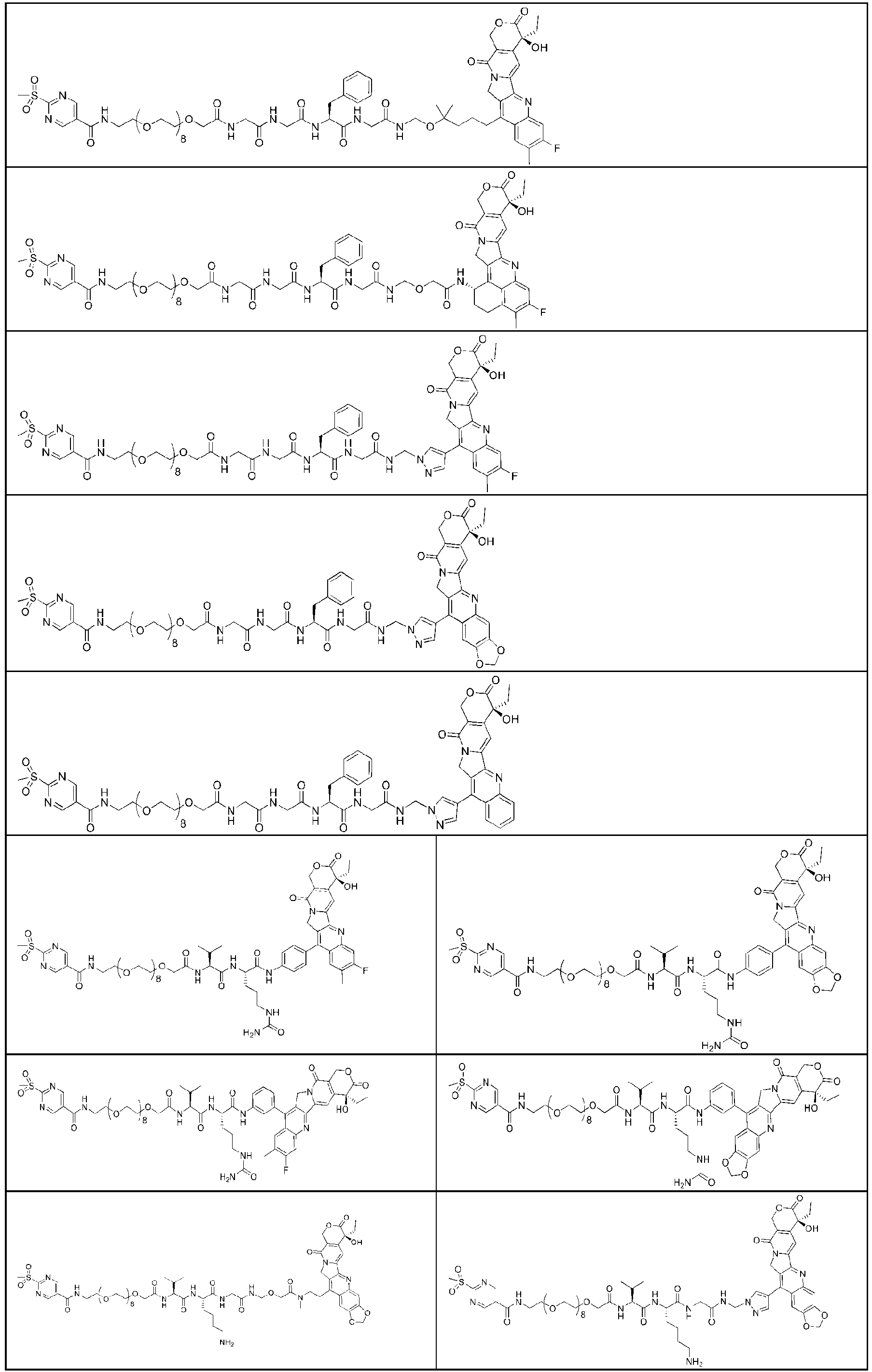 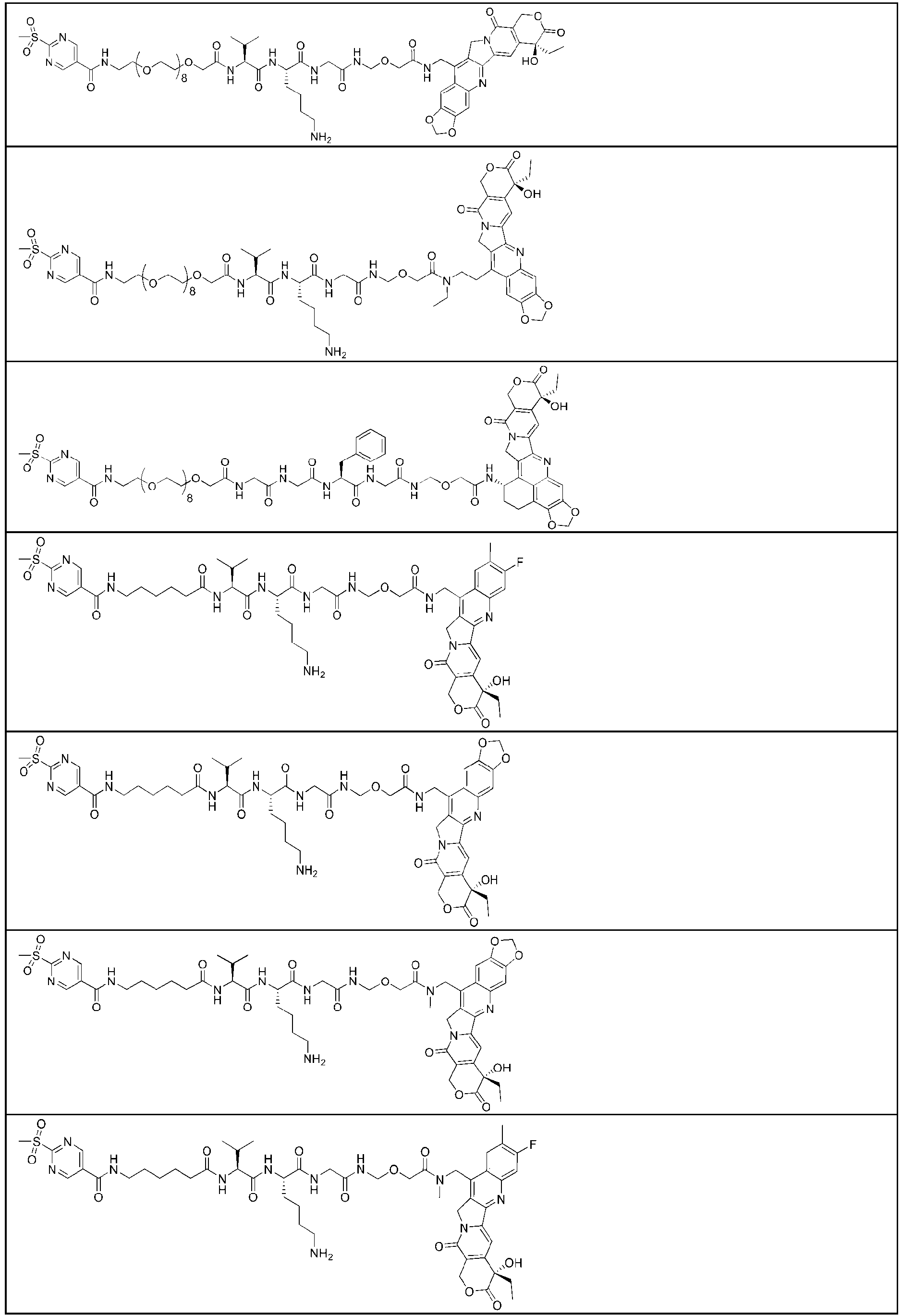 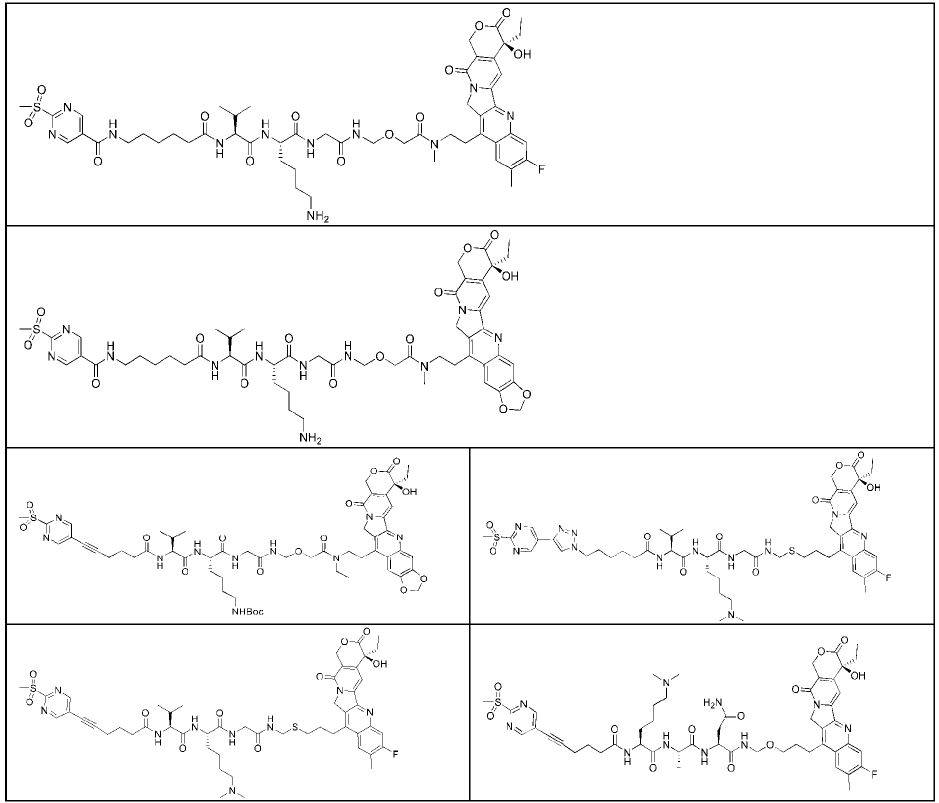
33. A process for preparing the ligand drug conjugate according to any one of clauses 28 to 32, which comprises:
   performing a coupling reaction of Tb and a drug linker conjugate of formula III 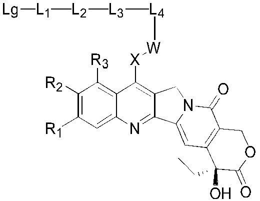 in a suitable solvent under suitable conditions;
   wherein:
      L₁, L₂, L₃, L₄ and Tb are as defined in any one of clauses 1 to 24;
      R₁, R₂, R₃, X and W are as defined in clauses 13 to 24;
   Lg is as defined in any one of clauses 28 to 32.
34. The process of clause 33, wherein the process comprises the step of performing a coupling reaction of Tb and drug linker conjugate of formula III 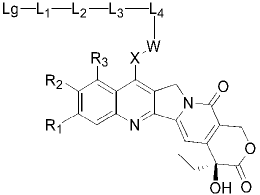 in a suitable solvent and under suitable conditions to form a C-S bond;
   preferably, the ratio of said Tb to said drug linker conjugate in amount of substance is 1: (1-20);
   preferably, the coupling reaction is carried out in water and/or an organic solvent; preferably, the organic solvent is selected from N, N-dimethylformamide, dimethylsulfoxide, N-methylpyrrolidone, nitriles (e.g. acetonitrile), alcohols (e.g. methanol, ethanol) or any combination thereof; the nitrile can be acetonitrile, and the alcohol can be methanol, ethanol;
   preferably, the process further comprises the step of purifying the coupling product; preferably, the coupling product is purified by chromatography; preferably, the chromatography comprises one or more of ion exchange chromatography, hydrophobic chromatography, reverse phase chromatography or affinity chromatography.
35. An antibody or antigen-binding fragment thereof that binds B7H3, said antiboy or antigen-binding fragment comprises the complementarity determining regions CDRs as follows:
   (a) HCDR1 or a variant of its sequence, HCDR2 or a variant of its sequence, and HCDR3 or a variant of its sequence as comprised in the heavy chain variable region VH set forth in SEQ ID NO:3 or 23, and/or
   (b) LCDR1 or a variant of its sequence, LCDR2 or a variant of its sequence, and LCDR3 or a variant of its sequence as comprised in the light chain variable region VL set forth in SEQ ID NO: 13 or 33;
   preferably, the variant of said sequence is a CDR which has one or more amino acid substitutions, deletions or additions, e.g. 1, 2 or 3 amino acid substitutions, deletions or additions, compared with the CDR from which it is derived; preferably, the substitution is a conservative substitution.
36. The antibody or antigen-binding fragment thereof of clause 35, wherein the antibody or antigen-binding fragment thereof comprises:
   (1) VH and/or VL, wherein defined by IMGT numbering system:
      (a) the VH comprises: HCDR1 consisting of the sequence of SEQ ID NO: 10, HCDR2 consisting of the sequence of SEQ ID NO: 11, HCDR3 consisting of the sequence of SEQ ID NO: 12; and/or
         the VL comprises: LCDR1 consisting of the sequence of SEQ ID NO: 20, LCDR2 consisting of the sequence GTF, and LCDR3 consisting of the sequence of SEQ ID NO: 22;
      (b) the VH comprises: HCDR1 consisting of the sequence of SEQ ID NO: 10, HCDR2 consisting of the sequence of SEQ ID NO: 11, HCDR3 consisting of the sequence of SEQ ID NO: 12; and/or
         the VL comprises: LCDR1 consisting of the sequence of SEQ ID NO: 40, LCDR2 consisting of the sequence GAS, and LCDR3 consisting of the sequence of SEQ ID NO: 42;
      (c) the VH comprises: HCDR1 consisting of the sequence of SEQ ID NO: 30, HCDR2 consisting of the sequence of SEQ ID NO: 31, HCDR3 consisting of the sequence of SEQ ID NO: 32; and/or
         the VL comprises: LCDR1 consisting of the sequence of SEQ ID NO: 40, LCDR2 consisting of the sequence GAS, and LCDR3 consisting of the sequence of SEQ ID NO: 42;
         or
      (d) the VH comprises: HCDR1 consisting of the sequence of SEQ ID NO: 30, HCDR2 consisting of the sequence of SEQ ID NO: 31, HCDR3 consisting of the sequence of SEQ ID NO: 32; and/or
      the VL comprises: LCDR1 consisting of the sequence of SEQ ID NO: 20, LCDR2 consisting of the sequence GTF, and LCDR3 consisting of the sequence of SEQ ID NO: 22;
   (2) VH and/or VL, wherein defined by Chothia numbering system:
      (a) the VH comprises: HCDR1 consisting of the sequence of SEQ ID NO: 4, HCDR2 consisting of the sequence of SEQ ID NO: 5, HCDR3 consisting of the sequence of SEQ ID NO: 6; and/or
         the VL comprises: LCDR1 consisting of the sequence of SEQ ID NO: 14, LCDR2 consisting of the sequence of SEQ ID NO:15, and LCDR3 consisting of the sequence of SEQ ID NO: 16;
      (b) the VH comprises: HCDR1 consisting of the sequence of SEQ ID NO: 24, HCDR2 consisting of the sequence of SEQ ID NO: 25, HCDR3 consisting of the sequence of SEQ ID NO: 26; and/or the VL comprises: LCDR1 consisting of the sequence of SEQ ID NO: 34, LCDR2 consisting of the sequence of SEQ ID NO:35, and LCDR3 consisting of the sequence of SEQ ID NO: 36;
      (c) the VH comprises: HCDR1 consisting of the sequence of SEQ ID NO: 4, HCDR2 consisting of the sequence of SEQ ID NO: 5, HCDR3 consisting of the sequence of SEQ ID NO: 6; and/or
         the VL comprises: LCDR1 consisting of the sequence of SEQ ID NO: 34, LCDR2 consisting of the sequence of SEQ ID NO:35, and LCDR3 consisting of the sequence of SEQ ID NO: 36;
         or
      (d) the VH comprises: HCDR1 consisting of the sequence of SEQ ID NO: 24, HCDR2 consisting of the sequence of SEQ ID NO:25, HCDR3 consisting of the sequence of SEQ ID NO: 26; and/or
      the VL comprises: LCDR1 consisting of the sequence of SEQ ID NO: 14, LCDR2 consisting of the sequence of SEQ ID NO:15, and LCDR3 consisting of the sequence of SEQ ID NO: 16;
   (3) VH and/or VL, wherein defined by the Kabat numbering system:
      (a) the VH comprises: HCDR1 consisting of the sequence of SEQ ID NO: 7, HCDR2 consisting of the sequence of SEQ ID NO: 8, HCDR3 consisting of the sequence of SEQ ID NO: 9; and/or
         the VL comprises: LCDR1 consisting of the sequence of SEQ ID NO: 17, LCDR2 consisting of the sequence of SEQ ID NO:18, and LCDR3 consisting of the sequence of SEQ ID NO: 19;
      (b) the VH comprises: HCDR1 consisting of the sequence of SEQ ID NO: 27, HCDR2 consisting of the sequence of SEQ ID NO: 28, HCDR3 consisting of the sequence of SEQ ID NO: 29; and/or
         the VL comprises: LCDR1 consisting of the sequence of SEQ ID NO: 37, LCDR2 consisting of the sequence of SEQ ID NO:38, and LCDR3 consisting of the sequence of SEQ ID NO: 39;
      (c) the VH comprises: HCDR1 consisting of the sequence of SEQ ID NO: 7, HCDR2 consisting of the sequence of SEQ ID NO: 8, HCDR3 consisting of the sequence of SEQ ID NO:9; and/or
         the VL comprises: LCDR1 consisting of the sequence of SEQ ID NO: 37, LCDR2 consisting of the sequence of SEQ ID NO:38, and LCDR3 consisting of the sequence of SEQ ID NO: 39;
         or
      (d) the VH comprises: HCDR1 consisting of the sequence of SEQ ID NO: 27, HCDR2 consisting of the sequence of SEQ ID NO:28, HCDR3 consisting of the sequence of SEQ ID NO: 29; and/or

      the VL comprises: LCDR1 consisting of the sequence of SEQ ID NO: 17, LCDR2 consisting of the sequence of SEQ ID NO:18, and LCDR3 consisting of the sequence of SEQ ID NO: 19;
      optionally, the said antibody or antigen binding fragment thereof of the present disclosure comprises a heavy chain variable region VH and/or a light chain variable region VL wherein at least one CDR of the heavy chain variable region VH and/or light chain variable region VL contains a mutation, as compared with the CDRs defined by the aforementioned IMGT, chothia or Kabat, wherein said mutation(s) are one or more amino acid substitutions, deletions or additions or any combination thereof, for example 1, 2 or 3 amino acid substitutions, deletions or additions or any combination thereof; preferably, the substitution is a conservative substitution;
      more preferably, the antibody or antigen-binding fragment thereof binds to human B7-H3 and/or monkey B7-H3.
37. The antibody or antigen-binding fragment thereof of clause 35 or 36, wherein the antibody or antigen-binding fragment thereof comprises:
   (a) a VH set forth in SEQ ID NO 3 or 23, and/or a VL set forth in any one of SEQ ID NO 13 or 33;
   (b) a VH having at least 70%, at least 80%, at least 85%, at least 90%, at least 91%, at least 92%, at least 93%, at least 94%, at least 95%, at least 96%, at least 97%, at least 98% or at least 99% sequence identity as compared with any one of the VHs in (a); and/or a VL having at least 70%, at least 80%, at least 85%, at least 90%, at least 91%, at least 92%, at least 93%, at least 94%, at least 95%, at least 96%, at least 97%, at least 98%, or at least 99% sequence identity as compared with any one of the VLs in (a); or
   (c) a VH having one or more amino acid substitutions, deletions or additions or any combination thereof, for example 1, 2, 3, 4, 5, 6, 7, 8, 9 or 10 amino acid substitutions, deletions or additions or any combination thereof, as compared with any one of the VHs in (a); and/or a VL having one or more amino acid substitutions, deletions or additions or any combination thereof, e.g. 1, 2, 3, 4, 5, 6, 7, 8, 9 or 10 amino acid substitutions, deletions or additions or any combination thereof, as compared with VLs in (a); preferably, the substitution is a conservative substitution.
38. The antibody or antigen-binding fragment thereof of any one of clauses 35 to 37, wherein the antibody or antigen-binding fragment thereof comprises:
   (a) VH consisting of the sequence set forth in SEQ ID NO: 3 and VL consisting of the sequence set forth in SEQ ID NO: 13;
   (b) VH consisting of the sequence set forth in SEQ ID NO: 23 and VL consisting of the sequence set forth in SEQ ID NO: 33;
   (c) VH consisting of the sequence set forth in SEQ ID NO: 3 and VL consisting of the sequence set forth in SEQ ID NO: 33;
   (d) VH consisting of the sequence set forth in SEQ ID NO: 23 and VL consisting of the sequence set forth in SEQ ID NO: 13;
   (e) VH and VL, wherein the VH has at least 70%, at least 80%, at least 85%, at least 90%, at least 91%, at least 92%, at least 93%, at least 94%, at least 95%, at least 96%, at least 97%, at least 98%, or at least 99% sequence identity; and/or, the VL has at least 70%, at least 80%, at least 85%, at least 90%, at least 91%, at least 92%, at least 93%, at least 94%, at least 95%, at least 96%, at least 97%, at least 98%, or at least 99% sequence identity, as compared with the VH and VL in anyone of items(a) to (d); or
   (f) VH and VL, wherein the VH has one or more amino acid substitutions, deletions or additions or any combination thereof, such as 1, 2, 3, 4, 5, 6, 7, 8, 9 or 10 amino acid substitutions, deletions or additions or any combination thereof; and/or, the VL has one or more amino acid substitutions, deletions or additions or any combination thereof, such as 1, 2, 3, 4, 5, 6, 7, 8, 9 or 10 amino acid substitutions, deletions or additions or any combination thereof, as compared with the VH and VL in anyone of items(a) to (d); preferably, the substitution is a conservative substitution.
39. The antibody or antigen-binding fragment thereof of any one of clauses 35 to 38, wherein the antibody or antigen-binding fragment thereof is a chimeric, humanized, or fully human antibody;
   optionally, the antibody or antigen-binding fragment thereof is selected from a scFv, Fab, Fab', (Fab') ₂, Fv fragments, disulfide-linked Fv (dsfv), diabody, and multi-specific antibody;
   optionally, the scFv comprises:
      (i) a sequence set forth in SEQ ID NO:1 or 2;
      (ii) a sequence having one or more amino acid substitutions, deletions or additions or any combination thereof (e.g. up to 50, up to 45, up to 40, up to 35, up to 30, up to 25, up to 20, up to 15, up to 10 or up to 5 amino acid substitutions, deletions or additions or any combination thereof; such as 1, 2, 3, 4, 5, 6, 7, 8, 9 or 10 amino acid substitutions, deletions or additions or any combination thereof) compared with the sequence set forth in (i); or
      (iii) a sequence having at least 80%, at least 85%, at least 90%, at least 91%, at least 92%, at least 93%, at least 94%, at least 95%, at least 96%, at least 97%, at least 98%, or at least 99% sequence identity to the sequence set forth in (i);
   preferably, the substitution(s) recited in (ii) are conservative substitution(s).
40. The antibody or antigen-binding fragment thereof of any one of clauses 35 to 39, wherein the antibody or antigen-binding fragment thereof further comprises:
   (a) a heavy chain constant region CH of a human immunoglobulin or a variant thereof; and/or
   (b) a light chain constant region CL of a human immunoglobulin or a variant thereof, wherein the variant has at least 70%, at least 80%, at least 85%, at least 90%, at least 91%, at least 92%, at least 93%, at least 94%, at least 95%, at least 96%, at least 97%, at least 98%, or at least 99% sequence identity compared with the wild-type sequence from which it is derived; or, the variant has one or more amino acid substitutions, deletions or additions or any combination thereof such as up to 50, up to 45, up to 40, up to 35, up to 30, up to 25, up to 20, up to 15, up to 10 or up to 5 amino acid substitutions, deletions or additions or any combination thereof; such as 1, 2, 3, 4, 5, 6, 7, 8, 9 or 10 amino acid substitutions, deletions or additions or any combination thereof, compared with the wild type sequence from which it is derived; preferably, the substitution(s) recited in (ii) are conservative substitution(s);

   preferably, the heavy chain constant region is an IgG heavy chain constant region, e.g., an IgG1, IgG2, IgG3, or IgG4 heavy chain constant region; and/or, the light chain constant region is a kappa or lambda light chain constant region;
   more preferably, the antibody or antigen-binding fragment thereof comprises a human IgG1 heavy chain constant region; and/or the antibody or antigen-binding fragment thereof comprises a human kappa light chain constant region.
41. The antibody or antigen-binding fragment thereof of clause 40, wherein,
   the heavy chain constant region comprises a CH set forth in SEQ ID NO 43 or a variant thereof having conservative substitutions of up to 20 amino acids, such as conservative substitutions of up to 20, up to 15, up to 10 or up to 5 amino acids, such as conservative substitutions of 1, 2, 3, 4, 5, 6, 7, 8, 9 or 10 amino acids as compared with SEQ ID NO: 43; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 43; and/or
   the light chain constant region comprises a CL set forth in SEQ ID NO:44 or a variant thereof having conservative substitutions of up to 20 amino acids, such as conservative substitutions of up to 20, up to 15, up to 10 or up to 5 amino acids, such as conservative substitutions of 1, 2, 3, 4, 5, 6, 7, 8, 9 or 10 amino acids as compared with SEQ ID NO: 44; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 44;
      preferably, the antibody or antigen-binding fragment thereof comprises the heavy chain constant region CH set forth in SEQ ID NO: 43 and the light chain constant region CL set forth in SEQ ID NO: 44.
42. The antibody or antigen-binding fragment thereof of any one of clauses 35 to 41, wherein the antibody comprises:
   (a) a heavy chain comprising the VH set forth in SEQ ID NO: 3 and the CH set forth in SEQ ID NO: 43, and a light chain comprising the VL set forth in SEQ ID NO: 13 and the CL set forth in SEQ ID NO: 44; preferably a heavy chain set forth in SEQ ID NO: 45 and a light chain set forth in SEQ ID NO: 46;
   (b) a heavy chain comprising the VH set forth in SEQ ID NO: 23 and the CH set forth in SEQ ID NO: 43, and a light chain comprising the VL set forth in SEQ ID NO: 33 and the CL set forth in SEQ ID NO: 44; preferably, a heavy chain set forth in SEQ ID NO: 47 and a light chain set forth in SEQ ID NO: 48.
43. A multispecific antibody comprising the antibody or antigen-binding fragment thereof of any one of clauses 35 to 42, and an additional antibody or fragment or antibody analog thereof;
   preferably, the multi-specific antibody is a bispecific antibody or a tri-specific antibody or a tetra-specific antibody.
   or a stereoisomer of said ligand drug conjugate, a prodrug thereof, a pharmaceutically acceptable salt thereof, or a pharmaceutically acceptable solvate thereof.
44. An isolated nucleic acid molecule encoding the antibody or antigen-binding fragment thereof of any one of clauses 35 to 42, or the multi-specific antibody of clause 43.
45. A vector comprising the isolated nucleic acid molecule of clause 44; preferably, the vector is a cloning vector or an expression vector.
46. A host cell comprising the isolated nucleic acid molecule of clause 44 or the vector of clause 45.
47. A method of preparing the antibody or antigen-binding fragment thereof of any one of clauses 35 to 42, or the multi-specific antibody of clause 43, comprising culturing the host cell of clause 46 under conditions that allow expression of the antibody or antigen-binding fragment thereof, and recovering the antibody or antigen-binding fragment thereof, or the multi-specific antibody, from the cultured host cell culture.
48. An antibody-drug conjugate, wherein the antibody is the antibody or antigen-binding fragment thereof of any one of clauses 35 to 42, or the multi-specific antibody of clause 43, said antibody is linked by a linker to a coupling moiety selected from: a detectable label, a radioisotope, a fluorescent agent, a luminescent agent, a colored agent, an enzyme, polyethylene glycol, a nuclide, a nucleic acid, a small molecule toxin, a polypeptide having binding activity, a protein, a receptor, a ligand, and other active agent that inhibits tumor cell growth and/or promotes tumor cell apoptosis or necrosis.
49. A ligand drug conjugate comprising the ligand drug conjugate of any one of clauses 1-28, said ligand drug conjugate having two or more q values;
   optionally, the ratio of drug to antibody (DAR) in the ligand drug conjugate is selected from an integer or decimal between 1 and 10;
   preferably, the ratio of drug to antibody (DAR) in the ligand drug conjugate is selected from 1.5-2.5, 3.5-4.5, 5.5-6.5 and 7.5-8.5;
   preferably, the ratio of drug to antibody (DAR) in the ligand drug conjugate is selected from about 2.0, 4.0, 6.0 and 8.0;
   preferably, the ratio of drug to antibody (DAR) in the ligand drug conjugate is selected from 2, 2.5, 3, 3.5, 4, 4.5,5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9 and 9.
50. A pharmaceutical composition comprising substance A and optionally one or more pharmaceutically acceptable adjuvants, wherein said substance A is the ligand drug conjugate of any one of clauses 1 to 24, or a stereoisomer of the ligand drug conjugate, a prodrug thereof, a pharmaceutically acceptable salt thereof, or a pharmaceutically acceptable solvate thereof, or the compound of clause 26 or 27, or the drug linker conjugate of any one of clauses 28 to 32, or the antibody or antigen-binding fragment thereof of any one of clauses 35 to 42, or the multi-specific antibody of clause 43, or the nucleic acid molecule of clause 44, or the vector of clause 45, or the host cell of clause 46, or the ligand drug conjugate of clause 49; preferably, the composition further comprises a pharmaceutically acceptable carrier and/or excipient.
51. Use of a substance A or a pharmaceutical composition according to clause 50 for the preclausetion of a medicament for the treatment and/or prevention of a disease associated with abnormal cell activity; wherein said substance A is the ligand drug conjugate of any one of clauses 1 to 24, or a stereoisomer of the ligand drug conjugate, a prodrug thereof, a pharmaceutically acceptable salt thereof, or a pharmaceutically acceptable solvate thereof, or the compound of clause 26 or 27, or the drug linker conjugate of any one of clauses 28 to 32, or the antibody or antigen-binding fragment thereof of any one of clauses 35 to 42, or the multi-specific antibody of clause 43, or the nucleic acid molecule of clause 44, or the vector of clause 45, or the host cell of clause 46, or the ligand drug conjugate of clause 49; wherein the disease associated with abnormal cell activity can be a cancer disease;
   preferably, the cancer disease is selected from esophageal cancer, brain tumor, lung cancer, squamous cell carcinoma, bladder cancer, stomach cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, urothelial cancer, epidermal cancer, non-Hodgkin lymphoma, central nervous system tumor, prostate cancer or thyroid cancer; wherein said esophageal cancer is e.g., esophageal adenocarcinoma or esophageal squamous cell carcinoma; said lung cancer is e.g., small cell lung cancer, non-small cell lung cancer or lung adenocarcinoma; said central nervous system tumor is e.g., neuroglioma, glioblastoma multiforme, glioma or sarcoma; said colon cancer is e.g., human colon adenocarcinoma;
   preferably, the cancer disease is selected from colon cancer, colorectal cancer, colon adenocarcinoma, lung cancer, breast cancer, prostate cancer, esophageal squamous carcinoma;
   more preferably, the cancer disease is a cancer disease associated with B7H3;
   more preferably, the cancer disease is a cancer disease associated with Her3;
   more preferably, the cancer disease is a cancer disease associated with EGFR;
   more preferably, the cancer disease is a cancer disease associated with Trop-2 or Her2;
   most preferably, the cancer disease is breast cancer or the lung cancer is non-small cell lung cancer.

## Claims

1. A ligand drug conjugate of formula I-A or formula I-B: 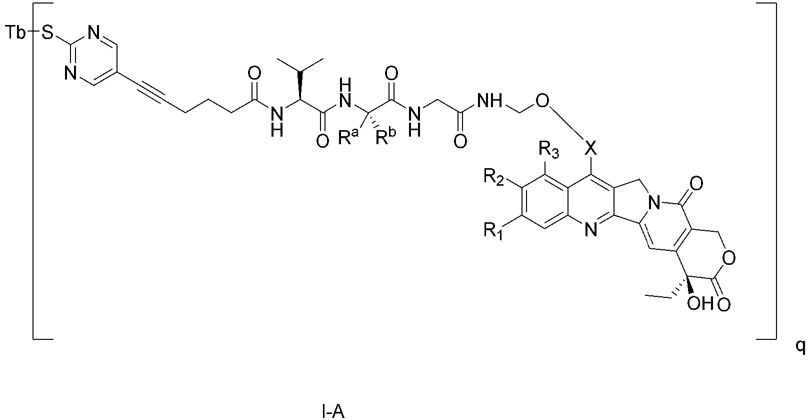 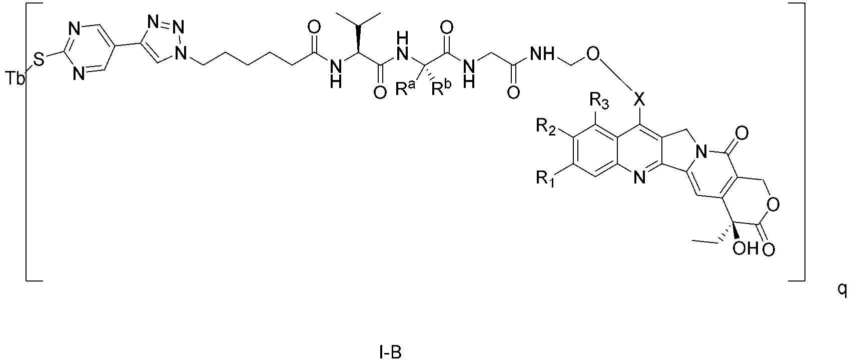
or a stereoisomer of said ligand drug conjugate, a pharmaceutically acceptable salt thereof, or a pharmaceutically acceptable solvate thereof,
wherein:
Tb is an anti-B7H3 antibody or antigen-binding fragment thereof, wherein the anti-B7H3 antibody or antigen-binding fragment thereof comprises the Complementarity Determining Regions (CDRs) as shown below, wherein the CDRs are defined by the Kabat numbering system:
HCDR1 consisting of the sequence of SEQ ID NO: 27, HCDR2 consisting of the sequence of SEQ ID NO: 28, HCDR3 consisting of the sequence of SEQ ID NO: 29; and/or,
LCDR1 consisting of the sequence of SEQ ID NO: 37, LCDR2 consisting of the sequence of SEQ ID NO: 38, and LCDR3 consisting of the sequence of SEQ ID NO: 39;
R₁ and R₂ are each independently selected from H, halogen, C1-4 alkyl; or, R₁ and R₂, together with the carbon atoms to which they are attached, form 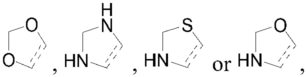
the dotted line indicates where the heterocyclic ring is fused to the benzene ring;
R₃ is H;
X is selected from 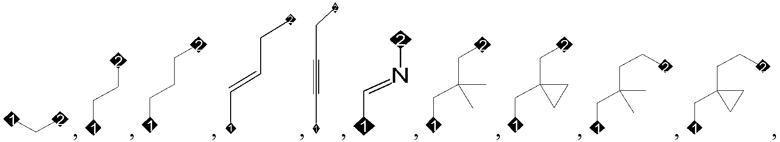 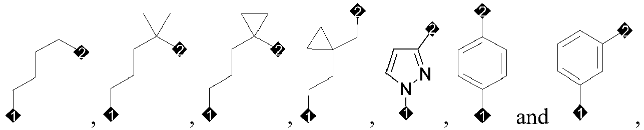 in which position 1 is attached to the parent ring and position 2 is attached to O;
wherein one of R^{a} and R^{b} is H, and the other is 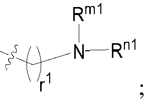
R^{m1} and Rⁿ¹ are each independently selected from H, C1-6 alkyl, C3-6 cycloalkyl and t-butoxycarbonyl;
r¹ is selected from 0, 1, 2, 3, 4, and 5; and
q is a drug-to-ligand coupling ratio selected from any numerical value between 0.1 and 16.0.

2. The ligand drug conjugate of claim 1, or a stereoisomer of said ligand drug conjugate, a pharmaceutically acceptable salt thereof, or a pharmaceutically acceptable solvate thereof, wherein,
R^{m1} and Rⁿ¹ are each independently selected from H, C1-6 alkyl, C3-6 cycloalkyl and t-butoxycarbonyl; optionally R^{m1} and Rⁿ¹ are each independently selected from H and C1-6 alkyl.

3. The ligand drug conjugate according to any one of claims 1 or 2, or a stereoisomer of said ligand drug conjugate, a pharmaceutically acceptable salt thereof, or a pharmaceutically acceptable solvate thereof, wherein R₁ and R₂ together with the carbon atoms to which they are attached form 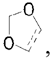 the dotted line indicates where the heterocyclic ring is fused to the benzene.

4. The ligand drug conjugate of any one of claims 1 to 3, or a stereoisomer of said ligand drug conjugate, a pharmaceutically acceptable salt thereof, or a pharmaceutically acceptable solvate thereof having the formula: 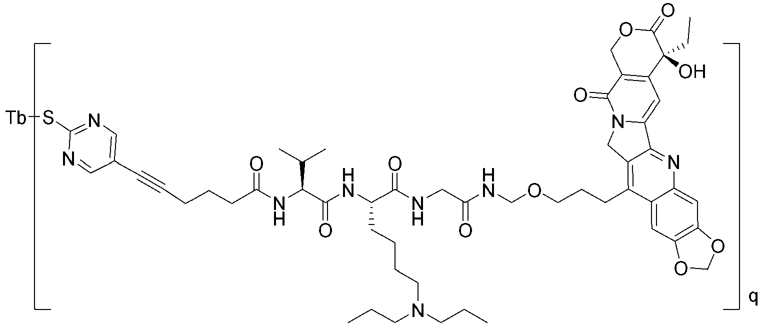 wherein Tb and q are as defined in any one of claims 1 to 4.

5. The ligand drug conjugate of any one of claims 1 to 4, or a stereoisomer of said ligand drug conjugate, a pharmaceutically acceptable salt thereof, or a pharmaceutically acceptable solvate thereof, wherein q is a drug-to-ligand coupling ratio selected from any numerical value between 2 and 8.

6. The ligand drug conjugate of any one of claims 1 to 5, wherein the anti-B7H3 antibody or antigen-binding fragment thereof comprises:
a) a VH set forth in SEQ ID NO: 23, and/or a VL set forth in SEQ ID NO: 33; or
b) a heavy chain set forth in SEQ ID NO: 47, and/or a light chain set forth in SEQ ID NO: 48; or
c) a heavy chain set forth in SEQ ID NO: 47, and a light chain set forth in SEQ ID NO: 48.

7. A ligand drug conjugate of the following formula 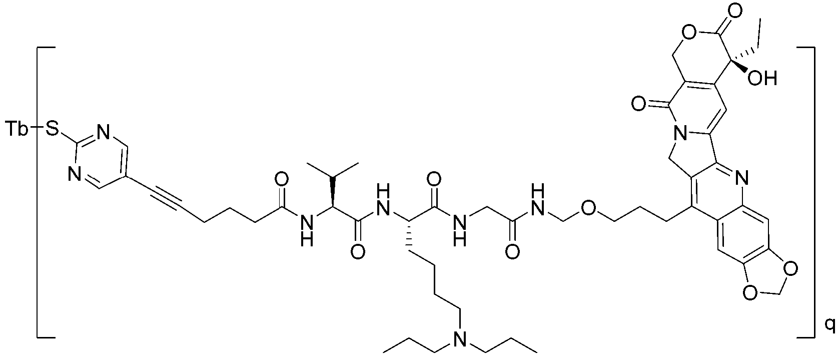
or a stereoisomer of said ligand drug conjugate, a pharmaceutically acceptable salt thereof, or a pharmaceutically acceptable solvate thereof,
wherein
Tb is an anti-B7H3 antibody having a heavy chain set forth in SEQ ID NO: 47 and a light chain set forth in SEQ ID NO: 48; and
q is 1-10, preferably 7.5-8.5.

8. A process for preparing the ligand drug conjugate according to any one of claims 1 to 7, which process comprises:
performing a coupling reaction of Tb and a drug linker conjugate of formula IIIA or IIIB 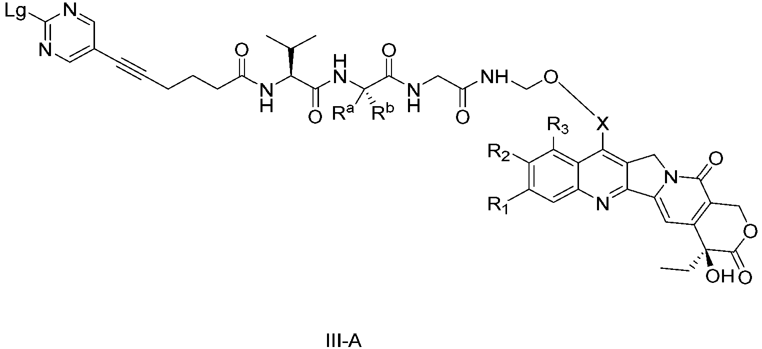 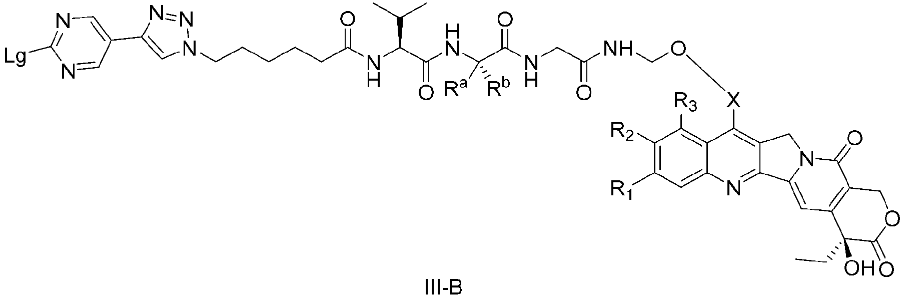
in a suitable solvent under suitable conditions;
wherein:
wherein X, R₁, R₂, R₃, R^{a}, R^{b}, and q are as defined in any one of claims 1 to 7;
Lg is a leaving group and is selected from halogen, sulfone group, tertiary amine salt group (Me₃N⁺, Et₃N⁺), diazonium salt group, -OMs, MeSO₂-, and CF₃SO₃-;
preferably, Lg is selected from F, Cl and MeSO₂-; the tertiary amine salt group is selected from Me₃N⁺ and Et₃N⁺;
more preferably, Lg is selected from F and MeSO₂-; optionally
wherein the process comprises the step of performing a coupling reaction of Tb and drug linker conjugate of formula IIIA or IIIB in a suitable solvent and under suitable conditions to form a C-S bond;
preferably, the ratio of said Tb to said drug linker conjugate in amount of substance is 1: (1-20);
preferably, the coupling reaction is carried out in water and/or an organic solvent; preferably, the organic solvent is selected from N, N-dimethylformamide, dimethylsulfoxide, N-methylpyrrolidone, nitriles (e.g. acetonitrile), alcohols (e.g. methanol, ethanol) or any combination thereof; the nitrile can be acetonitrile, and the alcohol can be methanol, ethanol;
preferably, the process further comprises the step of purifying the coupling product; preferably, the coupling product is purified by chromatography; preferably, the chromatography comprises one or more of ion exchange chromatography, hydrophobic chromatography, reverse phase chromatography or affinity chromatography.

9. The process according to claim 8, **characterized in that** the compound of Formula IIIA or IIIB is selected from the following:

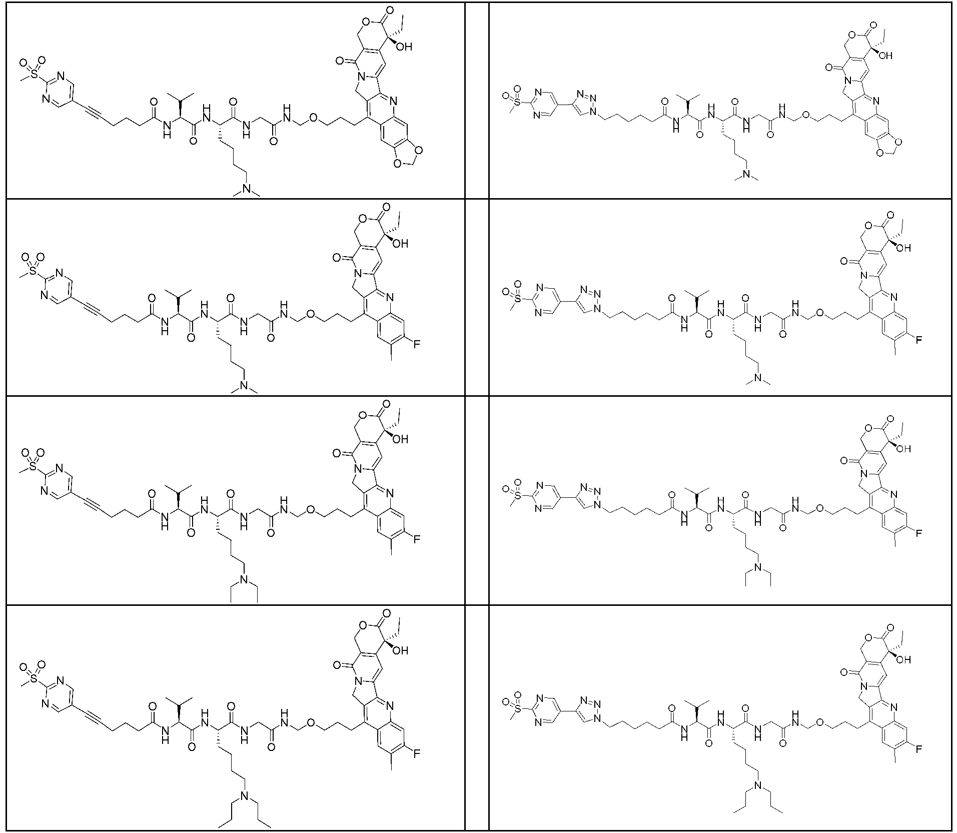

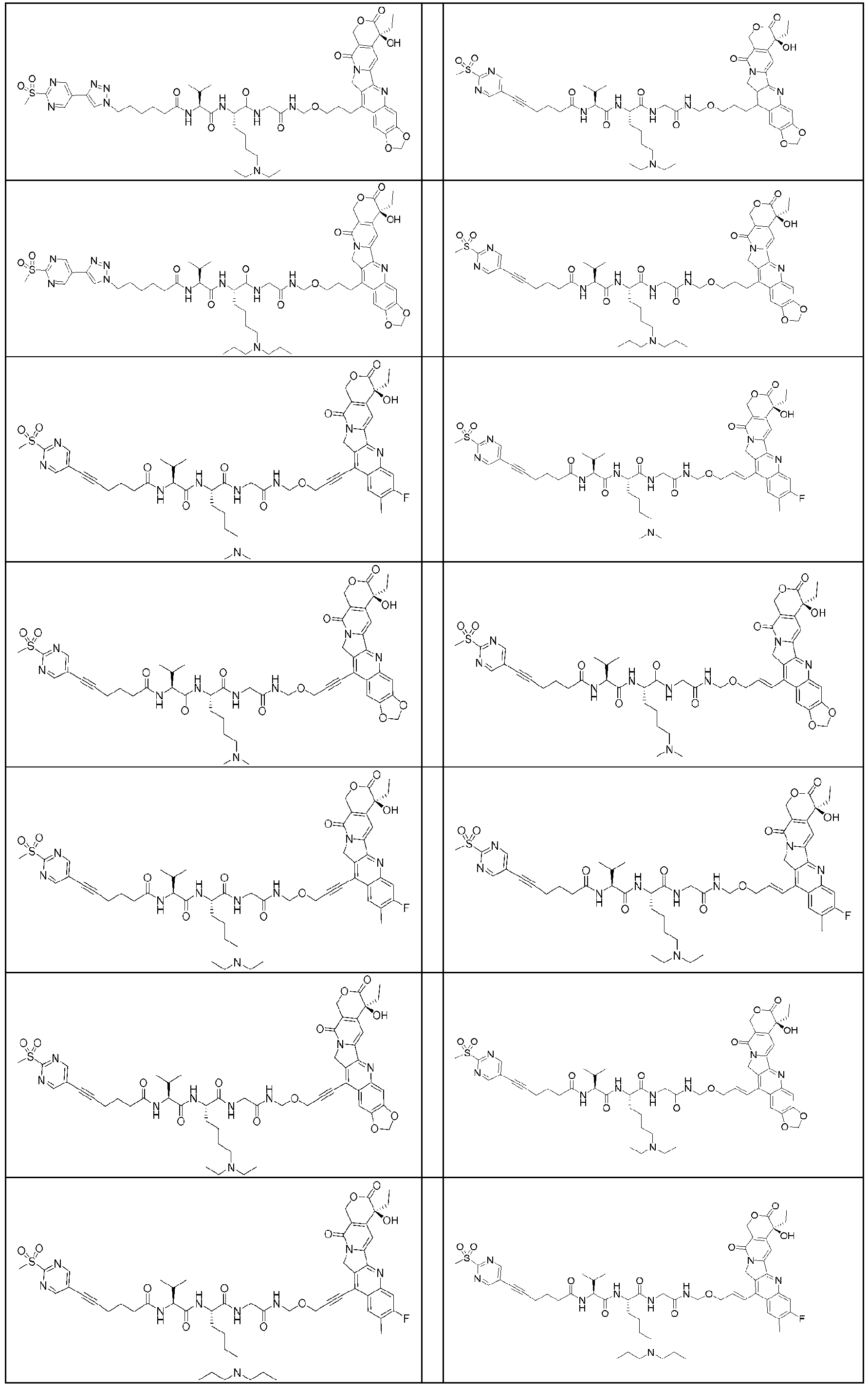

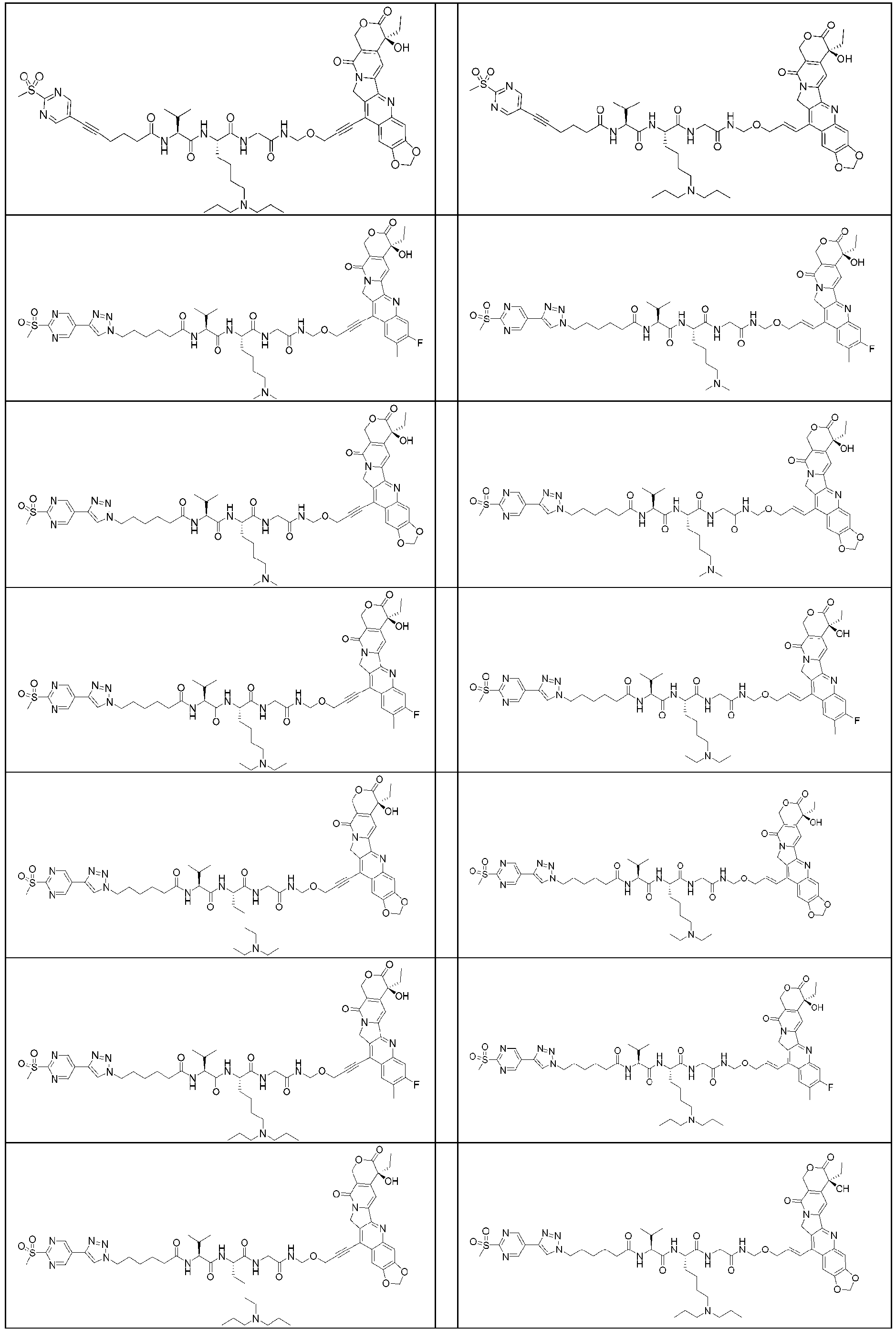

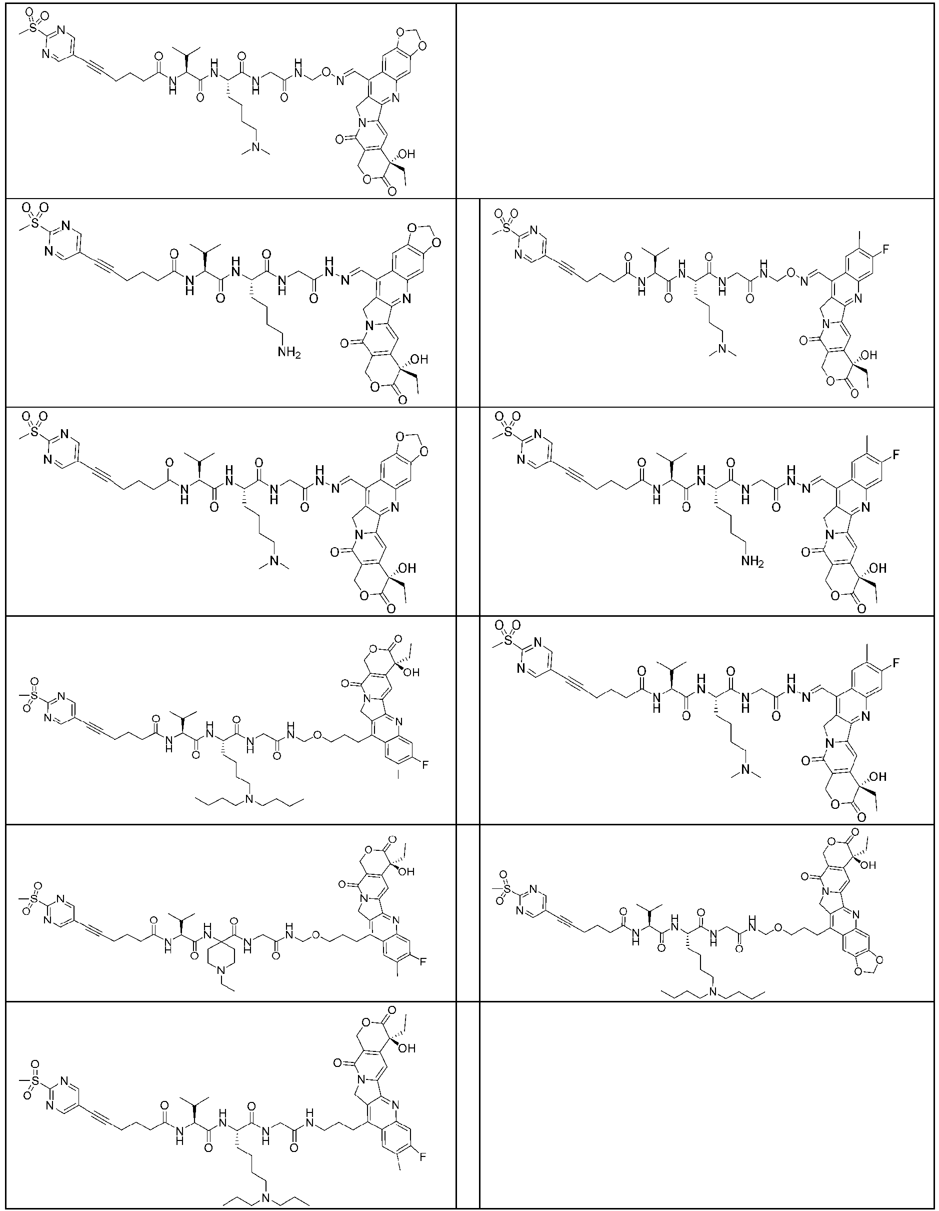

10. An antibody or antigen-binding fragment thereof that binds B7H3, said antibody or antigen-binding fragment comprises:
(a) the heavy chain variable region VH set forth in SEQ ID NO: 23, and
(b) the light chain variable region VL set forth in SEQ ID NO: 33; optionally
wherein:
the heavy chain constant region comprises a CH set forth in SEQ ID NO: 43 ; and
the light chain constant region comprises a CL set forth in SEQ ID NO: 44; optionally
wherein the antibody comprises: a heavy chain set forth in SEQ ID NO: 47 and a light chain set forth in SEQ ID NO: 48.

11. A multi-specific antibody comprising the antibody or antigen-binding fragment thereof of claim 10, and an additional antibody or fragment or antibody analog thereof;
preferably, the multi-specific antibody is a bispecific antibody or a tri-specific antibody or a tetra-specific antibody.
or a stereoisomer of said ligand drug conjugate, a pharmaceutically acceptable salt thereof, or a pharmaceutically acceptable solvate thereof.

12. An isolated nucleic acid molecule encoding the antibody or antigen-binding fragment thereof of claim 10, or the multi-specific antibody of claim 11.

13. A vector comprising the isolated nucleic acid molecule of claim 12; preferably, the vector is a cloning vector or an expression vector.

14. A host cell comprising the isolated nucleic acid molecule of claim 12 or the vector of claim 13.

15. A method of preparing the antibody or antigen-binding fragment thereof of claim 10, or the multi-specific antibody of claim 11, comprising culturing the host cell of claim 14 under conditions that allow expression of the antibody or antigen-binding fragment thereof, and recovering the antibody or antigen-binding fragment thereof, or the multi-specific antibody, from the cultured host cell culture.

16. The ligand drug conjugate of any one of claims 1 to 7, or process of any one of claims 8 to 9, wherein Tb is an antibody or antigen-binding fragment thereof that binds B7H3 of claim 10 or a multi-specific antibody of claim 11.

17. An antibody-drug conjugate, wherein the antibody is the antibody or antigen-binding fragment thereof of claim 10, or the multi-specific antibody of claim 11, said antibody is linked by a linker to a coupling moiety selected from: a detectable label, a radioisotope, a fluorescent agent, a luminescent agent, a colored agent, an enzyme, polyethylene glycol, a nuclide, a nucleic acid, a small molecule toxin, a polypeptide having binding activity, a protein, a receptor, a ligand, and other active agent that inhibits tumor cell growth and/or promotes tumor cell apoptosis or necrosis.

18. A population of ligand drug conjugates comprising the ligand drug conjugate of any one of claims 1 to 7, said ligand drug conjugate having two or more q values in the population; wherein
the mean ratio of drug to antibody (DAR) in the population of ligand drug conjugates is selected from 7.5-8.5.

19. A pharmaceutical composition comprising substance A and optionally one or more pharmaceutically acceptable adjuvants, wherein said substance A is the ligand drug conjugate of any one of claims 1 to 7, 16 or 17 or a stereoisomer of the ligand drug conjugate, a pharmaceutically acceptable salt thereof, or a pharmaceutically acceptable solvate thereof, or the antibody or antigen-binding fragment thereof of claim 10, or the multi-specific antibody of claim 11, or the nucleic acid molecule of claim 12, the vector of claim 13, or the host cell of claim 14, or the population of ligand drug conjugates of claim 18; preferably, the composition further comprises a pharmaceutically acceptable carrier and/or excipient.

20. A substance A or a pharmaceutical composition according to claim 19 for use in the treatment and/or prevention of a disease associated with abnormal cell activity; wherein said substance A is the ligand drug conjugate of any one of claims 1 to 7, 16 or 17 or a stereoisomer of the ligand drug conjugate, a pharmaceutically acceptable salt thereof, or a pharmaceutically acceptable solvate thereof, or the antibody or antigen-binding fragment thereof of claim 10, or the multi-specific antibody of claim 11, or the nucleic acid molecule of claim 12, the vector of claim 13, or the host cell of claim 14, or the population of ligand drug conjugates of claim 18; wherein the disease associated with abnormal cell activity can be a cancer disease;
preferably, the cancer disease is selected from esophageal cancer, brain tumor, lung cancer, squamous cell carcinoma, bladder cancer, stomach cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, urothelial cancer, epidermal cancer, non-Hodgkin lymphoma, central nervous system tumor, prostate cancer or thyroid cancer; wherein said esophageal cancer is e.g., esophageal adenocarcinoma or esophageal squamous cell carcinoma; said lung cancer is e.g., small cell lung cancer, non-small cell lung cancer or lung adenocarcinoma; said central nervous system tumor is e.g., neuroglioma, glioblastoma multiforme, glioma or sarcoma; said colon cancer is e.g., human colon adenocarcinoma;
preferably, the cancer disease is selected from colon cancer, colorectal cancer, colon adenocarcinoma, lung cancer, breast cancer, prostate cancer, esophageal squamous carcinoma;
more preferably, the cancer disease is a cancer disease associated with B7H3;
most preferably, the cancer disease is breast cancer or the lung cancer is non-small cell lung cancer.
